# EUROPEAN PATENT APPLICATION

(11) **EP 4 317 168 A1**
(43) Date of publication of application: **07.02.2024**
(21) Application number: 22779102.7
(22) Date of filing: 31.03.2022
(51) Int. Cl.: C07H 19/10, C07H 21/00, C07H 21/04, C12Q 1/68, A61K 31/70, A61K 31/7042, A61K 31/7052

(54) **NUCLEOTIDE ANALOGUE FOR SEQUENCING**

(30) Priority: 01.04.2021 CN 202110355785; 09.03.2022 WO PCT/CN2022/080021
(71) Applicant: BGI Shenzhen, Shenzhen, Guangdong 518083 (CN); BGI Shenzhen Group Holdings Co., Limited, Shenzhen, Guangdong 518083 (CN)
(72) Inventor: XU, Xun, Shenzhen, Guangdong 518083 (CN); TENG, Bo, Shenzhen, Guangdong 518083 (CN); ZHANG, Wenwei, Shenzhen, Guangdong 518083 (CN); SHEN, Liang, Shenzhen, Guangdong 518083 (CN); ZHUO, Shitian, Shenzhen, Guangdong 518083 (CN); YAN, Shengyi, Shenzhen, Guangdong 518083 (CN); GAO, Nanfeng, Shenzhen, Guangdong 518083 (CN); ZHANG, Yonghui, Shenzhen, Guangdong 518083 (CN); ZHAO, Jie, Shenzhen, Guangdong 518083 (CN); LIAO, Sha, Shenzhen, Guangdong 518083 (CN); WANG, Ming, Shenzhen, Guangdong 518083 (CN); ZHANG, Shaoqiao, Shenzhen, Guangdong 518083 (CN); CHEN, Ao, Shenzhen, Guangdong 518083 (CN); LI, Handong, Shenzhen, Guangdong 518083 (CN)
(74) Representative: Inspicos P/S
(86) International application number: PCT/CN2022/084601
(87) International publication number: WO 2022/206922

(57) **Abstract**

The present invention relates to the field of nucleic acid sequencing. Specifically, provided in the present invention is a nucleotide analogue for sequencing. The nucleotide analogue carries a linker on the base or the ribose ring 3'-OH, and can be used for NGS sequencing. The present invention further relates to a kit containing the nucleotide analogue, and a sequencing method based on the nucleotide analogue.

## Description

### Technical field

The present invention relates to the field of nucleic acid sequencing. Specifically, the present invention relates to nucleotide analogues for sequencing.

### Background art

The emergence of NGS sequencing overcomes the disadvantages of Sanger's high cost and time requirements, and greatly promotes the application of gene sequencing technology. At present, NGS sequencing has been deeply applied in fields such as prenatal screening, tumor diagnosis, tumor treatment, animal and plant breeding, and promotes the progress of science and medicine.

Deoxy-ribonucleoside triphosphate (dNTP) analogues carrying reversible blocking groups are key raw materials in NGS sequencing. Due to the incorporation of reversible blocking group, the 3'-OH group in dNTP can be retained, which overcomes the shortcomings of Sanger sequencing and ensures the accuracy of base recognition. It can be said that deoxy-ribonucleoside triphosphate (dNTP) analogue with reversible blocking group is the most critical technology in NGS sequencing, and the application of nucleotides with cleavable linkers and detectable labels in NGS sequencing is the key in realizing base detection.

### Summary of the invention

The invention intends to develop a type of dNTP analogues useful for NGS sequencing, such as those having ester or carbonate ester of cleavable detectable label on base or ribose ring 3'-OH of the nucleotide.

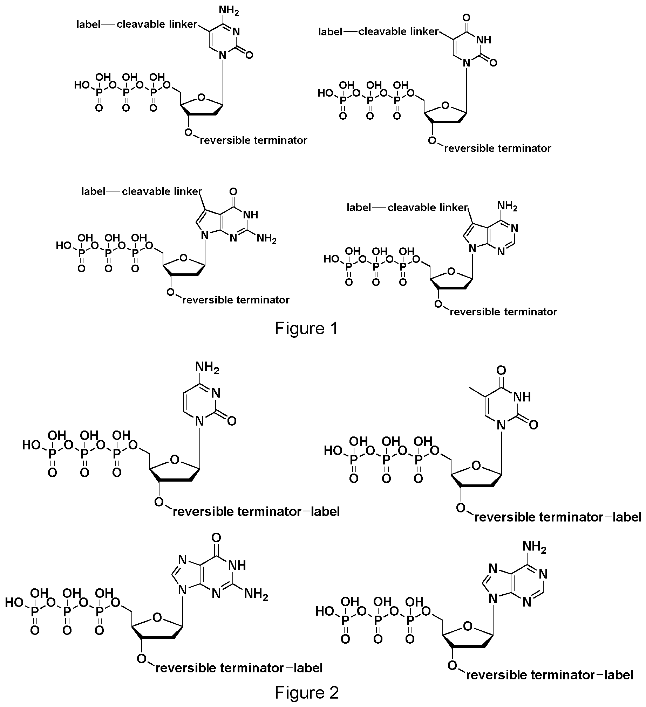

A first type of dNTP analogues has the general structures showed in figure 1, illustrating the nucleotides having cleavable detectable labels on base, including the main structures of 2'-deoxyuridine triphosphate, 2'-deoxycytidine triphosphate, 7-deaza-2'-deoxyadenosine triphosphate and 7-deaza-2'-deoxyguanosine triphosphate. In this first type of dNTP analogues, the ribose ring 3'-OH is connected with arbitrary reversible blocking groups that are conventionally used on 3'-OH in the art and result in excellent effects. A second type of dNTP analogues has the general structures showed in figure 2, illustrating the nucleotides with detectable labels connected to the reversible blocking group on the ribose ring 3'-OH.

To this end, in the first aspect of the invention, the present invention provides a compound of formula I or formula II or a salt thereof, 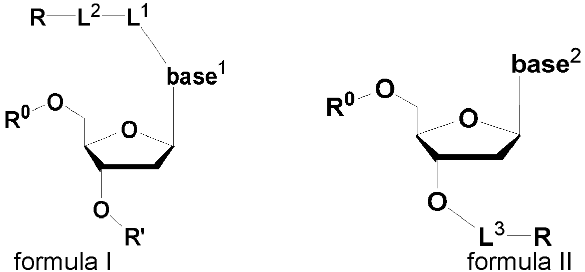 wherein:
L¹ is selected from 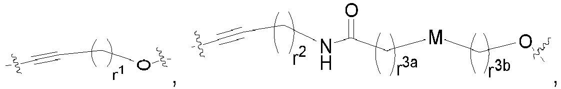 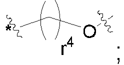
   r¹, r², r^{3a}, r^{3b}, r⁴, independently of each other, are selected from any integer between 1 and 6;
   M is selected from a direct bond, CH₂, NH, O, S;
L² is 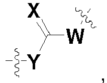 preferably, W being linked to R;
L³ is 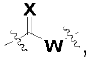 preferably, W being linked to R;
   each X, independently, is selected from O, S, NH;
   Y is selected from a direct bond, O, S, NH;
   each W, independently, is selected from a direct bond, O, S NH;
R is 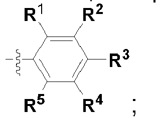 in R¹, R², R³, R⁴, R⁵, any one is 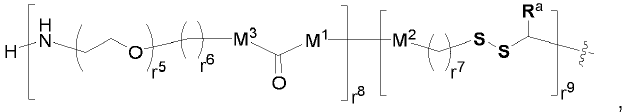 and the others, independently of each other, are selected from H, 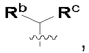 azido, nitro, amino, sulfo, carboxyl, aliphatic alkyl (e.g., C₁-C₆ alkyl), cycloalkyl (e.g., C₃-C₆ cycloalkyl), aromatic alkyl (e.g., phenyl -C₁-C₆ alkyl), F, I, Br, Cl, alkoxyl (e.g., C₁-C₆ alkoxyl);
   r⁵, r⁶, r⁷, independently of each other, are selected from any integer between 1 and 6;
   r⁸, r⁹, independently of each other, are selected from 0, 1, r⁸ and r⁹ being not 0 at the same time;
   M¹, M², M³, independently of each other, are selected from a direct bond, NH, O, S;
   R^{a} is selected from H, aliphatic alkyl (e.g., C₁-C₆ alkyl, such as Me, Et, iPr, tBu), aromatic alkyl (e.g., phenyl -C₁-C₆ alkyl), cycloalkyl (e.g., C₃-C₆ cycloalkyl);
   in R^{b}, R^{c}, any one is selected from -N₃, -SS-C₁-C₆ alkyl (e.g., -SS-Me, -SS-Et, -SS-iPr, -SS-t-Bu), -ONH₂, -OCORₓ, -OCONHRₓ, and the other is selected from H, aliphatic alkyl (e.g., C₁-C₆ alkyl, such as Me, Et, iPr, tBu), aromatic alkyl (e.g., phenyl -C₁-C₆ alkyl), cycloalkyl (e.g., C₃-C₆ cycloalkyl), wherein each Rₓ, independently, is selected from aliphatic alkyl (e.g., C₁-C₆ alkyl), cycloalkyl (e.g., C₃-C₆ cycloalkyl) or aromatic alkyl (e.g., phenyl C₁-C₆ alkyl);
R° is selected from H, monophosphate group 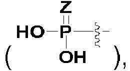 diphosphate group 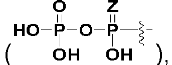 triphosphate group 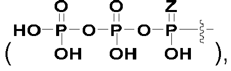 tetraphosphate group 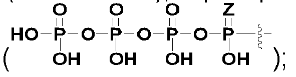 each Z, independently, is selected from O, S, BH;
base¹, base², independently of each other, are selected from a base, a deaza base or a tautomer thereof, for example, adenine, 7-deazaadenine, thymine, uracil, cytosine, guanine, 7-deazaguanine or a tautomer thereof;
in the formula I. R' reresents a reversible blocking group, in the formula II, 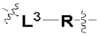 represents a reversible blocking group.

In some embodiments, L¹ is selected from

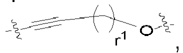

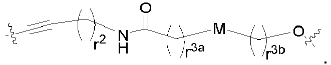

In some embodiments, L¹ is selected from

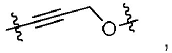

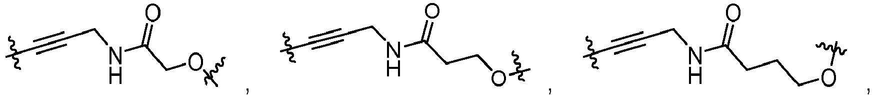

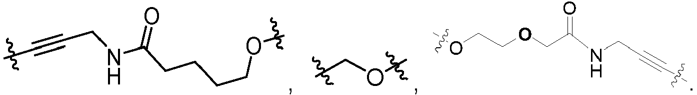

In some embodiments, L¹ is selected from

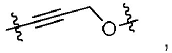

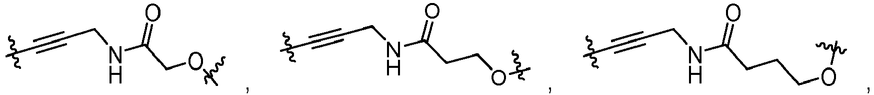

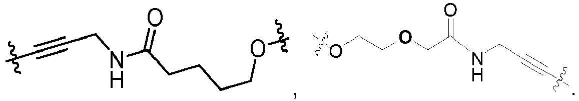

In some embodiments, L¹ is selected from

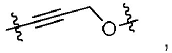

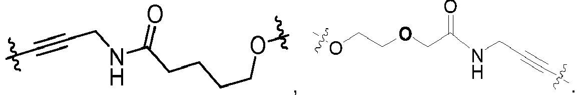

In some embodiments, r¹ is selected from 1, 2, 3.

In some embodiments, r¹ is 1.

In some embodiments, r² is selected from 1, 2, 3.

In some embodiments, r² is 1.

In some embodiments, r^{3a}, r^{3b}, independently of each other, are selected from 1, 2, 3, 4, 5.

In some embodiments, r^{3a}, r^{3b}, independently of each other, are selected from 1, 2, 3.

In some embodiments, r^{3a} is 1.

In some embodiments, r^{3b} is 2.

In some embodiments, r⁴ is selected from 1, 2, 3.

In some embodiments, r⁴ is 1.

In some embodiments, M is selected from CH₂, O.

In some embodiments, each X, independently, is selected from O, S.

In some embodiments, X is O.

In some embodiments, Y is a direct bond.

In some embodiments, W is a direct bond.

In some embodiments, in R¹, R², R³, R⁴, R⁵, any one is 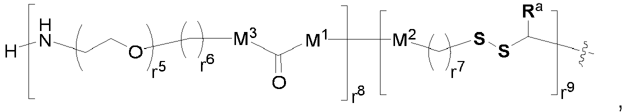 and the others, independently of each other, are selected from H, 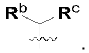 .

In some embodiments, in R¹, R², R³, R⁴, R⁵, any one is 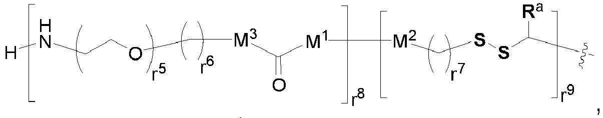 another (e.g., R¹ or R⁵) is selected from H, 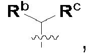 and the remaining three are H.

In some embodiments, r⁵ is selected from 1, 2, 3.

In some embodiments, r⁵ is 2.

In some embodiments, r⁶ is selected from 1, 2, 3.

In some embodiments, r⁶ is 1 or 2.

In some embodiments, r⁷ is selected from 1, 2, 3.

In some embodiments, r⁷ is 2.

In some embodiments, M¹ is selected from a direct bond, NH, O.

In some embodiments, M² is NH.

In some embodiments, M³ is selected from a direct bond, NH.

In some embodiments, R^{a} is selected from H, C₁-C₆ alkyl.

In some embodiments, R^{a} is methyl.

In some embodiments, in R^{b}, R^{c}, any one is selected from -N₃, -SS-C₁-C₆ alkyl (e.g., -SS-Me, -SS-Et, -SS-iPr, -SS-t-Bu), and the other is selected from C₁-C₆ alkyl.

In some embodiments, in R^{b}, R^{c}, any one is selected from -N₃, -SS-Me, - SS-Et, and the other is methyl.

In some embodiments, in R^{b}, R^{c}, any one is selected from -N₃, -SS-Me, and the other is methyl.

In some embodiments R is selected from

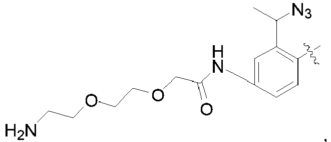

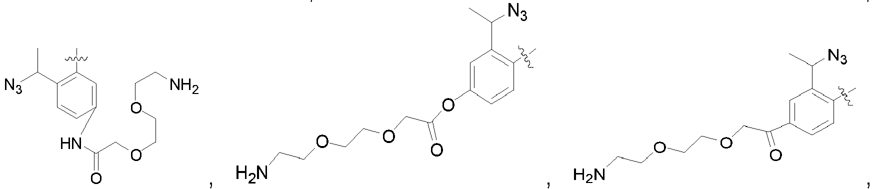

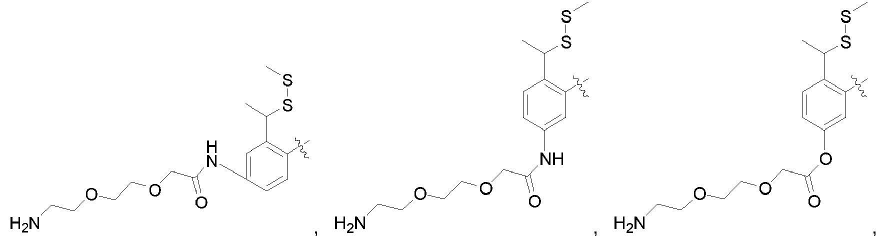

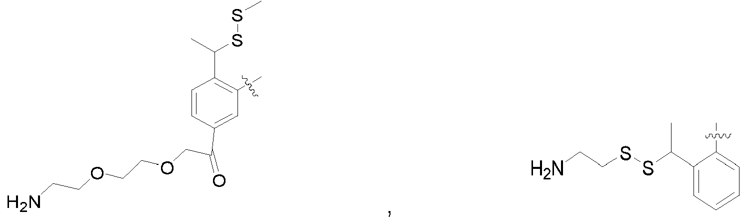

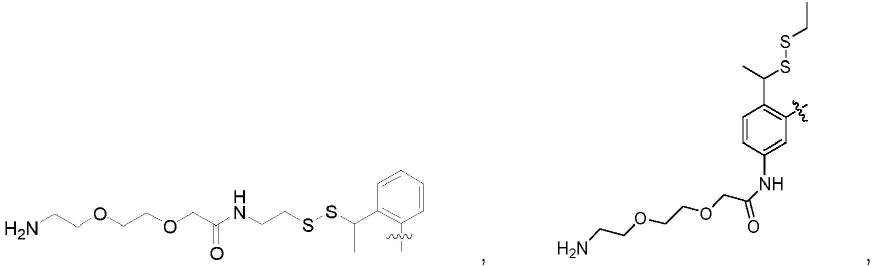

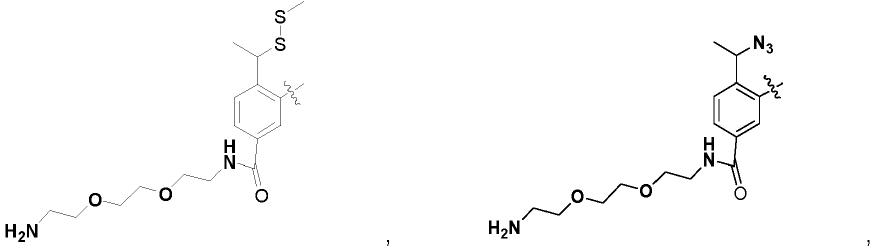

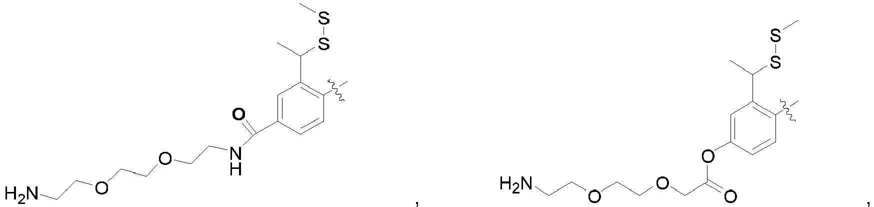

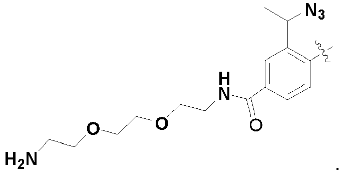

In some embodiments, R° is a triphosphate group

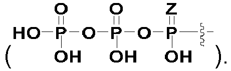

In some embodiments, Z is O.

In some embodiments, base¹ is selected from

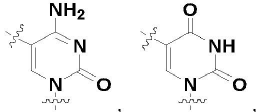

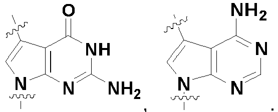

In some embodiments, base² is selected from

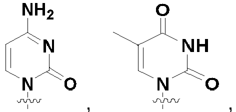

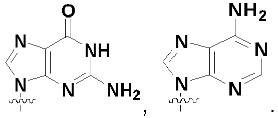

In the second aspect of the invention, the present invention provides a compound of formula I-1 or a salt thereof, 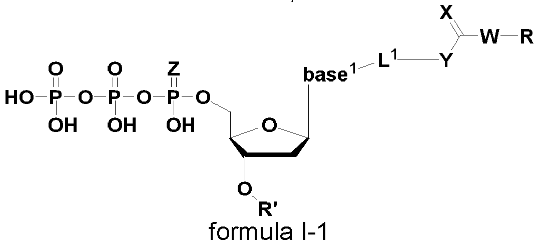 wherein:
L¹ is selected from 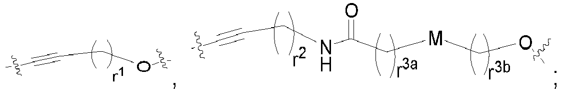
   r¹, r², r^{3a}, r^{3b}, independently of each other, are selected from any integer between 1 and 6;
   M is selected from a direct bond, CH₂, NH, O, S;
X is selected from O, S, NH;
Y is selected from a direct bond, O, S, NH;
W is selected from a direct bond, O, S, NH;
R is 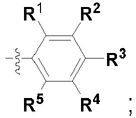 in R¹, R², R³, R⁴, R⁵, any one (e.g., R², R³ or R⁴) is 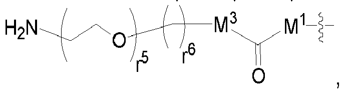 and the others, independently of each other, are selected from H, 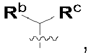 azido, nitro, amino, sulfo carboxyl, aliphatic alkyl (e.g., C₁-C₆ alkyl), cycloalkyl (e.g., C₃-C₆ cycloalkyl), aromatic alkyl (e.g., phenyl -C₁-C₆ alkyl), F, I, Br, Cl, alkoxyl (e.g., C₁-C₆ alkoxyl);
   r⁵, r⁶, independently of each other, are selected from any integer between 1 and 6;
   M¹, M³, independently of each other, are selected from a direct bond, NH, O, S;
   in R^{b}, R^{c}, any one is selected from -N₃, -SS-C₁-C₆ alkyl (e.g., -SS-Me, -SS-Et, -SS-iPr, -SS-t-Bu), -ONH₂, -OCORₓ, -OCONHRₓ, and the other is selected from H, aliphatic alkyl (e.g., C₁-C₆ alkyl, such as Me, Et, iPr, tBu), aromatic alkyl (e.g., phenyl -C₁-C₆ alkyl), cycloalkyl (e.g., C₃-C₆ cycloalkyl), wherein each Rₓ, independently, is selected from aliphatic alkyl (e.g., C₁-C₆ alkyl), cycloalkyl (e.g., C₃-C₆ cycloalkyl) or aromatic alkyl (e.g., phenyl C₁-C₆ alkyl);
Z is selected from O, S, BH;
base¹ is selected from a base, a deaza base or a tautomer thereof, for example, adenine, 7-deazaadenine, thymine, uracil, cytosine, guanine, 7-deazaguanine or a tautomer thereof;
R' represents a reversible blocking group.

In some embodiments, L¹ is selected from

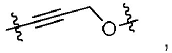

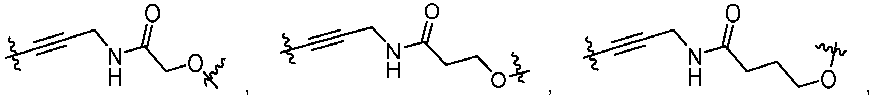

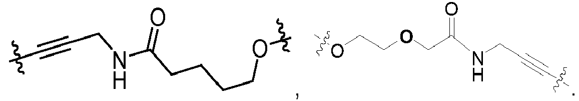

In some embodiments, L¹ is selected from

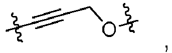

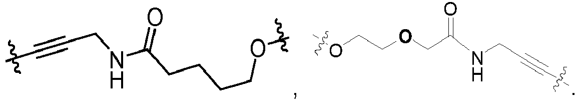

In some embodiments, r¹ is selected from 1, 2, 3.

In some embodiments, r¹ is 1.

In some embodiments, r² is selected from 1, 2, 3.

In some embodiments, r² is 1.

In some embodiments, r^{3a}, r^{3b}, independently of each other, are selected from 1, 2, 3, 4, 5.

In some embodiments, r^{3a}, r^{3b}, independently of each other, are selected from 1, 2, 3.

In some embodiments, r^{3a} is 1.

In some embodiments, r^{3b} is 2.

In some embodiments, M is selected from CH₂, O.

In some embodiments, X is selected from O, S.

In some embodiments, X is O.

In some embodiments, Y is a direct bond.

In some embodiments, W is a direct bond.

In some embodiments, in R¹, R², R³, R⁴, R⁵, any one (e.g., R², R³ or R⁴ is 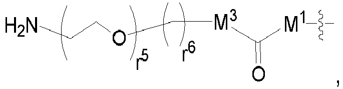 and the others, independently of each other, are selected from H,

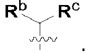

In some embodiments, in R¹, R², R³, R⁴, R⁵, any one (e.g., R², R³ or R⁴) is 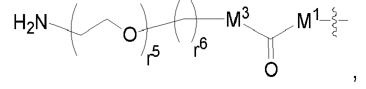 another (e.g., R¹ or R⁵) is 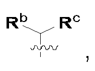 and the remaining three are H.

In some embodiments, in R¹, R², R³, R⁴, R⁵, R³ or R⁴ is 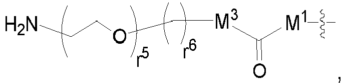 R¹ is 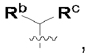 and the remaining three are H.

In some embodiments, R is selected from

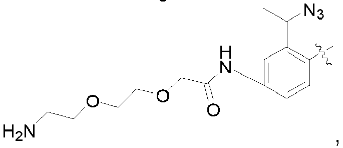

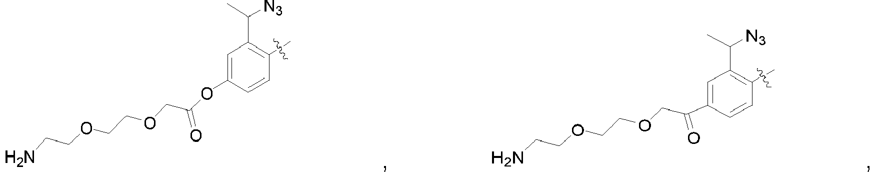

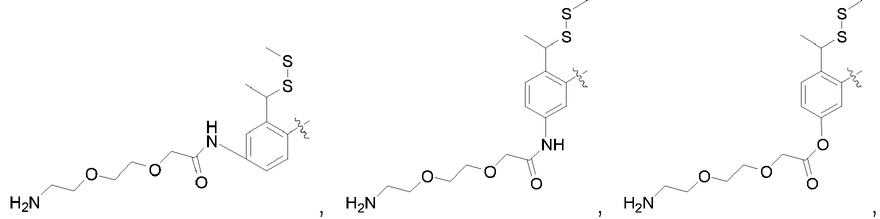

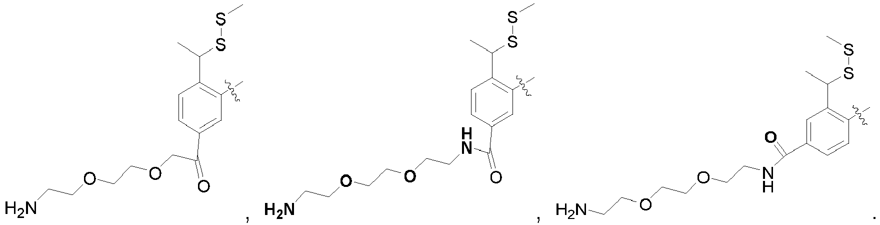

In some embodiments, r⁵ is selected from 1, 2, 3.

In some embodiments, r⁵ is 2.

In some embodiments, r⁶ is selected from 1, 2, 3.

In some embodiments, r⁶ is 1 or 2.

In some embodiments, M¹ is selected from a direct bond, NH, O.

In some embodiments, M³ is selected from a direct bond, NH.

In some embodiments, when M¹ is NH, O or S, M³ is a direct bond, and when M³ is NH, O or S, M¹ is a direct bond.

In some embodiments, in R^{b}, R^{c}, any one is selected from -N₃, -SS-C₁-C₆ alkyl (e.g., -SS-Me, -SS-Et, -SS-iPr, -SS-t-Bu), and the other is selected from C₁-C₆ alkyl.

In some embodiments, in R^{b}, R^{c}, any one is selected from -N₃, -SS-Me, and the other is methyl.

In some embodiments, Z is O.

In some embodiments, base¹ is selected from

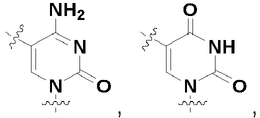

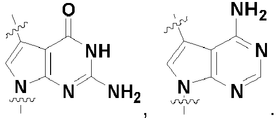

In the third aspect of the invention, the present invention provides a compound of formula I-2 or a salt thereof, 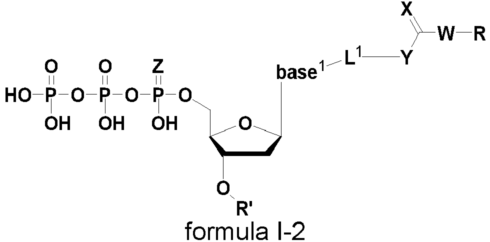 wherein:
L¹ is selected from 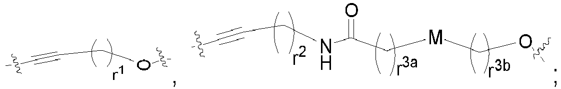
   r¹, r², r^{3a}, r^{3b}, independently of each other, are selected from any integer between 1 and 6;
   M is selected from a direct bond, CH₂, NH, O, S;
X is selected from O, S, NH;
Y is selected from a direct bond, O, S, NH;
W is selected from a direct bond, O, S, NH;
R is 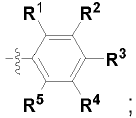 in R¹, R², R³, R⁴, R⁵, any one (e.g., R¹ or R⁵) is 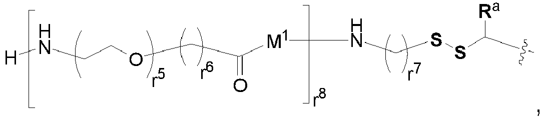 and the others, independently of each other, are selected from H, azido, nitro, amino, sulfo, carboxyl, aliphatic alkyl (e.g., C₁-C₆ alkyl), cycloalkyl (e.g., C₃-C₆ cycloalkyl), aromatic alkyl (e.g., phenyl -C₁-C₆ alkyl), F, I, Br, Cl, alkoxyl (e.g., C₁-C₆ alkoxyl);
   r⁵, r⁶, r⁷, independently of each other, are selected from any integer between 1 and 6;
   r⁸ is selected from 0, 1,
   M¹ is selected from a direct bond, NH, O, S;
   R^{a} is selected from H, aliphatic alkyl (e.g., C₁-C₆ alkyl, such as Me, Et, iPr, tBu), aromatic alkyl (e.g., phenyl -C₁-C₆ alkyl), cycloalkyl (e.g., C₃-C₆ cycloalkyl);
Z is selected from O, S, BH;
base¹ is selected from a base, a deaza base or a tautomer thereof, for example, adenine, 7-deazaadenine, thymine, uracil, cytosine, guanine, 7-deazaguanine or a tautomer thereof;
R' represents a reversible blocking group.

In some embodiments, L¹ is selected from

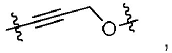

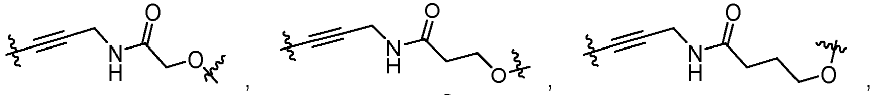

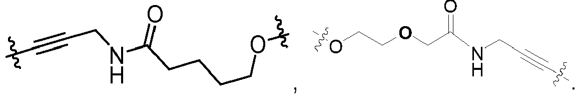

In some embodiments, L¹ is selected from

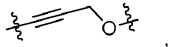

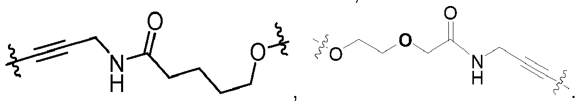

In some embodiments, r¹ is selected from 1, 2, 3.

In some embodiments, r¹ is 1.

In some embodiments, r² is selected from 1, 2, 3.

In some embodiments, r² is 1.

In some embodiments, r^{3a}, r^{3b}, independently of each other, are selected from 1, 2, 3, 4, 5.

In some embodiments, r^{3a}, r^{3b}, independently of each other, are selected from 1, 2, 3.

In some embodiments, r^{3a} is 1.

In some embodiments, r^{3b} is 2.

In some embodiments, M is selected from CH₂, O.

In some embodiments, X is selected from O, S.

In some embodiments, X is O.

In some embodiments, Y is a direct bond.

In some embodiments, W is a direct bond.

In some embodiments, in R¹, R², R³, R⁴, R⁵, any one (e.g., R¹ or R⁵) is 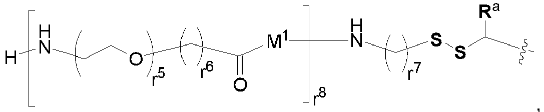 and the others are H.

In some embodiments, in R¹, R², R³, R⁴, R⁵, R¹ is 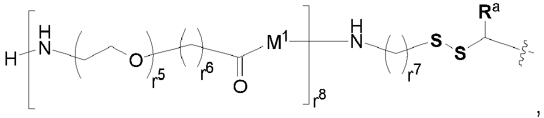 and the others are H.

In some embodiments, R is selected from

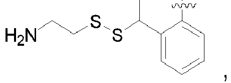

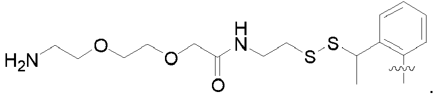

In some embodiments, r⁵ is selected from 1, 2, 3.

In some embodiments, r⁵ is 2.

In some embodiments, r⁶ is selected from 1, 2, 3.

In some embodiments, r⁶ is 1.

In some embodiments, r⁷ is selected from 1, 2, 3.

In some embodiments, r⁷ is 2.

In some embodiments, M¹ is a direct bond.

In some embodiments, R^{a} is selected from C₁-C₆ alkyl.

In some embodiments, R^{a} is methyl.

In some embodiments, Z is O.

In some embodiments, base¹ is selected from

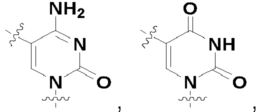

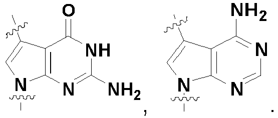

In the fourth aspect of the invention, the present invention provides a compound of formula II-1 or a salt thereof, 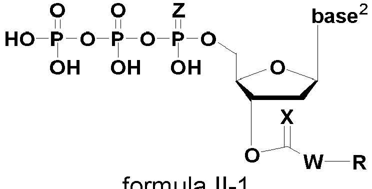 wherein:
X is selected from O, S, NH;
W is selected from a direct bond, O, S, NH;
R is 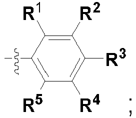 in R¹, R², R³ R⁴, R⁵, any one (e.q., R³ or R⁴ is 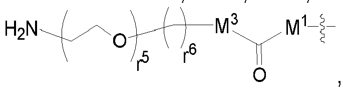 and the others, independently of each other, are selected from H, 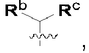 azido, nitro, amino, sulfo, carboxyl, aliphatic alkyl (e.g., C₁-C₆ alkyl), cycloalkyl (e.g., C₃-C₆ cycloalkyl), aromatic alkyl (e.g., phenyl -C₁-C₆ alkyl), F, I, Br, Cl, alkoxyl (e.g., C₁-C₆ alkoxyl);
   r⁵, r⁶, independently of each other, are selected from any integer between 1 and 6;
   M¹, M³, independently of each other, are selected from a direct bond, NH, O, S;
   in R^{b}, R^{c}, any one is selected from -N₃, -SS-C₁-C₆ alkyl (e.g., -SS-Me, -SS-Et, -SS-iPr, -SS-t-Bu), -ONH₂, -OCORₓ, -OCONHRₓ, and the other is selected from H, aliphatic alkyl (e.g., C₁-C₆ alkyl, such as Me, Et, iPr, tBu), aromatic alkyl (e.g., phenyl -C₁-C₆ alkyl), cycloalkyl (e.g., C₃-C₆ cycloalkyl), wherein each Rₓ, independently, is selected from aliphatic alkyl (e.g., C₁-C₆ alkyl), cycloalkyl (e.g., C₃-C₆ cycloalkyl) or aromatic alkyl (e.g., phenyl C₁-C₆ alkyl);
Z is selected from O, S, BH;
base² is selected from a base, a deaza base or a tautomer thereof, for example, adenine, 7-deazaadenine, thymine, uracil, cytosine, guanine, 7-deazaguanine or a tautomer thereof; 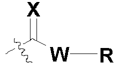 represents a reversible blocking group.

In some embodiments, X is selected from O, S.

In some embodiments, X is O.

In some embodiments, W is a direct bond.

In some embodiments, in R¹, R², R³, R⁴, R⁵, any one (e.g., R³ or R⁴ is 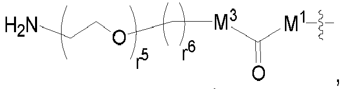 and the others, independently of each other, are selected from H,

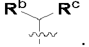

In some embodiments, in R¹, R², R³, R⁴, R⁵, any one (e.q., R³ or R⁴) is 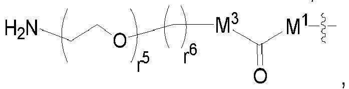 another (e.g., R¹ or R⁵) is 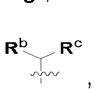 and the remaining three are H.

In some embodiments, in R¹, R², R³, R⁴, R⁵, R³ or R⁴ is 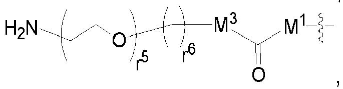 R¹ is 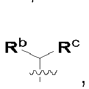 and the remaining three are H.

In some embodiments, R is selected from

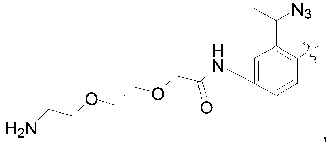

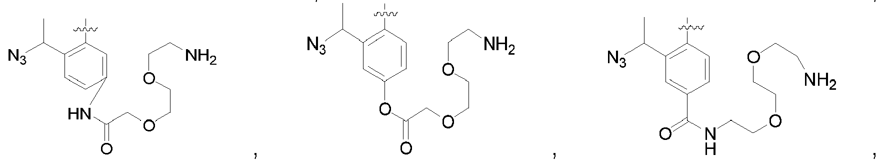

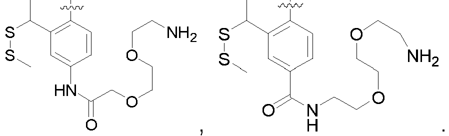

In some embodiments, r⁵ is selected from 1, 2, 3.

In some embodiments, r⁵ is 2.

In some embodiments, r⁶ is selected from 1, 2, 3.

In some embodiments, r⁶ is selected from 1, 2.

In some embodiments, M¹ is selected from a direct bond, NH, O.

In some embodiments, M³ is selected from a direct bond, NH.

In some embodiments, in R^{b}, R^{c}, any one is selected from -N₃, -SS-C₁-C₆ alkyl (e.g., -SS-Me, -SS-Et, -SS-iPr, -SS-t-Bu), and the other is selected from C₁-C₆ alkyl.

In some embodiments, in R^{b}, R^{c}, any one is -N₃ or -SS-Me, and the other is methyl.

In some embodiments Z is O.

In some embodiments, base² is selected from

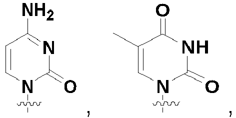

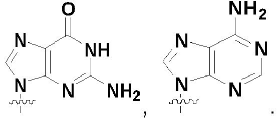

In the fifth aspect of the invention, the present invention provides a compound of formula II-2 or a salt thereof, 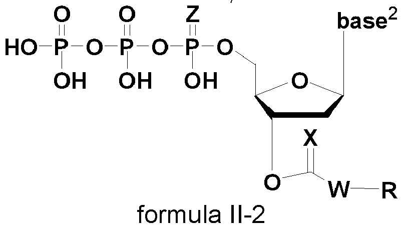 wherein:
X is selected from O, S, NH;
W is selected from a direct bond, O, S, NH;
R is 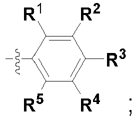 in R¹, R², R³, R⁴, R⁵, any one (e.g., R¹ or R⁵) is 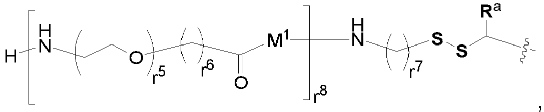 and the others, independently of each other, are selected from H, azido, nitro, amino, sulfo, carboxyl, aliphatic alkyl (e.g., C₁-C₆ alkyl), cycloalkyl (e.g., C₃-C₆ cycloalkyl), aromatic alkyl (e.g., phenyl -C₁-C₆ alkyl), F, I, Br, Cl, alkoxyl (e.g., C₁-C₆ alkoxyl);
   r⁵, r⁶, r⁷ are selected from any integer between 1 and 6;
   r⁸ is selected from 0, 1;
   M¹ is selected from a direct bond, NH, O, S;
   R^{a} is selected from H, aliphatic alkyl (e.g., C₁-C₆ alkyl, such as Me, Et, iPr, tBu), aromatic alkyl (e.g., phenyl -C₁-C₆ alkyl), cycloalkyl (e.g., C₃-C₆ cycloalkyl);
Z is selected from O, S, BH;
base² is selected from a base, a deaza base or a tautomer thereof, for example, adenine, 7-deazaadenine, thymine, uracil, cytosine, guanine, 7-deazaguanine or a tautomer thereof 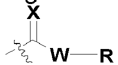 represents a reversible blocking group.

In some embodiments, X is selected from O, S.

In some embodiments, X is O.

In some embodiments, W is a direct bond.

In some embodiments, in R¹, R², R³, R⁴, R⁵, any one (e.g., R¹ or R⁵) is 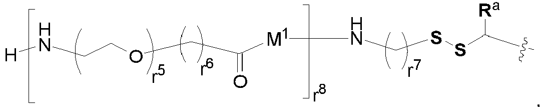 and the others are H.

In some embodiments, in R¹, R², R³, R⁴, R⁵, R¹ is 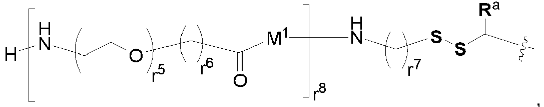 and the others are H.

In some embodiments, R is selected from

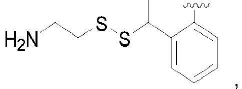

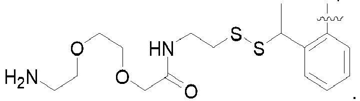

In some embodiments, r⁵ is selected from 1, 2, 3.

In some embodiments, r⁵ is 2.

In some embodiments, r⁶ is selected from 1, 2, 3.

In some embodiments, r⁶ is 1.

In some embodiments, r⁷ is selected from 1, 2, 3.

In some embodiments, r⁷ is 2.

In some embodiments, M¹ is a direct bond.

In some embodiments, R^{a} is selected from C₁-C₆ alkyl.

In some embodiments, R^{a} is methyl.

In some embodiments, Z is O.

In some embodiments, base² is selected from

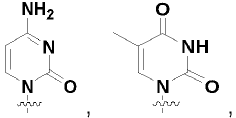

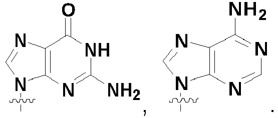

In some embodiments, the aforementioned reversible blocking group R' is selected from N₃-C₁-C₆ alkyl, C₁-C₆ alkyl-SS-C₁-C₆ alkyl, 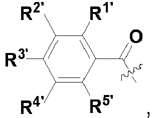 NH₂, - ONH₂, -OCOR_{z}, -OCONHR_{z}, wherein each R_{z}, independently, is selected from aliphatic alkyl (e.g., C₁-C₆ alkyl), cycloalkyl (e.g., C₃-C₆ cycloalkyl) or aromatic alkyl (e.g., phenyl C₁-C₆ alkyl).

In some embodiments, the reversible blocking group R' is selected from N₃-C₁-C₆ alkyl, C₁-C₆ alkyl-SS-C₁-C₆ alkyl, 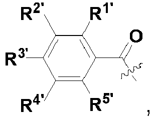 NH₂.

In some embodiments, the reversible blocking group R' is selected from N₃-C₁-C₆ alkyl, C₁-C₆ alkyl-SS-C₁-C₆ alkyl,

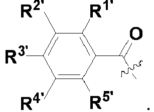

In some embodiments, the reversible blocking group R' is selected from N₃-CH₂-, CH₃-CH₂-S-S-CH₂-,

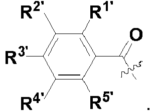

In some embodiments in R^{1'}, R^{2'}, R^{3'}, R^{4'}, R^{5'}, any one (e.g., R^{1'} or R^{5'}) is 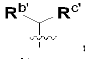 and the others, independently of each other, are selected from H, azido, nitro, amino, sulfo, carboxyl, aliphatic alkyl (e.g., C₁-C₆ alkyl), cycloalkyl (e.g., C₃-C₆ cycloalkyl), aromatic alkyl (e.g., phenyl -C₁-C₆ alkyl), F, I, Br, Cl, alkoxyl (e.g., C₁-C₆ alkoxyl).

In some embodiments, in R^{1'}, R^{2'}, R^{3'}, R^{4'}, R^{5'}, any one (e.g., R^{1'} or R^{5'} is 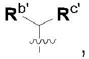 and the others are H.

In some embodiments in R^{1'}, R^{2'}, R^{3'}, R^{4'}, R^{5'}, R^{1'} is 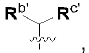 and the others are H.

In some embodiments, in R^{b'}, R^{c'}, any one is selected from -N₃, -SS-C₁-C₆ alkyl (e.g., -SS-Me, -SS-Et, -SS-iPr, -SS-t-Bu), -ONH₂, -OCORₓ, -OCONHRₓ, and the other is selected from H, aliphatic alkyl (e.g., C₁-C₆ alkyl, such as Me, Et, iPr, tBu), aromatic alkyl (e.g., phenyl -C₁-C₆ alkyl), cycloalkyl (e.g., C₃-C₆ cycloalkyl), wherein each Rₓ, independently, is selected from aliphatic alkyl (e.g., C₁-C₆ alkyl), cycloalkyl (e.g., C₃-C₆ cycloalkyl) or aromatic alkyl (e.g., phenyl C₁-C₆ alkyl).

In some embodiments, in R^{b'}, R^{c'}, any one is selected from -N₃, -SS-C₁-C₆ alkyl (e.g., -SS-Me, -SS-Et, -SS-iPr, -SS-t-Bu), and the other is selected from C₁-C₆ alkyl.

In some embodiments, in R^{b'}, R^{c'}, any one is selected from -N₃, -SS-Me, and the other is methyl.

In the sixth aspect of the invention, the present invention further provides a compound of formula III or a salt thereof, 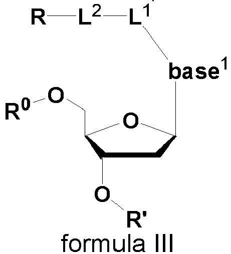 wherein:
L¹ is selected from 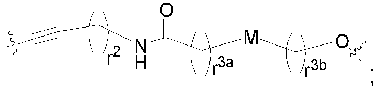
   r² is selected from 1, 2, 3;
   r^{3a} is selected from 1, 2, 3;
   r^{3b} is selected from 0, 1, 2, 3;
   M is selected from a direct bond O;
L² is 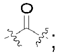
R is 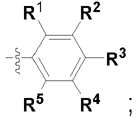
   in R¹, R², R³, R⁴, R⁵, any one (e.g., R³) is 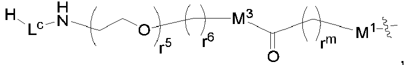 another (e.q., R' or R² is selected from 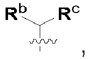 and the remaining three are H;
   L^{c} is selected from a direct bond, 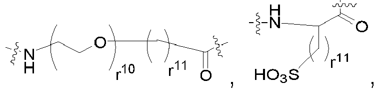 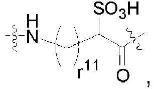 preferably, the NH end in L^{c} is linked to H, the =O end in L^{c} is linked to NH;
   r^{m} is selected from 0, 1, 2, 3;
   r⁵ is selected from 1, 2, 3;
   r⁶ is selected from 1, 2, 3;
   r¹⁰ is selected from any integer between 1 and 10;
   r¹¹ is selected from any integer between 1 and 6;
   M¹ is selected from NH, O;
   M¹ is selected from NH, O;
   M³ is selected from a direct bond, NH;
   in R^{b}, R^{c}, any one is selected from -SS-C₁-C₆ alkyl (e.g., -SS-Me, -SS-Et, -SS-iPr, -SS-t-Bu), and the other is selected from C₁-C₆ alkyl;
R° is selected from H, monophosphate group 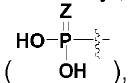 diphosphate group 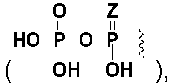 triphosphate group 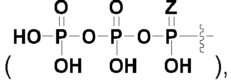 tetraphosphate group 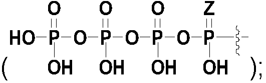 each Z, independently, is selected from O, S, BH;
base¹ is selected from a base, a deaza base or a tautomer thereof, for example, adenine, 7-deazaadenine, thymine, uracil, cytosine, guanine, 7-deazaguanine or a tautomer thereof;
in formula III, R' represents a reversible blocking group.

In some embodiments, L¹ is selected from

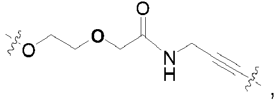

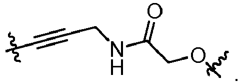

In some embodiments, r² is 1.

In some embodiments, r^{3a} is 1.

In some embodiments, r^{3b} is selected from 0, 2.

In some embodiments, L^{c} is selected from a direct bond 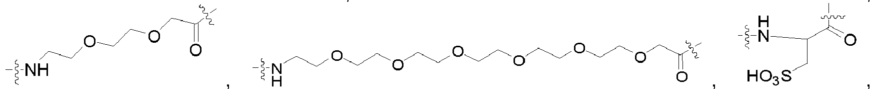 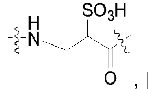 preferably, the NH end in L^{c} is linked to H, the =O end in L^{c} is linked to NH.

In some embodiments, r^{m} is selected from 0, 1.

In some embodiments, r⁵ is 2.

In some embodiments, r⁶ is 1 or 2.

In some embodiments, r¹⁰ is selected from any integer between 2 and 6.

In some embodiments, r¹⁰ is 2 or 6.

In some embodiments, r¹¹ is selected from 1, 2, 3.

In some embodiments, r¹¹ is 1.

In some embodiments, in R^{b}, R^{c}, any one is -SS-Me, and the other is methyl.

In some embodiments, R is selected from

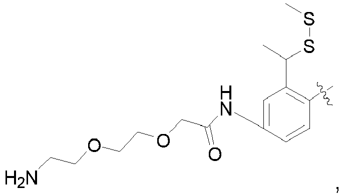

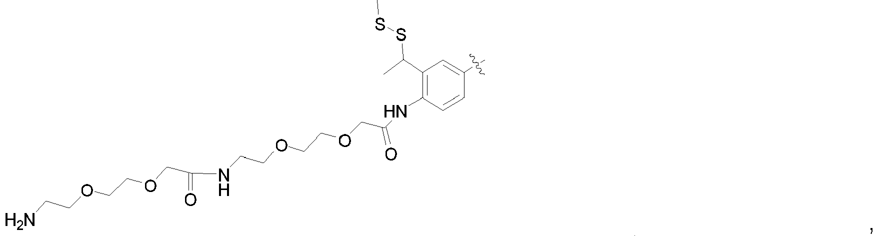

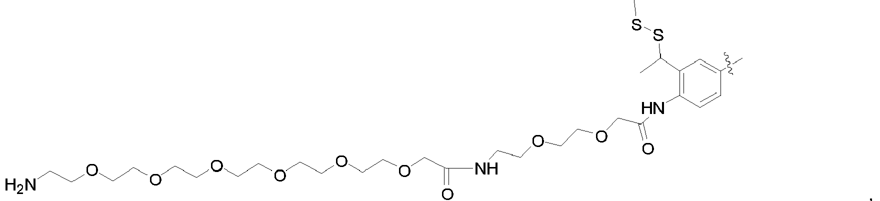

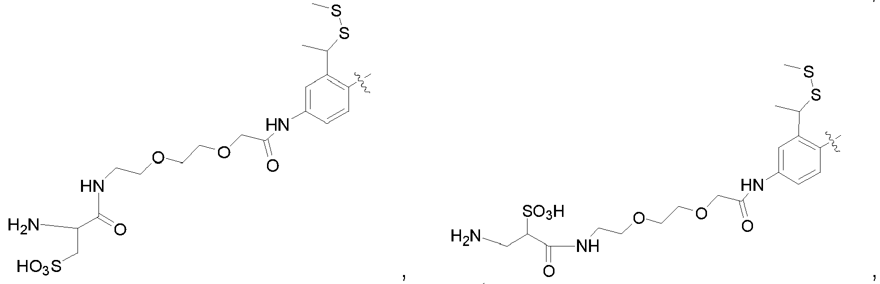

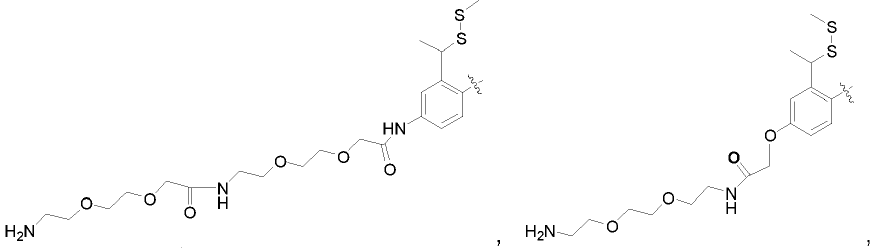

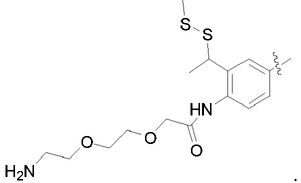

In some embodiments, R° is a triphosphate group

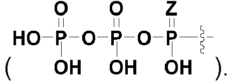

In some embodiments, Z is O.

In some embodiments, base¹ is selected from

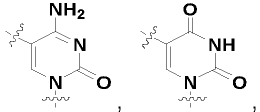

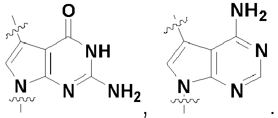

In some embodiments, the reversible blocking group R' is 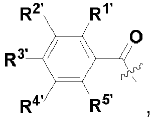 in R^{1'}, R^{2'}, R^{3'}, R^{4'}, R^{5'}, any one (e.g., R^{1'} or R^{5'}) is 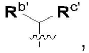 another (e.g., R^{3'}) is selected from H, C₁-C₆ alkoxyl (e.g., methoxy), and the others are H; in R^{b'}, R^{c'}, any one is selected from -N₃, -SS-C₁-C₆ alkyl (e.g., -SS-Me, -SS-Et, -SS-iPr, - SS-t-Bu), and the other is selected from C₁-C₆ alkyl.

In some embodiments in R^{1'}, R^{2'}, R^{3'}, R^{4'}, R^{5'}, R^{1'} is 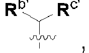 R^{3'} is selected from H, C₁-C₆ alkoxyl (e.g., methoxy), and the others are H.

In some embodiments, in R^{b'}, R^{c'}, any one is -N₃, -SS-Me, and the other is methyl.

In some embodiments, the compound is selected from the following Table A:

**Table A:**

| Serial Number | Structural formulae |
|---|---|
| 1A | 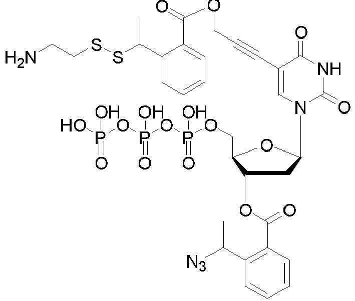 |
| 1B | 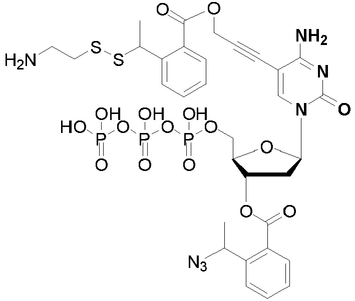 |
| 1C | 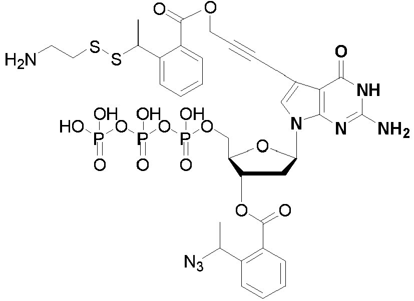 |
| 1D | 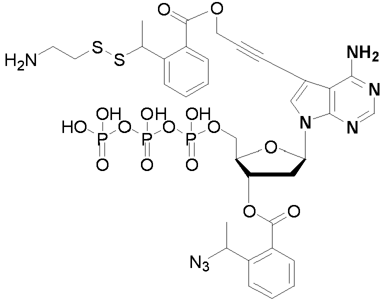 |
| 2A | 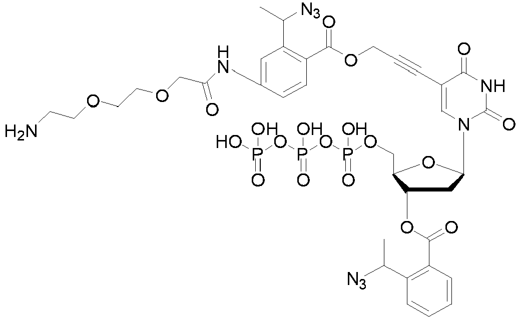 |
| 2B | 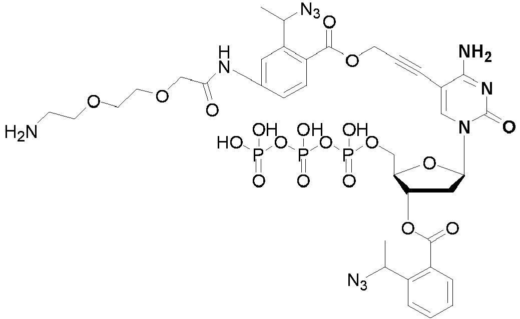 |
| 2C | 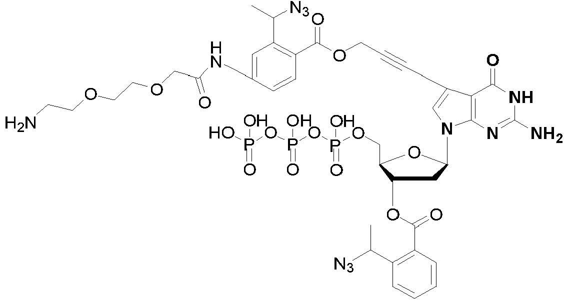 |
| 2D | 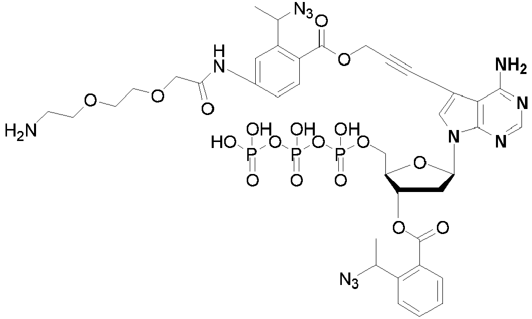 |
| 3A | 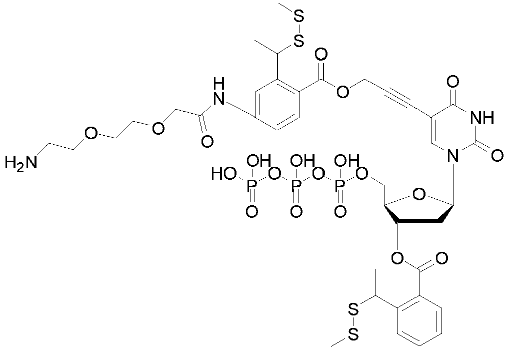 |
| 3B | 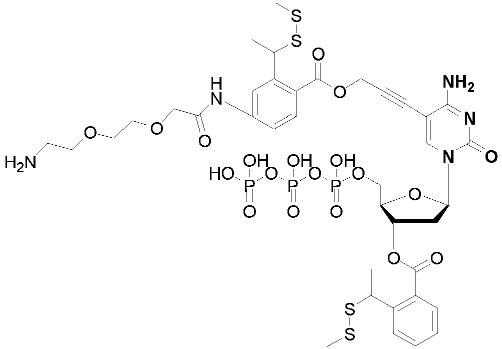 |
| 3C | 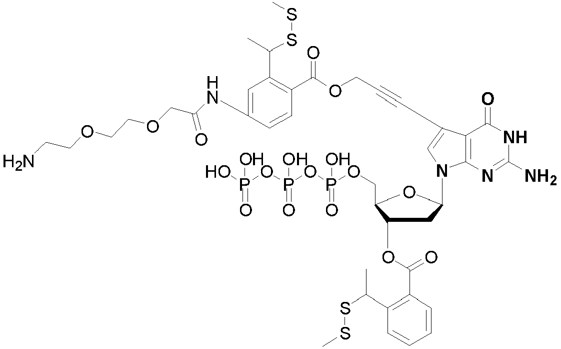 |
| 3D | 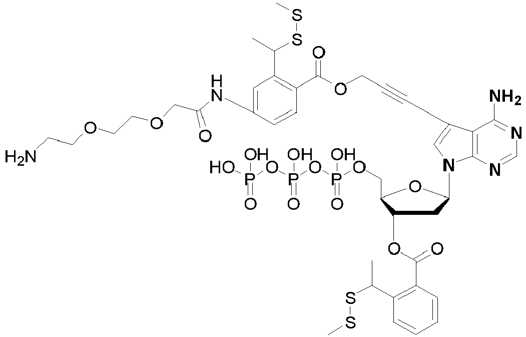 |
| 4A | 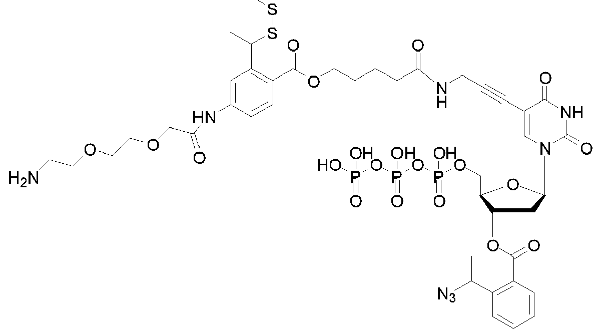 |
| 4B | 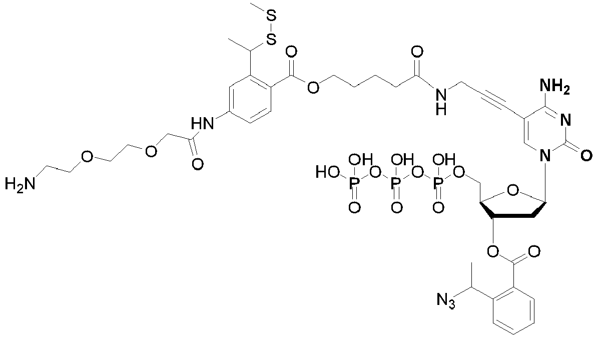 |
| 4C | 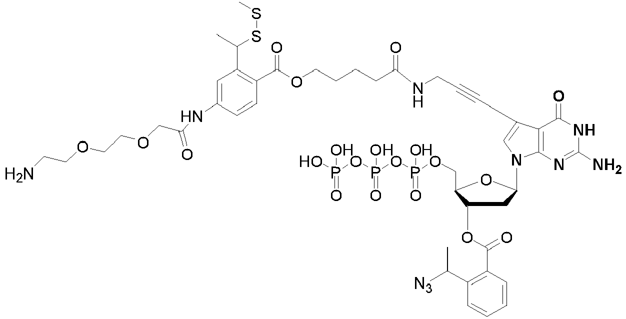 |
| 4D | 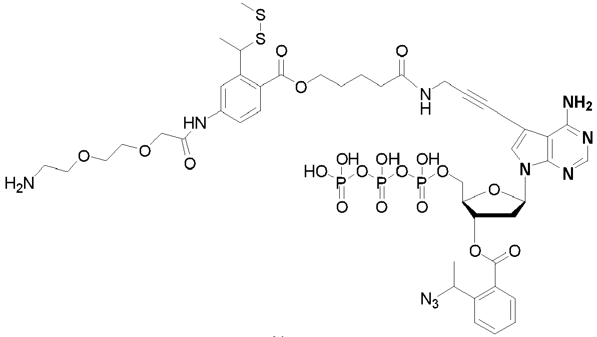 |
| 5A | 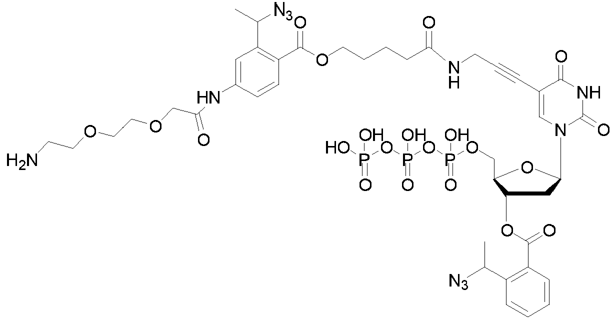 |
| 5B | 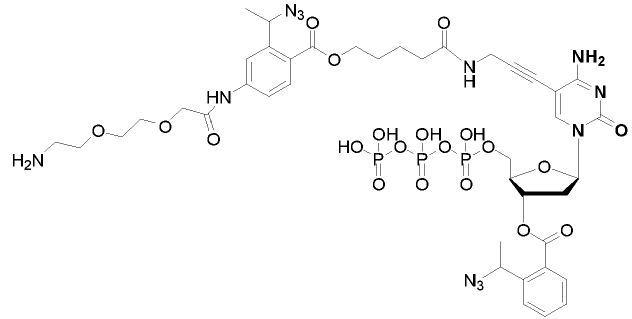 |
| 5C | 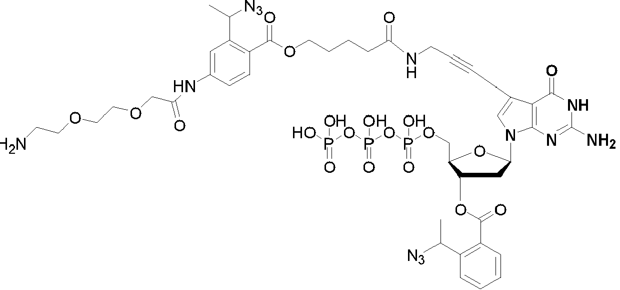 |
| 5D | 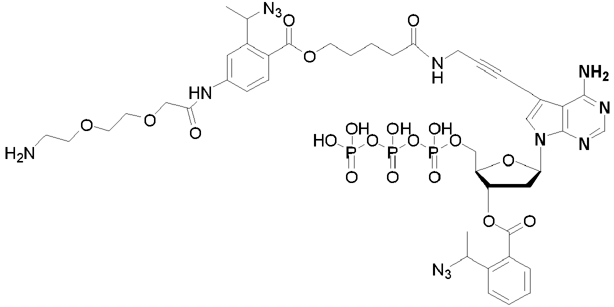 |
| 6A | 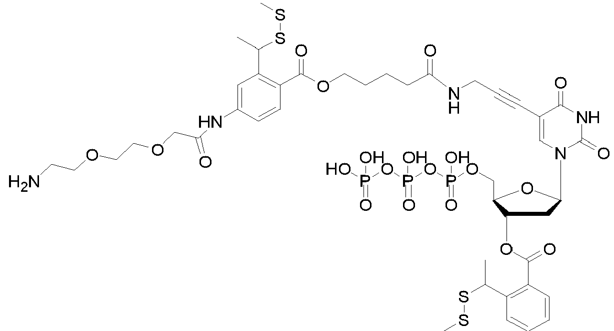 |
| 6B | 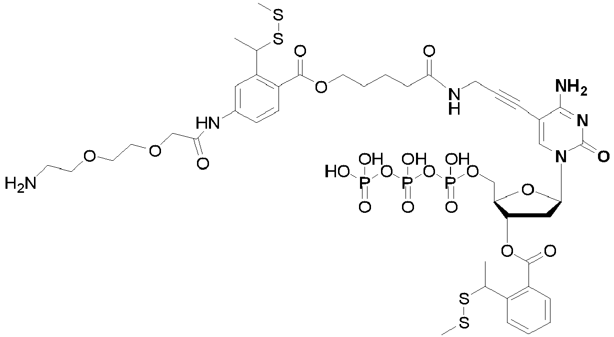 |
| 6C | 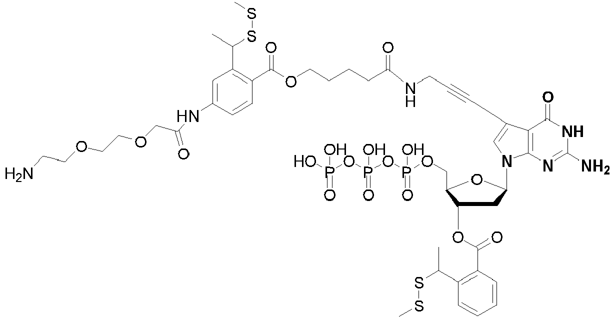 |
| 6D | 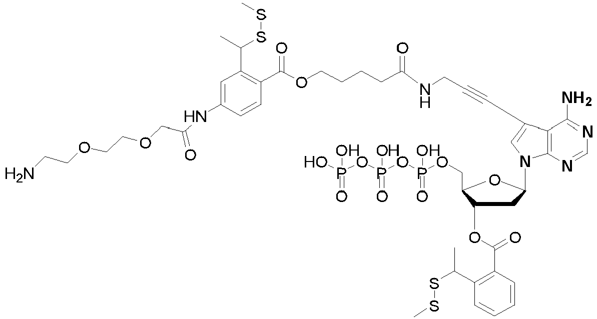 |
| 7A | 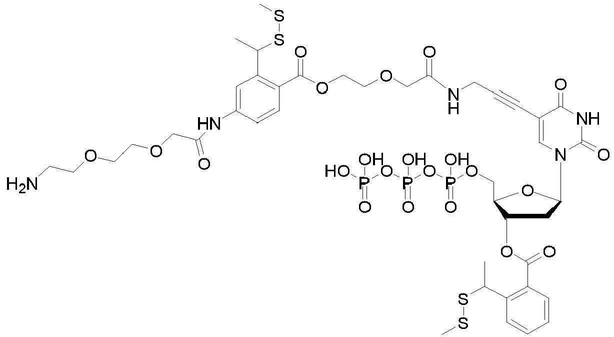 |
| 7B | 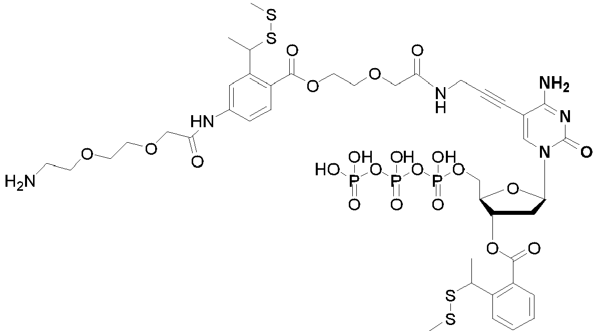 |
| 7C | 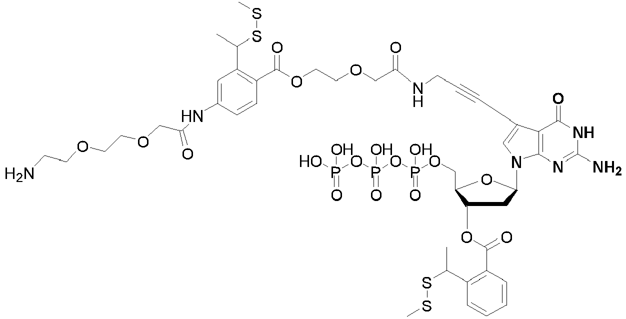 |
| 7D | 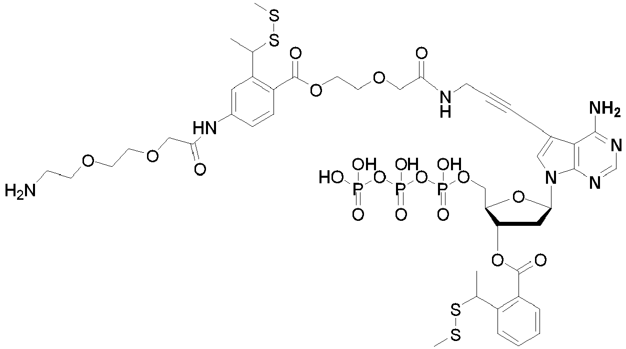 |
| 8-1A | 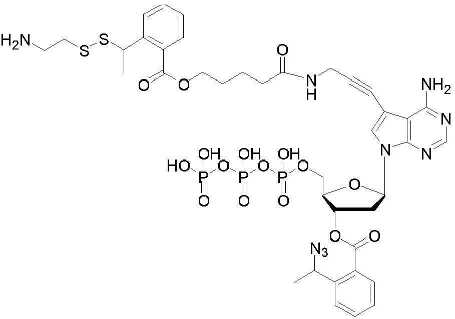 |
| 8-1B | 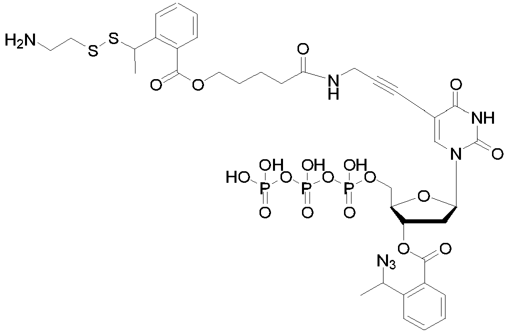 |
| 8-1C | 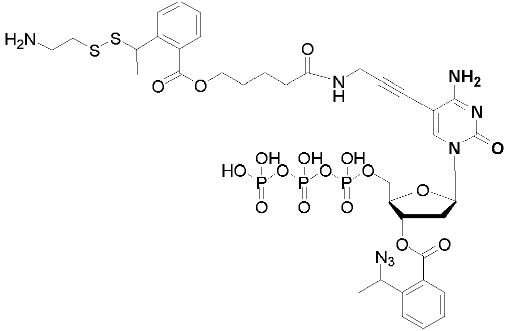 |
| 8-1D | 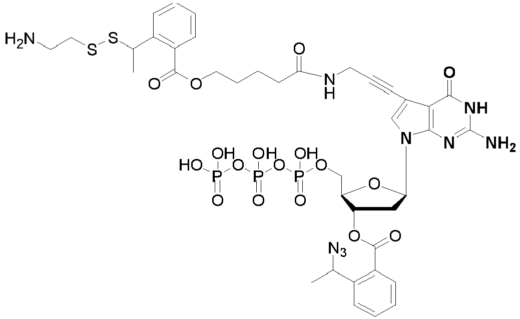 |
| 8-2A | 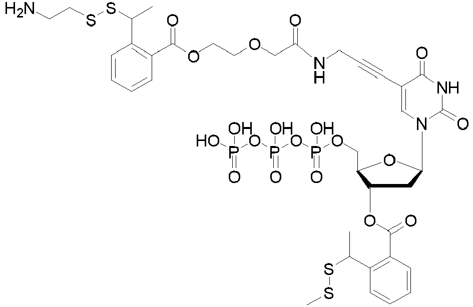 |
| 8-2 B | 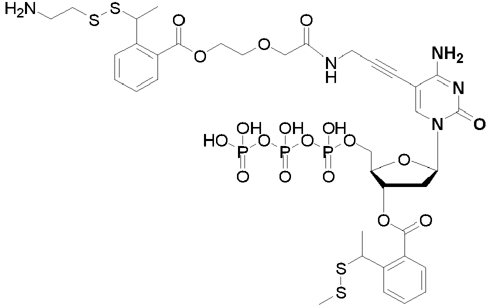 |
| 8-2C | 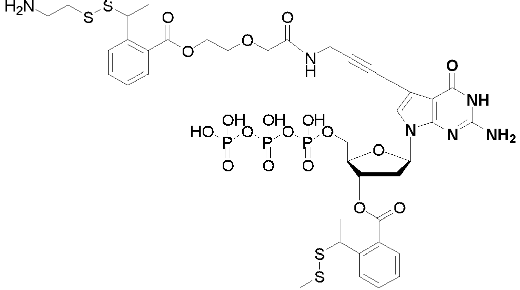 |
| 8-2D | 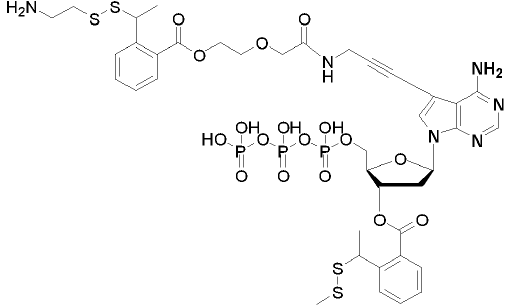 |
| 11A | 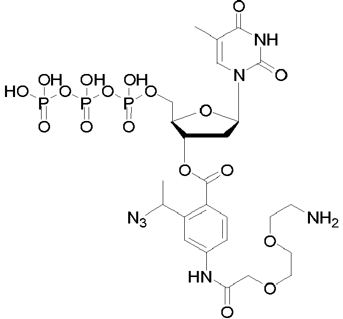 |
| 11B | 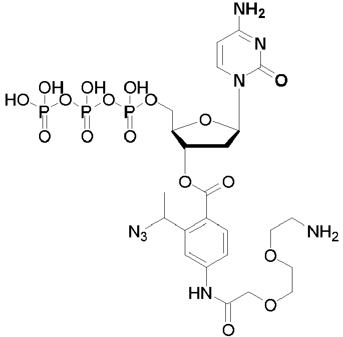 |
| 11C | 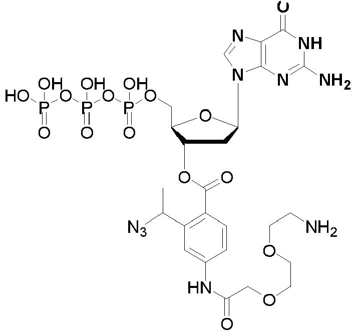 |
| 11D | 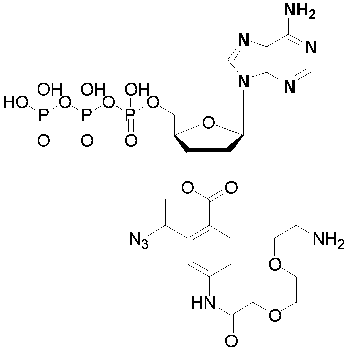 |
| 12A | 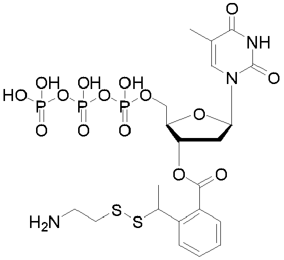 |
| 12B | 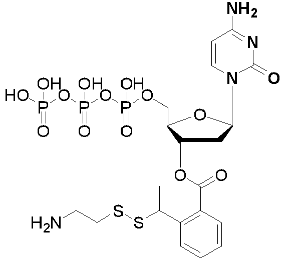 |
| 12C | 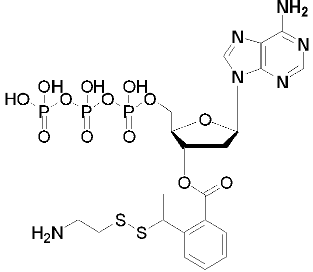 |
| 12D | 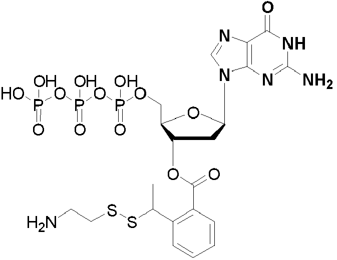 |
| 13A | 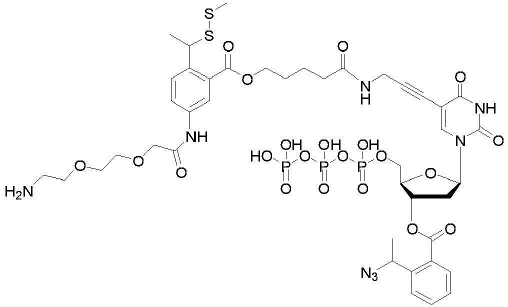 |
| 13B | 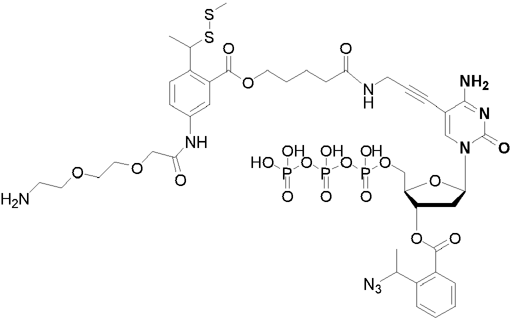 |
| 13C | 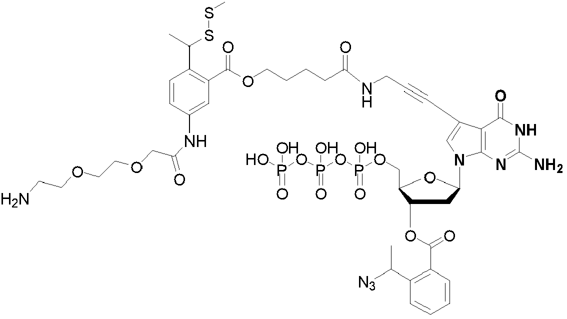 |
| 13D | 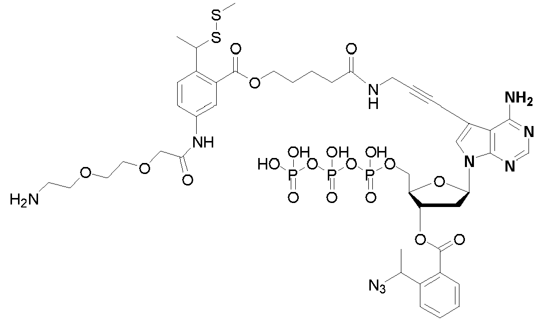 |
| 14A | 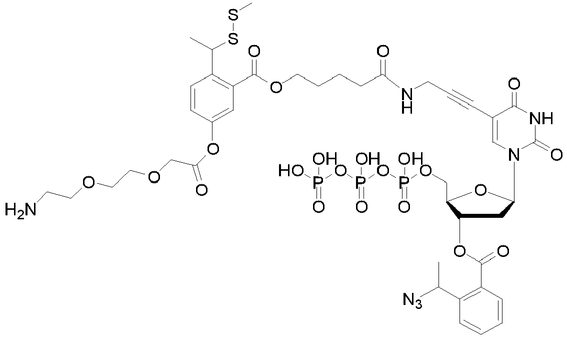 |
| 14B | 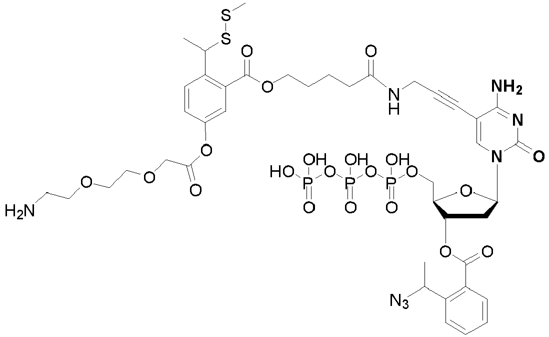 |
| 14C | 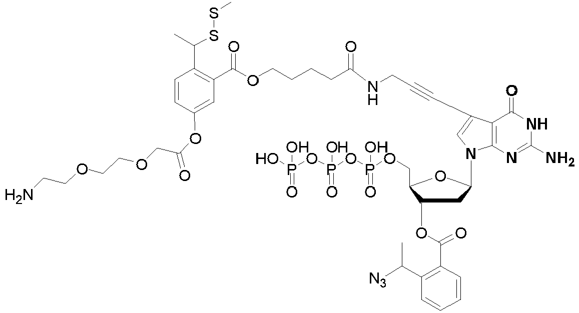 |
| 14D | 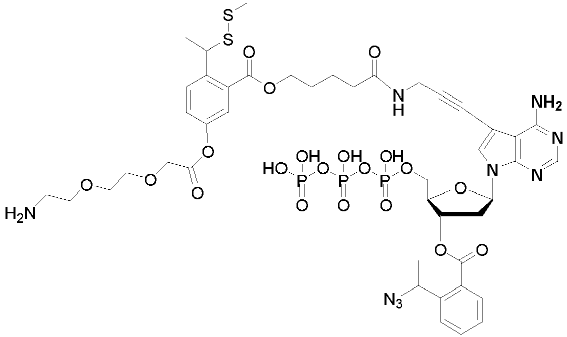 |
| 15A | 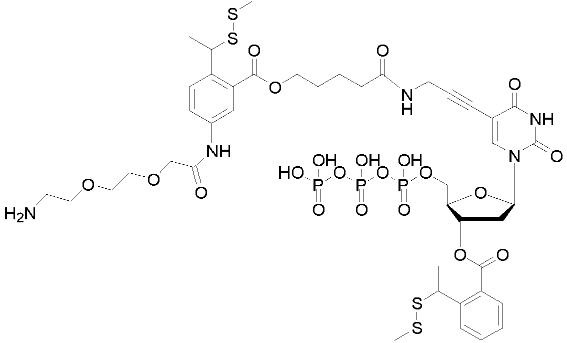 |
| 15B | 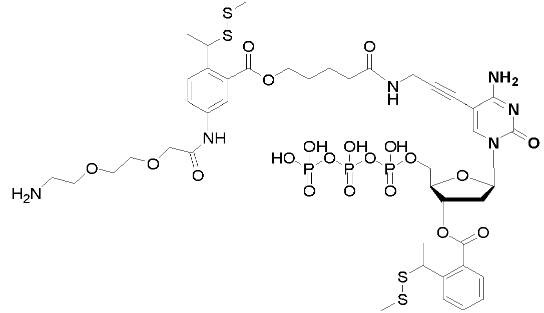 |
| 15C | 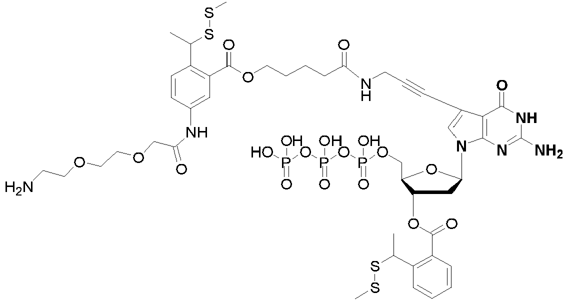 |
| 15D | 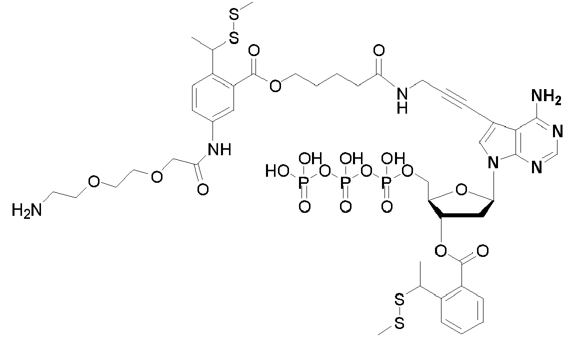 |
| 16A | 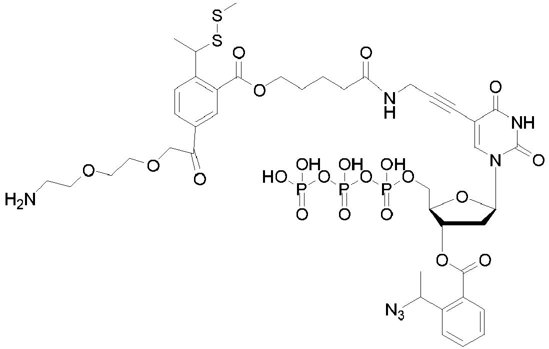 |
| 16B | 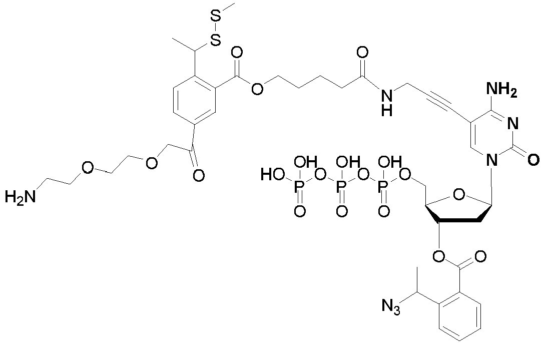 |
| 16C | 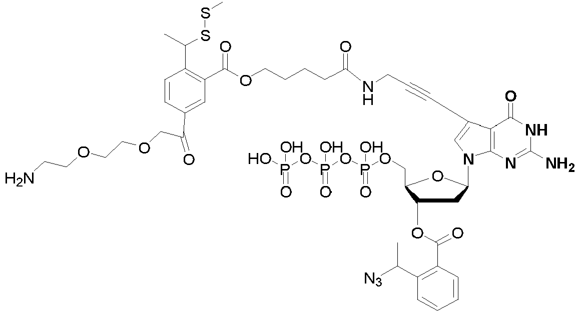 |
| 16D | 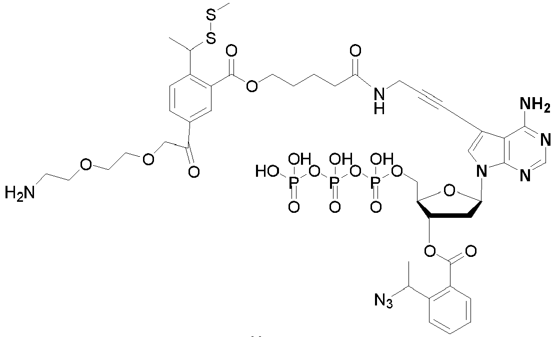 |
| 17A | 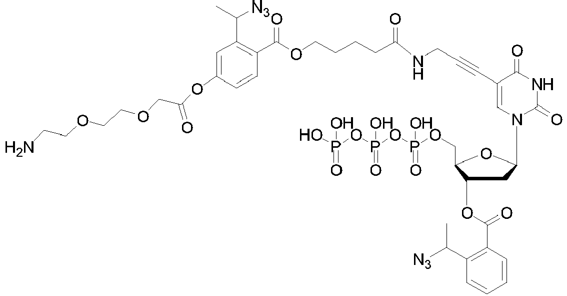 |
| 17B | 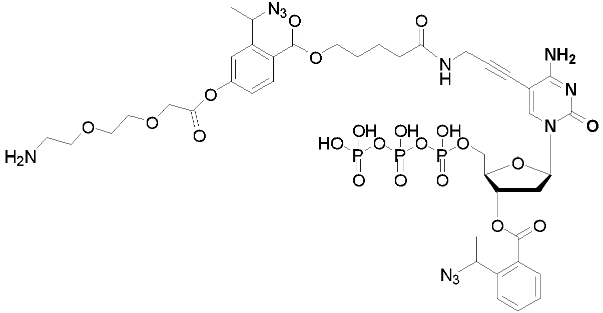 |
| 17C | 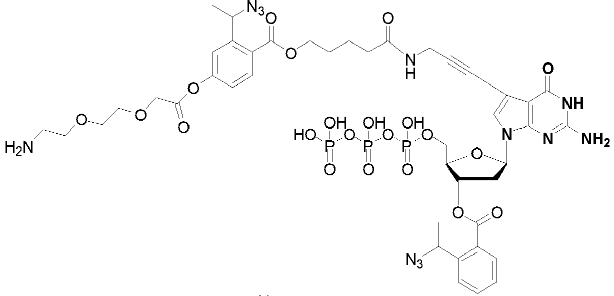 |
| 17D | 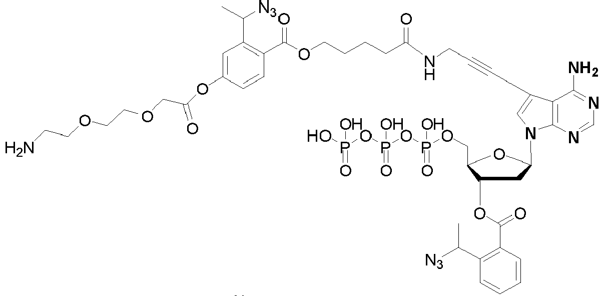 |
| 18A | 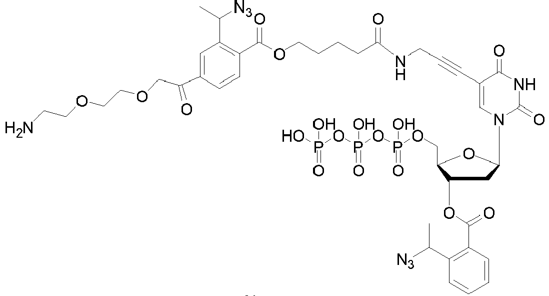 |
| 18B | 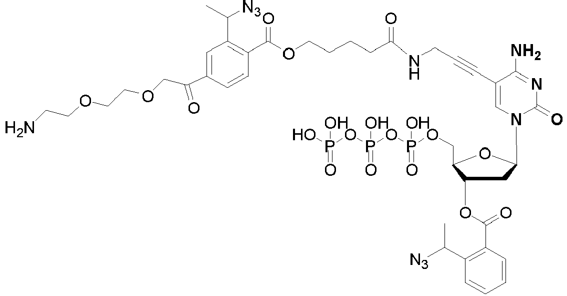 |
| 18C | 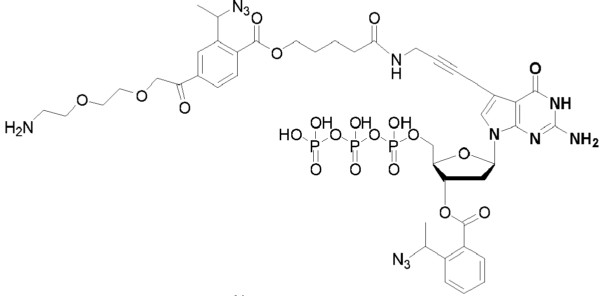 |
| 18D | 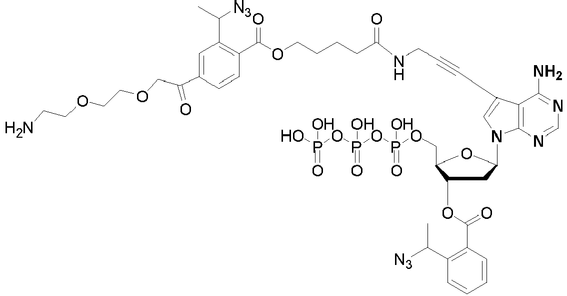 |
| 19A | 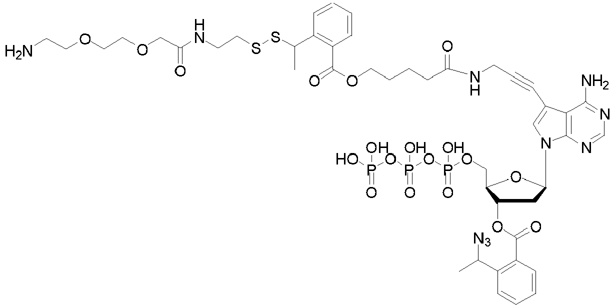 |
| 19B | 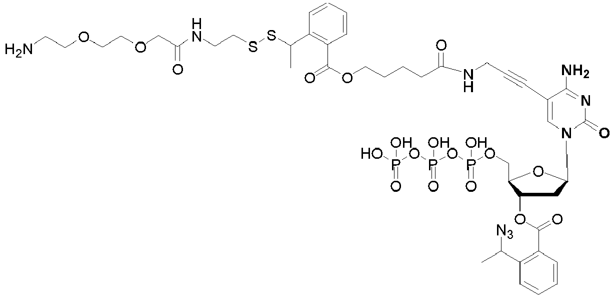 |
| 19C | 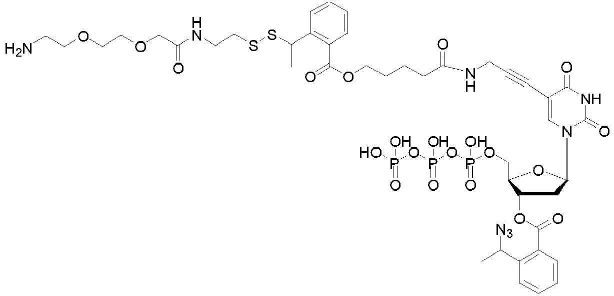 |
| 19D | 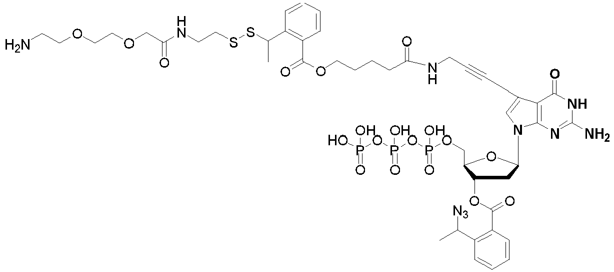 |
| 20A | 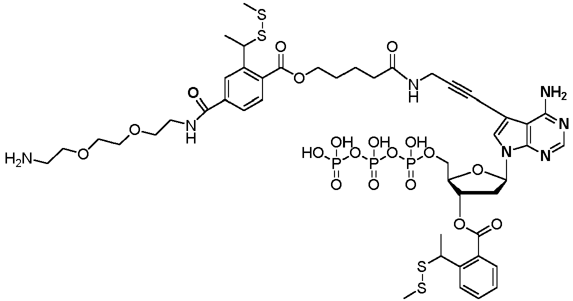 |
| 20B | 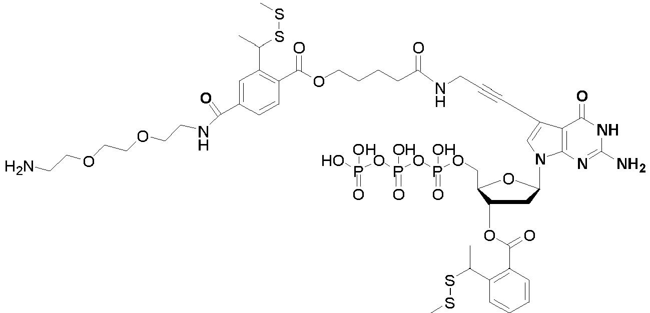 |
| 20C | 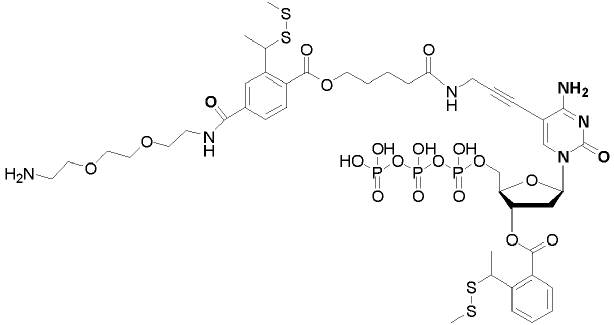 |
| 20D | 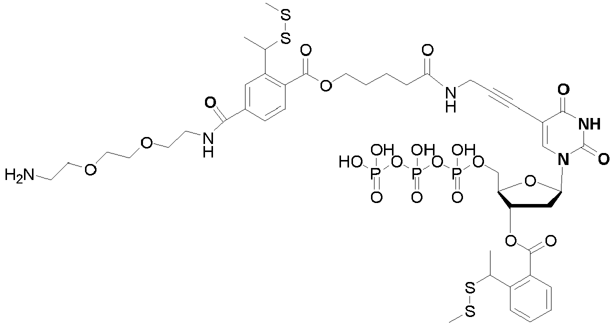 |
| 21A | 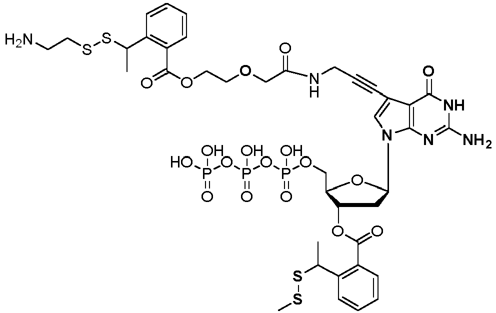 |
| 21B | 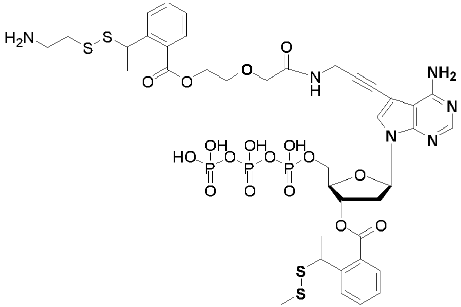 |
| 21C | 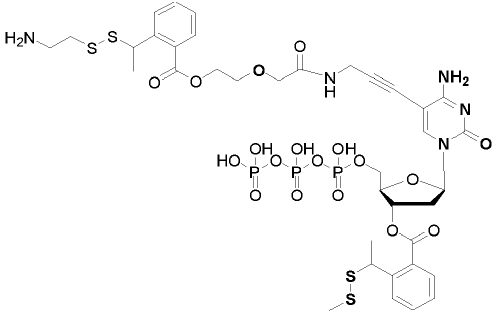 |
| 21D | 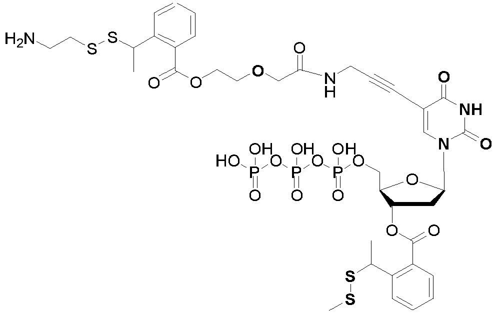 |
| 22A | 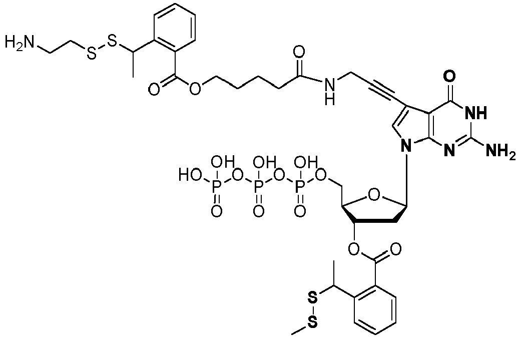 |
| 22B | 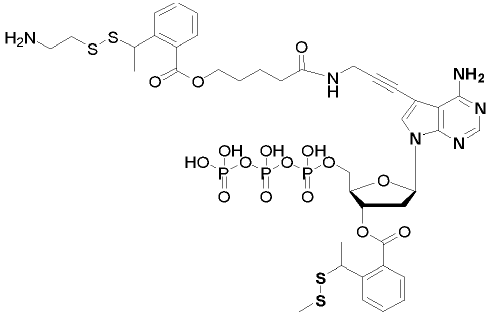 |
| 22C | 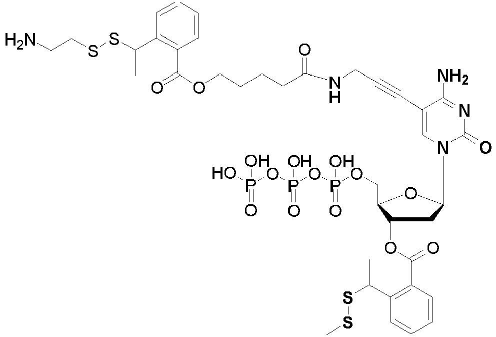 |
| 22D | 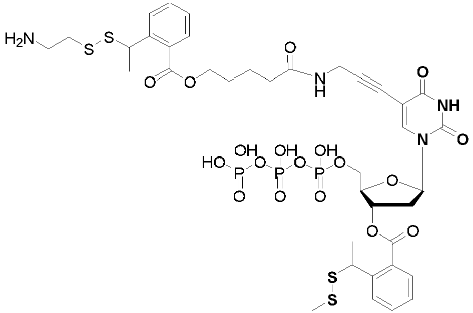 |
| 23A | 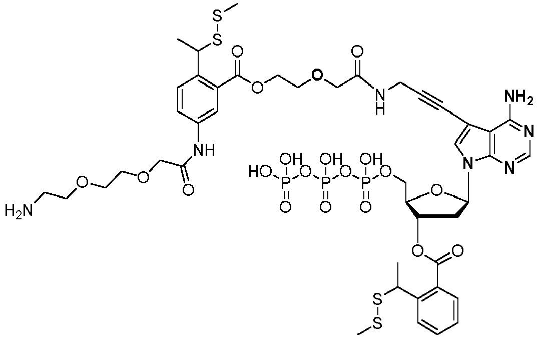 |
| 23B | 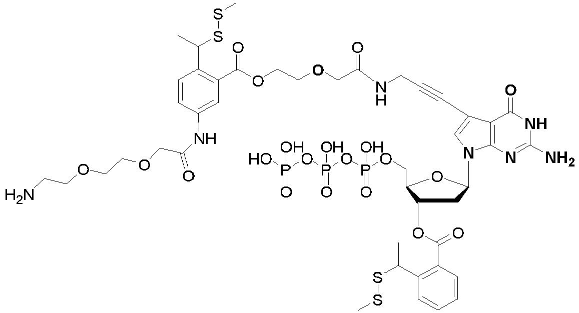 |
| 23C | 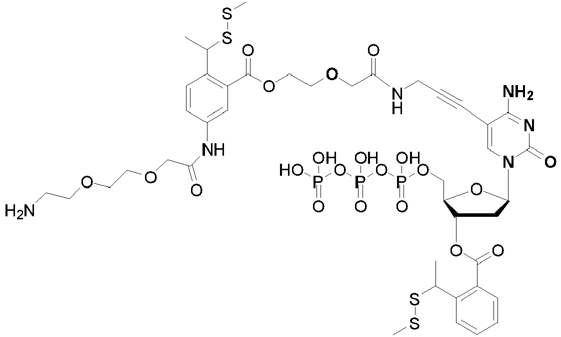 |
| 23D | 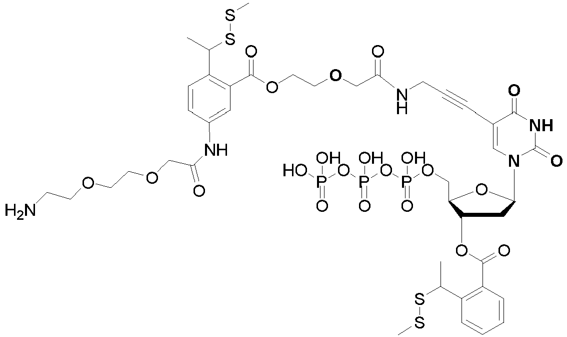 |
| 24A | 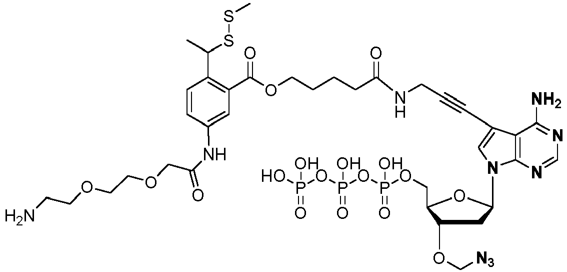 |
| 24B | 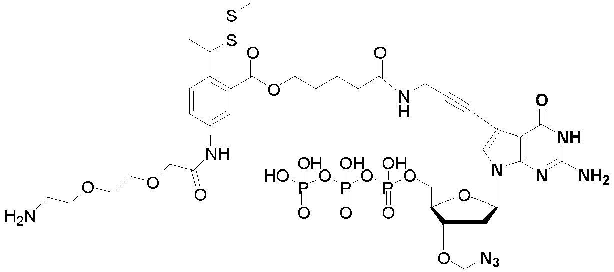 |
| 24C | 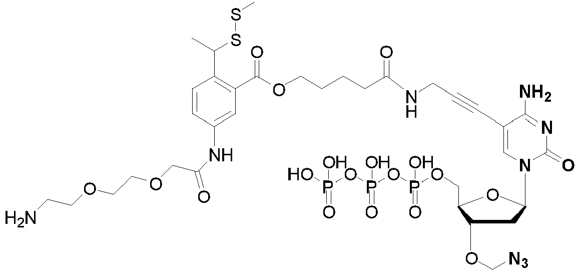 |
| 24D | 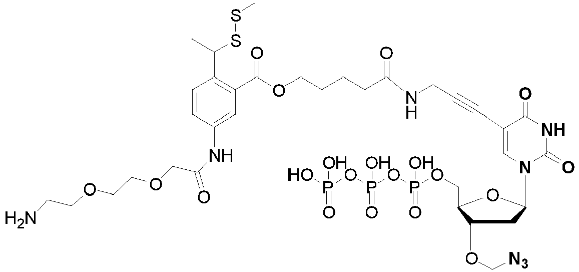 |
| 25A | 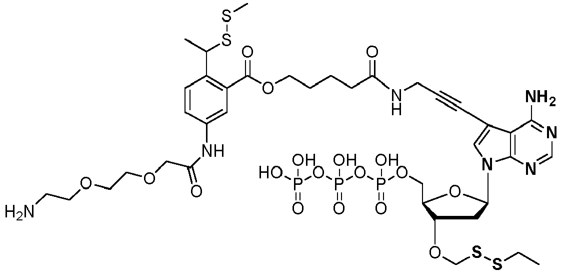 |
| 25B | 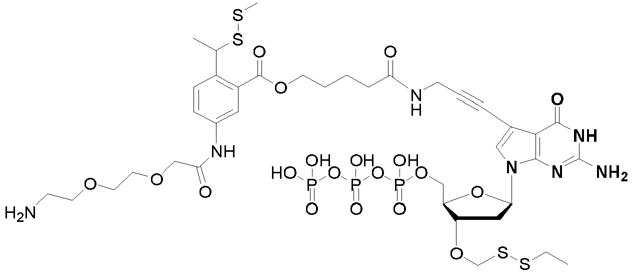 |
| 25C | 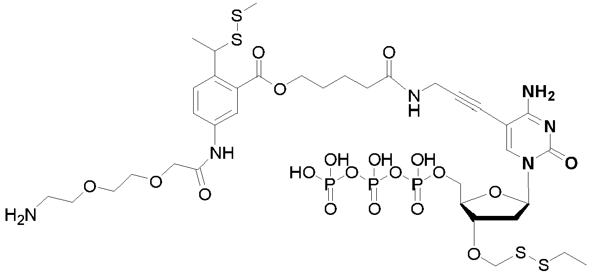 |
| 25D | 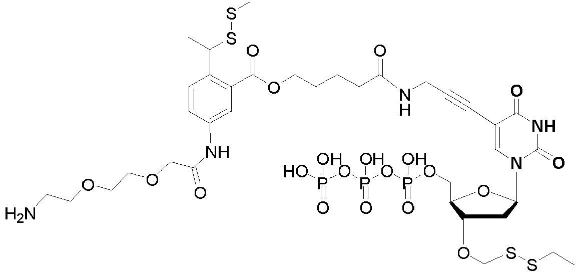 |
| 26A | 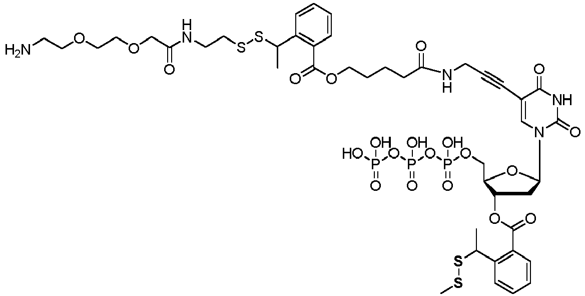 |
| 26B | 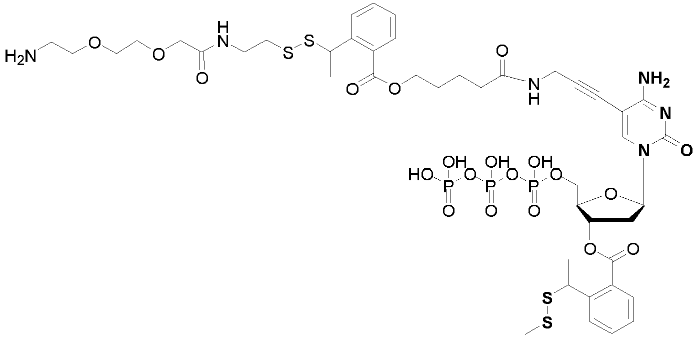 |
| 26C | 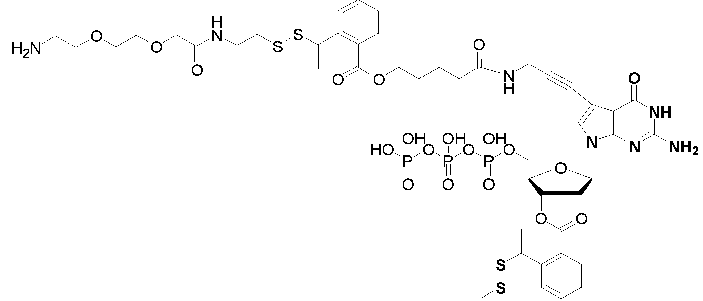 |
| 26D | 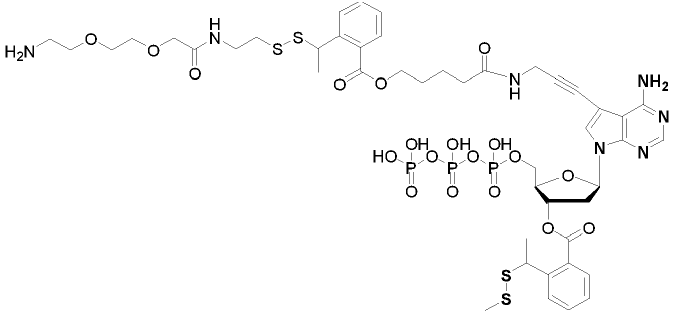 |
| 27A | 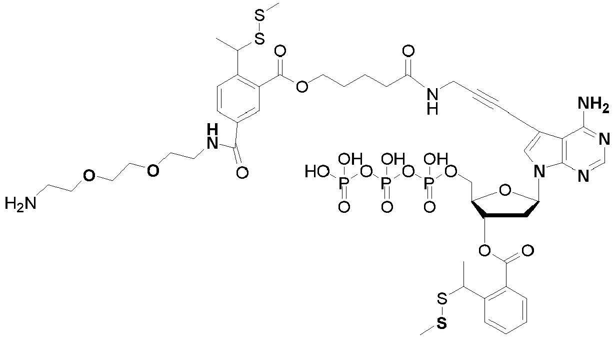 |
| 27B | 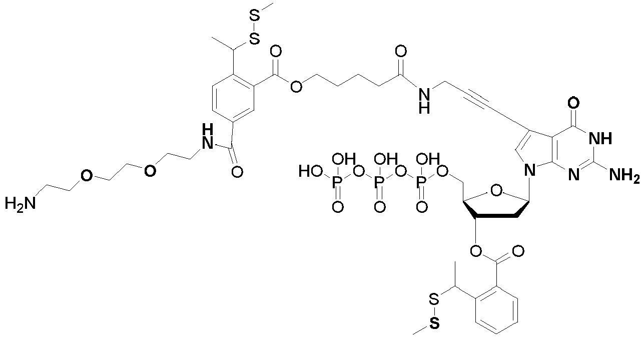 |
| 27C | 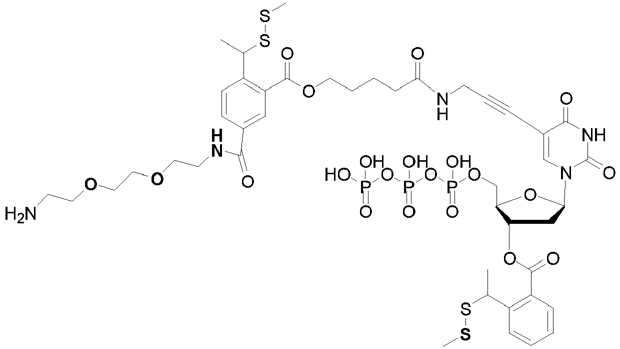 |
| 27D | 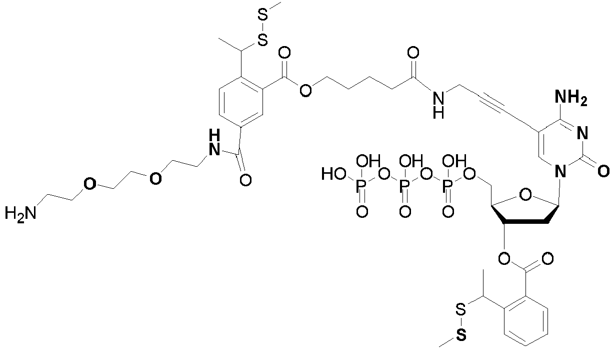 |
| 28A | 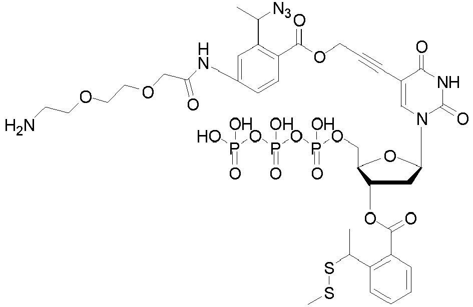 |
| 28B | 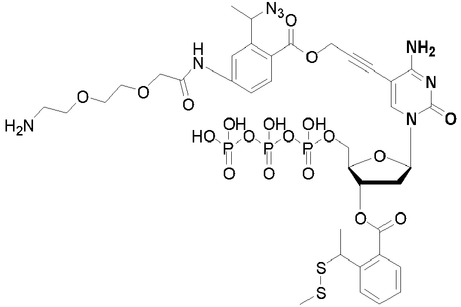 |
| 28C | 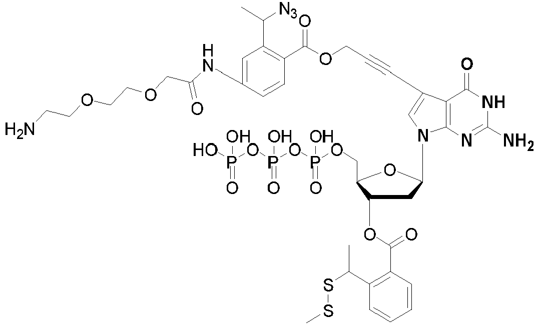 |
| 28D | 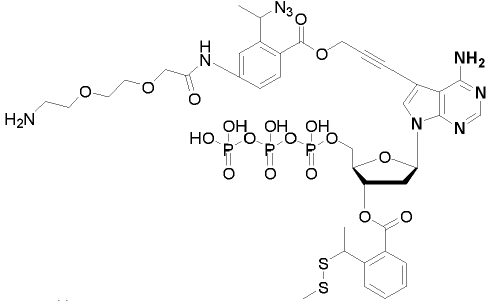 |
| 29A | 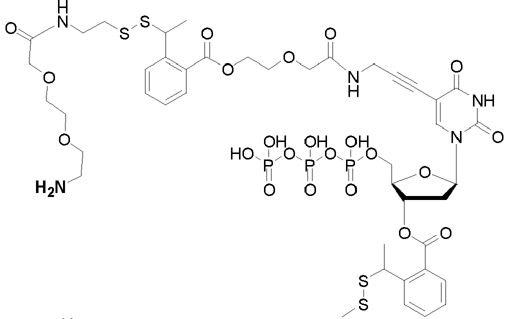 |
| 29B | 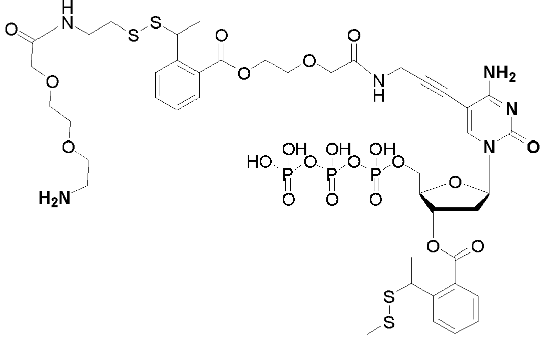 |
| 29C | 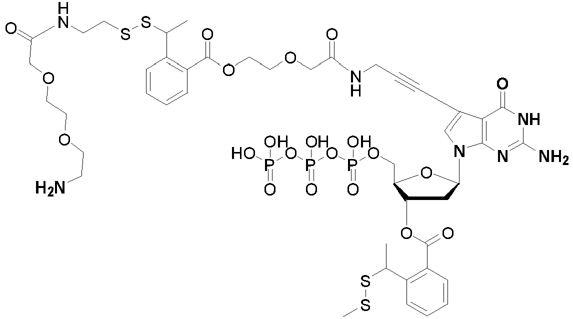 |
| 29D | 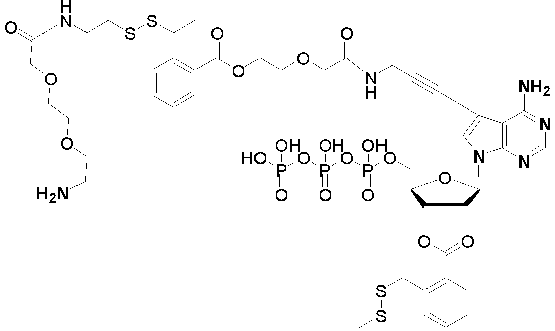 |
| 30A | 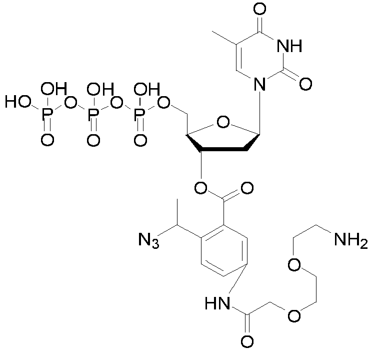 |
| 30B | 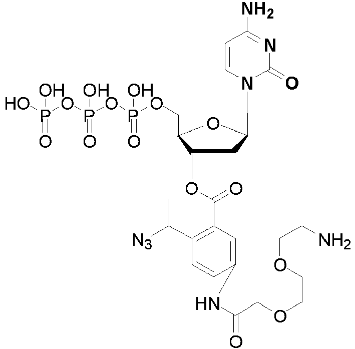 |
| 30C | 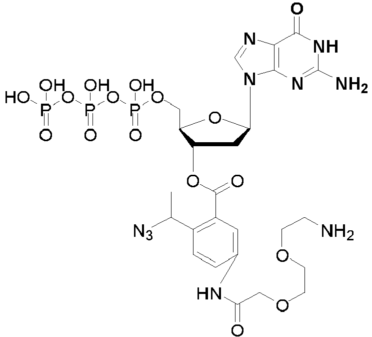 |
| 30D | 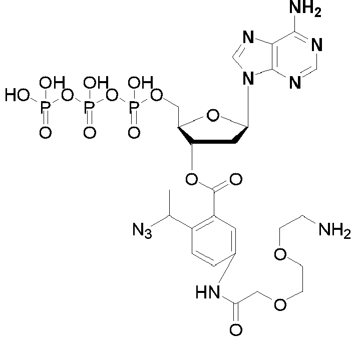 |
| 31A | 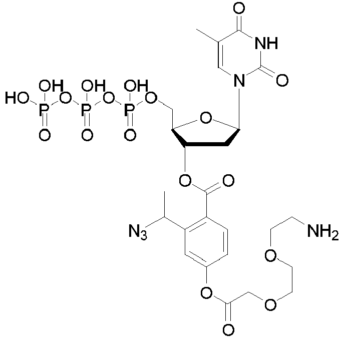 |
| 31B | 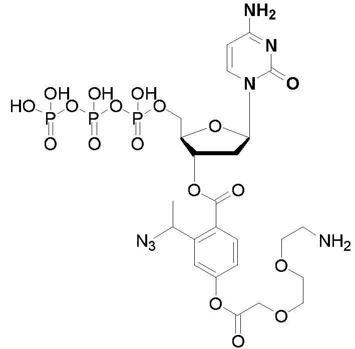 |
| 31C | 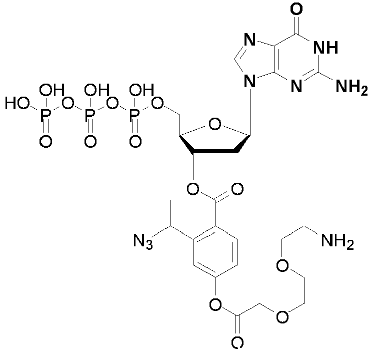 |
| 31D | 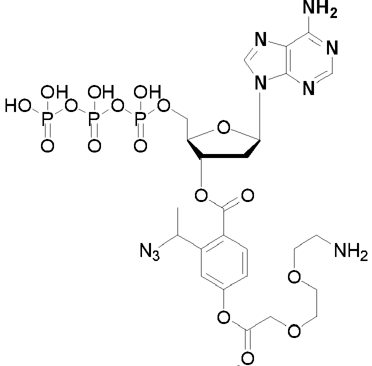 |
| 32A | 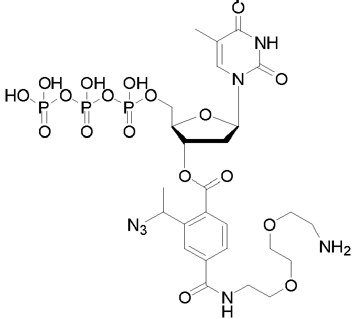 |
| 32B | 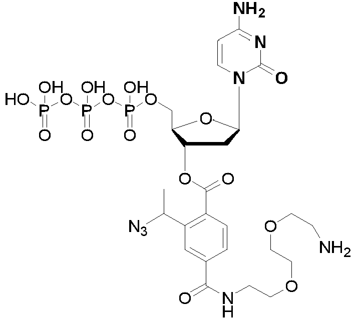 |
| 32C | 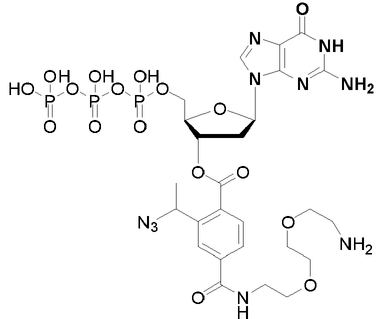 |
| 32D | 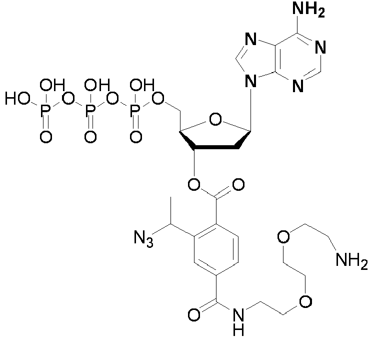 |
| 33A | 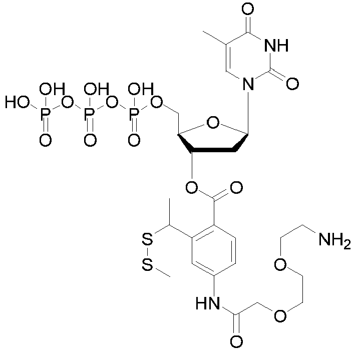 |
| 33B | 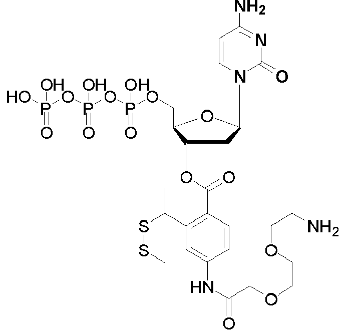 |
| 33C | 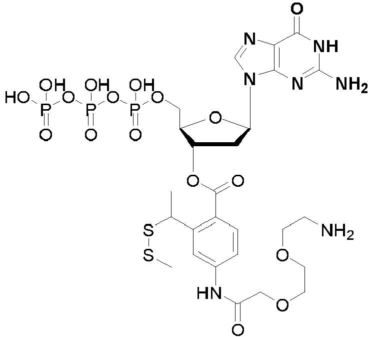 |
| 33D | 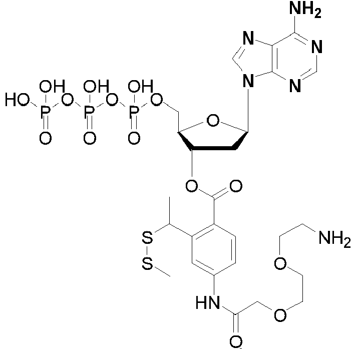 |
| 34A | 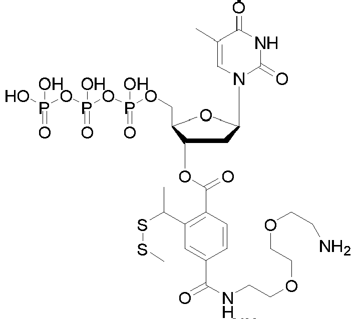 |
| 34B | 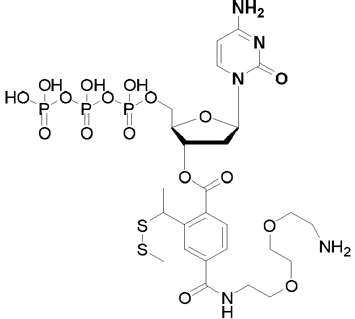 |
| 34C | 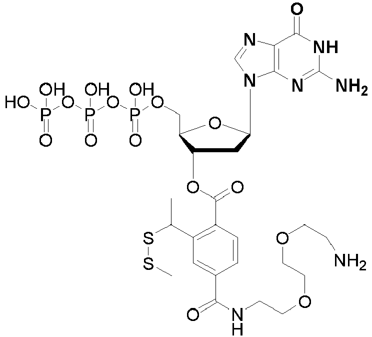 |
| 34D | 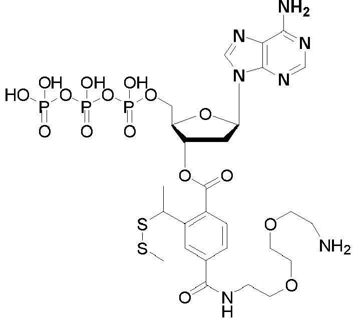 |
| 35A | 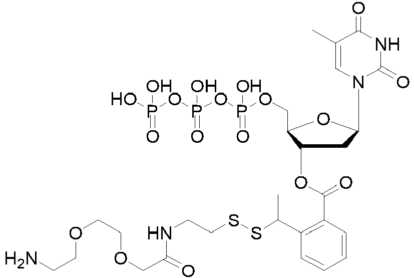 |
| 35B | 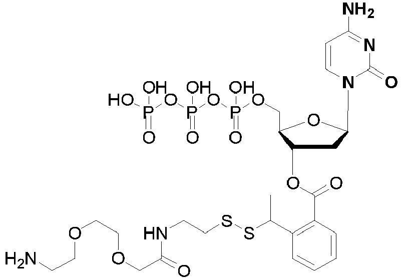 |
| 35C | 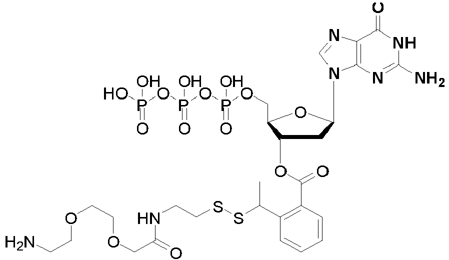 |
| 35D | 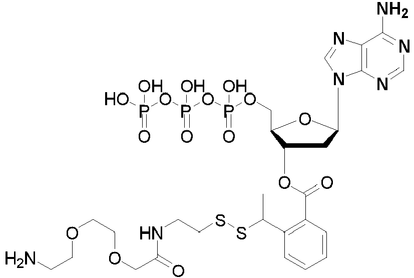 |
| 36A | 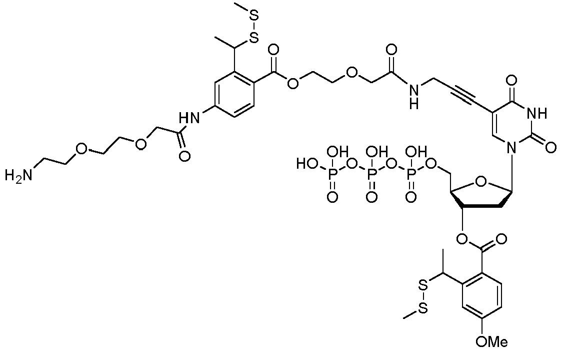 |
| 36B | 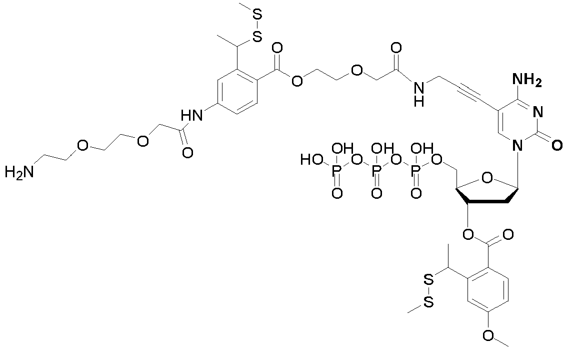 |
| 36C | 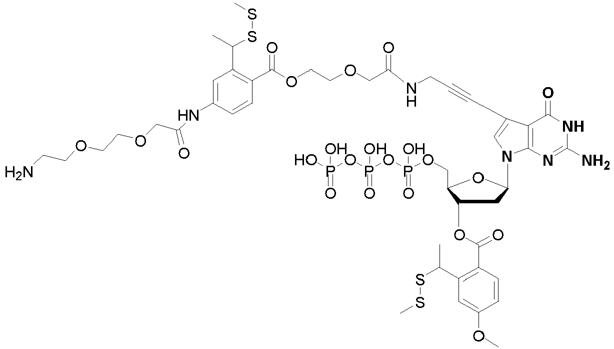 |
| 36D | 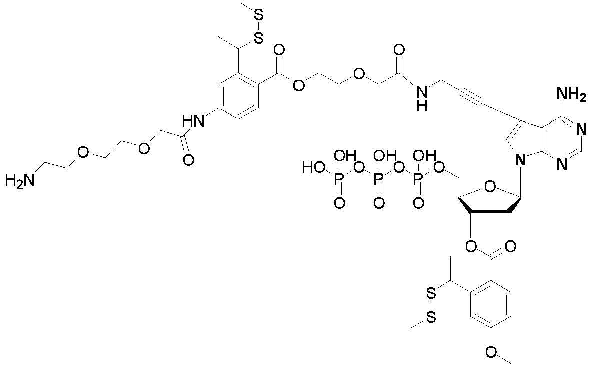 |
| 37A | 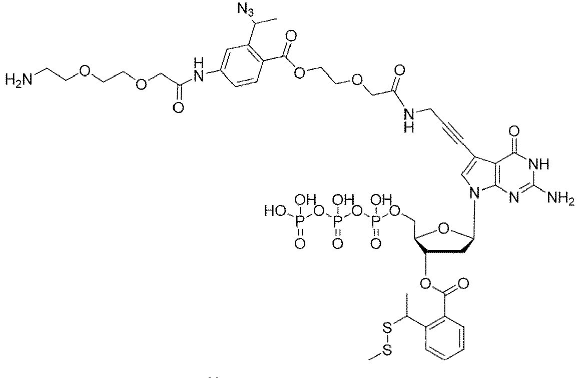 |
| 37B | 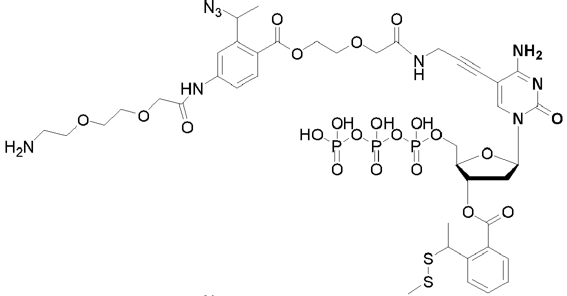 |
| 37C | 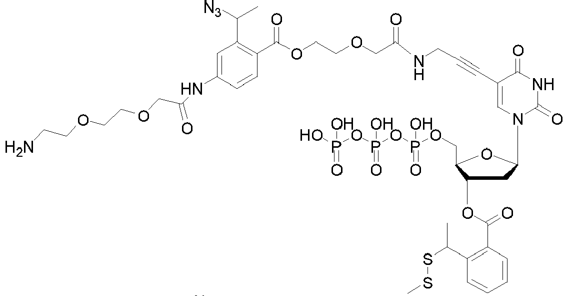 |
| 37D | 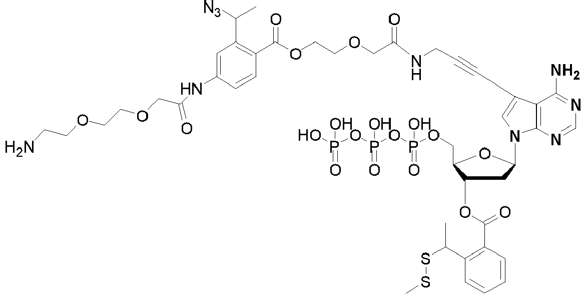 |
| 38A | 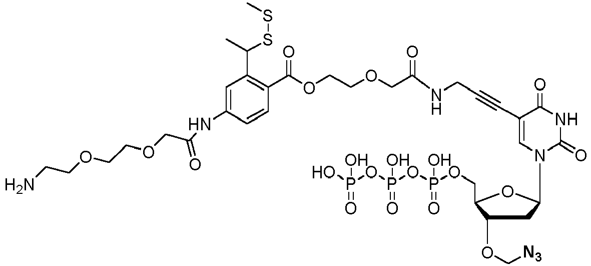 |
| 38B | 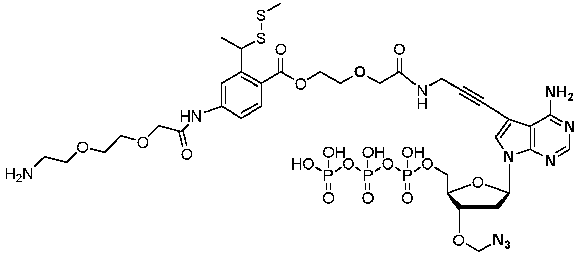 |
| 38C | 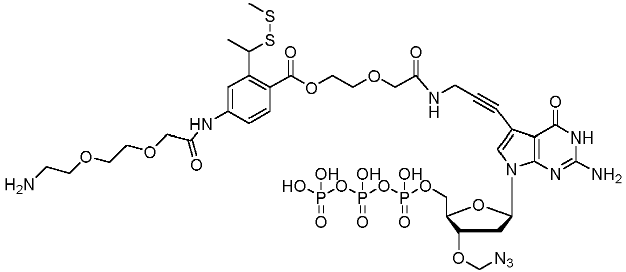 |
| 38D | 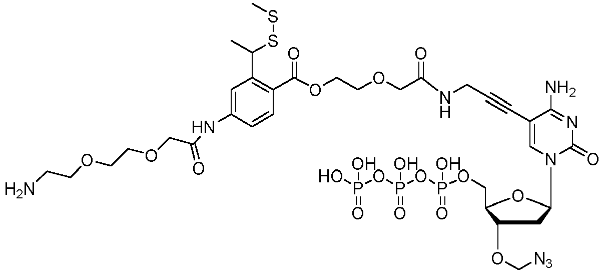 |
| 39A | 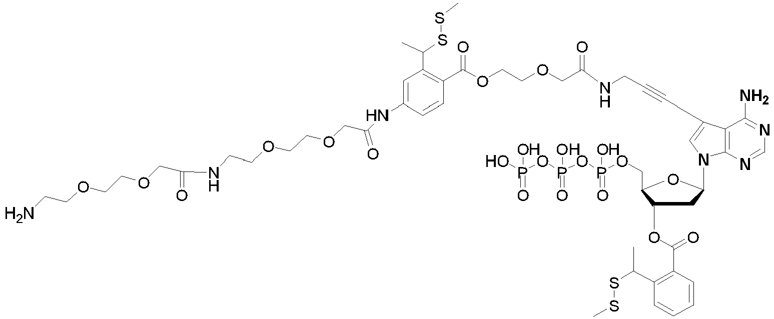 |
| 39B | 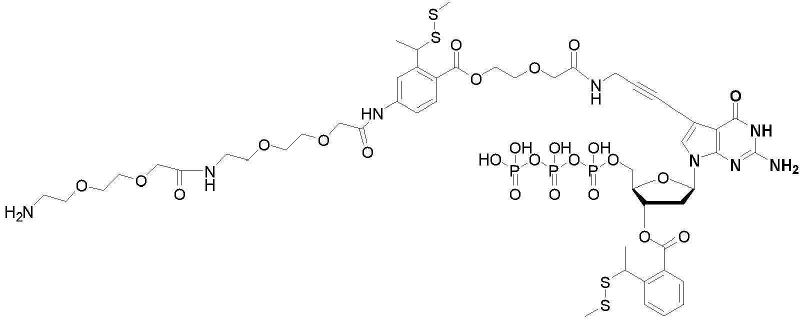 |
| 39C | 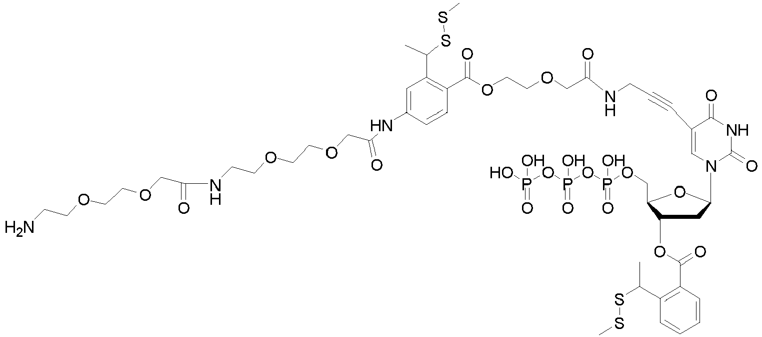 |
| 39D | 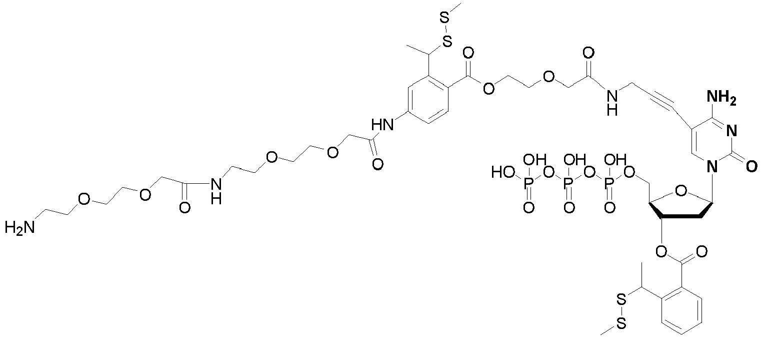 |
| 40A | 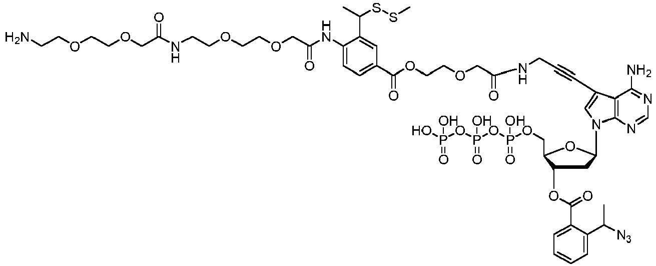 |
| 40B | 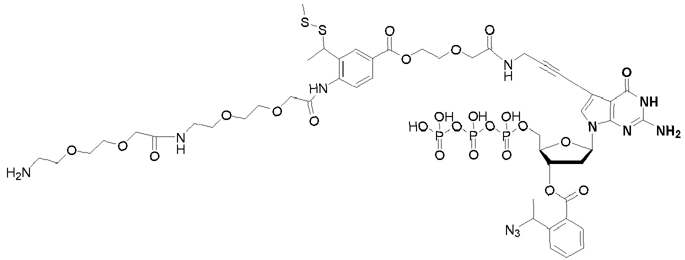 |
| 40C | 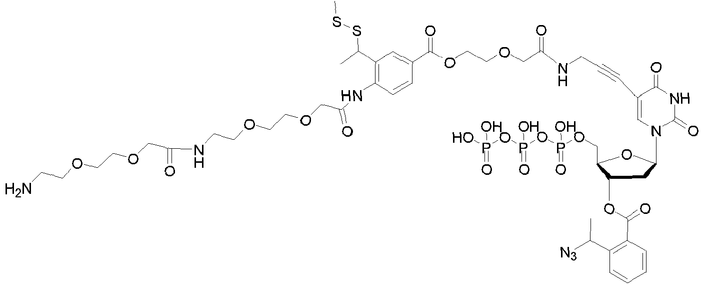 |
| 40D | 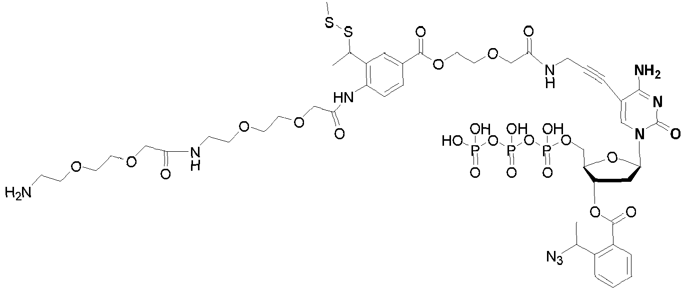 |
| 41A | 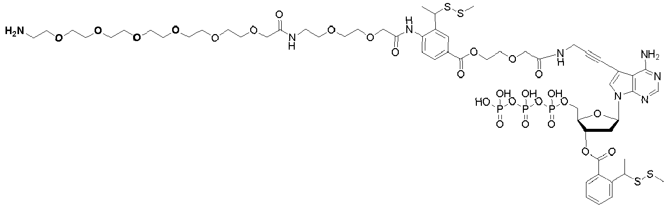 |
| 41B | 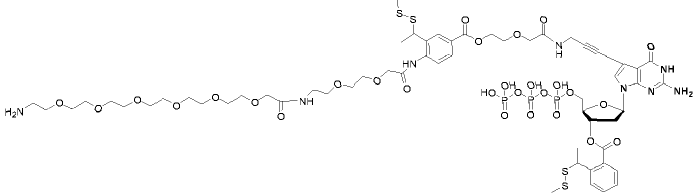 |
| 41C | 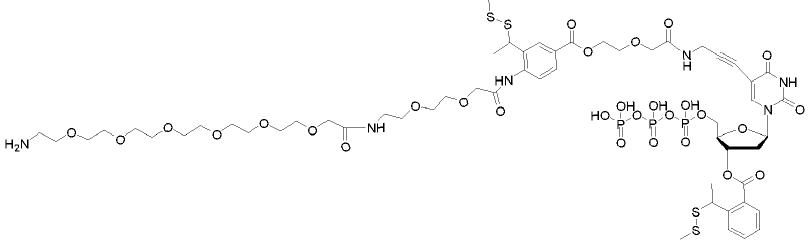 |
| 41D | 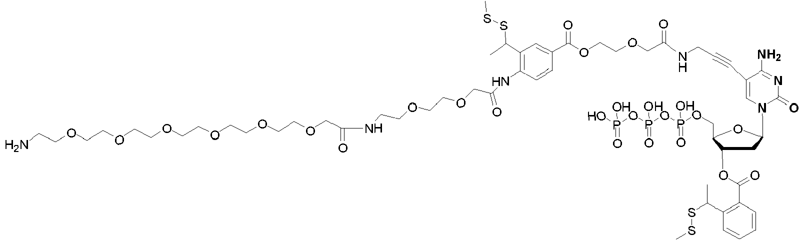 |
| 42A | 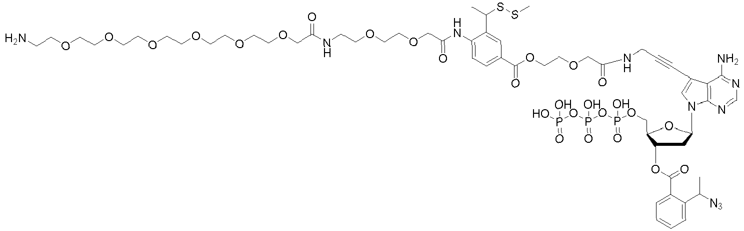 |
| 42B | 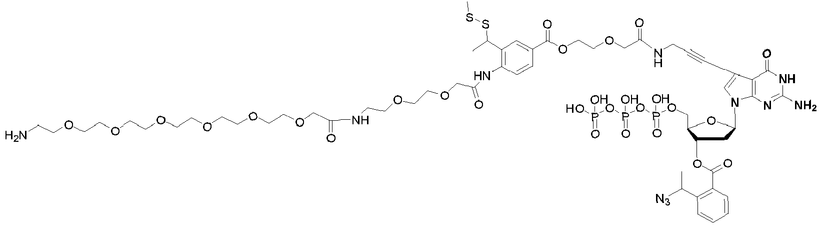 |
| 42C | 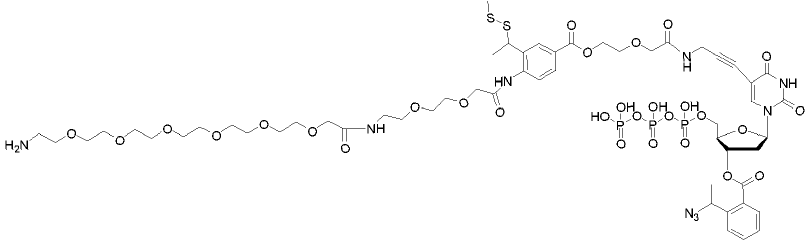 |
| 42D | 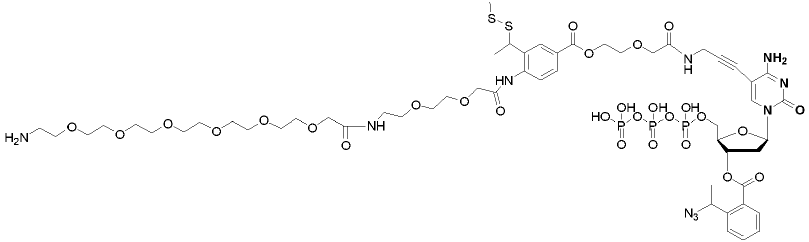 |
| 43A | 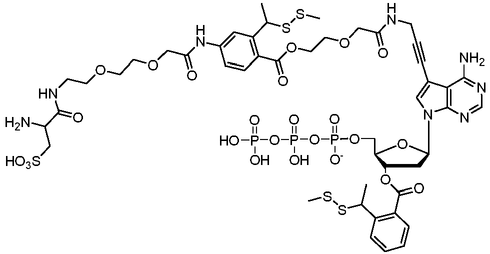 |
| 43B | 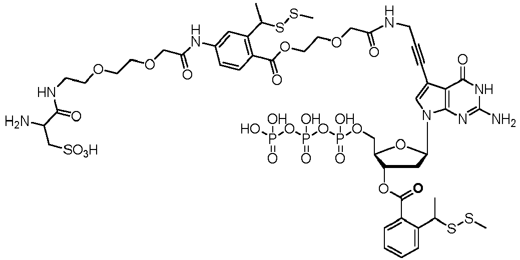 |
| 43C | 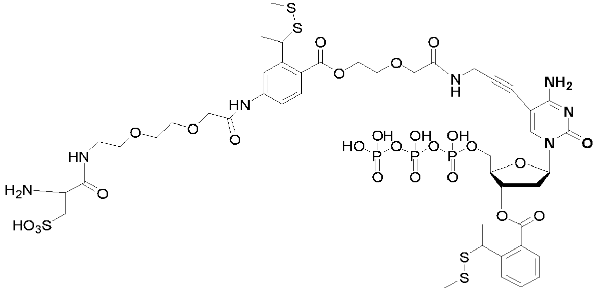 |
| 43D | 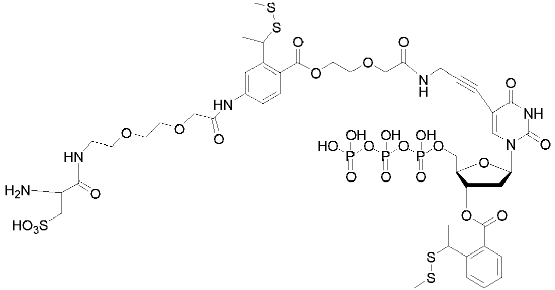 |
| 44A | 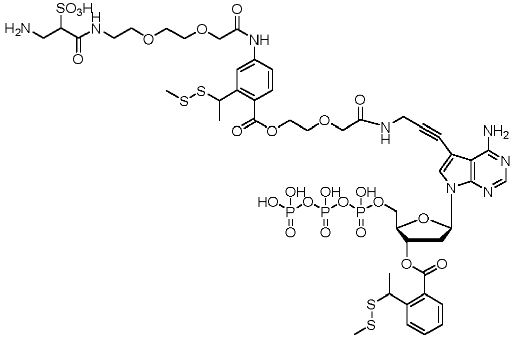 |
| 44B | 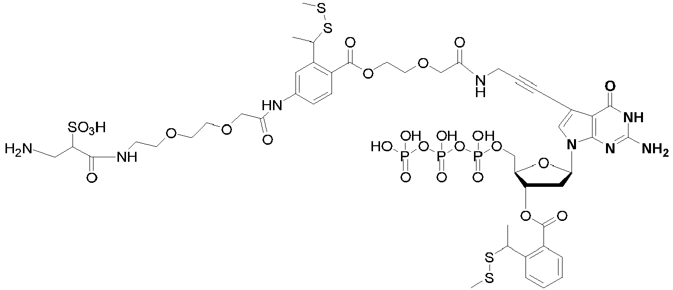 |
| 44C | 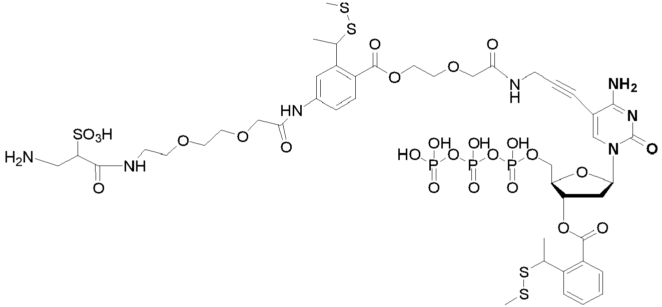 |
| 44D | 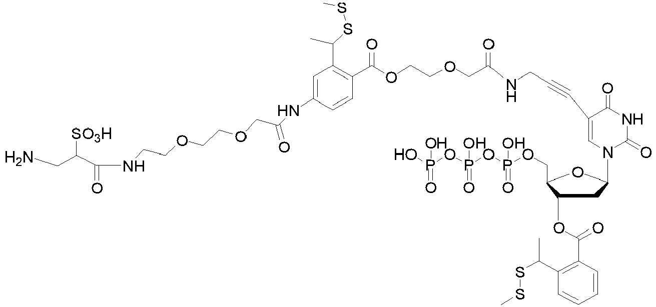 |
| 45A | 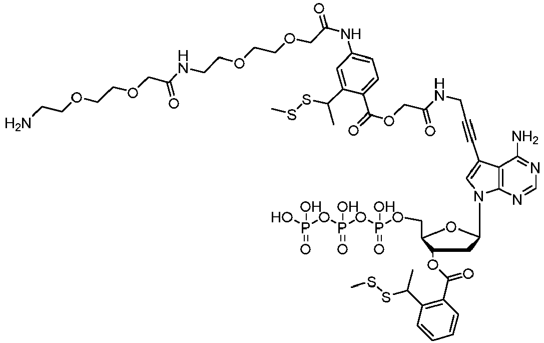 |
| 45B | 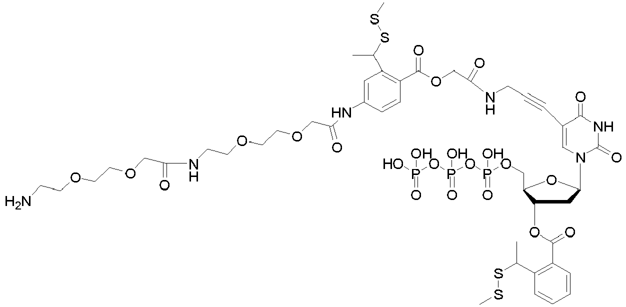 |
| 45C | 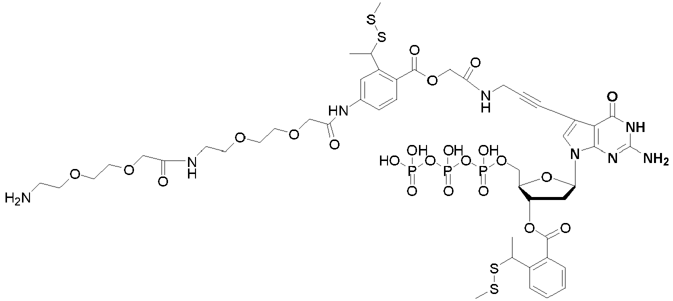 |
| 45D | 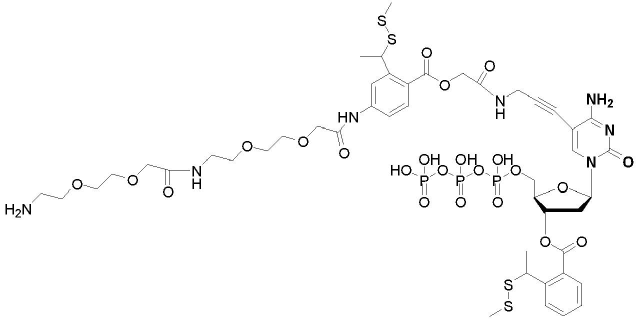 |
| 46A | 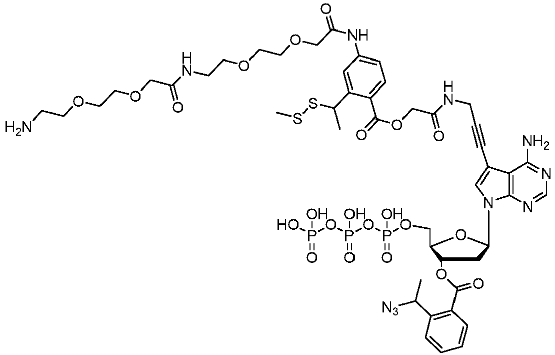 |
| 46B | 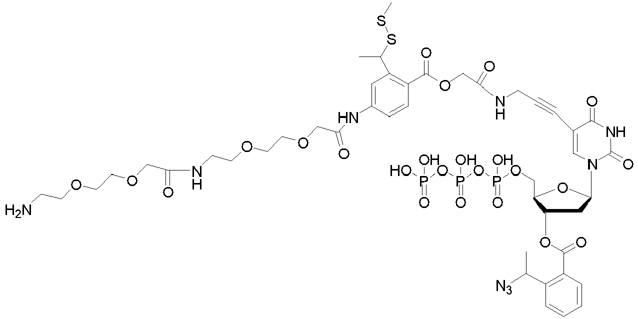 |
| 46C | 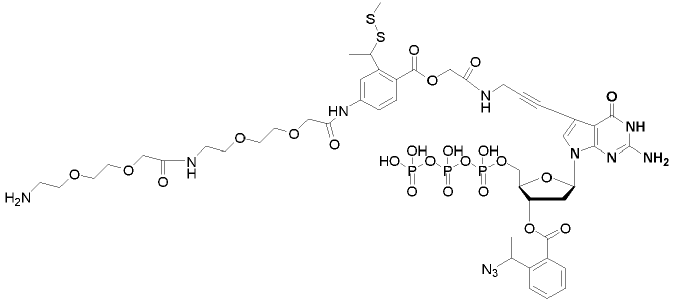 |
| 46D | 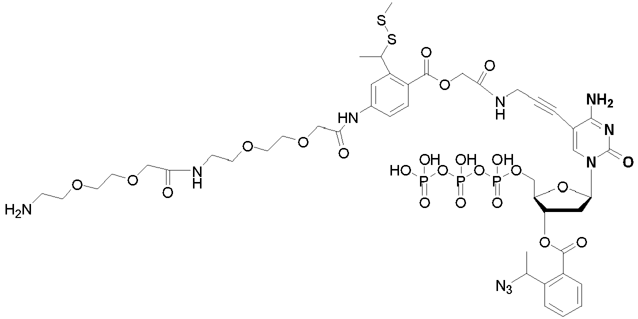 |
| 47A | 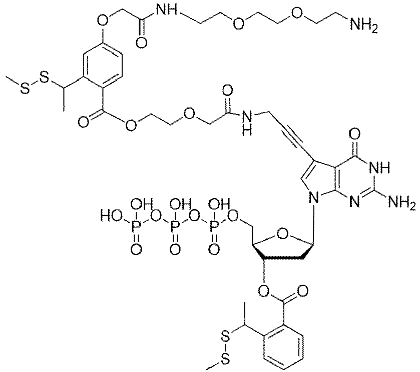 |
| 47B | 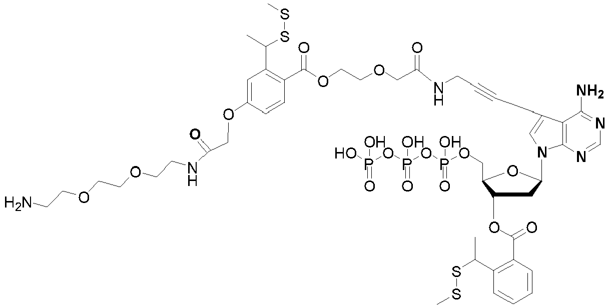 |
| 47C | 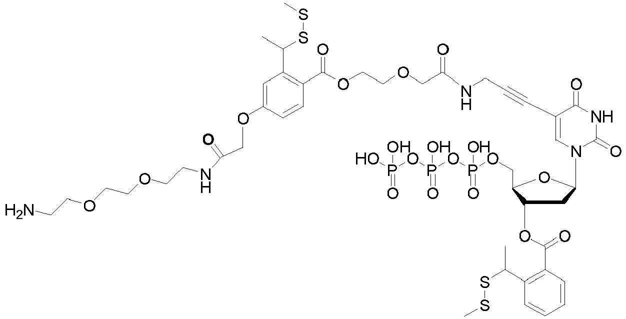 |
| 47D | 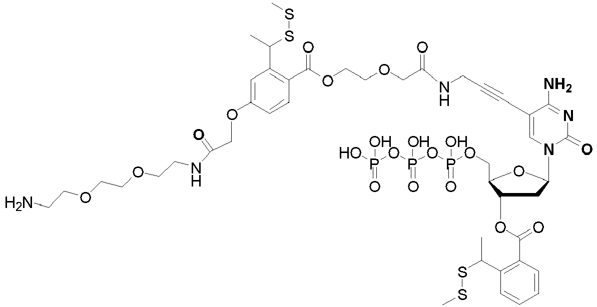 |
| 48A | 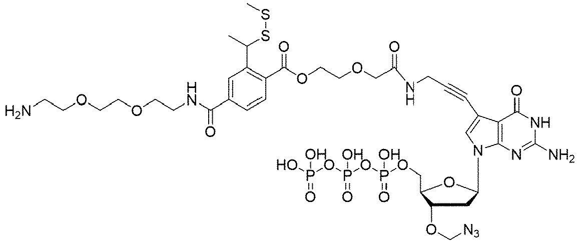 |
| 48B | 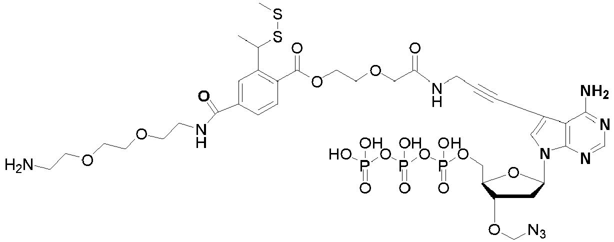 |
| 48C | 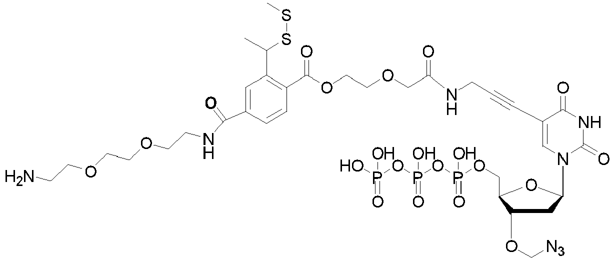 |
| 48D | 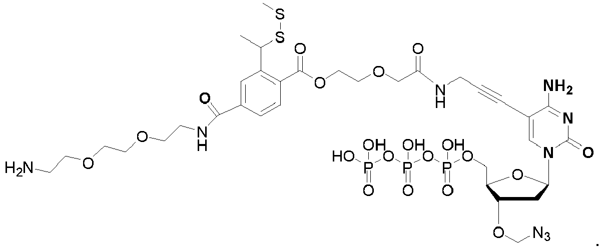 |

In some embodiments, the aforementioned compound or a salt thereof carries an additional detectable label.

In some embodiments, the additional detectable label carried by the compound or a salt thereof is introduced by an affinity reagent (e.g., antibody, aptamer, Affimer, Knottin), wherein the affinity reagent carries the detectable label, and the affinity reagent can specifically recognize and bind to an epitope of the compound or a salt thereof.

In some embodiments, the detectable label is linked to the R in the compound of formula I, formula II, formula I-1, formula I-2, formula II-1 or formula II-2 or to a structural moiety corresponding to the R in the aforementioned specific compound.

In some embodiments, the detectable label is linked to the terminal amino group in 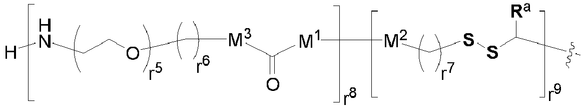 or a structural moiety correspondent thereto.

In some embodiments, the carboxyl in the detectable label is linked to the terminal amino group in 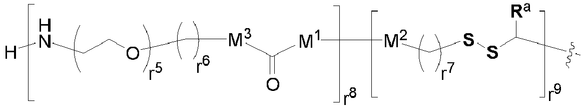 or a structural moiety correspondent thereto via formation of an amide bond.

In some embodiments, the detectable label is linked to the R in the compound of formula III or to a structural moiety corresponding to the R in the aforementioned specific compound.

In some embodiments, the detectable label is linked to the terminal amino group in 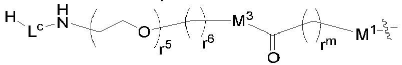 or a structural moiety correspondent thereto.

In some embodiments, the detectable label is linked to the terminal amino group in 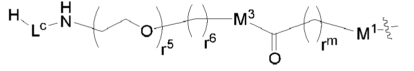 or a structural moiety correspondent thereto via formation of an amide bond.

In some embodiments, base¹ is different, and the additional detectable label carried by the compound of formula I varies.

In some embodiments, base² is different, and the additional detectable label carried by the compound of formula II varies.

In some embodiments, the detectable label is a fluorescent label.

In some embodiments, the detectable label is selected from: iF700,

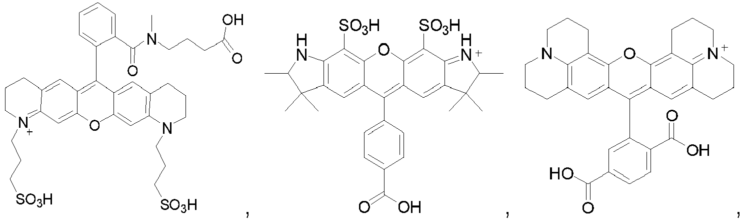

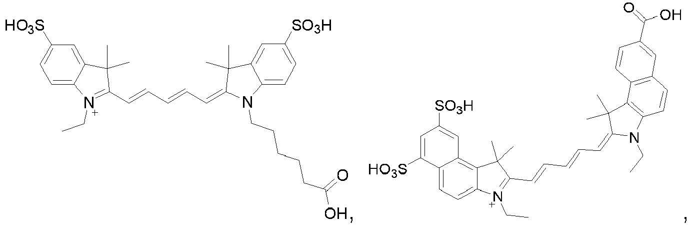

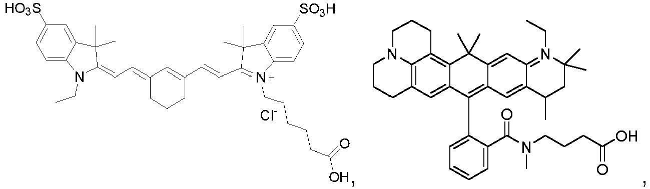

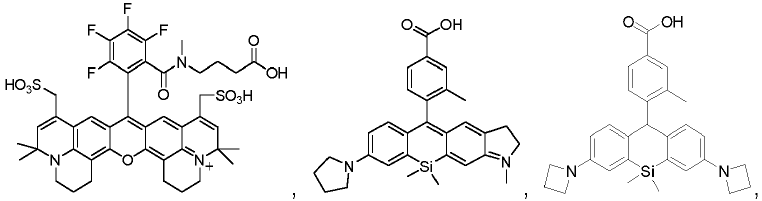

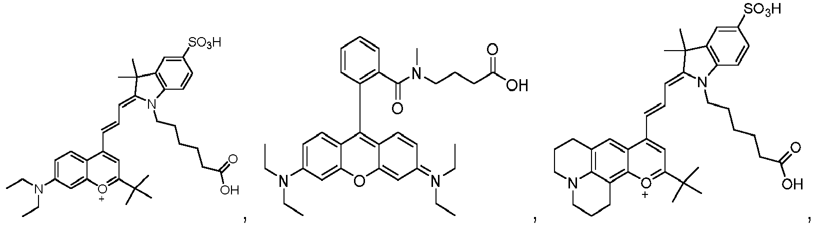

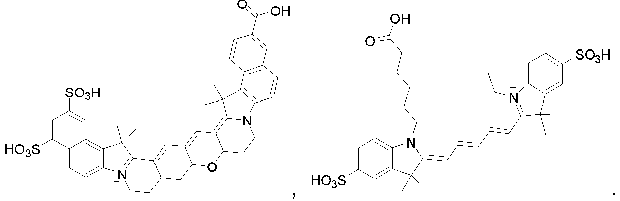

In some embodiments, the compound is selected from the following Table B:

**Table B:**

| Serial Number | Structural formulae |
|---|---|
| 1A' | 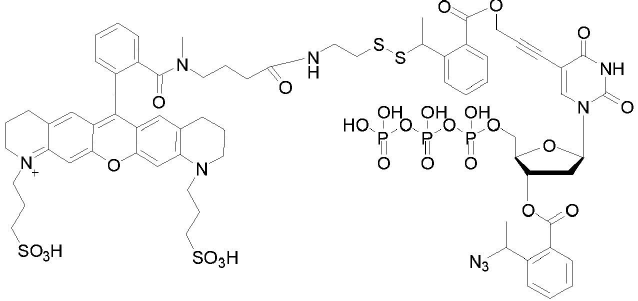 |
| 1B' | 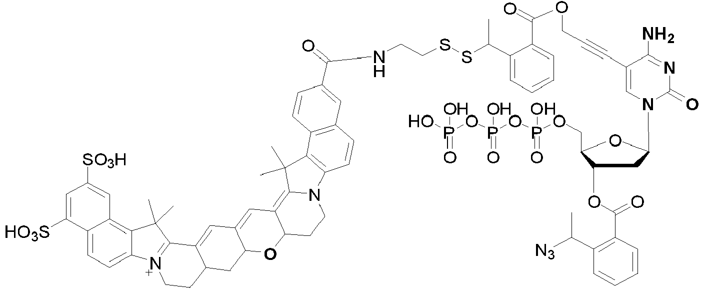 |
| 1C' | 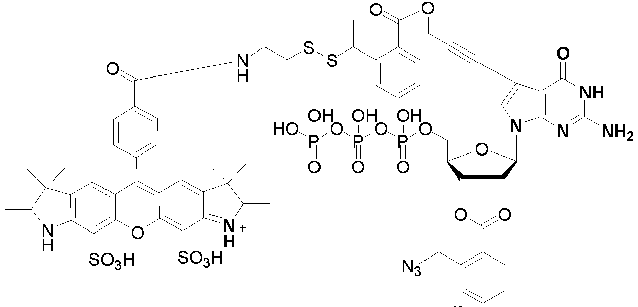 |
| 1D' | 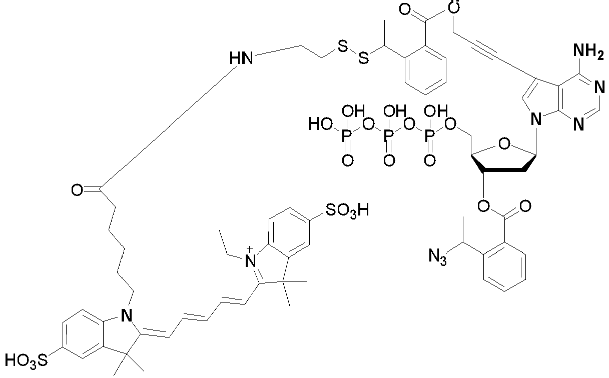 |
| 2A' | 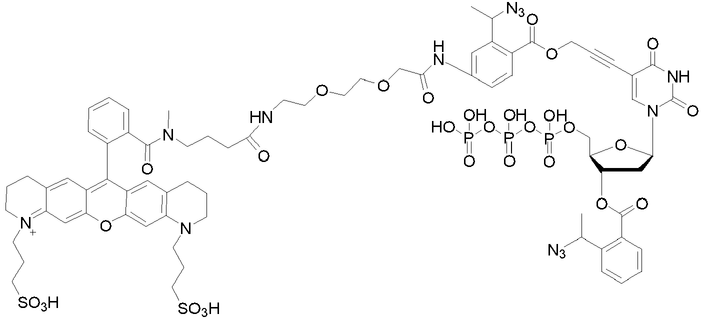 |
| 2B' | 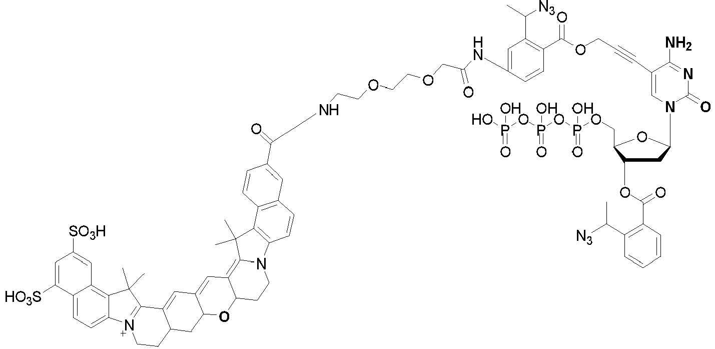 |
| 2C' | 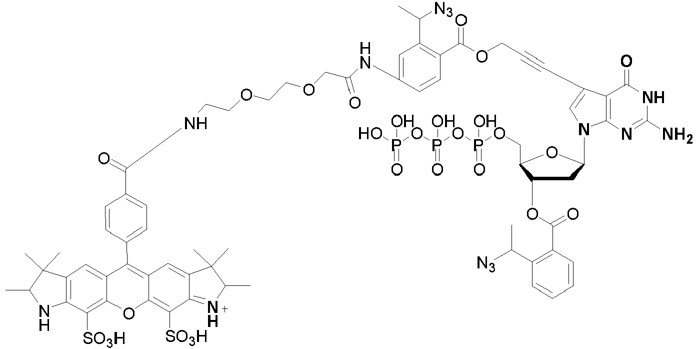 |
| 2D' | 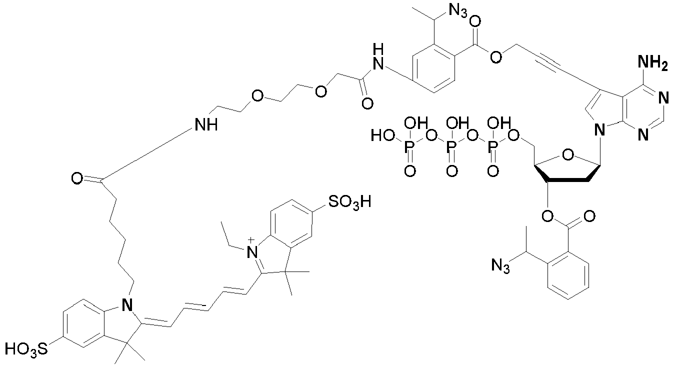 |
| 3A' | 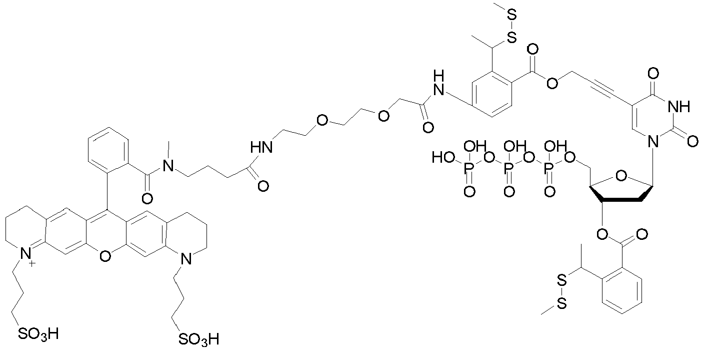 |
| 3B' | 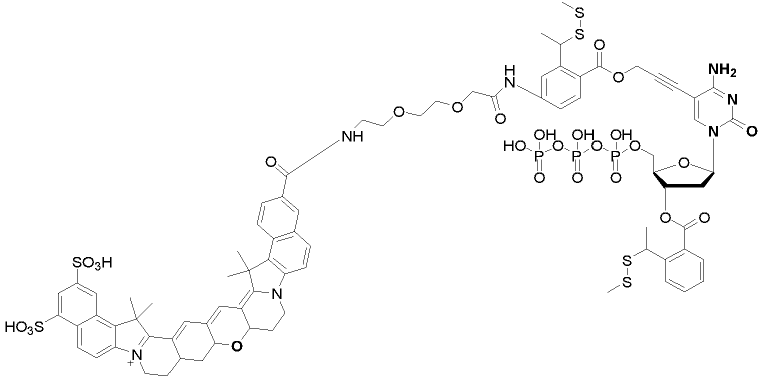 |
| 3C' | 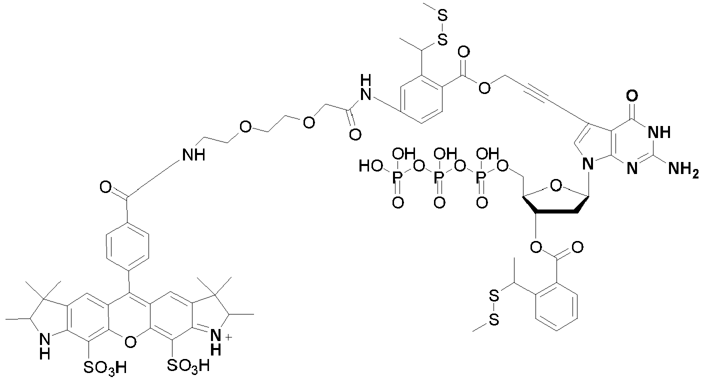 |
| 3D' | 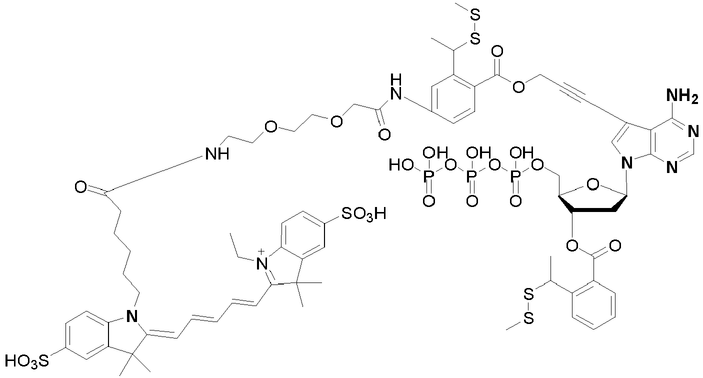 |
| 4A' | 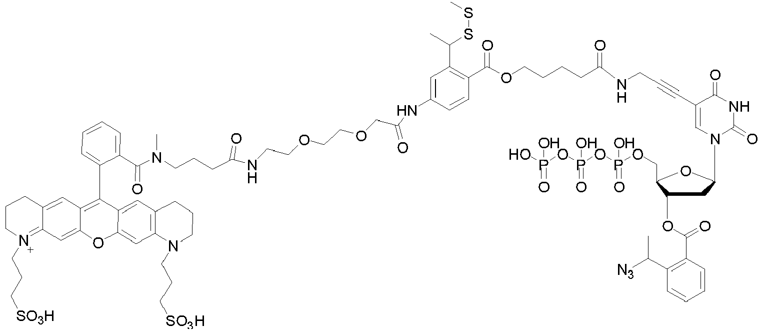 |
| 4B' | 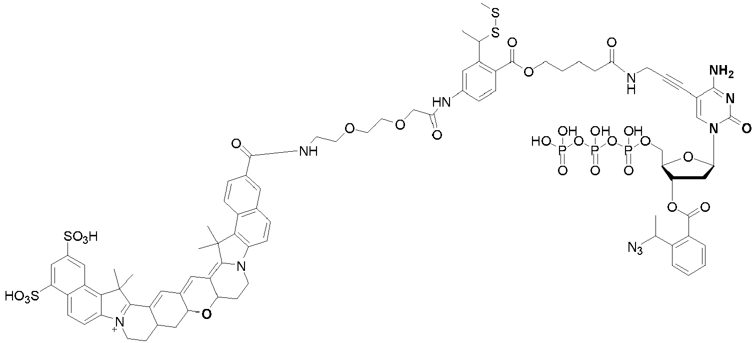 |
| 4C' | 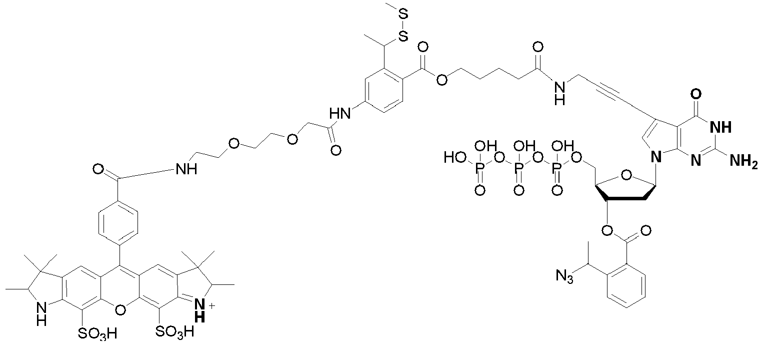 |
| 4D' | 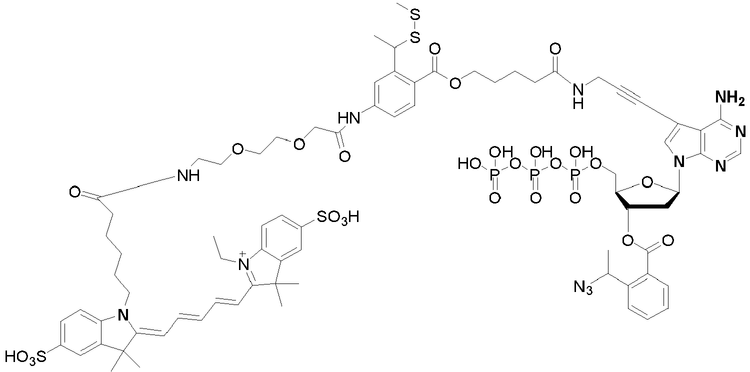 |
| 5A' | 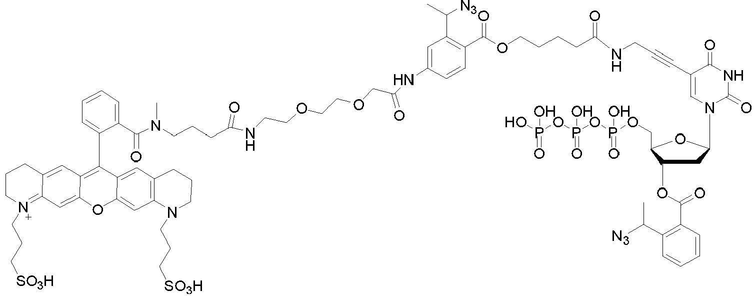 |
| 5B' | 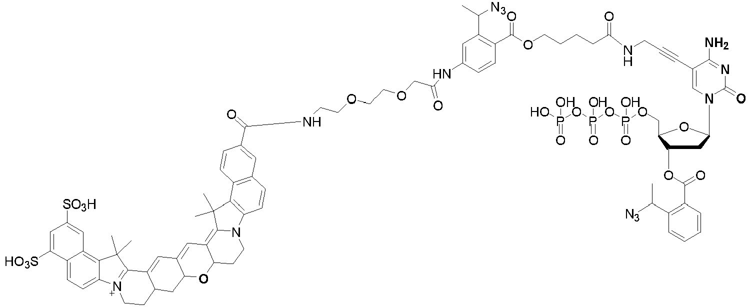 |
| 5C' | 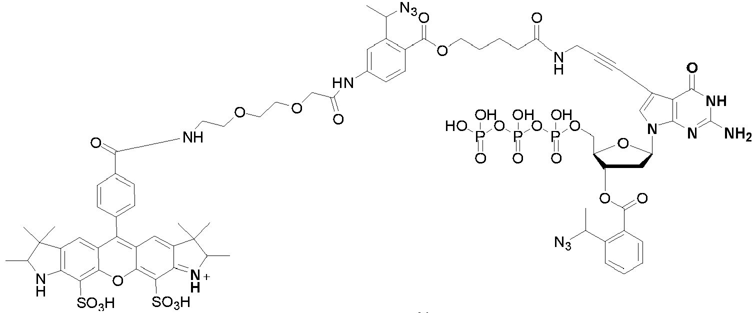 |
| 5D' | 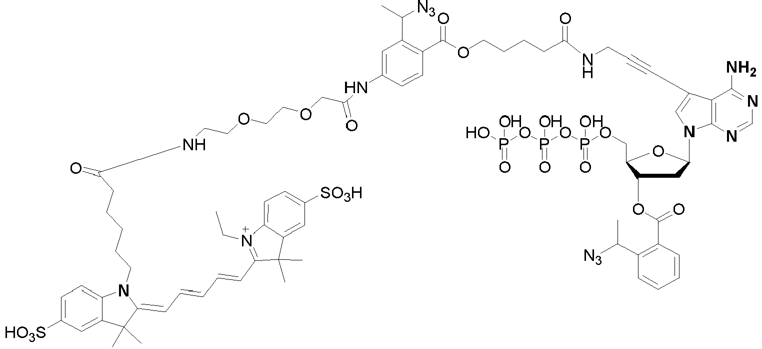 |
| 6A' | 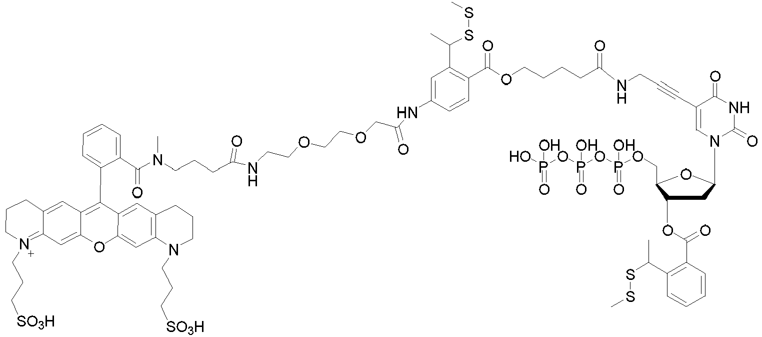 |
| 6B' | 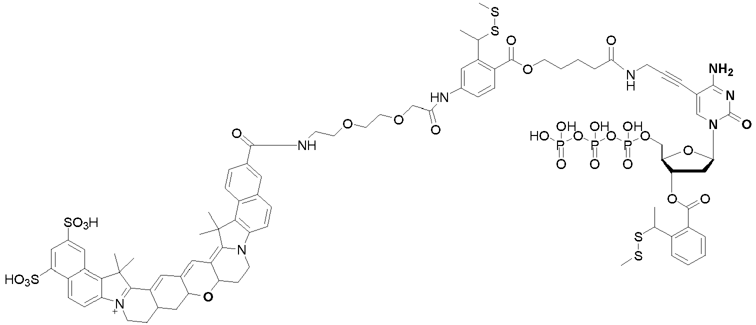 |
| 6C' | 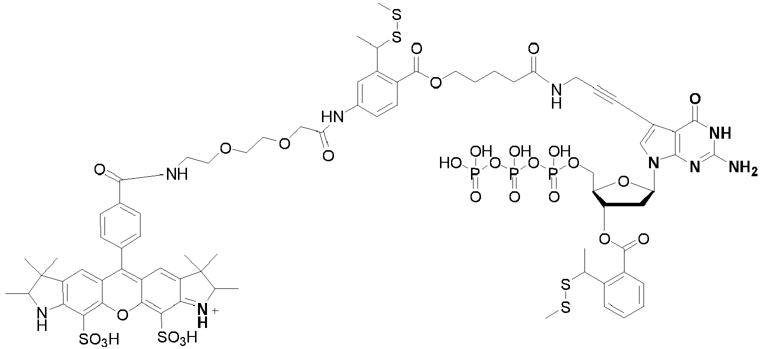 |
| 6D' | 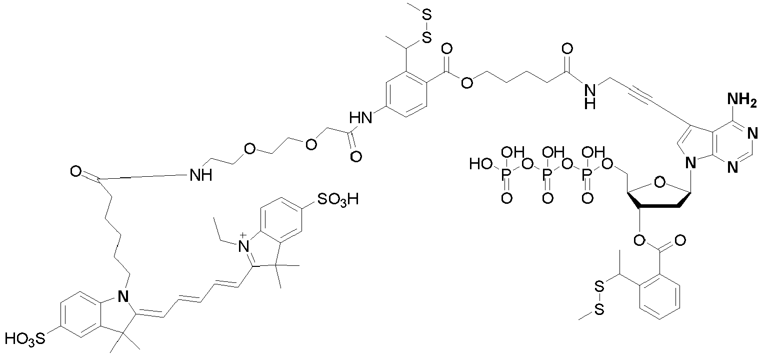 |
| 7A' | 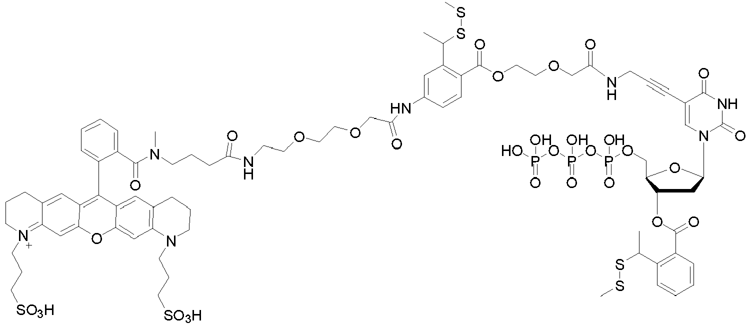 |
| 7B' | 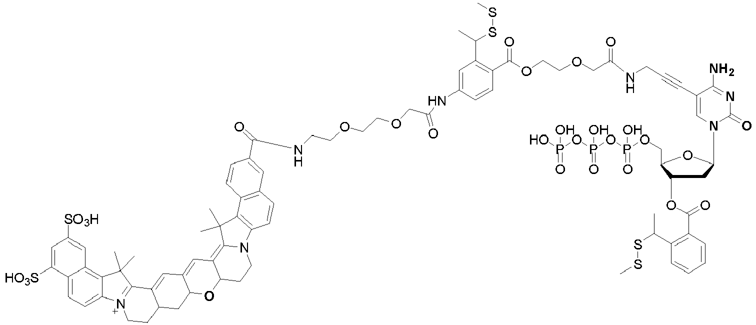 |
| 7B" | 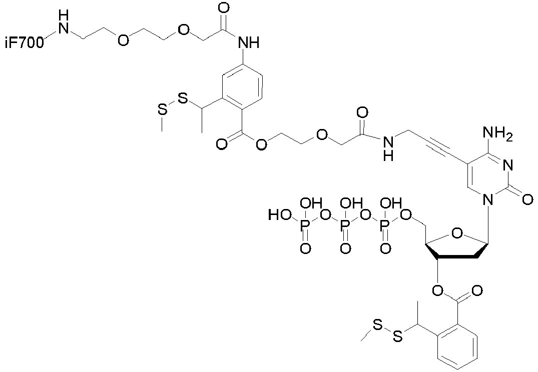 |
| 7C' | 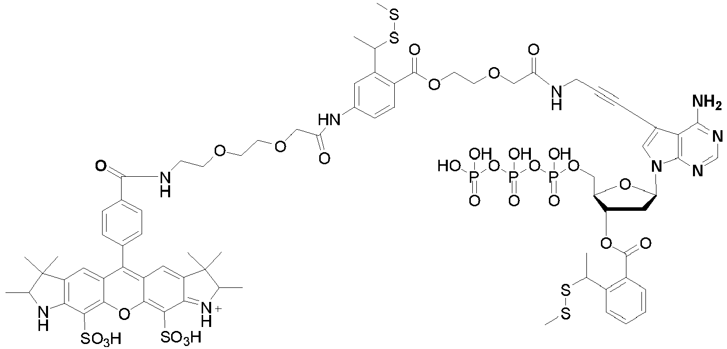 |
| 7D' | 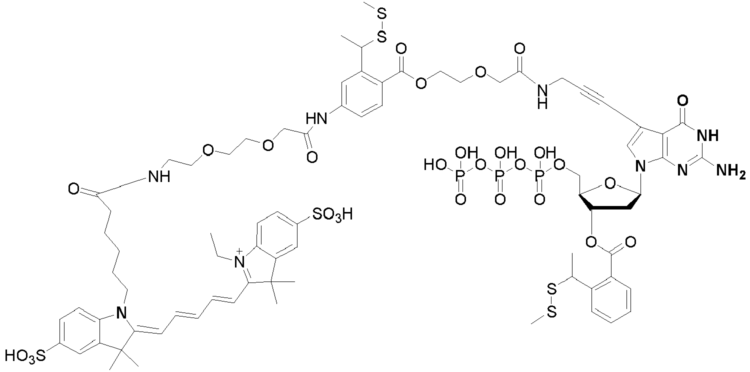 |
| 8-1A' | 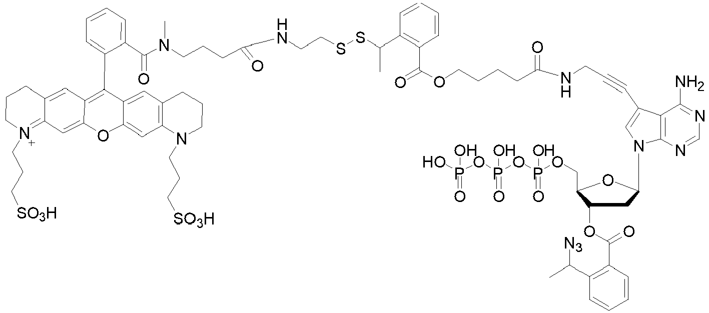 |
| 8-1B' | 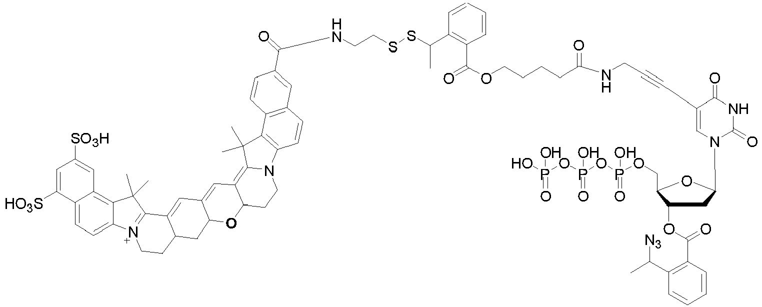 |
| 8-1C' | 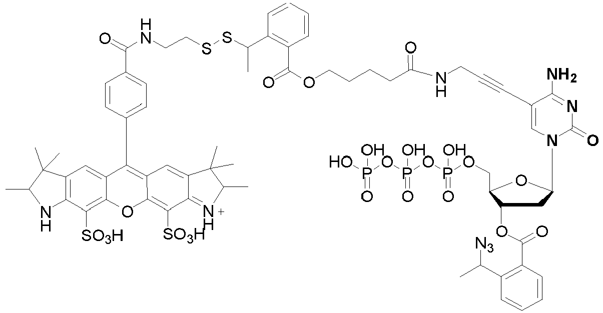 |
| 8-1D' | 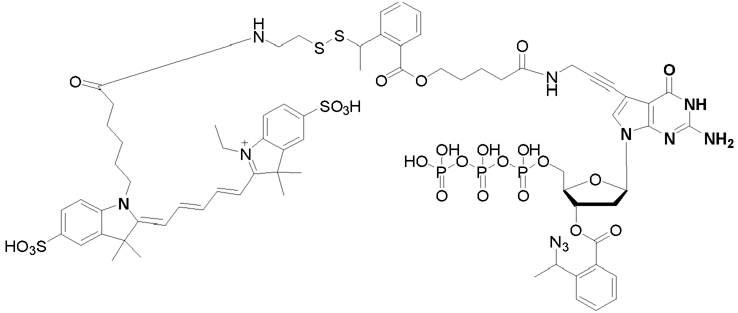 |
| 8-2A' | 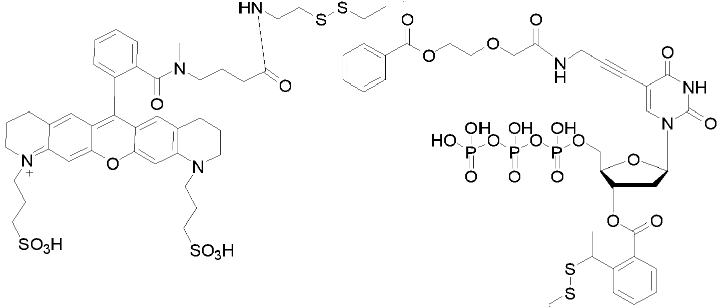 |
| 8-2B' | 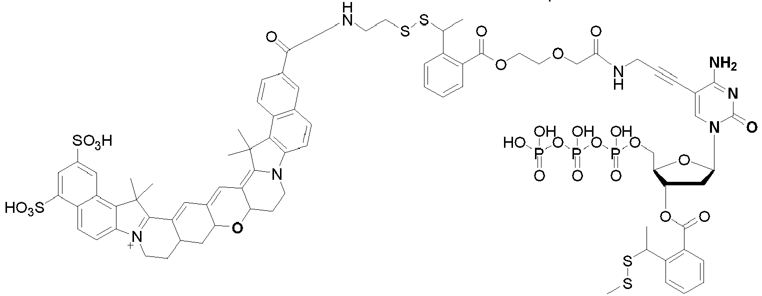 |
| 8-2C' | 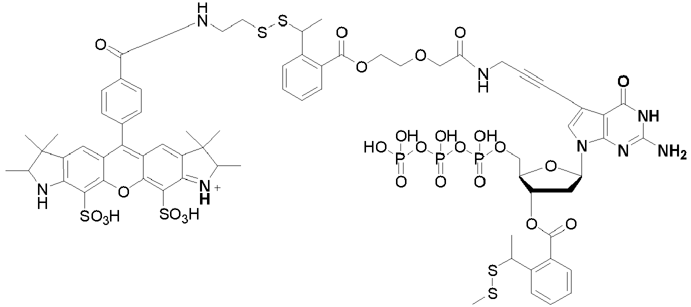 |
| 8-2D' | 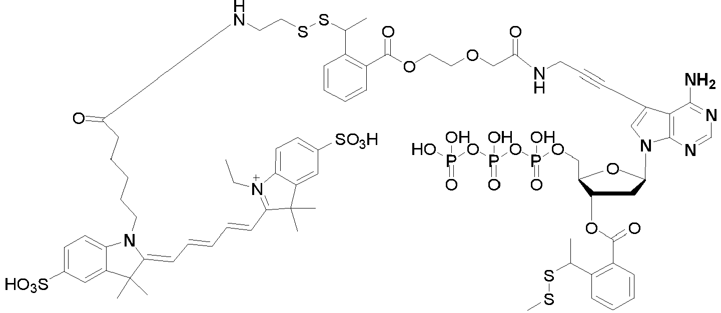 |
| 9A' | 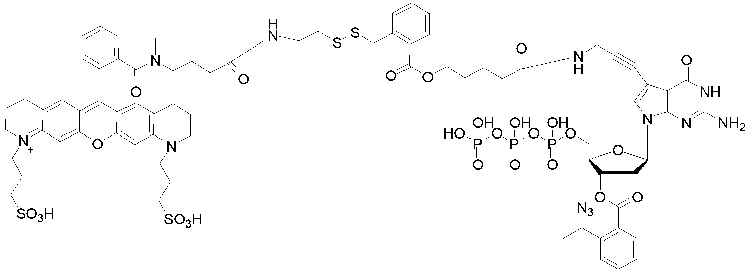 |
| 9B' | 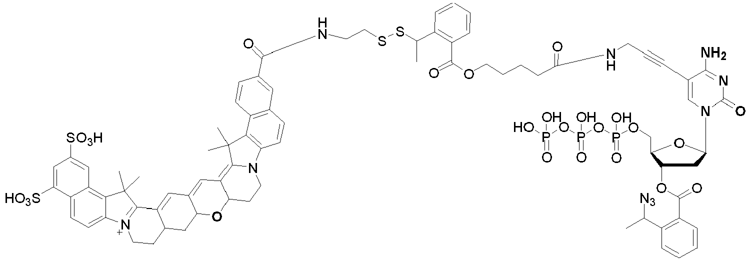 |
| 9C' | 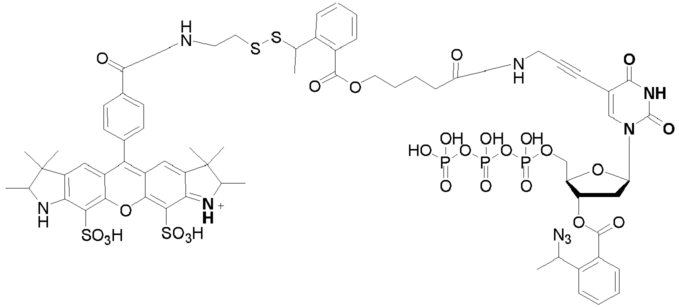 |
| 9D' | 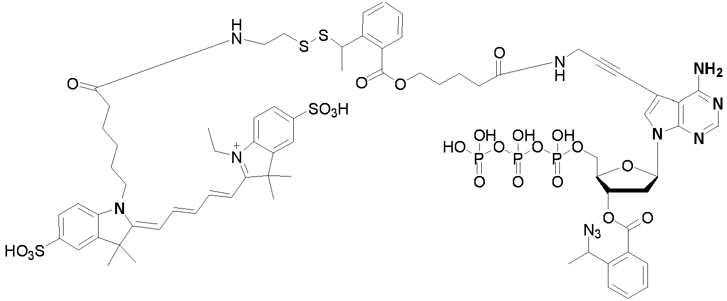 |
| 10A' | 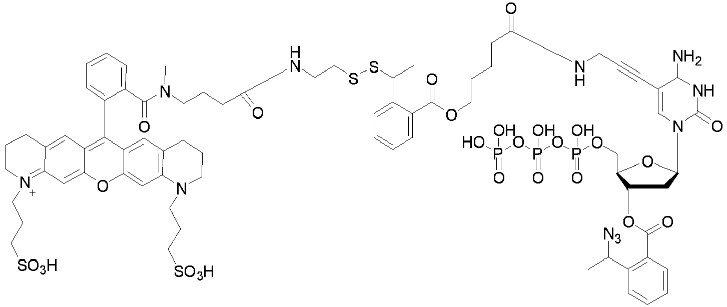 |
| 10B' | 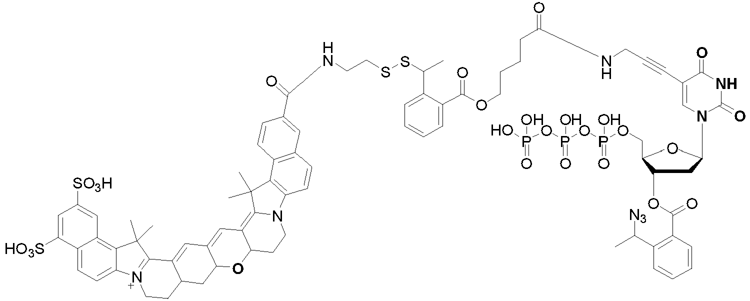 |
| 10C' | 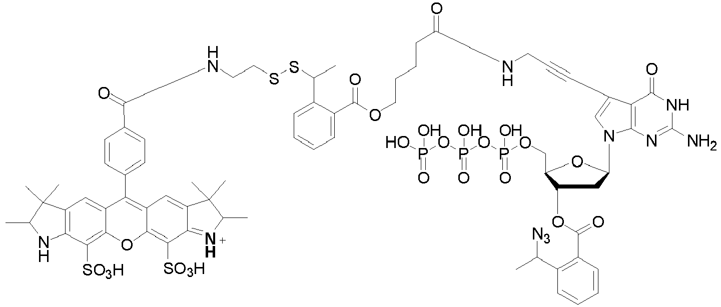 |
| 10D' | 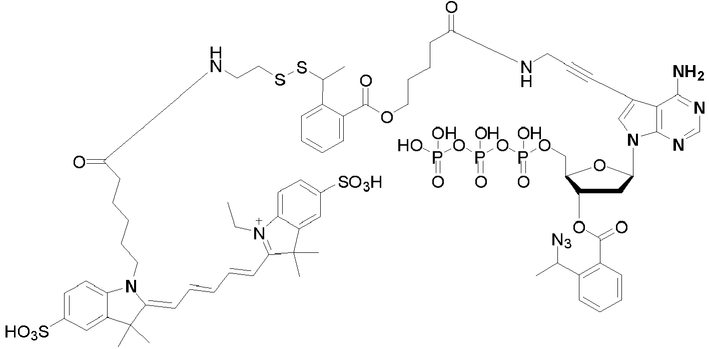 |
| 11A' | 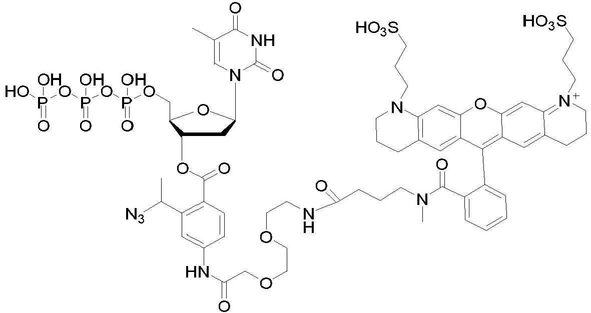 |
| 11B' | 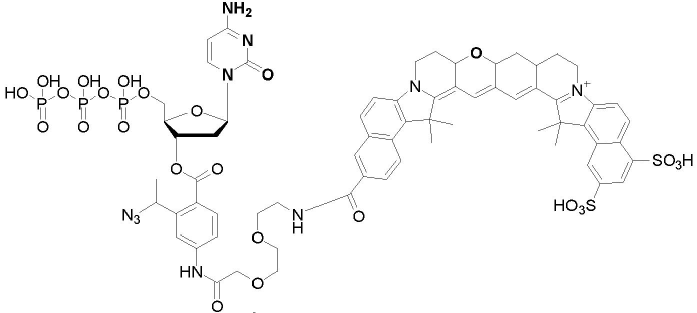 |
| 11C' | 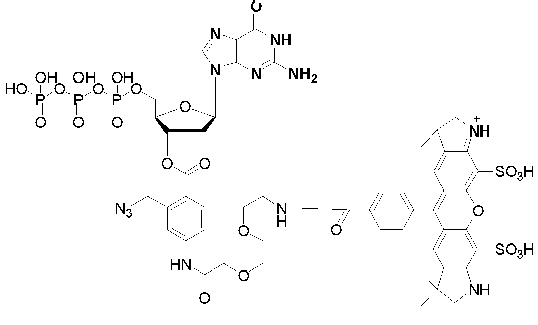 |
| 11D' | 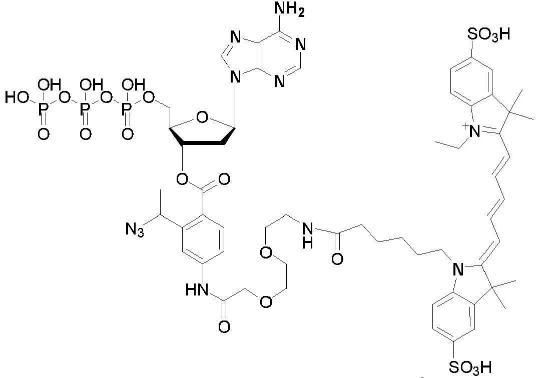 |
| 12A' | 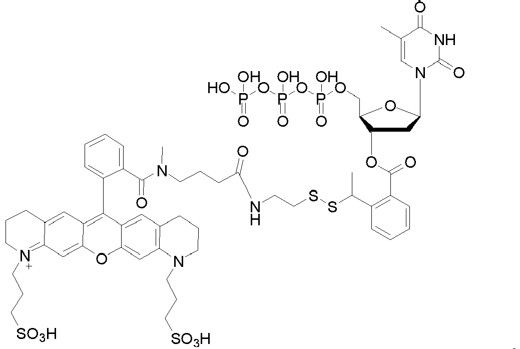 |
| 12B' | 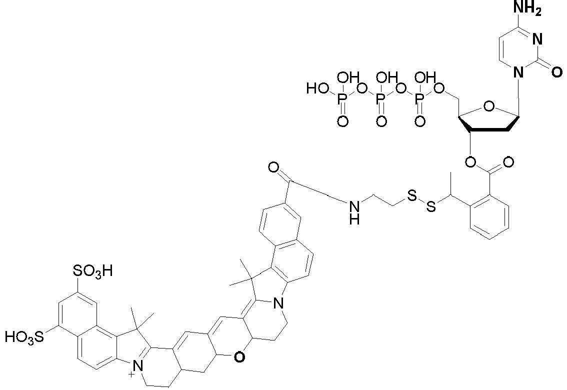 |
| 12C' | 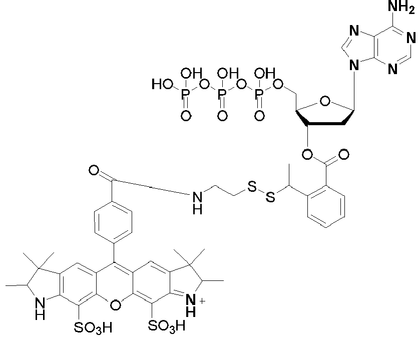 |
| 12D' | 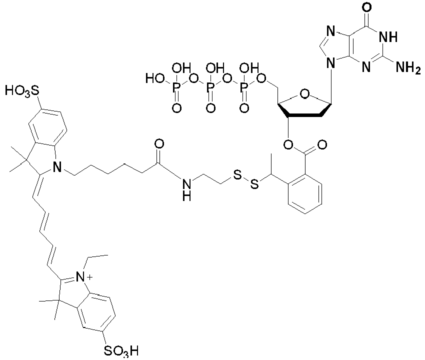 |
| 13A' | 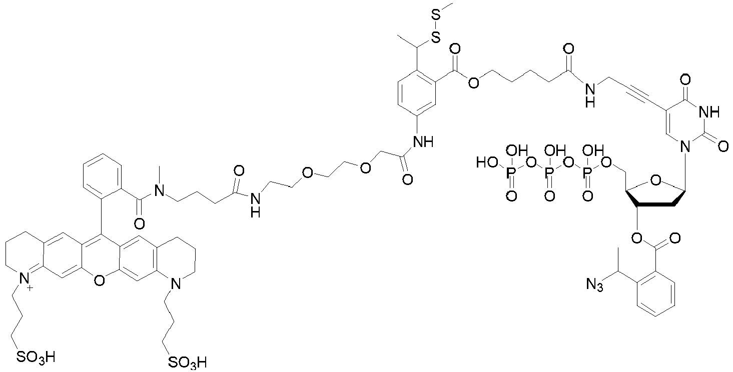 |
| 13B' | 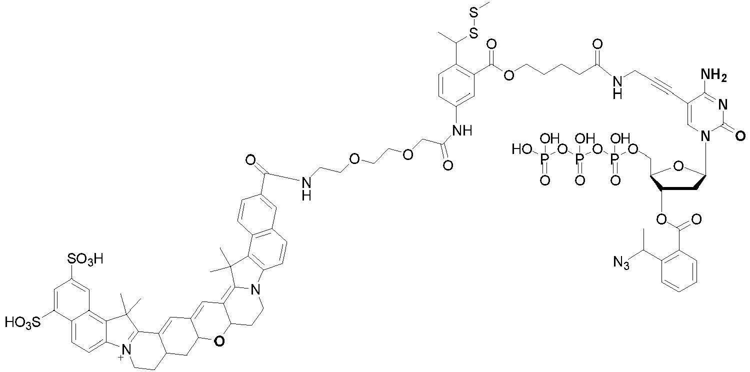 |
| 13C' | 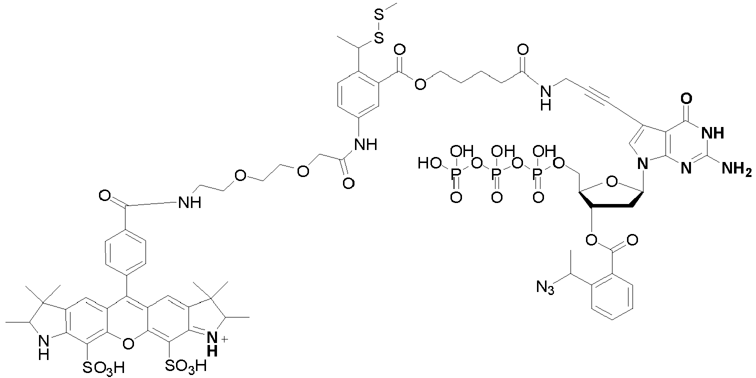 |
| 13D' | 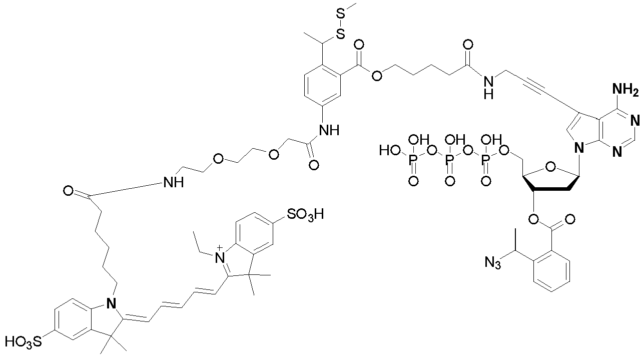 |
| 14A' | 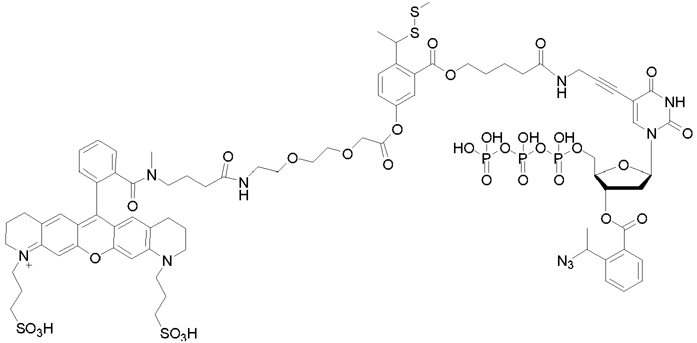 |
| 14B' | 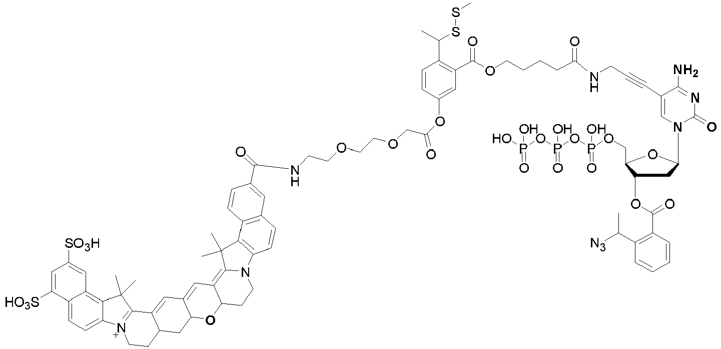 |
| 14C' | 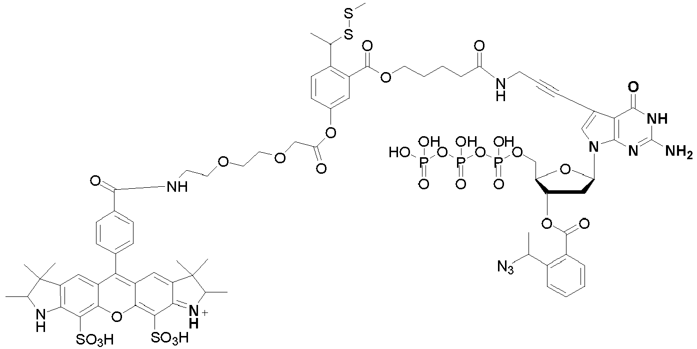 |
| 14D' | 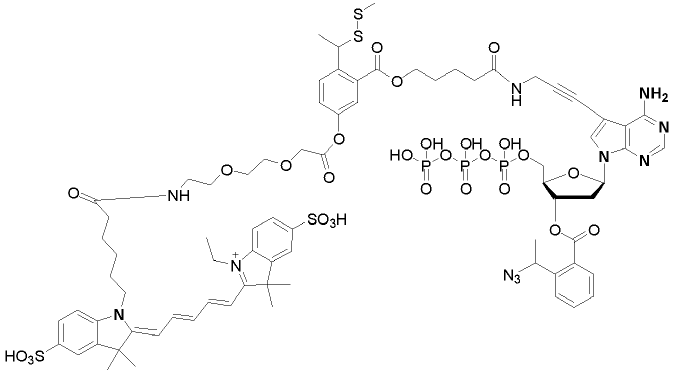 |
| 15A' | 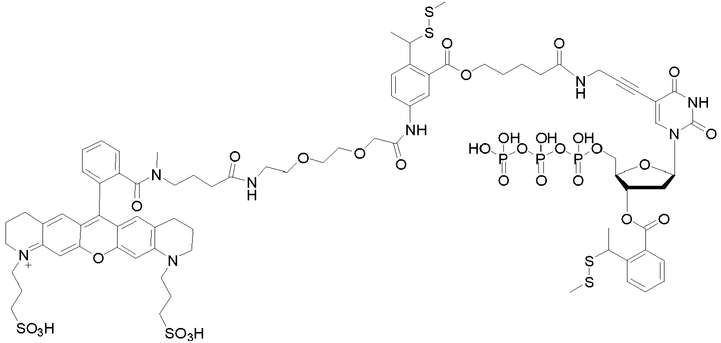 |
| 15B' | 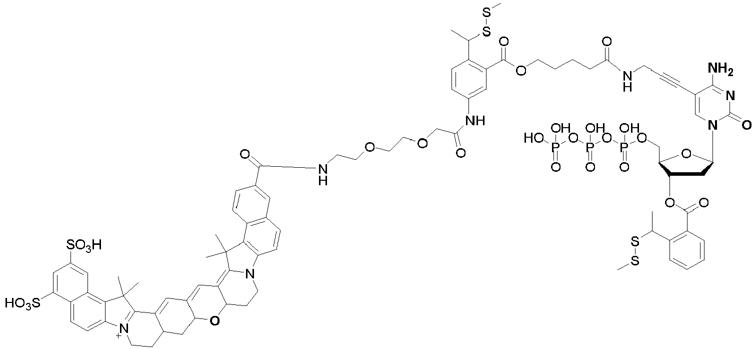 |
| 15C' | 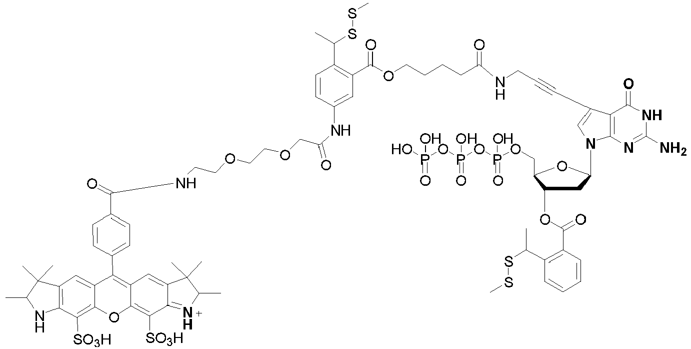 |
| 15D' | 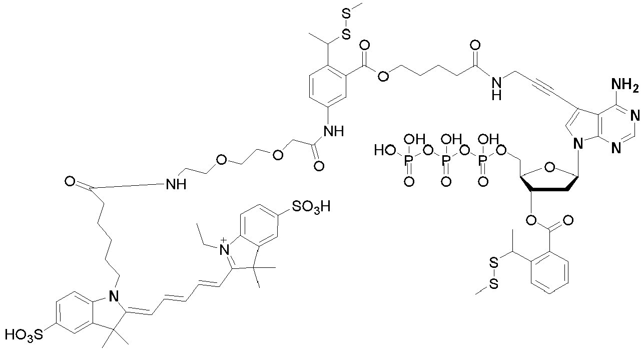 |
| 16A' | 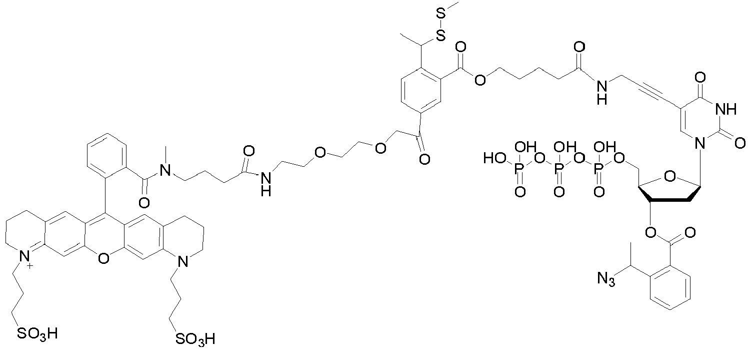 |
| 16B' | 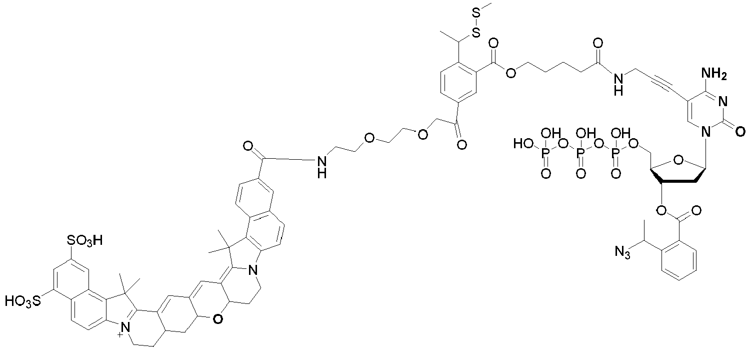 |
| 16C' | 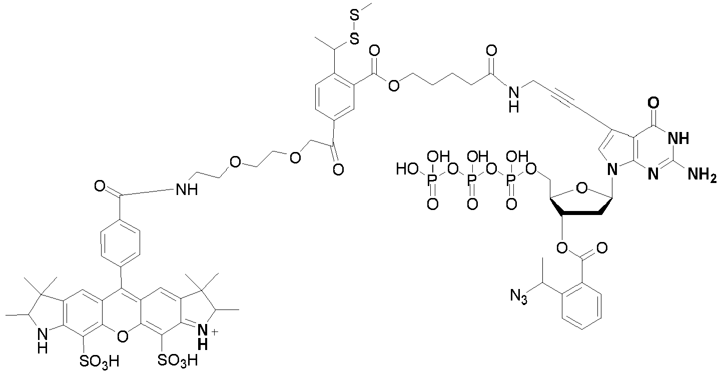 |
| 16D' | 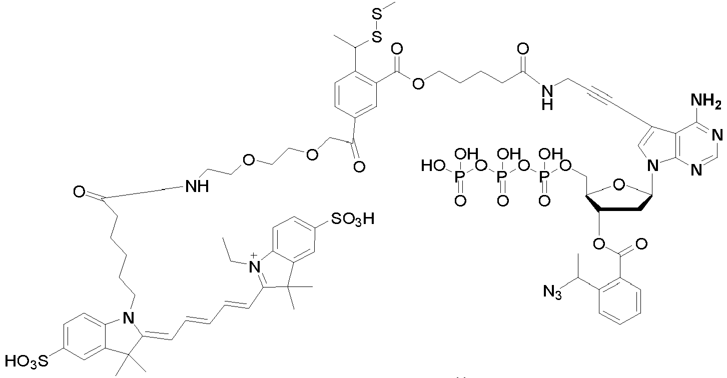 |
| 17A' | 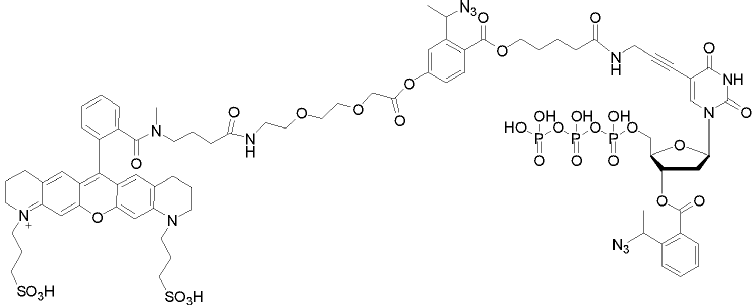 |
| 17B' | 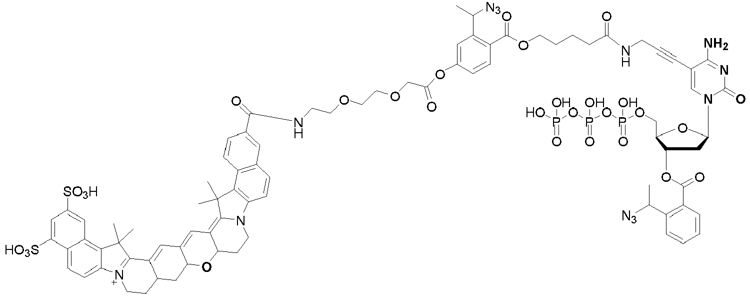 |
| 17C' | 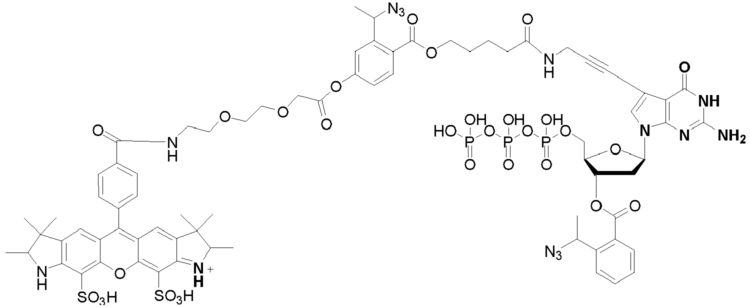 |
| 17D' | 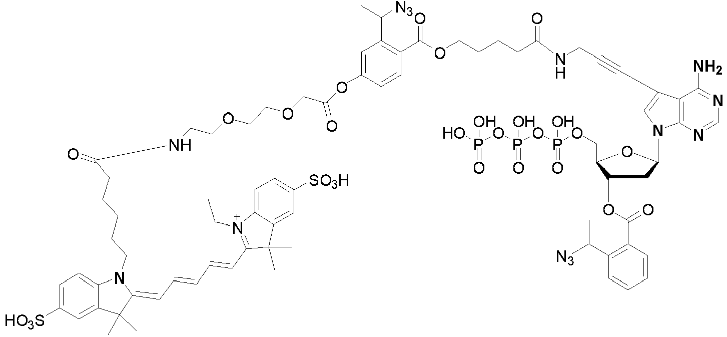 |
| 18A' | 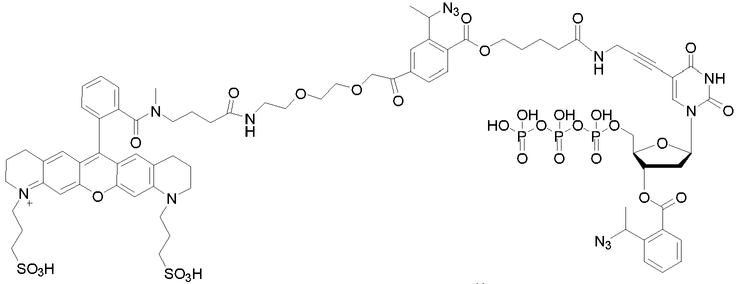 |
| 18B' | 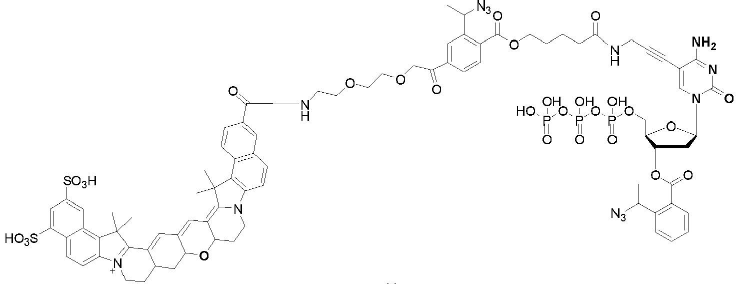 |
| 18C' | 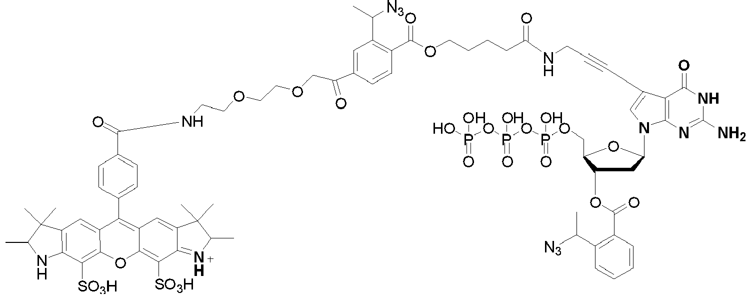 |
| 18D' | 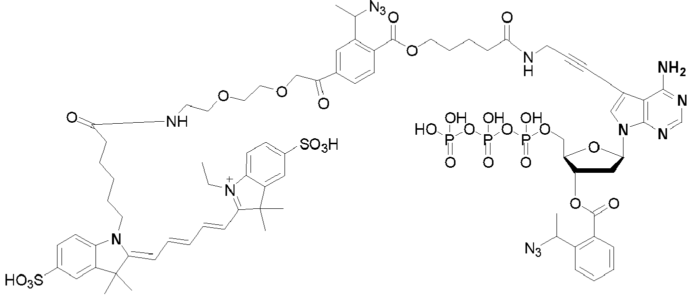 |
| 19A' | 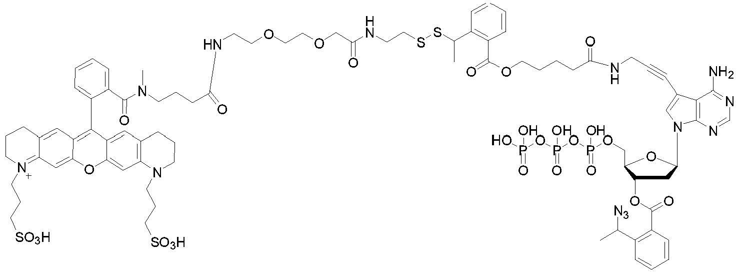 |
| 19B' | 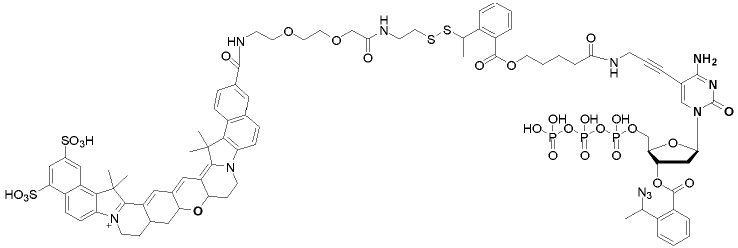 |
| 19C' | 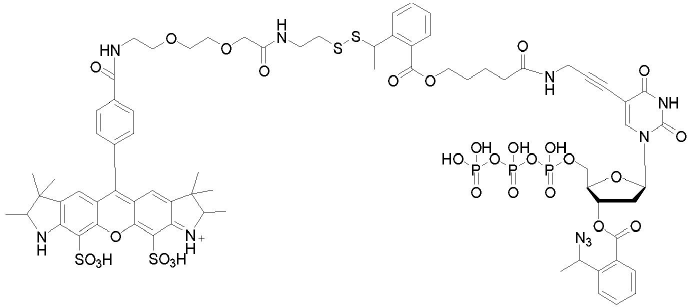 |
| 19D' | 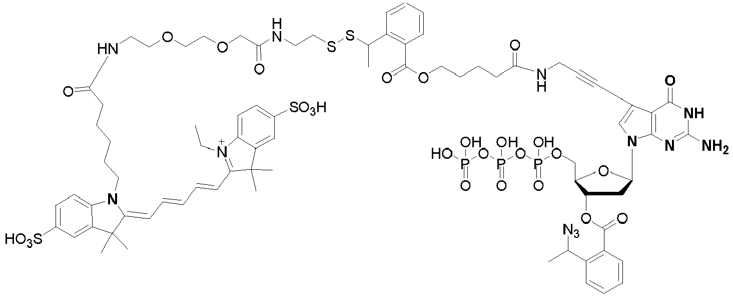 |
| 20A' | 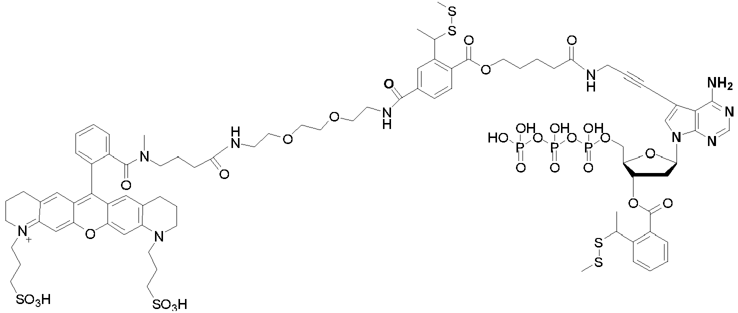 |
| 20B' | 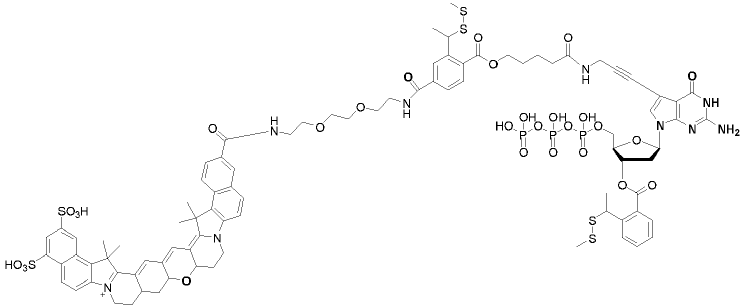 |
| 20C' | 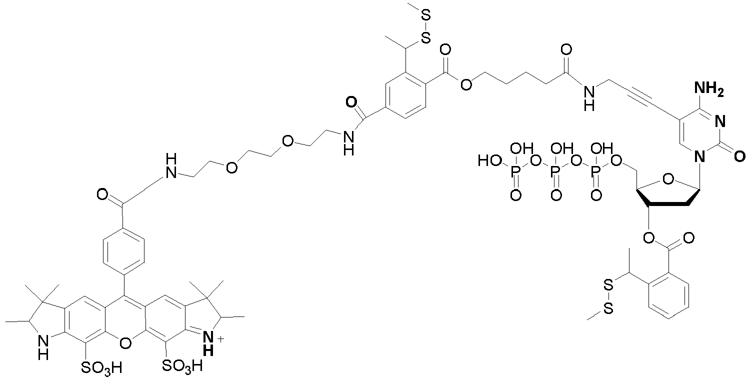 |
| 20D' | 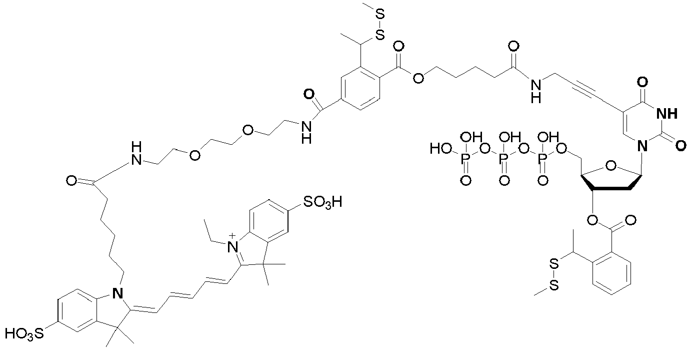 |
| 21A' | 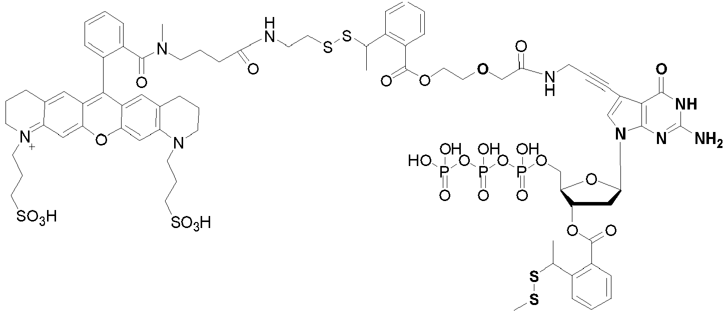 |
| 21B' | 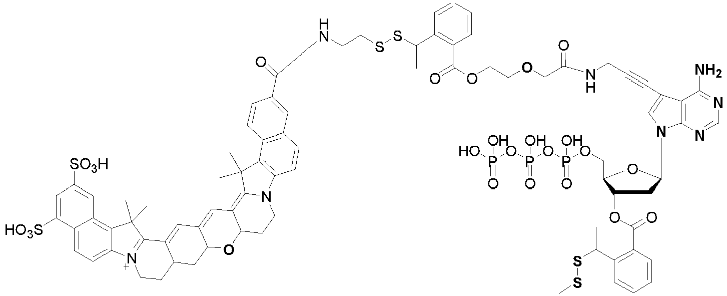 |
| 21C' | 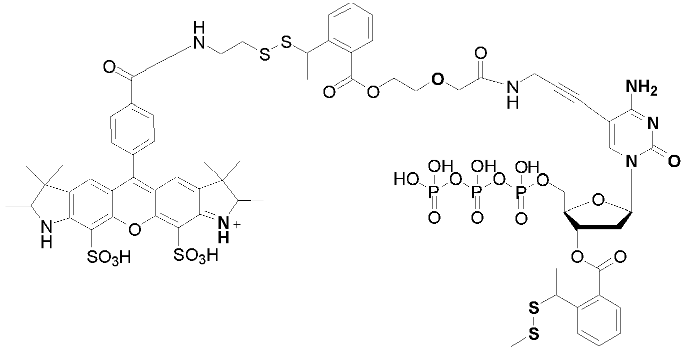 |
| 21D' | 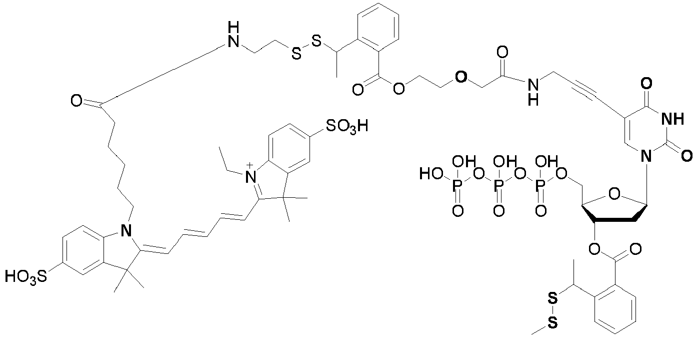 |
| 22A' | 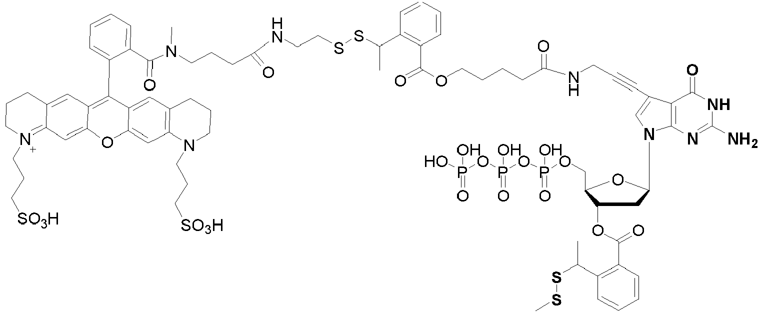 |
| 22B' | 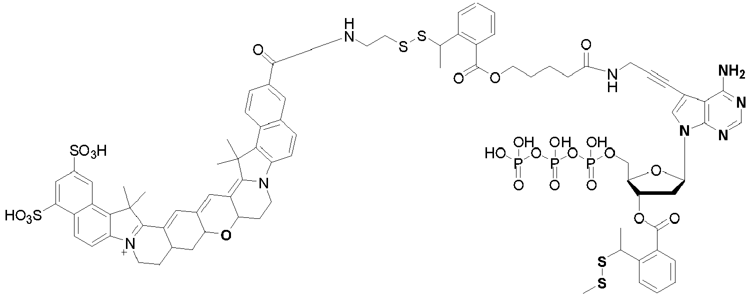 |
| 22C' | 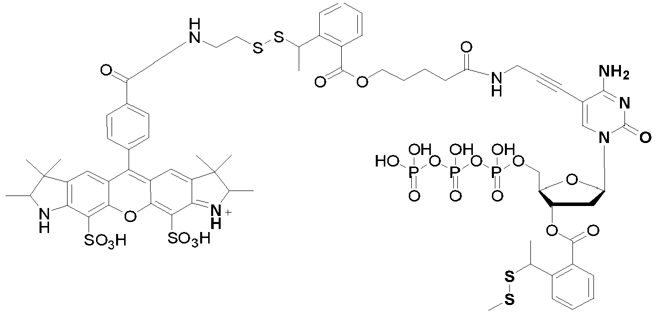 |
| 22D' | 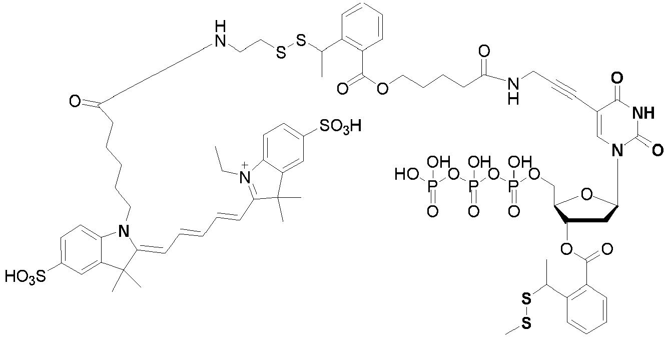 |
| 23A' | 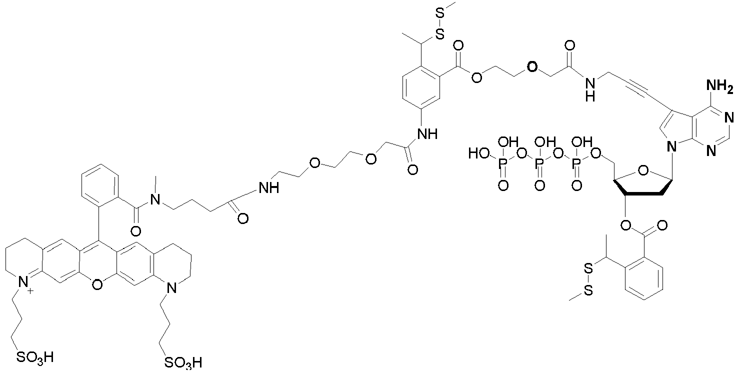 |
| 23B' | 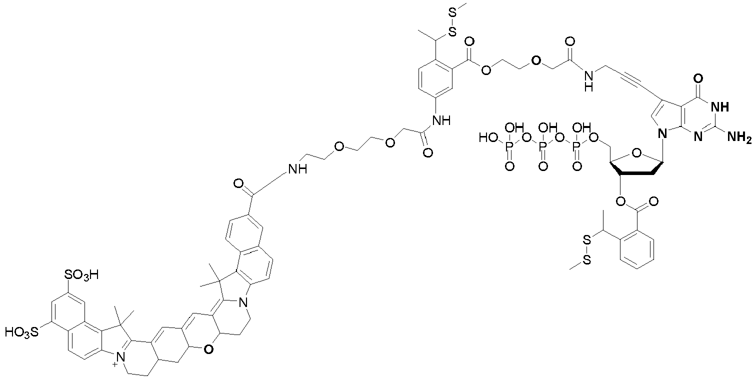 |
| 23B" | 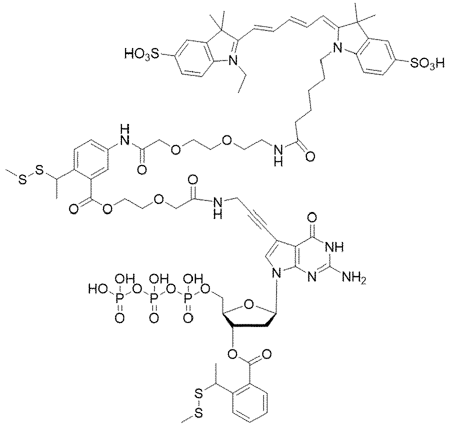 |
| 23C' | 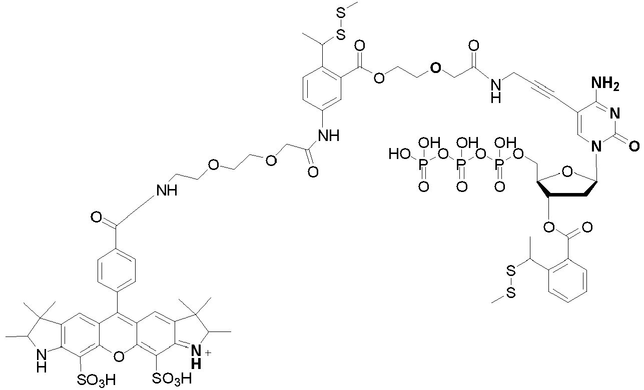 |
| 23D' | 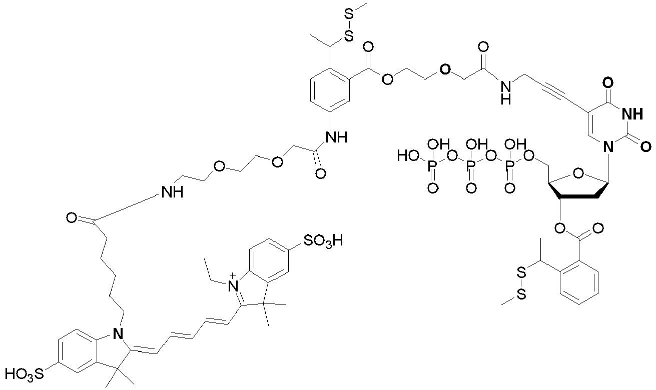 |
| 24A' | 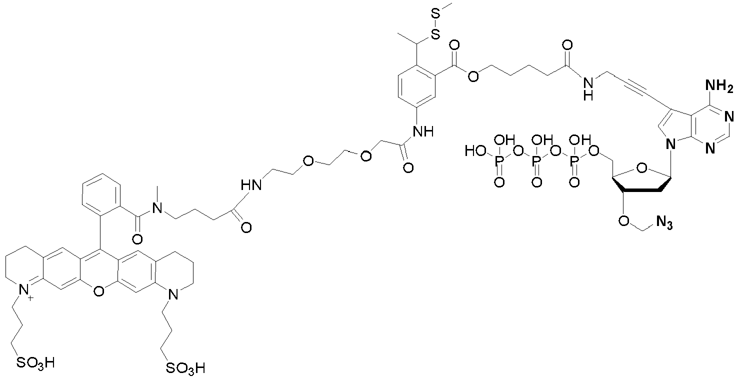 |
| 24B' | 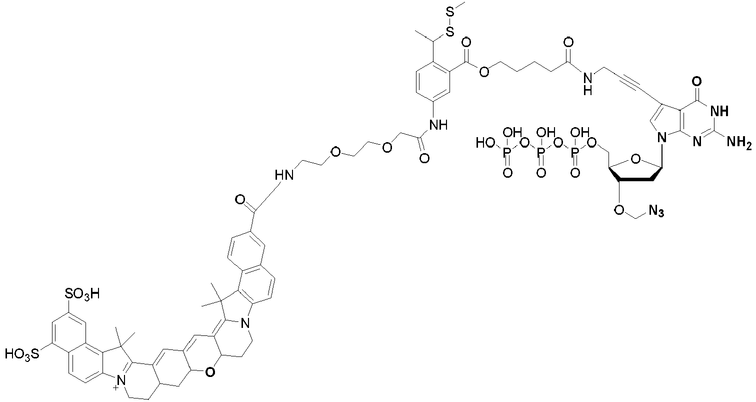 |
| 24C' | 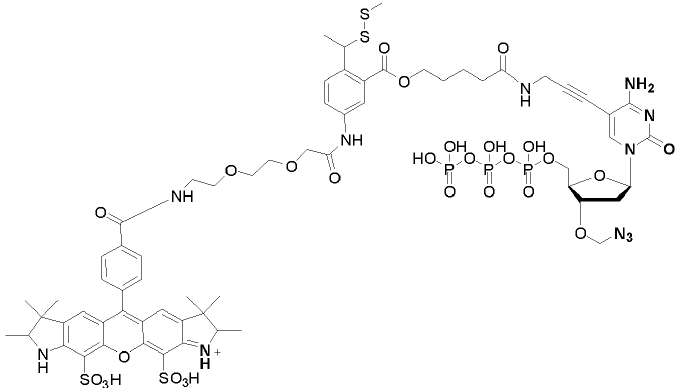 |
| 24D' | 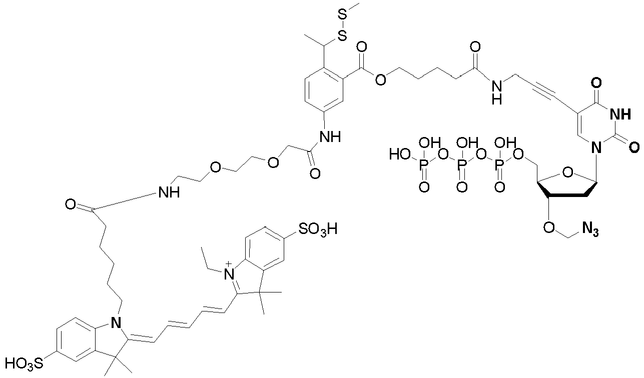 |
| 25A' | 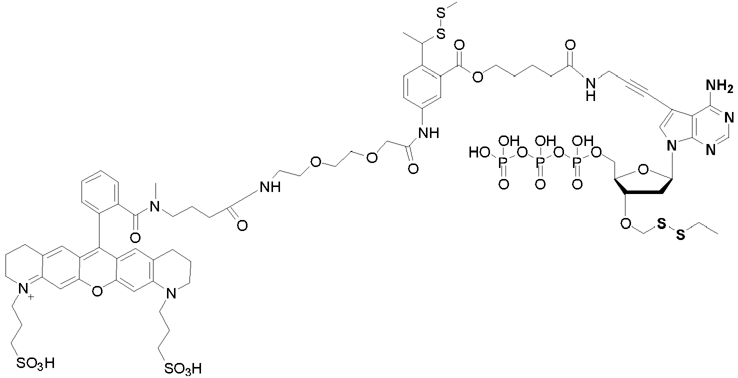 |
| 25B' | 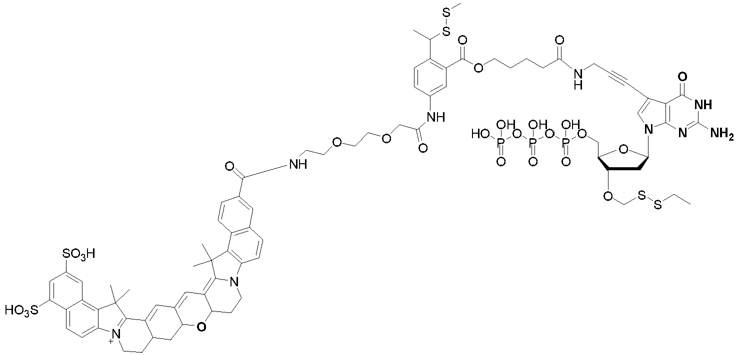 |
| 25C' | 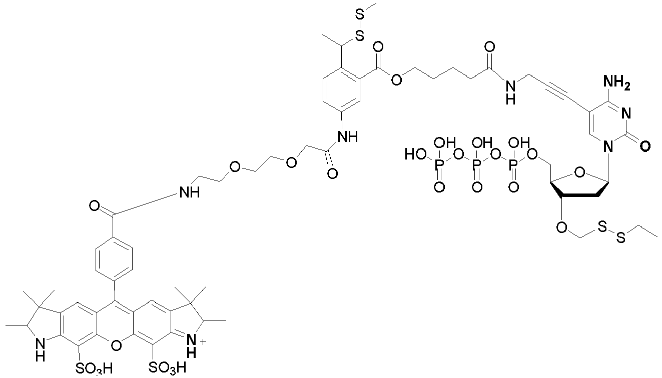 |
| 25D' | 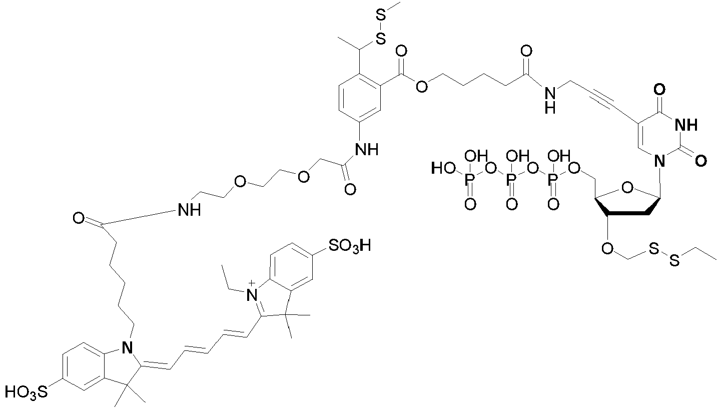 |
| 26A' | 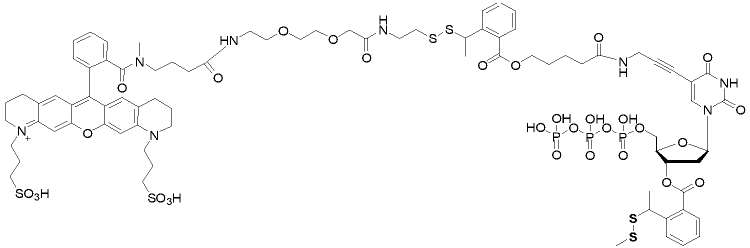 |
| 26B' | 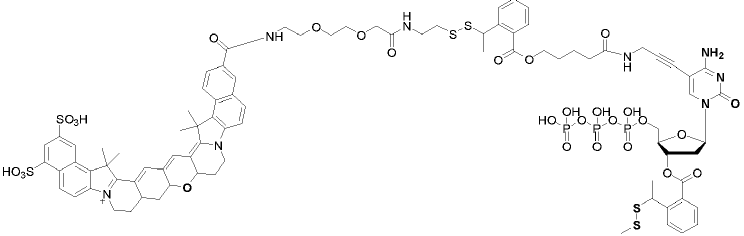 |
| 26C' | 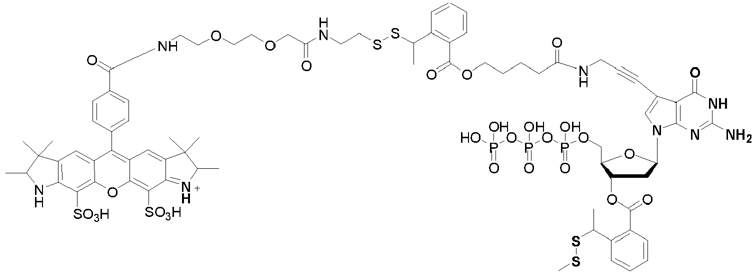 |
| 26D' | 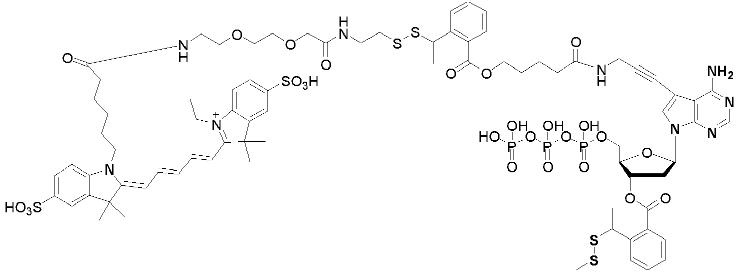 |
| 27A' | 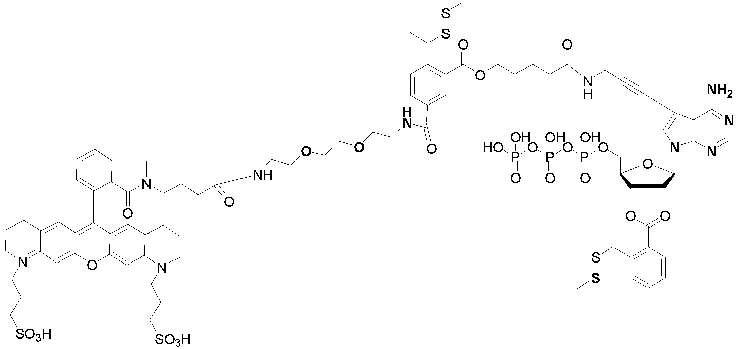 |
| 27B' | 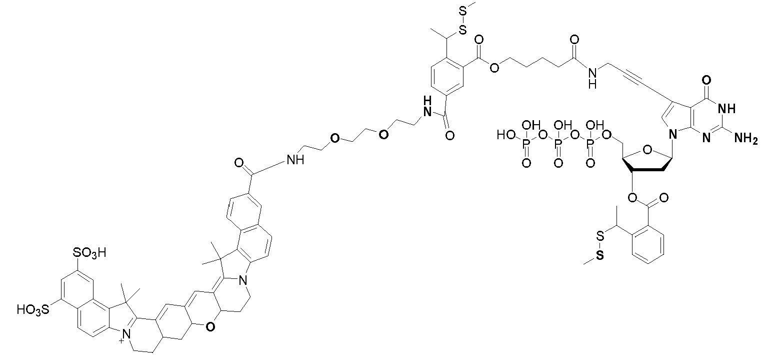 |
| 27C' | 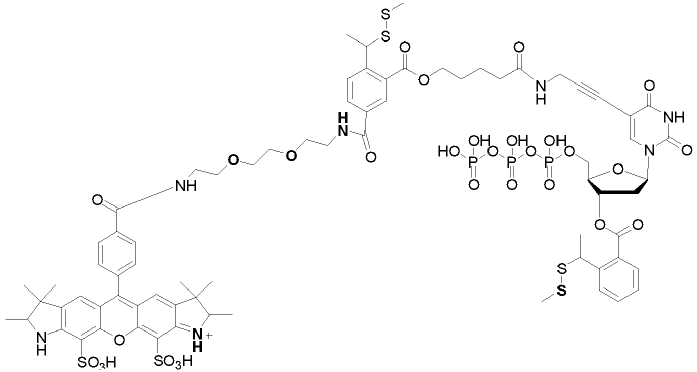 |
| 27D' | 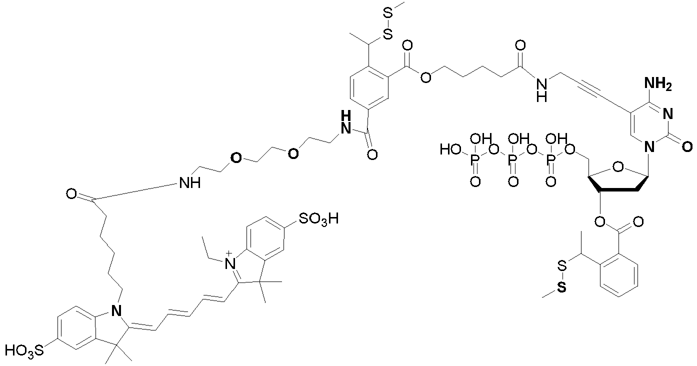 |
| 28A' | 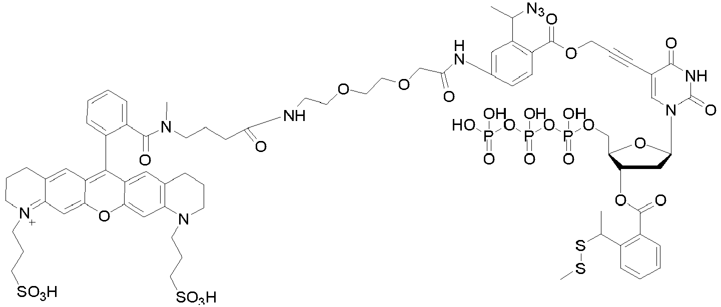 |
| 28B' | 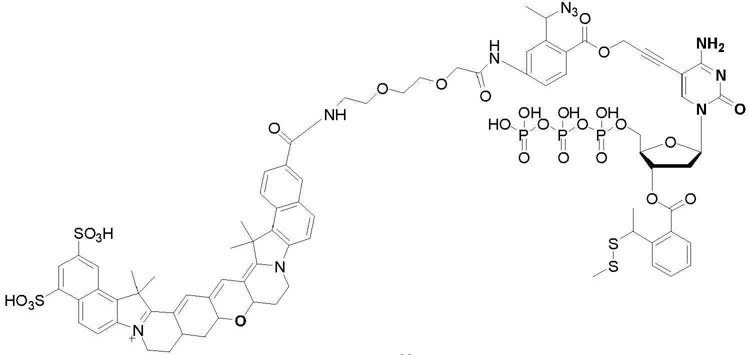 |
| 28C' | 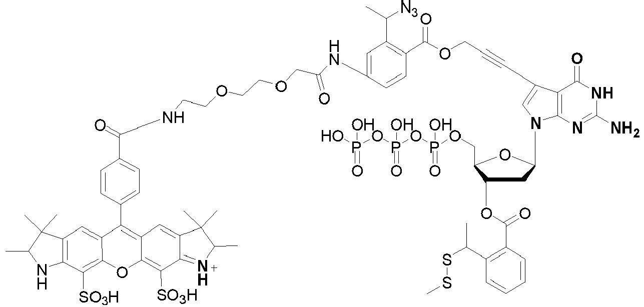 |
| 28D' | 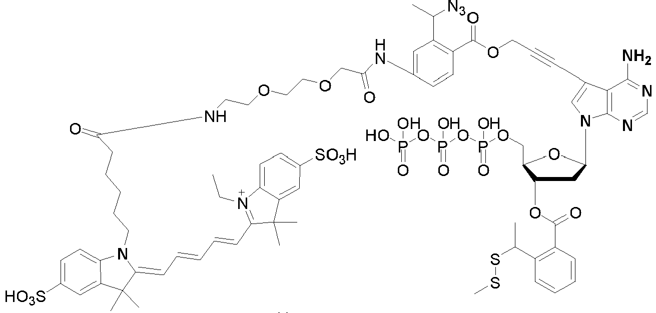 |
| 29A' | 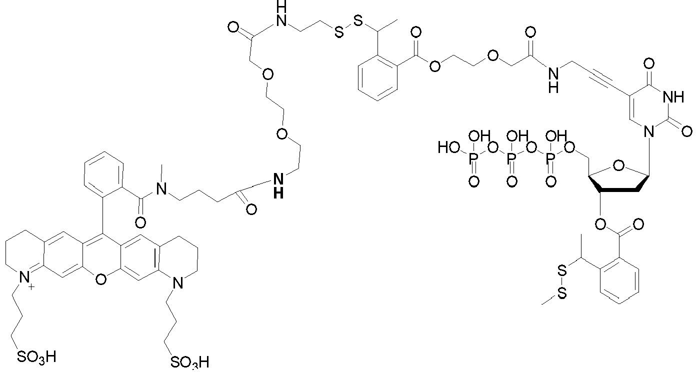 |
| 29B' | 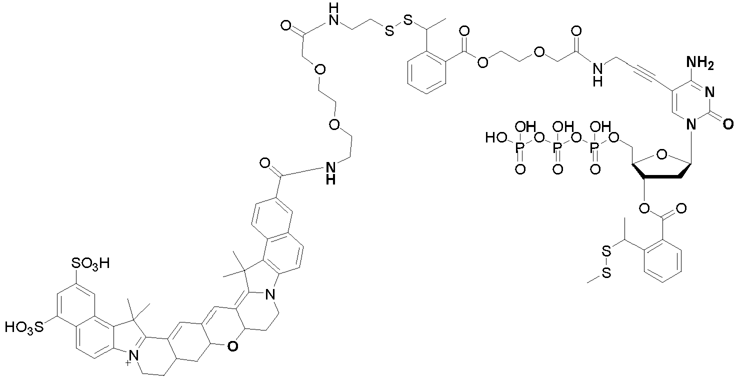 |
| 29C' | 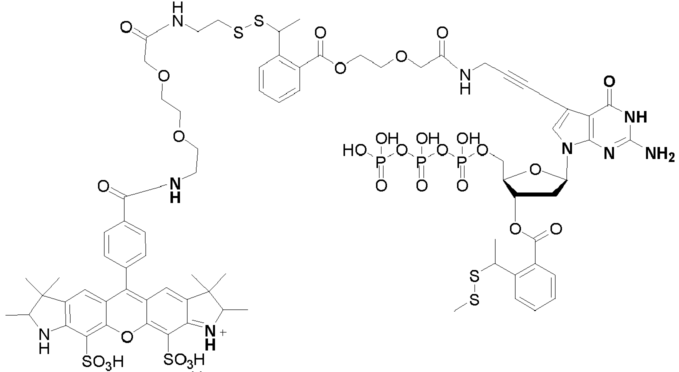 |
| 29D' | 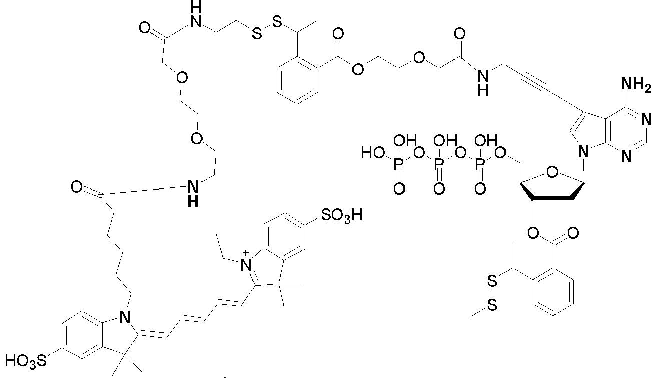 |
| 30A' | 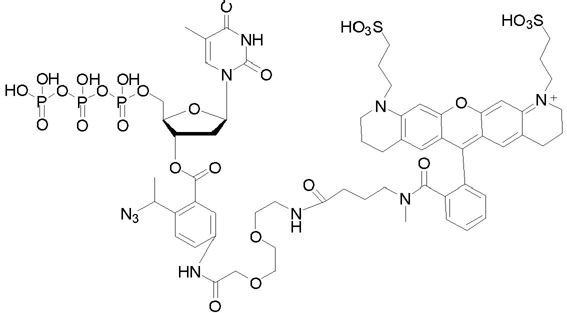 |
| 30B' | 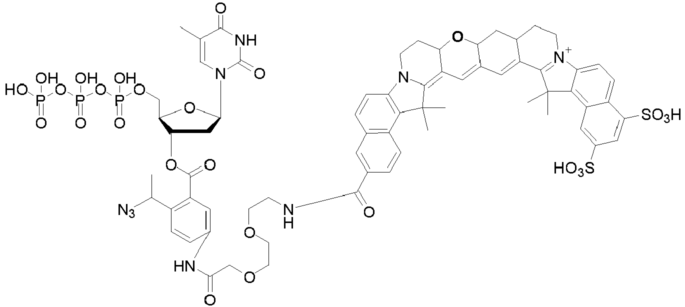 |
| 30C' | 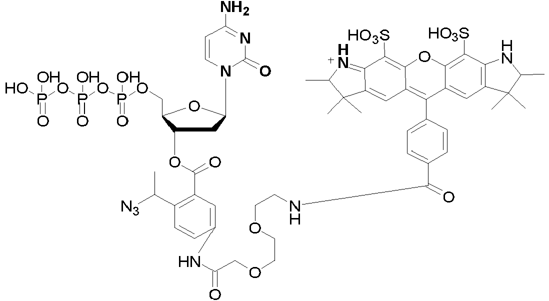 |
| 30D' | 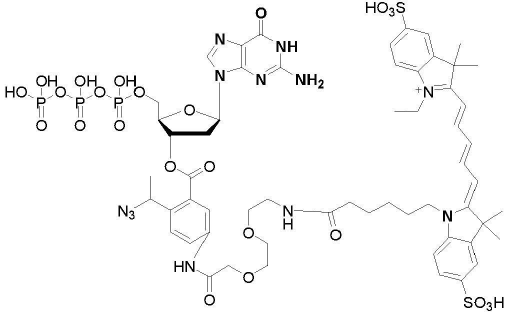 |
| 31A' | 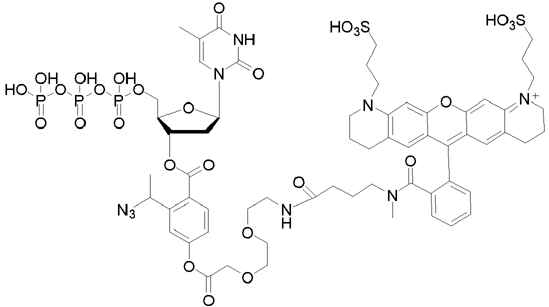 |
| 31B' | 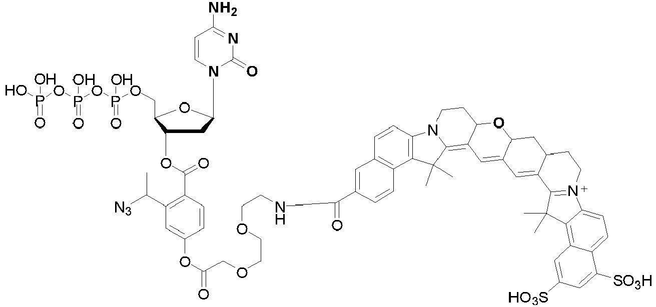 |
| 31C' | 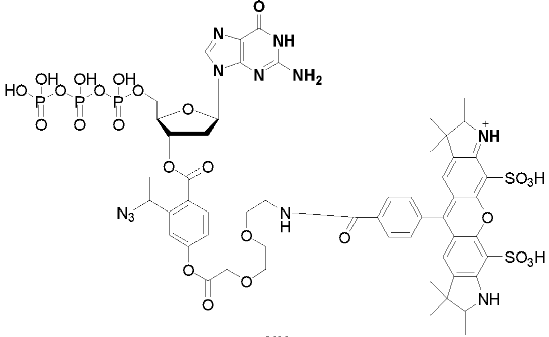 |
| 31D' | 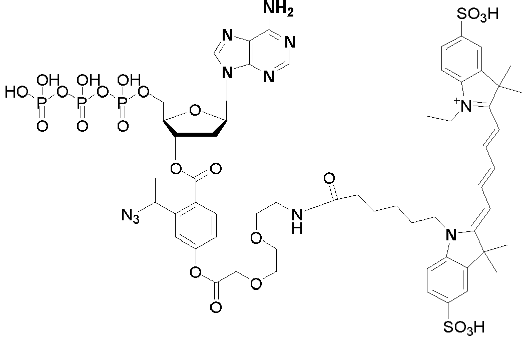 |
| 32A' | 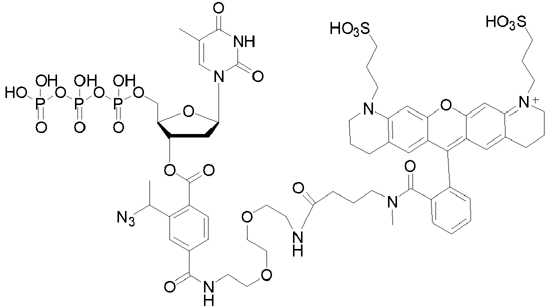 |
| 32B' | 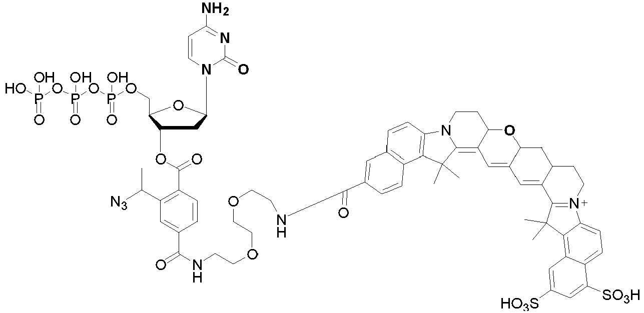 |
| 32C' | 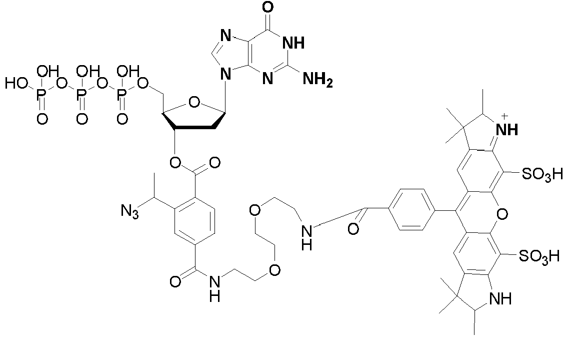 |
| 32D' | 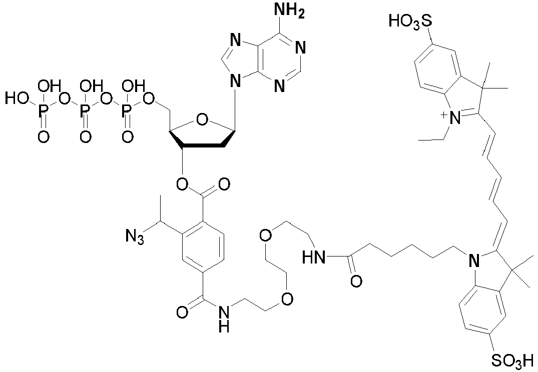 |
| 33A' | 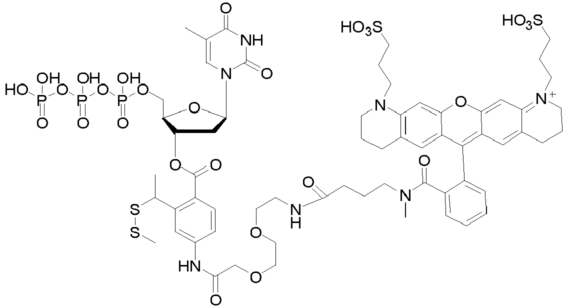 |
| 33B' | 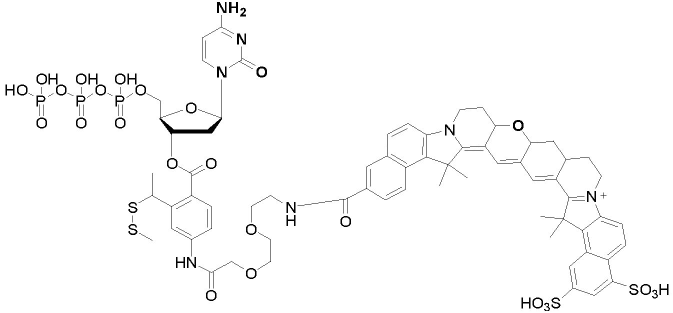 |
| 33C' | 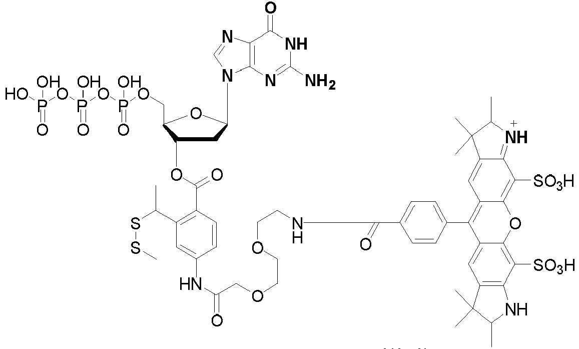 |
| 33D' | 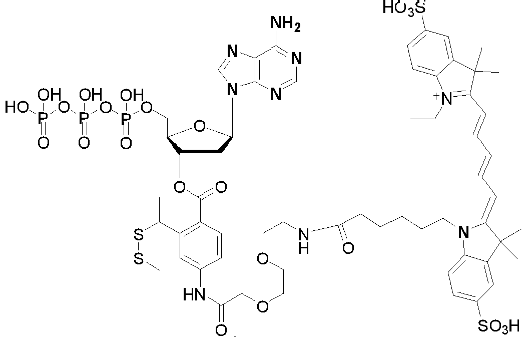 |
| 34A' | 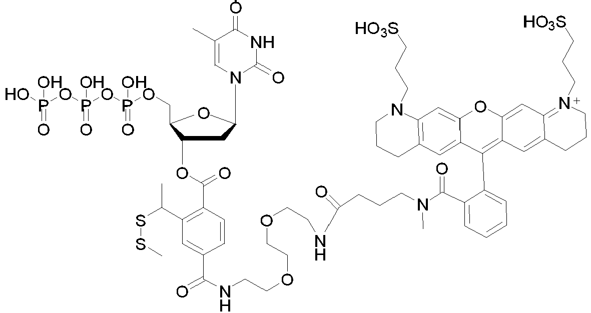 |
| 34B' | 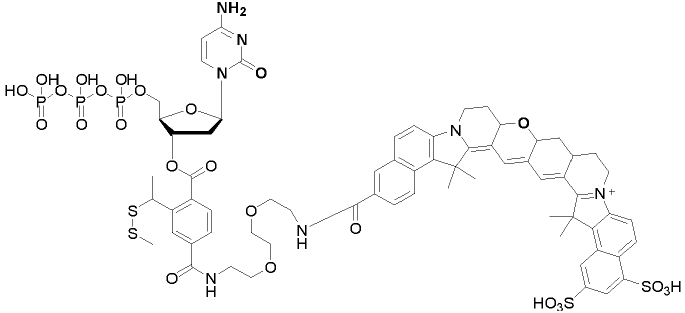 |
| 34C' | 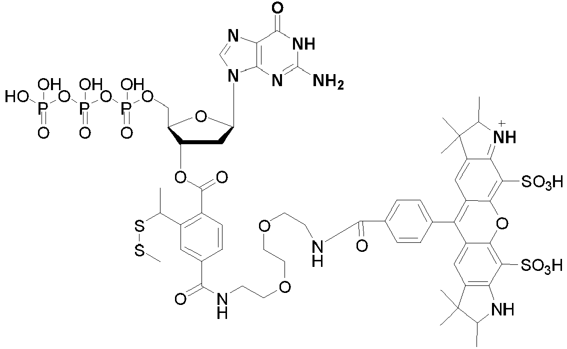 |
| 34D' | 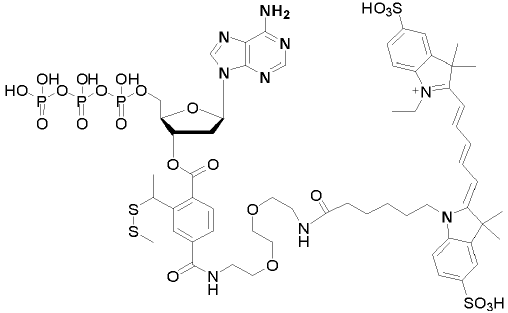 |
| 35A' | 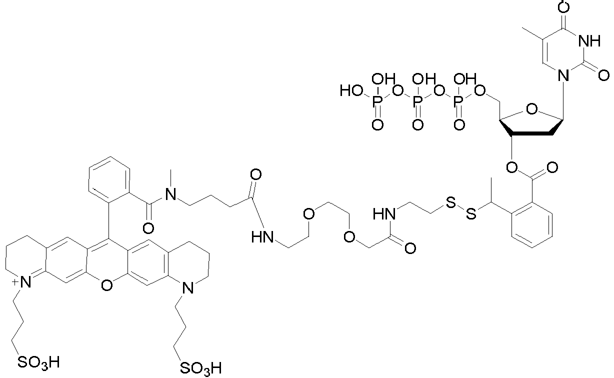 |
| 35B' | 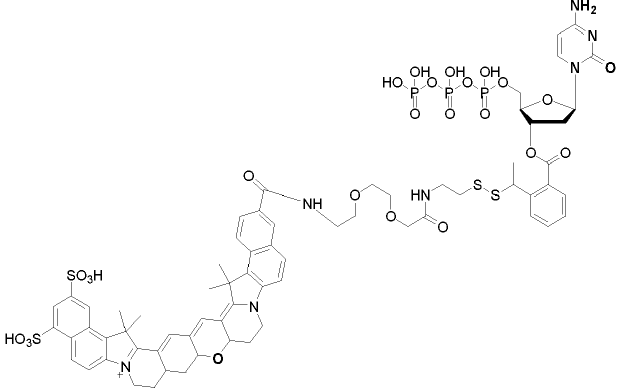 |
| 35C' | 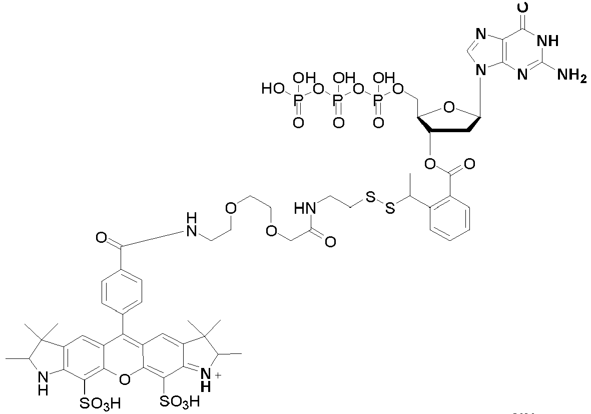 |
| 35D' | 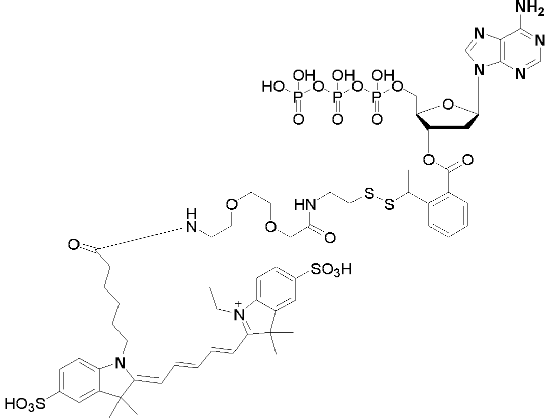 |
| | 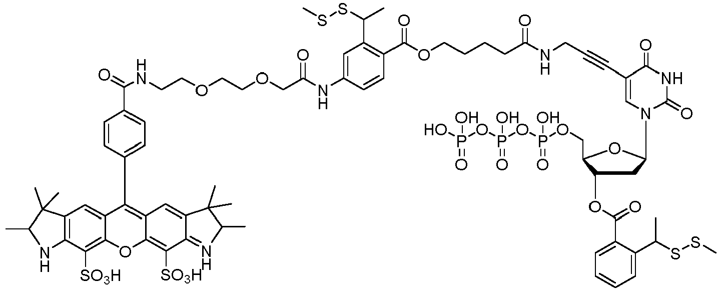 |
| | 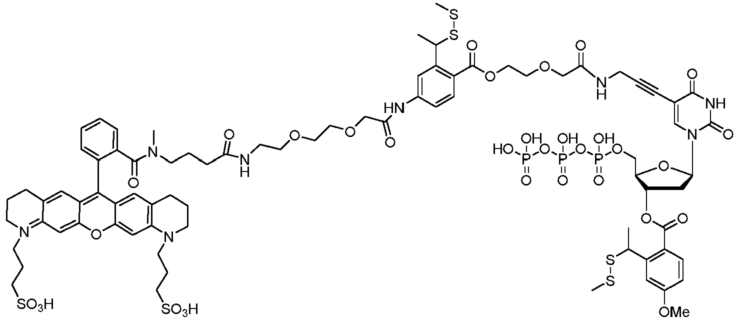 |
| | 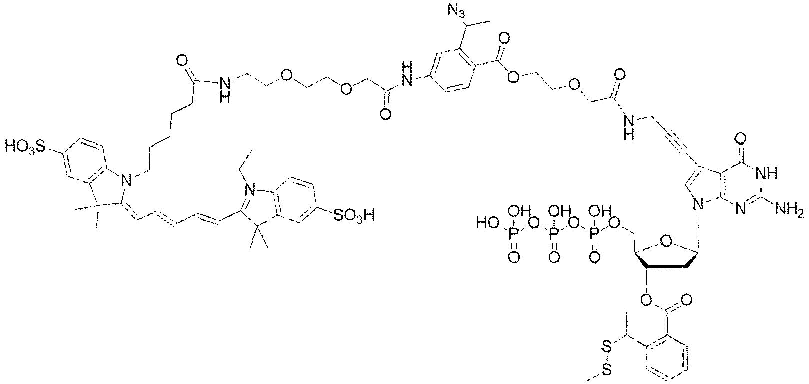 |
| | 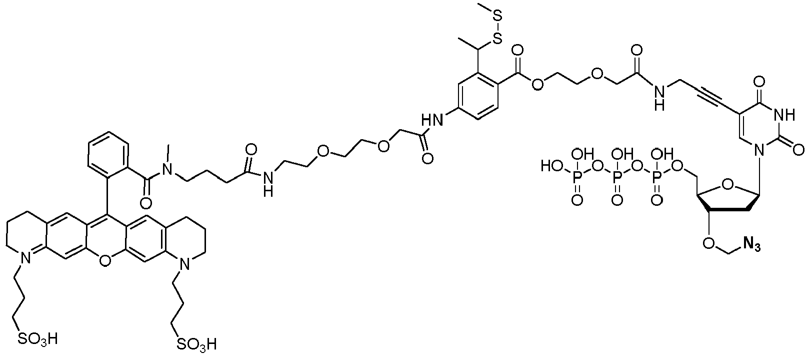 |
| | 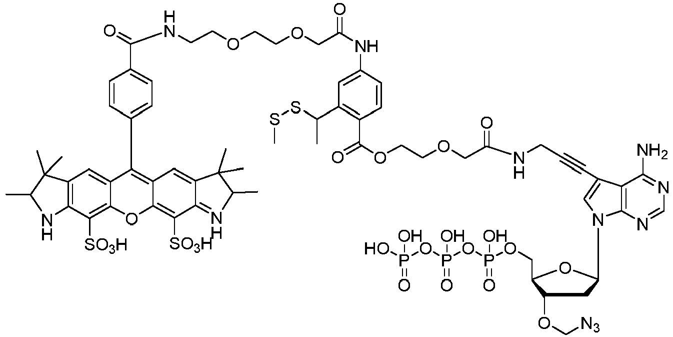 |
| | 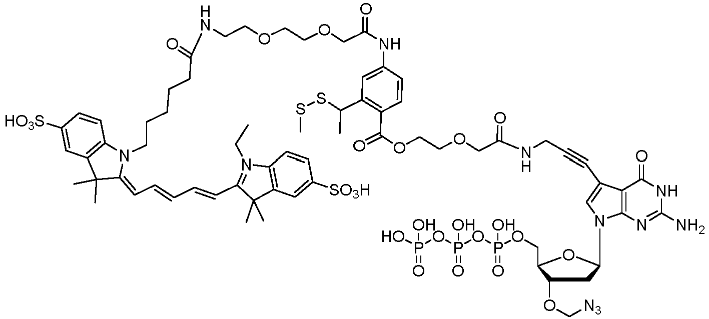 |
| | 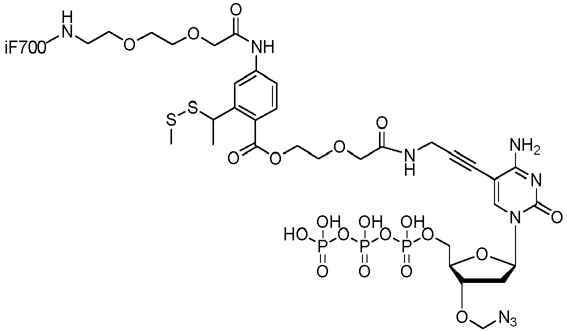 |
| | 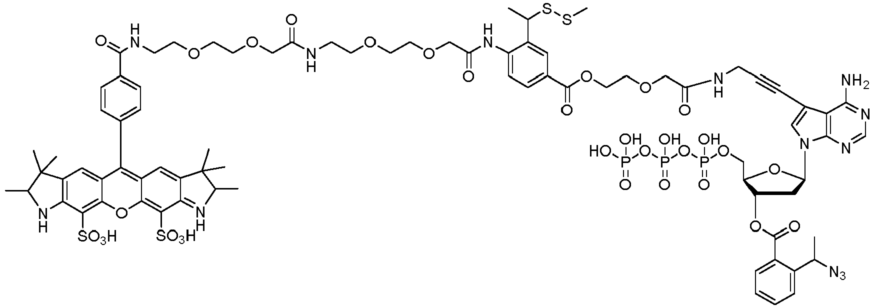 |
| | 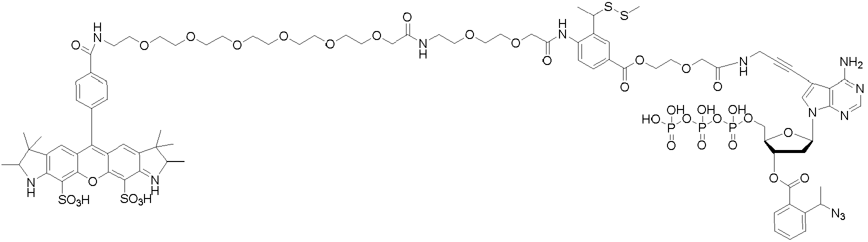 |
| | 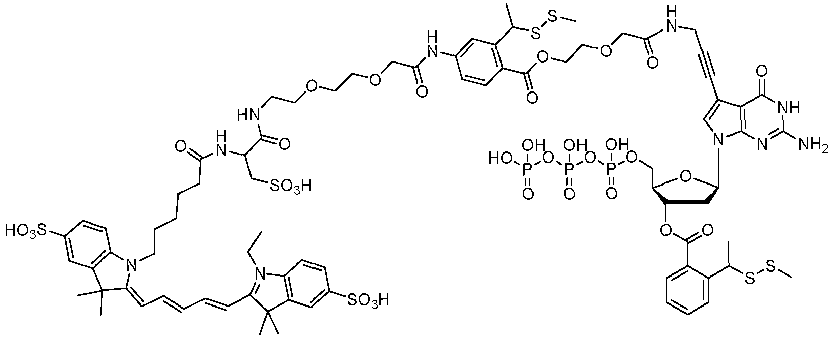 |
| | 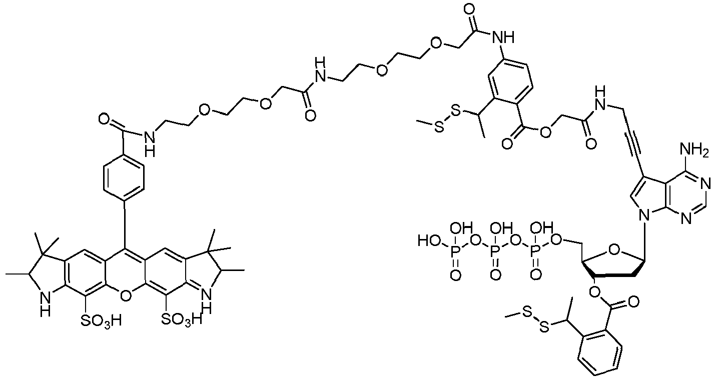 |
| | 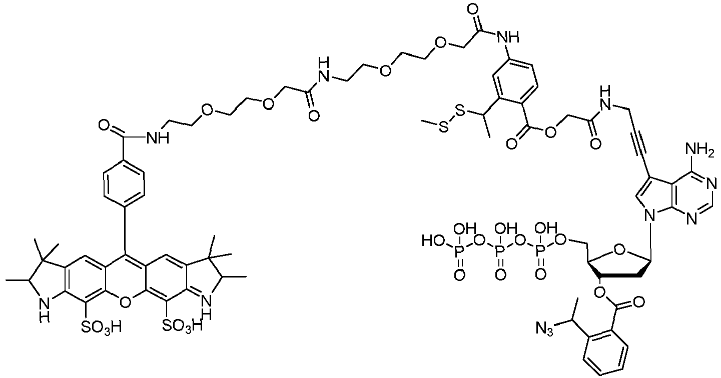 |
| | 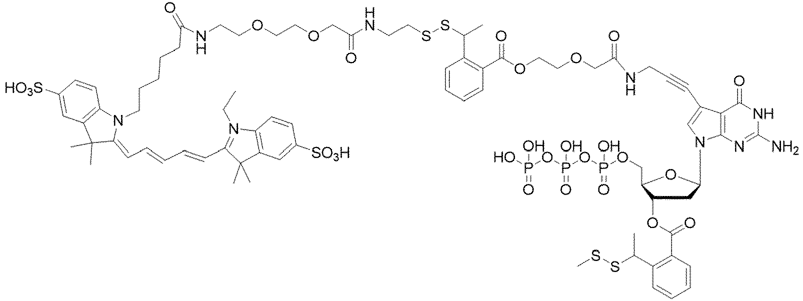 |
| | 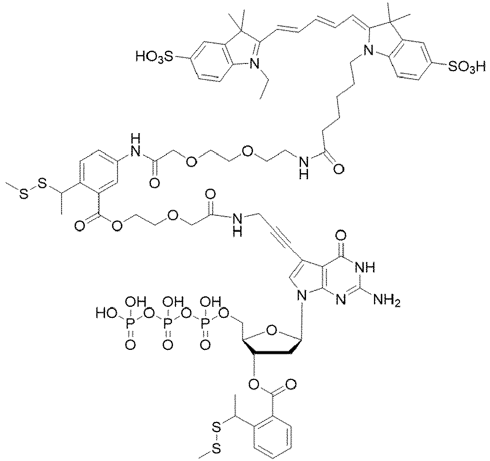 |
| | 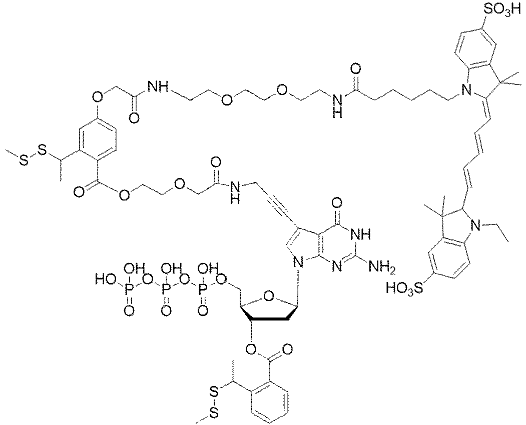 |
| | 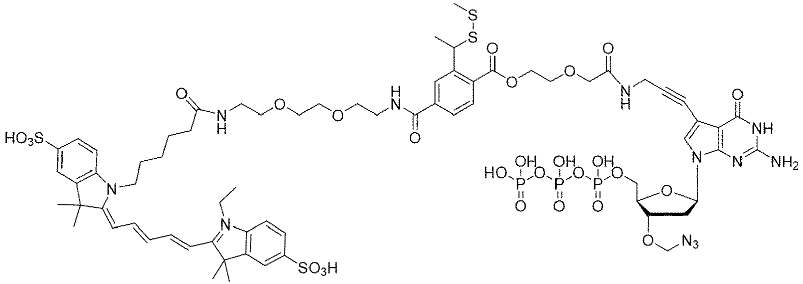 |

In the seventh aspect of the invention, the present invention provides a method for terminating a nucleic acid synthesis, which comprises incorporating the aforementioned compound or a salt thereof into a nucleic acid molecule to be terminated.

In some embodiments, the incorporation of the compound or a salt thereof is achieved by a terminal transferase, a terminal polymerase or a reverse transcriptase.

In some embodiments, the method comprises incorporating the compound or a salt thereof into the nucleic acid molecule to be terminated by using a polymerase.

In some embodiments, the method comprises performing a nucleotide polymerization reaction using a polymerase under conditions where the polymerase is allowed to carry out the nucleotide polymerization reaction, thereby incorporating the compound or a salt thereof into the 3' end of the nucleic acid molecule to be terminated.

In the eighth aspect of the invention, the present invention provides a method for preparing a growing polynucleotide complementary to a target single-stranded polynucleotide in a sequencing reaction, the method comprises incorporating the aforementioned compound or a salt thereof into the growing complementary polynucleotide, wherein the incorporation of the compound or a salt thereof prevents any subsequent nucleotides from being introduced into the growing complementary polynucleotide.

In some embodiments, the incorporation of the compound or a salt thereof is achieved by a terminal transferase, a terminal polymerase or a reverse transcriptase.

In some embodiments, the method comprises incorporating the compound or a salt thereof into the growing complementary polynucleotide by using a polymerase.

In some embodiments, the method comprises performing a nucleotide polymerization reaction using a polymerase under conditions where the polymerase is allowed to carry out the nucleotide polymerization reaction, thereby incorporating the compound or a salt thereof into the 3' end of the growing complementary polynucleotide.

In the ninth aspect of the invention, the present invention provides a nucleic acid intermediate, which is formed in determining the sequence of a target single-stranded polynucleotide, wherein the nucleic acid intermediate is formed according to the step:
incorporating a nucleotide complementary to the target single-stranded polynucleotide into a growing nucleic acid strand, to form the nucleic acid intermediate, wherein, the incorporated complementary nucleotide is the aforementioned compound or a salt thereof.

In the tenth aspect of the invention, the present invention provides a nucleic acid intermediate, which is formed in determining the sequence of a target single-stranded polynucleotide, wherein the nucleic acid intermediate is formed according to the step:
incorporating a nucleotide complementary to the target single-stranded polynucleotide into a growing nucleic acid strand, to form the nucleic acid intermediate, wherein, the incorporated complementary nucleotide is the aforementioned compound or a salt thereof, and the growing nucleic acid strand is pre-incorporated with at least one nucleotide complementary to the target single-stranded polynucleotide, the at least one pre-incorporated nucleotide complementary to the target single-stranded polynucleotide being the aforementioned compound or a salt thereof in which the reversible blocking group and optional detectable label have been removed.

In the eleventh aspect of the invention, the present invention provides a method for determining the sequence of a target single-stranded polynucleotide, which comprises:
1) monitoring the incorporation of nucleotides complementary to the target single-stranded polynucleotide in a growing nucleic acid strand, wherein at least one complementary nucleotide incorporated is the aforementioned compound or a salt thereof, and,
2) determining the types of the incorporated nucleotides.

In some embodiments, the reversible blocking group and optional detectable label are removed before a next complementary nucleotide is introduced.

In some embodiments, the reversible blocking group and the detectable label are removed simultaneously.

In some embodiments, the reversible blocking group and the detectable label are removed sequentially; for example, after the detectable label is removed, the reversible blocking group is removed, or, after the reversible blocking group is removed, the detectable label is removed.

In some embodiments, the method for determining the sequence of a target single-stranded polynucleotide comprises the following steps:
(a) providing a plurality of different nucleotides, wherein at least one nucleotide is the aforementioned compound or a salt thereof, optionally, the rest of nucleotides are the aforementioned compound or a salt thereof;
(b) incorporating the plurality of different nucleotides into a sequence complementary to a target single-stranded polynucleotide, wherein the plurality of different nucleotides can be distinguished from each other during the detection;
(c) detecting the nucleotides in step (b) to determine the types of nucleotides incorporated;
(d) removing the reversible blocking groups and optional detectable labels carried by the nucleotides in step (b); and
(e) optionally repeating steps (a)-(d) one or more times;
whereby the sequence of the target single-stranded polynucleotide is determined.

In some embodiments, the method for determining the sequence of a target single-stranded polynucleotide comprises the following steps:
(1) providing a first nucleotide, a second nucleotide, a third nucleotide and a fourth nucleotide, wherein at least one of the four nucleotides is the aforementioned compound or a salt thereof, optionally, the rest of nucleotides are the aforementioned compound or a salt thereof;
(2) contacting the four nucleotides with a target single-stranded polynucleotide; removing the nucleotides not incorporated into the growing nucleic acid strand; detecting the nucleotides incorporated into the growing nucleic acid strand; removing the reversible blocking groups and optional detectable labels carried in the nucleotides that are incorporated into the growing nucleic acid strand;
optionally, it also includes (3): repeating the steps (1)-(2) one or more times.

In some embodiments, the method for determining the sequence of a target single-stranded polynucleotide comprises the following steps:
(a) providing a mixture comprising a duplex, nucleotides comprising at least one aforementioned compound or a salt thereof, a polymerase, and an excision reagent; wherein the duplex comprises a growing nucleic acid strand and a nucleic acid strand to be sequenced;
(b) carrying out a reaction comprising the following steps (i), (ii) and (iii), optionally, repeating the steps for one or more times:
   step (i): incorporating the compound or a salt thereof into the growing nucleic acid strand by using a polymerase, to form a nucleic acid intermediate containing the reversible blocking group and optional detectable label;
   step (ii): detecting the nucleic acid intermediate;
   step (iii): removing the reversible blocking group and the optional detectable label contained in the nucleic acid intermediate by using the excision reagent.

In some embodiments, removal of the reversible blocking group and removal of the detectable label are performed simultaneously, or, removal of the reversible blocking group and removal of the detectable label are performed sequentially (for example, the reversible blocking group is removed first, or the detectable label is removed first).

In some embodiments, the excision reagent used for removal of the reversible blocking group is the same as that used for removal of the detectable label.

In some embodiments, the excision reagent used for removal of the reversible blocking group is different from that used for removal of the detectable label.

In some embodiments, the duplex is linked to a support.

In some embodiments, the growing nucleic acid strand is a primer.

In some embodiments, the primer is annealed to the nucleic acid strand to be sequenced to form the duplex.

In some embodiments, the duplex, the compound or a salt thereof, and the polymerase together form a reaction system containing a solution phase and a solid phase.

In some embodiments, the compound or a salt thereof is incorporated into the growing nucleic acid strand by using a polymerase under conditions where the polymerase is allowed to carry out a nucleotide polymerization reaction, thereby forming a nucleic acid intermediate containing the reversible blocking group and optional detectable label.

In some embodiments, the polymerase is selected from KOD polymerase or a mutant thereof (e.g., KOD POL151, KOD POL157, KOD POL171, KOD POL174, KOD POL376, KOD POL391).

In some embodiments, before any step of detecting the nucleic acid intermediate, the solution phase of the reaction system in the previous step is removed, and the duplex linked to the support is retained.

In some embodiments, the excision reagent is in contact with the duplex or the growing nucleic acid strand in the reaction system containing a solution phase and a solid phase.

In some embodiments, the excision reagent can remove the reversible blocking group and optional detectable label carried by the compound that is incorporated into the growing nucleic acid strand, without affecting the phosphodiester bond on the backbone of the duplex.

In some embodiments, after any step of removing the reversible blocking group and optional detectable label contained in the nucleic acid intermediate, the solution phase of the reaction system in this step is removed.

In some embodiments, a washing operation is performed after any step comprising a removal operation.

In some embodiments, after step (ii), the method further comprises: determining the type of compound incorporated into the growing nucleic acid strand in step (i) according to the signal detected in step (ii), and determining the type of nucleotide at a corresponding position in the nucleic acid strand to be sequenced based on the principle of base complementary pairing.

In the twelfth aspect of the invention, the present invention provides a kit, which comprises at least one aforementioned compound or a salt thereof.

In some embodiments, the kit comprises a first compound, a second compound, a third compound and a fourth compound, wherein the first, second, third and fourth compounds each are, independently, the aforementioned compound or a salt thereof.

In some embodiments, in the first compound, base¹ is selected from adenine, 7-deazaadenine or a tautomer thereof (e.g., 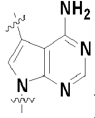 ); in the second compound, base¹ is selected from thymine, uracil or a tautomer thereof (e.g., 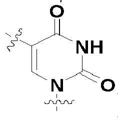 ); in the third compound, base¹ is selected from cytosine or a tautomer thereof (e.g., 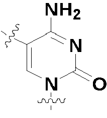 ); in the fourth compound, base¹ is selected from guanine, 7-deazaguanine or a tautomer thereof (e.g., 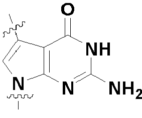 ).

In some embodiments, in the first compound, base² is selected from adenine, 7-deazaadenine or a tautomer thereof (e.g., 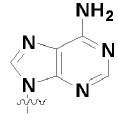 ); in the second compound, base² is selected from thymine, uracil or a tautomer thereof (e.g., 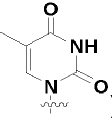 ); in the third compound, base² is selected from cytosine or a tautomer thereof (e.g., 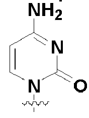 ); in the fourth compound, base² is selected from guanine, 7-deazaquanine or a tautomer thereof (e.g., 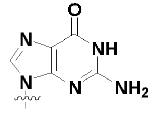 ).

In some embodiments, wherein the base¹ or base² contained in the first, second, third and fourth compounds are different from each other.

In some embodiments, wherein the additional detectable labels carried by the first, second, third and fourth compounds are different from each other.

In some embodiments, the kit further comprises: a reagent for pretreating the nucleic acid molecule; a support for linking nucleic acid molecule to be sequenced; a reagent for linking (for example, covalently or non-covalently linking) the nucleic acid molecule to be sequenced to the support; primers for initiating a nucleotide polymerization reaction; a polymerase for carrying out the nucleotide polymerization reaction; one or more buffer solutions; one or more washing solutions; or any combination thereof.

In the thirteenth aspect of the invention, the present invention provides the use of the aforementioned compound or a salt thereof or the aforementioned kit for determining the sequence of a target single-stranded polynucleotide.

Furthermore, in the fourteenth aspect of the invention, the present invention further provides a compound of formula I-1 or a salt thereof, 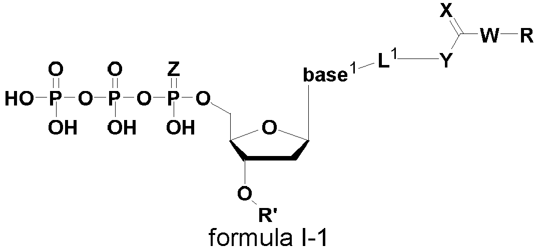 wherein:
L¹ is selected from 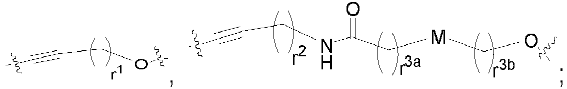 r¹, r², r^{3a}, r^{3b}, independently of each other, are selected from any integer between 1 and 6;
   M is selected from a direct bond, CH₂, NH, O, S;
X is selected from O, S, NH;
Y is selected from a direct bond, O, S, NH;
W is selected from a direct bond, O, S, NH;
R is 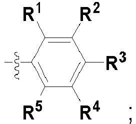
   in R¹, R², R³, R⁴, R⁵, any one (e.g., R², R³ or R⁴) is 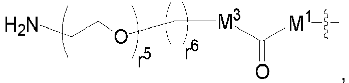 and the others, independently of each other, are selected from H, 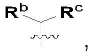 azido, nitro, amino, sulfo, carboxyl, aliphatic alkyl (e.g., C₁-C₆ alkyl), cycloalkyl (e.g., C₃-C₆ cycloalkyl), aromatic alkyl (e.g., phenyl -C₁-C₆ alkyl), F, I, Br, Cl, alkoxyl (e.g., C₁-C₆ alkoxyl);
   r⁵, r⁶, independently of each other, are selected from any integer between 1 and 6;
   M¹, M³, independently of each other, are selected from a direct bond, NH, O, S;
   in R^{b}, R^{c}, any one is selected from -N₃, -SS-C₁-C₆ alkyl (e.g., -SS-Me, -SS-Et, -SS-iPr, -SS-t-Bu), -ONH₂, -OCORₓ, -OCONHRₓ, and the other is selected from H, aliphatic alkyl (e.g., C₁-C₆ alkyl, such as Me, Et, iPr, tBu), aromatic alkyl (e.g., phenyl -C₁-C₆ alkyl), cycloalkyl (e.g., C₃-C₆ cycloalkyl), wherein each Rₓ, independently, is selected from aliphatic alkyl (e.g., C₁-C₆ alkyl), cycloalkyl (e.g., C₃-C₆ cycloalkyl) or aromatic alkyl (e.g., phenyl C₁-C₆ alkyl);
Z is selected from O, S, BH;
base¹ is selected from a base, a deaza base or a tautomer thereof, for example, adenine, 7-deazaadenine, thymine, uracil, cytosine, guanine, 7-deazaguanine or a tautomer thereof;
R' represents a reversible blocking group.

In some embodiments, L¹ is selected from

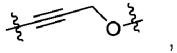

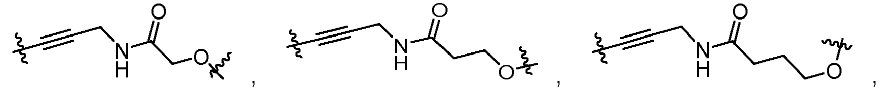

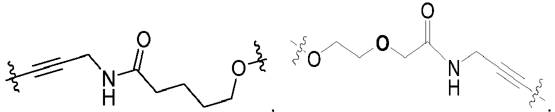

In some embodiments, L¹ is selected from

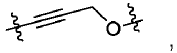

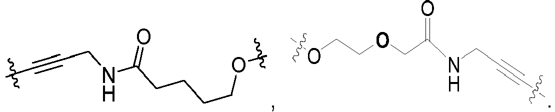

In some embodiments, r¹ is selected from 1, 2, 3.

In some embodiments, r¹ is 1.

In some embodiments, r² is selected from 1, 2, 3.

In some embodiments, r² is 1.

In some embodiments, r^{3a}, r^{3b}, independently of each other, are selected from 1, 2, 3, 4, 5.

In some embodiments, r^{3a}, r^{3b}, independently of each other, are selected from 1, 2, 3.

In some embodiments, r^{3a} is 1.

In some embodiments, r^{3b} is 2.

In some embodiments, M is selected from CH₂, O.

In some embodiments, X is selected from O, S.

In some embodiments, X is O.

In some embodiments, Y is a direct bond.

In some embodiments, W is a direct bond.

In some embodiments, in R¹, R², R³, R⁴, R⁵ any one (e.q., R², R³ or R⁴ is 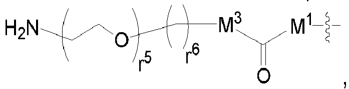 and the others, independently of each other, are selected from H

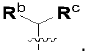

In some embodiments, in R¹, R², R³, R⁴, R⁵, any one (e.g., R², R³ or R⁴) is 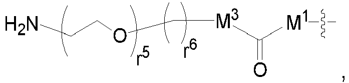 another (e.g., R¹ or R⁵) is 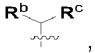 and the remaining three are H.

In some embodiments, in R¹, R², R³, R⁴, R⁵, R³ or R⁴ is 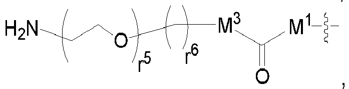 R¹ is 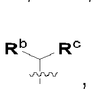 and the remaining three are H.

In some embodiments, R is selected from

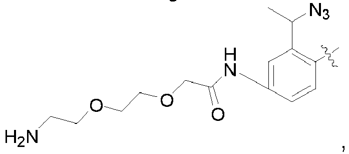

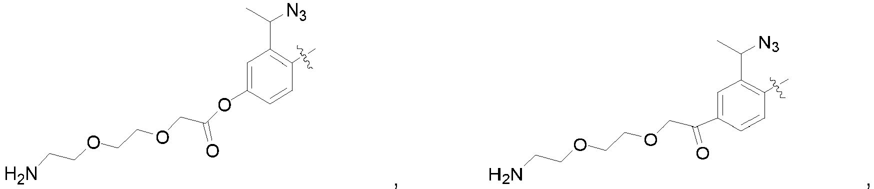

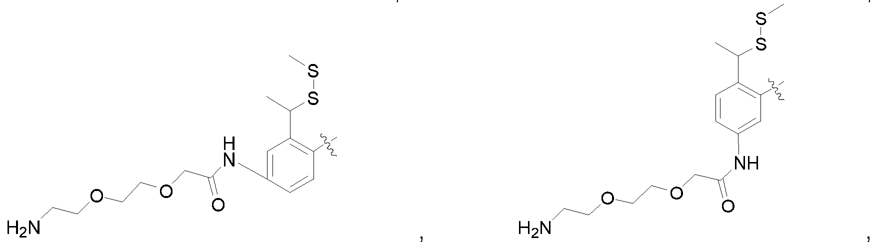

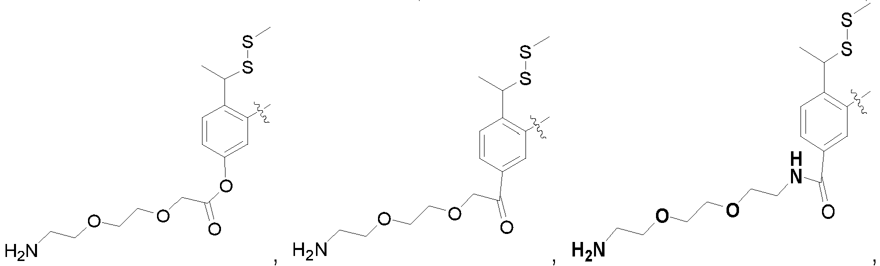

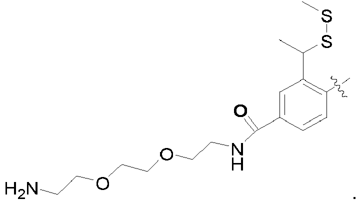

In some embodiments, r⁵ is selected from 1, 2, 3.

In some embodiments, r⁵ is 2.

In some embodiments, r⁶ is selected from 1, 2, 3.

In some embodiments, r⁶ is 1 or 2.

In some embodiments, M¹ is selected from a direct bond, NH, O.

In some embodiments, M³ is selected from a direct bond, NH.

In some embodiments, when M¹ is NH, O or S, M³ is a direct bond, and when M³ is NH, O or S, M¹ is a direct bond.

In some embodiments, in R^{b}, R^{c}, any one is selected from -N₃, -SS-C₁-C₆ alkyl (e.g., -SS-Me, -SS-Et, -SS-iPr, -SS-t-Bu), and the other is selected from C₁-C₆ alkyl.

In some embodiments, in R^{b}, R^{c}, any one is selected from -N₃, -SS-Me, and the other is methyl.

In some embodiments, Z is O.

In some embodiments, base¹ is selected from

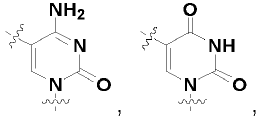

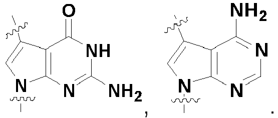

In some specific embodiments, in the compound of formula I-1:
L¹ is selected from 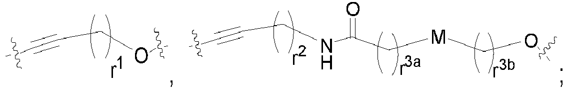
   r¹, r², r^{3a}, r^{3b}, independently of each other, are selected from any integer between 1 and 6;
   M is selected from a direct bond, CH₂, NH, O, S;
X is selected from O, S, NH;
Y is selected from a direct bond, O, S, NH;
W is selected from a direct bond, O, S, NH;
R is 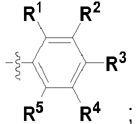
   in R¹, R², R³, R⁴, R⁵, any one (e.g., R², R³ or R⁴) is 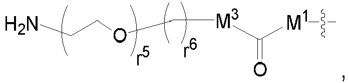 another (e.g., R¹ or R⁵) is 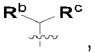 and the remaining three are H;
   r⁵, r⁶, independently of each other, are selected from any integer between 1 and 6;
   M¹, M³, independently of each other, are selected from a direct bond, NH, O, S;
   in R^{b}, R^{c}, any one is selected from -N₃, -SS-C₁-C₆ alkyl (e.g., -SS-Me, -SS-Et, -SS-iPr, -SS-t-Bu), and the other is selected from C₁-C₆ alkyl;
Z is selected from O, S, BH;
base¹ is selected from a base, a deaza base or a tautomer thereof, for example, adenine, 7-deazaadenine, thymine, uracil, cytosine, guanine, 7-deazaguanine or a tautomer thereof;
R' represents a reversible blocking group.

In some specific embodiments, L¹ is selected from

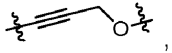

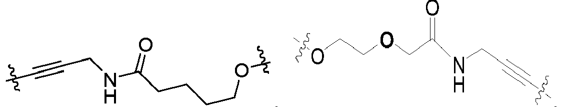

In some specific embodiments, r¹ is selected from 1, 2, 3.

In some specific embodiments, r¹ is 1.

In some specific embodiments, r² is selected from 1, 2, 3.

In some specific embodiments, r² is 1.

In some specific embodiments, r^{3a}, r^{3b}, independently of each other, are selected from 1, 2, 3, 4, 5.

In some specific embodiments, r^{3a}, r^{3b}, independently of each other, are selected from 1, 2, 3.

In some specific embodiments, r^{3a} is 1.

In some specific embodiments, r^{3b} is 2.

In some specific embodiments, M is selected from CH₂, O.

In some specific embodiments, X is selected from O, S.

In some specific embodiments, X is O.

In some specific embodiments, Y is a direct bond.

In some specific embodiments, W is a direct bond.

In some specific embodiments, in R¹, R², R³ R⁴, R⁵, R³ or R⁴ is 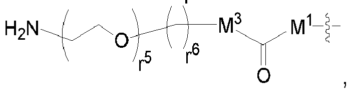 R¹ is 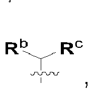 and the remaining three are H.

In some specific embodiments, r⁵ is selected from 1, 2, 3.

In some specific embodiments, r⁵ is 2.

In some specific embodiments, r⁶ is selected from 1, 2, 3.

In some specific embodiments, r⁶ is 1 or 2.

In some specific embodiments, M¹ is selected from a direct bond, NH, O.

In some specific embodiments, M³ is selected from a direct bond, NH.

In some specific embodiments, when M¹ is NH, O or S, M³ is a direct bond, and when M³ is NH, O or S, M¹ is a direct bond.

In some specific embodiments, in R^{b}, R^{c}, any one is selected from -N₃, - SS-Me, and the other is methyl.

In some specific embodiments, Z is O.

In some specific embodiments, base¹ is selected from

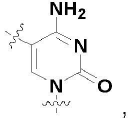

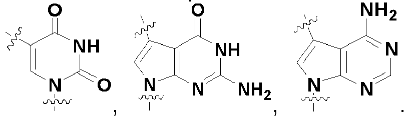

In some embodiments, the aforementioned reversible blocking group R' is selected from N₃-C₁-C₆ alkyl, C₁-C₆ alkyl-SS-C₁-C₆ alkyl, 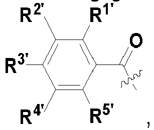 NH₂, - ONH₂, -OCOR_{z}, -OCONHR_{z}, wherein each R_{z}, independently, is selected from aliphatic alkyl (e.g., C₁-C₆ alkyl), cycloalkyl (e.g., C₃-C₆ cycloalkyl) or aromatic alkyl (e.g., phenyl C₁-C₆ alkyl).

In some embodiments, the reversible blocking group R' is selected from N₃-C₁-C₆ alkyl, C₁-C₆ alkyl-SS-C₁-C₆ alkyl,

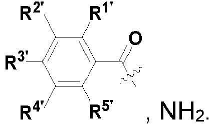

In some embodiments, the reversible blocking group R' is selected from N₃-C₁-C₆ alkyl, C₁-C₆ alkyl-SS-C₁-C₆ alkyl,

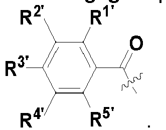

In some embodiments, the reversible blocking group R' is selected from N₃-CH₂-, CH₃-CH₂-S-S-CH₂-,

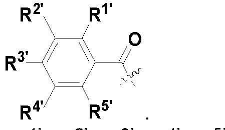

In some embodiments, in R^{1'}, R^{2'}, R^{3'}, R^{4'}, R^{5'}, any one (e.g., R^{1'} or R^{5'}) is 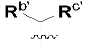 and the others, independently of each other, are selected from H, azido, nitro, amino, sulfo, carboxyl, aliphatic alkyl (e.g., C₁-C₆ alkyl), cycloalkyl (e.g., C₃-C₆ cycloalkyl), aromatic alkyl (e.g., phenyl -C₁-C₆ alkyl), F, I, Br, Cl, alkoxyl (e.g., C₁-C₆ alkoxyl).

In some embodiments, in R^{1'}, R^{2'}, R^{3'}, R^{4'}, R^{5'}, any one (e.g., R^{1'} or R^{5'}) is 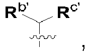 and the others are H.

In some embodiments, in R^{1'}, R^{2'}, R^{3'}, R^{4'}, R^{5'}, R^{1'} is are H. 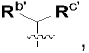 and the others

In some embodiments, in R^{b'}, R^{c'}, any one is selected from -N₃, -SS-C₁-C₆ alkyl (e.g., -SS-Me, -SS-Et, -SS-iPr, -SS-t-Bu), -ONH₂, -OCORₓ, -OCONHRₓ, and the other is selected from H, aliphatic alkyl (e.g., C₁-C₆ alkyl, such as Me, Et, iPr, tBu), aromatic alkyl (e.g., phenyl -C₁-C₆ alkyl), cycloalkyl (e.g., C₃-C₆ cycloalkyl), wherein each Rₓ, independently, is selected from aliphatic alkyl (e.g., C₁-C₆ alkyl), cycloalkyl (e.g., C₃-C₆ cycloalkyl) or aromatic alkyl (e.g., phenyl C₁-C₆ alkyl).

In some embodiments, in R^{b'}, R^{c'}, any one is selected from -N₃, -SS-C₁-C₆ alkyl (e.g., -SS-Me, -SS-Et, -SS-iPr, -SS-t-Bu), and the other is selected from C₁-C₆ alkyl.

In some embodiments, in R^{b'}, R^{c'}, any one is selected from -N₃, -SS-Me, and the other is methyl.

In some specific embodiments, the present invention provides a compound of formula I-1 or a salt thereof 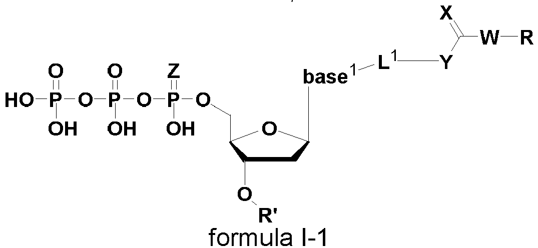 wherein:
L¹ is selected from 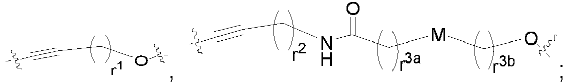 preferably, L¹ is selected from 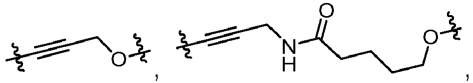 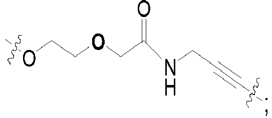
   r¹, r², r^{3a}, r^{3b}, independently of each other, are selected from any integer between 1 and 6;
   preferably, r' is selected from 1, 2, 3;
   more preferably, r¹ is 1;
   preferably, r² is selected from 1, 2, 3;
   more preferably, r² is 1;
   preferably, r^{3a}, r^{3b}, independently of each other, are selected from 1, 2, 3, 4, 5;
   more preferably, r^{3a}, r^{3b}, independently of each other, are selected from 1, 2, 3;
   most preferably, r^{3a} is 1;
   most preferably, r^{3b} is 2;
   M is selected from a direct bond, CH₂, NH, O, S;
   preferably, M is selected from CH₂, O;
   X is selected from O, S, NH;
   preferably, X is selected from O, S;
   more preferably, X is O;
   Y is selected from a direct bond, O, S, NH;
   preferably, Y is a direct bond;
   W is selected from a direct bond, O, S, NH;
   preferably, W is a direct bond;
R is 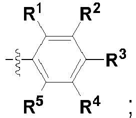
   in R¹, R², R³, R⁴, R⁵, any one (e.g., R², R³ or R⁴) is 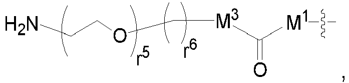 another (e.g., R¹ or R⁵) is 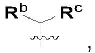 and the remaining three are H;
   preferably, in R¹, R², R³ R⁴, R⁵, R³ or R⁴ is 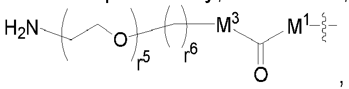 R¹ is 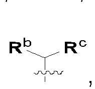 and the remaining three are H;
   r⁵, r⁶, independently of each other, are selected from any integer between 1 and 6;
   preferably, r⁵ is selected from 1, 2, 3;
   more preferably, r⁵ is 2;
   preferably, r⁶ is selected from 1, 2, 3;
   more preferably, r⁶ is 1 or 2;
   M¹, M³, independently of each other, are selected from a direct bond, NH, O, S;
   preferably, M¹ is selected from a direct bond, NH, O;
   preferably, M³ is selected from a direct bond, NH;
   more preferably, when M¹ is NH, O or S, M³ is a direct bond, and when M³ is NH, O or S, M¹ is a direct bond;
   in R^{b}, R^{c}, any one is selected from -N₃, -SS-C₁-C₆ alkyl (e.g., -SS-Me, -SS-Et, -SS-iPr, -SS-t-Bu), and the other is selected from C₁-C₆ alkyl;
   preferably, in R^{b}, R^{c}, any one is selected from -N₃, -SS-Me, and the other is methyl;
   Z is selected from O, S, BH;
   preferably, Z is O;
base¹ is selected from a base, a deaza base or a tautomer thereof, for example, adenine, 7-deazaadenine, thymine, uracil, cytosine, guanine, 7-deazaguanine or a tautomer thereof;
preferably, base¹ is selected from 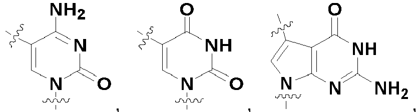 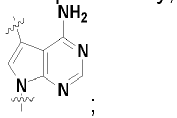
R represents a reversible blocking group.

In some specific embodiments, the reversible blocking group R' is selected from N₃-CH₂-, CH₃-CH₂-S-S-CH₂-, 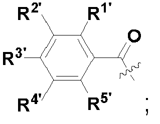
in R^{1'}, R^{2'}, R^{3'}, R^{4'}, R^{5'}, any one (e.g., R^{1'} or R^{5'}) is 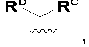 and the others are H,
preferably, in R^{1'}, R^{2'}, R^{3'}, R^{4'}, R^{5'}, R^{1'} is 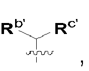 and the others are H,
in R^{b'}, R^{c'}, any one is selected from -N₃, -SS-C₁-C₆ alkyl (e.g., -SS-Me, -SS-Et, -SS-iPr, -SS-t-Bu), and the other is selected from C₁-C₆ alkyl,
preferably, in R^{b'}, R^{c'}, any one is selected from -N₃, -SS-Me, and the other is methyl.

In some embodiments, the aforementioned compound is selected from the following Table C:

**Table C:**

| Serial Number | Structural formulae |
|---|---|
| 7A | 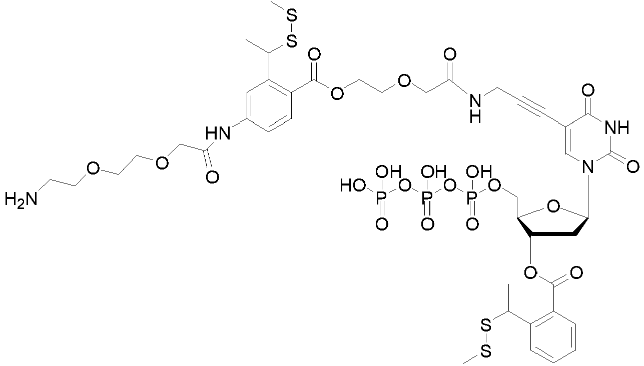 |
| 7B | 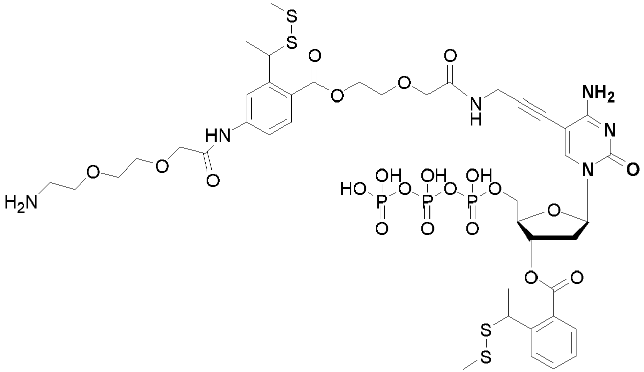 |
| 7C | 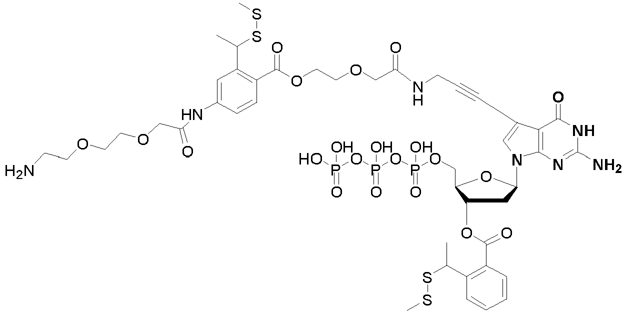 |
| 7D | 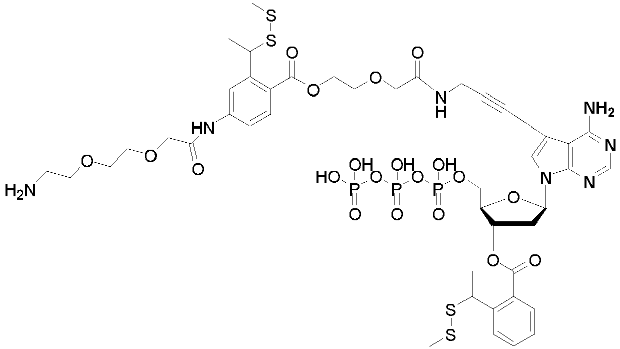 |
| 7D1 | 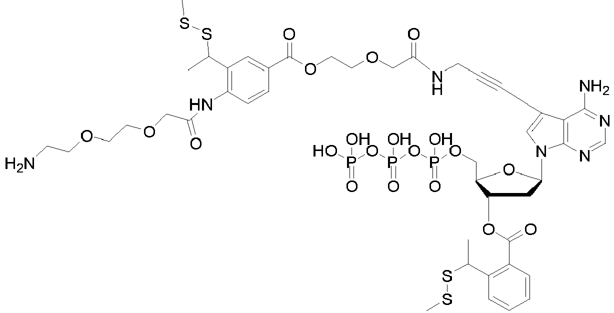 |

In some embodiments, the aforementioned compound or a salt thereof carries an additional detectable label.

In some embodiments, the additional detectable label carried by the compound or a salt thereof is introduced by an affinity reagent (e.g., antibody, aptamer, Affimer, Knottin), wherein the affinity reagent carries the detectable label, and the affinity reagent can specifically recognize and bind to an epitope of the compound or a salt thereof.

In some embodiments, the detectable label is linked to R in the compound of formula I-1 or to a structural moiety corresponding to the R in the aforementioned specific compound.

In some embodiments, the detectable label is linked to the terminal amino group in

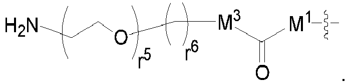

In some embodiments, the carboxyl in the detectable label is linked to the terminal amino group in 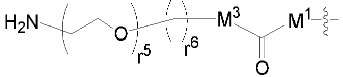 via formation of an amide bond.

In some embodiments, base¹ is different, and the additional detectable label carried by the compound of formula I-1 varies.

In some embodiments, the detectable label is a fluorescent label.

In some embodiments, the detectable label is selected from: iF700,

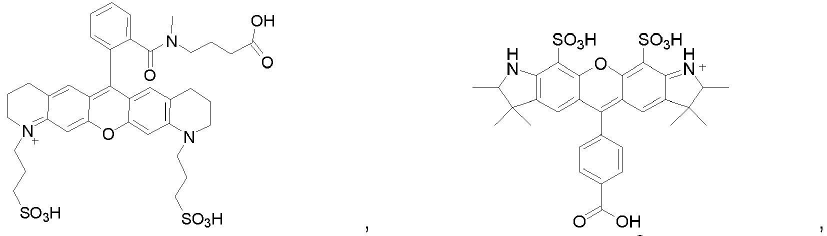

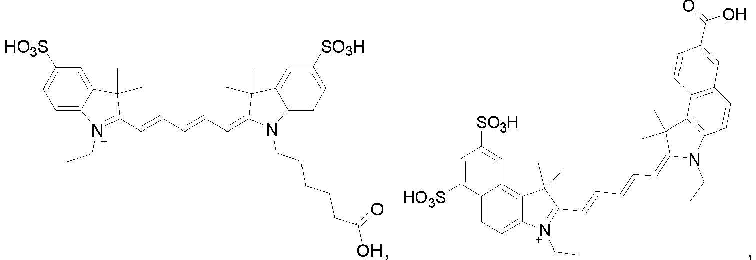

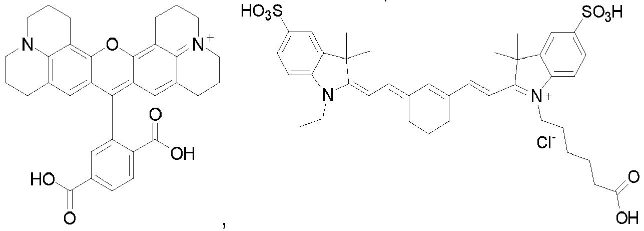

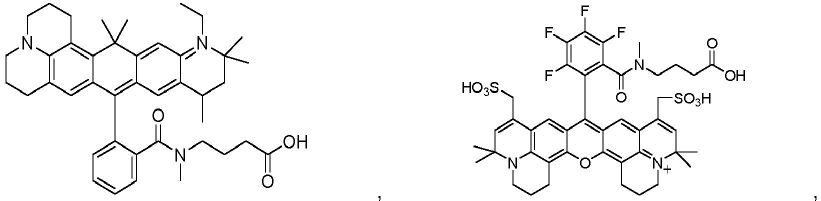

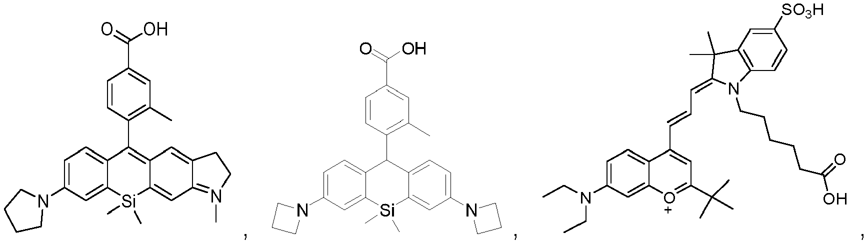

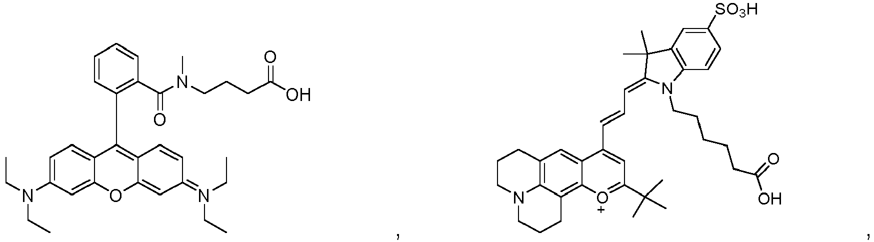

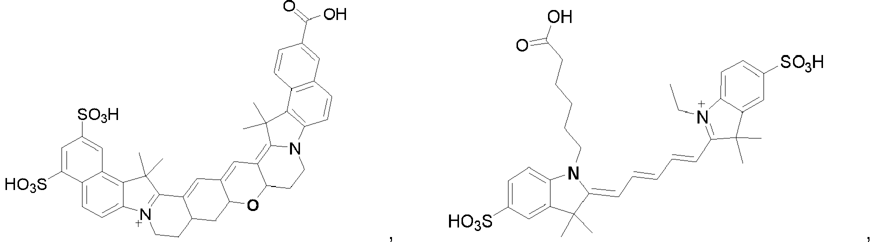

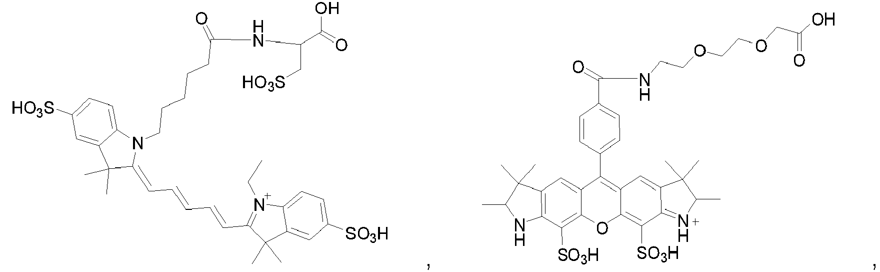

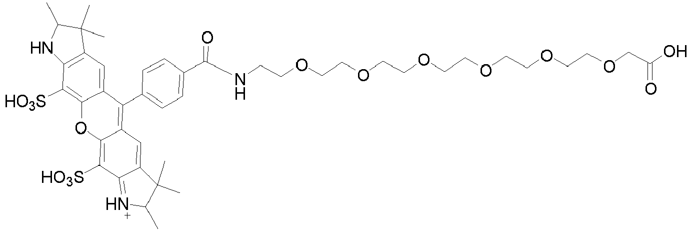

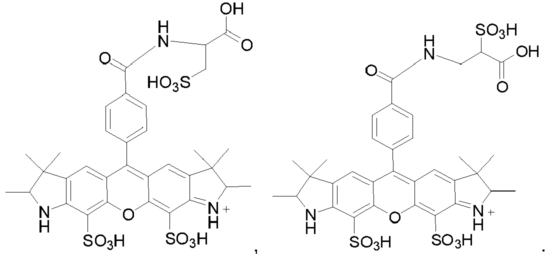

In some embodiments, the detectable label is selected from: iF700,

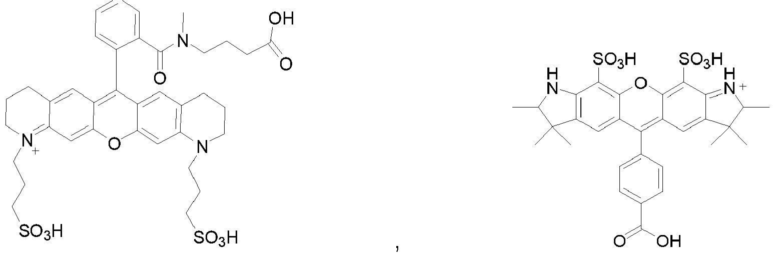

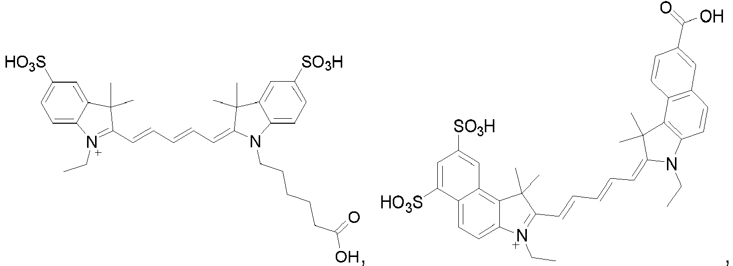

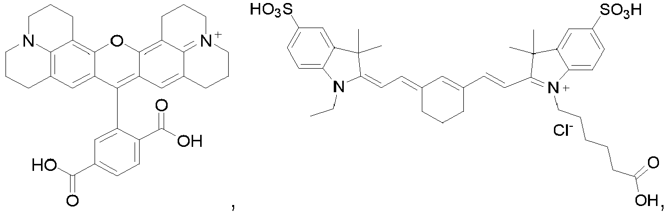

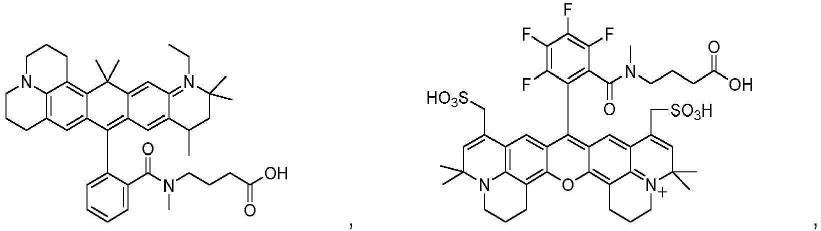

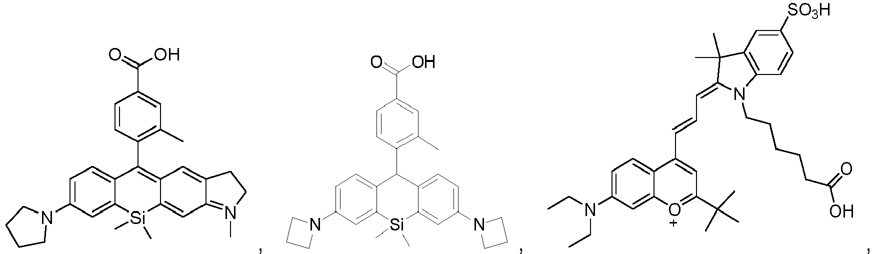

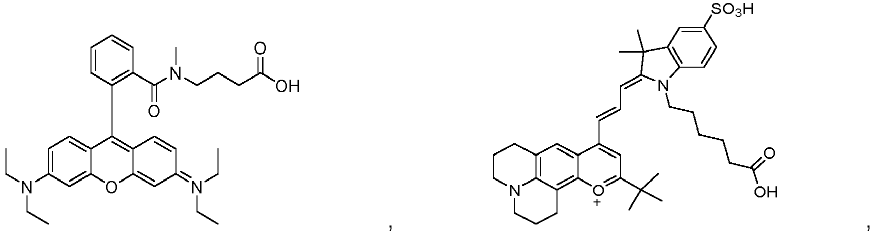

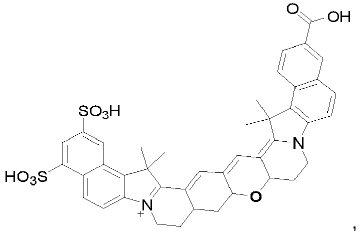

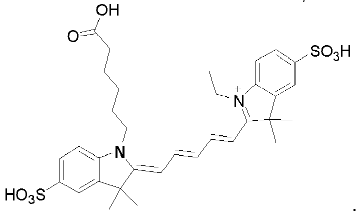

In some embodiments, the aforementioned compound is selected from the following Table D:

**Table D:**

| Serial Number | Structural formulae |
|---|---|
| 7A' | 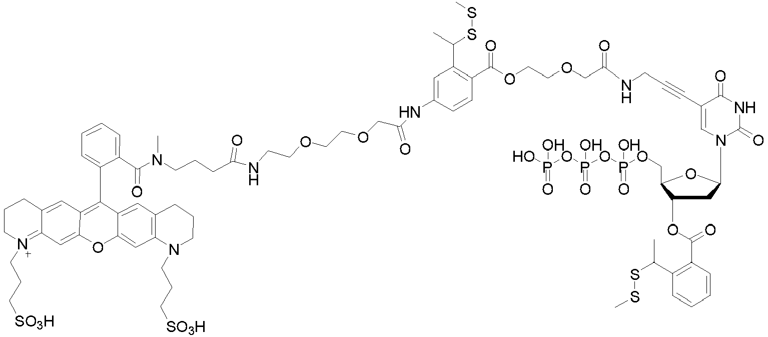 |
| 7B' | 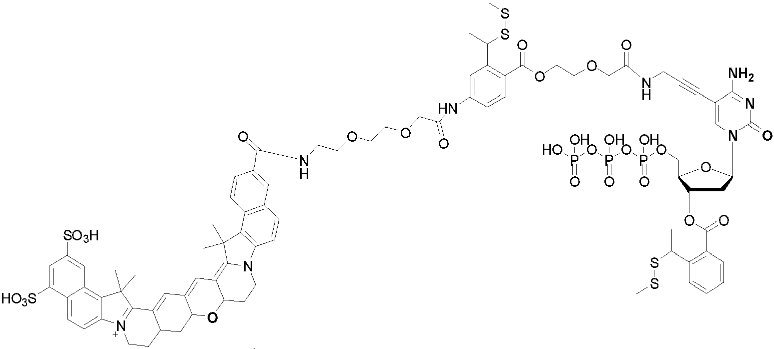 |
| 7B" | 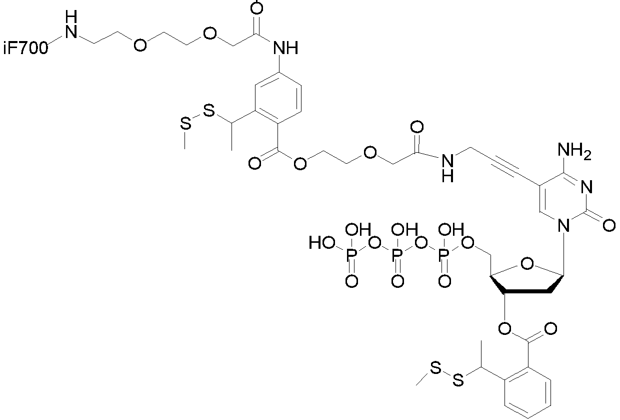 |
| 7C' | 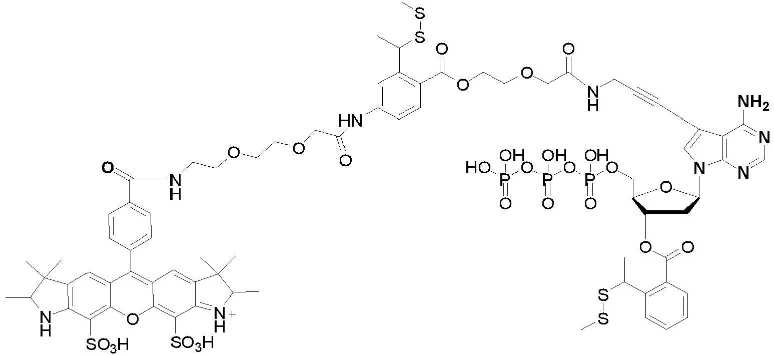 |
| 7C" | 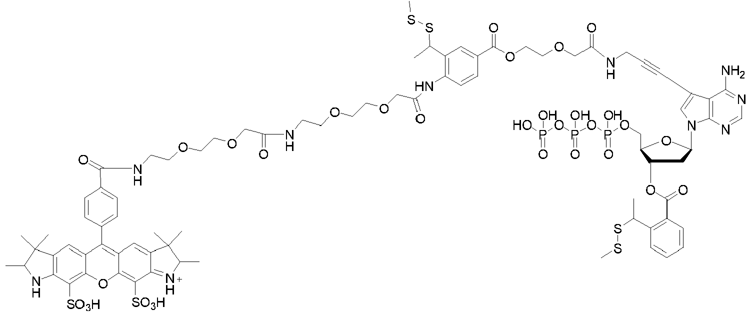 |
| 7C‴ | 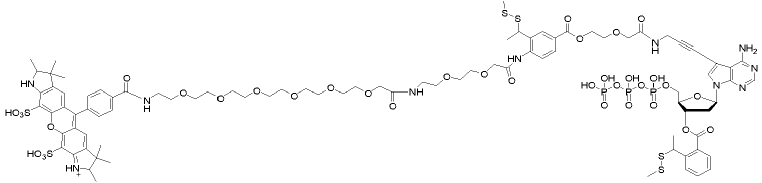 |
| 7Cʺʺ | 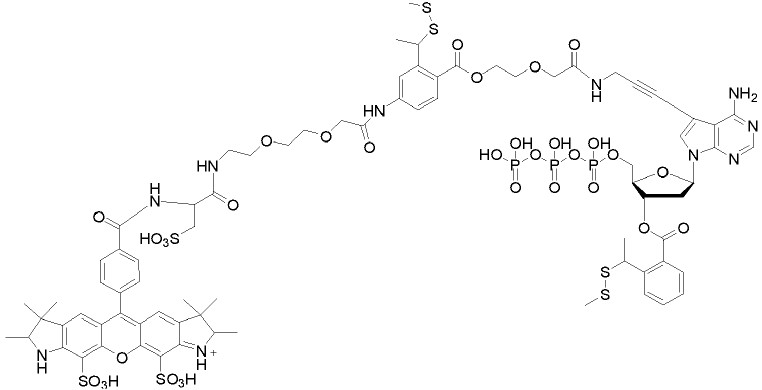 |
| 7Cʺ‴ | 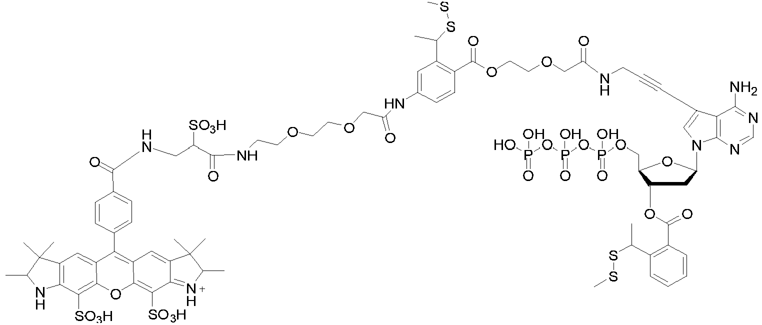 |
| 7C‴‴ | 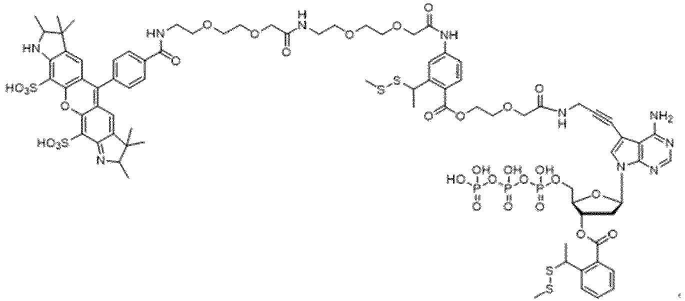 |
| 7D' | 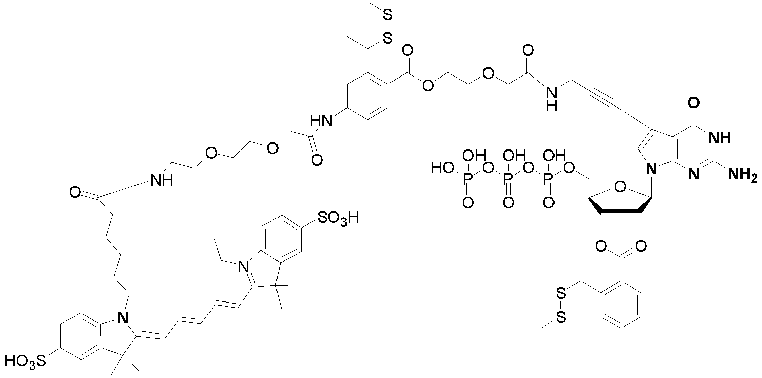 |
| 7D" | 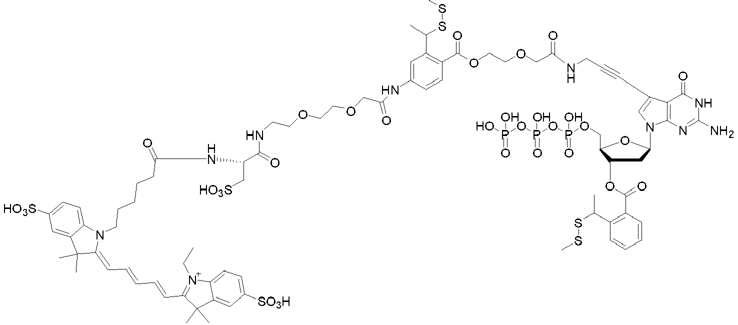 |

In the fifteenth aspect of the invention, the present invention provides a method for terminating a nucleic acid synthesis, which comprises incorporating the aforementioned compound or a salt thereof into a nucleic acid molecule to be terminated.

In some embodiments, the incorporation of the compound or a salt thereof is achieved by a terminal transferase, a terminal polymerase or a reverse transcriptase.

In some embodiments, the method comprises incorporating the compound or a salt thereof into the nucleic acid molecule to be terminated by using a polymerase.

In some embodiments, the method comprises performing a nucleotide polymerization reaction using a polymerase under conditions where the polymerase is allowed to carry out the nucleotide polymerization reaction, thereby incorporating the compound or a salt thereof into the 3' end of the nucleic acid molecule to be terminated.

In the sixteenth aspect of the invention, the present invention provides a method for preparing a growing polynucleotide complementary to a target single-stranded polynucleotide in a sequencing reaction, the method comprises incorporating the aforementioned compound or a salt thereof into the growing complementary polynucleotide, wherein the incorporation of the compound or a salt thereof prevents any subsequent nucleotides from being introduced into the growing complementary polynucleotide.

In some embodiments, the incorporation of the compound or a salt thereof is achieved by a terminal transferase, a terminal polymerase or a reverse transcriptase.

In some embodiments, the method comprises incorporating the compound or a salt thereof into the growing complementary polynucleotide by using a polymerase.

In some embodiments, the method comprises performing a nucleotide polymerization reaction using a polymerase under conditions where the polymerase is allowed to carry out the nucleotide polymerization reaction, thereby incorporating the compound or a salt thereof into the 3' end of the growing complementary polynucleotide.

In the seventeenth aspect of the invention, the present invention provides a nucleic acid intermediate, which is formed in determining the sequence of a target single-stranded polynucleotide, wherein the nucleic acid intermediate is formed according to the step:
incorporating a nucleotide complementary to the target single-stranded polynucleotide into a growing nucleic acid strand, to form the nucleic acid intermediate, wherein, the incorporated complementary nucleotide is the aforementioned compound or a salt thereof.

In the eighteenth aspect of the invention, the present invention provides a nucleic acid intermediate, which is formed in determining the sequence of a target single-stranded polynucleotide, wherein the nucleic acid intermediate is formed according to the step:
incorporating a nucleotide complementary to the target single-stranded polynucleotide into a growing nucleic acid strand, to form the nucleic acid intermediate, wherein, the incorporated complementary nucleotide is the aforementioned compound or a salt thereof, and the growing nucleic acid strand is pre-incorporated with at least one nucleotide complementary to the target single-stranded polynucleotide, the at least one pre-incorporated nucleotide complementary to the target single-stranded polynucleotide being the aforementioned compound or a salt thereof in which the reversible blocking group and optional detectable label have been removed.

In the nineteenth aspect of the invention, the present invention provides a method for determining the sequence of a target single-stranded polynucleotide, which comprises:
1) monitoring the incorporation of nucleotides complementary to the target single-stranded polynucleotide in a growing nucleic acid strand, wherein at least one complementary nucleotide incorporated is the aforementioned compound or a salt thereof, and,
2) determining the types of the incorporated nucleotides.

In some embodiments, the reversible blocking group and optional detectable label are removed before a next complementary nucleotide is introduced.

In some embodiments, the reversible blocking group and the detectable label are removed simultaneously.

In some embodiments, the reversible blocking group and the detectable label are removed sequentially; for example, after the detectable label is removed, the reversible blocking group is removed, or, after the reversible blocking group is removed, the detectable label is removed.

In some embodiments, the method for determining the sequence of a target single-stranded polynucleotide comprises the following steps:
(a) providing a plurality of different nucleotides, wherein at least one nucleotide is the aforementioned compound or a salt thereof, optionally, the rest of nucleotides are the aforementioned compound or a salt thereof;
(b) incorporating the plurality of different nucleotides into a sequence complementary to a target single-stranded polynucleotide, wherein the plurality of different nucleotides can be distinguished from each other during the detection;
(c) detecting the nucleotides in step (b) to determine the types of nucleotides incorporated;
(d) removing the reversible blocking groups and optional detectable labels carried by the nucleotides in step (b); and
(e) optionally repeating steps (a)-(d) one or more times;
whereby the sequence of the target single-stranded polynucleotide is determined.

In some embodiments, the method for determining the sequence of a target single-stranded polynucleotide comprises the following steps:
(1) providing a first nucleotide, a second nucleotide, a third nucleotide and a fourth nucleotide, wherein at least one of the four nucleotides is the aforementioned compound or a salt thereof, optionally, the rest of nucleotides are the aforementioned compound or a salt thereof;
(2) contacting the four nucleotides with a target single-stranded polynucleotide; removing the nucleotides not incorporated into the growing nucleic acid strand; detecting the nucleotides incorporated into the growing nucleic acid strand; removing the reversible blocking groups and optional detectable labels carried in the nucleotides that are incorporated into the growing nucleic acid strand;
optionally, it also includes (3): repeating the steps (1)-(2) one or more times.

In some embodiments, the method for determining the sequence of a target single-stranded polynucleotide comprises the following steps:
(a) providing a mixture comprising a duplex, nucleotides comprising at least one aforementioned compound or a salt thereof, a polymerase, and an excision reagent; wherein the duplex comprises a growing nucleic acid strand and a nucleic acid strand to be sequenced;
(b) carrying out a reaction comprising the following steps (i), (ii) and (iii), optionally, repeating the steps for one or more times:
   step (i): incorporating the compound or a salt thereof into the growing nucleic acid strand by using a polymerase, to form a nucleic acid intermediate containing the reversible blocking group and optional detectable label;
   step (ii): detecting the nucleic acid intermediate;
   step (iii): removing the reversible blocking group and optional detectable label contained in the nucleic acid intermediate by using the excision reagent.

In some embodiments, removal of the reversible blocking group and removal of the detectable label are performed simultaneously, or, removal of the reversible blocking group and removal of the detectable label are performed sequentially (for example, the reversible blocking group is removed first, or the detectable label is removed first).

In some embodiments, the excision reagent used for removal of the reversible blocking group is the same as that used for removal of the detectable label.

In some embodiments, the excision reagent used for removal of the reversible blocking group is different from that used for removal of the detectable label.

In some embodiments, the duplex is linked to a support.

In some embodiments, the growing nucleic acid strand is a primer.

In some embodiments, the primer is annealed to the nucleic acid strand to be sequenced to form the duplex.

In some embodiments, the duplex, the compound or a salt thereof, and the polymerase together form a reaction system containing a solution phase and a solid phase.

In some embodiments, the compound or a salt thereof is incorporated into the growing nucleic acid strand by using a polymerase under conditions where the polymerase is allowed to carry out a nucleotide polymerization reaction, thereby forming a nucleic acid intermediate containing the reversible blocking group and optional detectable label.

In some embodiments, the polymerase is selected from KOD polymerase or a mutant thereof (e.g., KOD POL151, KOD POL157, KOD POL171, KOD POL174, KOD POL376, KOD POL391).

In some embodiments, before any step of detecting the nucleic acid intermediate, the solution phase of the reaction system in the previous step is removed, and the duplex linked to the support is retained.

In some embodiments, the excision reagent is in contact with the duplex or the growing nucleic acid strand in the reaction system containing a solution phase and a solid phase.

In some embodiments, the excision reagent can remove the reversible blocking group and optional detectable label carried by the compound that is incorporated into the growing nucleic acid strand, without affecting the phosphodiester bond on the backbone of the duplex.

In some embodiments, after any step of removing the reversible blocking group and optional detectable label contained in the nucleic acid intermediate, the solution phase of the reaction system in this step is removed.

In some embodiments, a washing operation is performed after any step comprising a removal operation.

In some embodiments, after step (ii), the method further comprises: determining the type of compound incorporated into the growing nucleic acid strand in step (i) according to the signal detected in step (ii), and determining the type of nucleotide at a corresponding position in the nucleic acid strand to be sequenced based on the principle of base complementary pairing.

In the twentieth aspect of the invention, the present invention provides a kit, which comprises at least one aforementioned compound or a salt thereof.

In some embodiments, the kit comprises a first compound, a second compound, a third compound and a fourth compound, wherein the first, second, third and fourth compounds each are, independently, the aforementioned compound or a salt thereof.

In some embodiments, in the first compound, base¹ is selected from adenine, 7-deazaadenine or a tautomer thereof (e.g., 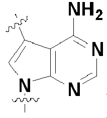 ); in the second compound, base¹ is selected from thymine, uracil or a tautomer thereof (e.g., 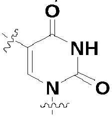 ); in the third compound, base¹ is selected from cytosine or a tautomer thereof (e.g., 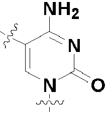 ); in the fourth compound, base¹ is selected from guanine, 7-deazaguanine or a tautomer thereof (e.g., 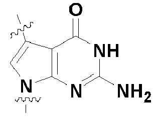 ).

In some embodiments, wherein the base¹ contained in the first, second, third and fourth compounds are different from each other.

In some embodiments, wherein the additional detectable labels carried by the first, second, third and fourth compounds are different from each other.

In some embodiments, the kit further comprises: a reagent for pretreating the nucleic acid molecule; a support for linking nucleic acid molecule to be sequenced; a reagent for linking (for example, covalently or non-covalently linking) the nucleic acid molecule to be sequenced to the support; primers for initiating a nucleotide polymerization reaction; a polymerase for carrying out the nucleotide polymerization reaction; one or more buffer solutions; one or more washing solutions; or any combination thereof.

In the twenty-first aspect of the invention, the present invention provides use of the aforementioned compound or a salt thereof or the aforementioned kit for determining the sequence of a target single-stranded polynucleotide.

Furthermore, in the twenty-second aspect of the invention, the present invention further provides a nucleotide analogue, which is formed of ribose or deoxyribose, a reversible blocking group, base or deaza base or a tautomer thereof, linker for linking to a detectable label and an optional phosphate group, wherein the linker comprises the structure shown in the following formula A or formula A': 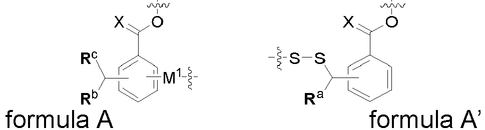 wherein:
in R^{b}, R^{c}, any one is selected from -N₃, -SS-C₁-C₆ alkyl (e.g., -SS-Me, -SS-Et, -SS-iPr, -SS-t-Bu), -ONH₂, -OCORₓ, -OCONHRₓ, -S-SO₂Rₓ, and the other is selected from H, aliphatic alkyl (e.g., C₁-C₆ alkyl, such as Me, Et, iPr, tBu), aromatic alkyl (e.g., phenyl -C₁-C₆ alkyl), cycloalkyl (e.g., C₃-C₆ cycloalkyl), wherein each Rₓ, independently, is selected from aliphatic alkyl (e.g., C₁-C₆ alkyl), cycloalkyl (e.g., C₃-C₆ cycloalkyl) or aromatic alkyl (e.g., phenyl C₁-C₆ alkyl);
preferably, in R^{b}, R^{c}, any one is selected from -N₃, -SS-C₁-C₆ alkyl (e.g., - SS-Me, -SS-Et, -SS-iPr, -SS-t-Bu), and the other is selected from C₁-C₆ alkyl;
more preferably, in R^{b}, R^{c}, any one is selected from -N₃, -SS-Me, -SS-Et, and the other is methyl;
most preferably, in R^{b}, R^{c}, any one is selected from -N₃, -SS-Me, and the other is methyl;
R^{a} is selected from H, aliphatic alkyl (e.g., C₁-C₆ alkyl, such as Me, Et, iPr, tBu), aromatic alkyl (e.g., phenyl -C₁-C₆ alkyl), cycloalkyl (e.g., C₃-C₆ cycloalkyl);
preferably, R^{a} is selected from H, C₁-C₆ alkyl;
more preferably, R^{a} is methyl;
X is selected from O, S, NH;
preferably, X is selected from O, S;
more preferably, X is O;
M¹ is selected from a direct bond, NH, O, S, CH₂;
preferably, M¹ is selected from a direct bond, NH, O;
preferably, the reversible blocking group is linked to the 3'-OH of the ribose or deoxyribose, the base or deaza base or a tautomer thereof is linked to the 1'-C of the ribose or deoxyribose, the optional phosphate group is linked to the 5'-OH of the ribose or deoxyribose, the linker is linked to the base or deaza base or a tautomer thereof.

In some embodiments, the structure of formula A is selected from formula A-1, formula A-2, formula A-3, formula A-4 or formula A-5, preferably the structure of formula A is formula A-1, formula A-2 or formula A-5; or, the structure of formula A' is selected from formula A'-1, formula A'-2 or formula A'-3, preferably the structure of formula A' is formula A'-1; 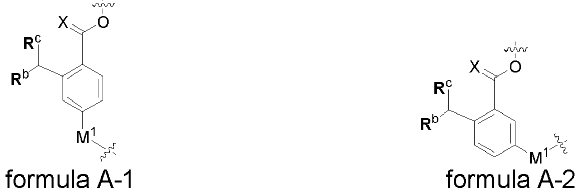 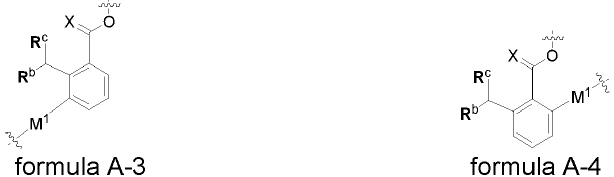 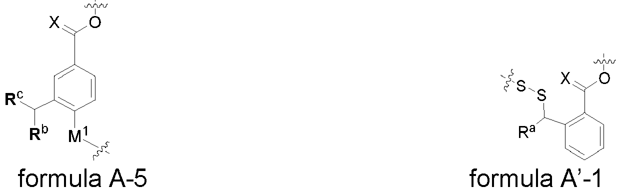 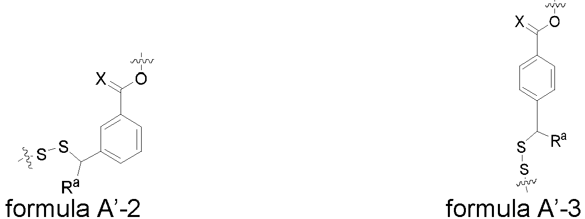 wherein:
R^{a}, R^{b}, R^{c}, X, M¹ have the same definitions as above.

In some embodiments, the structure of formula A is selected from: 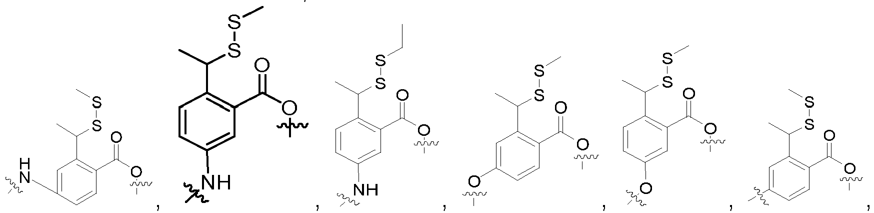 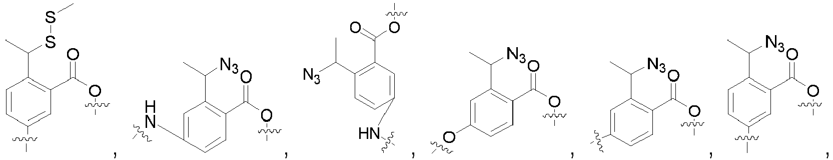 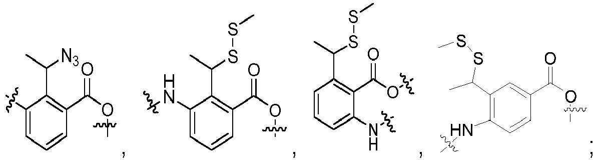 or the structure of formula A' is

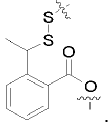

In some embodiments, the nucleotide analogue has a structure of the following formula B: 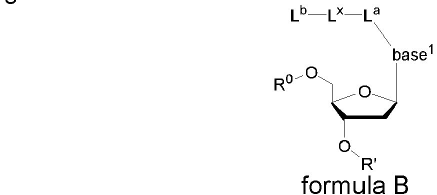
L^{x} has a structure of the aforementioned formula A, formula A', formula A-1, formula A-2, formula A-3, formula A-4, formula A-5, formula A'-1, formula A'-2 or formula A'-3, and the M¹ end or S end of L^{x} is linked to L^{b}, the O end of L^{x} is linked to L^{a}; 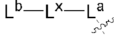
represents linker for linking to a detectable label;
base¹ represents base or deaza base or a tautomer thereof;
R' represents a reversible blocking group;
R⁰ represents H or phosphate group;
L^{a} represents the moiety in the linker for linking to the base or deaza base or a tautomer thereof;
L^{b} represents the moiety in the linker for linking to the detectable label.

In some embodiments, the nucleotide analogue has a structure of the following formula B: 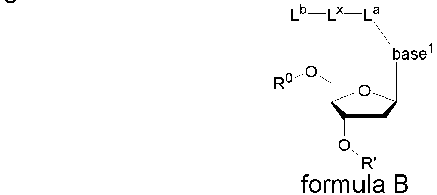 wherein:
L^{x} has a structure of the aforementioned formula A, formula A', formula A-1, formula A-2, formula A-3, formula A-4, formula A-5, formula A'-1, formula A'-2 or formula A'-3, and the M¹ end or S end of L^{x} is linked to L^{b}, the O end of L^{x} is linked to L^{a};
L^{a} is selected from 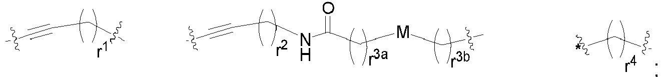
preferably, L^{a} is selected from 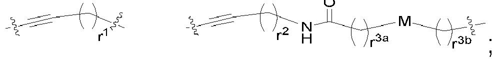
or preferably, L^{a} is selected from: 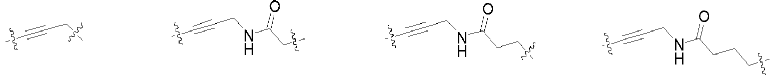 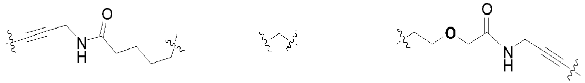
or more preferably, L^{a} is selected from: 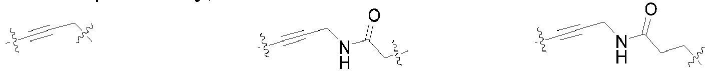 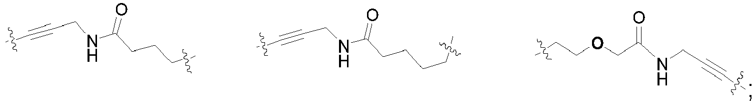
or further preferably, L^{a} is selected from: 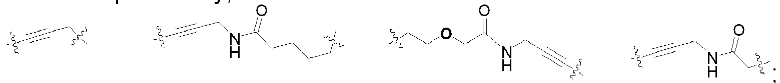
or most preferably, L^{a} is selected from: 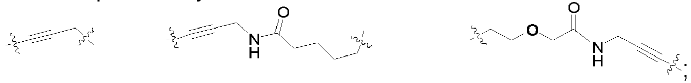
   r¹, r², r^{3a}, r^{3b}, r⁴, independently of each other, are selected from any integer between 1 and 6;
   preferably, r¹ is selected from 1, 2, 3;
   more preferably, r¹ is 1;
   preferably, r² is selected from 1, 2, 3;
   more preferably, r² is 1;
   preferably, r^{3a}, r^{3b}, independently of each other, are selected from 0, 1, 2, 3, 4, 5, and r^{3a}, r^{3b} being not 0 at the same time;
   more preferably, r^{3a}, r^{3b}, independently of each other, are selected from 0, 1, 2, 3, and r^{3a}, r^{3b} being not 0 at the same time;
   further preferably, r^{3a} is selected from 0, 1, r^{3b} is selected from 0, 2, and r^{3a}, r^{3b} being not 0 at the same time;
   most preferably, r^{3a} is 1;
   most preferably, r^{3b} is 2;
   preferably, r⁴ is selected from 1, 2, 3;
   more preferably, r⁴ is 1;
   M is selected from a direct bond, CH₂, NH, O, S;
   preferably, M is selected from a direct bond, CH₂, O;
   more preferably, M is selected from CH₂, O;
L^{b} is selected from 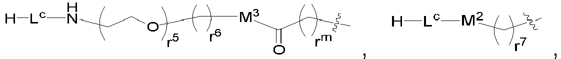 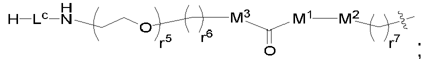
preferably, L^{b} is selected from 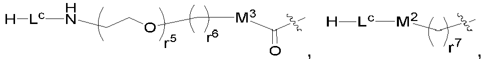 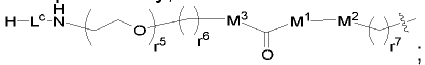
more preferably, L^{b} is selected from 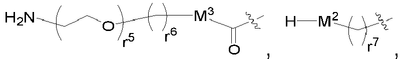 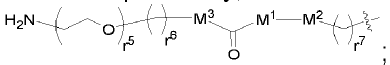
most preferably, L^{b} is selected from: 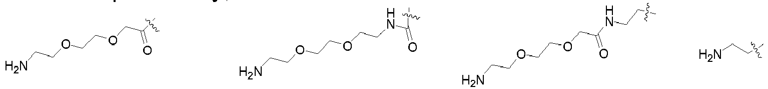 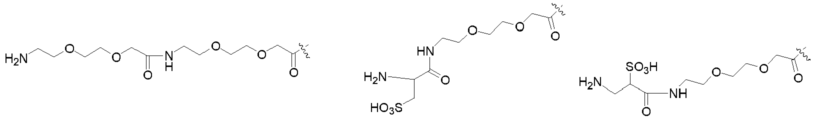 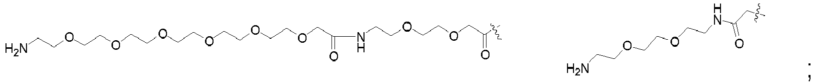
   L^{c} is selected from a direct bond, 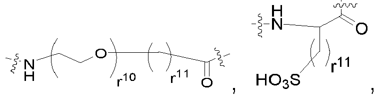 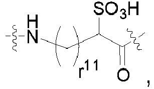 preferably, the NH end in L^{c} is linked to H in L^{b}, the =O end in L^{c} is linked to NH in L^{b};
   preferably, L^{c} is selected from a direct bond, 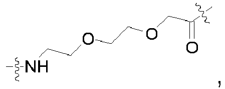 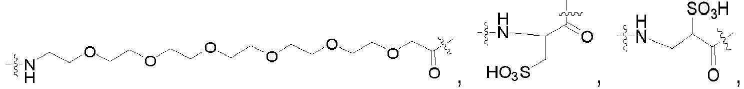 preferably, the NH end in L^{c} is linked to H in L^{b}, the =O end in L^{c} is linked to NH in L^{b};
   r^{m} is selected from any integer between 0 and 6;
   preferably, r^{m} is selected from 0, 1, 2, 3;
   more preferably, r^{m} is selected from 0, 1;
   r⁵, r⁶, r⁷, independently of each other, are selected from any integer between 1 and 6;
   preferably, r⁵ is selected from 1, 2, 3;
   more preferably, r⁵ is 2;
   preferably, r⁶ is selected from 1, 2, 3;
   more preferably, r⁶ is 1 or 2;
   preferably, r⁷ is selected from 1, 2, 3;
   more preferably, r⁷ is 2;
   r¹⁰ is selected from any integer between 1 and 10;
   preferably, r¹⁰ is selected from any integer between 2 and 6;
   more preferably, r¹⁰ is 2 or 6;
   r¹¹ is selected from any integer between 1 and 6;
   preferably, r¹¹ is selected from 1, 2, 3;
   more preferably, r¹¹ is 1;
   M¹, M², M³, independently of each other, are selected from a direct bond, NH, O, S, CH₂;
   preferably, M¹ is selected from a direct bond, NH, O;
   preferably, M² is NH;
   preferably, M³ is selected from a direct bond, NH;
base¹ represents base or deaza base;
R' represents a reversible blocking group; 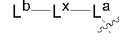
represents linker for linking to a detectable label;
R⁰ represents H or phosphate group.

In some embodiments, the base¹ is selected from adenine, 7-deazaadenine, thymine, uracil, cytosine, guanine, 7-deazaguanine or a tautomer thereof;
preferably, base¹ is selected from

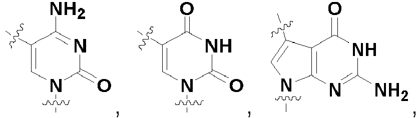

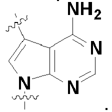

In some embodiments, R⁰ is selected from H, monophosphate group 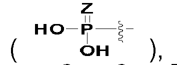 diphosphate group 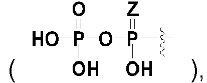 triphosphate group 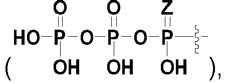 tetraphosphate group 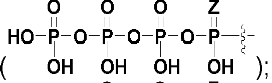
preferably, R° is a triphosphate group 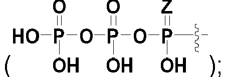
each Z, independently, is selected from O, S, BH;
preferably, Z is O.

In some embodiments, the reversible blocking group R' is selected from N₃-C₁-C₆ alkyl, C₁-C₆ alkyl-SS-C₁-C₆ alkyl, 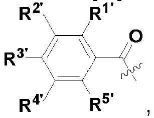 NH₂, -ONH₂, -OCOR_{z}, -OCONHR_{z}, wherein each R_{z}, independently, is selected from aliphatic alkyl (e.g., C₁-C₆ alkyl), cycloalkyl (e.g., C₃-C₆ cycloalkyl) or aromatic alkyl (e.g., phenyl C₁-C₆ alkyl);
preferably, the reversible blocking group R' is selected from N₃-C₁-C₆ alkyl, C₁-C₆ alkyl-SS-C₁-C₆ alkyl, 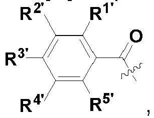 NH₂;
more preferably, the reversible blocking group R is selected from N₃-C₁-C₆ alkyl, C₁-C₆ alkyl-SS-C₁-C₆ alkyl, 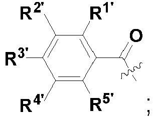
most preferably, the reversible blocking group R' is selected from N₃-CH₂-, CH₃-CH₂-S-S-CH₂-, 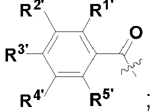
   in R^{1'}, R^{2'}, R^{3'}, R^{4'}, R^{5'}, any one (e.g., R^{1'} or R^{5'}) is 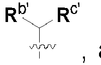 and the others, independently of each other, are selected from H, azido, nitro, amino, sulfo, carboxyl, aliphatic alkyl (e.g., C₁-C₆ alkyl), cycloalkyl (e.g., C₃-C₆ cycloalkyl), aromatic alkyl (e.g., phenyl -C₁-C₆ alkyl), F, I, Br, Cl, alkoxyl (e.g., C₁-C₆ alkoxyl),
   preferably, in R^{1'}, R^{2'}, R^{3'}, R^{4'}, R^{5'}, any one (e.g., R^{1'} or R^{5'}) is 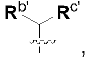 another (e.g., R^{3'}) is selected from H, C₁-C₆ alkoxyl (e.g., methoxy), and the others are H,
   more referably, in R^{1'}, R^{2'} R^{3'}, R^{4'}, R^{5'}, anv one (e.g., R^{1'} or R^{5'}) is 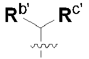 and the others are H,
   most preferably, in R^{1'}, R^{2'}, R^{3'}, R^{4'}, R^{5'}, R^{1'} is 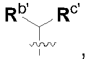 and the others are H,
   in R^{b'}, R^{c'}, any one is selected from -N₃, -SS-C₁-C₆ alkyl (e.g., -SS-Me, -SS-Et, -SS-iPr, -SS-t-Bu), -ONH₂, -OCORₓ, -OCONHRₓ, and the other is selected from H, aliphatic alkyl (e.g., C₁-C₆ alkyl, such as Me, Et, iPr, tBu), aromatic alkyl (e.g., phenyl -C₁-C₆ alkyl), cycloalkyl (e.g., C₃-C₆ cycloalkyl), wherein each Rₓ, independently, is selected from aliphatic alkyl (e.g., C₁-C₆ alkyl), cycloalkyl (e.g., C₃-C₆ cycloalkyl) or aromatic alkyl (e.g., phenyl C₁-C₆ alkyl),
   preferably, in R^{b'}, R^{c'}, any one is selected from -N₃, -SS-C₁-C₆ alkyl (e.g., -SS-Me, -SS-Et, -SS-iPr, -SS-t-Bu), and the other is selected from C₁-C₆ alkyl,
   more preferably, in R^{b'}, R^{c'}, any one is selected from -N₃, -SS-Me, and the other is methyl.

In some embodiments, the nucleotide analogue is selected from the compounds of the above Table A and Table C.

In some embodiments, the nucleotide analogue carries an additional detectable label, and the detectable label is linked to a linker (preferably L^{b});
preferably, the additional detectable label carried by the nucleotide analogue is introduced by an affinity reagent (e.g., antibody, aptamer, Affimer, Knottin), wherein the affinity reagent carries the detectable label, and the affinity reagent can specifically recognize and bind to an epitope of the nucleotide analogue; preferably, the detectable label is linked to the terminal amino group in L^{b};
preferably, the carboxyl in the detectable label is linked to the terminal amino group in L^{b} via formation of an amide bond;
preferably, base¹ is different, the additional detectable label carried by the nucleotide analogue varies;
preferably, the detectable label is a fluorescent label;
preferably, the detectable label is selected from: iF700,

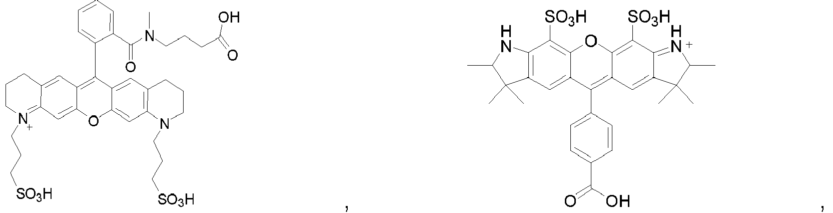

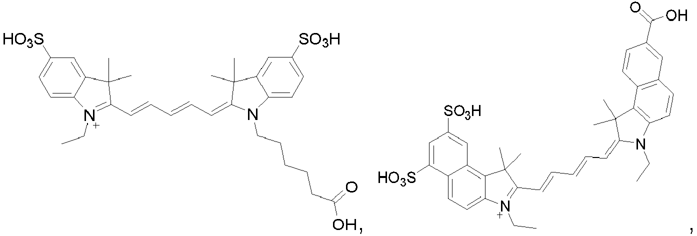

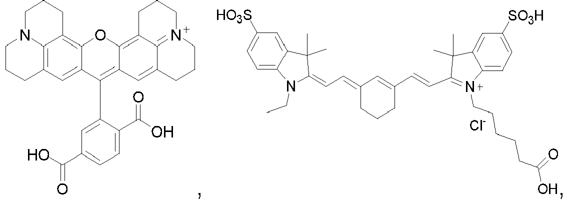

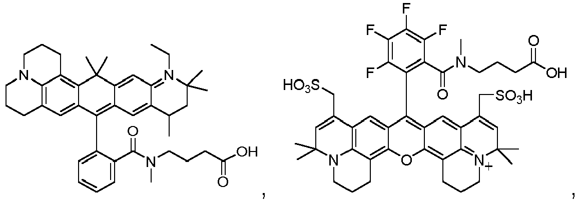

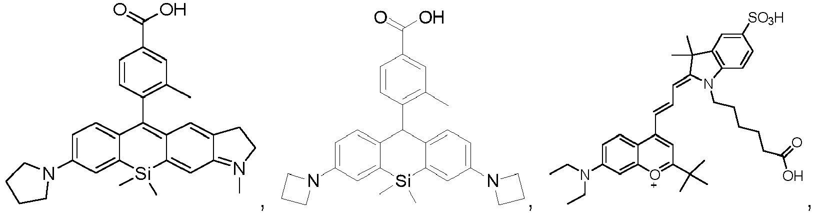

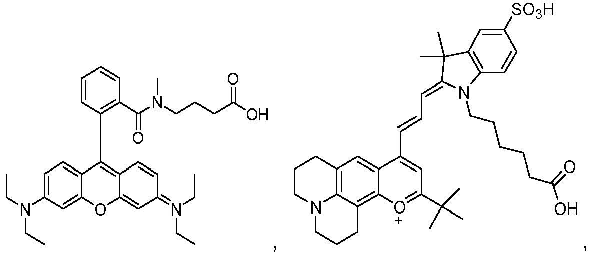

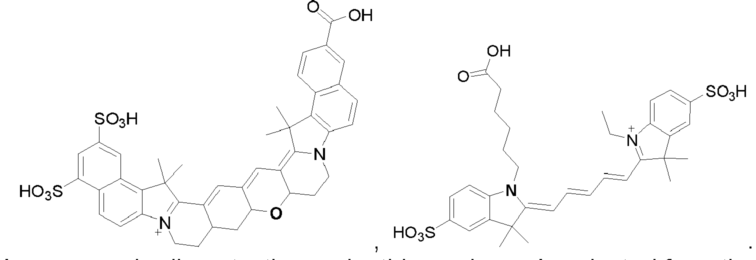

In some embodiments, the nucleotide analogue is selected from the compounds of the above Table B and Table D.

In the twenty-third aspect of the invention, the present invention provides a method for terminating a nucleic acid synthesis, which comprises incorporating the aforementioned nucleotide analogue into a nucleic acid molecule to be terminated;
preferably, the incorporation of the nucleotide analogue is achieved by a terminal transferase, a terminal polymerase or a reverse transcriptase;
preferably, the method comprises incorporating the nucleotide analogue into the nucleic acid molecule to be terminated by using a polymerase;
preferably, the method comprises performing a nucleotide polymerization reaction using a polymerase under conditions where the polymerase is allowed to carry out the nucleotide polymerization reaction, thereby incorporating the nucleotide analogue into the 3' end of the nucleic acid molecule to be terminated.

In the twenty-fourth aspect of the invention, the present invention provides a method for preparing a growing polynucleotide complementary to a target single-stranded polynucleotide in a sequencing reaction, the method comprises incorporating the aforementioned nucleotide analogue into the growing complementary polynucleotide, wherein the incorporation of the nucleotide analogue prevents any subsequent nucleotides from being introduced into the growing complementary polynucleotide;
preferably, the incorporation of the nucleotide analogue is achieved by a terminal transferase, a terminal polymerase or a reverse transcriptase;
preferably, the method comprises incorporating the nucleotide analogue into the growing complementary polynucleotide by using a polymerase;
preferably, the method comprises performing a nucleotide polymerization reaction using a polymerase under conditions where the polymerase is allowed to carry out the nucleotide polymerization reaction, thereby incorporating the nucleotide analogue into the 3' end of the growing complementary polynucleotide.

In the twenty-fifth aspect of the invention, the present invention provides a nucleic acid intermediate, which is formed in determining the sequence of a target single-stranded polynucleotide, wherein,
the nucleic acid intermediate is formed according to the step:
incorporating a nucleotide complementary to the target single-stranded polynucleotide into a growing nucleic acid strand, to form the nucleic acid intermediate, wherein, the incorporated complementary nucleotide is the aforementioned nucleotide analogue;
or, the nucleic acid intermediate is formed according to the step:
   incorporating a nucleotide complementary to the target single-stranded polynucleotide into a growing nucleic acid strand, to form the nucleic acid intermediate, wherein, the incorporated complementary nucleotide is the aforementioned nucleotide analogue, and the growing nucleic acid strand is pre-incorporated with at least one nucleotide complementary to the target single-stranded polynucleotide, the at least one pre-incorporated nucleotide complementary to the target single-stranded polynucleotide being the aforementioned nucleotide analogue in which the reversible blocking group and optional detectable label have been removed.

In the twenty-sixth aspect of the invention, the present invention provides a method for determining the sequence of a target single-stranded polynucleotide, which comprises:
1) monitoring the incorporation of nucleotides complementary to the target single-stranded polynucleotide in a growing nucleic acid strand, wherein at least one complementary nucleotide incorporated is the aforementioned nucleotide analogue, and,
2) determining the types of the incorporated nucleotides;

preferably, the reversible blocking group and optional detectable label are removed before a next complementary nucleotide is introduced;
preferably, the reversible blocking group and the detectable label are removed simultaneously;
preferably, the reversible blocking group and the detectable label are removed sequentially; for example, after the detectable label is removed, the reversible blocking group is removed, or, after the reversible blocking group is removed, the detectable label is removed.

In some embodiments, the method comprises the following steps:
(a) providing a plurality of different nucleotides, wherein at least one nucleotide is the aforementioned nucleotide analogue, optionally, the rest of nucleotides are the aforementioned nucleotide analogue;
(b) incorporating the plurality of different nucleotides into a sequence complementary to a target single-stranded polynucleotide, wherein the plurality of different nucleotides can be distinguished from each other during the detection;
(c) detecting the nucleotides in step (b) to determine the types of nucleotides incorporated;
(d) removing the reversible blocking groups and optional detectable labels carried by the nucleotides in step (b); and
(e) optionally repeating steps (a)-(d) one or more times;
whereby the sequence of the target single-stranded polynucleotide is determined.

In some embodiments, the method comprises the following steps:
(1) providing a first nucleotide, a second nucleotide, a third nucleotide and a fourth nucleotide, wherein at least one of the four nucleotides is the aforementioned nucleotide analogue, optionally, the rest of nucleotides are the aforementioned nucleotide analogue;
(2) contacting the four nucleotides with a target single-stranded polynucleotide; removing the nucleotides not incorporated into the growing nucleic acid strand; detecting the nucleotides incorporated into the growing nucleic acid strand; removing the reversible blocking groups and optional detectable labels carried in the nucleotides that are incorporated into the growing nucleic acid strand;
optionally, it also includes (3): repeating the steps (1)-(2) one or more times.

In some embodiments, the method comprises the following steps:
(a) providing a mixture comprising a duplex, nucleotides comprising at least one aforementioned nucleotide analogue, a polymerase, and an excision reagent; wherein the duplex comprises a growing nucleic acid strand and a nucleic acid strand to be sequenced;
(b) carrying out a reaction comprising the following steps (i), (ii) and (iii), optionally, repeating the steps for one or more times:
   step (i): incorporating the nucleotide analogue into the growing nucleic acid strand by using a polymerase to form a nucleic acid intermediate containing the reversible blocking group and optional detectable label:
   step (ii): detecting the nucleic acid intermediate;
   step (iii): removing the reversible blocking group and optional detectable label contained in the nucleic acid intermediate by using the excision reagent;
   preferably, removal of the reversible blocking group and removal of the detectable label are performed simultaneously, or, removal of the reversible blocking group and removal of the detectable label are performed sequentially (for example, the reversible blocking group is removed first, or the detectable label is removed first);
   preferably, the excision reagent used for removal of the reversible blocking group is the same as that used for removal of the detectable label;
   preferably, the excision reagent used for removal of the reversible blocking group is different from that used for removal of the detectable label.

In some embodiments, the duplex is linked to a support;
preferably, the growing nucleic acid strand is a primer;
preferably, the primer is annealed to the nucleic acid strand to be sequenced to form the duplex;
preferably, the duplex, the nucleotide analogue, and the polymerase together form a reaction system containing a solution phase and a solid phase;
preferably, the nucleotide analogue is incorporated into the growing nucleic acid strand by using a polymerase under conditions where the polymerase is allowed to carry out a nucleotide polymerization reaction, thereby forming a nucleic acid intermediate containing the reversible blocking group and optional detectable label;
preferably, the polymerase is selected from KOD polymerase or a mutant thereof (e.g., KOD POL151, KOD POL157, KOD POL171, KOD POL174, KOD POL376, KOD POL391);
preferably, before any step of detecting the nucleic acid intermediate, the solution phase of the reaction system in the previous step is removed, and the duplex linked to the support is retained;
preferably, the excision reagent is in contact with the duplex or the growing nucleic acid strand in the reaction system containing a solution phase and a solid phase;
preferably, the excision reagent can remove the reversible blocking group and optional detectable label carried by the nucleotide analogue that is incorporated into the growing nucleic acid strand, without affecting the phosphodiester bond on the backbone of the duplex;
preferably, after any step of removing the reversible blocking group and optional detectable label contained in the nucleic acid intermediate, the solution phase of the reaction system in this step is removed;
preferably, a washing operation is performed after any step comprising a removal operation;
preferably, after step (ii), the method further comprises: determining the type of the nucleotide analogue incorporated into the growing nucleic acid strand in step (i) according to the signal detected in step (ii), and
determining the type of nucleotide at a corresponding position in the nucleic acid strand to be sequenced based on the principle of base complementary pairing.

In the twenty-seventh aspect of the invention, the present invention provides a kit, which comprises at least one aforementioned nucleotide analogue;
preferably, the kit comprises a first compound, a second compound, a third compound and a fourth compound, wherein the first, second, third and fourth compounds each are, independently, the aforementioned nucleotide analogue;
preferably, in the first compound, base¹ is selected from adenine, 7-deazaadenine or a tautomer thereof (e.g., 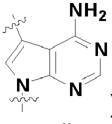 ); in the second compound, base¹ is selected from thymine, uracil or a tautomer thereof (e.g., 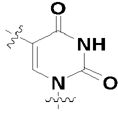 ); in the third compound, base¹ is selected from cytosine or a tautomer thereof (e.g., 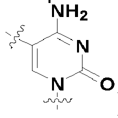 ); in the fourth compound, base¹ is selected from guanine, 7-deazaguanine or a tautomer thereof (e.g., 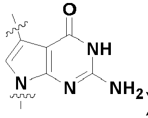 );
preferably, wherein the base¹ contained in the first, second, third and fourth compounds are different from each other;
preferably, wherein the additional detectable labels carried by the first, second, third and fourth compounds are different from each other.

In some embodiments, the kit further comprises: a reagent for pretreating the nucleic acid molecule; a support for linking nucleic acid molecule to be sequenced; a reagent for linking (for example, covalently or non-covalently linking) the nucleic acid molecule to be sequenced to the support; primers for initiating a nucleotide polymerization reaction; a polymerase for carrying out the nucleotide polymerization reaction; one or more buffer solutions; one or more washing solutions; or any combination thereof.

In the twenty-eighth aspect of the invention, the present invention provides use of the aforementioned nucleotide analogue or the aforementioned kit for determining the sequence of a target single-stranded polynucleotide.

### Brief introduction of the drawings

Figure 1 illustrates the nucleotide analogues with cleavable (fluorescent) label linked to base, as exemplified in Examples of the present invention;
Figure 2 illustrates the nucleotide analogues with (fluorescent) label linked to the reversible blocking group of 3'-OH, as exemplified in Examples of the present invention.

### Detailed description of the invention

The embodiments of the invention are described in detail below through specific embodiments, but in no case should they be interpreted as restrictions on the invention.

Unless otherwise specified, the above groups and substituents have common meanings in the field of pharmaceutical chemistry.

In various parts of the specification, the substituents of the compounds disclosed in the invention are disclosed according to the type or range of the group. It is particularly noted that the present invention includes each independent sub-combination of each and every member of these group types and ranges. For example, the term "C₁-C₆ alkyl" particularly refers to independently disclosed methyl, ethyl, C₃ alkyl, C₄ alkyl, C₅ alkyl and C₆ alkyl.

In addition, it should be noted that, unless otherwise clearly pointed out, the expressions "each... independently is/are selected from" and "... are each independently is/are selected from", as used throughout the specification, are interchangeable and both should be interpreted in a broad sense, i.e. it can mean that, in different groups, the specific options expressed by the same or different symbols are independent from each other, or it can mean that, in the same group, the specific options expressed by the same or different symbols are independent from each other.

The term "aliphatic alkyl" means any straight or branched saturated group containing from 1 to 20 carbon atoms, for example, C₁-C₁₂ alkyl, preferably C₁-C₆ alkyl.

The term "C₁-C₆ alkyl" means any straight or branched saturated group containing from 1 to 6 carbon atoms, such as, methyl (Me), ethyl (Et), n-propyl, isopropyl (iPr), n-butyl, isobutyl, tertbutyl (tBu), sec-butyl, n-amyl, tert-amyl, n-hexyl, etc.

The term "alkoxy" means any above-described alkyl (e.g., C₁-C₆ alkyl, etc.), which is connected to the rest of the molecule through the oxygen atom (-O-).

The term "cycloalkyl" means a saturated cyclic hydrocarbyl of 3-10 membered monocyclic ring system, for example, C₃-C₈ cycloalkyl, preferably C₃-C₆ cycloalkyl.

The term "C₃-C₆ cycloalkyl" means a saturated cyclic hydrocarbyl of 3-6 membered monocyclic ring system. C₃-C₆ cycloalkyl may be cyclopropyl, cyclobutyl, cyclopentyl, cyclohexyl, etc.

The term "aromatic alkyl" means an arylalkyl or heteroarylalkyl, wherein the alkyl is as defined above.

The term "heteroaryl" means an aromatic heterocycle, generally a 5-, 6-, 7-, or 8-memberd heterocycle containing from one to three heteroatoms selected from N, O or S; and the heteroaryl ring may optionally be further fused to an aromatic or non-aromatic carbocyclic or heterocyclic ring. The non-limiting examples of the heteroaryl are such as pyridyl, pyrazinyl, pyrimidinyl, pyridazinyl, indolyl, imidazolyl, thiazolyl, isothiazolyl, thioxazolyl, pyrrolyl, phenyl-pyrrolyl, furanyl, phenyl-furanyl, oxazolyl, isoxazolyl, pyrazolyl, thiophenyl, benzofuranyl, benzothiophenyl, benzo-1,3-dioxolane (benzodioxole), isodihydroindolyl, benzimidazolyl, indazolyl, quinolinyl, isoquinolinyl, 1,2,3-triazolyl, 1-phenyl-1,2,3-triazolyl, 2,3-dihydroindolyl, 2,3-dihydrobenzofuranyl, 2,3-dihydrobenzothienyl, benzopyranyl, 2,3-dihydrobenzoxazinyl, 2,3-dihydroquinoxalinyl, etc.

The term "aryl" means carbon-ring aromatic group having 6-14 carbon atoms, such as, C₆-C₁₀ aryl, preferably phenyl.

It is obvious to those skilled in the art from all the above descriptions that, any group having a combined name, such as "phenyl C₁-C₆ alkyl", should be understood as being conventionally constructed from the parts from which it is derived, such as constructed from C₁-C₆ alkyl substituted by phenyl, wherein the C₁-C₆ alkyl group is as defined above.

As used herein, the term "salt of the compound of formula I, formula I-1, formula I-2, formula II, formula II-1, formula II-2, formula III" may be exemplified by those organic acid addition salts of the organic acids forming the anion, including but not limiting to formate, acetate, propionate, benzoate, maleate, fumarate, succinate, tartrate, citrate, ascorbate, α-ketoglutarate, α-glycerophosphate, alkyl sulfonate or aryl sulfonate; preferably, the alkyl sulfonate is methanesulfonate or ethanesulfonate; the aryl sulfonate is benzenesulfonate or p-toluenesulfonate. Or alternatively, the term also means those inorganic acid salts, including but not limiting to hydrochloride, hydrobromide, hydroiodide, nitrate, bicarbonate and carbonate, sulfate or phosphate, etc.

The term "a direct bond" means that the group on both sides are directly connected. For example, in formula 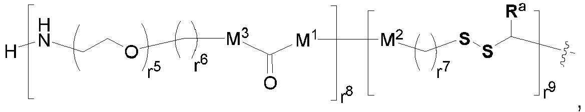 when M³ is a direct bond, it will be the following formula: 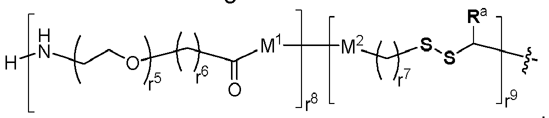 For another example, in formula 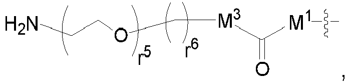 when M³ is a direct bond, it will be the following formula: 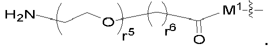 For yet another example, in formula 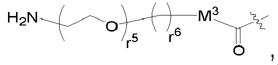 when M³ is a direct bond, it will be the following formula:

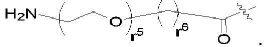

In the present invention, when the detectable label is linked to the terminal amino group in 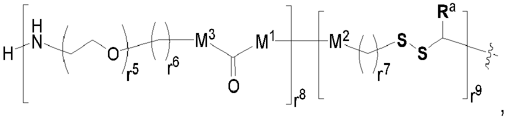 it may have the following two circumstances: 1) when r⁸ is not 0, the "terminal amino" refers to the last amino in the formula; and 2) when r⁸ is 0, the formula is 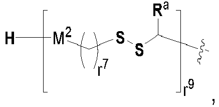 wherein M² may be NH, and in such circumstance, it can be understood that the "terminal amino" refers to the last amino in the changed formula.

In the present invention, when the detectable label is linked to the terminal amino group in 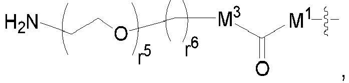 said "terminal amino" refers to the last amino in the formula.

In the present invention, when the detectable label is linked to the terminal amino group in L^{b} and the L^{b} is for example 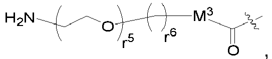 the "terminal amino" refers to the last amino in the formula.

In the method of the present invention, any substance composed of two strands, i.e., a growing nucleic acid strand and a nucleic acid strand to be sequenced, can be called a "duplex", regardless of the length of the growing nucleic acid strand or the nucleic acid strand to be sequenced. The nucleic acid strand to be sequenced may have a longer length than the growing nucleic acid strand.

In the method of the present invention, the nucleic acid strand to be sequenced may be any target nucleic acid molecule. In some preferred embodiments, the nucleic acid strand to be sequenced comprises deoxyribonucleotide, ribonucleotide, modified deoxyribonucleotide, modified ribonucleotide, or any combination thereof. In the method of the present invention, the nucleic acid strand to be sequenced is not limited by its type. In some preferred embodiments, the nucleic acid strand to be sequenced is DNA or RNA. In some preferred embodiments, the nucleic acid strand to be sequenced may be genomic DNA, mitochondrial DNA, chloroplast DNA, mRNA, cDNA, miRNA, or siRNA. In some preferred embodiments, the nucleic acid strand to be sequenced is linear or cyclic. In some preferred embodiments, the nucleic acid strand to be sequenced is double stranded or single stranded. For example, the nucleic acid strand to be sequenced may be a single stranded DNA (ssDNA), a double stranded DNA (dsDNA), a single stranded RNA (ssRNA), a double stranded RNA (dsRNA), or a hybrid of DNA and RNA. In some preferred embodiments, the nucleic acid strand to be sequenced is single stranded DNA. In some preferred embodiments, the nucleic acid strand to be sequenced is double stranded DNA.

In the method of the present invention, the nucleic acid strand to be sequenced is not limited by its source. In some preferred embodiments, the nucleic acid strand to be sequenced may be obtained from any source, such as from any cell, tissue, or organism (such as viruses, bacteria, fungi, plants, and animals). In some preferred embodiments, the nucleic acid strand to be sequenced originates from mammals (such as humans, non-human primates, rodents or canids), plants, birds, reptiles, fish, fungi, bacteria or viruses.

The methods for extracting or obtaining nucleic acid molecules from cells, tissues or organisms are well known to those skilled in the art. Suitable methods include but are not limited to such as ethanol precipitation and chloroform extraction. For a detailed description of such methods, see, for example, J. Sambrook et al., Molecular Cloning: A Laboratory Manual, 2nd Edition, Cold Spring Harbor Laboratory Press, 1989; and F.M. Ausubel et al., Short Protocols in Molecular Biology, 3rd Edition, John Wiley&Sons, Inc., 1995. In addition, various commercial kits may be used for extracting nucleic acid molecules from various sources (e.g., cells, tissues or organisms).

In the method of the invention, the nucleic acid molecule to be sequenced is not limited by its length. In some preferred embodiments, the length of the nucleic acid molecule to be sequenced may be at least 10bp, at least 20bp, at least 30bp, at least 40bp, at least 50bp, at least 100bp, at least 200bp, at least 300bp, at least 400bp, at least 500bp, at least 1000bp, or at least 2000bp. In some preferred embodiments, the length of nucleic acid molecules to be sequenced may be 10-20bp, 20-30bp, 30-40bp, 40-50bp, 50-100bp, 100-200bp, 200-300bp, 300-400bp, 400-500bp, 500-1000bp, 1000-2000bp, or more than 2000bp. In some preferred embodiments, the nucleic acid molecule to be sequenced can have a length of 10-1000bp, to facilitate high-throughput sequencing.

In the method for polynucleotides preparation or sequencing of the invention, suitable polymerase may be used for performing nucleotide polymerization reaction. In some exemplary embodiments, the polymerase can synthesize new DNA strands using DNA as a template (such as, a DNA polymerase). In some exemplary embodiments, the polymerase can synthesize new DNA strands using RNA as a template (e.g., a reverse transcriptase). In some exemplary embodiments, the polymerase can synthesize new RNA chains using DNA or RNA as templates (e.g., a RNA polymerase). Therefore, in some preferred embodiments, the polymerase is selected from DNA polymerase, RNA polymerase, and reverse transcriptase. Appropriate polymerase can be selected according to actual needs to carry out nucleotide polymerization reaction. In some preferred embodiments, the polymerization reaction is a polymerase chain reaction (PCR). In some preferred embodiments, the polymerization reaction is a reverse transcription reaction.

In the method of the present invention, KOD polymerase or its mutants may be used for the nucleotide polymerization reaction. KOD polymerase or its mutants (e.g., KOD POL151, KOD POL157, KOD POL171, KOD POL174, KOD POL376, KOD POL391) may lead to acceptable incorporation efficiency for the modified nucleoside or nucleotide of the invention. KOD POL391 and KOD POL171 lead to acceptable incorporation efficiency for the modified nucleotides of the invention. In some embodiments, the incorporation efficiency of KOD POL391 or KOD POL171 for the modified nucleotides of the invention is more than 70%, such as 70% - 80%, 80% - 90% or 90% - 100%.

In the method for polynucleotide preparation or sequencing of the invention, the polymerization reaction of nucleotides is carried out under suitable conditions. The suitable polymerization conditions include the composition of the solution phase, the concentration of each component, the pH of the solution phase, and the polymerization temperature. Polymerization is performed under suitable conditions to obtain acceptable and even higher incorporation efficiency.

In some embodiments of the present invention, the hydroxyl (-OH) at 3'-position of the deoxyribose in the compound of formula I, formula II, formula III, formula B is protected (by R'), thereby can terminate the polymerization caused by polymerase (such as, a DNA polymerase). For example, when the compound of formula I, formula II, formula III, formula B is introduced into the 3'-end of the growing nucleic acid chain, because there is no free hydroxyl (-OH) at the 3'-position of the deoxyribose of the compound, the polymerase cannot proceed to the next round of polymerization, and the polymerization is terminated. In such case, only one base will be incorporated into the growing nucleic acid chain in each round of polymerization.

In addition, the protective group (R') of the hydroxyl (-OH) at the 3'-position of the deoxyribose in the compound of formula I, formula II, formula III, formula B can be removed to restore the free hydroxyl (-OH). Then the growing nucleic acid chain may undergo next round of polymerization reaction using polymerase and the compound of formula I, formula II, formula III, formula B to incorporate therein a base again.

That is, the hydroxyl (-OH) at the 3'-position of the deoxyribose in the compound of formula I, formula II, formula III, formula B is reversibly blocked: when the compound of formula I, formula II, formula III, formula B is incorporated into the 3' end of the growing nucleic acid chain, it will terminate the polymerization caused by the polymerase and stop any further extension of the growing nucleic acid chain; and, after the blocking group contained in the compound of formula I, formula II, formula III, formula B is removed, the polymerase can continue the polymerization of the growing nucleic acid chain to extend the nucleic acid chain.

Some embodiments described herein relate to the use of conventional detectable labels. Detection may be realized by any suitable method, including fluorescence spectroscopy or other optical means. Preferred label is a fluorescent label, namely a fluorophore, which, after absorbing energy, emits radiation of certain wavelength. Many suitable fluorescent labels are known in the art. For example, Welch et al., (Chem. Eur. J. 5(3):951-960,1999) discloses dansyl-functionalised fluorescent moieties that can be used in the present invention. Zhu et al. (Cytometry 28:206-211, 1997) describes the use of the fluorescent labels Cy3 and Cy5, which can also be used in the present invention. Labels suitable for use are also disclosed in Prober et al. (Science 238:336-341, 1987), Connell et al. (BioTechniques 5(4):342-384, 1987), Ansorge et al. (Nucl. Acids Res. 15(11):4593-4602, 1987) and Smith et al. (Nature 321:674, 1986). Other commercially available fluorescent labels include, but are not limited to iF700, fluorescein, rhodamine (including TMR, texas red and Rox), alexa, BODIPY, acridine, coumarin, pyrene, benzanthracene and the cyanins. It is particularly noted that iF700 is one of commonly used fluorescent labels in the art, such as shown in Table 7 of US20180223358A1. This fluorescent label is commercially available.

Multiple labels can also be used in the present application, for example, bi-fluorophore FRET cassettes (Tet. Let. 46:8867-8871, 2000). Multi-fluor dendrimeric systems (J. Am. Chem. Soc. 123:8101-8108, 2001) can also be used. Although fluorescent labels are preferred, other forms of detectable labels will be apparent as useful to those of ordinary skill in the art. For example, microparticles, including quantum dots (Empodocles et al., Nature 399:126-130, 1999), gold nanoparticles (Reichert et al., Anal. Chem. 72:6025-6029, 2000) and microbeads (Lacoste et al., Proc. Natl. Acad. Sci USA 97(17):9461-9466, 2000) can all be used.

Multi-component labels can also be used in the present application. A multi-component label is one which is dependent on the interaction with a further compound for detection. The most common multi-component label used in biology is the biotin-streptavidin system. Biotin is used as the label attached to the nucleotide or modified nucleotide. Streptavidin is then added separately to enable detection to occur. Other multi-component systems can be used. For example, dinitrophenol has a commercially available fluorescent antibody that can be used for detection.

In some embodiments described herein, carrying the detectable labels described above on the modified nucleotides or nucleoside molecules can be achieved through the incorporation of affinity reagents (e.g., antibodies, aptamers, Affimer, and Knottin), wherein the affinity reagent can specifically recognize and bind to the epitopes of the modified nucleotides or nucleoside molecules. The specific principle is shown in WO2018129214A1, which is herein incorporated by reference in its entirety.

In other embodiments described herein, the modified nucleotide or nucleoside molecule may be linked to a detectable label described above. In some such embodiments, the linker used may be cleaved. The use of cleavable linker ensures that the labels can be removed as needed after the detection, and thus avoiding any interference signals from any subsequently added labeled nucleotides or nucleosides.

The linker can be cleaved by any suitable method, including exposure to acids, bases, nucleophiles, electrophiles, radicals, metals, reducing or oxidizing agents, light, temperature, enzymes etc. The linker as discussed herein may also be cleaved with the same catalyst used in cleavage of the protective group on base. Suitable linkers can be adapted from standard chemical protecting groups, as disclosed in Greene & Wuts, Protective Groups in Organic Synthesis, John Wiley & Sons. Further suitable cleavable linkers used in solid-phase synthesis are disclosed in Guillier et al. (Chem. Rev. 100:2092-2157, 2000).

The use of the term "cleavable linker" is not meant to imply that the whole linker is required to be removed from, e.g., the nucleotide or modified nucleotide. Where the detectable label is attached to the nucleotide or modified nucleotide, the nucleoside cleavage site can be located at a position on the linker that ensures that part of the linker remains attached to the nucleotide or modified nucleotide after cleavage.

It can be known by those skilled in the art that the nucleotides of the present invention may be effective in Sanger sequencing, next generation high-throughput sequencing (NGS sequencing) and the third generation sequencing (single molecule sequencing), because the type of nucleotides incorporated in sequencing can be detected one by one by using the reversible blocking nucleotide analogue described herein.

The present invention will be further explained below in conjunction with specific examples. It should be noted that the following examples only illustrate the preparation of nucleotide analogues for one of the four kinds of base, and those skilled in the art can refer to such preparation process for preparing nucleotide analogues for the remaining three kinds of base. Moreover, when the compound is connected to a dye in the final step of the preparation process as illustrated in the following examples, any dye known in the art can be used for the connection, and the ones exemplified in each of the following examples are only for illustration, but not a limitation on the selection scope of dye. In addition, unless otherwise specified, all raw materials are commercially available.

### I. Preparation examples of the compounds

### Example 1

### Synthesis of linker 1

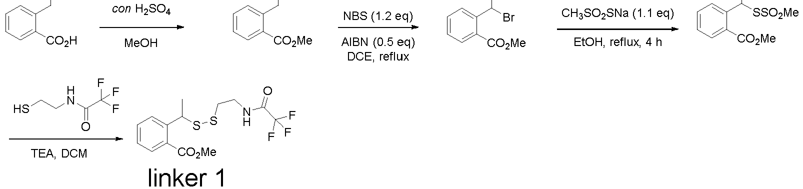

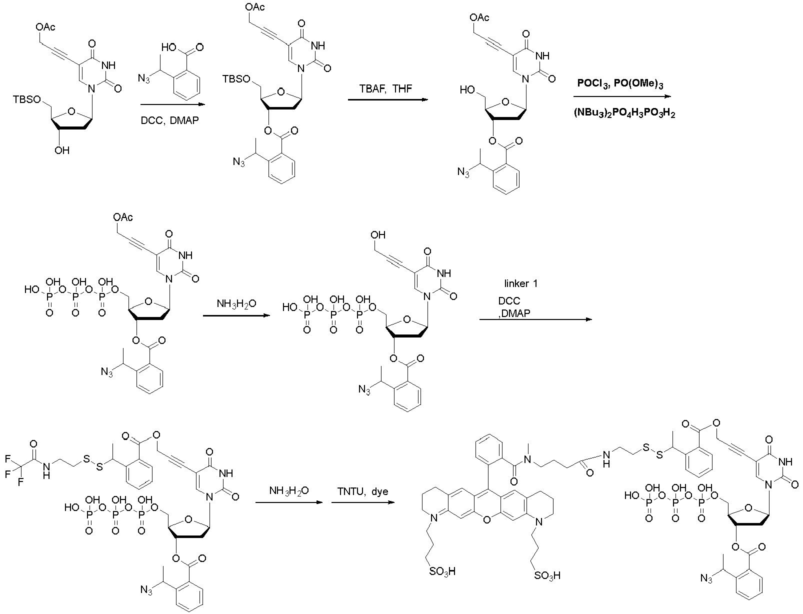

The nucleoside was condensed with acid in the presence of DCC, treated by TBAF for protective group removal, and then triphosphorylated. After aminolysis, it was linked to linker 1, and, after removal of trifluoroacetyl, coupled with fluorescent dye to obtain the final product.

### Example 2

### Synthesis of linker 2

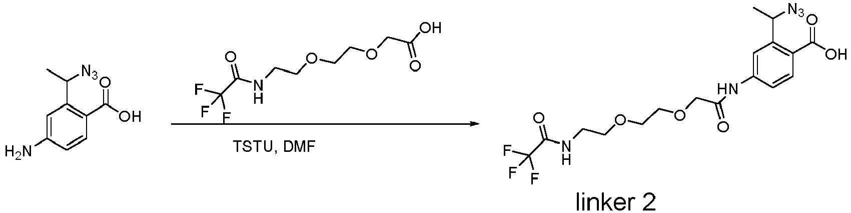

The condensation of p-amino azidobenzoic acid and trifluoroacetyl-protected PEG linker led to linker 2.

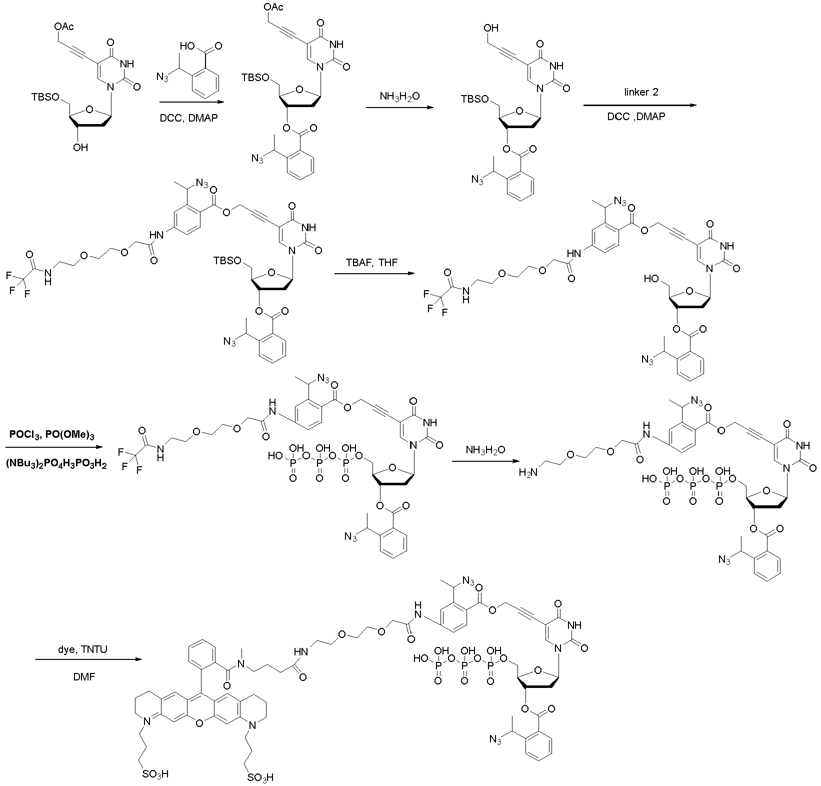

The nucleoside was condensed with acid in the presence of DCC, treated by ammonia to remove the acetyl group, and then subjected to another condensation in the presence of DCC. It was treated by TBAF for protective group removal, and then triphosphorylated. After aminolysis for removing the trifluoroacetyl group, it was coupled with fluorescent dye to obtain the final product.

### Example 3

### Synthesis of linker 3

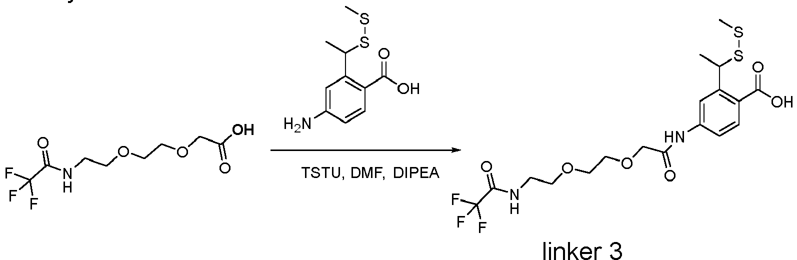

For synthesis process and product identification, refer to Example 7-1.

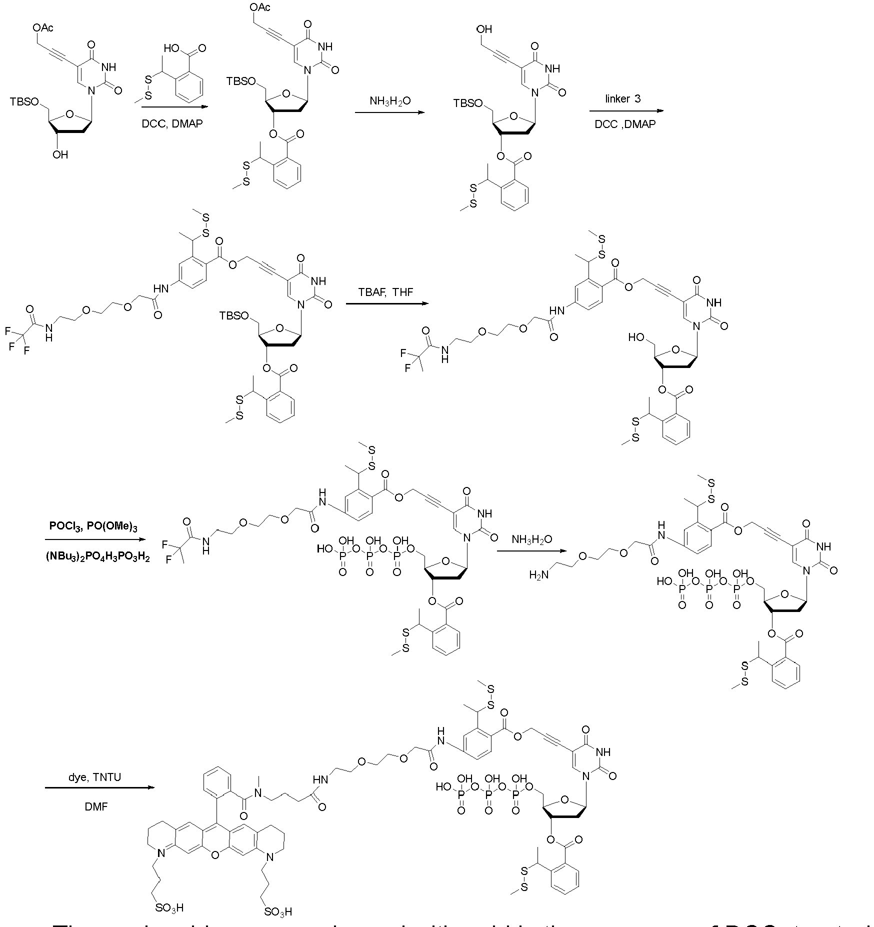

The nucleoside was condensed with acid in the presence of DCC, treated by ammonia to remove the acetyl group, and then subjected to another condensation in the presence of DCC. It was treated by TBAF for protective group removal, and then triphosphorylated. After aminolysis for removing trifluoroacetyl group, it was coupled with fluorescent dye to obtain the final product.

### Example 4

### Synthesis of linker 4

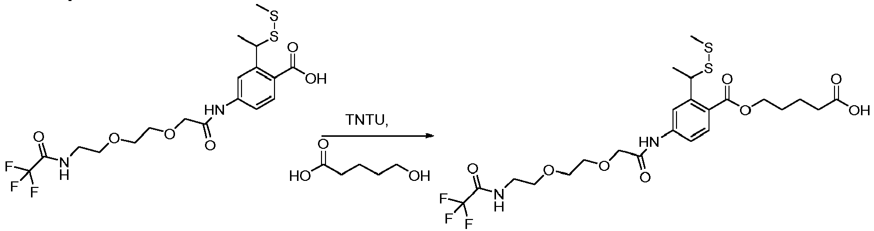

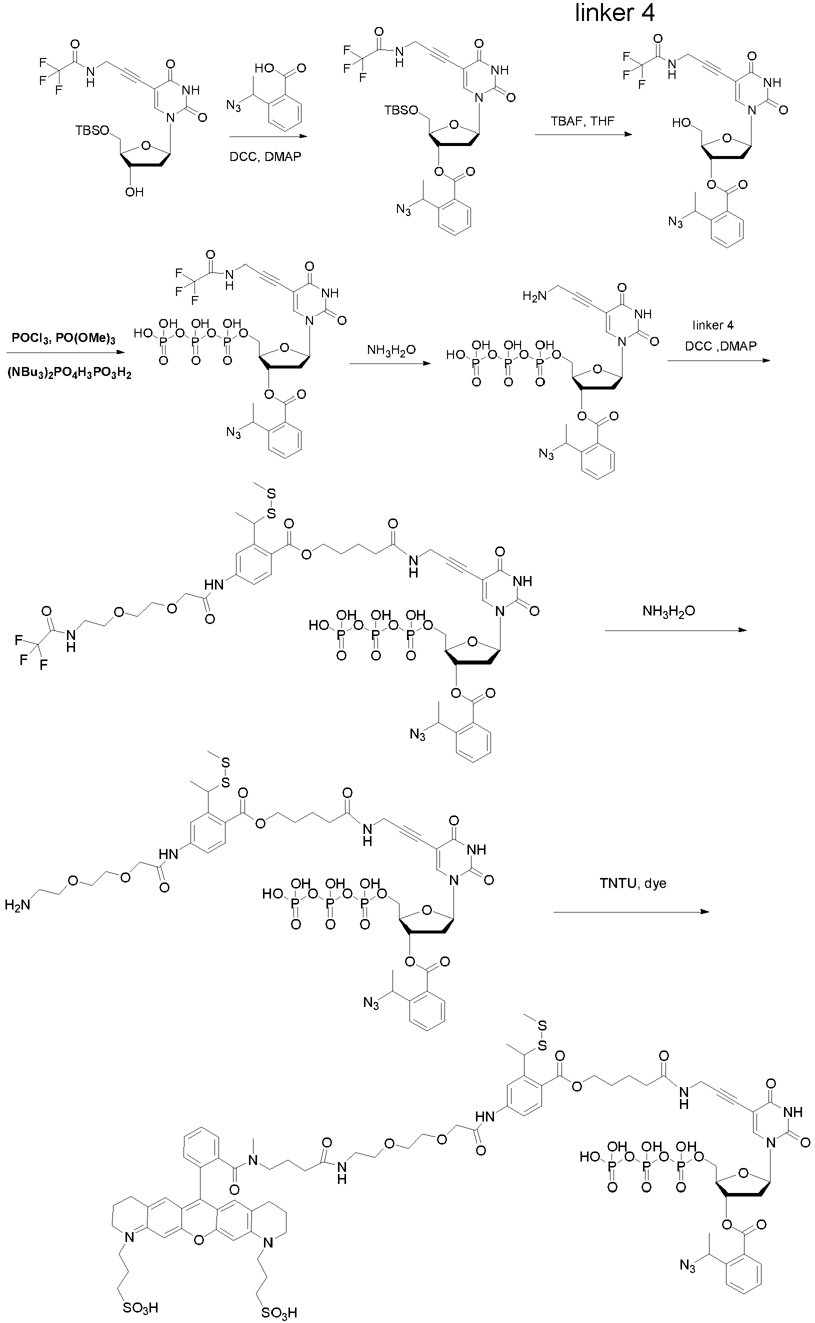

The nucleoside was condensed with acid in the presence of DCC, treated by TBAF for protective group removal, and then triphosphorylated. After aminolysis for removing the trifluoroacetyl group, it was coupled with linker 4, followed by removal of the trifluoroacetyl group in the linker, and coupled with fluorescent dye to obtain the final product.

### Example 5

### Synthesis of linker 5

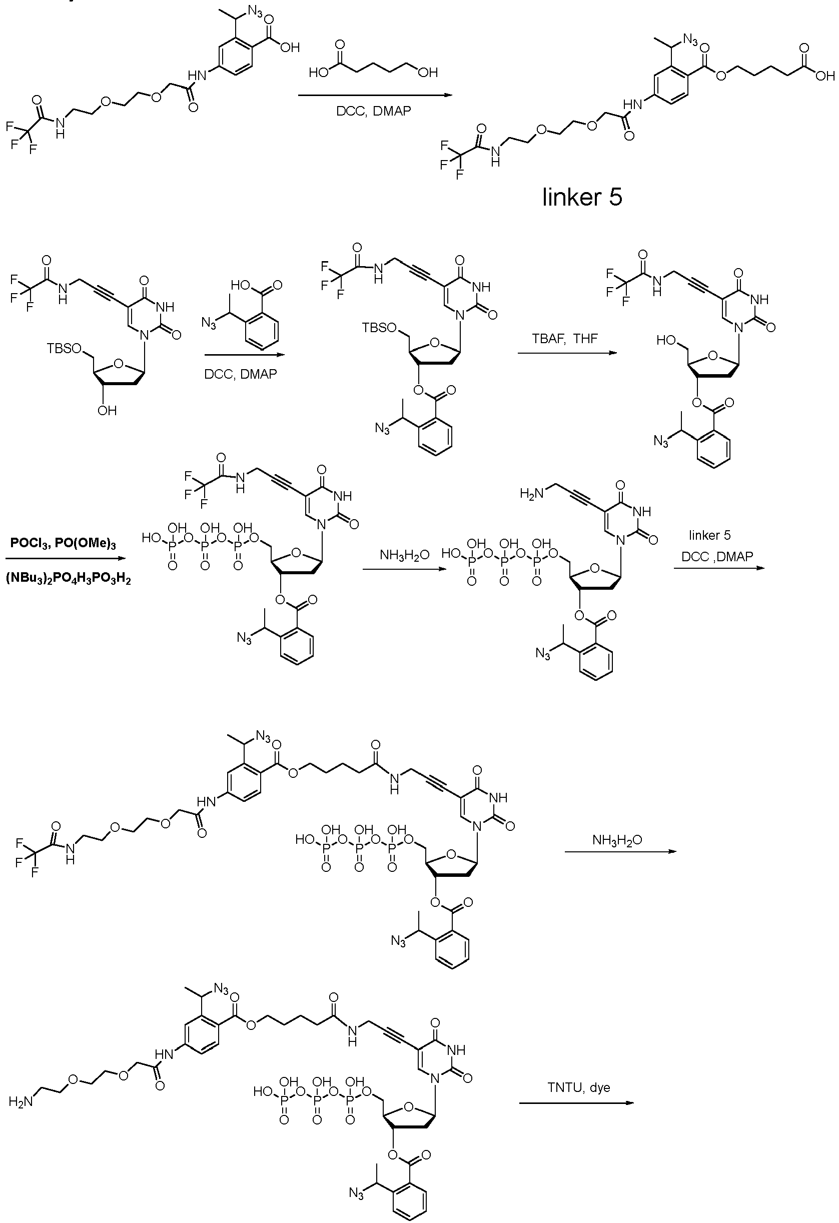

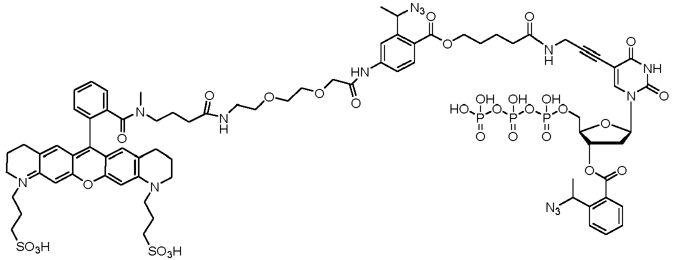

The nucleoside was condensed with acid in the presence of DCC, treated by TBAF for protective group removal, and then triphosphorylated. After aminolysis for removing the trifluoroacetyl group, it was coupled with linker 5, followed by removal of the trifluoroacetyl group in the linker, and coupled with fluorescent dye to obtain the final product.

### Example 6-1

### Synthesis of linker 6

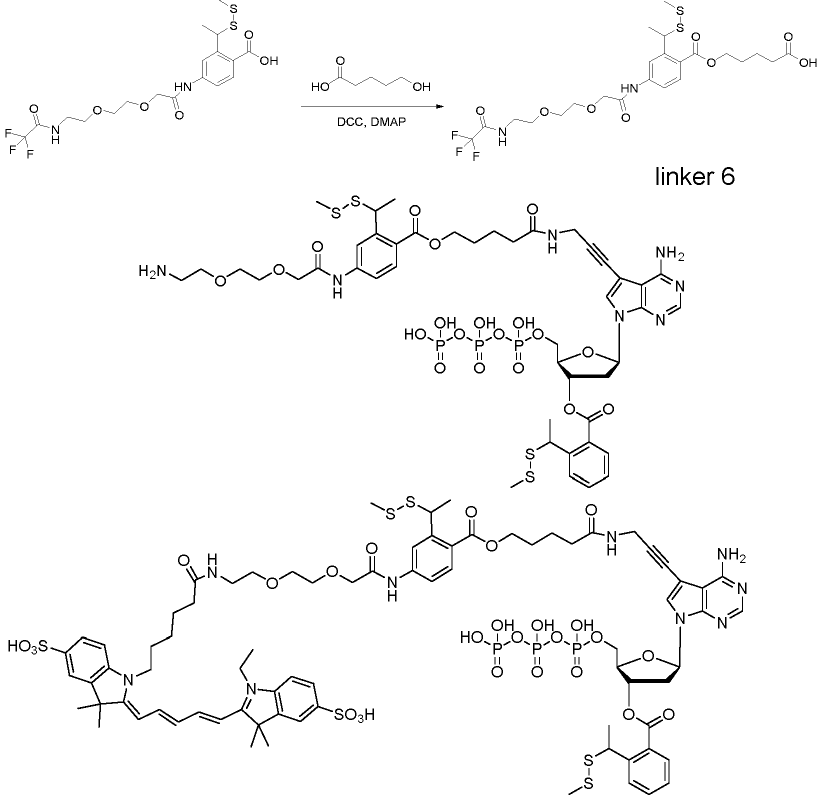

The synthesis process was similar to that of Example 7-2. 15mg of solid, HPLC purity > 99%. MS[ES(-)], m/z1861.4. ¹H NMR (400 MHz, DMSO-*d*₆) δ 10.27 (s, 1H), 8.63 - 8.58 (m, 1H), 8.36 (t, J = 13.1 Hz, 2H), 8.09 (d, J = 6.3 Hz, 1H), 8.00 (s, 1H), 7.92 - 7.88 (m, 2H), 7.84 (d, J = 4.5 Hz, 1H), 7.81 (s, 2H), 7.78 (d, J = 8.6 Hz, 1H), 7.71 - 7.67 (m, 1H), 7.65 - 7.62 (m, 4H), 7.49 - 7.43 (m, 1H), 7.31 (dd, J = 8.3, 3.1 Hz, 2H), 6.67 - 6.54 (m, 2H), 6.31 (dd, J = 13.8, 5.3 Hz, 2H), 5.68 (s, 1H), 5.30 (q, J = 6.9 Hz, 1H), 5.26 - 5.17 (m, 1H), 4.34 (dt, J = 22.7, 3.7 Hz, 1H), 4.22 (t, J = 4.8 Hz, 2H), 4.13 (s, 2H), 4.11 (d, J = 5.0 Hz, 2H), 4.08 - 4.00 (m, 2H), 3.67 - 3.65 (m, 1H), 3.59 - 3.56 (m, 2H), 3.22 - 3.15 (m, 2H), 2.21 (t, J = 6.6 Hz, 2H), 2.11 (d, J = 2.3 Hz, 4H), 2.09 - 2.04 (m, 4H), 1.66 (s, 3H), 1.60 (d, J = 6.9 Hz, 3H), 1.52 (dd, J = 14.9, 7.4 Hz, 2H), 1.37 - 1.29 (m, 2H), 1.26 (t, J = 7.1 Hz, 4H).

### Example 6-2

### Synthesis of linker

The synthesis process of linker 6 was the same as that of Example 6-1.

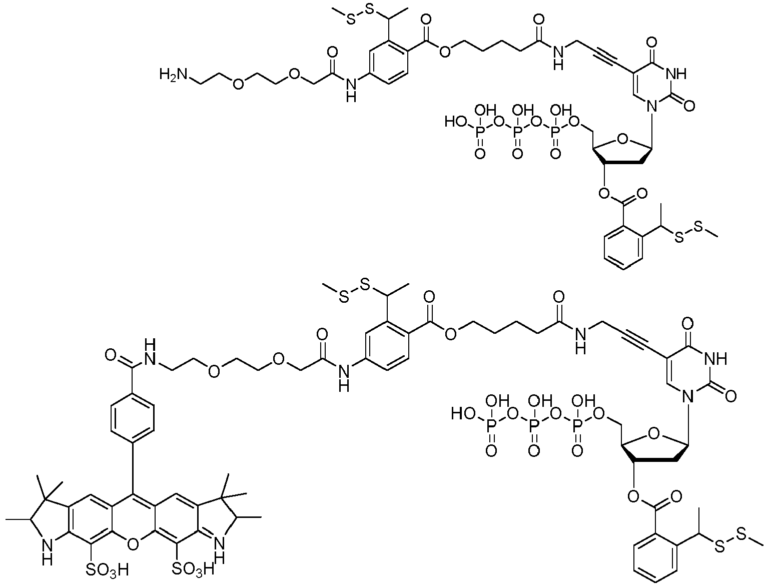

The synthesis process was similar to that of Example 7-1. 16mg of solid, HPLC purity > 99%. MS[ES(-)], m/z 1809.3. ¹H NMR (400 MHz, DMSO-*d*₆) δ 10.16 (s, 1H), 8.95 (d, J = 8.2 Hz, 1H), 8.85 (t, J = 5.5 Hz, 1H), 8.80 (s, 2H), 8.23 (d, J = 8.2 Hz, 1H), 8.14 (d, J = 8.4 Hz, 2H), 7.99 (d, J = 1.7 Hz, 1H), 7.86 (d, J = 7.6 Hz, 1H), 7.82 (d, J = 8.5 Hz, 1H), 7.69 (dd, J = 8.7, 1.6 Hz, 1H), 7.62 (d, J = 3.5 Hz, 2H), 7.52 (d, J = 7.9 Hz, 2H), 7.45 - 7.37 (m, 1H), 6.72 (s, 2H), 6.30 - 6.20 (m, 1H), 5.57 - 5.54 (m, 1H), 5.31 (q, J = 6.9 Hz, 1H), 5.22 - 5.13 (m, 1H), 4.36 (d, J = 20.9 Hz, 1H), 4.24 - 4.19 (m, 2H), 4.14 (s, 2H), 4.08 (d, J = 4.0 Hz, 1H), 4.06 - 4.00 (m, 2H), 3.80 (dd, J = 13.1, 6.8 Hz, 2H), 3.73 - 3.69 (m, 2H), 3.67 (dd, J = 7.0, 4.1 Hz, 2H), 3.63 (d, J = 5.6 Hz, 2H), 2.22 (t, J = 7.0 Hz, 2H), 2.13 (s, 3H), 2.07 (t, J = 7.8 Hz, 5H), 1.73 - 1.66 (m, 2H), 1.65 (d, J = 6.9 Hz, 3H), 1.60 (d, J = 7.0 Hz, 3H), 1.17 (d, J = 6.7 Hz, 12H), 1.00 (d, J = 2.0 Hz, 6H).

### Example 7-1

### (1) step 1

### Synthesis of linker 7

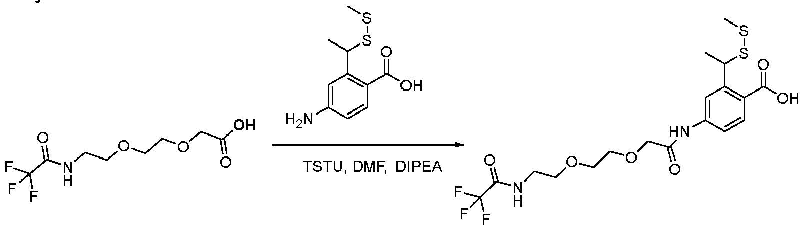

1g of PEG-CF3 substrate (OKeanos Tech, cat. No. OK20A409) was dissolved in DMF, added with TSTU (2 eq) and N,N-diisopropylethylamine (3 eq). The reaction mixture was stirred at room temperature for 2h, added with aniline substrate (OKeanos Tech, cat. No. OK20A408) (1.2 eq). The reaction was allowed to proceed for 4h, quenched by adding water, and then extracted with dichloromethane. After being concentrated to dry, the residue was purified by column chromatography, to provide a colorless oily compound. MS[ES(-)], m/z 484.3. ¹H NMR (400 MHz, DMSO-*d₆*) δ 12.92 (s, 1H), 9.98 (s, 1H), 9.53 (t, J = 5.2 Hz, 1H), 7.88 (d, J = 2.0 Hz, 1H), 7.81 (d, J = 8.4 Hz, 1H), 7.69 (dd, J = 8.4, 2.0 Hz, 1H), 5.48 (q, J = 6.8 Hz, 1H), 4.11 (s, 2H), 3.68 - 3.66 (m, 2H), 3.62 - 3.60 (m, 2H), 3.55 (t, J = 5.6 Hz, 2H), 3.37 (q, J = 5.6 Hz, 2H), 2.15 (s, 3H), 1.60 (d, J = 6.8 Hz, 3H).

### (2) step 2

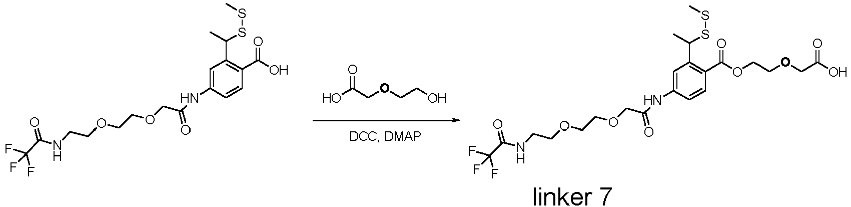

100mg of the substrate obtained in the above step 1 was dissolved in 3mL of DMF, added with DCC (1.2 eq) and DMAP (10% mol). The reaction mixture was stirred for 30 minutes, added with hydroxyl acid substrate (Shanghai Bidepharm, cat. No. BD628858) (2 eq). The reaction mixture was stirred for 12 hours, and then directly separated by preparative HPLC (C18 reverse phase chromatography column, 250*30 mm), to provide a white solid (linker 7) 85mg. MS[ES(-)], m/z 585.2. ¹H NMR (400 MHz, DMSO-*d₆*) δ 10.06 (s, 1H), 9.53 (t, J = 5.2 Hz, 1H), 7.92 (d, J = 2.0 Hz, 1H), 7.82 (d, J = 8.4 Hz, 1H), 7.73 (dd, J = 8.4, 2.0 Hz, 1H), 5.31 (q, J = 6.8 Hz, 1H), 4.40 - 4.30 (m, 3H), 4.11 (s, 2H), 3.88 (s, 2H), 3.78 (t, J = 4.8 Hz, 2H), 3.68 - 3.66 (m, 2H), 3.62 - 3.60 (m, 2H), 3.55 (t, J = 5.6 Hz, 2H), 3.37 (q, J = 5.6 Hz, 2H), 2.13 (s, 3H), 1.61 (d, J = 6.8 Hz, 3H).

### (3) step 3

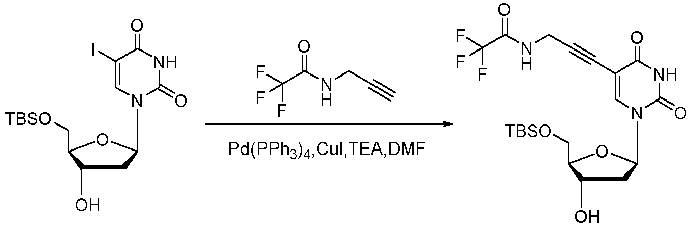

1g of iodo-U nucleoside substrate (OKeanos Tech, cat. No. OK-N-16001) was dissolved in DMF, added with Pd(PPh₃)₄ (10 mol%), Cul (15 mol%), triethylamine (3 eq) and substrate propargylamine (OKeanos Tech, cat. No. OK20A410) (1.5 eq). The reaction was allowed to proceed at 60°C for 12h, quenched by adding water, and then extracted with DCM. After concentration, the residue was subjected to column separation, to provide a white solid product 1.1 g. MS[ES(-)], m/z 491.1. ¹H NMR (400 MHz, DMSO-d6) δ 11.67 (s, 1H), 10.00 (t, J = 5.5 Hz, 1H), 7.94 (s, 1H), 6.12 (dd, J = 7.6, 5.9 Hz, 1H), 5.28 (d, J = 4.1 Hz, 1H), 4.26 - 4.12 (m, 3H), 3.88 (q, J = 2.8 Hz, 1H), 3.81 (dd, J = 11.5, 2.6 Hz, 1H), 3.73 (dd, J = 11.5, 3.1 Hz, 1H), 2.17 (ddd, J = 13.2, 6.0, 2.8 Hz, 1H), 2.05 (ddd, J = 13.3, 7.7, 5.8 Hz, 1H), 0.87 (s, 9H), 0.08 (d, J = 1.8 Hz, 6H).

### (4) step 4

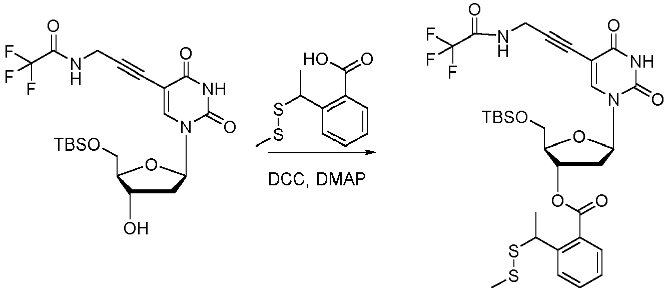

300mg of the substrate nucleoside obtained in the above step 3 was dissolved in 10mL of DMF, added with DCC (1.2 eq) and DMAP (10% mol). The reaction mixture was stirred for 30 minutes, added with disulfide carboxylic acid substrate (OKeanos Tech, cat. No. OK20A420) (1.5 eq). The reaction mixture was stirred for 12 hours, and then directly separated by column chromatography, to provide a white solid 359mg. MS[ES(-)], m/z 700.3. ¹H NMR (400 MHz, DMSO-*d₆*) δ 11.74 (s, 1H), 10.03 (t, J = 5.5 Hz, 1H), 7.98 (s, 1H), 7.90 - 7.82 (m, 1H), 7.68 - 7.58 (m, 2H), 7.47 - 7.40 (m, 1H), 6.23 - 6.18 (m, 1H), 5.45 - 5.42 (m, 1H), 5.20 - 5.12 (m, 1H), 4.38 - 4.16 (m, 3H), 3.98 - 3.87 (m, 2H), 2.60 - 2.53 (m, 1H), 2.40 - 2.31 (m, 1H), 2.06 (d, J = 0.6 Hz, 3H), 1.65 (dd, J = 7.0, 1.0 Hz, 3H), 0.90 (s, 9H), 0.13 (d, J = 1.2 Hz, 6H).

### (5) step 5

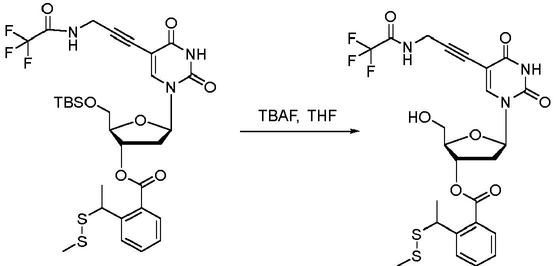

300mg of the substrate nucleoside obtained in the above step 4 was dissolved in 10mL of THF, added at 0°C with TBAF (2 eq, 1M in THF). The reaction mixture was stirred at 0°C for 30 minutes, warmed to room temperature and stirred for 4 hours. The reaction mixture was directly separated by column chromatography, to provide a white solid 200mg. MS[ES(-)], m/z 587.2. ¹H NMR (400 MHz, DMSO-*d₆*) δ 11.68 (d, J = 3.2 Hz, 1H), 10.06 (t, J = 5.6 Hz, 1H), 8.23 (d, J = 3.2 Hz, 1H), 7.88 - 7.78 (m, 1H), 7.66 - 7.56 (m, 2H), 7.43 - 7.39 (m, 1H), 6.25 - 6.13 (m, 1H), 5.48 - 5.45 (m, 1H), 5.31 (t, J = 5.2 Hz, 1H), 5.18 - 5.12 (m, 1H), 4.28 - 4.16 (m, 3H), 3.76 - 3.67 (m, 2H), 2.50 - 2.40 (m, 2H), 2.04 (d, J = 9.2 Hz, 3H), 1.68 - 1.59 (m, 3H).

### (6) step 6

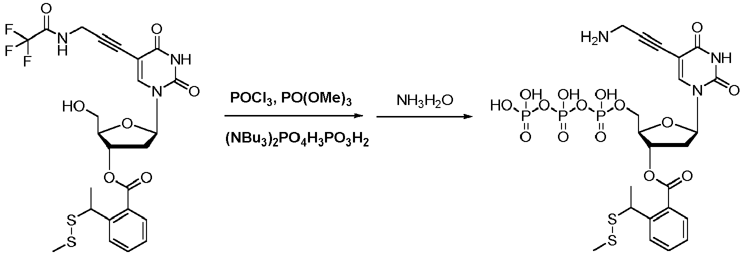

200mg of the substrate nucleoside obtained in the above step 5 was dissolved in 5mL of trimethyl phosphate. After being added with phosphorus oxychloride (1.5 eq) at 0°C, the reaction mixture was stirred at 0°C for 120 minutes, and then added to a solution of tributyl ammonium pyrophosphate (2 eq) in DMF (5 mL). The mixture was continued with stirring at 0°C for 3h, added with 0.1 M TEAB buffer for quenching, and then separated by preparative HPLC reversed phase column chromatography (C18, mobile phase: 0.1 M TEAB-acetonitrile). After concentration, the residue was added with 3 mL of concentrated aqueous ammonia and allowed to react for 2h, and then separated by preparative HPLC reversed phase column chromatography (C18, mobile phase: 0.1 M TEAB-acetonitrile), to provide a white solid 120mg. MS[ES(-)], m/z 745.5. ¹H NMR (400 MHz, D₂O) δ 8.51 (s, 1H), 7.90 (dd, J = 7.9, 1.4 Hz, 1H), 7.73 (d, J = 7.8 Hz, 1H), 7.67 (td, J = 7.6, 1.4 Hz, 1H), 7.48 (t, J = 7.5 Hz, 1H), 6.45 (td, J = 9.2, 5.7 Hz, 1H), 5.75 (t, J = 4.7 Hz, 1H), 5.15 - 5.07 (m, 1H), 4.72 - 4.64 (m, 1H), 4.39 (d, J = 3.3 Hz, 2H), 4.07 (s, 2H), 2.82 - 2.76 (m, 1H), 2.64 - 2.54 (m, 1H), 2.02 (d, J = 6.4 Hz, 3H), 1.73 (dd, J = 7.1, 2.2 Hz, 3H); 31P NMR (162 MHz, D2O) δ -9.79 (dd, J = 20.3, 9.6 Hz, 1P), -11.68 (d, J = 19.2 Hz, 1P), -22.82 (td, J = 19.2, 6.1 Hz, 1P).

### (7) step 7

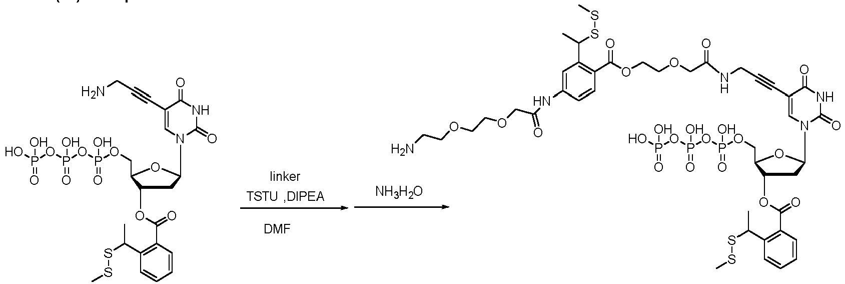

20mg of linker 7 substrate obtained in the above step 2 was dissolved in 1mL of DMF, added with TSTU (1.5 eq) and diisopropylethylamine (3 eq), and then stirred at room temperature for 120minutes. The reaction mixture was added with nucleotide substrate (2 eq) obtained in the above step 6, and continued with stirring at room temperature for 3h, added with 0.1 M TEAB buffer for quenching, and then separated by preparative HPLC reversed phase column chromatography (C18, mobile phase: 0.1 M TEAB-acetonitrile). After concentration, the residue was added with 3 mL of concentrated aqueous ammonia and allowed to react for 2h, and then separated by preparative HPLC reversed phase column chromatography (C18, mobile phase: 0.1 M TEAB-acetonitrile), to provide a white solid 15mg. MS[ES(-)], m/z 1201.7. ¹H NMR (400 MHz, DMSO-d6) δ 10.91 (s, 1H), 8.96 - 8.84 (m, 1H), 8.28 (d, J = 2.0 Hz, 1H), 8.18 (d, J = 11.6 Hz, 1H), 7.92 (dd, J = 8.6, 1.5 Hz, 1H), 7.86 (d, J = 7.1 Hz, 1H), 7.66 (d, J = 8.7 Hz, 1H), 7.63 - 7.57 (m, 2H), 7.46 - 7.38 (m, 1H), 6.21 (ddd, J = 14.8, 8.4, 6.2 Hz, 1H), 5.57 (s, 1H), 5.35 (q, J = 6.8 Hz, 1H), 5.21 - 5.13 (m, 1H), 4.39 (d, J = 12.5 Hz, 2H), 4.32 (s, 2H), 4.22 (s, 2H), 4.14 (d, J = 6.3 Hz, 2H), 4.09 (d, J = 3.7 Hz, 2H), 4.05 (s, 2H), 3.82 - 3.79 (m, 4H), 3.68 - 3.64 (m, 2H), 3.63 - 3.59 (m, 2H), 2.99 - 2.93 (m, 2H), 2.60 - 2.52 (m, 1H), 2.47 - 2.36 (m, 1H), 2.15 (s, 3H), 2.05 (d, J = 21.1 Hz, 3H), 1.64 (d, J = 7.0 Hz, 3H), 1.61 (d, J = 6.9 Hz, 3H).

### (8) step 8

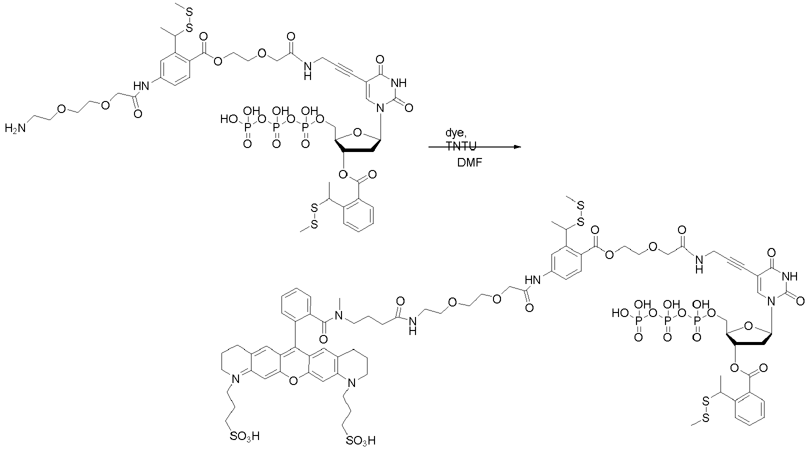

10mg of dye (Beijing Chemdow Co., Ltd, cat. No. CD0014) was dissolved in 0.5 mL of DMF, added with TNTU (1.5 eq) and diisopropylethylamine (3 eq), and then stirred at room temperature for 120 minutes. After being added with the nucleotide substrate obtained in the above step 7 (2 eq), the reaction mixture was continued with stirring at room temperature for 3h, added with 0.1 M TEAB buffer for quenching, and then separated by preparative HPLC reversed phase column chromatography (C18, mobile phase: 0.1 M TEAB-acetonitrile), to provide a red solid 8 mg. HPLC purity > 99%.
MS[ES(-)], m/z 1938.7. ¹H NMR (400 MHz, DMSO-*d₆*) δ 10.39 (brs, 1H), 9.15 - 9.10 (m, 1H), 8.28 (d, J = 8.8 Hz, 1H), 8.03 - 8.00 (m, 1H), 7.88 - 7.79 (m, 3H), 7.76 - 7.67 (m, 3H), 7.66 - 7.60 (m, 3H), 7.58 - 7.39 (m, 3H), 6.99 (s, 2H), 6.80 (s, 2H), 6.27 - 6.20 (m, 1H), 5.59 (d, J = 5.6 Hz, 1H), 5.34 - 5.29 ( m, 1H), 5.18 (p, J = 6.8 Hz, 1H), 4.44 - 4.29 (m, 4H), 4.14 - 4.04 (m, 9H), 3.81 (t, J = 4.8 Hz, 2H), 3.77 - 3.61 (m, 7H), 3.59 - 3.51 (m, 6H), 3.40 (t, J = 6.0 Hz, 2H), 3.19 - 3.14 (m, 2H), 3.11 (t, J = 7.6 Hz, 2H), 2.69 - 2.65 (d, J = 6.5 Hz, 4H), 2.59 - 2.55 (m, 5H), 2.46 - 2.35 (m, 1H), 2.14 (d, J = 3.5 Hz, 3H), 2.08 (s, 1H), 2.04 (s, 1H), 2.00 - 1.91 (m, 4H), 1.87 - 1.80 (m, 4H), 1.70 (t, J = 7.6 Hz, 2H), 1.65 (d, J = 7.2 Hz, 3H), 1.60 (d, J = 6.8 Hz, 3H). 31P NMR (162 MHz, DMSO-d6) δ -11.24 (d, J = 22.7 Hz, 1P), -12.05 (d, J = 22.9 Hz, 1P), -23.63 (t, J = 22.7 Hz, 1P).

### Example 7-2

### Synthesis of linker

The synthesis process and product identification of linker 7 were the same as those of Example 7-1.

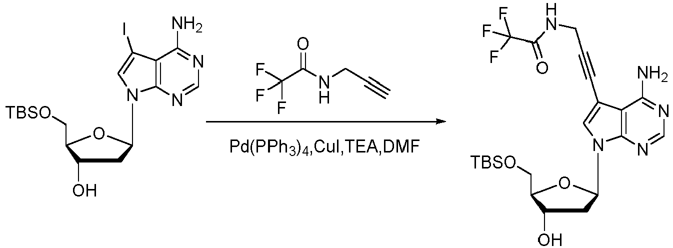

The synthesis process was similar to that of Example 7-1. Iodo-A nucleoside substrate (OKeanos Tech, cat. No. OK-N-16003) was used in place of iodo-U nucleoside substrate. MS[ES(-)], m/z 513.2. ¹H NMR (400 MHz, DMSO-*d₆*) δ 10.04 (t, J = 5.5 Hz, 1H), 8.09 (s, 1H), 7.67 (s, 1H), 6.85 (s, 2H), 6.47 (t, J = 6.7 Hz, 1H), 5.29 (d, J = 4.2 Hz, 1H), 4.33 - 4.29 (m, 1H), 4.27 (d, J = 5.4 Hz, 2H), 3.81 (q, J = 3.9 Hz, 1H), 3.75 (dd, J = 11.1, 4.1 Hz, 1H), 3.67 (dd, J = 11.1, 4.1 Hz, 1H), 2.43 - 2.36 (m, 1H), 2.24 - 2.18 (m, 1H), 0.85 (s, 9H), 0.03 (d, J = 1.8 Hz, 6H).

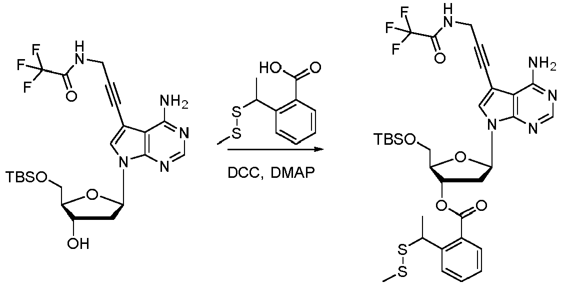

The synthesis process was similar to that of Example 7-1. MS[ES(-)], m/z 723.1. ¹H NMR (400 MHz, DMSO-*d₆*) δ 10.05 (s, 1H), 8.10 (d, J = 4.4 Hz, 1H), 7.91 - 7.83 (m, 1H), 7.74 (d, J = 3.6 Hz, 1H), 7.65 - 7.56 (m, 2H), 7.46 - 7.40 (m, 1H), 6.84 (brs, 2H), 6.64 - 6.48 (m, 1H), 5.58 - 5.52 (m, 1H), 5.24 - 5.10 (m, 1H), 4.37 - 4.15 (m, 3H), 3.95 - 3.74 (m, 2H), 2.84 - 2.75 (m, 1H), 2.70 - 2.54 (m, 1H), 2.06 (d, J = 1.2 Hz, 3H), 1.64 (d, J = 7.0 Hz, 3H), 1.28 - 1.17 (m, 1H), 0.87 (s, 9H), 0.07 (dd, J = 4.8, 2.7 Hz, 6H).

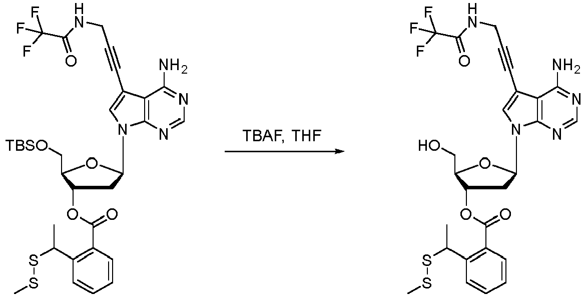

The synthesis process was similar to that of Example 7-1. MS[ES(-)], m/z 609.2. ¹H NMR (400 MHz, DMSO-*d₆*) δ 10.10 (t, J = 5.2 Hz, 1H), 8.12 (d, J = 4.8 Hz, 1H), 7.90 (dd, J = 7.4, 5.0 Hz, 1H), 7.85 (s, 1H), 7.66 - 7.61 (m, 2H), 7.48 - 7.43 (m, 1H), 6.82 (brs, 2H), 6.60 - 6.51 (m, 1H), 5.59 (d, J = 4.4 Hz, 1H), 5.36 (td, J = 5.5, 1.5 Hz, 1H), 5.21 (qd, J = 7.0, 4.1 Hz, 1H), 4.32 (d, J = 5.3 Hz, 2H), 4.26 - 4.20 (m, 1H), 3.75 - 3.64 (m, 2H), 2.90 - 2.77 (m, 1H), 2.65 - 2.51 (m, 1H), 2.09 (d, J = 8.6 Hz, 3H), 1.67 (d, J = 7.0 Hz, 3H).

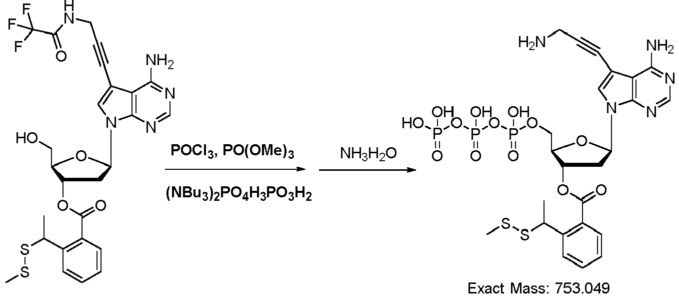

The synthesis process was similar to that of Example 7-1. MS[ES(-)], m/z 753.0. ¹H NMR (400 MHz, D₂O) δ 8.04 (d, J = 7.1 Hz, 1H), 7.95 - 7.83 (m, 2H), 7.72 - 7.56 (m, 2H), 7.49 - 7.44 (m, 1H), 6.70 - 6.48 (m, 1H), 5.71 (dd, J = 17.3, 4.9 Hz, 1H), 5.12 - 5.00 (m, 1H), 4.62 (d, J = 10.7 Hz, 1H), 4.41 - 4.21 (m, 2H), 4.18 (d, J = 8.9 Hz, 2H), 2.78 - 2.31 (m, 2H), 2.03 - 1.83 (m, 3H), 1.70 (dd, J = 10.3, 7.1 Hz, 3H); ³¹P NMR (162 MHz, D₂O) δ -7.18 (dd, J = 20.1, 13.4 Hz 1P), -11.24 (d, J = 19.0 Hz 1P), -22.25 (td, J = 19.6, 11.0 Hz, 1P).

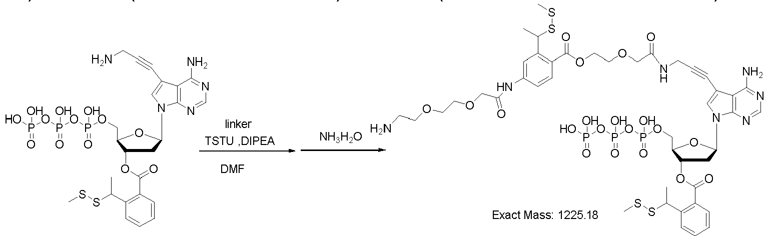

The synthesis process was similar to that of Example 7-1. MS[ES(-)], m/z 1225.1. ¹H NMR (400 MHz, DMSO-*d₆*) δ 11.18 (d, *J* = 6.1 Hz, 1H), 8.37 (t, *J* = 5.4 Hz, 1H), 8.21 (dd, *J* = 7.4, 1.7 Hz, 1H), 8.09 (s, 1H), 7.84 (d, *J* = 8.5 Hz, 1H), 7.69 - 7.58 (m, 3H), 7.47 - 7.40 (m, 1H), 7.29 (s, 1H), 6.40 (s, 2H), 6.30 (dd, J = 9.4, 5.7 Hz, 1H), 5.66 (s, 1H), 5.35 (q, *J* = 6.9 Hz, 1H), 5.20 (q, *J* = 6.9 Hz, 1H), 4.41 (d, *J* = 12.9 Hz, 1H), 4.34 (dd, *J* = 8.4, 4.2 Hz, 1H), 4.30 (s, 2H), 4.13 - 4.08 (m, 2H), 4.06 - 4.03 (m, 2H), 4.01 (s, 2H), 3.83 - 3.80 (m, 2H), 3.67 (d, *J* = 2.8 Hz, 2H), 3.63 - 3.61 (m, 6H), 2.98 - 2.93 (m, 2H), 2.60 - 2.52 (m, 1H), 2.47 - 2.36 (m, 1H), 2.13 (s, 3H), 2.07 (s, 3H), 1.64 (d, *J* = 7.0 Hz, 3H), 1.61 (d, *J* = 6.9 Hz, 3H).

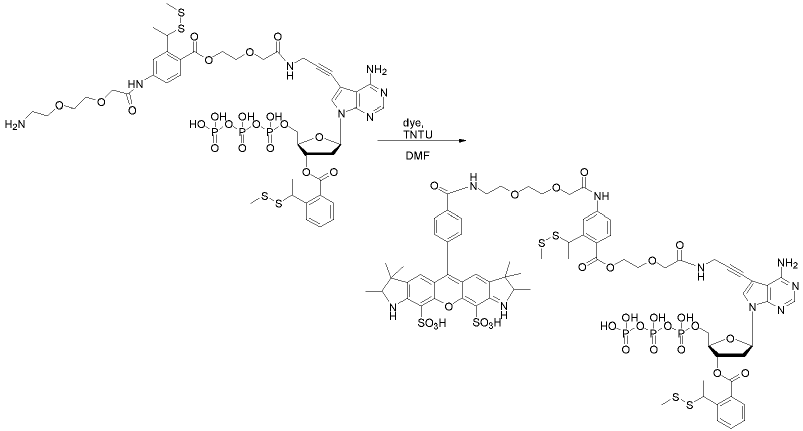

The synthesis process was similar to that of Example 7-1, wherein the dye was replaced by AF532 (commercially available from OKeanos Tech, cat. No. OK F 532). 13mg of solid, HPLC purity > 99%.
MS[ES(-)], m/z 1833.0. ¹H NMR (400 MHz, DMSO-*d₆*) δ 10.43 (t, J = 4.8 Hz, 1H), 8.91 (t, J = 5.6 Hz, 1H), 8.80 (s, 2H), 8.50 (t, J = 5.6 Hz, 1H), 8.15 (d, J = 8.8 Hz, 2H), 8.07 (d, J = 6.4 Hz, 1H), 8.03 (s, 1H), 7.90 (dd, J = 7.6, 2.4 Hz, 1H), 7.84 - 7.79 (m, 2H), 7.69 (d, J = 8.4 Hz, 1H), 7.63 (d, J = 4.4 Hz, 2H), 7.49 - 7.42 (m, 3H), 6.70 (s, 2H), 6.65 - 6.58 (m, 1H), 5.69 (d, J = 2.8 Hz, 1H), 5.33 - 5.27 (m, 1H), 5.21 (qd, J = 7.2, 2.0 Hz, 1H), 4.45 - 4.34 (m, 3H), 4.32 - 4.30 (m, 1H), 4.18 (s, 2H), 4.14 (d, J = 5.6 Hz, 3H), 4.01 (s, 3H), 3.81 - 3.77 (m, 7H), 3.73 - 3.69 (m, 4H), 3.68 - 3.63 (m, 7H), 3.52 (q, J = 5.6 Hz, 4H), 2.12 (d, J = 2.4 Hz, 3H), 2.10 (s, 1H), 2.05 (s, 1H), 1.65 (d, J = 6.8 Hz, 3H), 1.59 (d, J = 7.2 Hz, 3H), 0.99 (d, J = 2.8 Hz, 5H). ³¹P NMR (162 MHz, DMSO-*d₆*) δ -10.99 (d, J = 20.6 Hz, 1P), -12.48 (d, J = 24.0 Hz, 1P), -23.41 (t, J = 22.5 Hz, 1P).

### Example 7-3

### Synthesis of linker

The synthesis process and product identification of linker 7 were the same as those of Example 7-1.

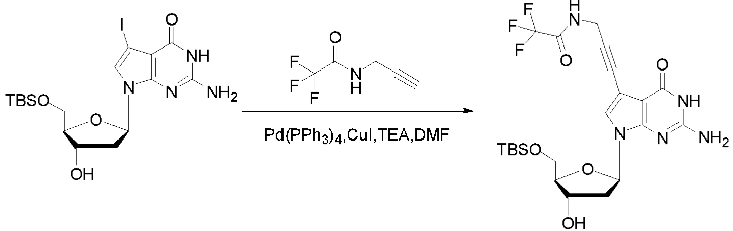

The synthesis process was similar to that of Example 7-1. Iodo-G nucleoside substrate (OKeanos Tech, cat. No. OK-N-16004) was used in place of iodo-U nucleoside substrate. MS[ES(-)], m/z 529.3. ¹H NMR (400 MHz, DMSO-*d*₆) δ 10.48 (s, 1H), 9.98 (t, *J* = 5.6 Hz, 1H), 7.21 (s, 1H), 6.38 - 6.21 (m, 3H), 5.23 (d, *J* = 3.8 Hz, 1H), 4.26 (s, 1H), 4.18 (d, *J* = 5.6 Hz, 2H), 3.78 (q, *J* = 4.0 Hz, 1H), 3.73-3.60 (m, 2H), 2.32-2.19 (m, 1H), 2.13-2.08 (m, 1H), 2.04 (s, 1H), 0.85 (s, 9H), 0.03 (d, *J* = 1.8 Hz, 6H).

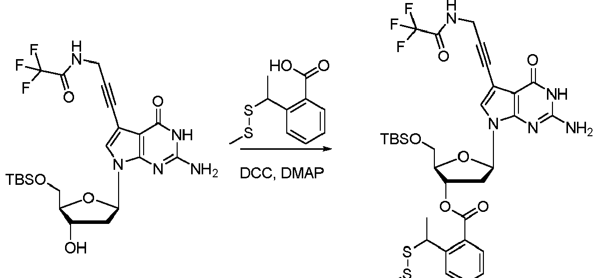

The synthesis process was similar to that of Example 7-1. MS[ES(-)], m/z 739.2. ¹H NMR (400 MHz, DMSO-*d₆*) δ 10.53 (s, 1H), 10.00 (t, J = 5.6 Hz, 1H), 7.83 (dd, J = 7.5, 3.6 Hz, 1H), 7.61 - 7.58 (m, 2H), 7.48 - 7.39 (m, 1H), 7.29 (d, J = 2.0 Hz, 1H), 6.42 - 6.25 (m, 3H), 5.72 (s, 1H), 5.58 - 5.46 (m, 1H), 5.16 (p, J = 7.2 Hz, 1H), 4.25 - 4.14 (m, 3H), 3.82 (t, J = 3.6 Hz, 2H), 2.71 - 2.60 (m, 1H), 2.50 - 2.45 (m, 1H), 2.07 (s, 3H), 1.75 - 1.55 (m, 4H), 1.29 - 0.96 (m, 2H), 0.88 (s, 9H), 0.13 - 0.02 (m, 6H).

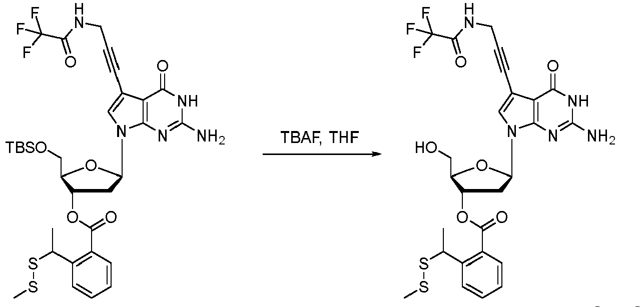

The synthesis process was similar to that of Example 7-1. MS[ES(-)], m/z 625.7. ¹H NMR (400 MHz, DMSO-*d₆*) δ 10.53 (s, 1H), 10.00 (t, J = 5.6 Hz, 1H), 7.83 (dd, J = 7.5, 3.6 Hz, 1H), 7.61 - 7.58 (m, 2H), 7.48 - 7.39 (m, 1H), 7.29 (d, J = 2.0 Hz, 1H), 6.42 - 6.25 (m, 3H), 5.72 (s, 1H), 5.58 - 5.46 (m, 1H), 5.16 (p, J = 7.2 Hz, 1H), 4.25 - 4.14 (m, 3H), 3.82 (t, J = 3.6 Hz, 2H), 2.71 - 2.60 (m, 1H), 2.50 - 2.45 (m, 1H), 2.07 (s, 3H), 1.75 - 1.55 (m, 4H), 1.29 - 0.96 (m, 2H), 0.88 (s, 9H), 0.13 - 0.02 (m, 6H).

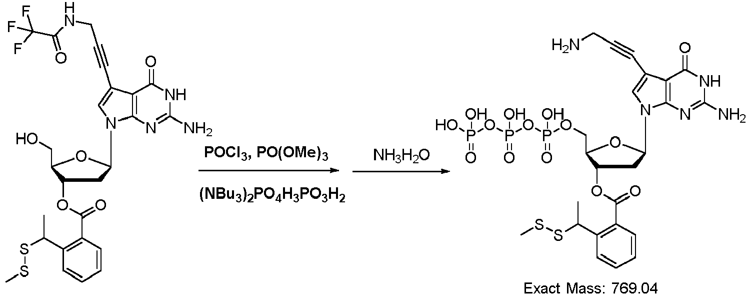

The synthesis process was similar to that of Example 7-1. MS[ES(-)], m/z 769.2. ¹H NMR (400 MHz, D₂O) δ 7.81 - 7.74 (m, 1H), 7.51 - 7.40 (m, 3H), 7.31 (q, J = 6.9 Hz, 1H), 6.38 - 6.19 (m, 1H), 5.65 (dd, J = 11.2, 5.2 Hz, 1H), 5.07 - 4.96 (m, 1H), 4.53 (d, J = 20.8 Hz, 1H), 4.35 - 4.18 (m, 2H), 4.10 (s, 2H), 2.87 - 2.70 (m, 1H), 2.54 - 2.40 (m, 1H), 1.97 - 1.76 (m, 3H), 1.59 - 1.54 (m, 3H); ³¹P NMR (162 MHz, D₂O) δ -9.27 (d, J = 19.6 Hz, 1P), -11.36 (d, J = 18.5 Hz, 1P), -22.79 (td, J = 19.2, 4.7 Hz, 1P).

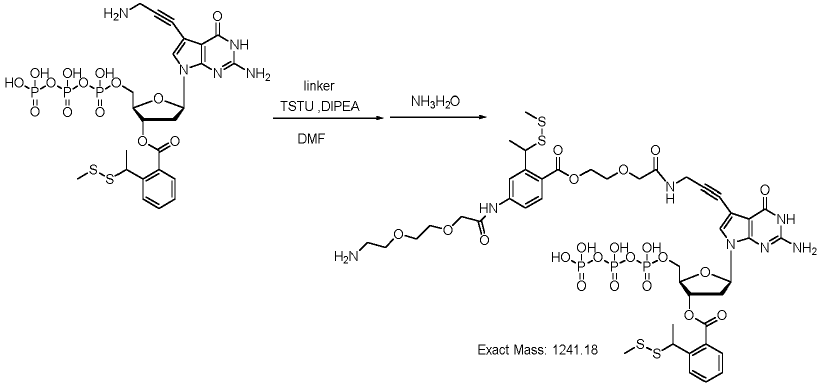

The synthesis process was similar to that of Example 7-1. MS[ES(-)], m/z 1241.6. ¹H NMR (400 MHz, DMSO-*d*₆) δ 11.25 (s, 1H), 8.29 (d, J = 7.7 Hz, 2H), 7.87 (d, J = 7.0 Hz, 2H), 7.69 - 7.58 (m, 3H), 7.47 - 7.40 (m, 1H), 7.29 (s, 1H), 6.40 (s, 2H), 6.30 (dd, J = 9.4, 5.7 Hz, 1H), 5.66 (s, 1H), 5.35 (q, J = 6.9 Hz, 1H), 5.20 (q, J = 6.9 Hz, 1H), 4.41 (d, J = 12.9 Hz, 1H), 4.34 (dd, J = 8.4, 4.2 Hz, 1H), 4.30 (s, 2H), 4.13 - 4.08 (m, 2H), 4.06 - 4.03 (m, 2H), 4.01 (s, 2H), 3.83 - 3.80 (m, 2H), 3.67 (d, J = 2.8 Hz, 2H), 3.63 - 3.61 (m, 6H), 2.98 - 2.93 (m, 2H), 2.47 - 2.42 (m, 1H), 2.41 - 2.30 (m, 1H), 2.13 (s, 3H), 2.07 (s, 3H), 1.66 (d, J = 7.0 Hz, 3H), 1.62 (d, J = 6.9 Hz, 3H).

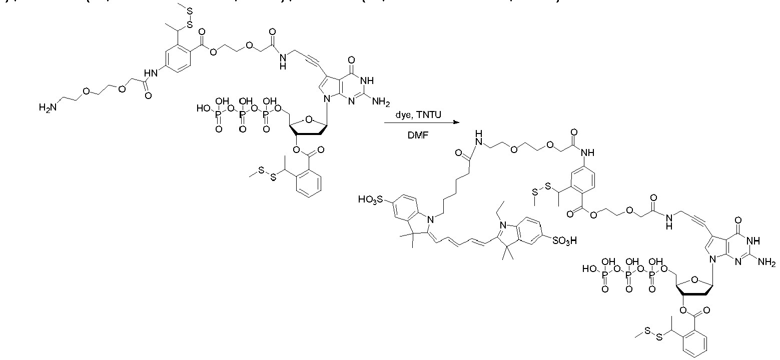

The synthesis process was similar to that of Example 7-1, wherein the dye was replaced by CY5 (commercially available from OKeanos Tech, cat. No. OK-F-13103). 18mg of solid, HPLC purity > 99%.
MS[ES(-)], m/z 1878.9. ¹H NMR (400 MHz, DMSO-*d₆*) δ 10.66 (brs, 1H), 10.43 (s, 1H), 8.43 - 8.32 (m, 2H), 8.03 (s, 1H), 7.92 - 7.82 (m, 2H), 7.82 (d, J = 1.6 Hz, 2H), 7.72 (dd, J = 8.8, 2.0 Hz, 1H), 7.66 - 7.60 (m, 3H), 7.46 - 7.42 (m, 1H), 7.31 (dd, J = 8.0, 3.6 Hz, 2H), 6.59 (t, J = 12.4 Hz, 1H), 6.58 - 6.47 (m, 1H), 6.35 - 6.27 (m, 2H), 5.66 (t, J = 5.2 Hz, 1H), 5.31 (q, J = 7.2 Hz, 1H), 5.20 (q, J = 7.2 Hz, 1H), 4.45 - 4.34 (m, 2H), 4.31 (t, J = 5.2 Hz, 1H), 4.25 (t, J = 5.2 Hz, 1H), 4.14 (s, 2H), 4.12 - 4.05 (m, 5H), 4.00 (s, 2H), 3.81 (t, J = 4.4 Hz, 2H), 3.66 -3.64 (m, 2H), 3.58 -3.55 (m, 2H), 3.41 (t, J = 5.6 Hz, 2H), 3.19 (q, J = 5.6 Hz, 2H), 2.13 (d, J = 3.6 Hz, 3H), 2.08 -2.05 (m, 2H), 1.68 (s, 9H), 1.65 (d, J = 7.2 Hz, 2H), 1.61 (d, J = 7.2 Hz, 2H), 1.56 -1.49 (m, 2H), 1.35 -1.30 (m, 2H), 1.25 (t, J = 7.2 Hz, 3H). ³¹P NMR (162 MHz, DMSO-*d₆*) δ -11.33 (d, J = 22.6 Hz, 1P), -11.92 (d, J = 22.8 Hz, 1P), -23.68 (t, J = 22.6 Hz, 1P).

### Example 7-4

### Synthesis of linker

The synthesis process and product identification of linker 7 were the same as those of Example 7-1.

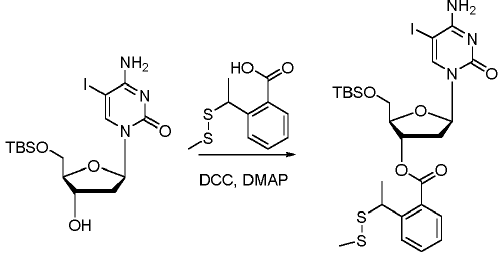

The synthesis process was similar to that of Example 7-1. Iodo-C nucleoside substrate (OKeanos Tech, cat. No. OK-N-16002) was used in place of iodo-U nucleoside substrate. MS[ES(-)], m/z 678.1. ¹H NMR (400 MHz, DMSO-*d*₆) δ 7.97 (s, 1H), 7.94 - 7.79 (m, 2H), 7.65 - 7.54 (m, 2H), 7.46 - 7.33 (m, 1H), 6.68 (s, 1H), 6.18 - 6.14 (m, 1H), 5.40 (d, J = 6.1 Hz, 1H), 5.16 (qd, J = 7.0, 2.1 Hz, 1H), 4.35 - 4.24 (m, 1H), 3.97 - 3.85 (m, 2H), 2.55 - 2.47 (m, 1H), 2.24 - 2.16 (m, 1H), 2.04 (d, *J* = 3.2 Hz, 3H), 1.63 (d, *J* = 7.0 Hz, 3H), 0.89 (s, 9H), 0.12 (s, 6H).

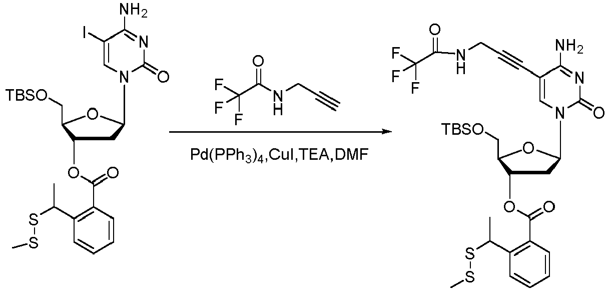

The synthesis process was similar to that of Example 7-1. MS[ES(-)], m/z 700.4. ¹H NMR (400 MHz, DMSO-*d₆*) δ 9.93 (t, J = 5.2 Hz, 1H), 7.99 (s, 1H), 7.91 (s, 1H), 7.85 (d, J = 7.6 Hz, 1H), 7.62 (d, J = 5.3 Hz, 2H), 7.47 - 7.40 (m, 1H), 6.97 (s, 1H), 6.25 - 6.20 (m, 1H), 5.42 (d, J = 5.9 Hz, 1H), 5.17 (qd, J = 7.0, 2.2 Hz, 1H), 4.36 - 4.29 (m, 1H), 4.27 (d, J = 5.2 Hz, 2H), 3.95 - 3.87 (m, 2H), 2.65 - 2.52 (m, 1H), 2.27 - 2.17 (m, 1H), 2.06 (d, J = 2.8 Hz, 3H), 1.65 (d, J = 7.0 Hz, 3H), 0.88 (s, 9H), 0.11 (d, J = 0.8 Hz, 6H).

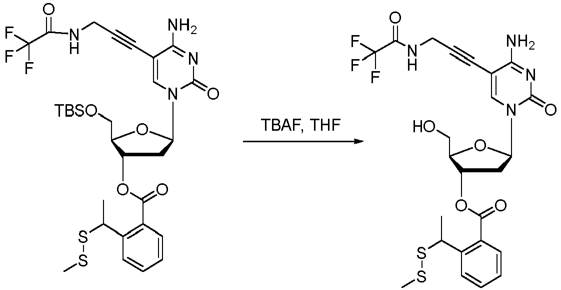

The synthesis process was similar to that of Example 7-1. MS[ES(-)], m/z 580.5. ¹H NMR (400 MHz, DMSO-*d₆*) δ 9.94 (s, 1H), 8.18 (s, 1H), 7.89 (s, 1H), 7.82 (d, J = 7.6 Hz, 1H), 7.61 (q, J = 4.1, 3.0 Hz, 2H), 7.41 (t, J = 8.1 Hz, 1H), 6.91 (s, 1H), 6.26 - 6.17 (m, 1H), 5.45 (d, J = 6.0 Hz, 1H), 5.28 (t, J = 5.2 Hz, 1H), 5.15 (qd, J = 7.0, 3.6 Hz, 1H), 4.35 - 4.16 (m, 3H), 3.70 (dt, J = 5.6, 3.3 Hz, 2H), 2.54 - 2.44 (m, 1H), 2.33 - 2.26 (m, 1H), 2.04 (d, J = 5.7 Hz, 3H), 1.63 (d, J = 7.0 Hz, 3H).

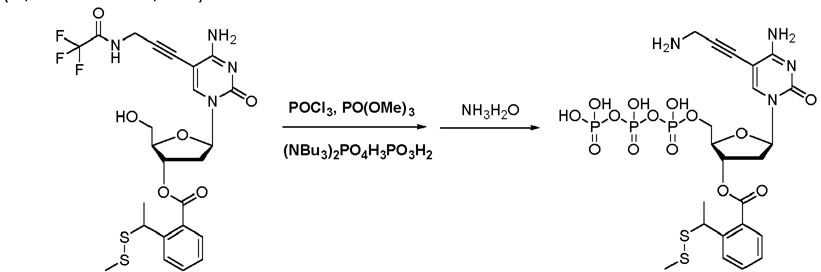

The synthesis process was similar to that of Example 7-1. MS[ES(-)], m/z 745.3. ¹H NMR (400 MHz, D₂O) δ 8.51 (s, 1H), 7.91 (d, J = 7.7 Hz, 1H), 7.74 (d, J = 7.9 Hz, 1H), 7.68 (t, J = 7.7 Hz, 1H), 7.48 (t, J = 7.5 Hz, 1H), 6.47 - 6.42 (m, 1H), 5.73 (d, J = 5.6 Hz, 1H), 5.18 - 5.06 (m, 1H), 4.71 (d, J = 9.0 Hz, 1H), 4.39 (q, J = 12.5 Hz, 2H), 4.09 (d, J = 1.6 Hz, 2H), 2.83 - 2.79 (m, 1H), 2.58 - 2.50 (m, 1H), 2.03 (dd, J = 3.0, 1.5 Hz, 3H), 1.74 (d, J = 7.0 Hz, 3H); ³¹P NMR (162 MHz, D₂O) δ -9.99 (d, J = 19.4 Hz, 1P), -11.68 (d, J = 19.1 Hz, 1P), -22.93 (t, J = 19.4 Hz, 1P).

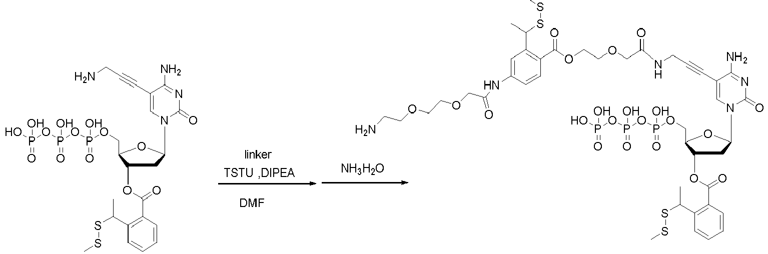

The synthesis process was similar to that of Example 7-1. MS[ES(-)], m/z 1201.6. ¹H NMR (400 MHz, DMSO-*d*₆) δ 10.99 (s, 1H), 8.75 (d, J = 7.2 Hz, 1H), 8.23 (s, 1H), 8.07 (d, J = 7.3 Hz, 1H), 7.87 - 7.84 (m, 3H), 7.70 - 7.58 (m, 3H), 7.47 - 7.37 (m, 1H), 6.95 (s, 1H), 6.23 (dd, J = 16.7, 8.2 Hz, 1H), 5.55 (s, 1H), 5.33 (q, J = 6.8 Hz, 1H), 5.21 - 5.14 (m, 1H), 4.44 - 4.36 (m, 2H), 4.32 (dd, J = 7.8, 4.1 Hz, 2H), 4.24 (s, 2H), 4.13 (d, J = 3.3 Hz, 4H), 4.04 (s,2H), 3.82 (s, 2H), 3.68 - 3.55 (m, 6H), 2.97 (s, 2H), 2.46 - 2.34 (m, 2H), 2.13 (d, J = 2.1 Hz, 3H), 2.05 (d, J = 16.9 Hz, 3H), 1.64 (d, J = 6.7 Hz, 3H), 1.61 (d, J = 6.9 Hz, 3H).

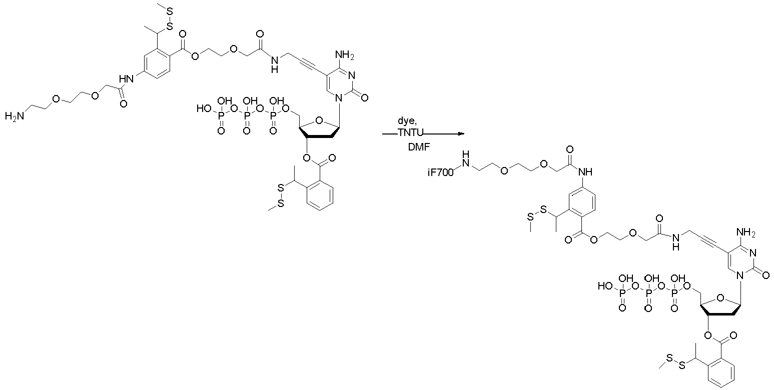

The synthesis process was similar to that of Example 7-1, wherein the dye was replaced by iF700 product (iFluor^{™} 700 succinimidyl ester, commercially available from AAT Bioquest). 16mg of solid, HPLC purity > 99%. MS[ES(-)], m/z 1962.8. ¹H NMR (400 MHz, DMSO-*d₆*) δ 10.30 (s, 1H), 8.91 - 8.84 (m, 1H), 8.80 - 8.74 (m, 1H), 8.41 - 8.35 (m, 3H), 8.25 (t, J = 13.2 Hz, 1H), 8.13 (d, J = 6.4 Hz, 1H), 7.99 (s, 1H), 7.90 - 7.78 (m, 4H), 7.71 (d, J = 8.8 Hz, 1H), 7.65 - 7.60 (m, 2H), 7.53 (dd, J = 8.0, 1.6 Hz, 1H), 7.48 - 7.37 (m, 3H), 6.94 (d, J = 8.8 Hz, 2H), 6.65 (d, J = 8.0z, 2H), 6.54 (t, J = 12.6 Hz, 1H), 6.38 (d, J = 13.6 Hz, 1H), 6.28 - 6.22 (m, 1H), 6.08 (d, J = 13.6 Hz, 1H), 5.55 (s, 1H), 5.31 (q, J = 6.8 Hz, 1H), 5.18 (p, J = 7.2 Hz, 1H), 4.64 (s, 2H), 4.44 - 4.30 (m, 4H), 4.14 - 4.04 (m, 8H), 3.82 (t, J = 4.4 Hz, 2H), 3.68 - 3.63 (m, 3H), 3.60 - 3.43 (m, 8H), 3.39 - 3.31 (m, 4H), 3.17 (s, 3H), 2.56 (t, J = 7.2 Hz, 2H), 2.46 - 2.41 (m, 1H), 2.31 - 2.25 (m, 2H), 2.12 (s, 2H), 2.08 (s, 1H), 2.04 (s, 1H), 1.80 (s, 2H), 1.65 (d, J = 6.8 Hz, 2H), 1.60 (d, J = 7.2 Hz, 2H), 1.47 (s, 5H). ³¹P NMR (162 MHz, DMSO-*d₆*) δ -11.13 (d, J = 22.3 Hz, 1P), -11.90 (d, J = 22.6 Hz, 1P), - 23.56 (t, J = 22.3 Hz, 1P).

### Example 8

### Synthesis of linker

The synthesis process and product identification of linker 7 were the same as those of Example 7-1.

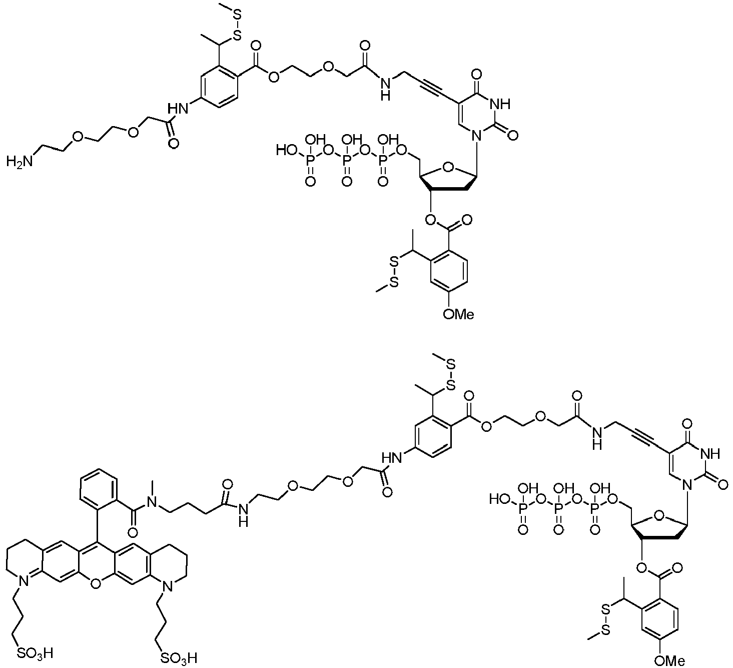

The synthesis process was similar to that of Example 7-1. 15mg of solid, HPLC purity > 99%.
MS[ES(-)], m/z 1968.4. ¹H NMR (400 MHz, DMSO-*d*₆) δ 10.31 (d, J = 18.9 Hz, 1H), 9.00 (s, 1H), 8.23 (d, J = 8.9 Hz, 1H), 8.05 - 7.88 (m, 3H), 7.87 - 7.77 (m, 2H), 7.72 - 7.67 (m, 3H), 7.63 (dd, J = 5.2, 3.6 Hz, 1H), 7.57 - 7.43 (m, 1H), 7.09 (s, 1H), 6.99 (s, 3H), 6.79 (d, J = 5.3 Hz, 2H), 6.27 - 6.20 (m, 1H), 5.51 (s, 1H), 5.34 - 5.28 (m, 2H), 4.44 - 4.36 (m, 2H), 4.32 (d, J = 16.2 Hz, 2H), 4.13 (s, 2H), 4.09 (s, 2H), 4.03 (s, 2H), 3.84 (s, 3H), 3.82 - 3.79 (m, 2H), 3.67 - 3.64 (m, 2H), 3.42 - 3.38 (m,2H), 3.19 - 3.14 (m, 2H), 3.13 - 3.08 (m, 2H), 2.89 (d, J = 3.8 Hz, 2H), 2.70 - 2.64 (m, 4H), 2.59 - 2.53 (m, 6H), 2.13 (s, 5H), 2.08 (d, J = 11.3 Hz, 2H), 1.97 (dt, J = 10.7, 7.0 Hz, 5H), 1.83 (dt, J = 12.2, 6.0 Hz, 4H), 1.71 (t, J = 7.2 Hz, 2H), 1.63 (d, J = 7.0 Hz, 3H), 1.60 (d, J = 6.9 Hz, 3H), 1.23 (s, 3H).

### Example 9

### Synthesis of linker

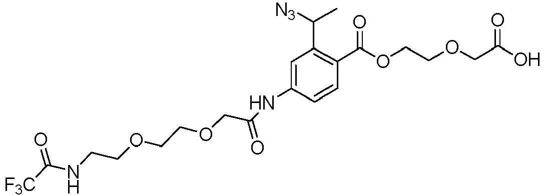

The synthesis process of linker 8 was same as that of Example 7-1. MS[ES(-)], m/z 548.2. ¹H NMR (500 MHz, DMSO-*d*₆) δ 10.08 (s, 1H), 9.51 (s, 1H), 7.94 (d, J = 2.0 Hz, 1H), 7.86 (d, J = 8.6 Hz, 1H), 7.77 (dd, J = 8.6, 2.0 Hz, 1H), 5.70 (q, J = 6.6 Hz, 1H), 4.41 - 4.32 (m, 2H), 4.12 (s, 2H), 3.96 (s, 2H), 3.79 (t, J = 4.6 Hz, 2H), 3.67 (dd, J = 5.7, 3.2 Hz, 2H), 3.61 (dd, J = 5.7, 3.1 Hz, 2H), 3.55 (t, J = 5.7 Hz, 2H), 3.37 (dd, J = 11.3, 5.6 Hz, 2H), 2.07 (s, 1H), 1.44 (d, J = 6.7 Hz 3H).

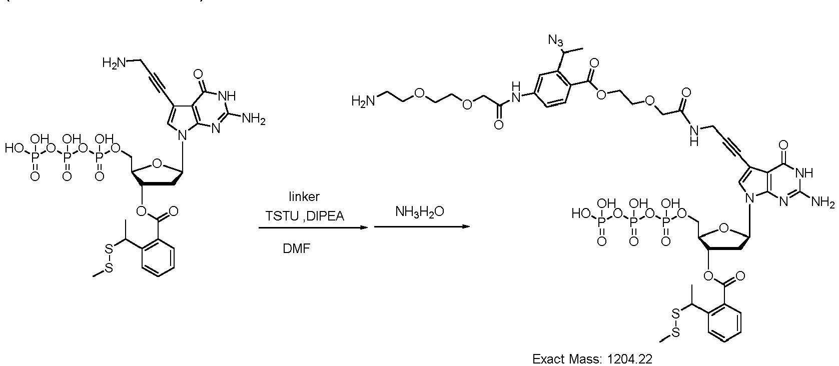

The synthesis process was similar to that of Example 7-3. MS[ES(-)], m/z 1203.2. ¹H NMR (500 MHz, DMSO-*d*₆) δ 11.18 (s, 1H), 8.28 (d, J = 6.3 Hz, 2H), 7.92 (d, J = 8.5 Hz, 1H), 7.87 (d, J = 7.7 Hz, 1H), 7.69 (d, J = 7.1 Hz, 1H), 7.65 - 7.58 (m, 2H), 7.43 (t, J = 5.3 Hz, 1H), 7.27 (d, J = 4.3 Hz, 1H), 6.47 - 6.32 (m, 2H), 6.32 - 6.24 (m, 1H), 5.70 (q, J = 6.5 Hz, 1H), 5.63 (s, 1H), 5.22 - 5.18 (m, 1H), 4.41 - 4.37 (m, 2H), 4.32 (t, J = 4.9 Hz, 1H), 4.28 (s, 2H), 4.26 (s, 1H), 4.11 (d, J = 5.9 Hz, 2H), 4.04 (s, 2H), 4.01 (s, 2H), 3.82 (s, 2H), 3.66 (d, J = 13.7 Hz, 2H), 3.63 - 3.59 (m, 4H), 2.97 (s, 2H), 2.10 (d, J = 32.7 Hz, 3H), 1.65 (d, J = 6.9 Hz, 3H), 1.44 (d, J = 6.6 Hz, 3H).

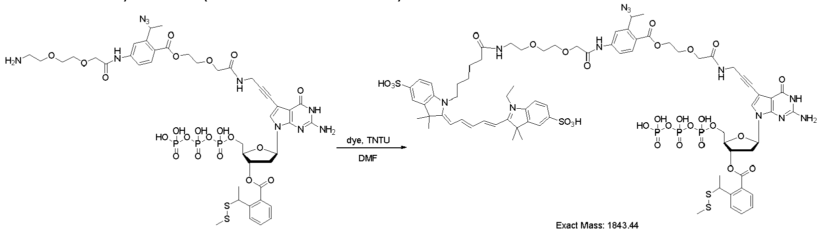

The synthesis process was similar to that of Example 7-3. 20mg of solid, HPLC purity > 99%.
MS[ES(-)], m/z 1842.4. ¹H NMR (500 MHz, DMSO-*d*₆) δ 10.55 (s, 1H), 10.38 (s, 1H), 8.42 (s, 1H), 8.35 (t, J = 13.1 Hz, 2H), 8.03 (s, 1H), 7.88 (dd, J = 10.9, 6.8 Hz, 3H), 7.81 (s, 2H), 7.75 (d, J = 8.8 Hz, 1H), 7.68 - 7.58 (m, 4H), 7.47 - 7.40 (m, 1H), 7.35 - 7.25 (m, 3H), 6.59 (t, J = 12.3 Hz, 1H), 6.40 (s, 2H), 6.30 (dd, J = 13.7, 7.2 Hz, 3H), 5.69 (q, J = 6.6 Hz, 1H), 5.63 (s, 1H), 5.20 (q, J = 6.8 Hz, 1H), 4.44 - 4.36 (m, 2H), 4.32 (t, J = 4.7 Hz, 1H), 4.25 (t, J = 4.4 Hz, 1H), 4.16 - 4.09 (m, 6H), 4.06 (s, 2H), 4.00 (s, 2H), 3.84 - 3.79 (m, 2H), 3.65 (dd, J = 5.4, 3.5 Hz, 2H), 3.59 - 3.55 (m, 2H), 3.43 - 3.39 (m, 2H), 3.19 (dd, J = 11.5, 5.8 Hz, 2H), 2.45 (dd, J = 11.6, 6.5 Hz, 1H), 2.38 (dd, J = 13.6, 5.4 Hz, 1H), 2.10 (d, J = 29.1 Hz, 3H), 2.04 (dd, J = 12.6, 6.9 Hz, 2H), 1.68 (s, 12H), 1.65 (d, J = 7.0 Hz, 3H), 1.56 - 1.49 (m, 2H), 1.43 (d, J = 6.6 Hz, 3H), 1.32 (dd, J = 14.2, 7.8 Hz, 2H), 1.26 (d, J = 7.0 Hz, 3H), 1.23 (s, 2H).

### Example 10-1

### Synthesis of linker

The synthesis process and product identification of linker 7 were the same as those of Example 7-1.

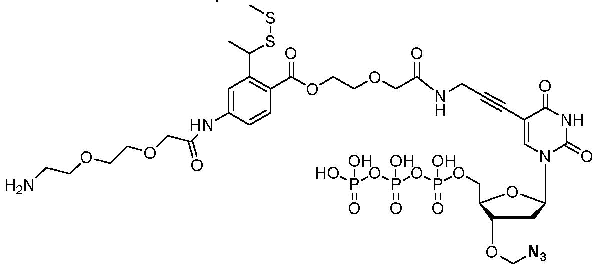

The synthesis process was similar to that of Example 7-1. MS[ES(-)], m/z 1047.2. ¹H NMR (400 MHz, DMSO-*d*₆) δ 11.06 (s, 1H), 8.81 (d, J = 5.8 Hz, 1H), 8.32 (s, 1H), 8.04 (s, 1H), 7.92 (d, J = 8.6 Hz, 1H), 7.65 (d, J = 8.7 Hz, 1H), 6.10 (t, J = 7.0 Hz, 1H), 5.36 (q, J = 6.9 Hz, 1H), 4.92 (d, J = 8.9 Hz, 1H), 4.81 (d, J = 8.9 Hz, 1H), 4.49 - 4.43 (m, 1H), 4.41 - 4.35 (m, 1H), 4.33 - 4.26 (m, 1H), 4.24 (s, 2H), 4.10 (dd, J = 10.1, 3.4 Hz, 2H), 4.04 (s, 2H), 4.00 (dd, J = 11.0, 5.5 Hz, 2H), 3.83 - 3.78 (m, 2H), 3.66 (d, J = 4.8 Hz, 2H), 3.64 - 3.57 (m, 6H), 2.99 - 2.91 (m, 2H), 2.37 - 2.27 (m, 2H), 2.15 (d, J = 1.1 Hz, 3H), 1.61 (d, J = 6.9 Hz, 3H).

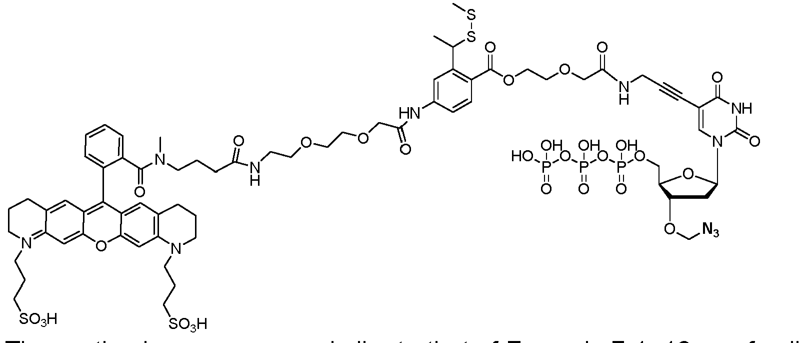

The synthesis process was similar to that of Example 7-1. 16mg of solid, HPLC purity > 99%.
MS[ES(-)], m/z 1783.4. ¹H NMR (400 MHz, DMSO-*d*₆) δ 10.39 (d, J = 18.2 Hz, 1H), 9.00 (t, J = 5.0 Hz, 1H), 8.13 (s, 1H), 8.03 - 7.98 (m, 1H), 7.82 (d, J = 8.6 Hz, 2H), 7.75 - 7.66 (m, 3H), 7.66 - 7.60 (m, 1H), 7.58 - 7.41 (m, 1H), 6.99 (s, 2H), 6.79 (d, J = 5.6 Hz, 2H), 6.11 (t, J = 6.9 Hz, 1H), 5.30 (q, J = 6.9 Hz, 1H), 4.91 (d, J = 8.9 Hz, 1H), 4.83 (d, J = 8.9 Hz, 1H), 4.49 - 4.45 (m, 1H), 4.44 - 4.32 (m, 2H), 4.14 (s, 2H), 4.11 - 4.07 (m, 2H), 4.03 (s, 4H), 3.81 (t, J = 4.4 Hz, 2H), 3.70 (d, J = 7.8 Hz, 2H), 3.68 - 3.63 (m, 2H), 3.56 (dd, J = 9.5, 5.3 Hz, 6H), 3.40 (t, J = 5.9 Hz, 2H), 3.20 - 3.14 (m, 2H), 3.13 - 3.06 (m, 2H), 2.67 (s, 3H), 2.58 (t, J = 7.1 Hz, 4H), 2.40 - 2.26 (m, 2H), 2.13 (s, 3H), 2.02 - 1.91 (m, 4H), 1.84 (d, J = 5.0 Hz, 4H), 1.70 (t, J = 7.2 Hz, 1H), 1.60 (d, J = 6.9 Hz, 3H), 1.28 - 1.16 (m, 3H), 1.01 - 0.92 (m, 1H); ³¹P NMR (162 MHz, DMSO-*d*₆) δ -11.29 (d, J = 23.1 Hz), -12.09 (d, J = 23.0 Hz), -23.70 (t, J = 22.7 Hz).

### Example 10-2

### Synthesis of linker

The synthesis process and product identification of linker 7 were the same as those of Example 7-1.

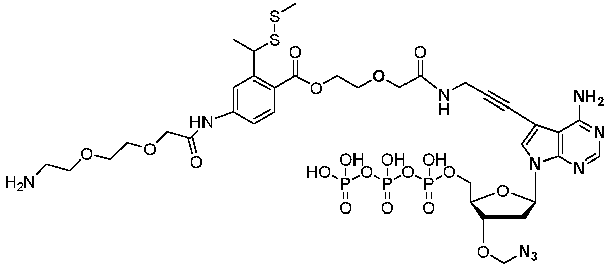

The synthesis process was similar to that of Example 7-2. MS[ES(-)], m/z 1069.2. ¹H NMR (400 MHz, DMSO-*d*₆) δ 11.18 (d, J = 6.1 Hz, 1H), 8.37 (t, J = 5.4 Hz, 1H), 8.21 (dd, J = 7.4, 1.7 Hz, 1H), 8.09 (s, 1H), 7.84 (d, J = 8.5 Hz, 1H), 7.74 (d, J = 2.4 Hz, 1H), 7.68 - 7.60 (m, 1H), 6.50 (dd, J = 8.5, 6.1 Hz, 1H), 5.31 (q, J = 6.9 Hz, 1H), 4.93 (d, J = 8.9 Hz, 1H), 4.87 (d, J = 8.9 Hz, 1H), 4.60 (d, J = 4.3 Hz, 1H), 4.43 - 4.32 (m, 2H), 4.29 (s, 2H), 4.16 - 4.09 (m, 4H), 4.01 (s, 2H), 3.98 - 3.93 (m, 2H), 3.83 - 3.79 (m, 2H), 3.67 (d, J = 3.0 Hz, 2H), 3.62 (d, J = 7.5 Hz, 4H), 2.95 (t, J = 4.9 Hz, 2H), 2.69 - 2.62 (m, 1H), 2.39 (dd, J = 13.0 6.0 Hz, 1H 2.11 (d, J = 5.6 Hz 3H), 1.59 (d, J = 6.8 Hz 3H).

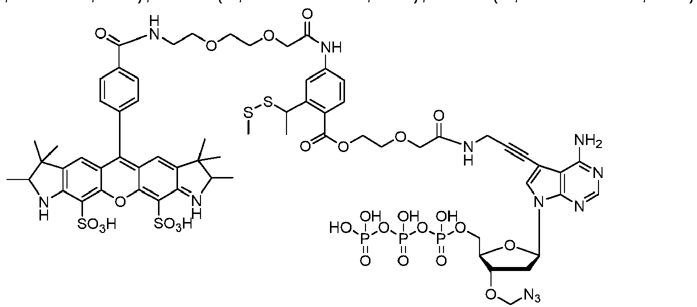

The synthesis process was similar to that of Example 7-2. 18mg of solid, HPLC purity > 99%.
MS[ES(-)], m/z 1678.3. ¹H NMR (400 MHz, DMSO-*d*₆) δ 10.52 (d, J = 4.9 Hz, 1H), 8.94 (t, J = 5.0 Hz, 1H), 8.80 (s, 2H), 8.48 (t, J = 5.3 Hz, 1H), 8.16 (d, J = 8.4 Hz, 2H), 8.08 (s, 1H), 8.04 (s, 1H), 7.82 - 7.74 (m, 2H), 7.70 (d, J = 8.5 Hz, 1H), 7.49 (d, J = 7.9 Hz, 2H), 6.71 (s, 2H), 6.49 (dd, J = 8.7, 5.9 Hz, 1H), 5.29 (q, J = 7.0 Hz, 1H), 4.92 (d, J = 8.9 Hz, 1H), 4.87 (d, J = 8.9 Hz, 1H), 4.60 (d, J = 4.8 Hz, 1H), 4.44 - 4.34 (m, 2H), 4.19 (s, 2H), 4.13 (d, J = 5.4 Hz, 2H), 4.00 (s, 2H), 3.97 - 3.92 (m, 2H), 3.82 - 3.77 (m, 4H), 3.74 - 3.70 (m, 2H), 3.68 - 3.63 (m, 4H), 3.53 (dd, J = 10.6, 5.0 Hz, 2H), 2.68 - 2.63 (m, 1H), 2.39 (dd, J = 13.1, 6.1 Hz, 1H), 2.12 (d, J = 1.4 Hz, 3H), 1.58 (d, J = 6.9 Hz, 3H), 1.16 (d, J = 6.5 Hz, 6H), 1.13 (s, 6H), 0.99 (d, J = 2.0 Hz, 6H); ³¹P NMR (162 MHz, DMSO-*d*₆) δ -11.12 (d, J = 22.8 Hz), -12.12 (d, J = 23.4 Hz), -23.53 (t, J = 22.7 Hz).

### Example 10-3

### Synthesis of linker

The synthesis process and product identification of linker 7 were the same as those of Example 7-1.

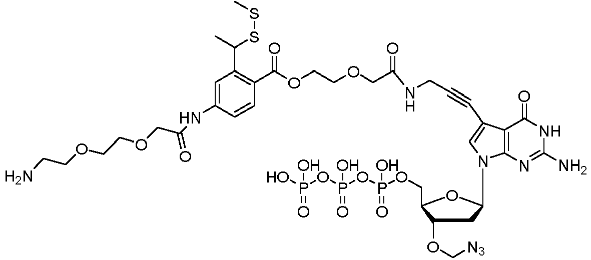

The synthesis process was similar to that of Example 7-3. MS[ES(-)], m/z 1085.2. ¹H NMR (400 MHz, DMSO-*d*₆) δ 11.24 (s, 1H), 8.25 (s, 2H), 7.86 (d, J = 8.5 Hz, 1H), 7.66 (d, J = 8.7 Hz, 1H), 7.18 (d, J = 2.3 Hz, 1H), 6.49 (s, 2H), 6.23 (dd, J = 8.9, 5.8 Hz, 1H), 5.34 (q, J = 6.9 Hz, 1H), 4.91 (d, J = 8.8 Hz, 1H), 4.85 (d, J = 8.8 Hz, 1H), 4.54 (d, J = 3.8 Hz, 1H), 4.42 - 4.31 (m, 2H), 4.29 (s, 2H), 4.12 - 4.06 (m, 2H), 4.00 (s, 2H), 3.92 (s, 2H), 3.84 - 3.78 (m, 2H), 3.66 (s, 2H), 3.61 (d, J = 4.9 Hz, 4H), 2.98 - 2.89 (m, 2H), 2.61 - 2.54 (m, 1H), 2.28 (dd, J = 13.1, 5.1 Hz, 1H), 2.13 (d, J = 1.6 Hz, 3H), 1.61 (d, J = 6.9 Hz, 3H).

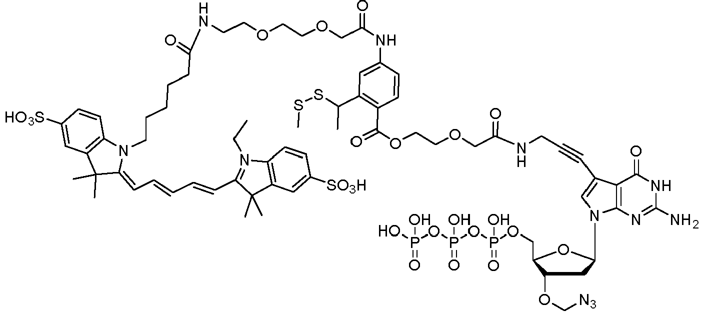

The synthesis process was similar to that of Example 7-3. 14mg of solid, HPLC purity > 99%.
MS[ES(-)], m/z 1724.4. ¹H NMR (400 MHz, DMSO-*d*₆) δ 10.56 (s, 1H), 10.35 (s, 1H), 8.43 - 8.29 (m, 3H), 8.01 (d, J = 1.7 Hz, 1H), 7.89 (t, J = 5.5 Hz, 1H), 7.86 - 7.79 (m, 3H), 7.74 - 7.68 (m, 1H), 7.67 - 7.59 (m, 2H), 7.31 (dd, J = 8.3, 2.9 Hz, 2H), 7.21 (s, 1H), 6.59 (t, J = 12.3 Hz, 1H), 6.47 (s, 2H), 6.30 (dd, J = 13.8, 5.2 Hz, 2H), 6.22 (dd, J = 8.9, 5.8 Hz, 1H), 5.31 (q, J = 6.9 Hz, 1H), 4.91 (d, J = 8.9 Hz, 1H), 4.86 (d, J = 8.9 Hz, 1H), 4.53 (d, J = 4.5 Hz, 1H), 4.44 - 4.35 (m, 2H), 4.16 - 4.09 (m, 4H), 4.09 - 4.04 (m, 2H), 3.99 (s, 2H), 3.97 - 3.88 (m, 2H), 3.83 - 3.79 (m, 2H), 3.65 (dd, J = 5.7, 3.4 Hz, 2H), 3.59 - 3.55 (m, 2H), 3.41 (t, J = 5.8 Hz, 2H), 3.19 (dd, J = 11.6, 5.9 Hz, 2H), 2.62 (dd, J = 14.8, 5.7 Hz, 1H), 2.29 (dd, J = 13.3, 6.0 Hz, 1H), 2.13 (s, 3H), 2.06 (dd, J = 9.4, 5.0 Hz, 2H), 1.60 (d, J = 7.0 Hz, 3H), 1.52 (dt, J = 14.8, 7.3 Hz, 2H), 1.33 (d, J = 6.7 Hz, 2H), 1.25 (t, J = 7.1 Hz, 3H); ³¹P NMR (162 MHz, DMSO-*d*₆) δ -11.06 (d, J = 21.0 Hz), -12.40 (d, J = 23.7 Hz), -23.60 (t, J = 22.2 Hz).

### Example 10-4

### Synthesis of linker

The synthesis process and product identification of linker 7 were the same as those of Example 7-1.

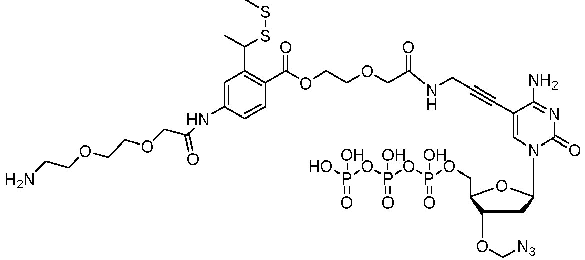

The synthesis process was similar to that of Example 7-4. MS[ES(-)], m/z 1047.2. ¹H NMR (400 MHz, DMSO-*d*₆) δ 11.15 (s, 1H), 8.66 (d, J = 3.9 Hz, 1H), 8.29 (dd, J = 4.5, 1.8 Hz, 1H), 7.94 (s, 1H), 7.88 (d, J = 8.6 Hz, 1H), 7.78 (s, 1H), 7.69 - 7.62 (m, 1H), 6.90 (s, 1H), 6.11 (t, J = 6.3 Hz, 1H), 5.35 (q, J = 6.9 Hz, 1H), 4.91 (d, J = 8.9 Hz, 1H), 4.81 (d, J = 8.9 Hz, 1H), 4.46 - 4.36 (m, 2H), 4.26 (s, 2H), 4.11 (d, J = 3.7 Hz, 4H), 4.04 (s, 2H), 4.02 - 3.91 (m, 2H), 3.84 - 3.78 (m, 2H), 3.69 - 3.64 (m, 2H), 3.64 - 3.56 (m, 4H), 2.94 (t, J = 4.9 Hz, 2H), 2.31 (dd, J = 12.2, 4.5 Hz, 1H), 2.18 - 2.15 (m, 1H), 2.14 (s, 3H), 1.61 (d, J = 6.9 Hz, 3H).

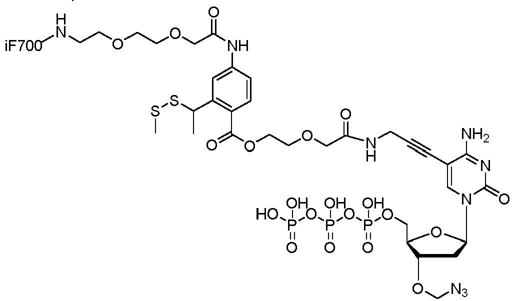

The synthesis process was similar to that of Example 7-4. 20mg of solid, HPLC purity > 99%.
MS[ES(-)], m/z 1807.2. ¹H NMR (400 MHz, DMSO-*d*₆) δ 10.28 (s, 1H), 8.89 (s, 1H), 8.76 (d, J = 5.9 Hz, 2H), 8.39 (d, J = 18.8 Hz, 3H), 8.25 (t, J = 12.6 Hz, 1H), 7.99 (d, J = 9.1 Hz, 2H), 7.87 (s, 1H), 7.82 (d, J = 8.6 Hz, 1H), 7.78 (s, 1H), 7.71 (d, J = 9.7 Hz, 1H), 7.56 - 7.51 (m, 1H), 7.47 (d, J = 8.2 Hz, 2H), 6.99 - 6.87 (m, 2H), 6.65 (d, J = 8.2 Hz, 2H), 6.54 (t, J = 12.6 Hz, 1H), 6.38 (d, J = 12.7 Hz, 1H), 6.10 (t, J = 6.0 Hz, 1H), 5.30 (q, J = 6.9 Hz, 1H), 4.90 (d, J = 8.9 Hz, 1H), 4.82 (d, J = 8.9 Hz, 1H), 4.64 (s, 1H), 4.44 - 4.34 (m, 2H), 4.11 (d, J = 9.2 Hz, 4H), 4.02 (s, 2H), 3.81 (t, J = 4.3 Hz, 2H), 3.66 - 3.62 (m, 2H), 3.59 - 3.52 (m, 4H), 3.52 - 3.46 (m, 2H), 3.35 (dd, J = 11.9, 5.9 Hz, 2H), 3.17 (s, 2H), 2.55 (d, J = 7.2 Hz, 1H), 2.35 - 2.22 (m, 3H), 2.21 - 2.15 (m, 1H), 2.12 (s, 3H), 1.80 (s, 2H), 1.59 (d, J = 6.9 Hz, 3H), 1.47 (s, 5H); ³¹P NMR (162 MHz, DMSO-*d*₆) δ -11.10 (d, J = 21.6 Hz), -12.24 (d, J = 23.1 Hz), -23.61 (t, J = 22.4 Hz).

### Example 11

### (1) Synthesis of linker

The synthesis process and product identification of linker 7 were the same as those of Example 7-1.

### (2) Synthesis of the dye

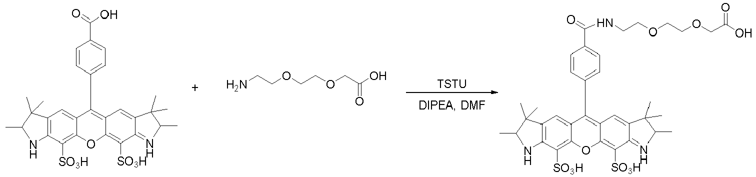

Dye AF532 (63 mg, 0.1 mmol, 1.0 eq) and TSTU (1.5 eq, 45.2 mg) were sequentially added into a 50 mL round bottom flask, and dissolved with adding 3 ml of DMF, and then added with DIPEA (2.5 eq, 52 µL). The reaction mixture was stirred in oil bath at 45°C for 3 h, added with PEG linker 2-(2-(2-aminoethoxy)ethoxy)acetic acid (40 mg, 2.0 eq), and allowed to react at 45°C overnight. The reaction was quenched by adding water and then purified by preparative liquid phase chromatography, to provide the desired product (65.6 mg, 85% yield). The product was identified by MS. MS[ES(-)], m/z 771.2.

### (3) Synthesis of final product

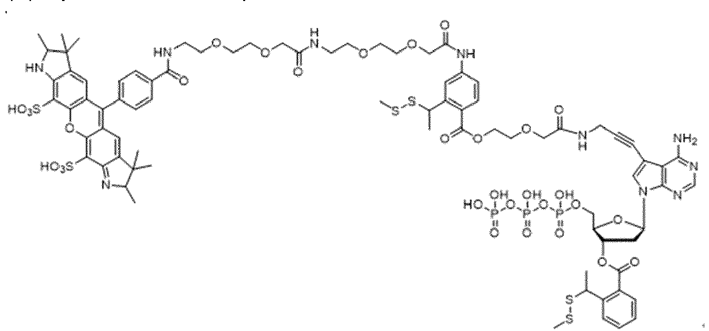

The synthesis process was similar to that of Example 7-2, wherein the dye was replaced by the dye obtained in the above step (2). 21mg of solid, HPLC purity > 99%.
MS[ES(-)], m/z 1978.4. ¹H NMR (400 MHz, DMSO-*d₆*) δ 10.46 (s, 1H), 8.88 (t, J = 5.2 Hz, 1H), 8.80 (s, 2H), 8.52 (t, J = 5.1 Hz, 1H), 8.14 (d, J = 8.4 Hz, 2H), 8.06 (d, J = 6.6 Hz, 1H), 8.04 (s, 1H), 7.90 (dd, J = 7.7, 3.0 Hz, 1H), 7.84 (dd, J = 11.5, 6.6 Hz, 2H), 7.74 (t, J = 5.7 Hz, 1H), 7.69 (d, J = 8.7 Hz, 1H), 7.64 (d, J = 3.8 Hz, 2H), 7.49 (d, J = 8.0 Hz, 2H), 7.47 - 7.41 (m, 1H), 6.70 (s, 2H), 6.66 - 6.56 (m, 1H), 5.70 (d, J = 4.2 Hz, 1H), 5.30 (q, J = 7.0 Hz, 1H), 5.21 (qd, J = 6.9, 2.1 Hz, 1H), 4.45 - 4.38 (m, 2H), 4.36 - 4.28 (m, 2H), 4.14 (s, 4H), 4.09 - 4.05 (m, 2H), 4.02 (s, 2H), 3.89 (s,2H), 3.83 - 3.76 (m, 4H), 3.65 - 3.63 (m, 2H), 3.61 (s, 2H), 3.58 - 3.56 (m, 2H), 3.53 - 3.49 (m, 2H), 3.48 - 3.44 (m, 2H), 3.30 - 3.26 (m, 2H), 2.12 (d, J = 2.0 Hz, 3H), 2.08 (d, J = 19.2 Hz, 3H), 1.66 (d, J = 7.0 Hz, 3H), 1.60 (d, J = 6.9 Hz, 3H), 1.16 (d, J = 6.6 Hz, 6H), 1.13 (s, 6H), 1.00 (s, 6H).

### Example 12

### Synthesis of linker

The synthesis process and product identification of linker 7 were the same as those of Example 7-1.

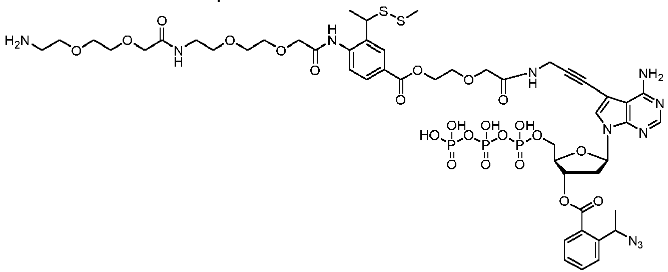

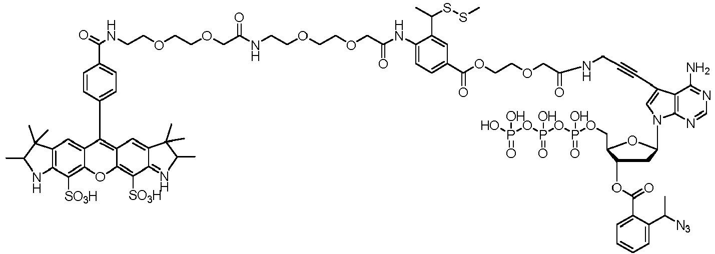

The synthesis process was similar to that of Example 11.

### Example 13

### (1) Synthesis of linker

The synthesis process and product identification of linker 7 were the same as those of Example 7-1.

### (2) Synthesis of the dye

The synthesis process of the dye was similar to that of Example 11.

### (3) Synthesis of final product

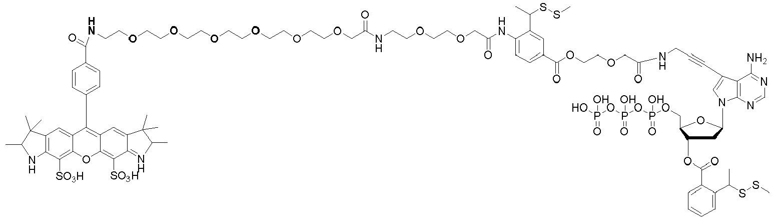

The synthesis process was similar to that of Example 11, wherein the dye was replaced by the dye obtained in the above step (2). 23mg of solid, HPLC purity > 99%.
MS[ES(-)], m/z 2154.5. ¹H NMR (400 MHz, DMSO-*d*₆) δ 8.94 (s, 1H), 8.88 - 8.83 (m, 1H), 8.80 (s, 2H), 8.56 (s, 1H), 8.13 (d, J = 8.4 Hz, 2H), 8.10 - 8.06 (m, 2H), 7.94 - 7.83 (m, 4H), 7.82 (d, J = 4.5 Hz, 1H), 7.63 (d, J = 3.9 Hz, 2H), 7.51 (d, J = 8.2 Hz, 2H), 7.47 - 7.43 (m, 1H), 6.71 (s, 2H), 6.65 - 6.57 (m, 1H), 5.67 (s, 1H), 5.26 - 5.11 (m, 2H), 4.53 - 4.41 (m, 2H), 4.40 - 4.28 (m, 2H), 4.15 (d, J = 5.2 Hz, 2H), 4.12 - 4.05 (m, 2H), 4.02 (s, 4H), 3.83 (dd, J = 8.2, 4.3 Hz, 4H), 3.80 - 3.75 (m, 2H), 3.59 (s, 4H), 3.56 (d, J = 5.9 Hz, 4H), 2.10 (d, J = 7.5 Hz, 5H), 2.06 (s, 2H), 1.66 (d, J = 6.3 Hz, 6H), 1.17 (s, 6H), 0.99 (s, 6H).

### Example 14

### Synthesis of linker

The synthesis process and product identification of linker 7 were the same as those of Example 7-1.

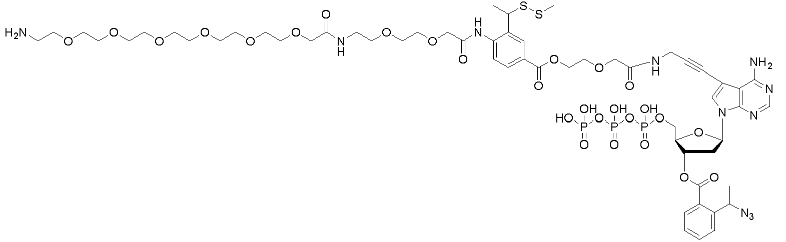

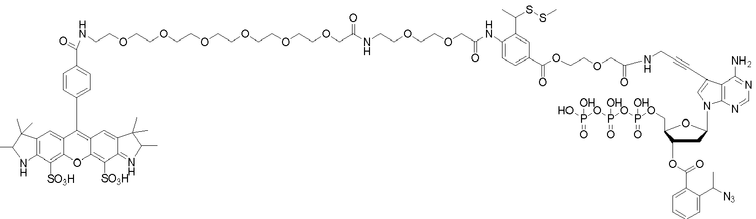

The dye used and the synthesis process of the final product were similar to those of Example 11.

### Example 15-1

### (1) Synthesis of linker

The synthesis process and product identification of linker 7 were the same as those of Example 7-1.

### (2) Synthesis of the dye

The synthesis process of the dye was similar to that of Example 15-2. Dye AF532 was used in place of CY5.

### (3) Synthesis of final product

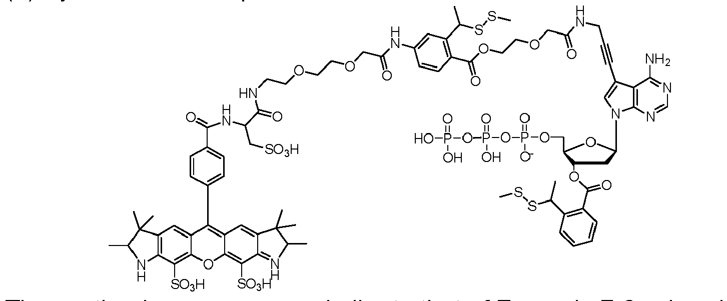

The synthesis process was similar to that of Example 7-2, wherein the dye was replaced by the dye obtained in the above step (2). 18mg of solid, HPLC purity > 99%.

MS[ES(-)], m/z 1983.3. ¹H NMR (400 MHz, DMSO-*d*₆) δ 10.42 (d, J = 144.3 Hz, 1H), 8.97 - 8.80 (m, 2H), 8.50 (d, J = 30.7 Hz, 1H), 8.14 - 8.05 (m, 3H), 7.91 (d, J = 7.8 Hz, 1H), 7.84 (dd, J = 12.3, 8.6 Hz, 2H), 7.75 - 7.60 (m, 3H), 7.55 (d, J = 8.3 Hz, 1H), 7.49 - 7.42 (m, 1H), 7.39 - 7.25 (m, 2H), 6.97 - 6.89 (m, 1H), 6.71 (s, 1H), 6.64 - 6.58 (m, 1H), 5.70 - 5.64 (m, 1H), 5.34 - 5.26 (m, 1H), 5.22 (qd, J = 7.0, 1.8 Hz, 1H), 4.58 - 4.30 (m, 4H), 4.19 (s, 1H), 4.14 (s, 2H), 4.02 (s, 2H), 3.84 - 3.79 (m, 2H), 3.68 - 3.65 (m, 2H), 3.60 (d, J = 4.6 Hz, 2H), 3.27 (d, J = 4.5 Hz, 2H), 3.15 (d, J = 8.9 Hz, 2H), 2.74 (s, 6H), 2.12 (s, 3H), 2.09 (d, J = 19.8 Hz, 3H), 1.67 (d, J = 6.9 Hz, 3H), 1.60 (dd, J = 6.6, 2.4 Hz, 3H), 1.23 (s, 2H), 1.18 - 1.13 (m, 6H), 1.00 (d, J = 1.8 Hz, 3H).

### Example 15-2

### (1) Synthesis of linker

The synthesis process and product identification of linker 7 were the same as those of Example 7-1.

### (2) Synthesis of the dye

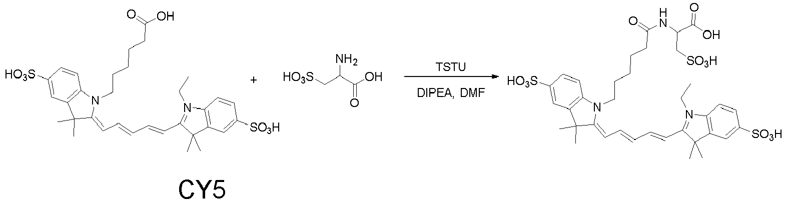

Dye CY5 (250 mg, 0.38 mmol, 1.0 eq) and TSTU (1.2 eq, 110 mg) were sequentially added into a 50 mL dry round bottom flask, and dissolved with adding 5 ml of DMF, and then added with DIPEA (3.0 eq, 200 µL). The reaction mixture was stirred at room temperature for 3 h, added with a compound having the fragment of sulfonic acid functionality (114 mg, 2.0 eq), and stirred at room temperature for 6h. When the reaction was completed, the reaction was quenched by adding water and purified by preparative liquid phase chromatography, to provide the target product (325 mg, yield 89%). The product was identified by MS. MS[ES(-)], m/z 807.2.

### (3) Synthesis of final product

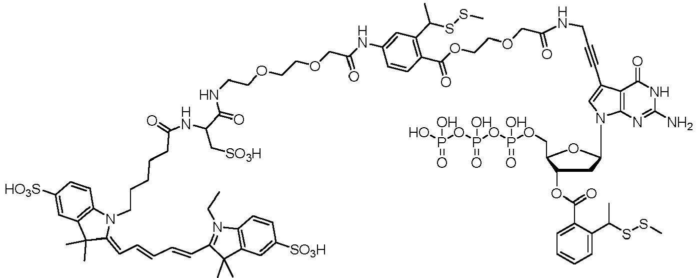

The synthesis process was similar to that of Example 7-3, wherein the dye was replaced by the dye obtained in the above step (2). 25mg of solid, HPLC purity > 99%.

MS[ES(-)], m/z 2030.4. ¹H NMR (400 MHz, DMSO-*d*₆) δ 10.52 (s, 1H), 10.28 (s, 1H), 8.43 (d, J = 2.5 Hz, 1H), 8.34 (t, J = 13.0 Hz, 2H), 8.00 (s, 1H), 7.97 (d, J = 6.3 Hz, 1H), 7.87 (d, J = 7.6 Hz, 1H), 7.86 - 7.81 (m, 2H), 7.80 (s, 2H), 7.73 (dd, J = 8.6, 1.9 Hz, 1H), 7.67 - 7.58 (m, 4H), 7.47 - 7.41 (m, 1H), 7.34 (d, J = 8.4 Hz, 1H), 7.30 (dd, J = 5.8, 2.4 Hz, 2H), 6.70 (t, J = 12.4 Hz, 1H), 6.39 (s, 2H), 6.37 - 6.24 (m, 3H), 5.64 (t, J = 4.0 Hz, 1H), 5.31 (q, J = 6.8 Hz, 1H), 5.24 - 5.16 (m, 1H), 4.42 - 4.34 (m, 3H), 4.33 - 4.23 (m, 2H), 4.13 (s, 2H), 4.12 (d, J = 5.4 Hz, 2H), 4.08 - 4.02 (m, 2H), 4.00 (s, 2H), 3.83 - 3.79 (m, 2H), 3.66 - 3.62 (m, 2H), 3.58 - 3.55 (m, 2H), 3.43 - 3.39 (m, 2H), 3.23 - 3.16 (m, 2H), 2.76 (dd, J = 13.9, 7.9 Hz, 2H), 2.41 - 2.32 (m, 1H), 2.13 (d, J = 1.9 Hz, 5H), 2.08 (s, 1H), 1.66 (d, J = 7.1 Hz, 3H), 1.61 (d, J = 6.9 Hz, 3H), 1.59 - 1.50 (m, 2H), 1.40 (dd, J = 16.1, 9.0 Hz, 2H), 1.25 (t, J = 7.1 Hz, 4H).

### Example 16

### (1) Synthesis of linker

The synthesis process and product identification of linker 7 were the same as those of Example 7-1.

### (2) Synthesis of the dye

The synthesis process of the dye was similar to that of Example 15-2. Dye AF532 was used in place of CY5.

### (3) Synthesis of final product

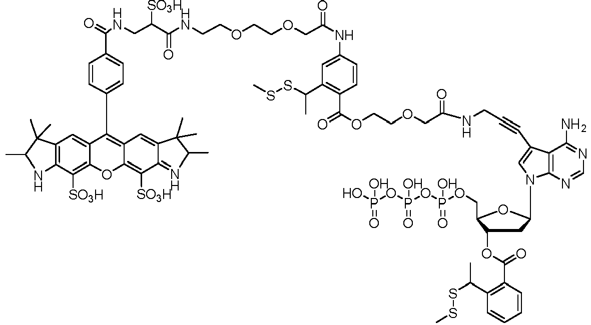

The synthesis process was similar to that of Example 7-2, wherein the dye was replaced by the dye obtained in the above step (2). 22mg of solid, HPLC purity > 99%.
MS[ES(-)], m/z 1984.3. ¹H NMR (400 MHz, DMSO-*d*₆) δ 10.28 (s, 1H), 8.81 (s, 3H), 8.51 (s, 1H), 8.13 (d, J = 8.4 Hz, 2H), 8.08 (d, J = 6.2 Hz, 1H), 8.00 (s, 1H), 7.96 - 7.88 (m, 2H), 7.82 (dd, J = 12.3, 6.3 Hz, 2H), 7.67 (dd, J = 12.5, 6.7 Hz, 2H), 7.64 (s, 2H), 7.52 (d, J = 8.0 Hz, 2H), 7.48 - 7.41 (m, 1H), 6.72 (s, 2H), 6.64 - 6.57 (m, 1H), 5.67 - 5,64 (m, 1H), 5.30 (q, J = 6.4 Hz, 1H), 5.21 (t, J = 6.1 Hz, 1H), 4.47- 4.27 (m, 4H), 4.13 (s, 4H), 4.11 - 4.06 (m, 2H), 4.02 (s, 2H), 3.89 (s, 2H), 3.80 (dd, J = 12.6, 5.7 Hz, 4H), 3.68 - 3.64 (m, 2H), 3.62 (s, 2H), 3.57 (dd, J = 11.3, 5.1 Hz, 2H), 3.49 (s, 4H), 3.47 (d, J = 2.6 Hz, 4H), 3.25 (d, J = 5.7 Hz, 2H), 3.21 (dd, J = 11.6, 5.8 Hz, 2H), 2.69 - 2.65 (m, 1H), 2.57 (dd, J = 5.7, 2.7 Hz, 1H), 2.31 (t, J = 6.5 Hz, 3H), 2.12 (s, 2H), 2.11 (d, J = 3.4 Hz, 3H), 2.07 (s, 2H), 2.06 (s, 2H), 1.66 (d, J = 7.1 Hz, 3H), 1.60 (d, J = 7.1 Hz, 3H), 1.17 (d, J = 6.6 Hz, 6H), 1.14 (s, 6H), 1.01 (d, J = 1.9 Hz, 6H).

### Example 17

### Synthesis of linker

Synthesis process of the linker 9 was similar to that of Example 7-1.

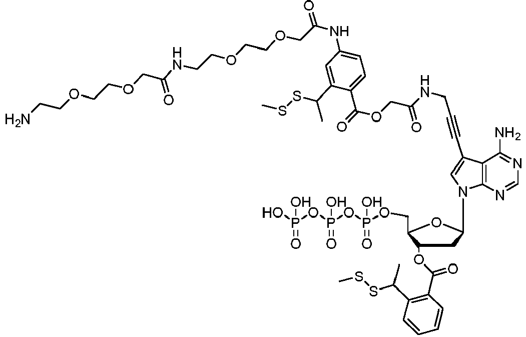

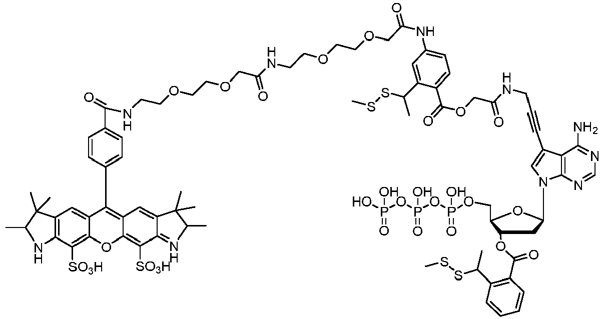

The synthesis process was similar to that of Example 7-2. 25mg of solid, HPLC purity > 99%.
MS[ES(-)], m/z 1934.3. ¹H NMR (400 MHz, DMSO-*d*₆) δ 10.21 (d, J = 3.5 Hz, 1H), 9.05 (d, J = 4.9 Hz, 1H), 8.85 (t, J = 5.5 Hz, 1H), 8.81 (s, 2H), 8.13 (d, J = 8.4 Hz, 2H), 8.09 (d, J = 6.6 Hz, 1H), 7.99 (s, 1H), 7.95 - 7.88 (m, 3H), 7.72 (dd, J = 13.2, 7.5 Hz, 2H), 7.66 - 7.59 (m, 2H), 7.51 - 7.43 (m, 3H), 6.71 (s, 2H), 6.67 - 6.58 (m, 1H), 5.70 (d, J = 3.4 Hz, 1H), 5.30 (q, J = 6.6 Hz, 1H), 5.25 - 5.17 (m, 1H), 4.82 - 4.70 (m, 2H), 4.38 - 4.28 (m, 2H), 4.21 (d, J = 4.3 Hz, 3H), 4.12 (s, 2H), 4.09 - 4.01 (m, 2H), 3.89 (s, 2H), 3.82 - 3.77 (m, 2H), 3.64 (dd, J = 4.7, 2.1 Hz, 2H), 3.61 (s, 2H), 3.58 (dd, J = 5.4, 2.9 Hz, 2H), 3.53 - 3.49 (m, 2H), 3.49 - 3.43 (m, 2H), 3.29 (dd, J = 11.8, 5.9 Hz, 2H), 2.62 - 2.52 (m, 2H), 2.16 (s, 3H), 2.08 (d, J = 17.6 Hz, 3H), 1.66 (d, J = 7.0 Hz, 3H), 1.59 (d, J = 6.9 Hz, 3H), 1.16 (d, J = 6.5 Hz, 6H), 1.14 (d, J = 1.2 Hz, 6H), 1.00 (d, J = 2.1 Hz, 6H).

### Example 18

### Synthesis of linker

Synthesis process of the linker 9 was similar to that of Example 7-1.

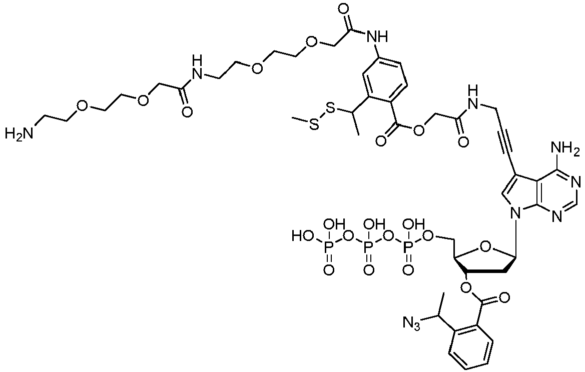

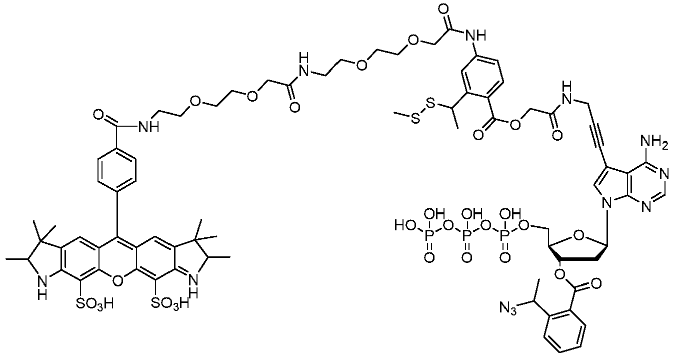

### Example 19

### Synthesis of linker

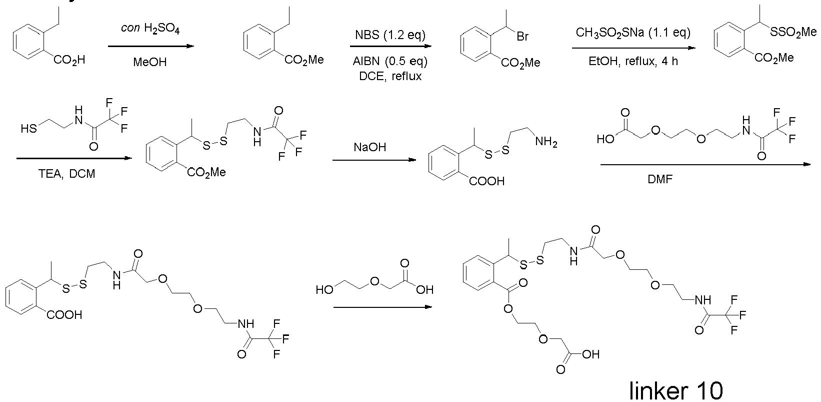

Ethyl benzoic acid was esterified under acid conditions, and then brominated by using NBS to obtain brominated product. The brominated product was reacted with sulfate to produce the intermediate, which was further reacted with the mercaptamine compound to produce the disulfide compound. The disulfide compound was hydrolyzed and condensed with PEG fragment, to provide the linker 10. MS[ES(-)], m/z 599.1. ¹H NMR (500 MHz, DMSO-*d*₆) δ 9.59 (s, 1H), 7.80 (dd, J = 14.3, 6.9 Hz, 2H), 7.59 (q, J = 8.1 Hz, 2H), 7.42 - 7.36 (m, 1H), 5.13 (q, J = 6.9 Hz, 1H), 4.44 - 4.33 (m, 2H), 3.89 (s,2H), 3.85 (s, 2H), 3.81 - 3.75 (m, 2H), 3.61 (s, 2H), 3.55 (s, 4H), 3.51 (t, J = 5.7 Hz, 2H), 3.35 (dd, J = 10.6, 5.2 Hz, 2H), 3.30 - 3.23 (m, 2H), 1.63 (d, J = 7.0 Hz, 3H).

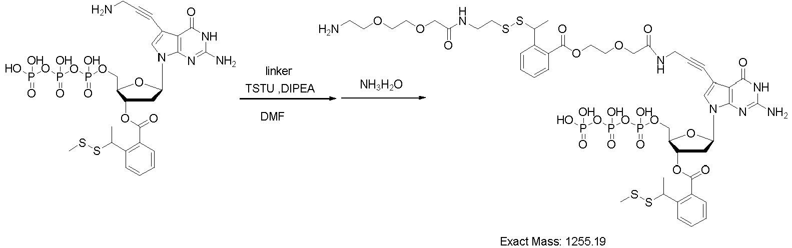

The synthesis process was similar to that of Example 7-3. MS[ES(-)], m/z 1254.2. ¹H NMR (500 MHz, DMSO-*d*₆) δ 8.54 (s, 1H), 7.95 (s, 1H), 7.89 - 7.83 (m, 1H), 7.76 (d, J = 5.1 Hz, 1H), 7.63 (d, J = 6.0 Hz, 2H), 7.58 (d, J = 6.7 Hz, 2H), 7.46 - 7.37 (m, 2H), 7.30 - 7.18 (m, 1H), 6.69 - 6.63 (m, 1H), 6.35 (s, 2H), 5.56 (dd, J = 6.2, 5.2 Hz, 1H), 5.32 (t, J = 4.8 Hz, 1H), 5.20 (dd, J = 9.6, 4.7 Hz, 1H), 5.14 - 5.07 (m, 1H), 4.44 (s, 2H), 4.37 - 4.25 (m, 2H), 4.12 (dd, J = 3.2, 2.3 Hz, 2H), 4.01 (s, 4H), 3.89 - 3.84 (m, 2H), 3.83 (dd, J = 6.8, 4.4 Hz, 2H), 3.63 (d, J = 7.7 Hz, 2H), 3.56 (s, 4H), 3.45 - 3.42 (m, 2H), 2.45 (d, J = 1.9 Hz, 1H), 2.36 (d, J = 0.6 Hz, 1H), 2.02 - 1.96 (m, 6H), 1.66 - 1.60 (m, 6H).

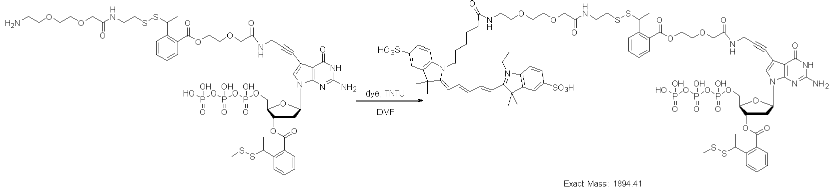

The synthesis process was similar to that of Example 7-3. 16mg of solid, HPLC purity > 99%.
MS[ES(-)], m/z 1893.4. ¹H NMR (500 MHz, DMSO-*d*₆) δ 10.53 (s, 1H), 8.46 - 8.30 (m, 2H), 7.87 (d, J = 6.8 Hz, 2H), 7.81 (s, 2H), 7.79 (d, J = 7.8 Hz, 1H), 7.65 - 7.62 (m, 3H), 7.58 (q, J = 7.8 Hz, 2H), 7.46 - 7.36 (m, 2H), 7.31 (d, J = 8.2 Hz, 2H), 7.29 (s, 1H), 6.59 (t, J = 12.3 Hz, 1H), 6.40 (s, 2H), 6.34 - 6.20 (m, 3H), 5.63 (s, 1H), 5.22 - 5.18 (m, 1H), 5.13 (q, J = 6.9 Hz, 1H), 4.49 - 4.44(m, 1H), 4.41 - 4.37 (m, 1H), 4.32 (s, 1H), 4.26 (s, 1H), 4.12 (d, J = 5.0 Hz, 2H), 4.07 (t, J = 8.0 Hz, 2H), 4.00 (s, 2H), 3.85 (s, 2H), 3.82 (t, J = 4.0 Hz,, 3H), 3.53 (s, 4H), 3.52 (s, 2H), 3.40 - 3.35 (m, 2H), 3.29 - 3.24 (m, 2H), 3.17 (d, J = 5.7 Hz, 2H), 2.13 (s, 1H), 2.06 (d, J = 4.0 Hz, 1H), 1.67 (s, 12H), 1.65 (d, J = 6.9 Hz, 3H), 1.62 (d, J = 6.9 Hz, 3H), 1.56 - 1.49 (m, 2H), 1.35 - 1.29 (m, 2H), 1.26 - 1.23 (m, 6H).

### Example 20-1

### Synthesis of linker

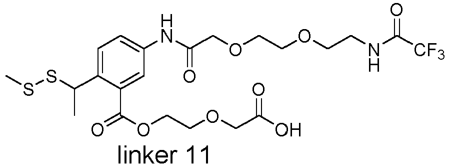

The synthesis process of the linker 11 was similar to that of Example 7-1. MS[ES(-)], m/z 585.1. ¹H NMR (500 MHz, DMSO-*d*₆) δ 9.97 (s, 1H), 9.59 (s, 1H), 8.04 (d, J = 2.1 Hz, 1H), 7.89 (dd, J = 8.6, 2.1 Hz, 1H), 7.55 (d, J = 8.6 Hz, 1H), 5.13 (q, J = 6.9 Hz, 1H), 4.44 - 4.34 (m, 2H), 4.09 (s, 2H), 3.87 (s, 2H), 3.78 (s, 2H), 3.66 (dd, J = 5.7, 3.1 Hz, 2H), 3.61 (dd, J = 5.6, 3.1 Hz, 2H), 3.55 (t, J = 5.7 Hz, 2H), 3.37 (dd, J = 11.0, 5.4 Hz, 2H), 2.10 (s, 3H), 1.62 (d, J = 7.0 Hz, 3H).

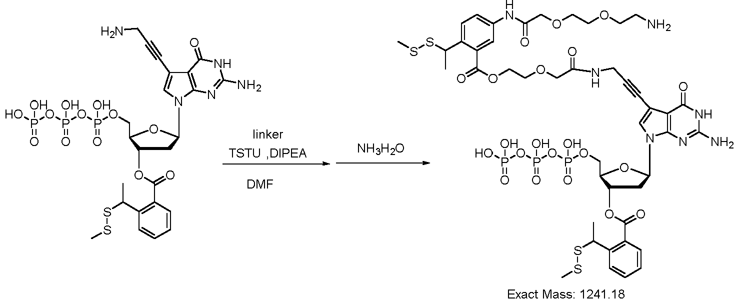

The synthesis process was similar to that of Example 7-3. MS[ES(-)], m/z 1240.2. ¹H NMR (500 MHz, DMSO-*d*₆) δ 10.83 (s, 1H), 8.44 - 8.36 (m, 1H), 8.25 (s, 1H), 8.03 - 7.91 (m, 1H), 7.87 (d, J = 7.8 Hz, 1H), 7.66 - 7.58 (m, 2H), 7.52 (d, J = 8.7 Hz, 1H), 7.48 - 7.40 (m, 1H), 7.33 (d, J = 8.2 Hz, 1H), 7.30 - 7.23 (m, 1H), 7.07 (d, J = 8.3 Hz, 1H), 6.41 - 6.37 (m, 2H), 6.35 - 6.27 (m, 1H), 5.64 (s, 1H), 5.22 - 5.16 (m, 2H), 4.51 - 4.41 (m, 1H), 4.41 - 4.35 (m, 1H), 4.34 (d, J = 4.7 Hz, 1H), 4.26 (s, 1H), 4.25 (s, 2H), 4.15 - 4.10 (m, 2H), 4.05 (s, 4H), 3.82 (s, 2H), 3.68 - 3.58 (m, 6H), 3.55 - 3.51 (m, 2H), 2.63 (t, J = 7.1 Hz, 1H), 2.37 (d, J = 13.0 Hz, 1H), 2.11 (d, J = 18.7 Hz, 3H), 2.08 (s, 3H), 1.66 (d, J = 6.9 Hz, 3H), 1.60 (d, J = 6.9 Hz, 3H).

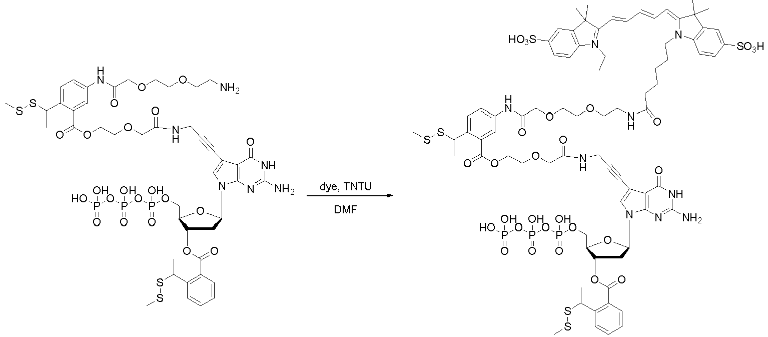

The synthesis process was similar to that of Example 7-3. 18mg of solid, HPLC purity > 99%.
MS[ES(-)], m/z 1779.4. ¹H NMR (500 MHz, DMSO-*d*₆) δ 10.51 (s, 1H), 10.21 (s, 1H), 8.43 - 8.29 (m, 3H), 8.12 (s, 1H), 7.91 (dd, J = 10.0, 3.2 Hz, 2H), 7.87 (d, J = 7.4 Hz, 1H), 7.81 (s, 2H), 7.64 - 7.62 (m, 4H), 7.53 (d, J = 8.6 Hz, 1H), 7.44 (td, J = 7.2, 1.8 Hz, 1H), 7.35 - 7.28 (m, 3H), 7.27 (d, J = 4.8 Hz, 1H), 7.07 (d, J = 8.5 Hz, 1H), 6.59 (t, J = 12.3 Hz, 1H), 6.37 (s, 1H), 6.31 (dd, J = 14.2, 6.9 Hz, 3H), 5.63 (s, 1H), 5.23 - 5.16 (m, 1H), 5.13 (q, J = 7.0 Hz, 1H), 4.48 - 4.39 (m, 2H), 4.33 (t, J = 4.2 Hz, 1H), 4.26 (t, J = 3.5 Hz, 1H), 4.13 (s, 2H), 4.11 (s, 2H), 4.06 (s, 2H), 4.00 (s, 2H), 3.83 - 3.80 (m, 2H), 3.65 - 3.63 (m, 2H), 3.56 (dd, J = 7.8, 3.4 Hz, 2H), 3.53 (d, J = 7.3 Hz, 2H), 3.40 (d, J = 5.4 Hz, 2H), 3.19 (dd, J = 11.3, 5.7 Hz, 2H), 2.63 (t, J = 5.4 Hz, 1H), 2.41 - 2.34 (m, 1H), 2.13 (s, 1H), 2.10 - 2.06 (m, 6H), 1.68 (s, 12H), 1.65 (d, J = 7.0 Hz, 3H), 1.61 (d, J = 6.9 Hz, 3H), 1.54 - 1.49 (m, 2H), 1.32 (dd, J = 12.2, 6.8 Hz, 2H), 1.25 (dd, J = 11.3, 4.5 Hz, 9H).

### Example 20-2

### Synthesis of linker

The synthesis process of the linker was similar to that of Example 20-1.

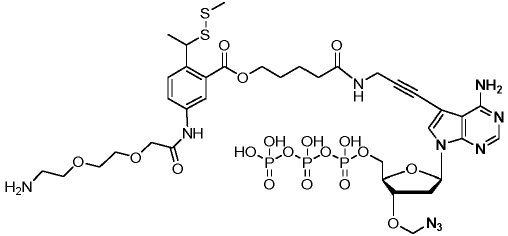

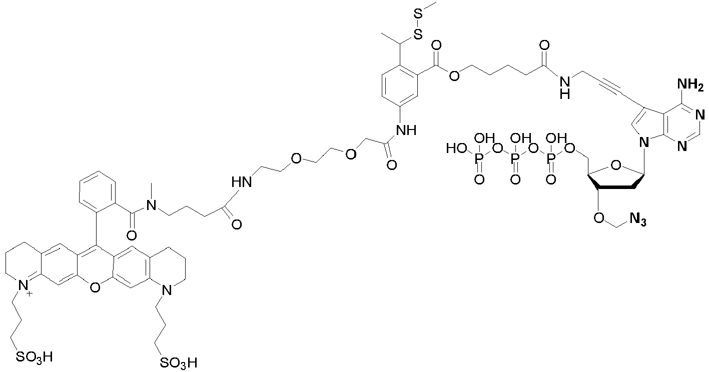

The synthesis process was similar to that of Example 20-1.

### Example 21

### Synthesis of linker

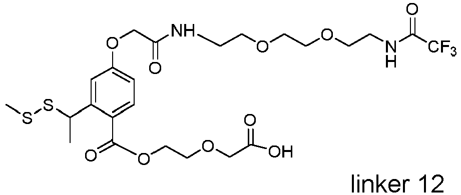

The synthesis process of the linker 12 was similar to that of Example 7-1. MS[ES(-)], m/z 629.2. ¹H NMR (500 MHz, DMSO-*d*₆) δ 9.53 (s, 1H), 8.22 (t, J = 5.7 Hz, 1H), 7.85 (d, J = 8.7 Hz, 1H), 7.14 (d, J = 2.5 Hz, 1H), 6.96 (dd, J = 8.8, 2.5 Hz, 1H), 5.33 (q, J = 6.8 Hz, 1H), 4.63 - 4.53 (m, 2H), 4.40 - 4.30 (m, 2H), 3.94 (s, 2H), 3.79 (t, J = 4.5 Hz, 2H), 3.52 - 3.48 (m, 4H), 3.46 - 3.43 (m, 2H), 3.36 - 3.32 (m, 4H), 3.29 (dd, J = 11.8, 5.9 Hz, 2H), 2.13 (s, 3H), 1.62 (d, J = 7.0 Hz, 3H).

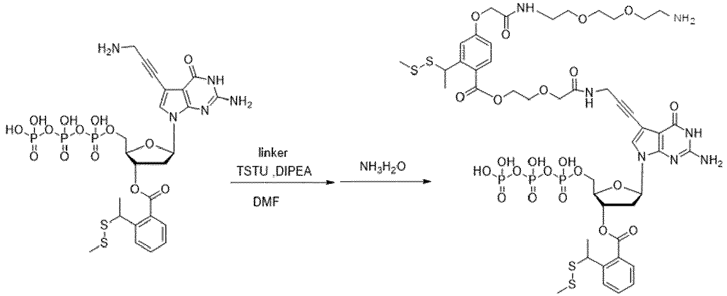

The synthesis process was similar to that of Example 7-3. MS[ES(-)], m/z 1284.2. ¹H NMR (500 MHz, DMSO-*d*₆) δ 8.77 (s, 1H), 8.46 (dd, J = 14.7, 6.2 Hz, 1H), 7.89 (dd, J = 17.9, 8.1 Hz, 2H), 7.66 - 7.60 (m, 2H), 7.47 - 7.41 (m, 1H), 7.30 (d, J = 4.2 Hz, 1H), 7.09 (d, J = 1.9 Hz, 1H), 7.07 - 6.99 (m, 1H), 6.51 - 6.35 (m, 2H), 6.35 - 6.23 (m, 1H), 5.64 (s, 1H), 5.35 (d, J = 6.9 Hz, 1H), 5.20 (dd, J = 6.9, 2.8 Hz, 1H), 4.66 (s, 2H), 4.44 - 4.38 (m, 1H), 4.37 - 4.33 (m, 1H), 4.32 (d, J = 4.8 Hz, 1H), 4.25 (t, J = 4.2 Hz, 1H), 4.11 (d, J = 5.2 Hz, 2H), 4.05 - 4.02 (m, 2H), 4.01 (s, 2H), 3.83 - 3.80 (m, 2H), 3.62 (s, 2H), 3.51 (d, J = 7.5 Hz, 4H), 3.45 (d, J = 5.5 Hz, 4H), 3.29 - 3.25 (m, 2H), 2.49 - 2.43 (m, 1H), 2.41 - 2.36 (m, 1H), 2.10 (d, J = 26.1 Hz, 6H), 1.66 (d, J = 7.0 Hz, 3H), 1.62 (d, J = 6.9 Hz, 3H).

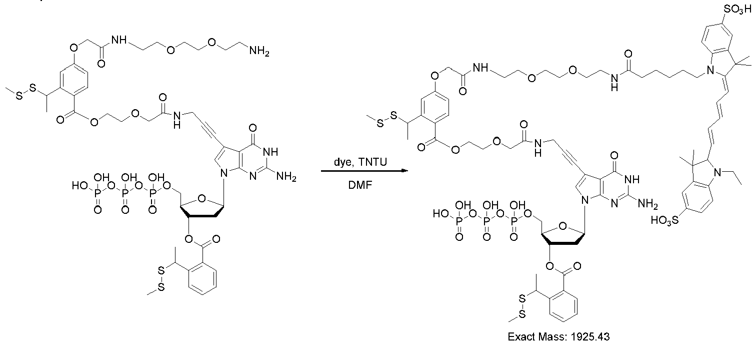

The synthesis process was similar to that of Example 7-3. 21mg of solid, HPLC purity > 99%.
MS[ES(-)], m/z 1924.4. ¹H NMR (500 MHz, DMSO-*d*₆) δ 10.55 (s, 1H), 8.49 (d, J = 6.0 Hz, 1H), 8.36 (t, J = 12.9 Hz, 2H), 7.89 - 7.84 (m, 2H), 7.81 (s, 2H), 7.65 - 7.59 (m, 3H), 7.47 - 7.40 (m, 1H), 7.31 (dd, J = 8.2, 1.4 Hz, 2H), 7.12 (d, J = 2.2 Hz, 1H), 7.01 - 6.94 (m, 1H), 6.59 (t, J = 12.2 Hz, 1H), 6.40 (s, 2H), 6.30 (dd, J = 13.2, 4.5 Hz, 2H), 5.60 (d, J = 58.2 Hz, 1H), 5.33 (q, J = 6.9 Hz, 1H), 5.20 (dd, J = 12.8, 5.8 Hz, 1H), 4.58 (s, 2H), 4.44 - 4.33 (m, 2H), 4.33 - 4.28 (m, 1H), 4.28 - 4.22 (m, 1H), 4.13 (dd, J = 11.7, 5.6 Hz, 2H), 4.09 - 4.05 (m, 2H), 4.00 (s, 2H), 3.82 - 3.79 (m, 2H), 3.48 (s, 2H), 3.45 - 3.42 (m, 2H), 3.38 - 3.34 (m, 2H), 3.28 (dd, J = 11.5, 5.8 Hz, 2H), 3.16 (dd, J = 11.3, 5.5 Hz, 2H), 2.13 (s, 1H), 2.12 (s, 2H), 2.08 - 2.03 (m, 3H), 1.68 (s, 9H), 1.65 (d, J = 6.2 Hz, 3H), 1.61 (d, J = 6.9 Hz, 3H), 1.55 - 1.49 (m, 2H), 1.35 - 1.30 (m, 2H), 1.25 (dd, J = 12.0, 4.4 Hz, 6H).

### Example 22

### Synthesis of linker

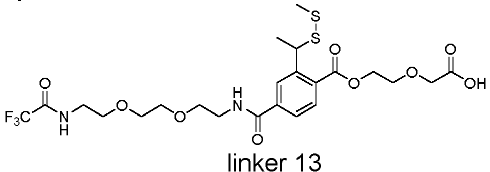

The synthesis process of the linker 13 was similar to that of Example 7-1. MS[ES(-)], m/z 599.1. ¹H NMR (500 MHz, DMSO-*d*₆) δ 9.53 (s, 1H), 8.81 (t, J = 5.5 Hz, 1H), 8.05 (s, 1H), 7.84 (s, 2H), 5.17 (q, J = 7.0 Hz, 1H), 4.50 - 4.31 (m, 2H), 3.84 (s, 2H), 3.80 - 3.77 (m, 2H), 3.55 (d, J = 6.1 Hz, 2H), 3.53 (s, 4H), 3.49 (t, J = 5.8 Hz, 2H), 3.43 (dd, J = 11.5, 5.7 Hz, 2H), 3.32 (dd, J = 10.7, 5.3 Hz, 2H), 2.09 (s, 3H), 1.67 (d, J = 7.0 Hz, 3H).

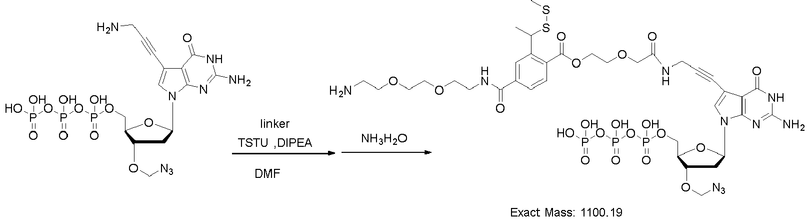

The synthesis process was similar to that of Example 7-3. MS[ES(-)], m/z 1099.2. ¹H NMR (500 MHz, DMSO-*d*₆) δ 9.36 (s, 1H), 8.43 (s, 1H), 8.06 (d, J = 7.9 Hz, 2H), 7.92 (d, J = 8.2 Hz, 1H), 7.20 (s, 1H), 6.45 (s, 2H), 6.23 (dd, J = 8.8, 6.0 Hz, 1H), 5.18 (q, J = 7.1 Hz, 1H), 4.90 (d, J = 8.8 Hz, 1H), 4.85 (d, J = 8.8 Hz, 1H), 4.53 (d, J = 4.0 Hz, 1H), 4.51 - 4.36 (m, 2H), 4.14 - 4.05 (m, 4H), 4.01 (s, 2H), 3.90 (dd, J = 11.1, 6.1 Hz, 2H), 3.85 - 3.80 (m, 2H), 3.63 - 3.58 (m, 6H), 3.54 (s, 4H), 2.62 - 2.54 (m, 1H), 2.28 (dd, J = 12.9, 5.5 Hz, 1H), 2.09 (d, J = 2.8 Hz, 3H), 1.66 (d, J = 7.0 Hz, 3H).

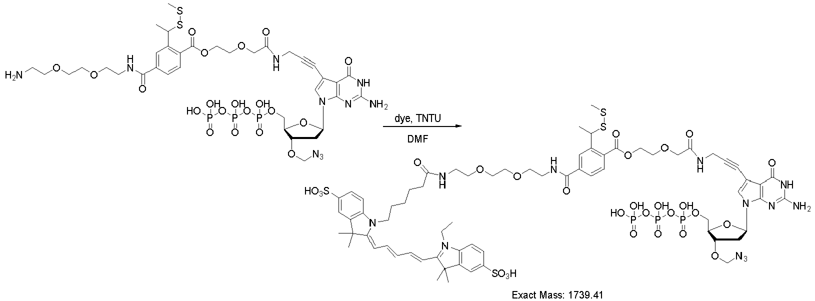

The synthesis process was similar to that of Example 7-3. 13mg of solid, HPLC purity > 99%.
MS[ES(-)], m/z 1738.4. ¹H NMR (500 MHz, DMSO-*d*₆) δ 10.55 (s, 1H), 8.91 (s, 1H), 8.45 (t, J = 5.6 Hz, 1H), 8.35 (t, J = 13.0 Hz, 2H), 8.04 (s, 1H), 7.92 - 7.84 (m, 3H), 7.80 (d, J = 1.7 Hz, 2H), 7.64 (ddd, J = 8.2, 3.7, 1.5 Hz, 2H), 7.31 (dd, J = 8.3, 3.0 Hz, 2H), 7.22 (s, 1H), 6.59 (t, J = 12.3 Hz, 1H), 6.46 (s, 2H), 6.30 (dd, J = 13.7, 4.3 Hz, 2H), 6.21 (dd, J = 8.9, 5.8 Hz, 1H), 5.15 (q, J = 6.9 Hz, 1H), 4.90 (d, J = 8.9 Hz, 1H), 4.85 (d, J = 8.9 Hz, 1H), 4.53 (d, J = 3.9 Hz, 1H), 4.51 - 4.44 (m, 1H), 4.44 - 4.38 (m, 1H), 4.15 - 4.09 (m, 4H), 4.09 - 4.03 (m, 2H), 3.99 (s, 2H), 3.97 - 3.87 (m, 2H), 3.84 - 3.80 (m, 2H), 3.76 - 3.72 (m, 2H), 3.44 - 3.41 (m, 2H), 3.38 - 3.34 (m, 4H), 3.15 (dd, J = 11.6, 5.8 Hz, 2H), 2.91 - 2.88 (m, 2H), 2.66 - 2.60 (m, 1H), 2.28 (dd, J = 13.1, 5.6 Hz, 1H), 2.08 (s, 3H), 2.07 - 2.02 (m, 2H), 1.67 (s, 12H), 1.65 (d, J = 8.0 Hz, 3H), 1.56 - 1.48 (m, 2H), 1.35 - 1.29 (m, 2H), 1.26 - 1.22 (m, 5H).

### Example 23

### Synthesis of linker

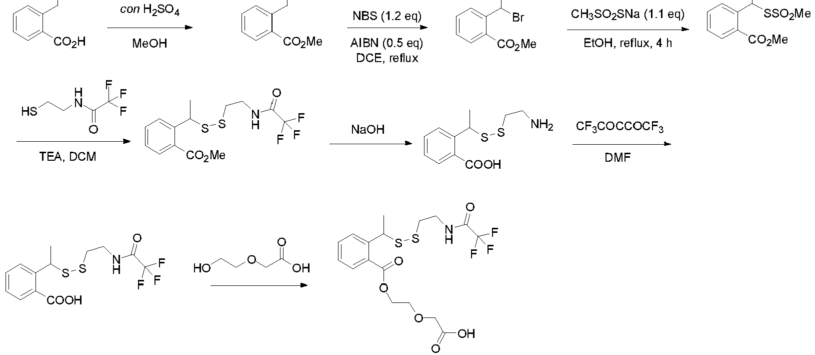

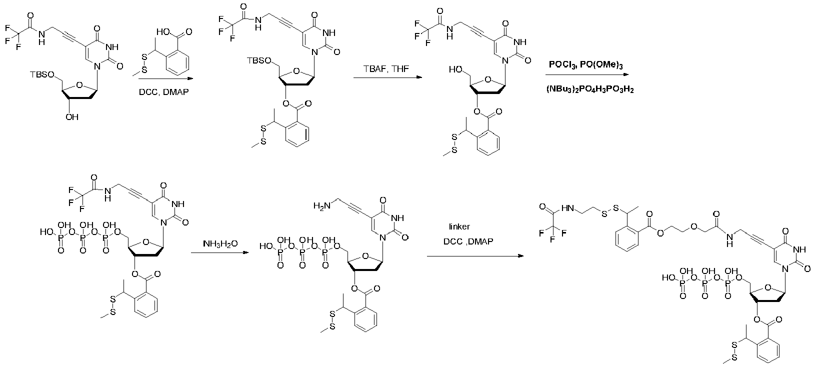

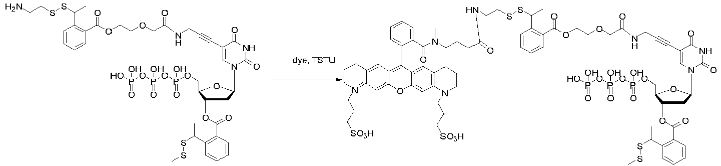

The synthesis process was similar to that of Example 19.

### Example 24

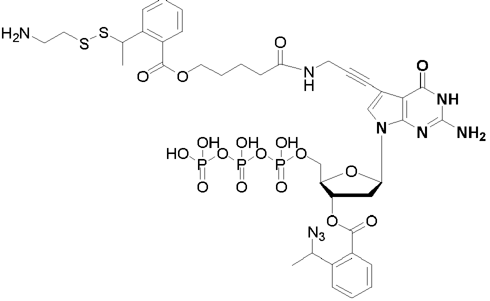

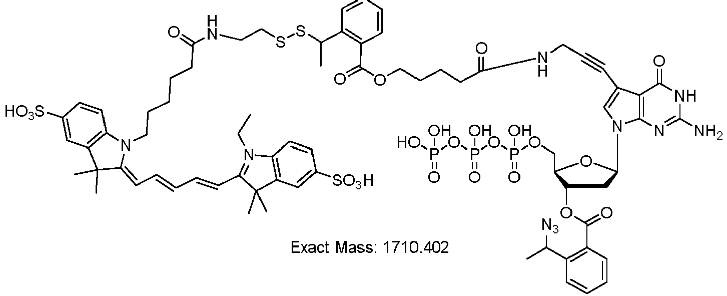

The synthesis process was similar to that of Example 7-3.

### Example 25

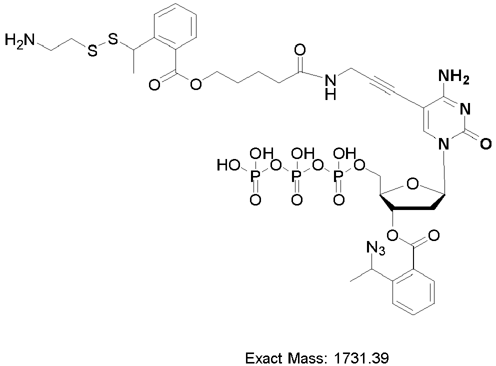

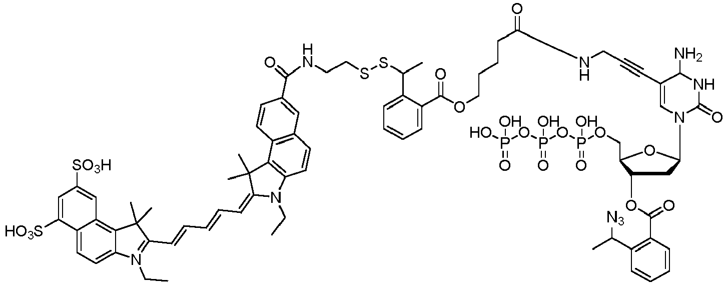

The synthesis process was similar to that of Example 7-3.

### Example 26

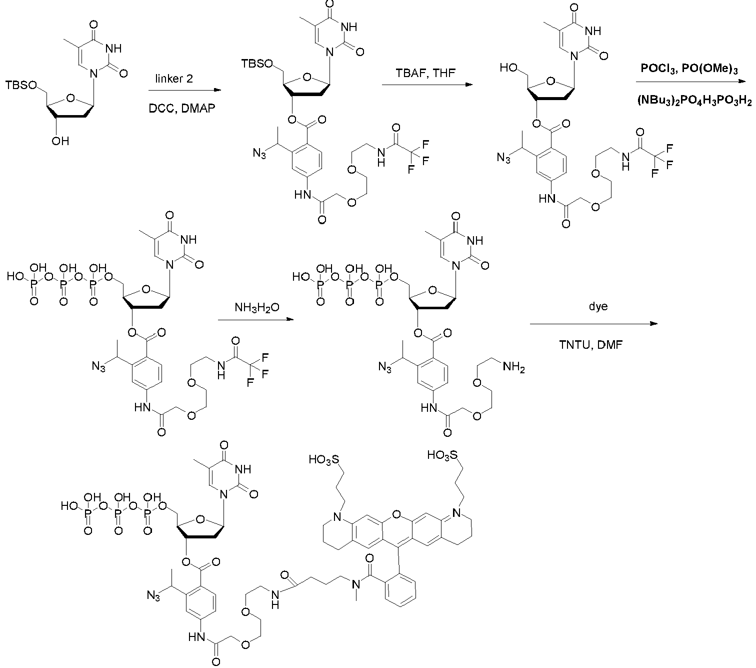

For synthesis process of linker 2, refer to Example 2.

The nucleoside was condensed with the linker 2 in the presence of DCC, to provide the esterified product, which was subjected to removal of TBS protective group, triphosphorylation, and aminolysis, to produce the desired fragment. The coupling of the fragment with the dye led to the target product.

### Example 27

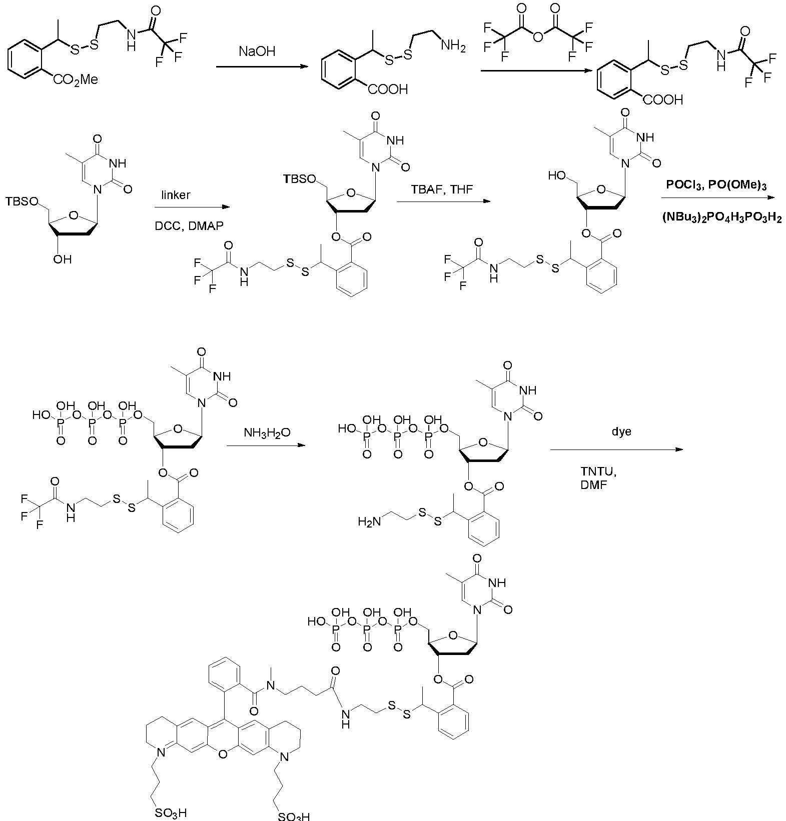

The synthesis process was similar to that of Example 19.

### Example 28

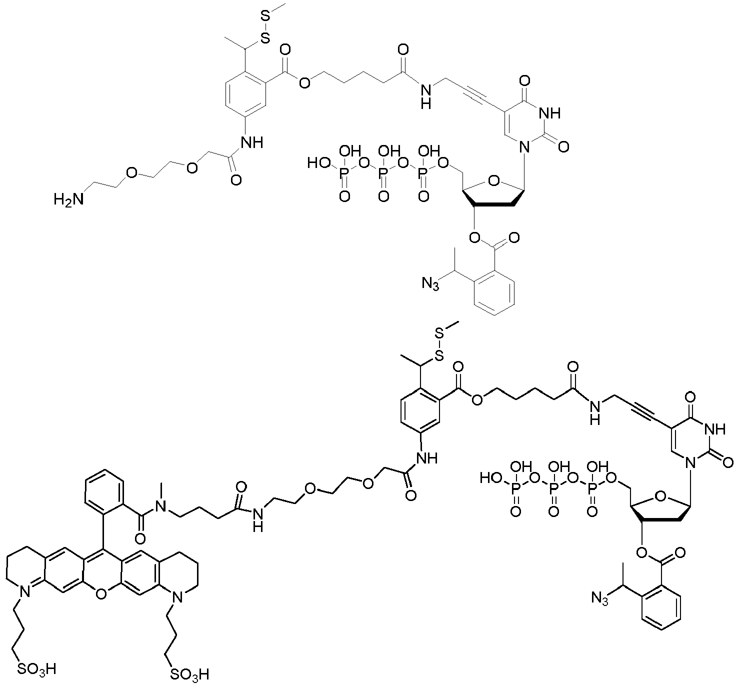

The synthesis process was similar to that of Example 7-1.

### Example 29

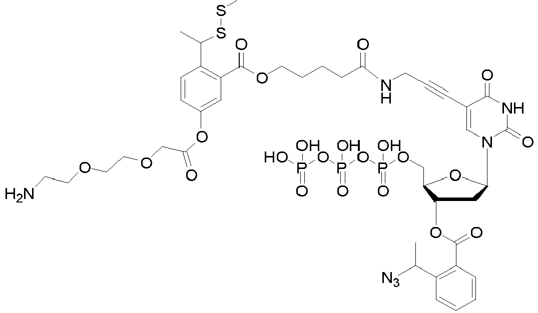

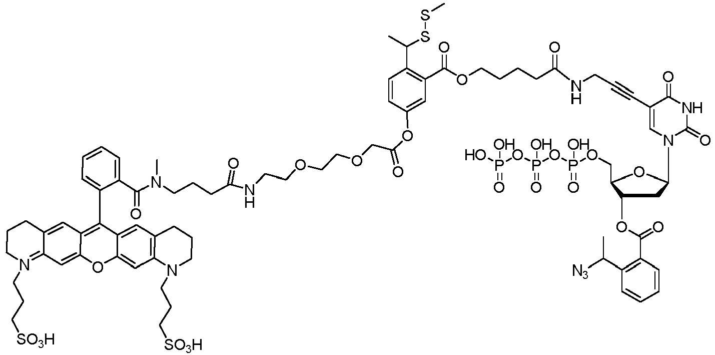

The synthesis process was similar to that of Example 7-1.

### Example 30

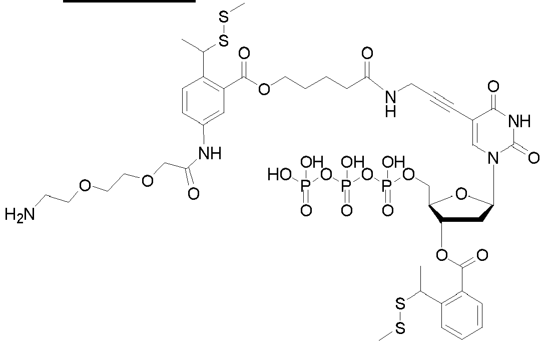

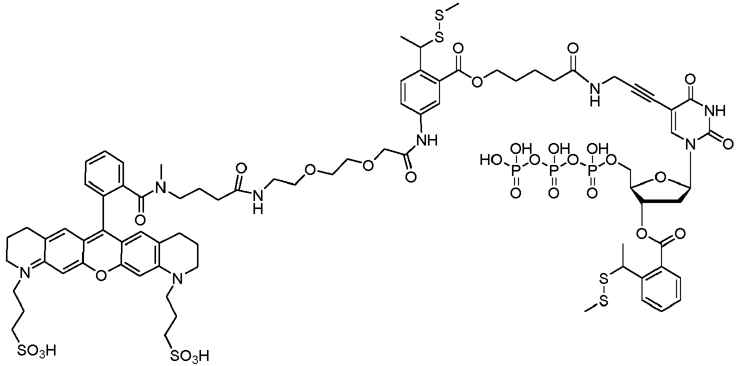

The synthesis process was similar to that of Example 7-1.

### Example 31

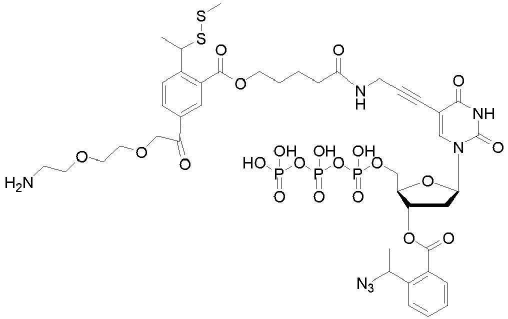

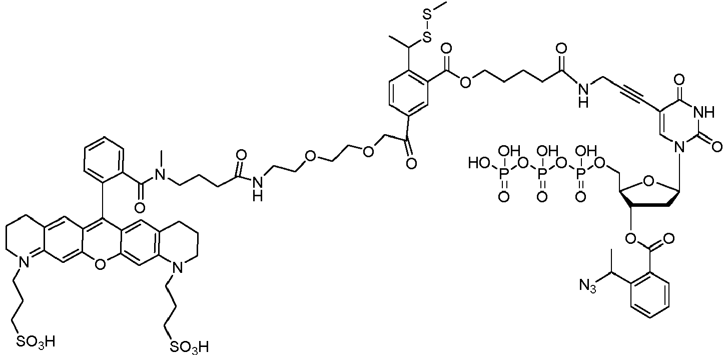

The synthesis process was similar to that of Example 7-1.

### Example 32

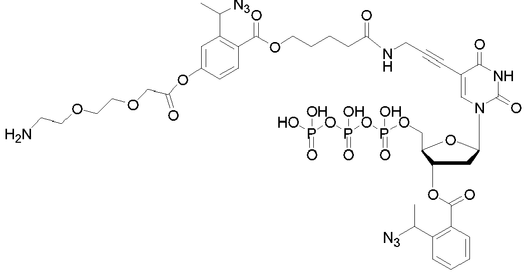

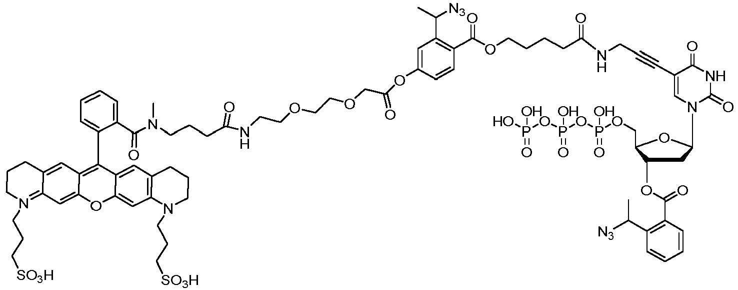

The synthesis process was similar to that of Example 7-1.

### Example 33

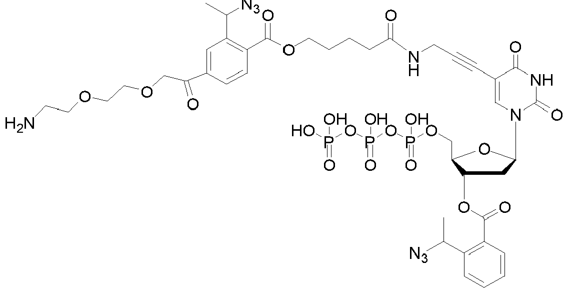

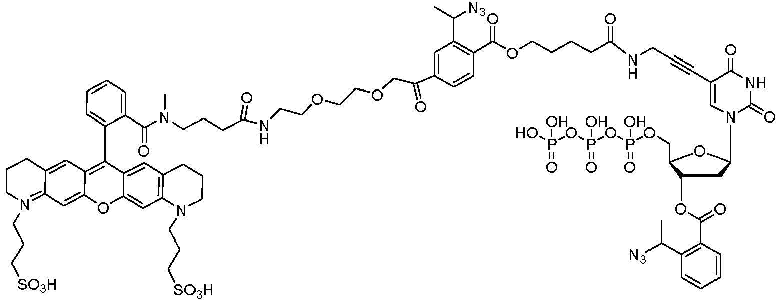

The synthesis process was similar to that of Example 7-1.

### Example 34

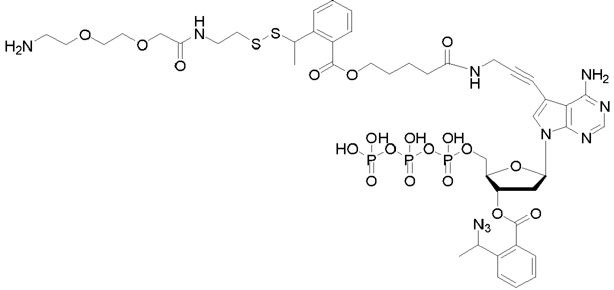

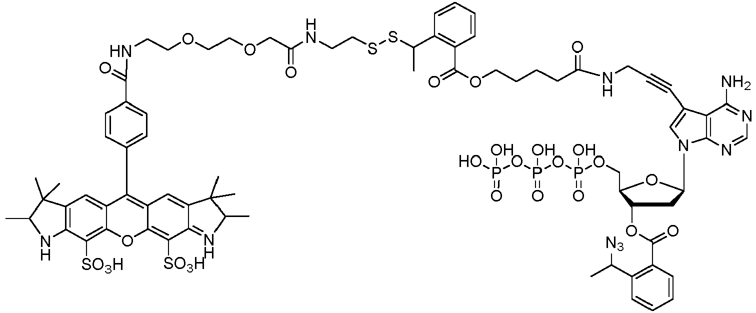

For synthesis process, refer to Example 19.

### Example 35

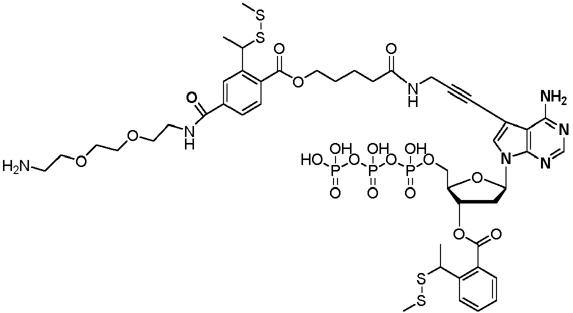

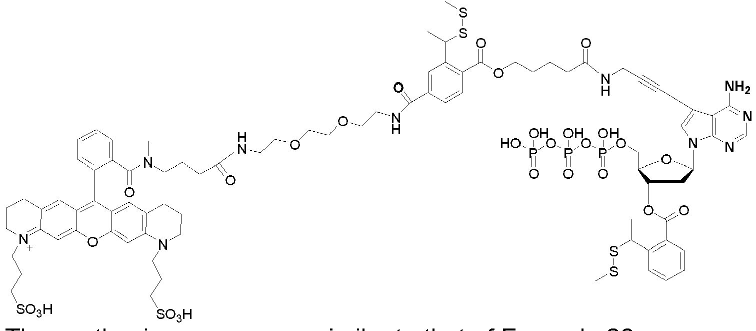

The synthesis process was similar to that of Example 22.

### Example 36

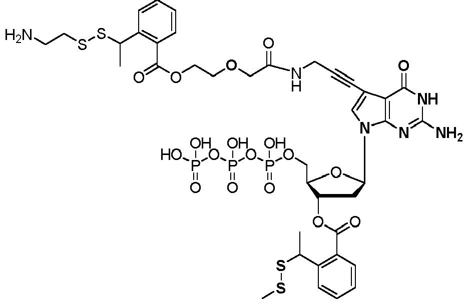

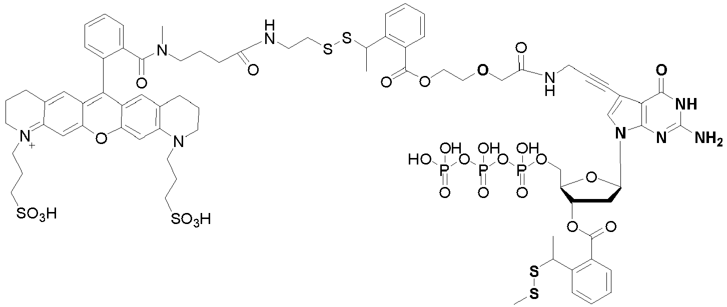

The synthesis process was similar to that of Example 19.

### Example 37

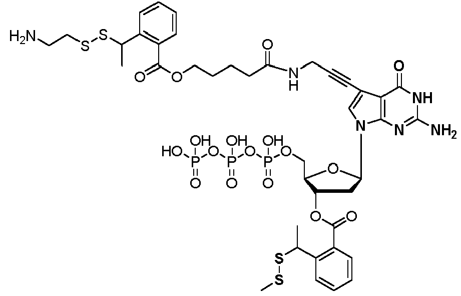

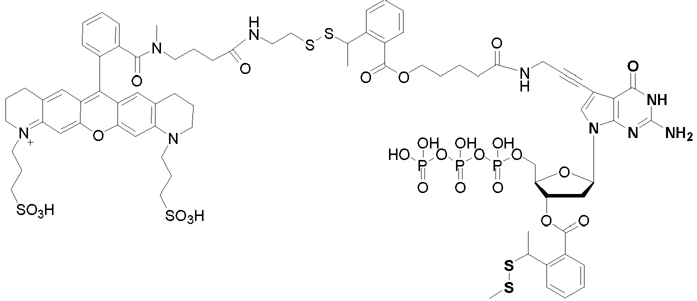

The synthesis process was similar to that of Example 8.

### Example 38

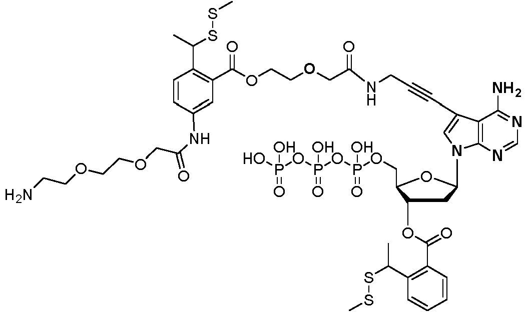

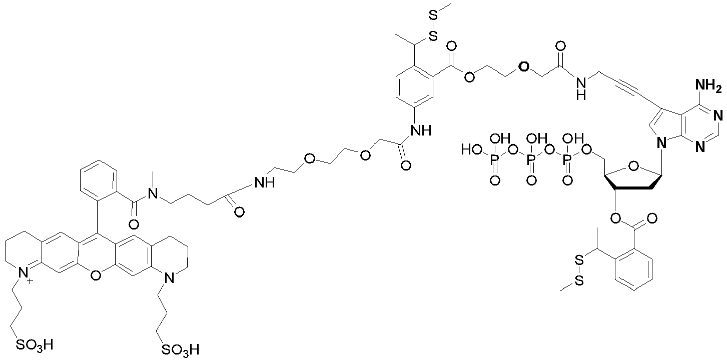

The synthesis process was similar to that of Example 7-2.

### Example 39

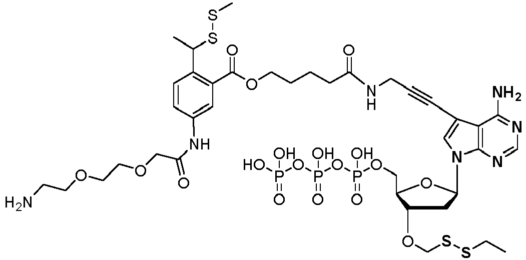

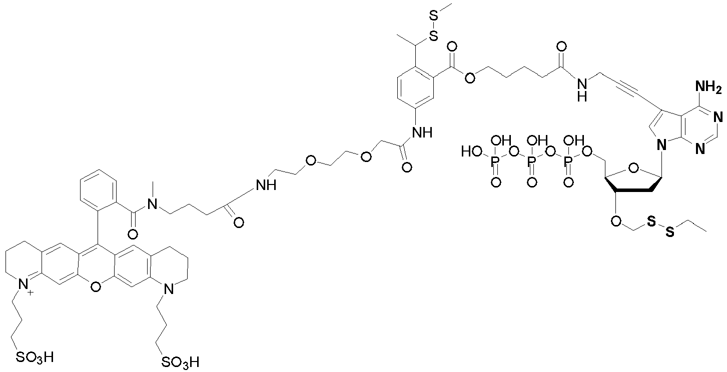

The synthesis process was similar to that of Example 6-2.

### Example 40

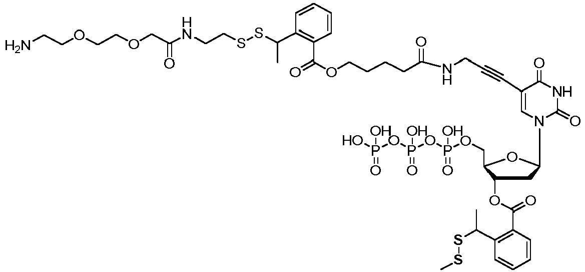

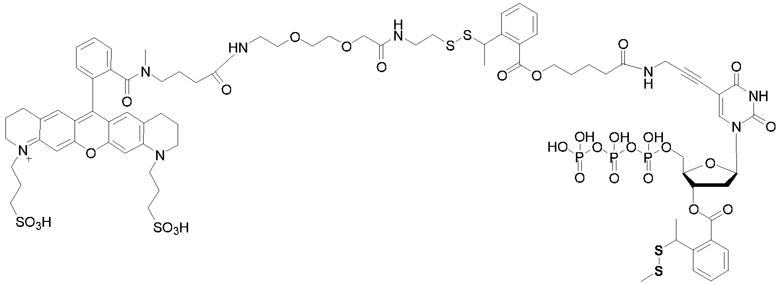

The synthesis process was similar to that of Example 19.

### Example 41

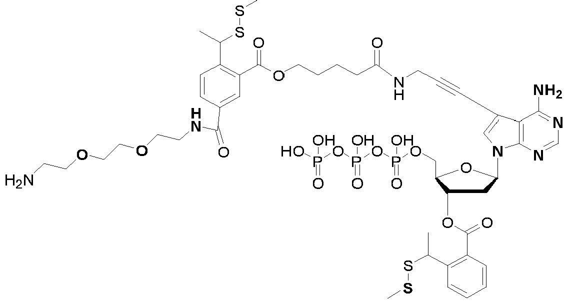

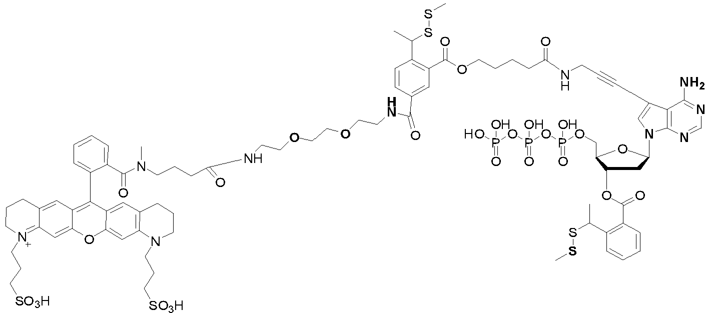

The synthesis process was similar to that of Example 22.

### Example 42

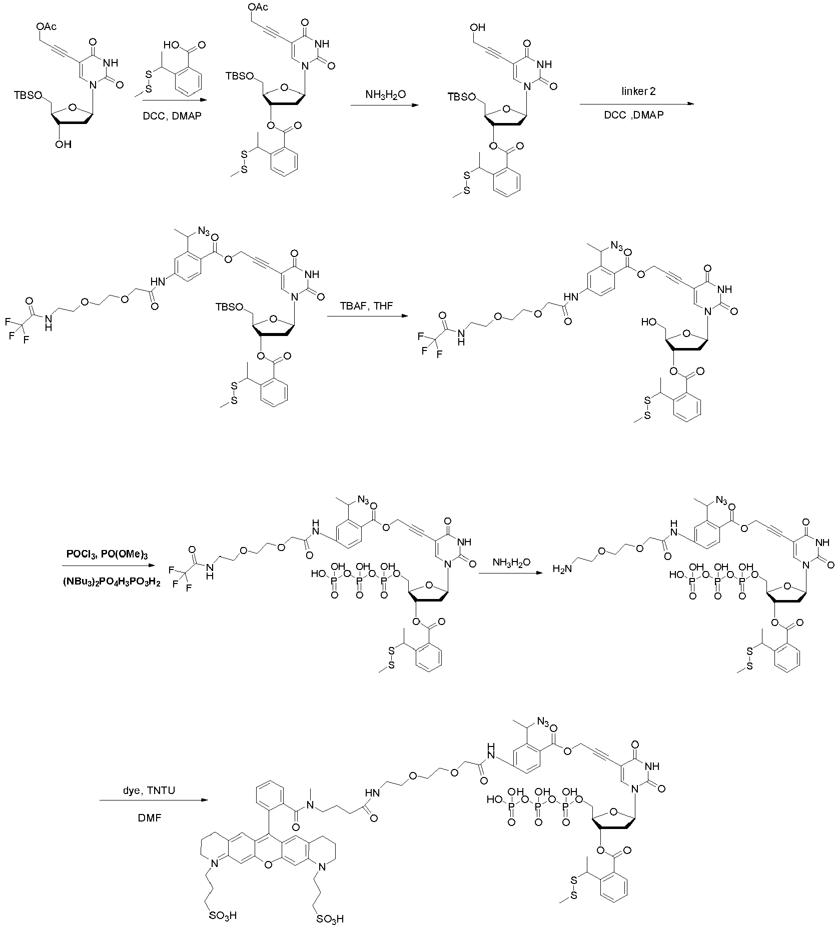

For synthesis of linker 2, refer to Example 2.

The compound was subjected to condensation to produce the TBS-protected nucleoside. After removal of the acetyl group by aminolysis, the compound was condensed with linker 2 in the presence of DCC, followed by removal of the TBS protective group, triphosphorylation and then aminolysis, to obtain the amino compound, which was finally labeled with fluorescent dye to provide the product.

### Example 43

### Synthesis of linker

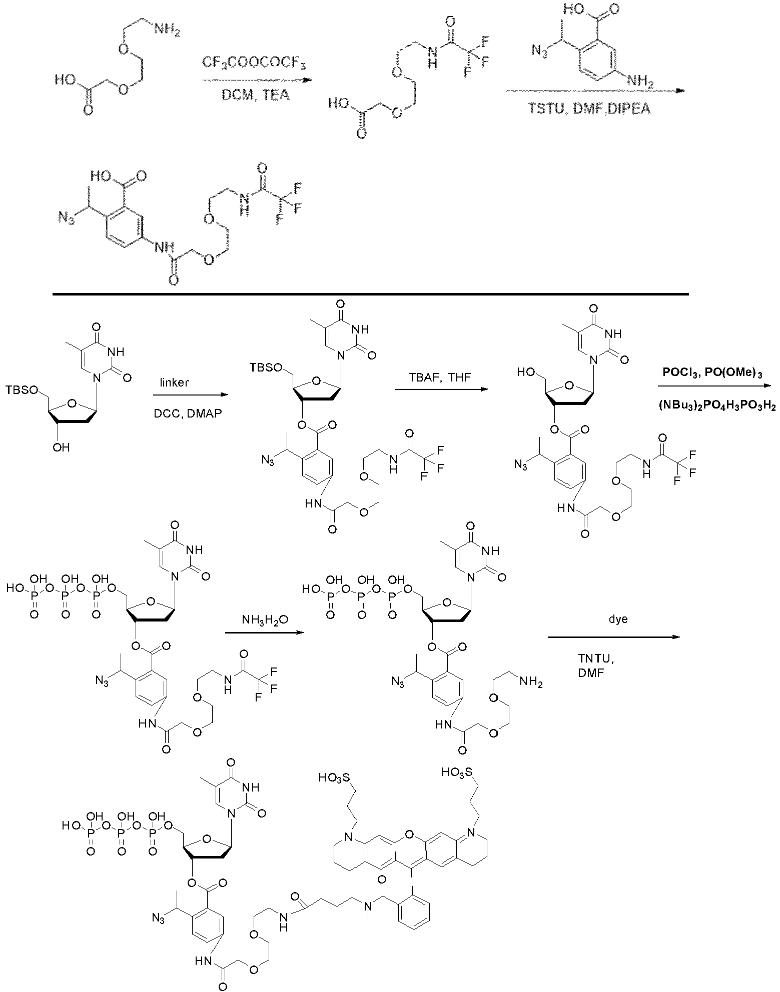

The linker was reacted under DCC condensation conditions, followed by removal of the TBS protective group, triphosphorylation to produce the triphosphate produce, aminolysis to remove the trifluoroacetyl group, and finally condensed with the fluorescent dye to provide the product.

### Example 44

### Synthesis of linker 2

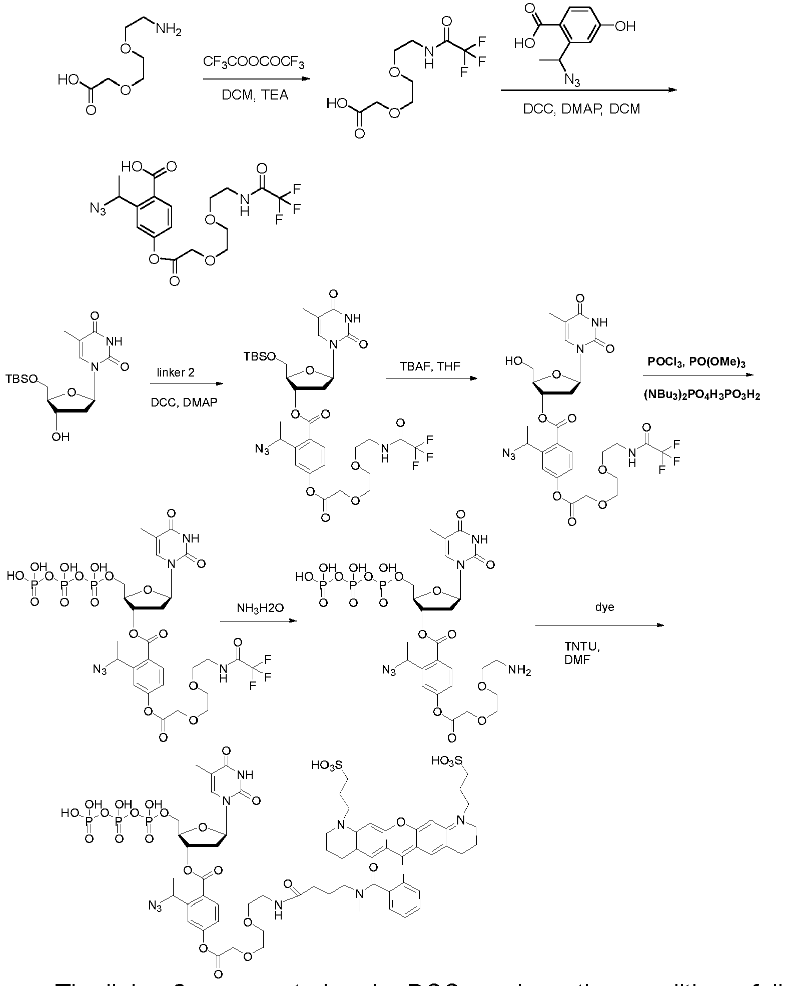

The linker 2 was reacted under DCC condensation conditions, followed by removal of the TBS protective group, triphosphorylation to produce the triphosphate product, aminolysis to remove the trifluoroacetyl group, and finally condensed with the fluorescent dye to provide the product.

### Example 45

### Synthesis of linker

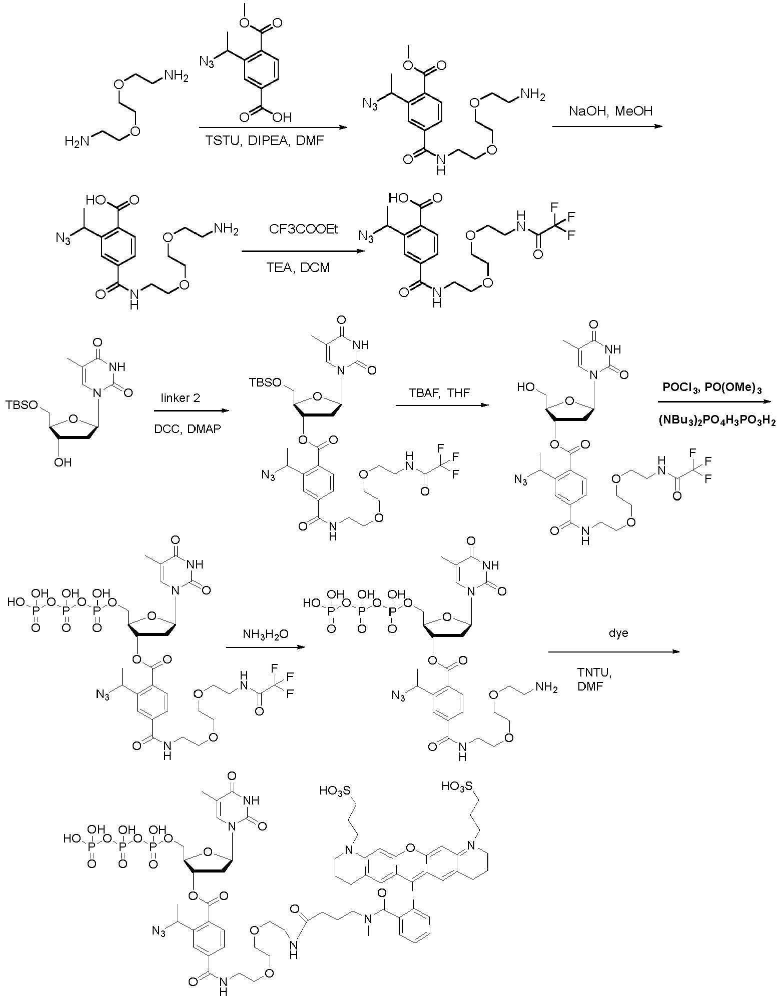

The synthesis process was similar to that of Example 2.

### Example 46

### Synthesis of linker

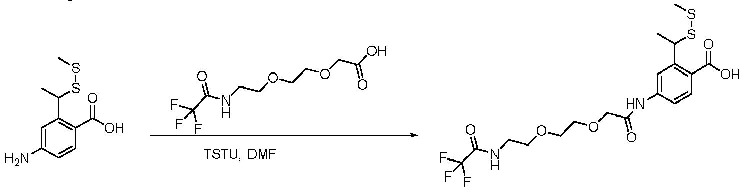

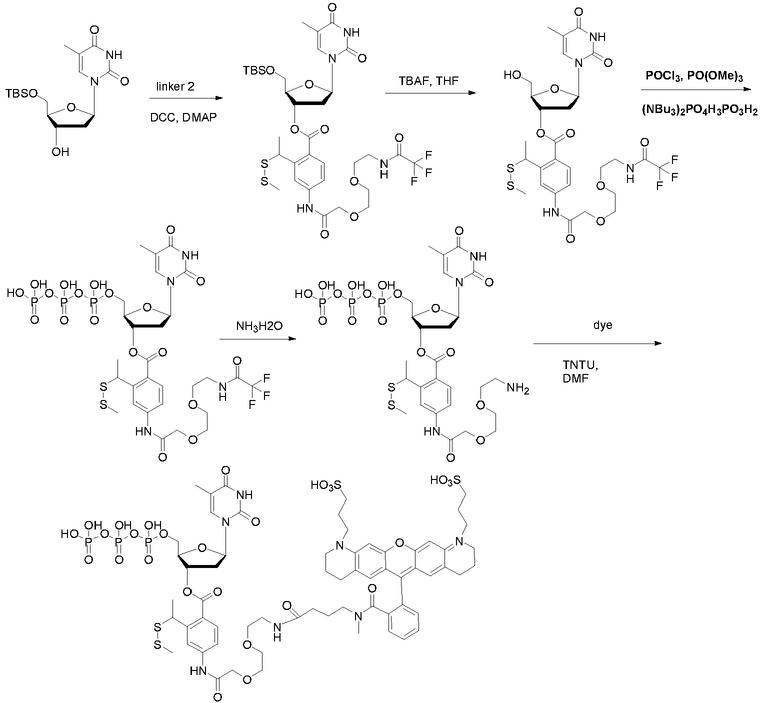

For synthesis of the linker, refer to Example 3.

### Example 47

### Synthesis of linker

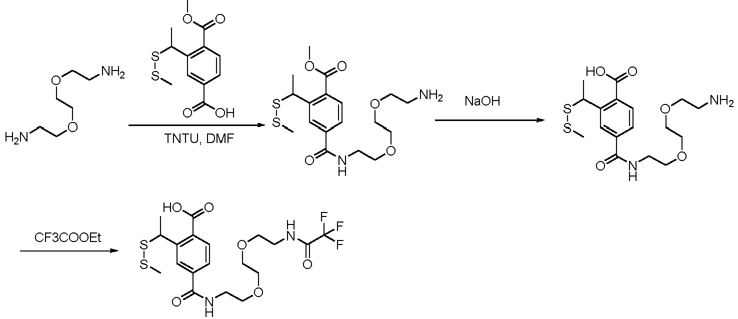

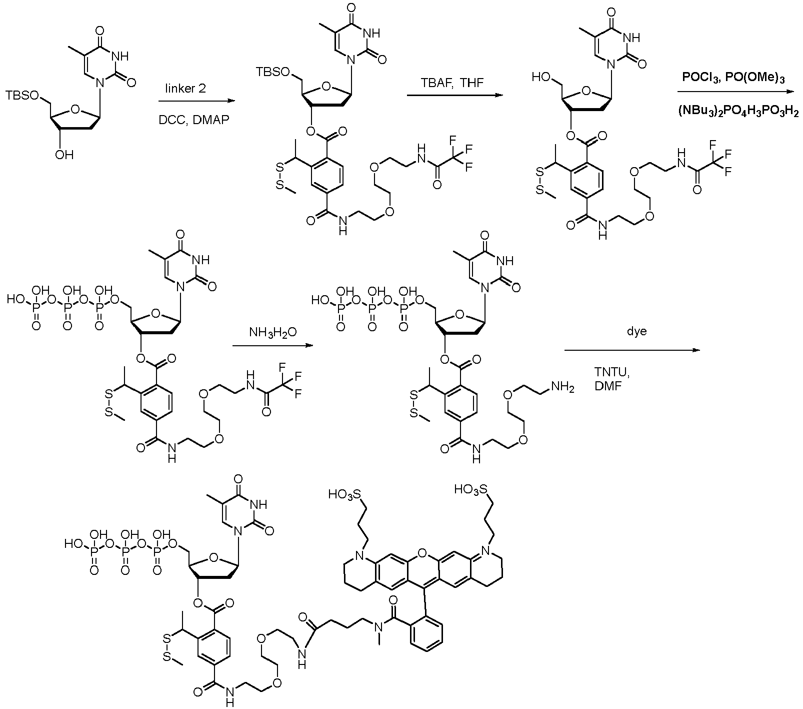

The synthesis process was similar to that of Example 2.

### Example 48

### Synthesis of linker

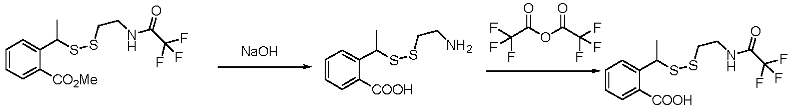

The linker was 2-(2-(2-aminoethoxy)ethoxy)acetate hydrochloride, commercially available from Energy Chemical, cat. No. A012027.

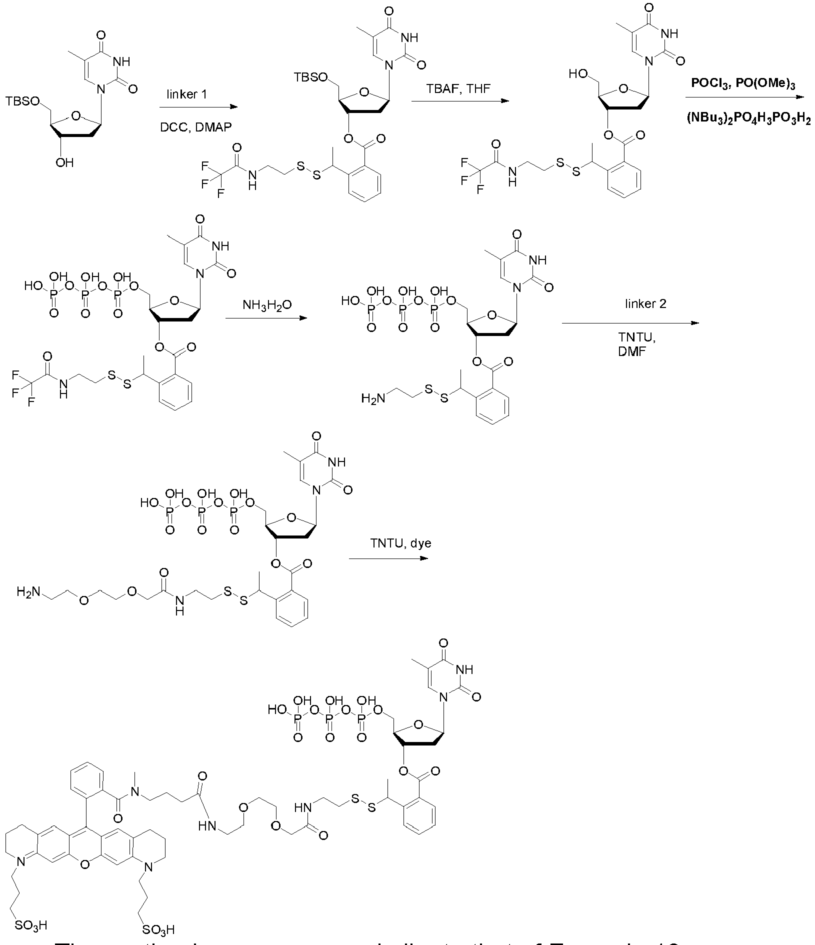

The synthesis process was similar to that of Example 19.

### II. Evaluation examples for sequencing results

The following four cold dNTPs, used as control, were synthesized according to the preparation method disclosed in PCT/CN2021/125262, Example 30, Example 31, Example 32, and Example 33.

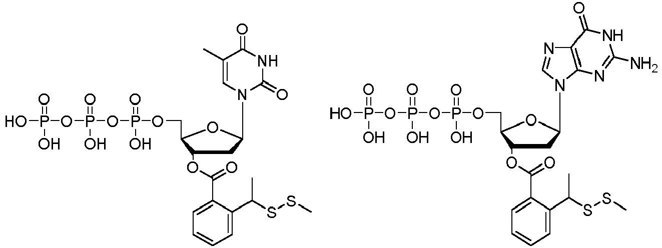

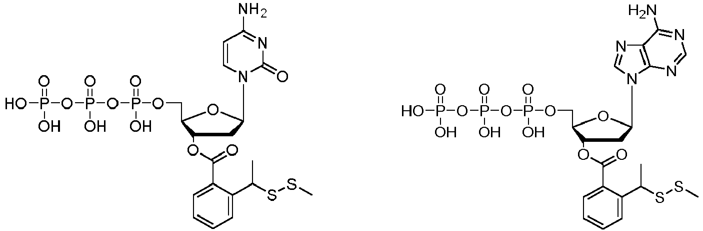

Specifically: 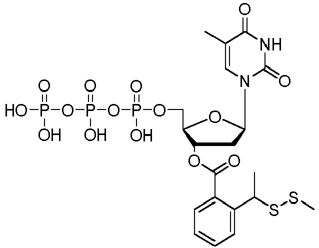 was synthesized as follows: 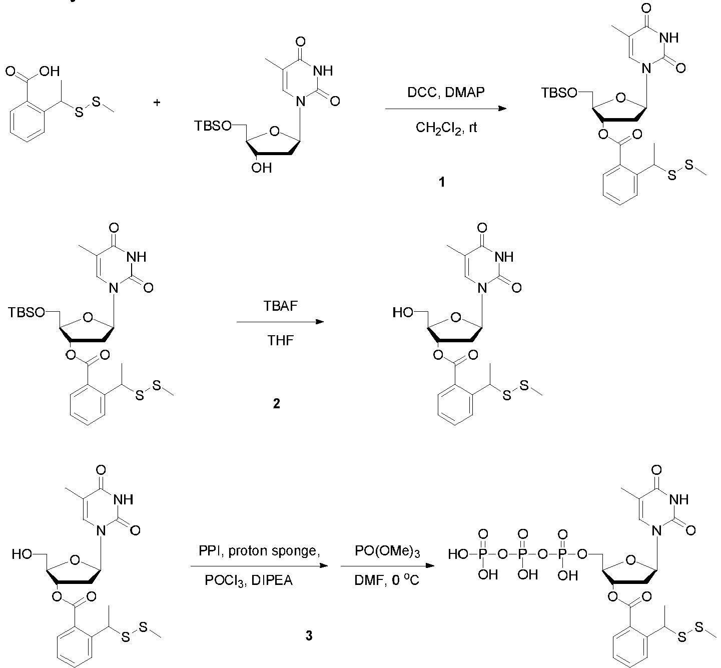 MW = 692. ¹H NMR (400 MHz, D₂O) δ 7.90 - 7.82 (m, 2H), 7.69 - 7.58 (m, 2H), 7.46 - 7.39 (m, 1H), 6.47 - 6.35 (m, 1H), 5.73 (s, 1H), 5.10 - 5.01 (m, 1H), 4.57 (dd, *J* = 13.6, 3.0 Hz, 1H), 4.41 - 4.24 (m, 2H), 2.65 - 2.52 (m, 2H), 1.98 (d, *J* = 15.2 Hz, 3H), 1.94 (s, 3H), 1.68 (d, *J* = 7.2 Hz, 3H). 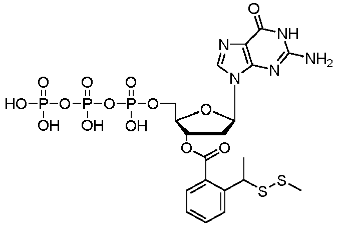 was synthesized as follows:

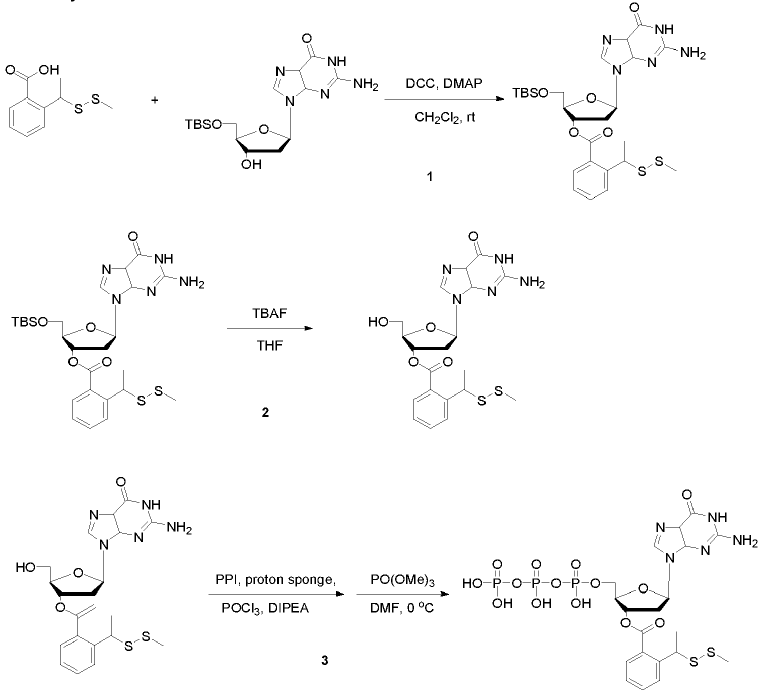

MW = 717. ¹H NMR (400 MHz, D₂O) δ 8.14 (t, *J* = 3.1 Hz, 1H), 7.82 (dd, *J* = 8.1, 3.1 Hz, 1H), 7.55 (q, *J* = 3.2 Hz, 2H), 7.38 - 7.34 (m, 1H), 6.31 - 6.22 (m, 1H), 5.76 (d, *J* = 4.1 Hz, 1H), 5.10 - 5.00 (m, 1H), 4.65 - 4.55 (m, 1H), 4.36 - 4.21 (m, 2H), 3.02 - 2.94 (m, 1H), 2.71 - 2.61 (m, 1H), 1.94 (dd, *J* = 22.2, 3.2 Hz, 3H), 1.62 (dd, *J* = 7.2, 3.3 Hz, 3H). 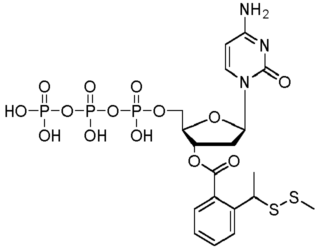 was synthesized as follows: 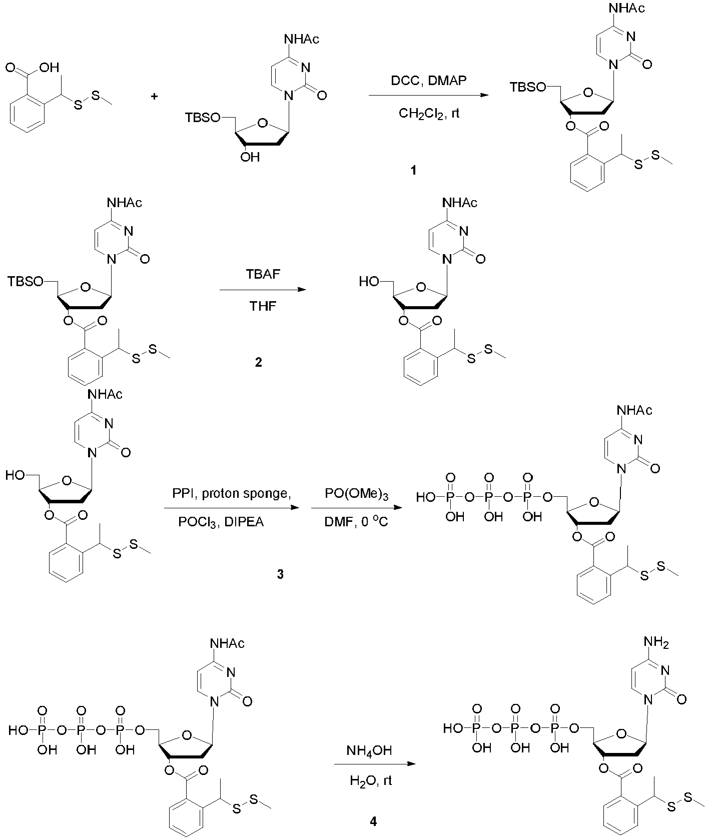 MW = 677. ¹H NMR (400 MHz, D₂O) δ 8.08 - 8.00 (m, 1H), 7.91 - 7.84 (m, 1H), 7.72 - 7.59 (m, 2H), 7.49 - 7.40 (m, 1H), 6.48 - 6.40 (m, 1H), 6.19 (dd, *J* = 7.7, 2.3 Hz, 1H), 5.73 - 5.64 (m, 1H), 5.13 - 5.02 (m, 1H), 4.60 (d, *J* = 12.6 Hz, 1H), 4.36 - 4.27 (m, 2H), 2.71 - 2.64 (m, 1H), 2.55 - 2.44 (m, 1H), 2.00 (dd, *J* = 9.5, 2.2 Hz, 3H), 1.69 (dd, *J* = 7.2, 2.1 Hz, 3H). 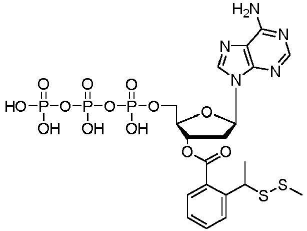 was synthesized as follows: 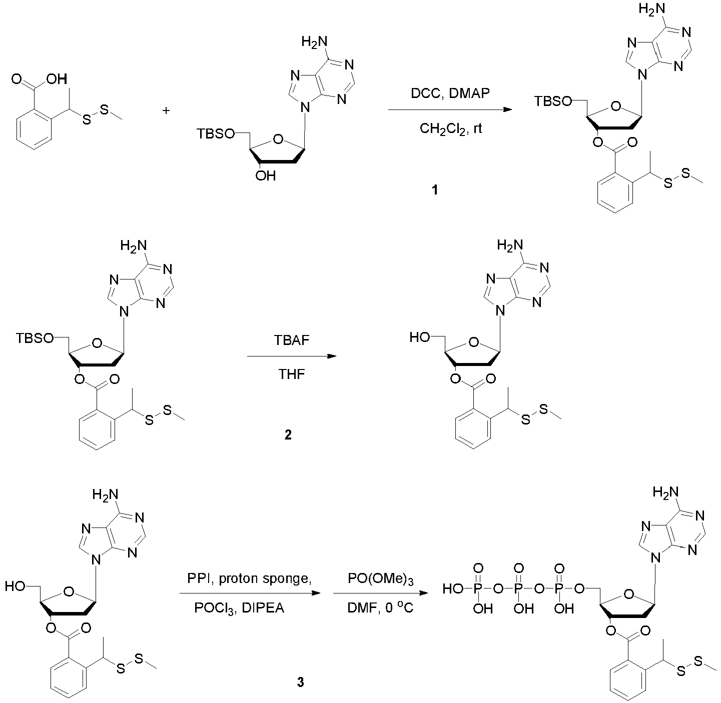 MW = 701. ¹H NMR (400 MHz, D₂O) δ 8.53 (dd, *J* = 5.2, 1.8 Hz, 1H), 8.18 - 8.10 (m, 1H), 7.83 (d, *J* = 7.8 Hz, 1H), 7.55 - 7.51 (m, 2H), 7.38 - 7.34 (m, 1H), 6.49 - 6.40 (m, 1H), 5.81 - 5.73 (m, 1H), 5.07 - 5.00 (m, 1H), 4.67 - 4.57 (m, 1H), 4.39 - 4.18 (m, 2H), 3.06 - 2.95 (m, 1H), 2.75 (ddd, *J* = 21.2, 14.2, 5.6 Hz, 1H), 1.93 (dd, *J* = 25.1, 1.9 Hz, 3H), 1.60 (dd, *J* = 6.9, 4.0 Hz, 3H).

### Sequencing example 1

### Nucleotide substrates

(1) hot dNTP: Four final products were obtained from the syntheses of Examples 7-1 to 7-4 (named as **SSEB Hot Mix_V8**), with structures showed below. 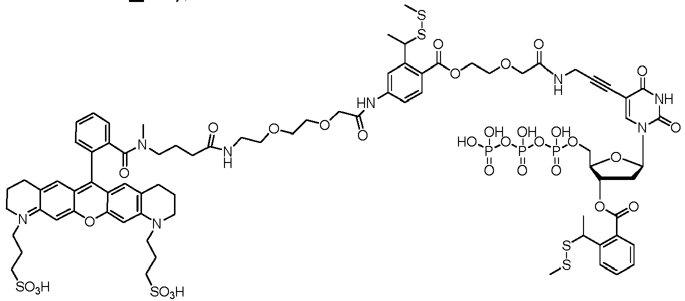 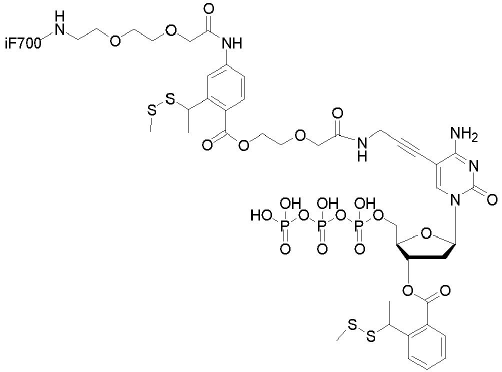 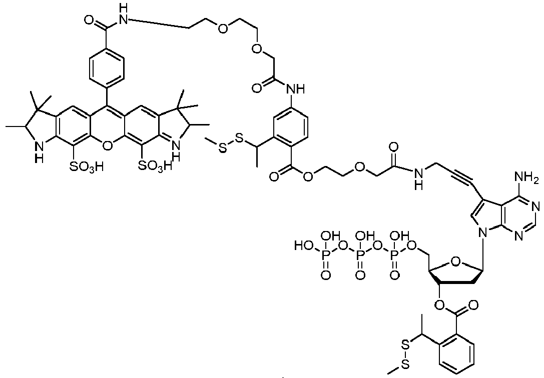 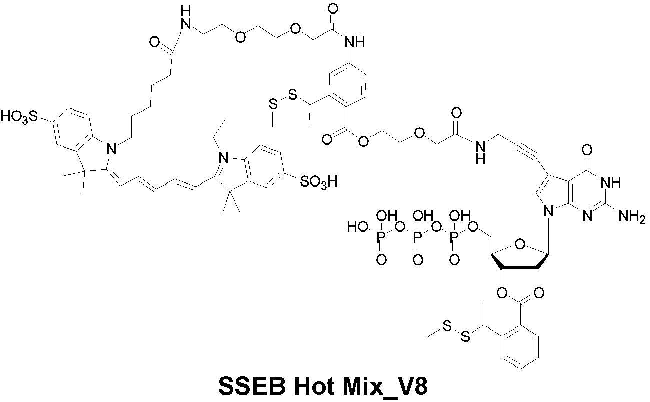
(2) cold dNTP: Four cold dNTPs were synthesized by the aforementioned process, with structures showed below.

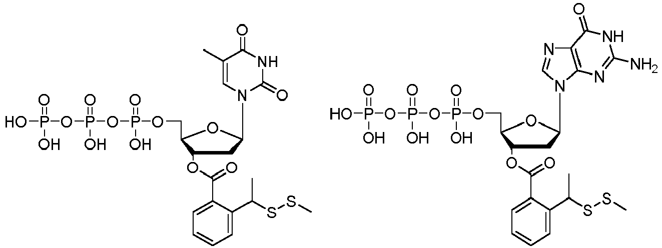

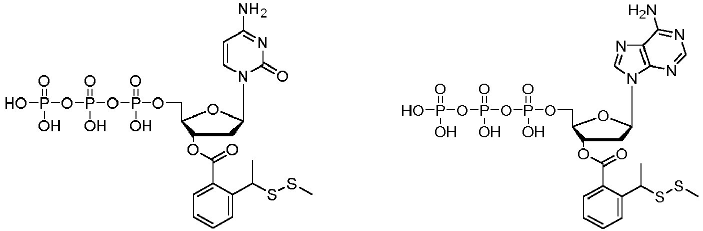

Sequencing was conducted according to the operation protocol of MGISEQ-2000 sequencer, using the above nucleotide substrates and MGISEQ-2000 high-throughput sequencing kit (FCL SE50).
1. Preparing DNA nanoballs by using Ecoli sequencing library;
2. Loading DNA nanoballs onto the BGISEQ2000 sequencing chip;
3. Amounting the loaded sequencing chip onto the BGISEQ2000 sequencer and set the sequencing process, hot dNTP incorporation: 60°C 2min; cold dNTP incorporation: 60°C 2min; signal acquisition; cleavage of blocking group: 65°C 2min;
4. Conducting Basecall analysis on offline data and outputting results of sequencing index including mapping rate, error rate, Q30, etc. The results were showed in Table 1.

The results show that this kind of nucleotide analogue results in good reversible blocking effect in high-throughput sequencing and enables one by one detection of the types of nucleotides incorporated in sequencing.

**Table 1. Basecall analysis results**

| Nucleotide analogue hot dNTP Mix | SSEB Hot Mix_V8 |
|---|---|
| Reference | Ecoli |
| Cycle Number | 51 |
| Q30(%) | 92.67 |
| Lag(%) | 0.43 |
| Runon(%) | 0.31 |
| ESR(%) | 82.60 |
| Mapping Rate (%) | 98.15 |
| AvgError Rate (%) | 0.50 |

| | |
|---|---|
| Notes: 1. Q30(%) refers to the proportion of bases with an error rate of less than 0.001 (accuracy higher than 99.9%) in Basecall results; 2. Lag(%) means the proportion of sequencing lag; 3. Runon(%) means the proportion of sequencing ahead; 4. ESR(%) refers to the ratio of Total Reads obtained by filtering the final sequencing to the DNB number recognized by Basecall. | |

### Sequencing example 2

### Nucleotide substrates

(1) hot dNTP: Four final products were obtained from the syntheses of Examples 7-1, 7-4, 11, 15-2 (named as SSEB Hot Mix-V8V9V11), with structures showed below. 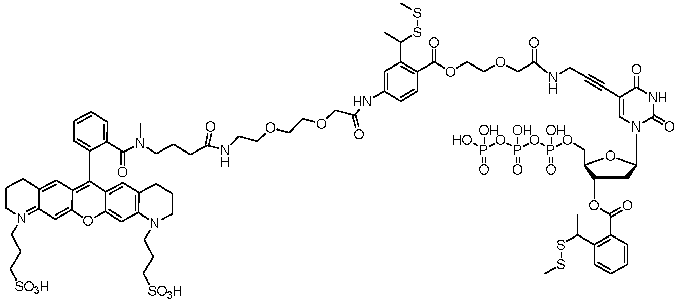 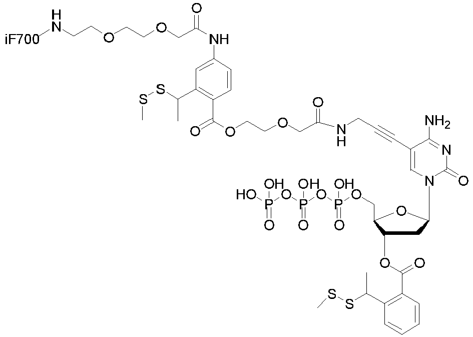 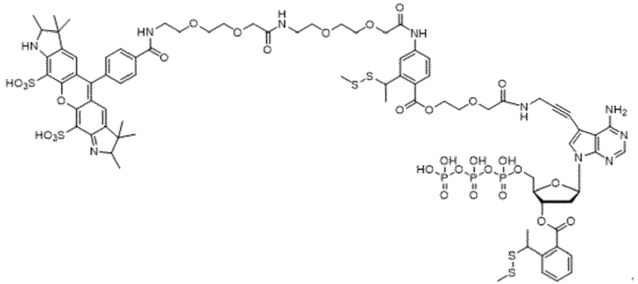 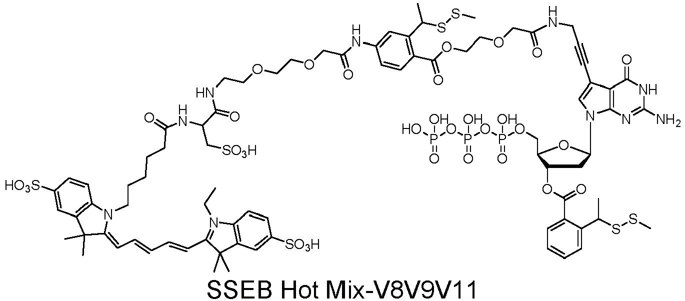
(2) cold dNTP: Four types of cold dNTPs were synthesized through conventional methods, with structures showed below.

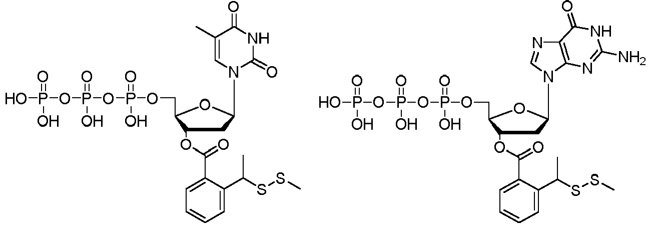

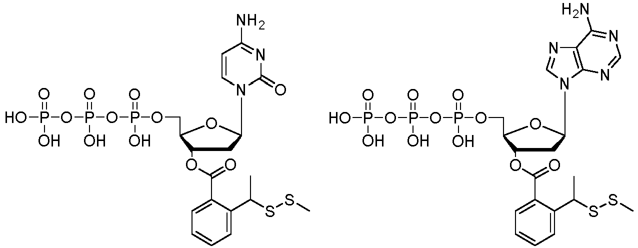

Sequencing was conducted according to the operation protocol of MGISEQ-2000 sequencer, using the above nucleotide substrates and MGISEQ-2000 high-throughput sequencing kit (FCL PE100).
1. Preparing DNA nanoballs by using Ecoli sequencing library;
2. Loading DNA nanoballs onto the BGISEQ2000 sequencing chip;
3. Amounting the loaded sequencing chip onto the BGISEQ2000 sequencer and set the sequencing process, hot dNTP incorporation: 55°C 30s; cold dNTP incorporation: 55°C 60s; signal acquisition; cleavage of blocking group: 55°C 8s;
4. Conducting Basecall analysis on offline data and outputting results of sequencing index including mapping rate, error rate, Q30, etc. The results were showed in Table 2.

The results show that this kind of nucleotide analogue results in good reversible blocking effect in high-throughput sequencing and enables one by one detection of the types of nucleotides incorporated in sequencing.

**Table 2. Basecall analysis results**

| Nucleotide analogue hot dNTP Mix | SSEB Hot Mix-V8V9V11 |
|---|---|
| Reference | Ecoli |
| Cycle Number | 212 |
| Q30(%) | 95.79 |
| Lag 1(%) | 0.08 |
| Lag 2(%) | 0.11 |
| Runon 1(%) | 0.12 |
| Runon 2(%) | 0.26 |
| ESR(%) | 76.81 |
| Mapping Rate (%) | 99.89 |
| AvgError Rate (%) | 0.15 |

### Sequencing example 3

### Nucleotide substrates

(1) hot dNTP: Four final products were obtained from the syntheses of Examples 10-1, 10-2, 10-3, 10-4 (named as SSEB Hot Mix-N₃), with structures showed below. 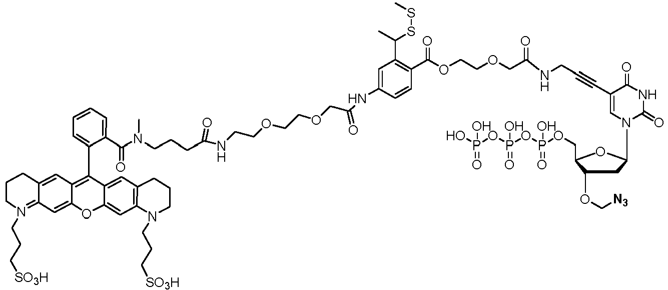 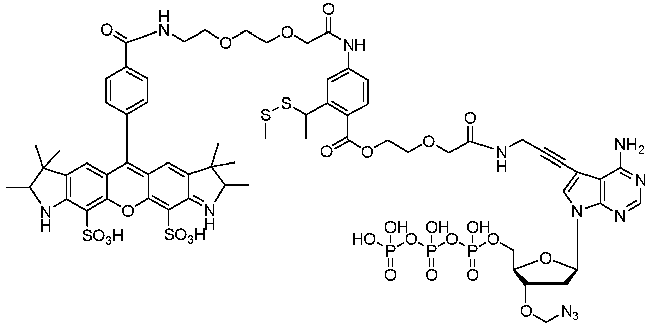 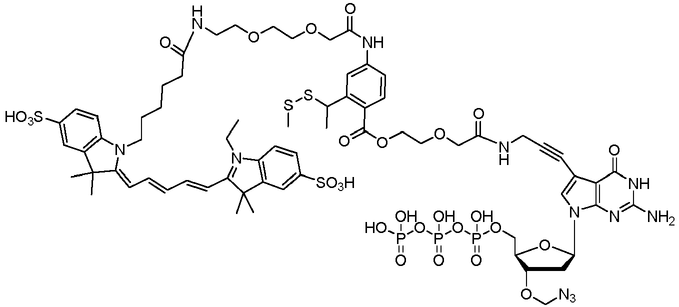 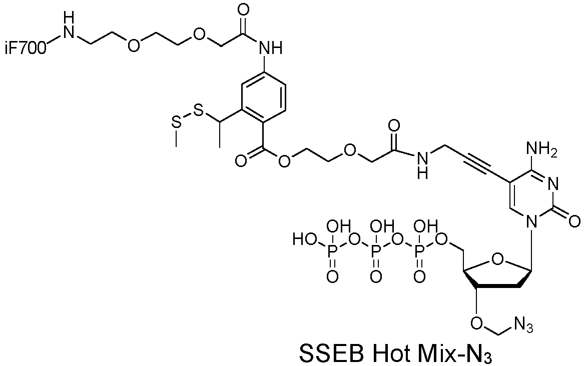
(2) cold dNTP: Four types of cold dNTPs were synthesized through conventional methods, with structures showed below.

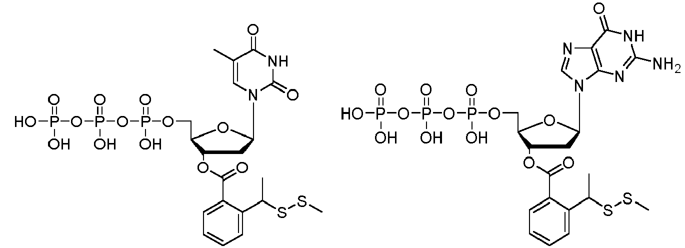

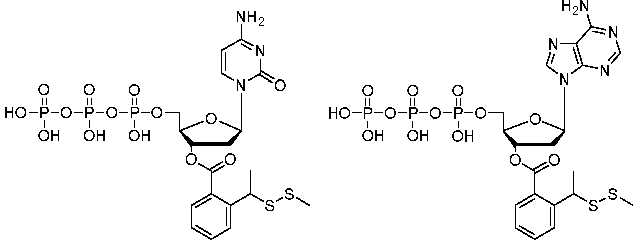

Sequencing was conducted according to the operation protocol of MGISEQ-2000 sequencer, using the above nucleotide substrates and MGISEQ-2000 high-throughput sequencing kit (FCL PE100).
1. Preparing DNA nanoballs by using Ecoli sequencing library;
2. Loading DNA nanoballs onto the BGISEQ2000 sequencing chip;
3. Amounting the loaded sequencing chip onto the BGISEQ2000 sequencer and set the sequencing process, hot dNTP incorporation: 60°C 2min; cold dNTP incorporation: 60°C 2min; signal acquisition; cleavage of blocking group: 65°C 2min;
4. Conducting Basecall analysis on offline data and outputting results of sequencing index including mapping rate, error rate, Q30, etc. The results were showed in Table 3.

The results show that this kind of nucleotide analogue results in good reversible blocking effect in high-throughput sequencing and enables one by one detection of the types of nucleotides incorporated in sequencing.

**Table 3. Basecall analysis results**

| Nucleotide analogue hot dNTP Mix | SSEB Hot Mix-N₃ | |
|---|---|---|
| Reference | | Ecoli |
| Cycle Number | | 53 |
| Q30(%) | | 95.45 |
| Lag 1(%) | | 0.20 |
| Lag 2(%) | | 0.22 |
| Runon 1(%) | | 0.43 |
| Runon 2(%) | | 0.45 |
| ESR(%) | | 80.29 |
| Mapping Rate (%) | | 96.82 |
| AvgError Rate (%) | | 0.57 |

## Claims

1. A compound of formula I or formula II or a salt thereof, 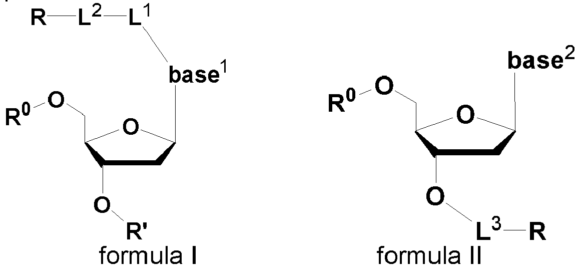 wherein:
L¹ is selected from 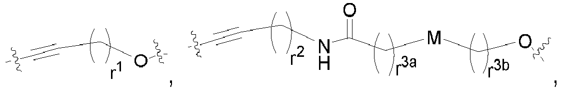 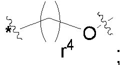
preferably, L¹ is selected from 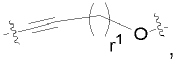 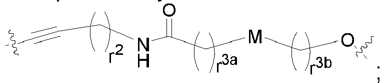
or preferably, L¹ is selected from 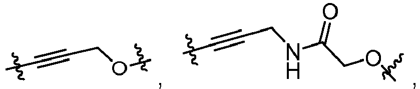 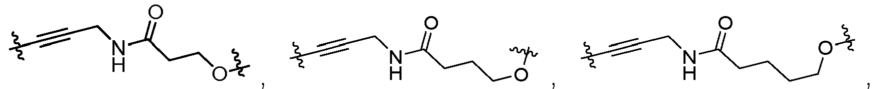 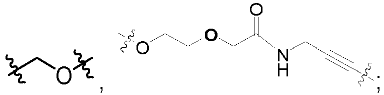
or more preferably, L¹ is selected from 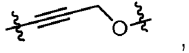 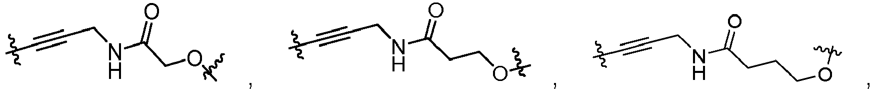 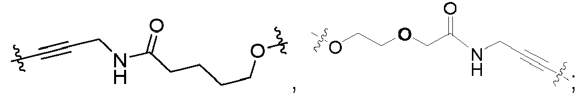
or most preferably, L¹ is selected from 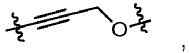 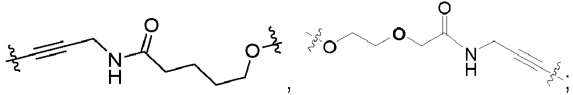
r¹, r², r^{3a}, r^{3b}, r⁴, independently of each other, are selected from any integer between 1 and 6;
preferably, r¹ is selected from 1, 2, 3;
more preferably, r¹ is 1;
preferably, r² is selected from 1, 2, 3;
more preferably, r² is 1;
preferably, r^{3a}, r^{3b}, independently of each other, are selected from 1, 2, 3, 4, 5;
more preferably, r^{3a}, r^{3b}, independently of each other, are selected from 1, 2, 3;
most preferably, r^{3a} is 1;
most preferably, r^{3b} is 2;
preferably, r⁴ is selected from 1, 2, 3;
more preferably, r⁴ is 1;
M is selected from a direct bond, CH₂, NH, O, S;
preferably, M is selected from CH₂, O;
L² is 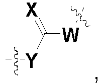 preferably, W being linked to R;
L³ is 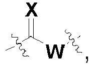 preferably, W being linked to R;
each X, independently, is selected from O, S, NH;
preferably, each X, independently, is selected from O, S;
more preferably, X is O;
Y is selected from a direct bond, O, S, NH;
preferably, Y is a direct bond;
each W, independently, is selected from a direct bond, O, S, NH;
preferably, W is a direct bond;
R is 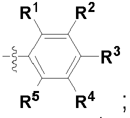
in R¹, R², R³, R⁴, R⁵, any one is 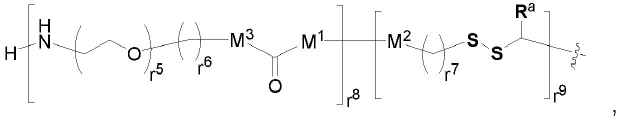 and the others, independently of each other, are selected from H, 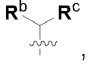 azido, nitro, amino, sulfo, carboxyl, aliphatic alkyl (e.g., C₁-C₆ alkyl), cycloalkyl (e.g., C₃-C₆ cycloalkyl), aromatic alkyl (e.g., phenyl -C₁-C₆ alkyl), F, I, Br, Cl, alkoxyl (e.g., C₁-C₆ alkoxyl);
preferably, in R¹, R², R³, R⁴, R⁵, any one is 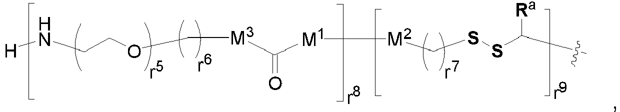 and the others independently of each other, are selected from H, 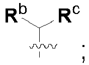
more preferably, in R¹, R², R³, R⁴, R⁵, any one is 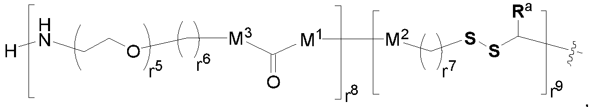 another (e.a.. R¹ or R⁵) is selected from H, 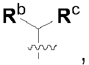 and the remaining three are H;
r⁵, r⁶, r⁷, independently of each other, are selected from any integer between 1 and 6;
preferably, r⁵ is selected from 1, 2, 3;
more preferably, r⁵ is 2;
preferably, r⁶ is selected from 1, 2, 3;
more preferably, r⁶ is 1 or 2;
preferably, r⁷ is selected from 1, 2, 3;
more preferably, r⁷ is 2;
r⁸, r⁹, independently of each other, are selected from 0, 1, r⁸ and r⁹ being not 0 at the same time;
M¹, M², M³, independently of each other, are selected from a direct bond, NH, O, S;
preferably, M¹ is selected from a direct bond, NH, O;
preferably, M² is NH;
preferably, M³ is selected from a direct bond, NH;
R^{a} is selected from H, aliphatic alkyl (e.g., C₁-C₆ alkyl, such as Me, Et, iPr, tBu), aromatic alkyl (e.g., phenyl -C₁-C₆ alkyl), cycloalkyl (e.g., C₃-C₆ cycloalkyl);
preferably, R^{a} is selected from H, C₁-C₆ alkyl;
more preferably, R^{a} is methyl;
in R^{b}, R^{c}, any one is selected from -N₃, -SS-C₁-C₆ alkyl (e.g., -SS-Me, -SS-Et, -SS-iPr, -SS-t-Bu), -ONH₂, -OCORₓ, -OCONHRₓ, and the other is selected from H, aliphatic alkyl (e.g., C₁-C₆ alkyl, such as Me, Et, iPr, tBu), aromatic alkyl (e.g., phenyl -C₁-C₆ alkyl), cycloalkyl (e.g., C₃-C₆ cycloalkyl), wherein each Rₓ, independently, is selected from aliphatic alkyl (e.g., C₁-C₆ alkyl), cycloalkyl (e.g., C₃-C₆ cycloalkyl) or aromatic alkyl (e.g., phenyl C₁-C₆ alkyl);
preferably, in R^{b}, R^{c}, any one is selected from -N₃, -SS-C₁-C₆ alkyl (e.g., -SS-Me, -SS-Et, -SS-iPr, -SS-t-Bu), and the other is selected from C₁-C₆ alkyl;
more preferably, in R^{b}, R^{c}, any one is selected from -N₃, -SS-Me, -SS-Et, and the other is methyl;
most preferably, in R^{b}, R^{c}, any one is selected from -N₃, -SS-Me, and the other is methyl;
preferably, R is selected from 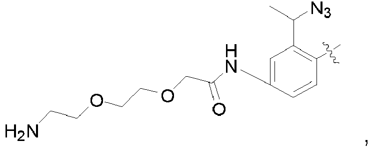 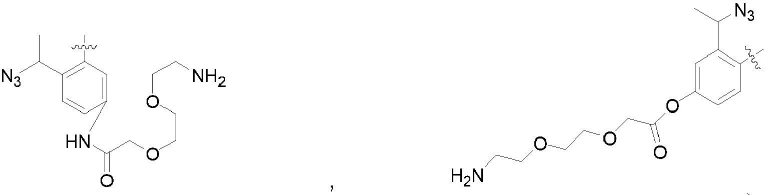 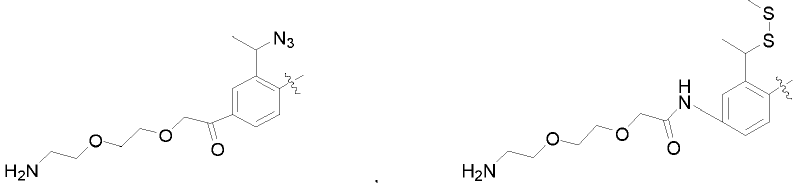 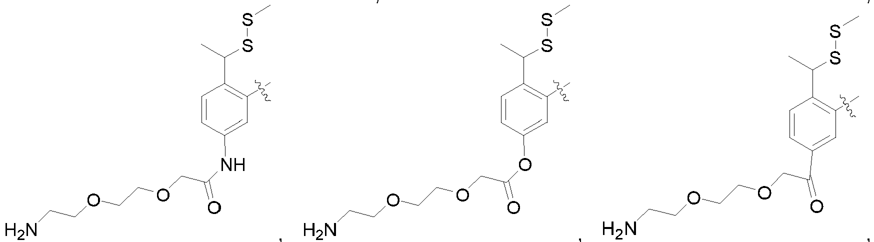 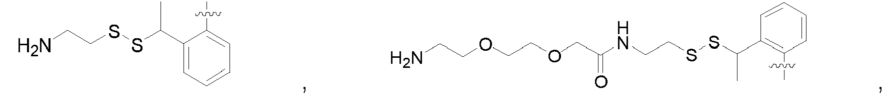 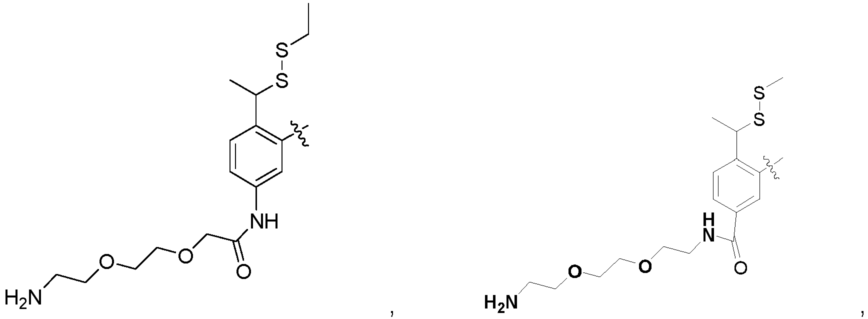 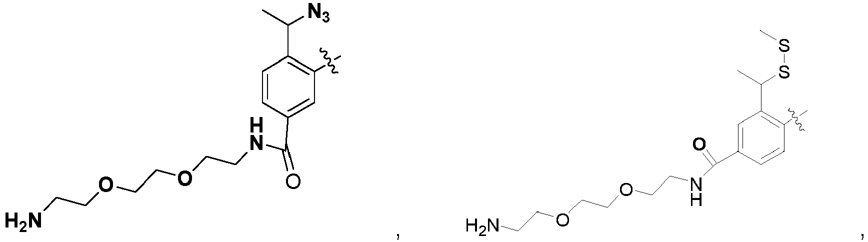 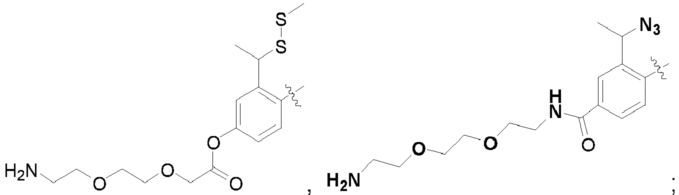
R⁰ is selected from H monophosphate group 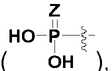 diphosphate group 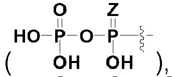 triphosphate group 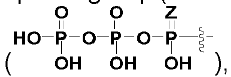 tetraphosphate group 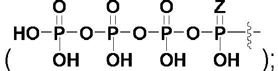
preferably, R° is a triphosphate group 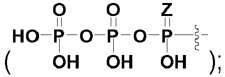
each Z, independently, is selected from O, S, BH;
preferably, Z is O;
base¹, base², independently of each other, are selected from a base, a deaza base or a tautomer thereof, for example, adenine, 7-deazaadenine, thymine, uracil, cytosine, guanine, 7-deazaguanine or a tautomer thereof;
preferably, base¹ is selected from 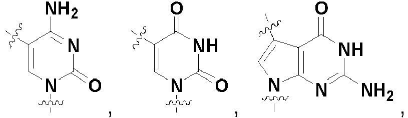 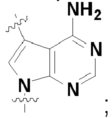
preferably, base² is selected from 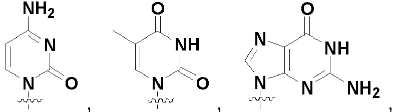 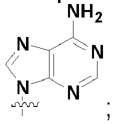
in the formula I, R' represents a reversible blocking group, and in the formula II, 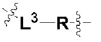 L³-R- represents a reversible blocking group.

2. The compound or a salt thereof according to claim 1, wherein the compound is represented by the formula I-1, 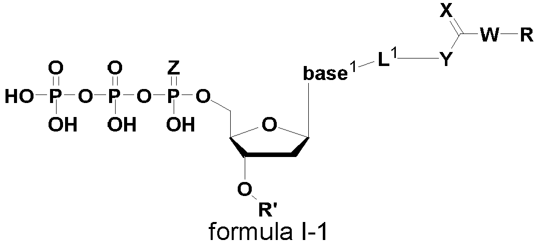 wherein:
L¹ is selected from 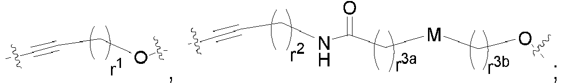
preferably, L¹ is selected from 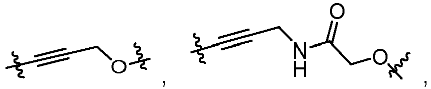 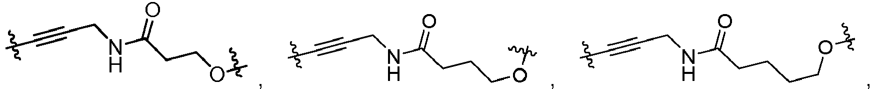 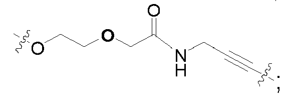
more preferably, L¹ is selected from 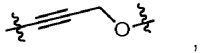 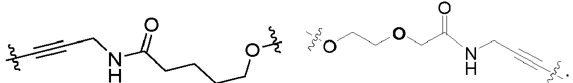
r¹, r², r^{3a}, r^{3b}, independently of each other, are selected from any integer between 1 and 6;
preferably, r¹ is selected from 1, 2, 3;
more preferably, r¹ is 1;
preferably, r² is selected from 1, 2, 3;
more preferably, r² is 1;
preferably, r^{3a}, r^{3b}, independently of each other, are selected from 1, 2, 3, 4, 5;
more preferably, r^{3a}, r^{3b}, independently of each other, are selected from 1, 2, 3;
most preferably, r^{3a} is 1;
most preferably, r^{3b} is 2;
M is selected from a direct bond, CH₂, NH, O, S;
preferably, M is selected from CH₂, O;
X is selected from O, S, NH;
preferably, X is selected from O, S;
more preferably, X is O;
Y is selected from a direct bond, O, S, NH;
preferably, Y is a direct bond;
W is selected from a direct bond, O, S, NH;
preferably, W is a direct bond;
R is 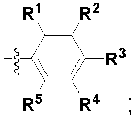
in R¹, R², R³, R⁴, R⁵, any one (e.g., R², R³ or R⁴) is 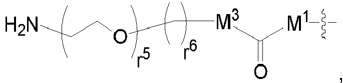 and the others, independently of each other, are selected from H, 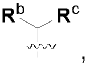 azido, nitro, amino, sulfo, carboxyl, aliphatic alkyl (e.g., C₁-C₆ alkyl), cycloalkyl (e.g., C₃-C₆ cycloalkyl), aromatic alkyl (e.g., phenyl -C₁-C₆ alkyl), F, I, Br, Cl, alkoxyl (e.g., C₁-C₆ alkoxyl);
preferably, in R¹, R², R³, R⁴, R⁵, any one (e.g., R², R³ or R⁴) is 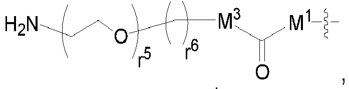 and the others, independently of each other, are selected from H, 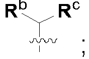
more preferably, in R¹, R², R³, R⁴, R⁵, any one (e.g., R², R³ or R⁴) is 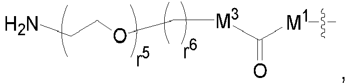 another (e.g., R¹ or R⁵) is 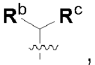 and the remaining three are H;
further preferably, in R¹, R², R³ R⁴, R⁵, R³ or R⁴ is 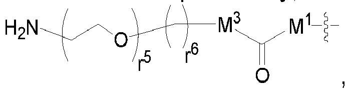 R¹ is 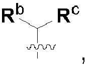 and the remaining three are H;
most preferably, R is selected from 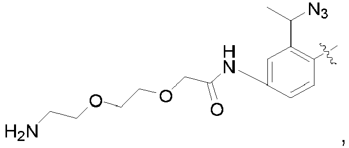 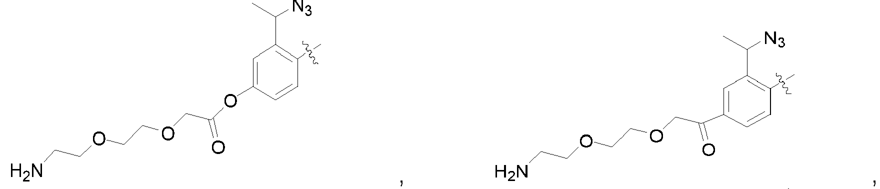 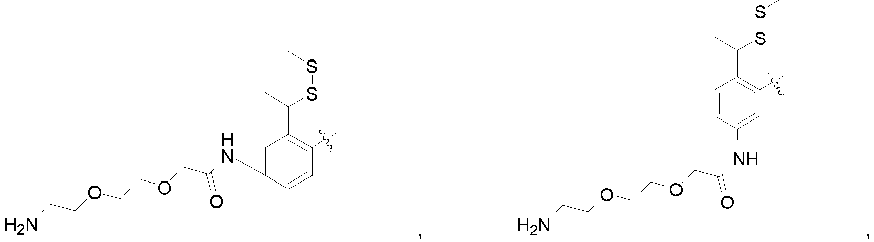 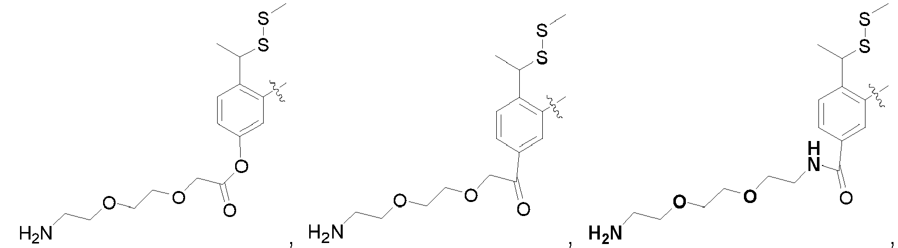 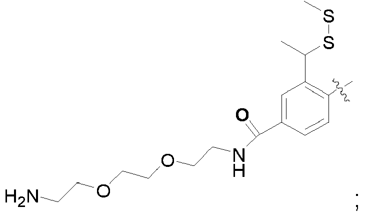
r⁵, r⁶, independently of each other, are selected from any integer between 1 and 6;
preferably, r⁵ is selected from 1, 2, 3;
more preferably, r⁵ is 2;
preferably, r⁶ is selected from 1, 2, 3;
more preferably, r⁶ is 1 or 2;
M¹, M³, independently of each other, are selected from a direct bond, NH, O, S;
preferably, M¹ is selected from a direct bond, NH, O;
preferably, M³ is selected from a direct bond, NH;
more preferably, when M¹ is NH, O or S, M³ is a direct bond, and when M³ is NH, O or S, M¹ is a direct bond;
in R^{b}, R^{c}, any one is selected from -N₃, -SS-C₁-C₆ alkyl (e.g., -SS-Me, -SS-Et, -SS-iPr, -SS-t-Bu), -ONH₂, -OCORₓ, -OCONHRₓ, and the other is selected from H, aliphatic alkyl (e.g., C₁-C₆ alkyl, such as Me, Et, iPr, tBu), aromatic alkyl (e.g., phenyl -C₁-C₆ alkyl), cycloalkyl (e.g., C₃-C₆ cycloalkyl), wherein each Rₓ, independently, is selected from aliphatic alkyl (e.g., C₁-C₆ alkyl), cycloalkyl (e.g., C₃-C₆ cycloalkyl) or aromatic alkyl (e.g., phenyl C₁-C₆ alkyl);
preferably, in R^{b}, R^{c}, any one is selected from -N₃, -SS-C₁-C₆ alkyl (e.g., -SS-Me, -SS-Et, -SS-iPr, -SS-t-Bu), and the other is selected from C₁-C₆ alkyl;
more preferably, in R^{b}, R^{c}, any one is selected from -N₃, -SS-Me, and the other is methyl;
Z is selected from O, S, BH;
preferably, Z is O;
base¹ is selected from a base, a deaza base or a tautomer thereof, for example, adenine, 7-deazaadenine, thymine, uracil, cytosine, guanine, 7-deazaguanine or a tautomer thereof;
preferably, base¹ is selected from 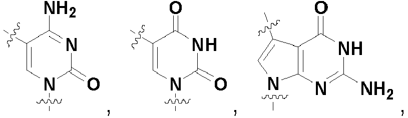 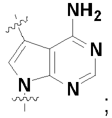
R' represents a reversible blocking group.

3. The compound or a salt thereof according to claim 1, wherein the compound is represented by the formula 1-2, 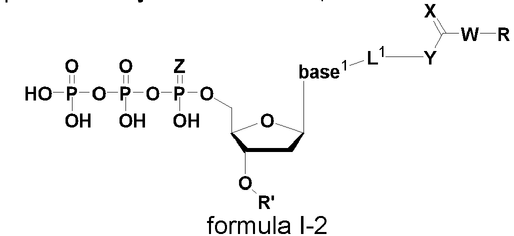 wherein:
L¹ is selected from 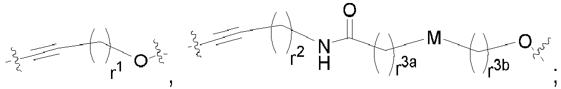
preferably, L¹ is selected from 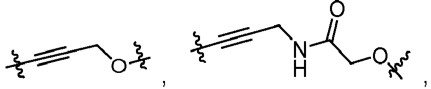 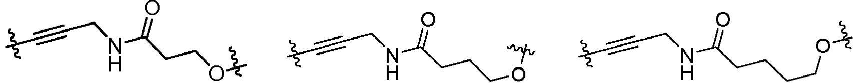 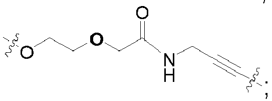
more preferably, L¹ is selected from 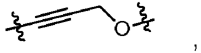 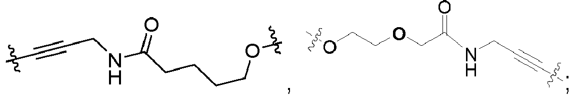
r¹, r², r^{3a}, r^{3b}, independently of each other, are selected from any integer between 1 and 6;
preferably, r¹ is selected from 1, 2, 3;
more preferably, r¹ is 1;
preferably, r² is selected from 1, 2, 3;
more preferably, r² is 1;
preferably, r^{3a}, r^{3b}, independently of each other, are selected from 1, 2, 3, 4, 5;
more preferably, r^{3a}, r^{3b}, independently of each other, are selected from 1, 2, 3;
most preferably, r^{3a} is 1;
most preferably, r^{3b} is 2;
M is selected from a direct bond, CH₂, NH, O, S;
preferably, M is selected from CH₂, O;
X is selected from O, S, NH;
preferably, X is selected from O, S;
more preferably, X is O;
Y is selected from a direct bond, O, S, NH;
preferably, Y is a direct bond;
W is selected from a direct bond, O, S, NH;
preferably, W is a direct bond;
R is 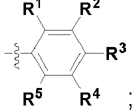
in R¹, R², R³, R⁴, R⁵, any one (e.g., R¹ or R⁵) is 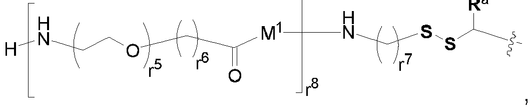 and the others, independently of each other, are selected from H, azido, nitro, amino, sulfo, carboxyl, aliphatic alkyl (e.g., C₁-C₆ alkyl), cycloalkyl (e.g., C₃-C₆ cycloalkyl), aromatic alkyl (e.g., phenyl -C₁-C₆ alkyl), F, I, Br, Cl, alkoxyl (e.g., C₁-C₆ alkoxyl);
preferably, in R¹, R², R³, R⁴, R⁵, any one (e.g., R¹ or R⁵) is 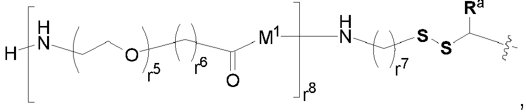 and the others are H;
more preferably, in R¹, R², R³, R⁴, R⁵, R¹ is 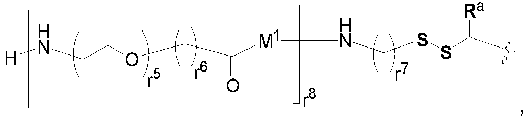 and the others are H;
most preferably, R is selected from 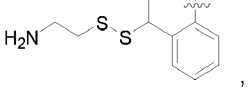 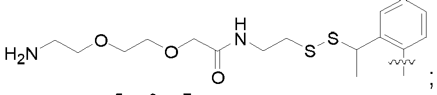
r^{b}, r⁶, r⁷, independently of each other, are selected from any integer between 1 and 6;
preferably, r⁵ is selected from 1, 2, 3;
more preferably, r⁵ is 2;
preferably, r⁶ is selected from 1, 2, 3;
more preferably, r⁶ is 1;
preferably, r⁷ is selected from 1, 2, 3;
more preferably, r⁷ is 2;
r⁸ is selected from 0, 1;
M¹ is selected from a direct bond, NH, O, S;
preferably, M¹ is a direct bond;
R^{a} is selected from H, aliphatic alkyl (e.g., C₁-C₆ alkyl, such as Me, Et, iPr, tBu), aromatic alkyl (e.g., phenyl -C₁-C₆ alkyl), cycloalkyl (e.g., C₃-C₆ cycloalkyl);
preferably, R^{a} is selected from C₁-C₆ alkyl;
more preferably, R^{a} is methyl;
Z is selected from O, S, BH;
preferably, Z is O;
base¹ is selected from a base, a deaza base or a tautomer thereof, for example, adenine, 7-deazaadenine, thymine, uracil, cytosine, guanine, 7-deazaguanine or a tautomer thereof;
preferably, base¹ is selected from 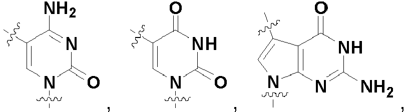 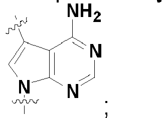
R' represents a reversible blocking group.

4. The compound or a salt thereof according to claim 1, wherein the compound is represented by the formula II-1, 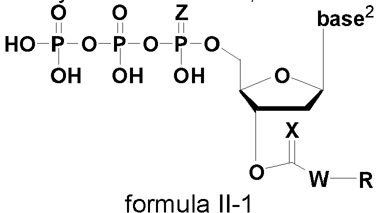 wherein:
X is selected from O, S, NH;
preferably, X is selected from O, S;
more preferably, X is O;
W is selected from a direct bond, O, S, NH;
preferably, W is a direct bond;
R is 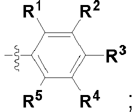
in R¹, R², R³, R⁴, R⁵, any one (e.g., R³ or R⁴) is 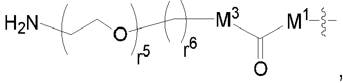 and the others, independently of each other, are selected from H, 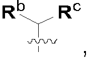 azido, nitro, amino, sulfo, carboxyl, aliphatic alkyl (e.g., C₁-C₆ alkyl), cycloalkyl (e.g., C₃-C₆ cycloalkyl), aromatic alkyl (e.g., phenyl -C₁-C₆ alkyl), F, I, Br, Cl, alkoxyl (e.g., C₁-C₆ alkoxyl);
preferably, in R¹, R², R³, R⁴, R⁵, any one (e.g., R³ or R⁴) is 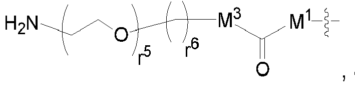 and the others, independently of each other, are selected from H, 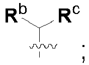
more preferably, in R¹, R², R³, R⁴, R⁵, any one (e.g., R³ or R⁴) is 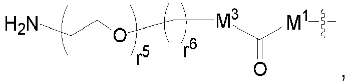 another (e.g., R¹ or R⁵) is 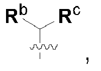 and the remaining three are H;
further preferably, in R¹, R², R³, R⁴, R⁵, R³ or R⁴ is 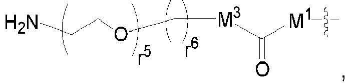 R¹ is 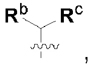 and the remaining three are H;
most preferably R is selected from H₂N 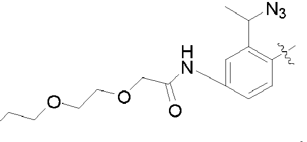 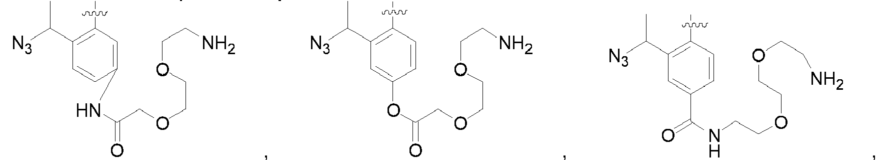 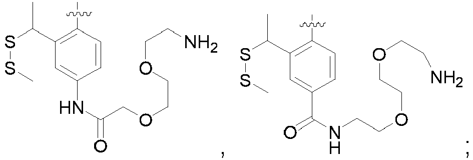
r⁵, r⁶, independently of each other, are selected from any integer between 1 and 6;
preferably, r⁵ is selected from 1, 2, 3;
more preferably, r⁵ is 2;
preferably, r⁶ is selected from 1, 2, 3;
more preferably, r⁶ is selected from 1, 2;
M¹, M³, independently of each other, are selected from a direct bond, NH, O, S;
preferably, M¹ is selected from a direct bond, NH, O;
preferably, M³ is selected from a direct bond, NH;
in R^{b}, R^{c}, any one is selected from -N₃, -SS-C₁-C₆ alkyl (e.g., -SS-Me, -SS-Et, -SS-iPr, -SS-t-Bu), -ONH₂, -OCORₓ, -OCONHRₓ, and the other is selected from H, aliphatic alkyl (e.g., C₁-C₆ alkyl, such as Me, Et, iPr, tBu), aromatic alkyl (e.g., phenyl -C₁-C₆ alkyl), cycloalkyl (e.g., C₃-C₆ cycloalkyl), wherein each Rₓ, independently, is selected from aliphatic alkyl (e.g., C₁-C₆ alkyl), cycloalkyl (e.g., C₃-C₆ cycloalkyl) or aromatic alkyl (e.g., phenyl C₁-C₆ alkyl);
preferably, in R^{b}, R^{c}, any one is selected from -N₃, -SS-C₁-C₆ alkyl (e.g., -SS-Me, -SS-Et, -SS-iPr, -SS-t-Bu), and the other is selected from C₁-C₆ alkyl;
more preferably, in R^{b}, R^{c}, any one is -N₃ or -SS-Me, and the other is methyl;
Z is selected from O, S, BH;
preferably, Z is O;
base² is selected from a base, a deaza base or a tautomer thereof, for example, adenine, 7-deazaadenine, thymine, uracil, cytosine, guanine, 7-deazaguanine or a tautomer thereof;
preferably, base² is selected from 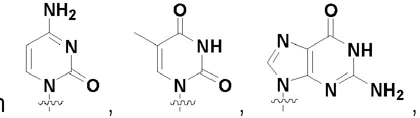 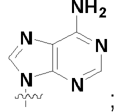 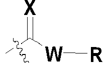 represents a reversible blocking group.

5. The compound or a salt thereof according to claim 1, wherein the compound is represented by the formula II-2, 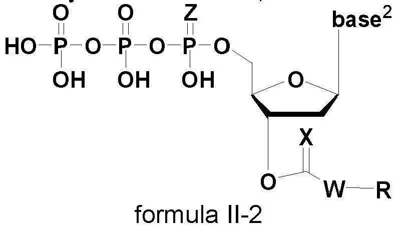 wherein:
X is selected from O, S, NH;
preferably, X is selected from O, S;
more preferably, X is O;
W is selected from a direct bond, O, S, NH;
preferably, W is a direct bond;
R is 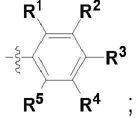
in R¹, R², R³, R⁴, R⁵, any one (e.g., R¹ or R⁵) is 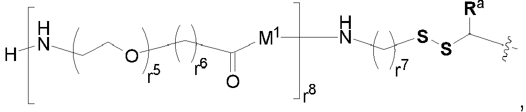 and the others, independently of each other, are selected from H, azido, nitro, amino, sulfo, carboxyl, aliphatic alkyl (e.g., C₁-C₆ alkyl), cycloalkyl (e.g., C₃-C₆ cycloalkyl), aromatic alkyl (e.g., phenyl -C₁-C₆ alkyl), F, I, Br, Cl, alkoxyl (e.g., C₁-C₆ alkoxyl);
preferably, in R¹, R², R³, R⁴, R⁵, any one (e.g., R¹ or R⁵) is 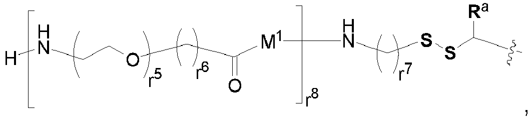 and the others are H;
more preferably, in R¹, R², R³, R⁴, R⁵, R¹ is 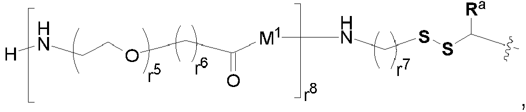 and the others are H;
most preferably, R is selected from 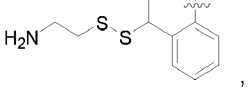 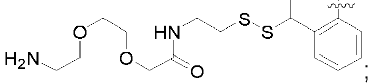
r⁵, r⁶, r⁷ are selected from any integer between 1 and 6;
preferably, r⁵ is selected from 1, 2, 3;
more preferably, r⁵ is 2;
preferably, r⁶ is selected from 1, 2, 3;
more preferably, r⁶ is 1;
preferably, r⁷ is selected from 1, 2, 3;
more preferably, r⁷ is 2;
r⁸ is selected from 0, 1;
M¹ is selected from a direct bond, NH, O, S;
preferably, M¹ is a direct bond;
R^{a} is selected from H, aliphatic alkyl (e.g., C₁-C₆ alkyl, such as Me, Et, iPr, tBu), aromatic alkyl (e.g., phenyl -C₁-C₆ alkyl), cycloalkyl (e.g., C₃-C₆ cycloalkyl);
preferably, R^{a} is selected from C₁-C₆ alkyl;
more preferably, R^{a} is methyl;
Z is selected from O, S, BH;
preferably, Z is O;
base² is selected from a base, a deaza base or a tautomer thereof, for example, adenine, 7-deazaadenine, thymine, uracil, cytosine, guanine, 7-deazaguanine or a tautomer thereof;
preferably, base² is selected from 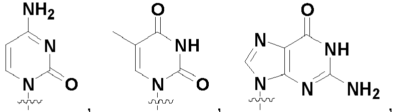 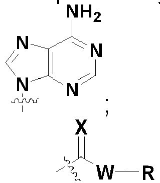 represents a reversible blocking group.

6. The compound or a salt thereof according to any one of claims 1 to 3, wherein the reversible blocking group R' is selected from N₃-C₁-C₆ alkyl, C₁-C₆ alkyl-SS-C₁-C₆ alkyl, 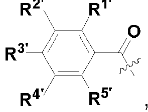 NH₂, -ONH₂, -OCOR_{z}, -OCONHR_{z}, wherein each R_{z}, independently, is selected from aliphatic alkyl (e.g., C₁-C₆ alkyl), cycloalkyl (e.g., C₃-C₆ cycloalkyl) or aromatic alkyl (e.g., phenyl C₁-C₆ alkyl);
preferably, the reversible blocking group R' is selected from N₃-C₁-C₆ alkyl, C₁-C₆ alkyl-SS-C₁-C₆ alkyl, 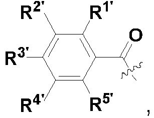 NH₂;
more preferably, the reversible blocking qroup R' is selected from N₃-C₁-C₆ alkyl, C₁-C₆ alkyl-SS-C₁-C₆ alkyl, 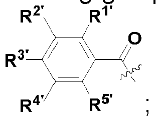
most preferably, the reversible blocking group R' is selected from N₃-CH₂-, CH₃-CH₂-S-S-CH₂-, 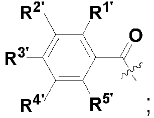
in R^{1'}, R^{2'}, R^{3'}, R^{4'}, R^{5'}, any one (e.g., R^{1'} or R^{5'}) is 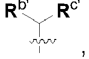 and the others, independently of each other, are selected from H, azido, nitro, amino, sulfo, carboxyl, aliphatic alkyl (e.g., C₁-C₆ alkyl), cycloalkyl (e.g., C₃-C₆ cycloalkyl), aromatic alkyl (e.g., phenyl -C₁-C₆ alkyl), F, I, Br, Cl, alkoxyl (e.g., C₁-C₆ alkoxyl),
preferably, in R^{1'}, R^{2'}, R^{3'}, R^{4'}, R^{5'}, any one (e.g., R^{1'} or R^{5'}) is 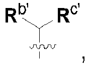 and the others are H,
more preferably, in R^{1'}, R^{2'}, R^{3'}, R^{4'}, R^{5'}, R^{1'} is 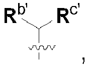 and the others are H,
in R^{b'}, R^{c'}, any one is selected from -N₃, -SS-C₁-C₆ alkyl (e.g., -SS-Me, -SS-Et, -SS-iPr, -SS-t-Bu), -ONH₂, -OCORₓ, -OCONHRₓ, and the other is selected from H, aliphatic alkyl (e.g., C₁-C₆ alkyl, such as Me, Et, iPr, tBu), aromatic alkyl (e.g., phenyl -C₁-C₆ alkyl), cycloalkyl (e.g., C₃-C₆ cycloalkyl), wherein each Rₓ, independently, is selected from aliphatic alkyl (e.g., C₁-C₆ alkyl), cycloalkyl (e.g., C₃-C₆ cycloalkyl) or aromatic alkyl (e.g., phenyl C₁-C₆ alkyl),
preferably, in R^{b'}, R^{c'}, any one is selected from -N₃, -SS-C₁-C₆ alkyl (e.g., -SS-Me, -SS-Et, -SS-iPr, -SS-t-Bu), and the other is selected from C₁-C₆ alkyl,
more preferably, in R^{b'}, R^{c'}, any one is selected from -N₃, -SS-Me, and the other is methyl.

7. A compound of formula III or a salt thereof, 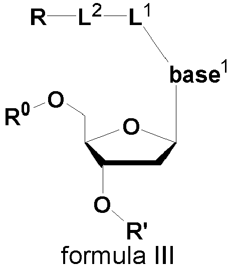 wherein:
L¹ is selected from 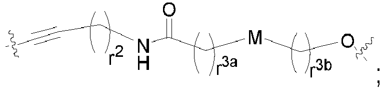
preferably, L¹ is selected from 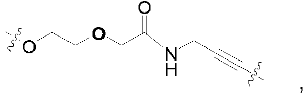 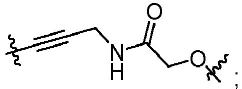
r² is selected from 1, 2, 3;
preferably, r² is 1;
r^{3a} is selected from 1, 2, 3;
preferably, r^{3a} is 1;
r^{3b} is selected from 0, 1, 2, 3;
preferably, r^{3b} is selected from 0, 2;
M is selected from a direct bond, O;
L² is 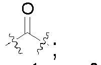
R is 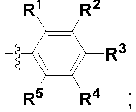
in R¹, R², R³, R⁴, R⁵, any one (e.g., R³) is 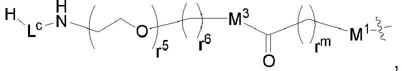 another e., R¹ or R² is selected from 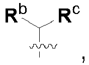 and the remaining three are H;
L^{c} is selected from a direct bond, 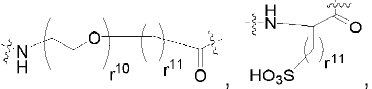 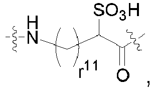 preferably, the NH end in L^{c} is linked to H, the =O end in L^{c} is linked to NH;
preferably, L^{c} is selected from a direct bond, 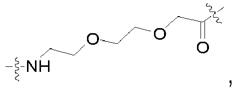 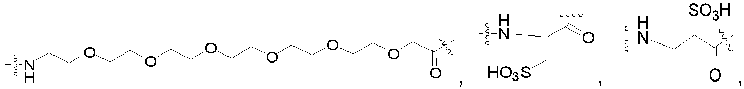 preferably, the NH end in L^{c} is linked to H, the =O end in L^{c} is linked to NH;
r^{m} is selected from 0, 1, 2, 3;
preferably, r^{m} is selected from 0, 1;
r⁵ is selected from 1, 2, 3;
preferably, r⁵ is 2;
r⁶ is selected from 1, 2, 3;
preferably, r⁶ is 1 or 2;
r¹⁰ is selected from any integer between 1 and 10;
preferably, r¹⁰ is selected from any integer between 2 and 6;
more preferably, r¹⁰ is 2 or 6;
r¹¹ is selected from any integer between 1 and 6;
preferably, r¹¹ is selected from 1, 2, 3;
more preferably, r¹¹ is 1;
M¹ is selected from NH, O;
M³ is selected from a direct bond, NH;
in R^{b}, R^{c}, any one is selected from -SS-C₁-C₆ alkyl (e.g., -SS-Me, -SS-Et, -SS-iPr, -SS-t-Bu), and the other is selected from C₁-C₆ alkyl;
preferably, in R^{b}, R^{c}, any one is -SS-Me, and the other is methyl;
preferably, R is selected from 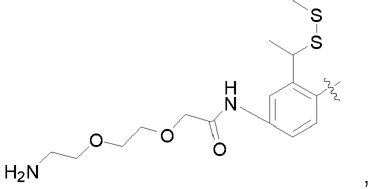 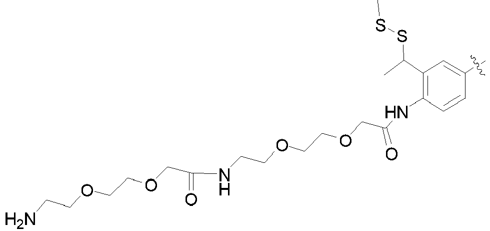 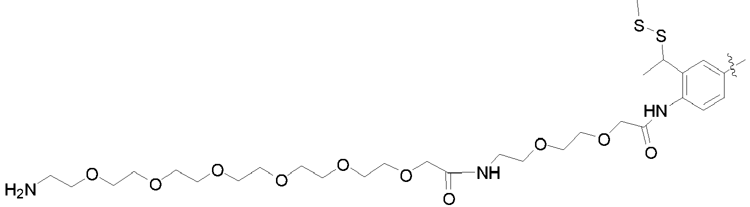 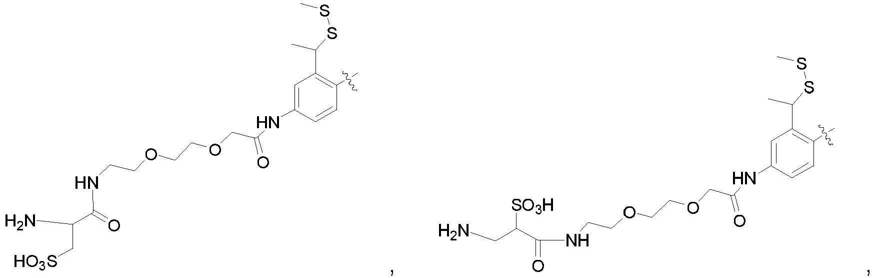 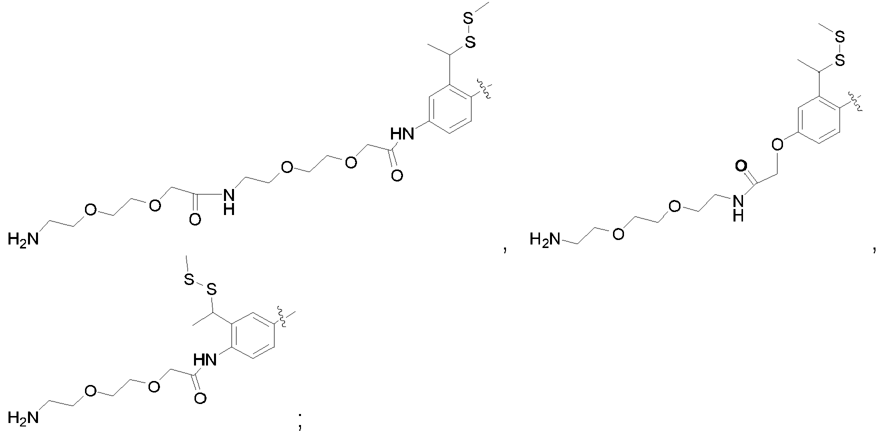
R⁰ is selected from H, monophosphate group 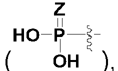 diphosphate group 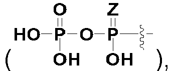 triphosphate group 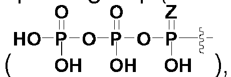 tetraphosphate group 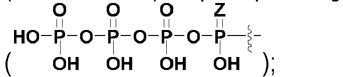
preferably, R° is a triphosphate group 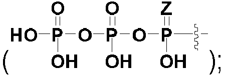
each Z, independently, is selected from O, S, BH;
preferably, Z is O;
base¹ is selected from a base, a deaza base or a tautomer thereof, for example, adenine, 7-deazaadenine, thymine, uracil, cytosine, guanine, 7-deazaquanine or a tautomer thereof;
preferably, base¹ is selected from 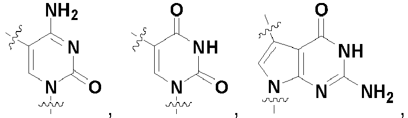 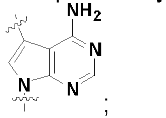 in formula III, R' represents a reversible blocking group.

8. The compound or a salt thereof according to claim 7, wherein the reversible blocking group R' is 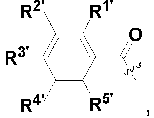
in R^{1'}, R^{2'}, R^{3'}, R^{4'}, R^{5'}, any one (e.g., R^{1'} or R^{5'}) is 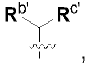 another (e.g., R^{3'}) is selected from H, C₁-C₆ alkoxyl (e.g., methoxy), and the others are H
preferably, in R^{1'}, R^{2'}, R^{3'}, R^{4'}, R^{5'}, R^{1'} is 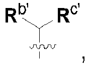 R^{3'} is selected from H, C₁-C₆ alkoxyl (e.g., methoxy), and the others are H,
in R^{b'}, R^{c'}, any one is selected from -N₃, -SS-C₁-C₆ alkyl (e.g., -SS-Me, -SS-Et, -SS-iPr, -SS-t-Bu), and the other is selected from C₁-C₆ alkyl,
preferably, in R^{b'}, R^{c'}, any one is -N₃, -SS-Me, and the other is methyl.

9. The compound or a salt thereof according to any one of claims 1 to 8, wherein the compound is selected from:
| Serial Number | Structural formulae |
|---|---|
| 1A | 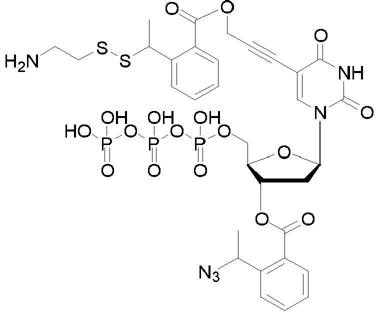 |
| 1B | 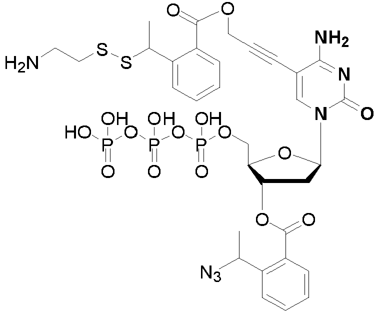 |
| 1C | 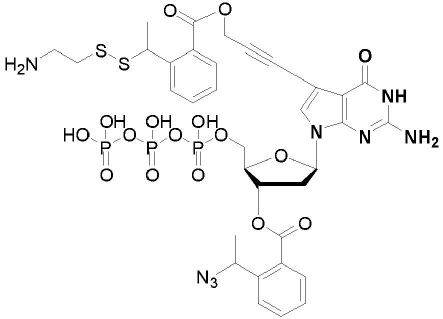 |
| 1D | 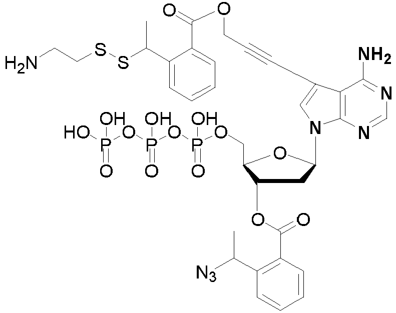 |
| 2A | 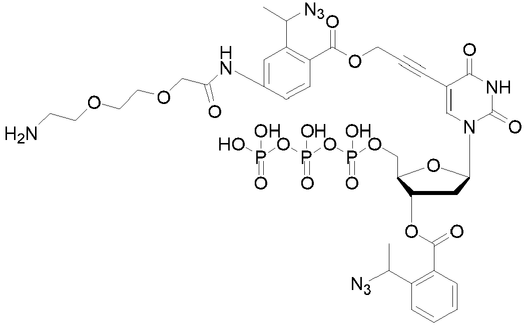 |
| 2B | 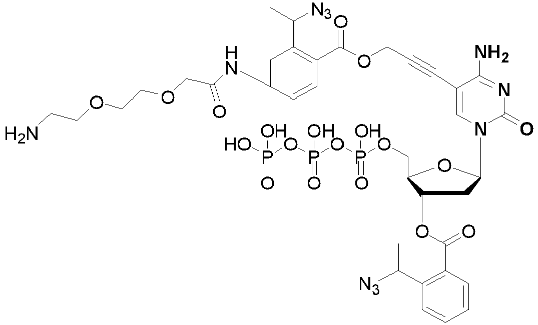 |
| 2C | 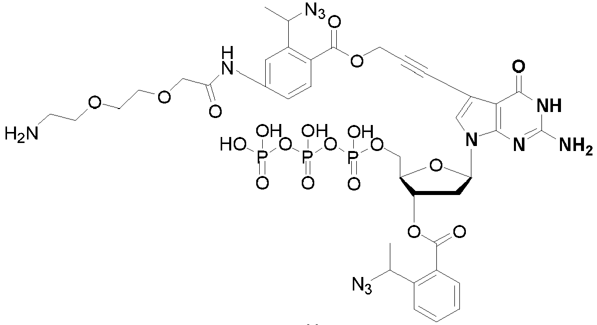 |
| 2D | 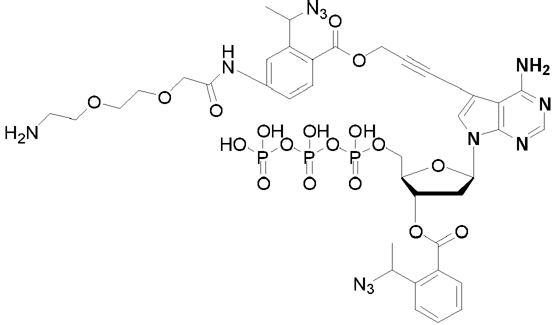 |
| 3A | 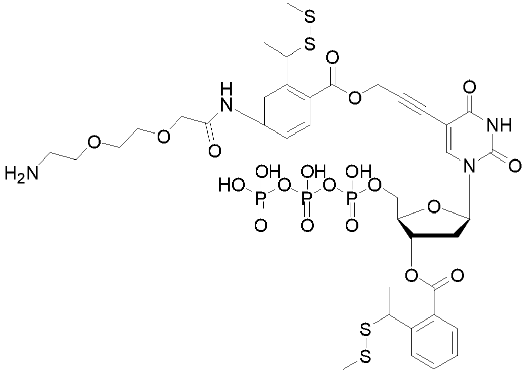 |
| 3B | 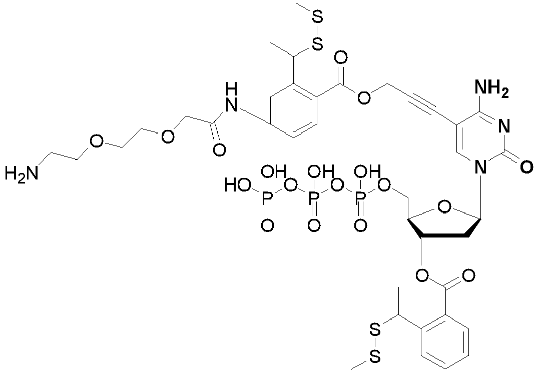 |
| 3C | 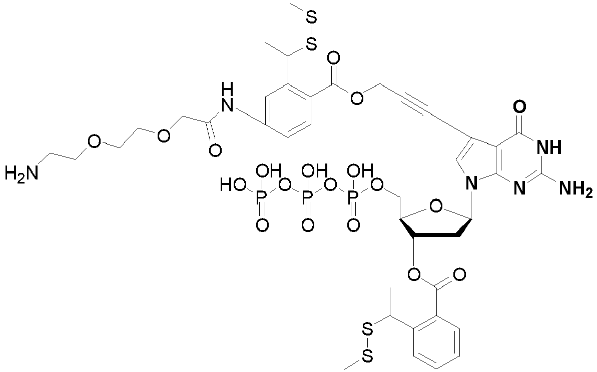 |
| 3D | 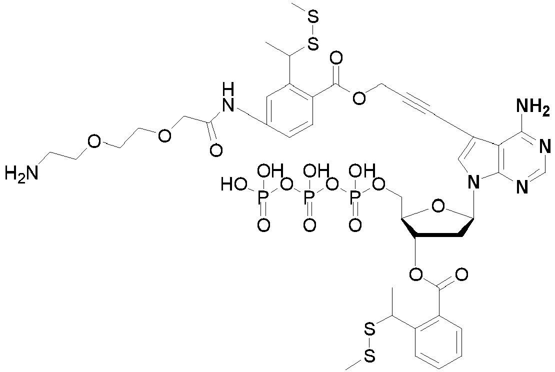 |
| 4A | 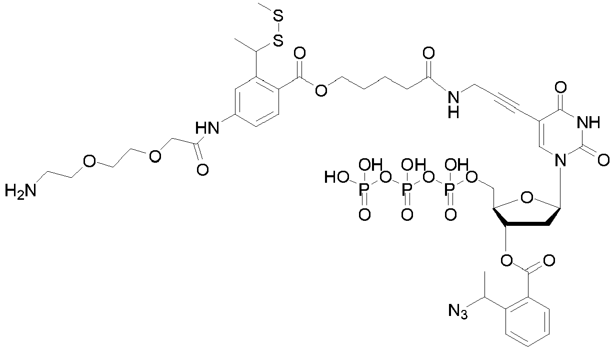 |
| 4B | 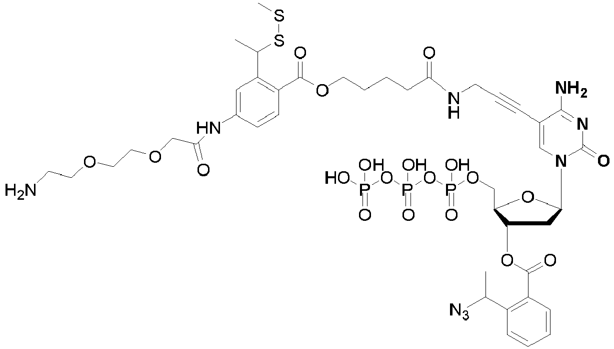 |
| 4C | 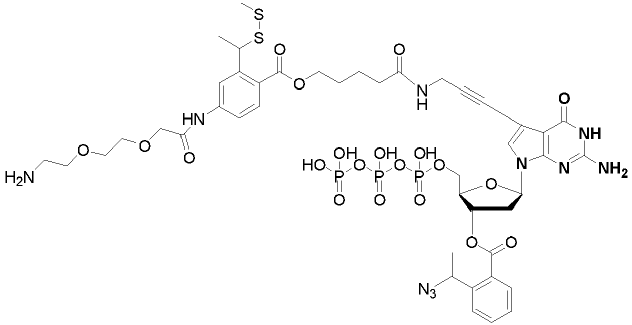 |
| 4D | 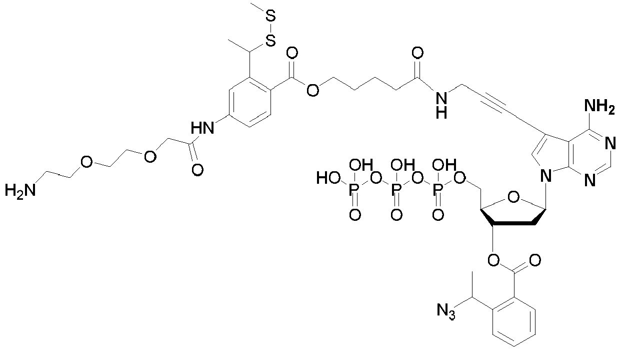 |
| 5A | 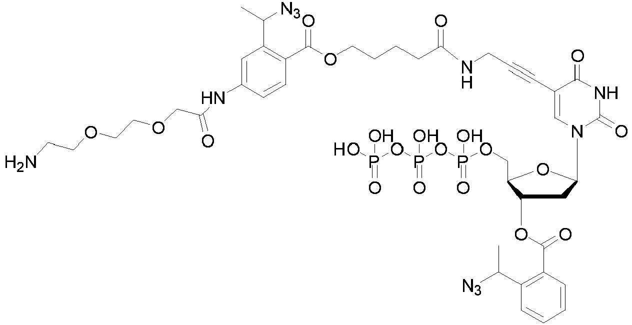 |
| 5B | 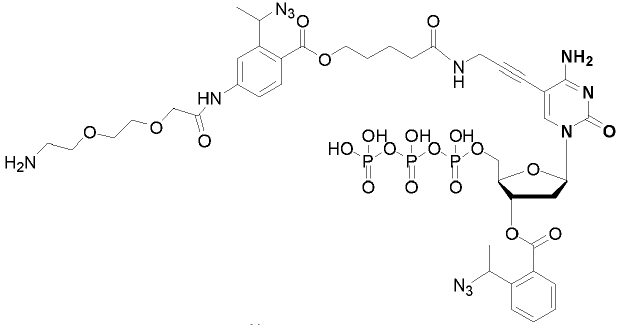 |
| 5C | 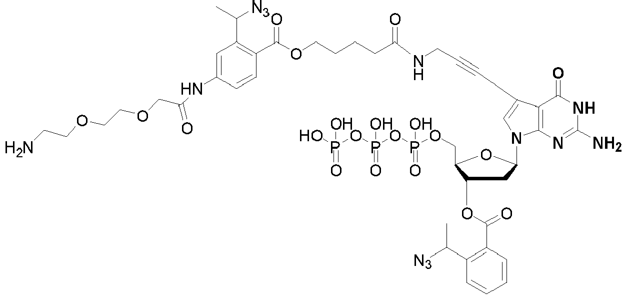 |
| 5D | 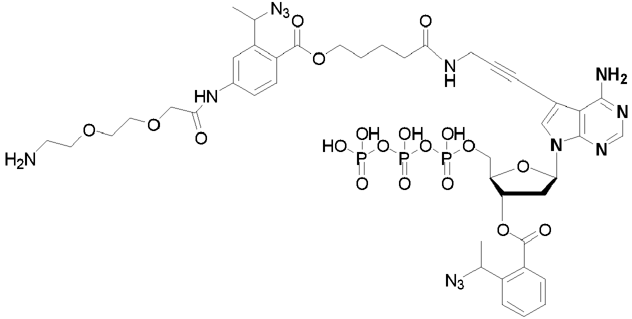 |
| 6A | 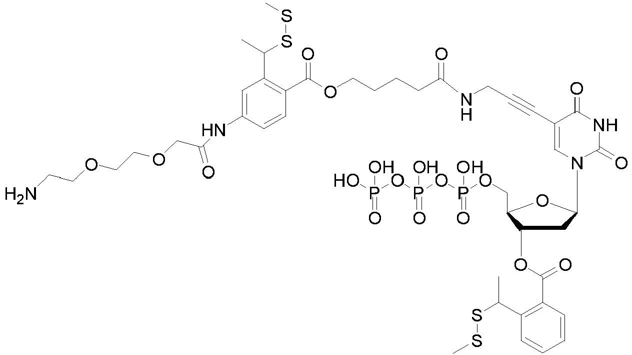 |
| 6B | 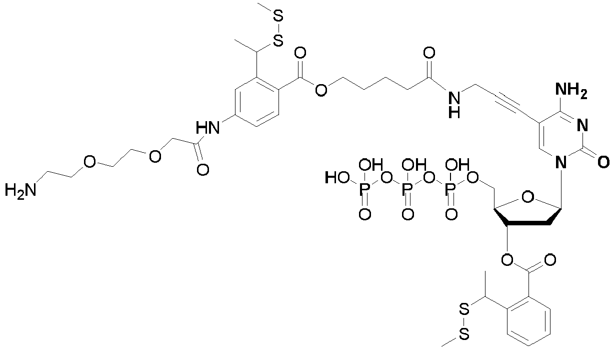 |
| 6C | 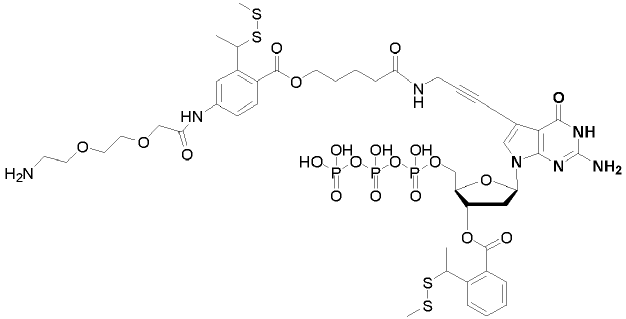 |
| 6D | 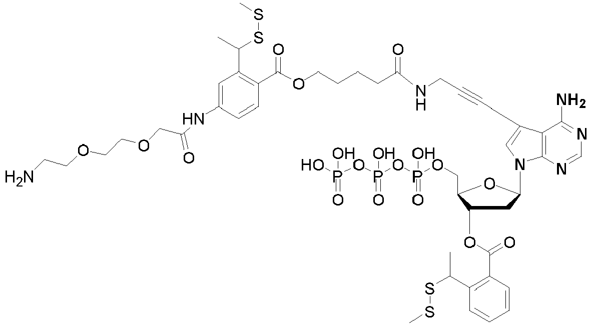 |
| 7A | 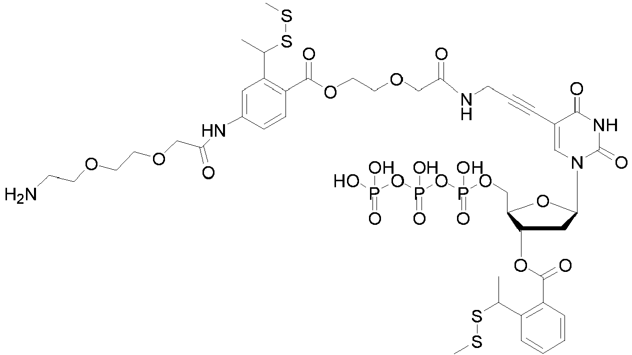 |
| 7B | 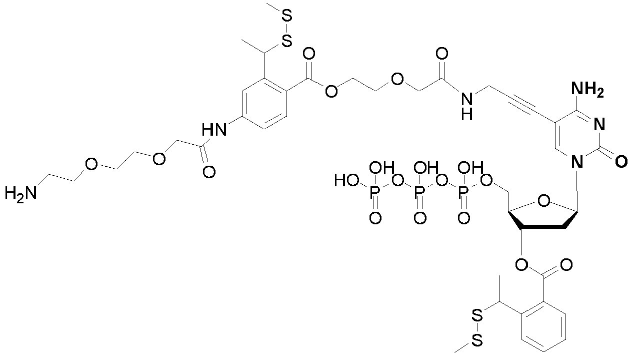 |
| 7C | 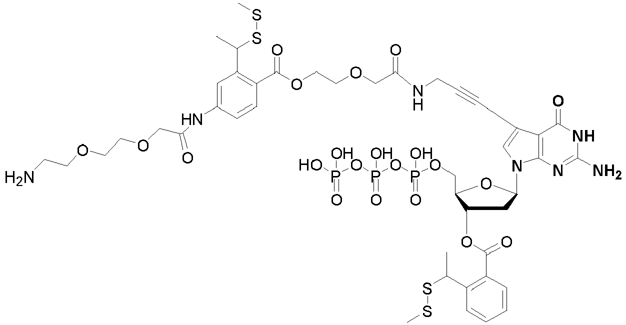 |
| 7D | 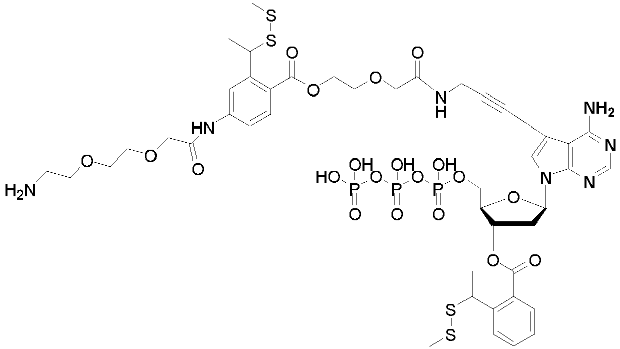 |
| 7D1 | 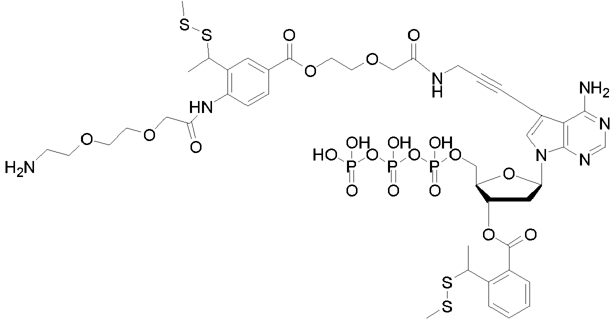 |
| 8-1A | 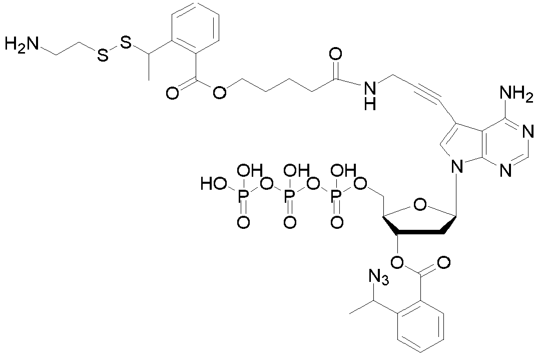 |
| 8-1B | 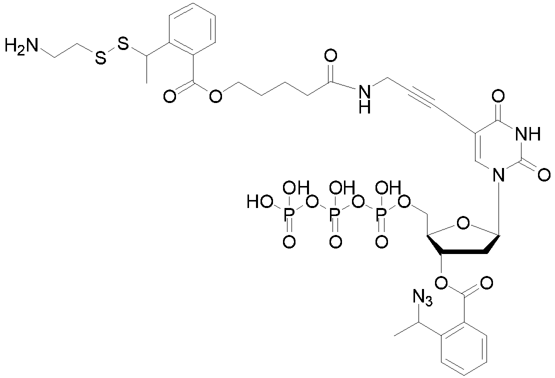 |
| 8-1C | 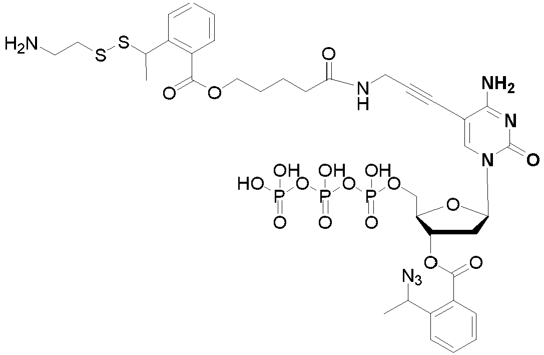 |
| 8-1D | 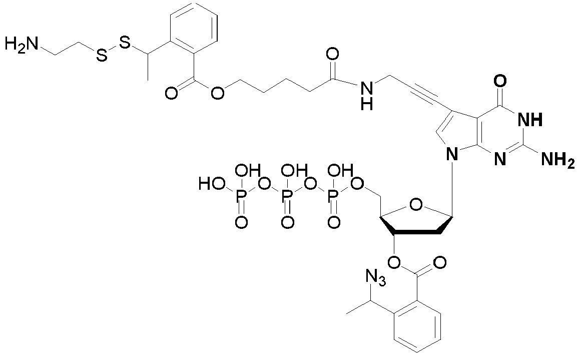 |
| 8-2A | 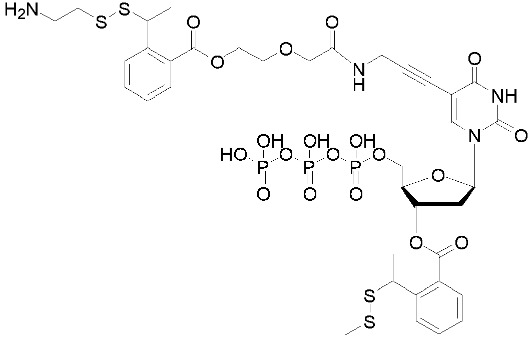 |
| 8-2 B | 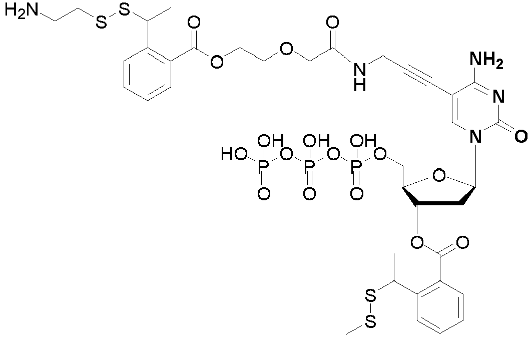 |
| 8-2C | 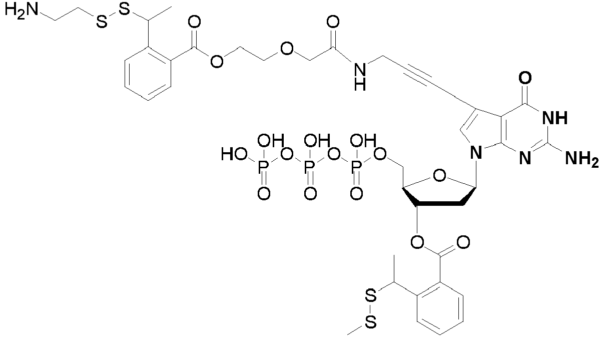 |
| 8-2 D | 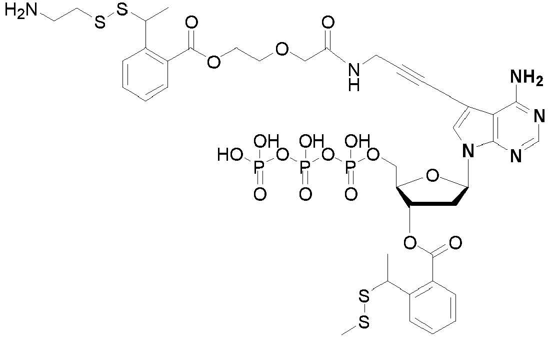 |
| 11A | 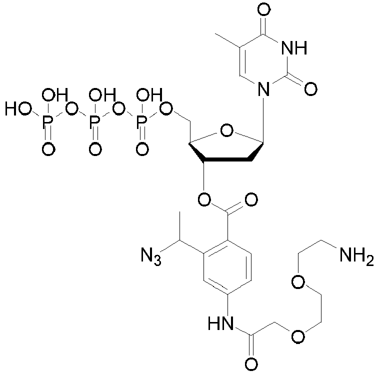 |
| 11B | 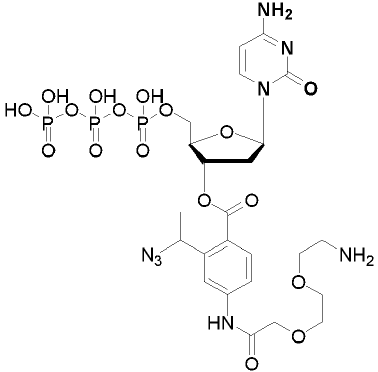 |
| 11C | 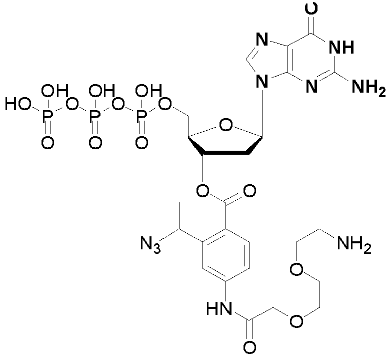 |
| 11D | 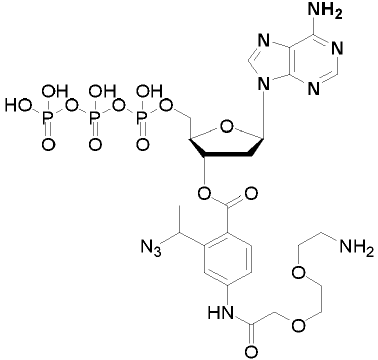 |
| 12A | 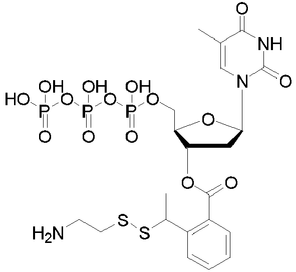 |
| 12B | 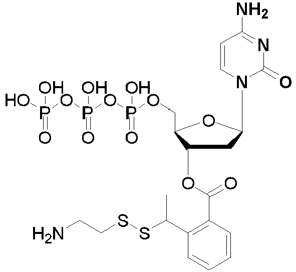 |
| 12C | 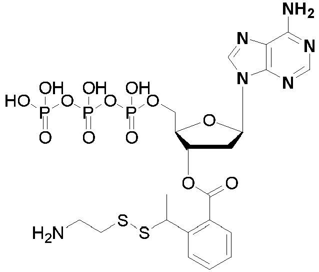 |
| 12D | 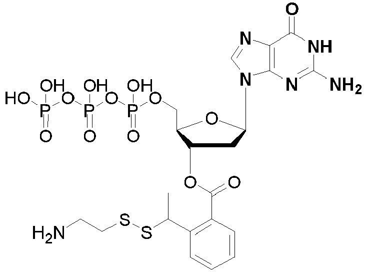 |
| 13A | 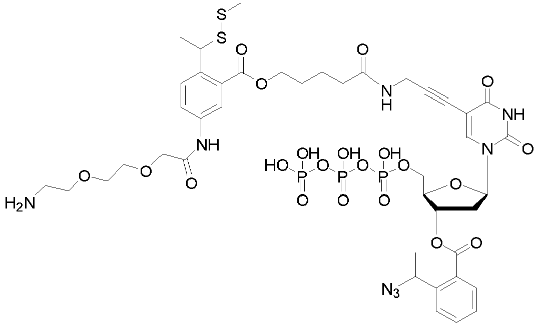 |
| 13B | 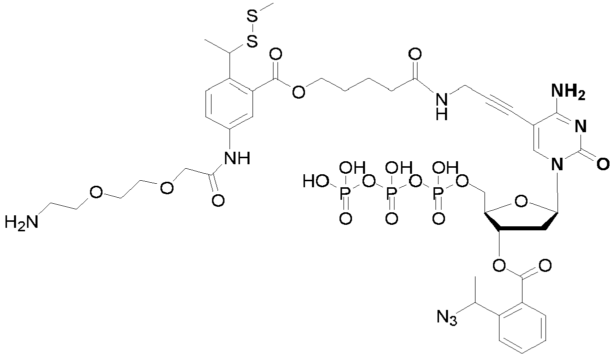 |
| 13C | 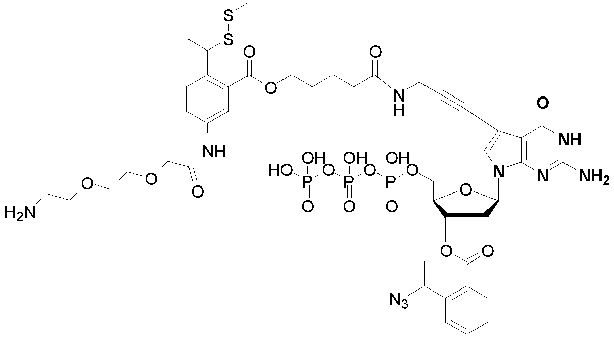 |
| 13D | 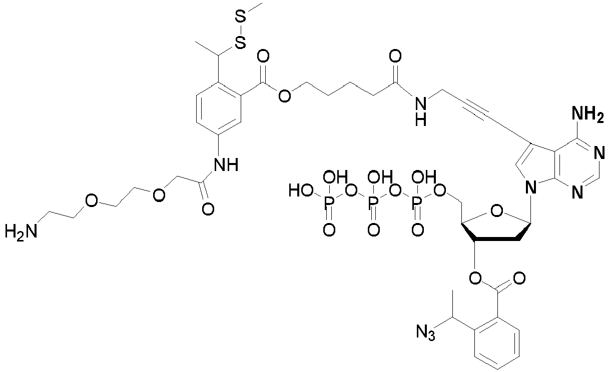 |
| 14A | 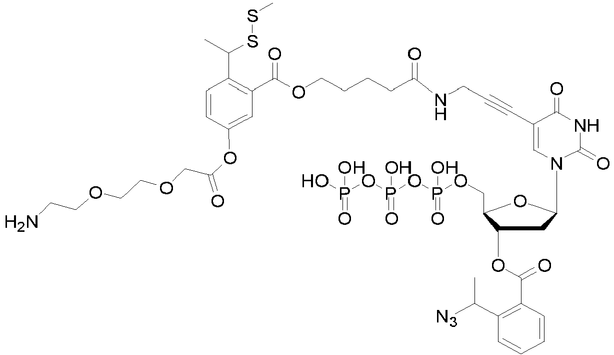 |
| 14B | 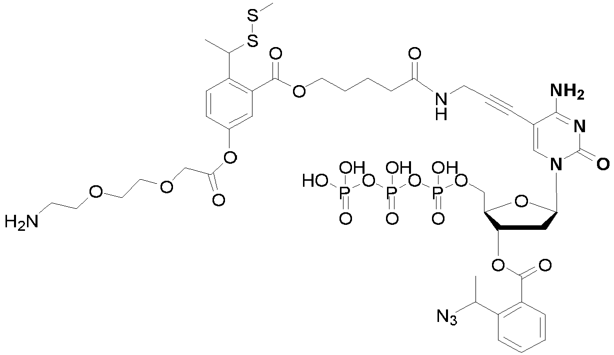 |
| 14C | 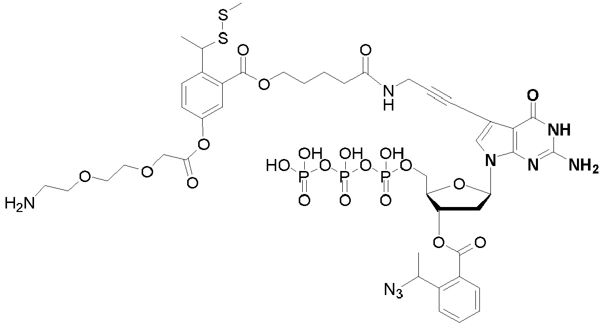 |
| 14D | 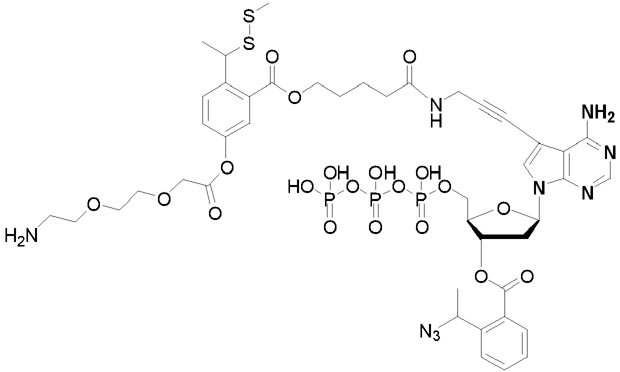 |
| 15A | 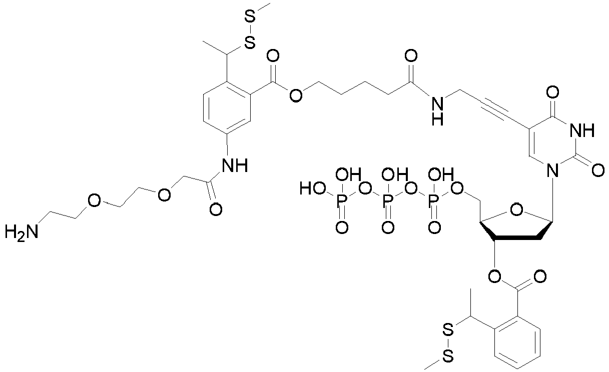 |
| 15B | 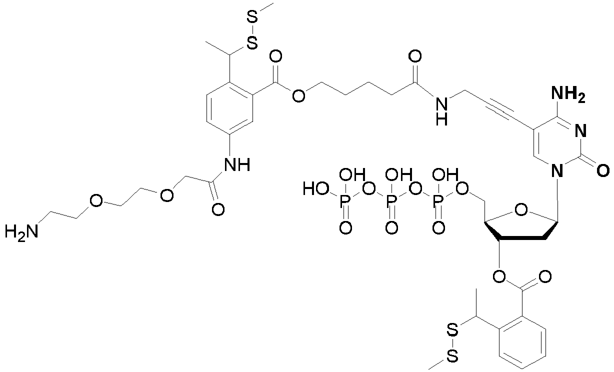 |
| 15C | 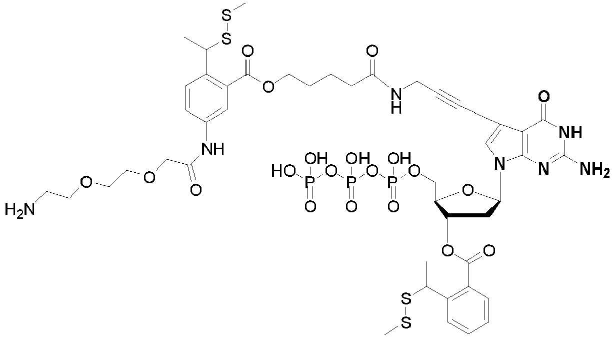 |
| 15D | 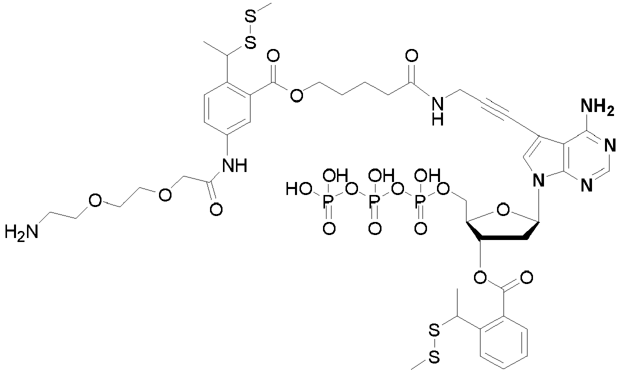 |
| 16A | 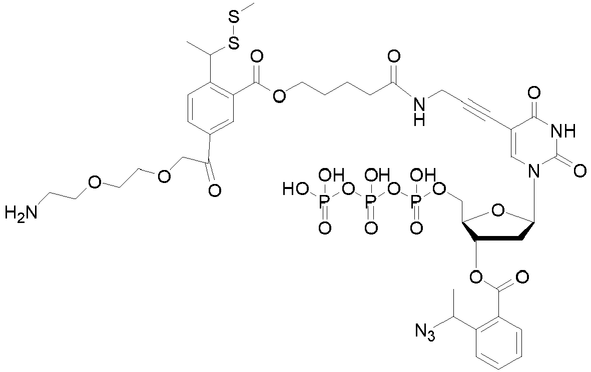 |
| 16B | 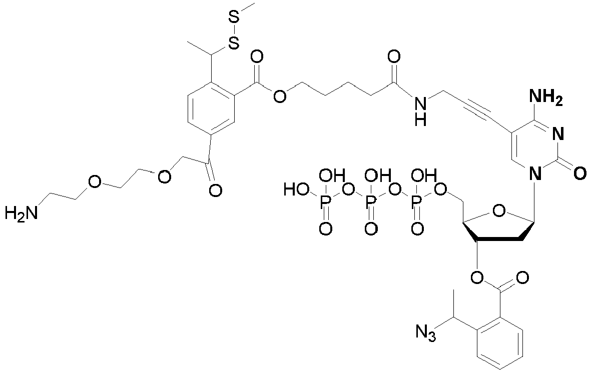 |
| 16C | 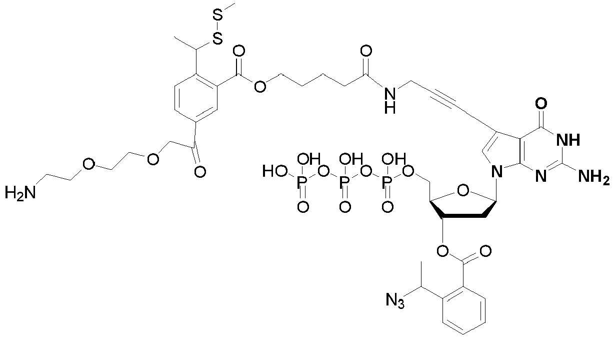 |
| 16D | 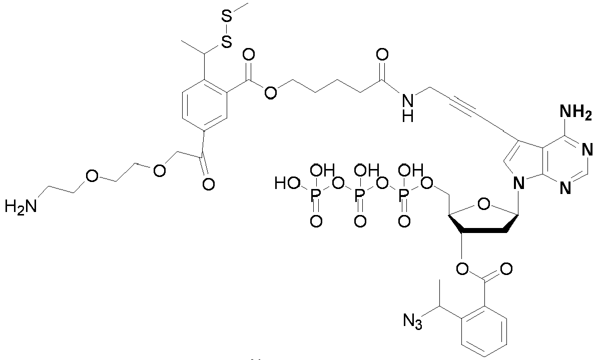 |
| 17A | 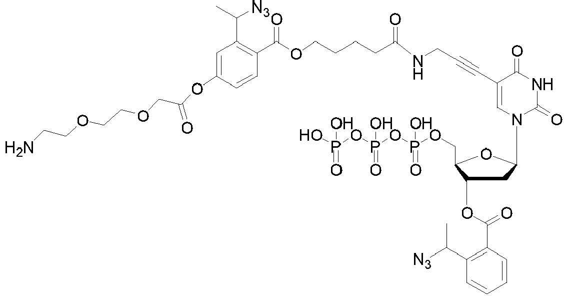 |
| 17B | 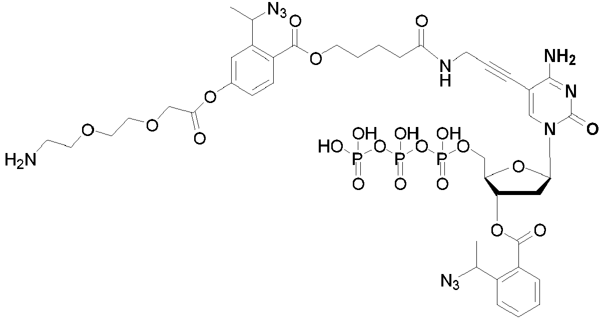 |
| 17C | 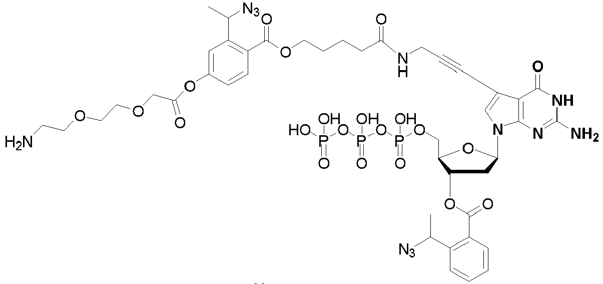 |
| 17D | 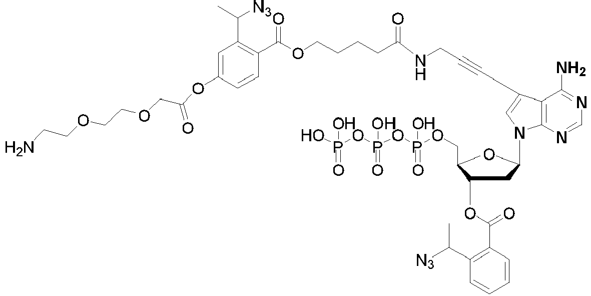 |
| 18A | 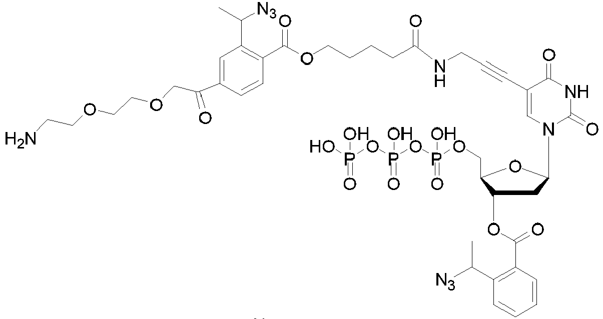 |
| 18B | 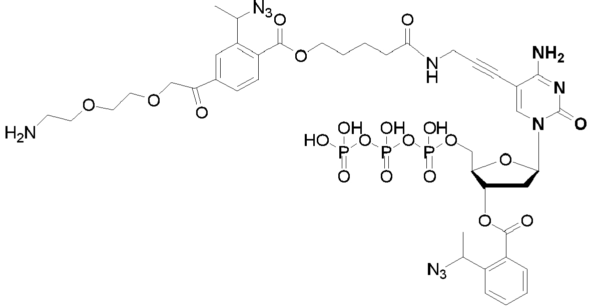 |
| 18C | 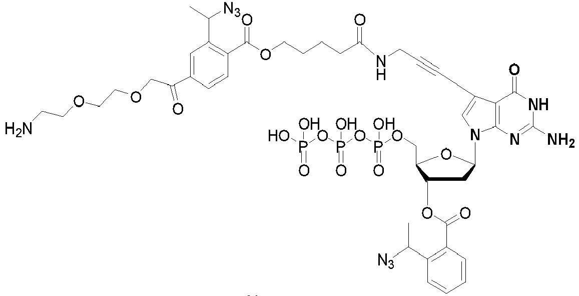 |
| 18D | 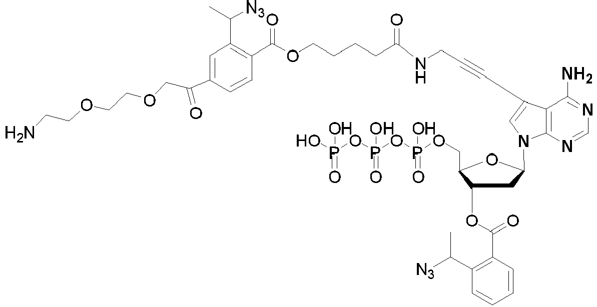 |
| 19A | 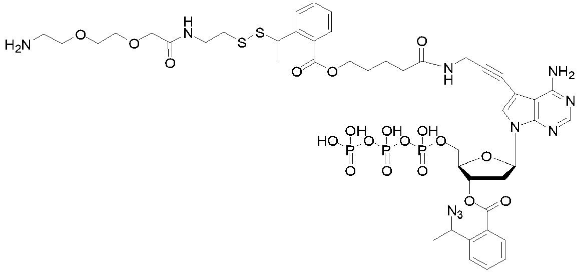 |
| 19B | 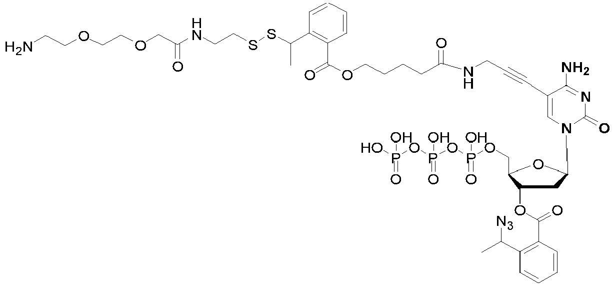 |
| 19C | 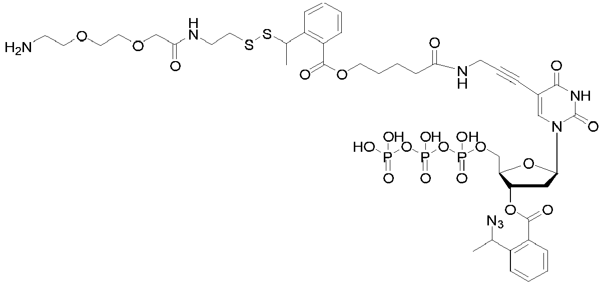 |
| 19D | 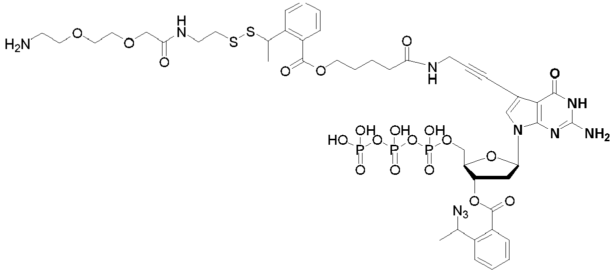 |
| 20A | 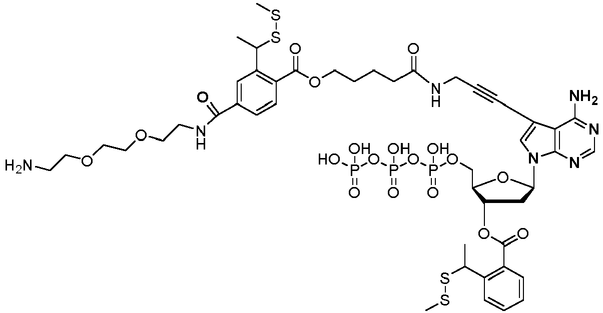 |
| 20B | 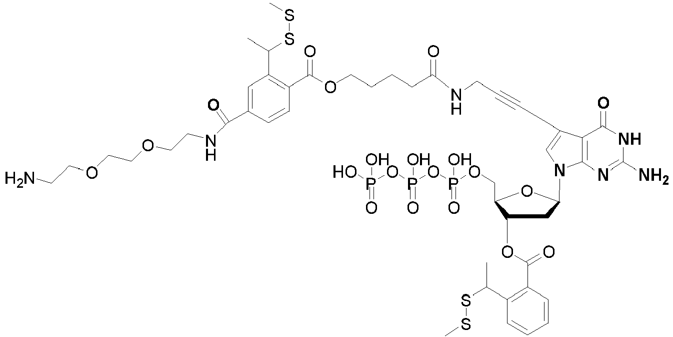 |
| 20C | 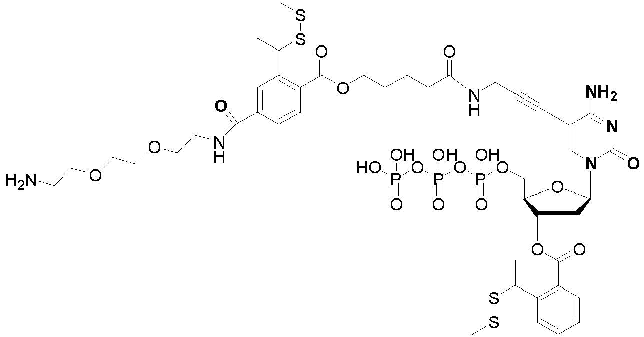 |
| 20D | 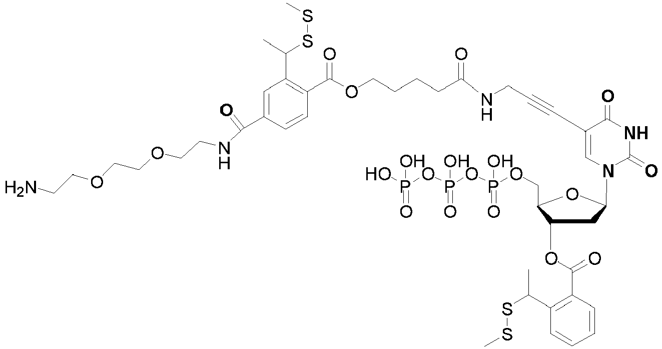 |
| 21A | 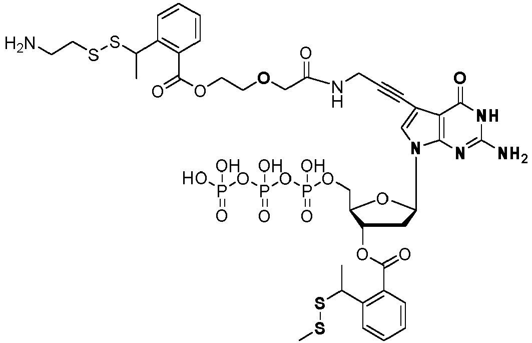 |
| 21B | 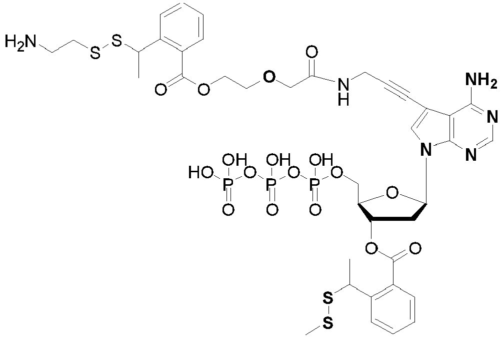 |
| 21C | 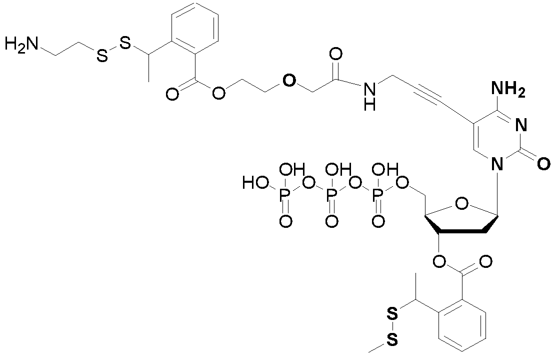 |
| 21D | 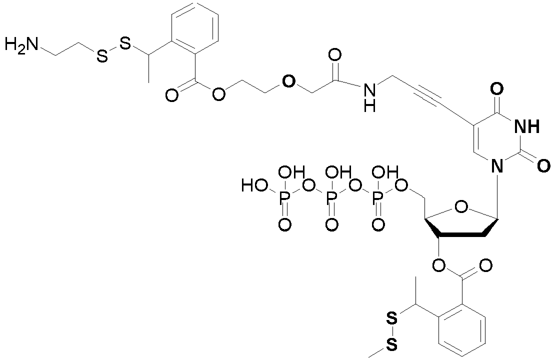 |
| 22A | 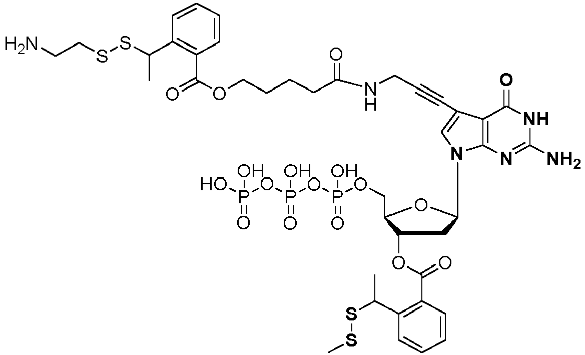 |
| 22B | 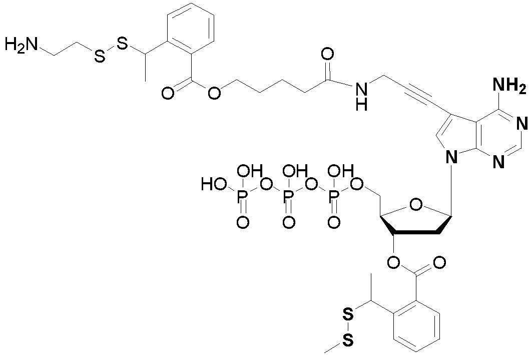 |
| 22C | 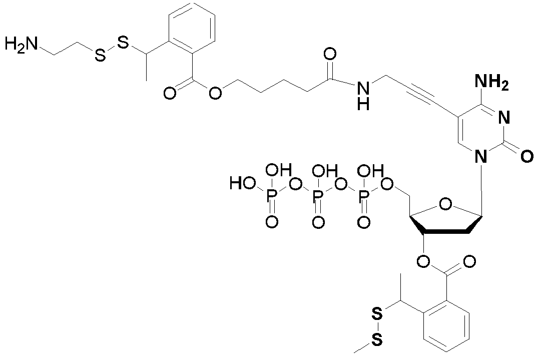 |
| 22D | 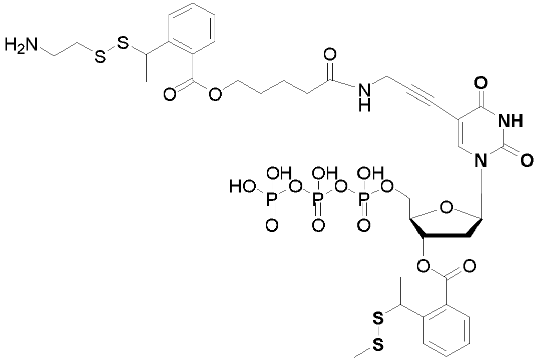 |
| 23A | 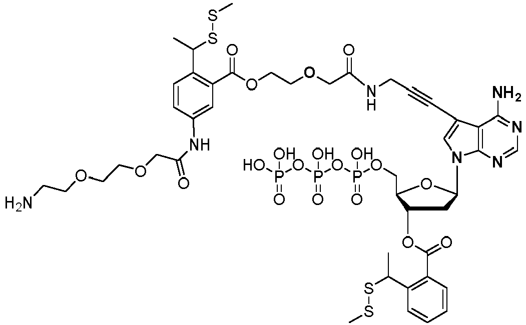 |
| 23B | 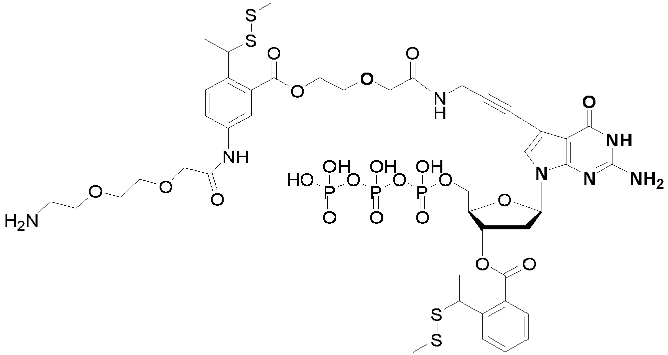 |
| 23C | 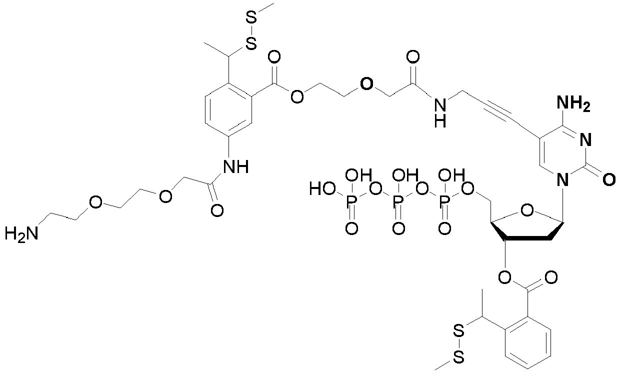 |
| 23D | 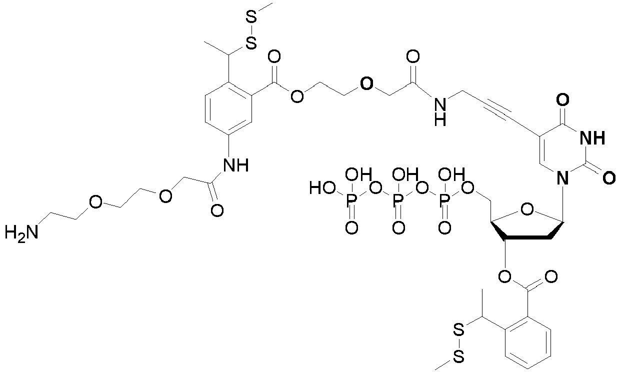 |
| 24A | 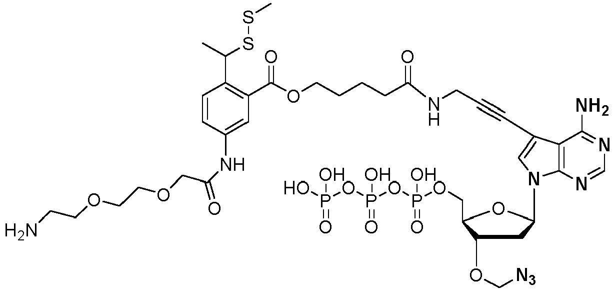 |
| 24B | 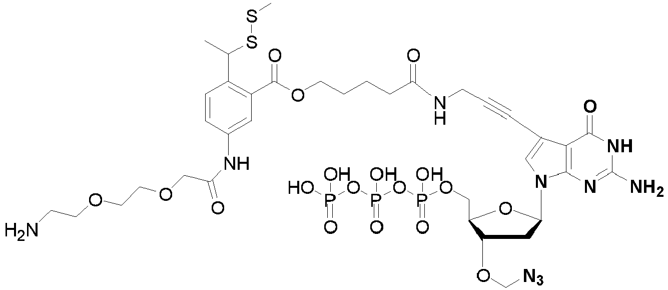 |
| 24C | 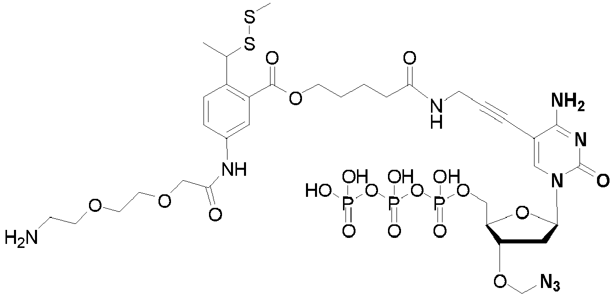 |
| 24D | 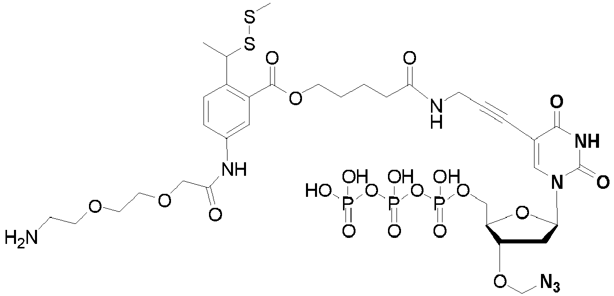 |
| 25A | 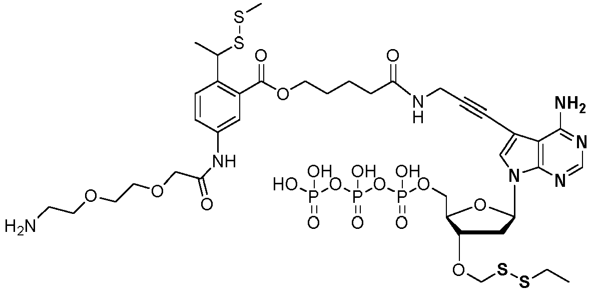 |
| 25B | 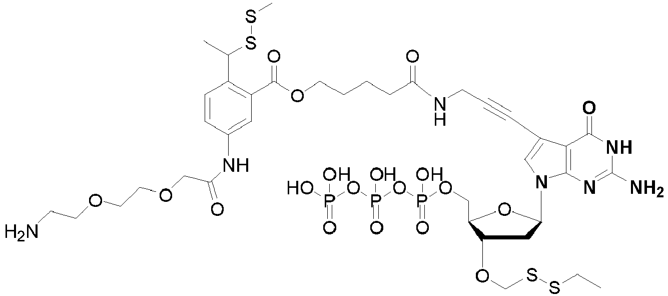 |
| 25C | 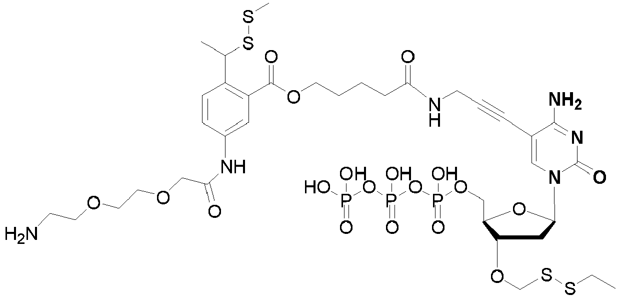 |
| 25D | 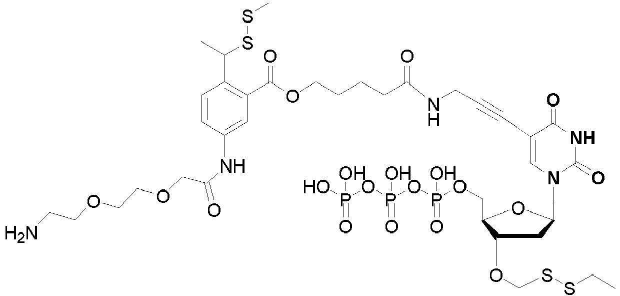 |
| 26A | 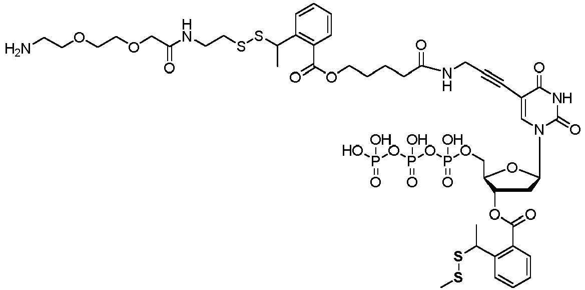 |
| 26B | 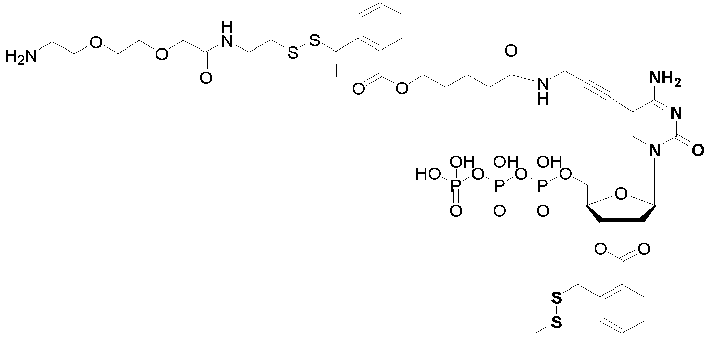 |
| 26C | 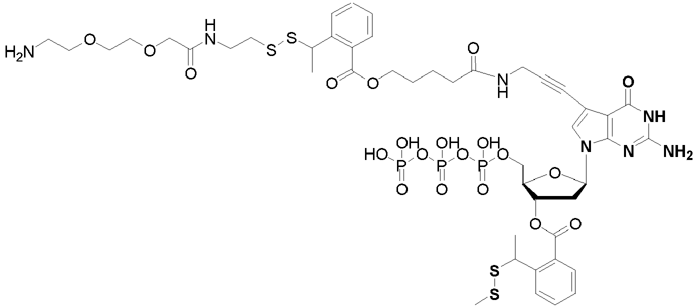 |
| 26D | 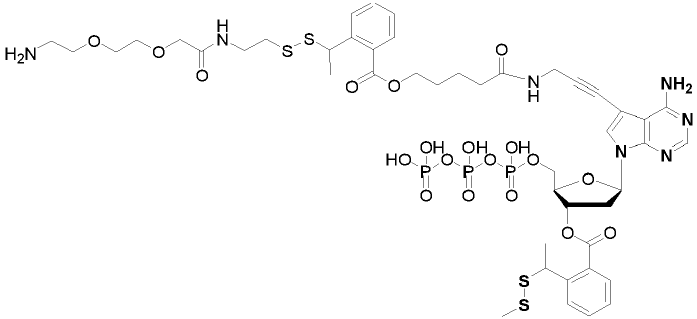 |
| 27A | 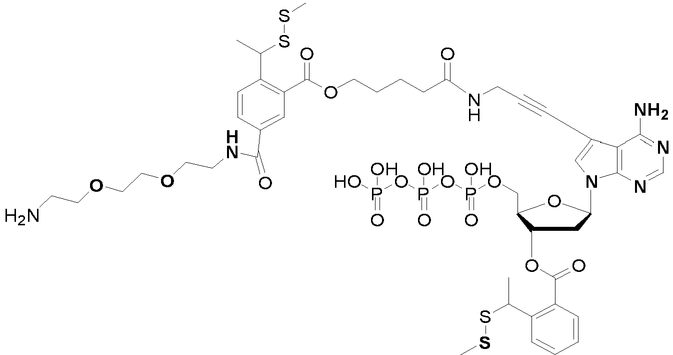 |
| 27B | 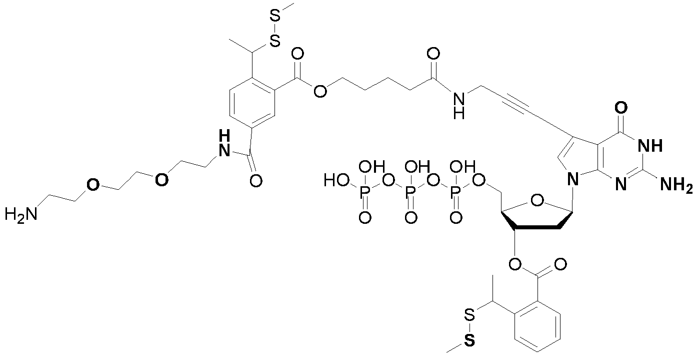 |
| 27C | 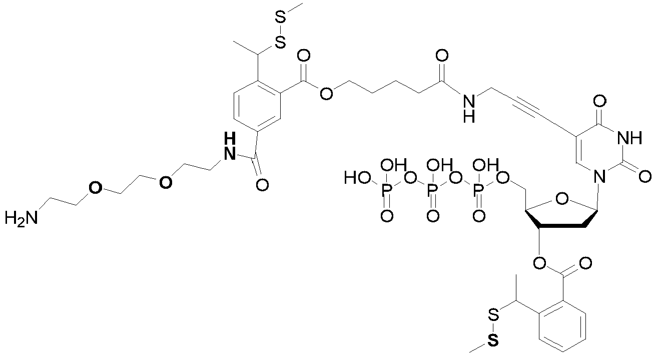 |
| 27D | 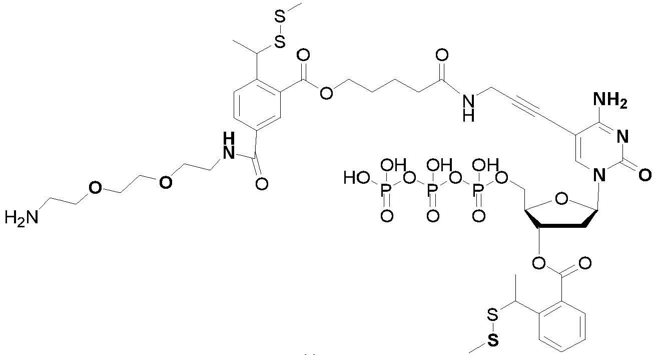 |
| 28A | 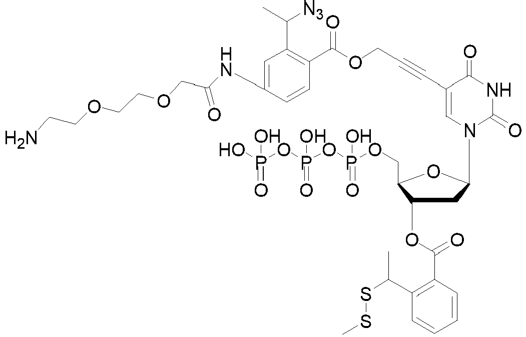 |
| 28B | 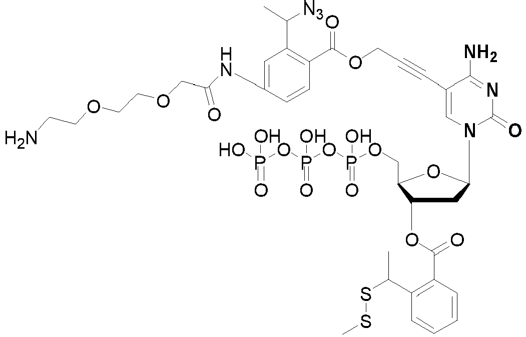 |
| 28C | 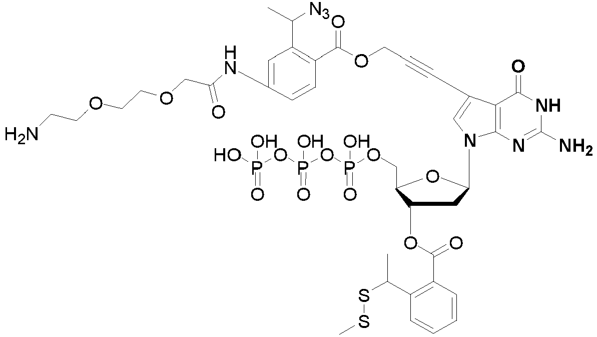 |
| 28D | 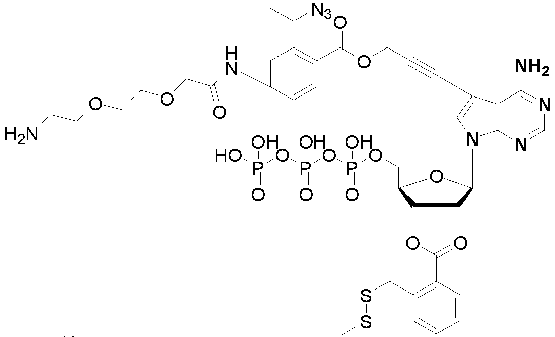 |
| 29A | 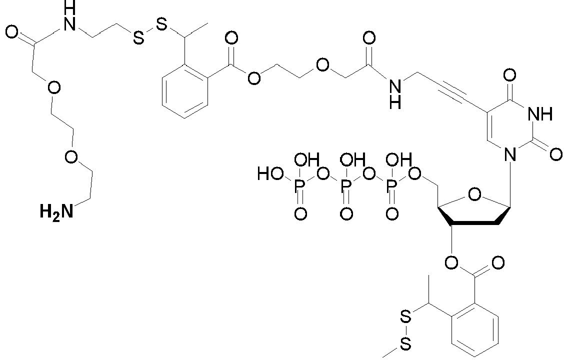 |
| 29B | 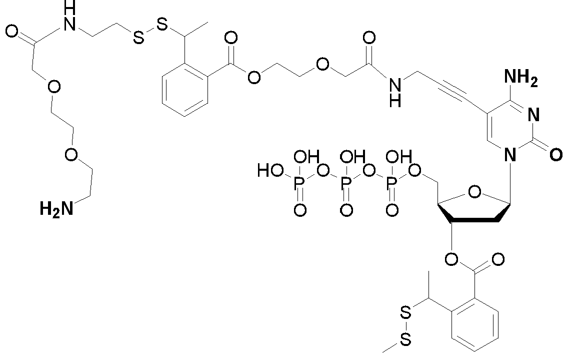 |
| 29C | 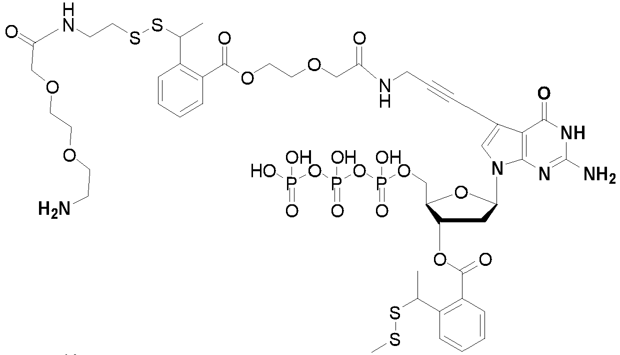 |
| 29D | 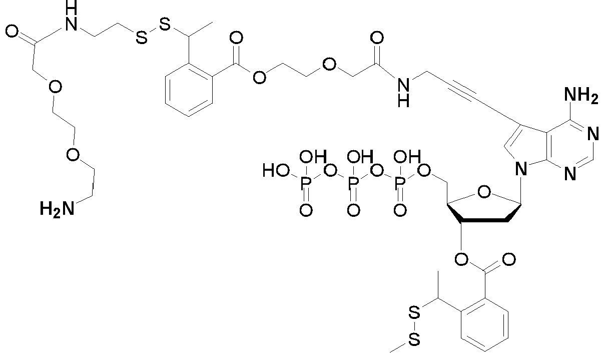 |
| 30A | 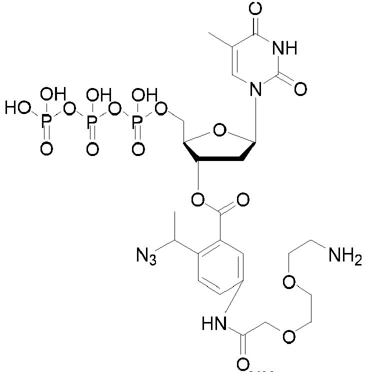 |
| 30B | 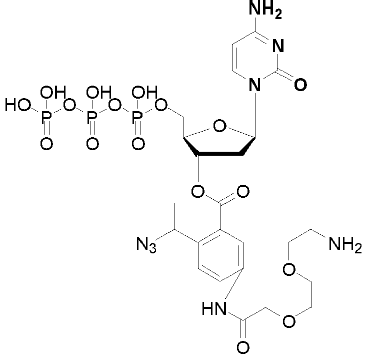 |
| 30C | 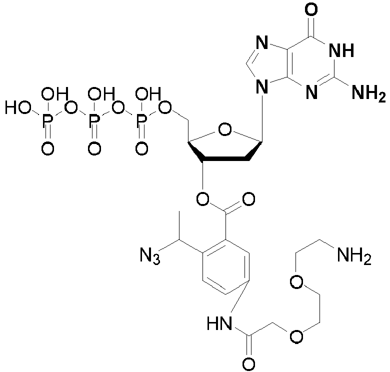 |
| 30D | 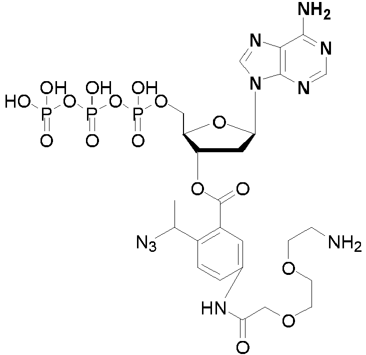 |
| 31A | 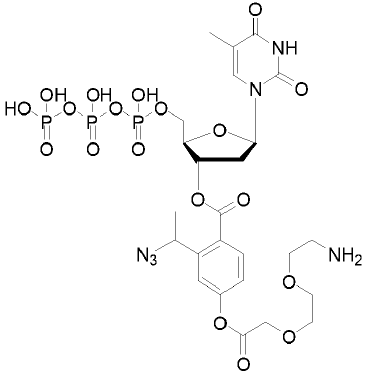 |
| 31B | 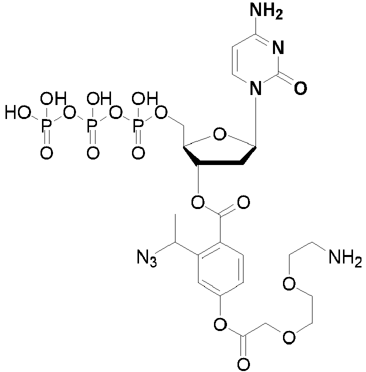 |
| 31C | 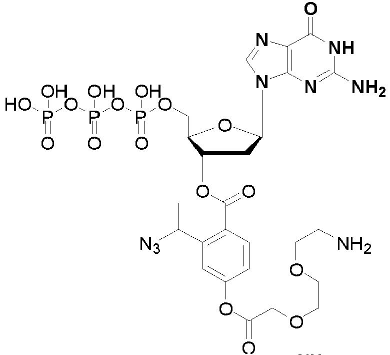 |
| 31D | 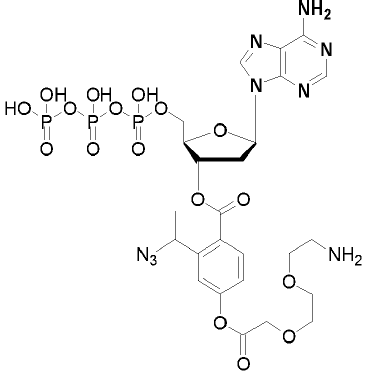 |
| 32A | 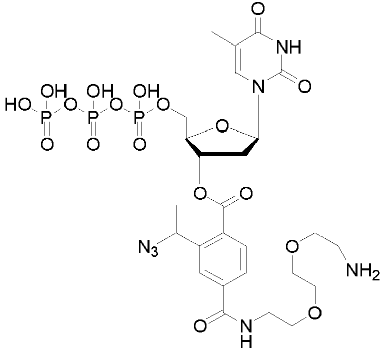 |
| 32 B | 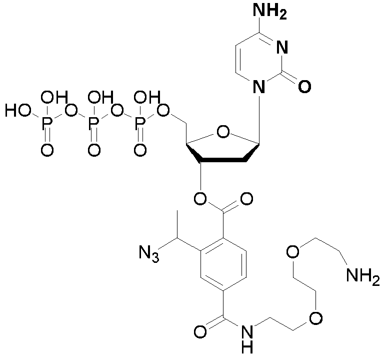 |
| 32C | 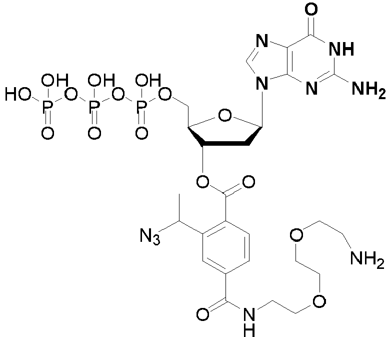 |
| 32 D | 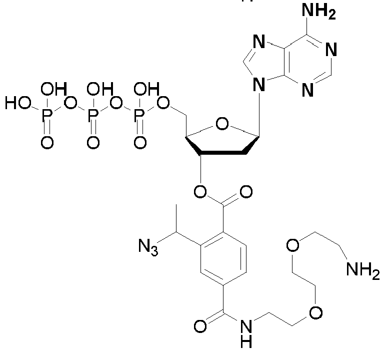 |
| 33A | 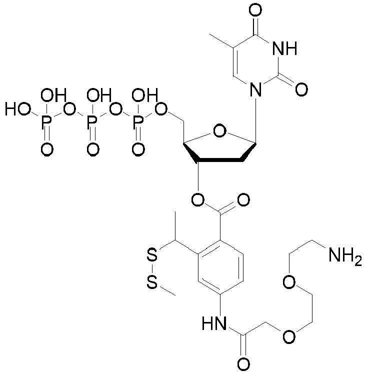 |
| 33B | 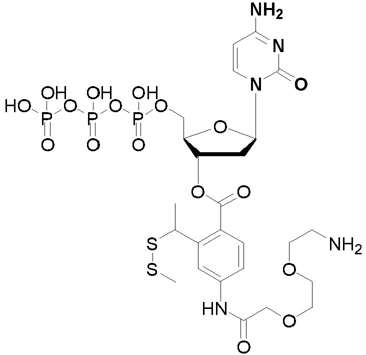 |
| 33C | 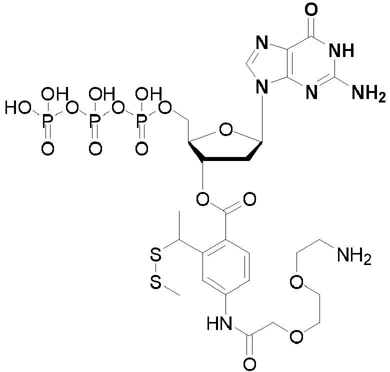 |
| 33D | 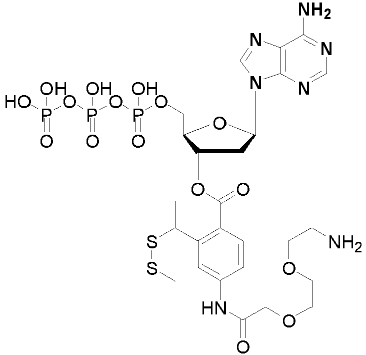 |
| 34A | 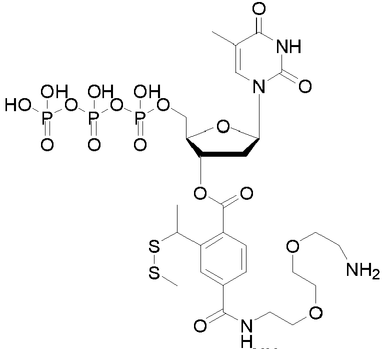 |
| 34B | 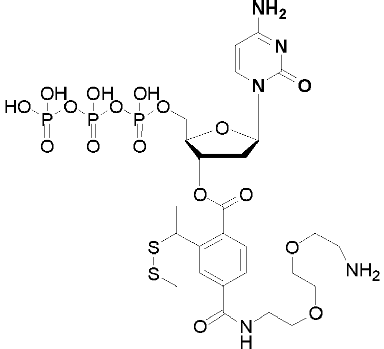 |
| 34C | 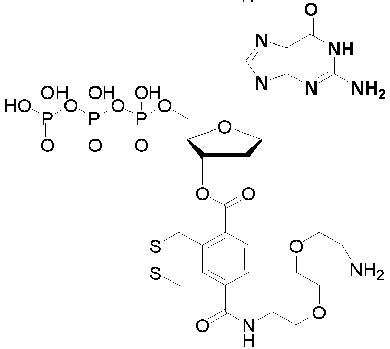 |
| 34D | 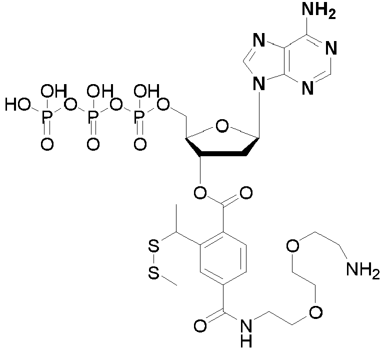 |
| 35A | 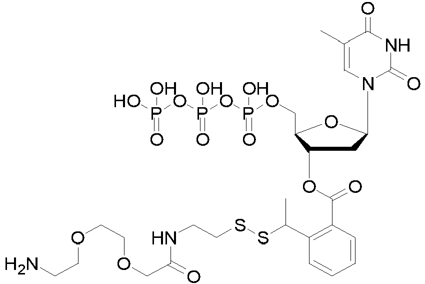 |
| 35B | 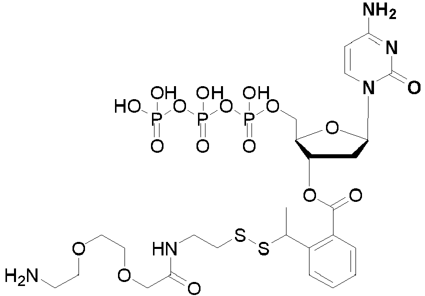 |
| 35C | 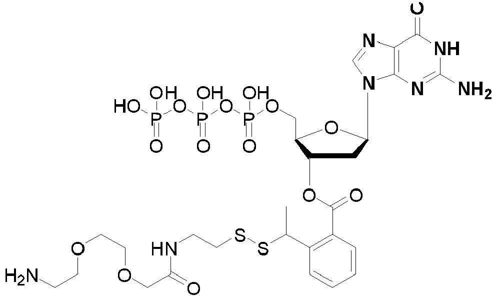 |
| 35D | 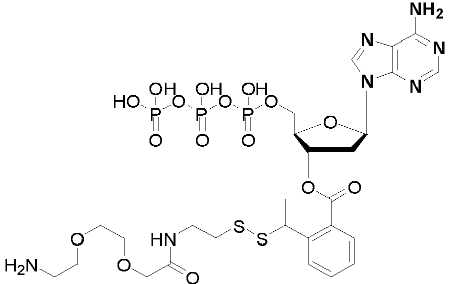 |
| 36A | 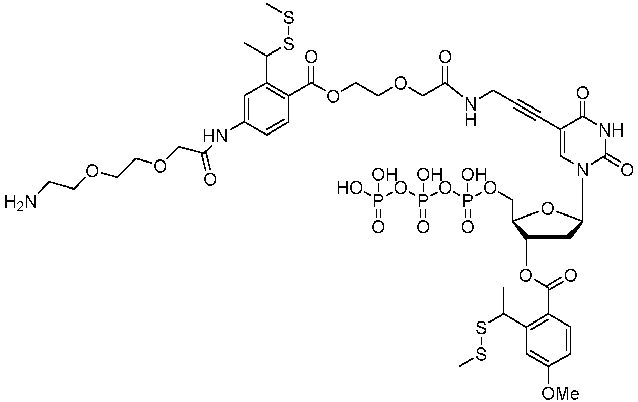 |
| 36B | 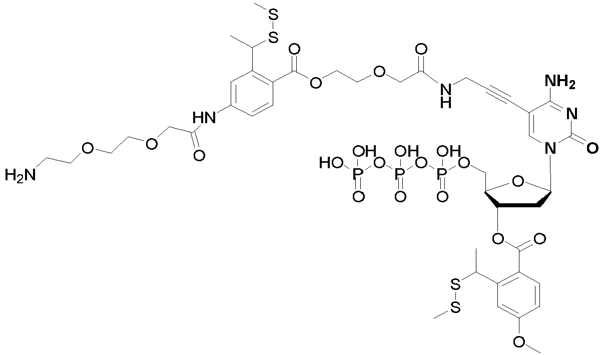 |
| 36C | 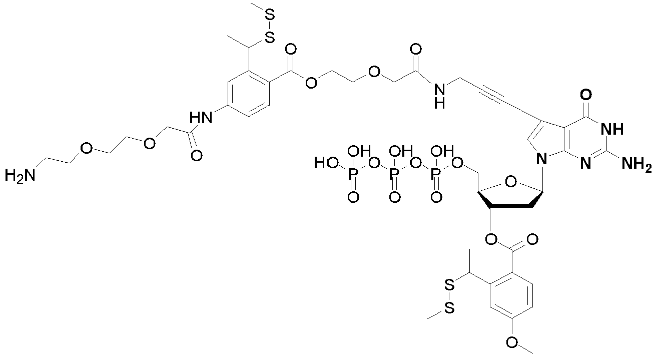 |
| 36D | 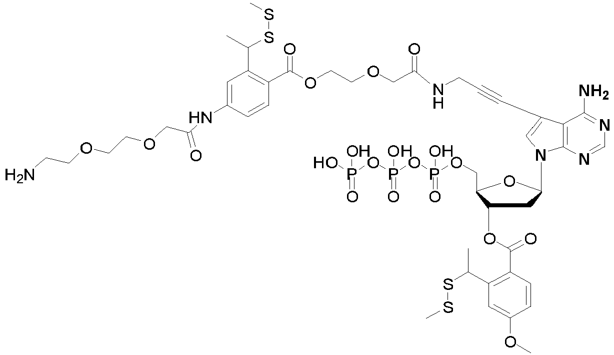 |
| 37A | 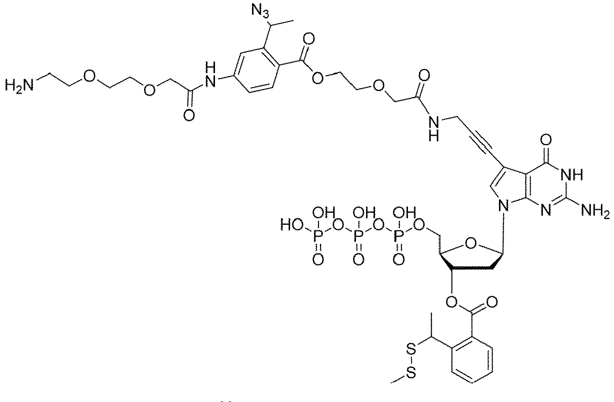 |
| 37B | 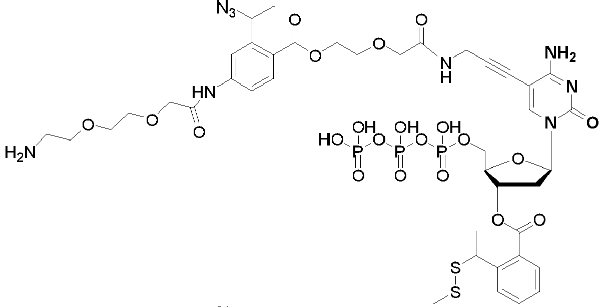 |
| 37C | 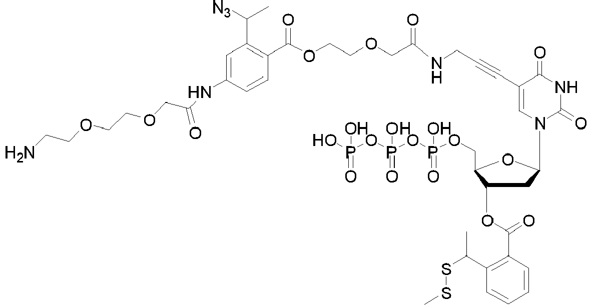 |
| 37D | 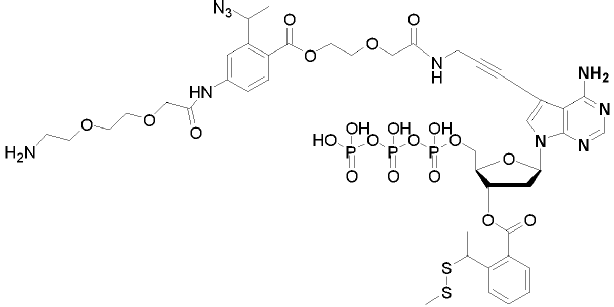 |
| 38A | 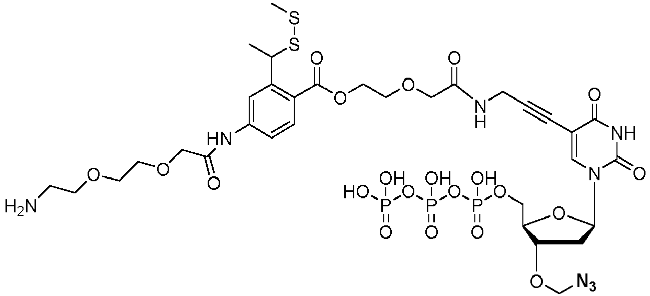 |
| 38B | 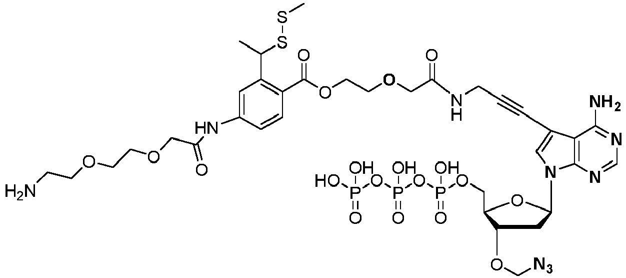 |
| 38C | 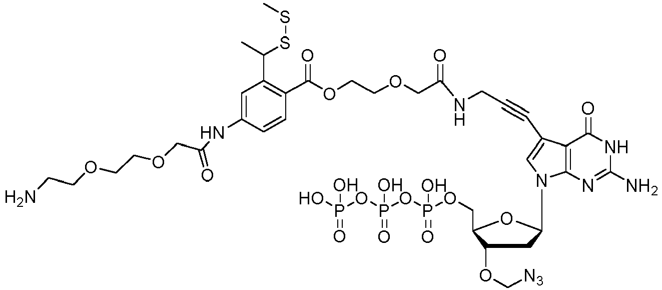 |
| 38D | 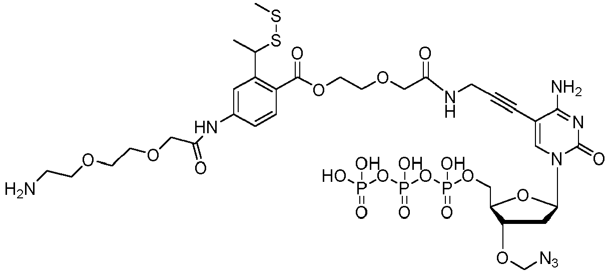 |
| 39A | 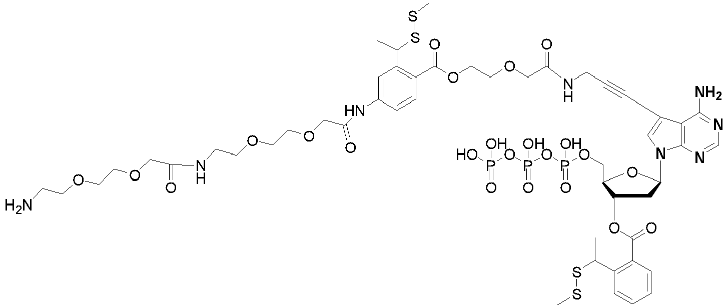 |
| 39B | 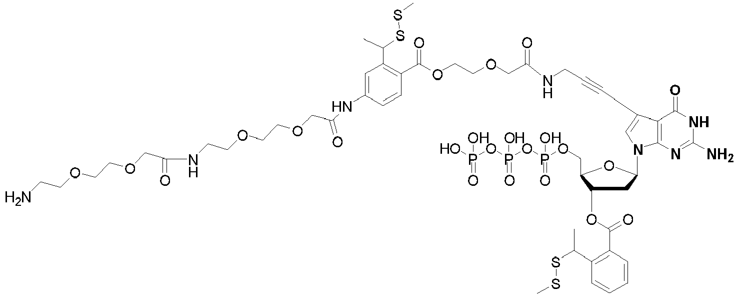 |
| 39C | 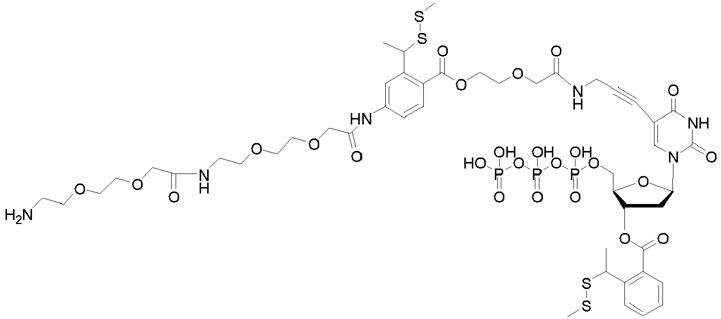 |
| 39D | 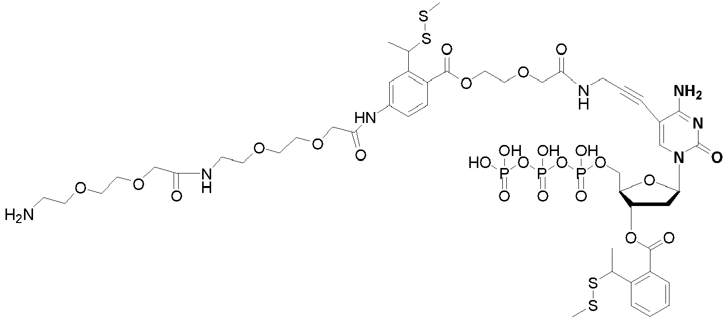 |
| 40A | 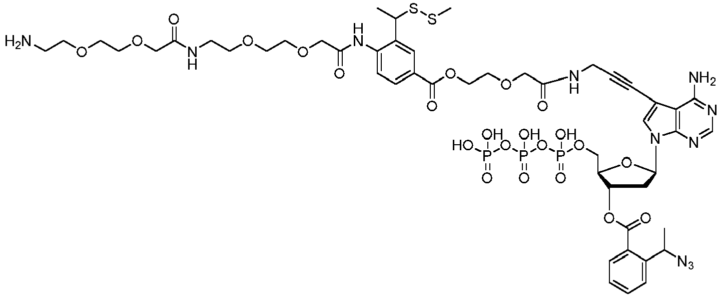 |
| 40B | 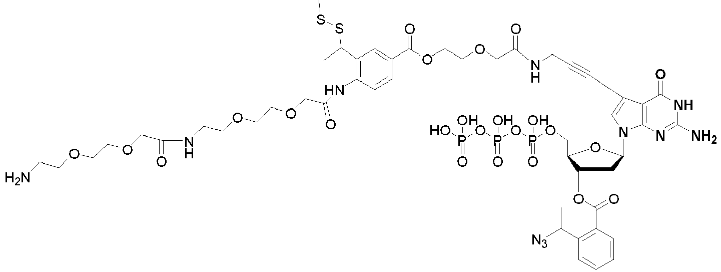 |
| 40C | 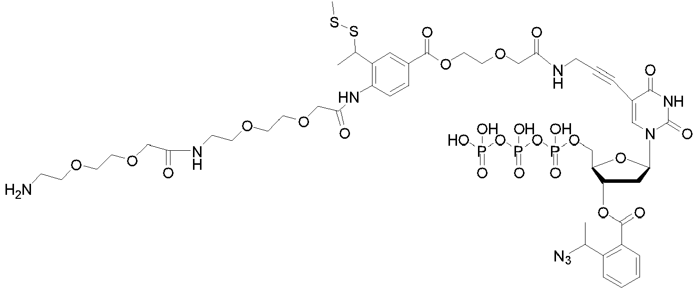 |
| 40D | 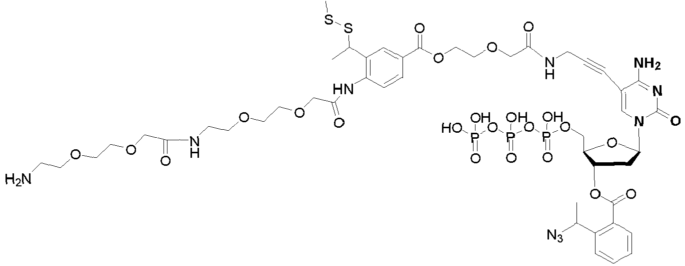 |
| 41A | 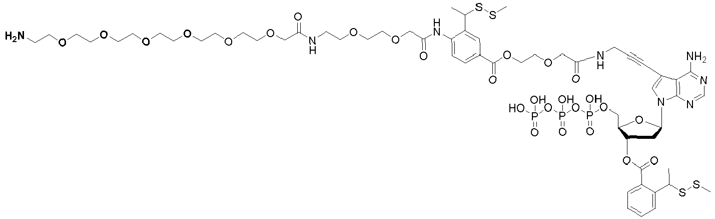 |
| 41B | 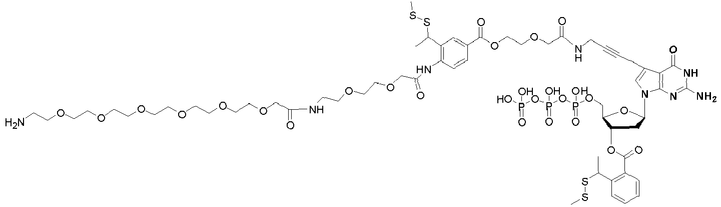 |
| 41C | 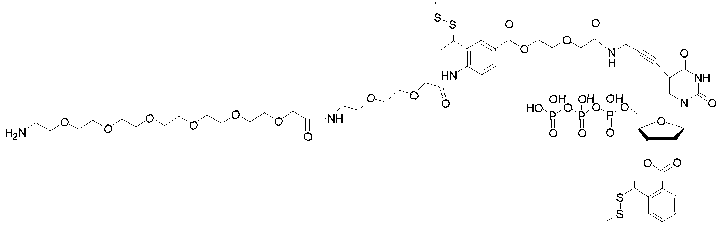 |
| 41D | 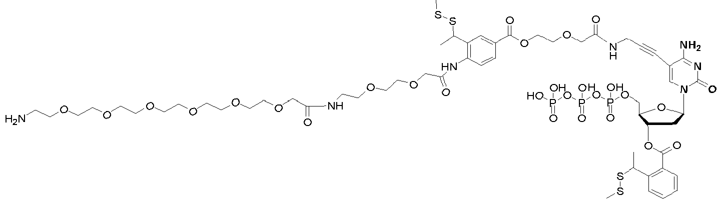 |
| 42A | 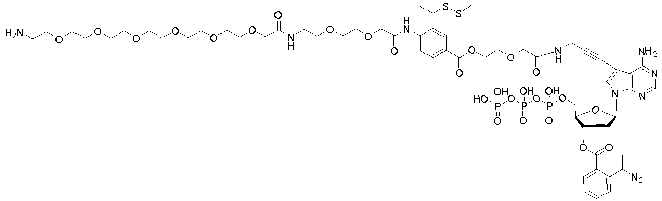 |
| 42 B | 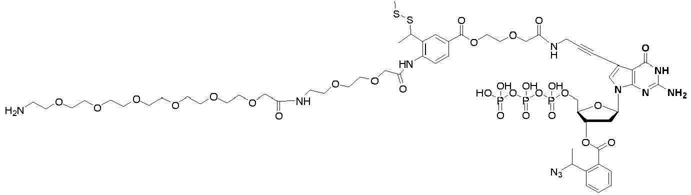 |
| 42C | 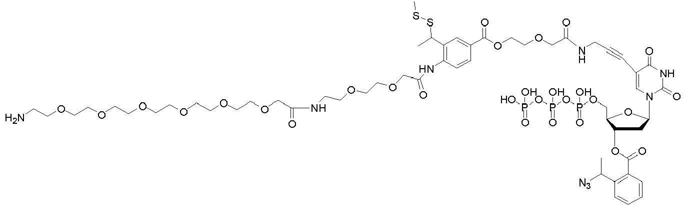 |
| 42 D | 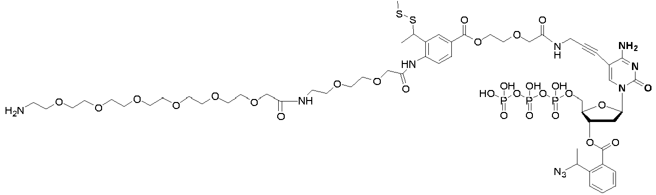 |
| 43A | 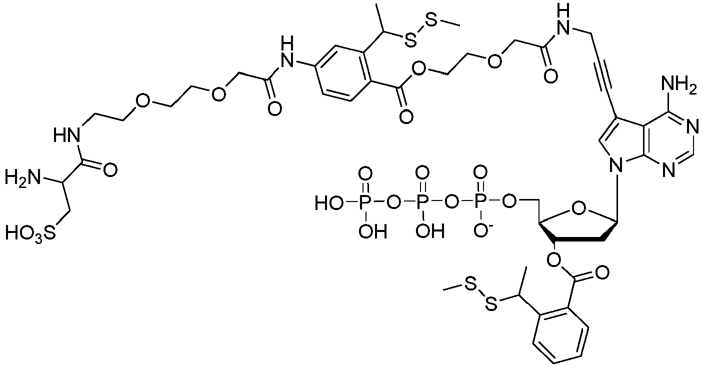 |
| 43B | 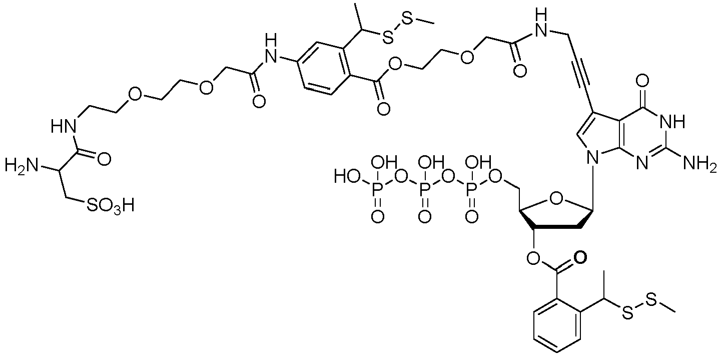 |
| 43C | 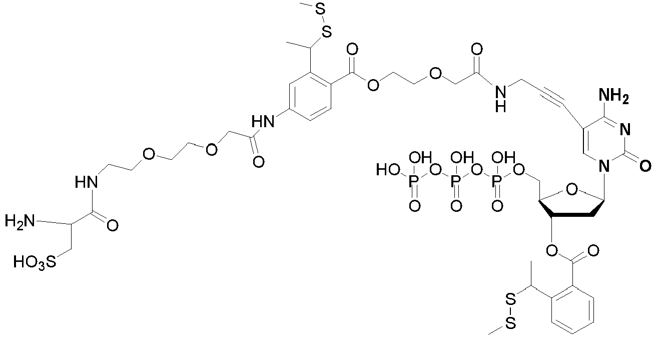 |
| 43D | 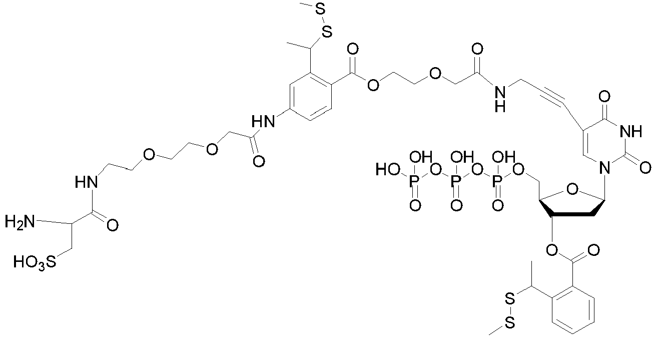 |
| 44A | 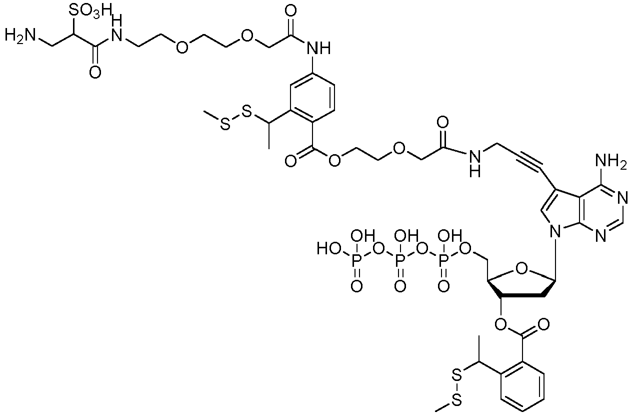 |
| 44B | 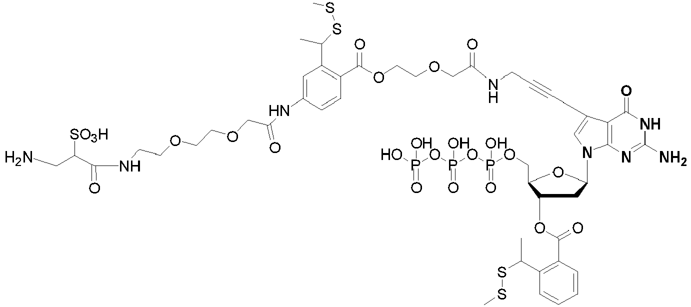 |
| 44C | 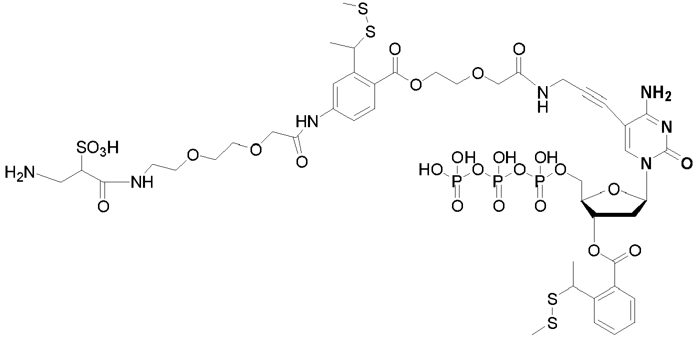 |
| 44D | 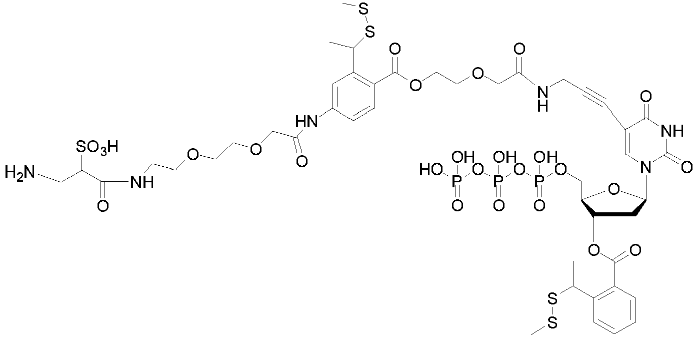 |
| 45A | 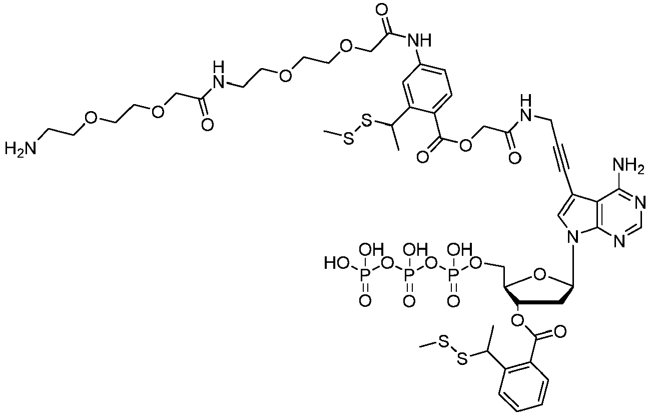 |
| 45B | 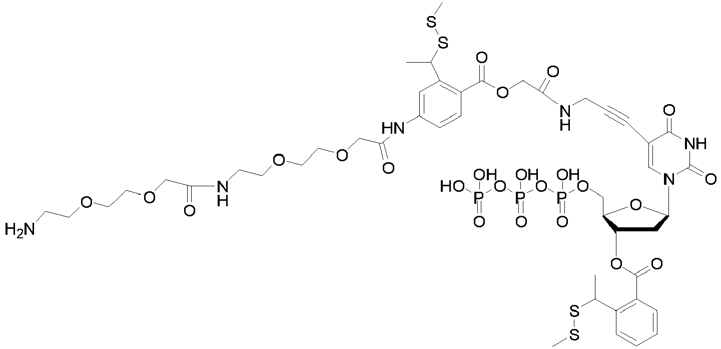 |
| 45C | 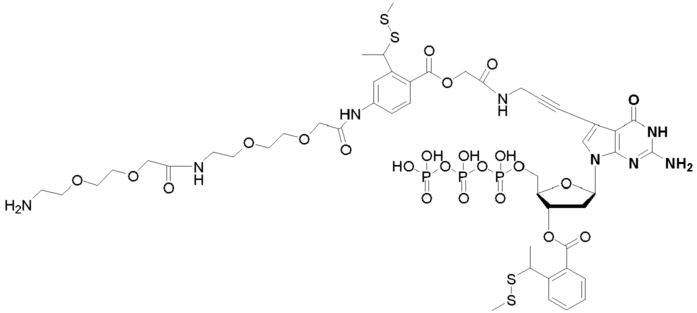 |
| 45D | 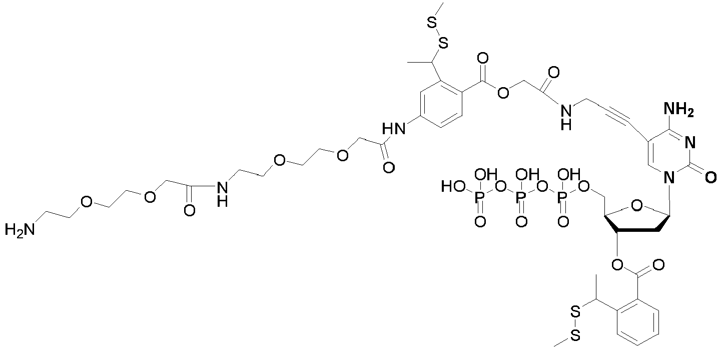 |
| 46A | 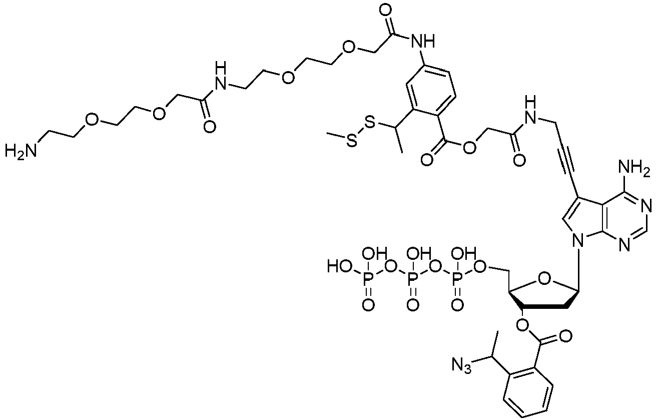 |
| 46B | 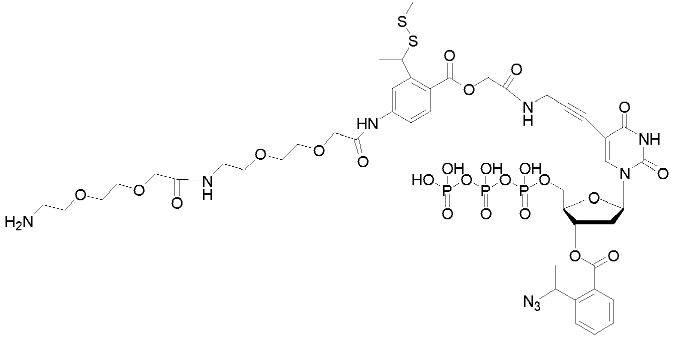 |
| 46C | 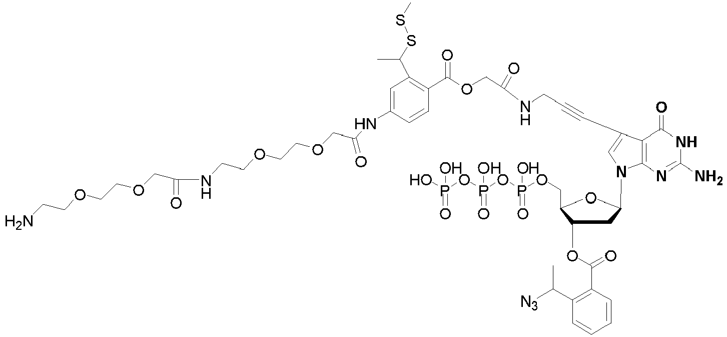 |
| 46D | 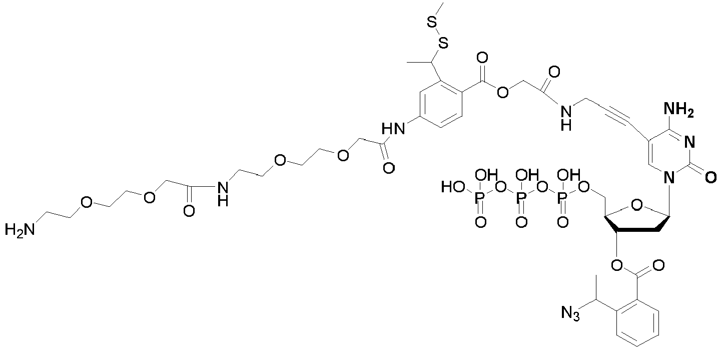 |
| 47A | 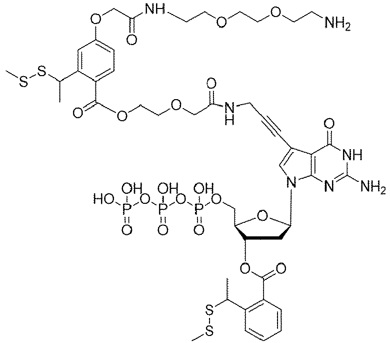 |
| 47B | 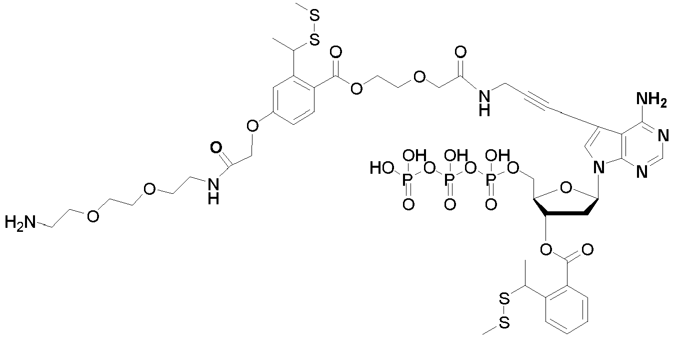 |
| 47C | 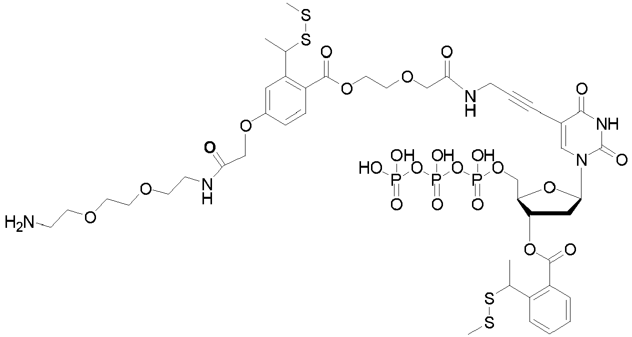 |
| 47D | 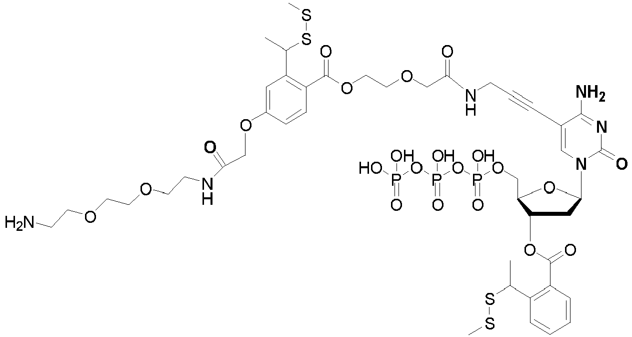 |
| 48A | 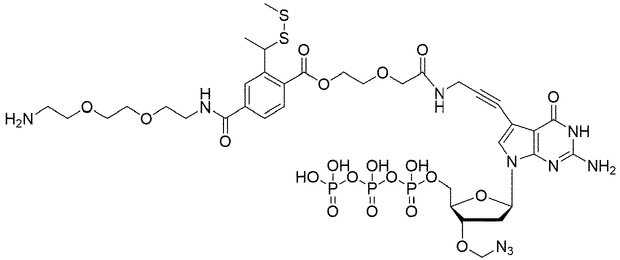 |
| 48B | 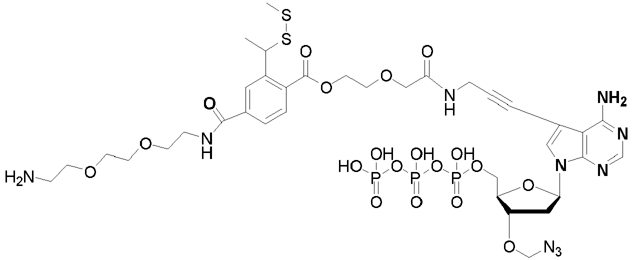 |
| 48C | 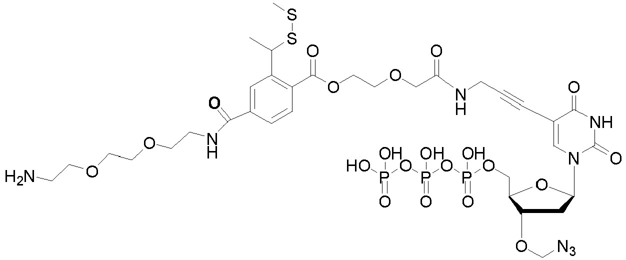 |
| 48D | 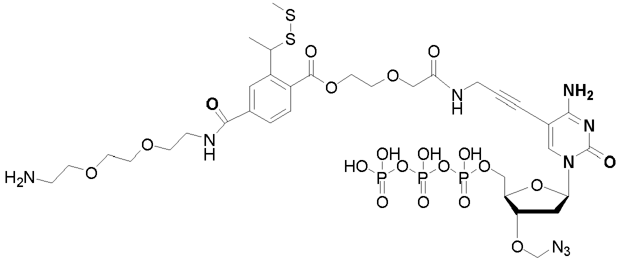 |

10. The compound or a salt thereof according to any one of claims 1 to 9, wherein the compound or a salt thereof carries an additional detectable label;
preferably, the additional detectable label carried by the compound or a salt thereof is introduced by affinity reagent (e.g., antibody, aptamer, Affimer, Knottin), wherein the affinity reagent carries the detectable label, and the affinity reagent can specifically recognize and bind to an epitope of the compound or a salt thereof;
or preferably, the detectable label is linked to the R in the compound of formula I, formula II, formula 1-1, formula I-2, formula II-1 or formula II-2 or to a structural moiety corresponding to the R in the compound according to claim 9;
preferably, the detectable label is linked to the terminal amino group in 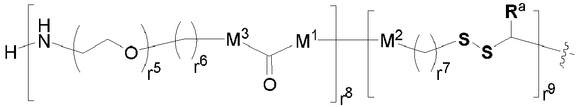 or a structural moiety correspondent thereto;
preferably, the carboxyl in the detectable label is linked to the terminal amino group in 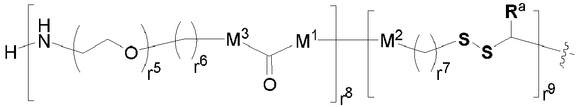 or a structural moiety correspondent thereto via formation of an amide bond;
or preferably, the detectable label is linked to the R in the compound of formula III or to a structural moiety corresponding to the R in the compound according to claim 9;
or preferably, the detectable label is linked to the terminal amino group in 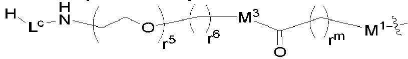 or a structural moiety correspondent thereto;
or preferably, the detectable label is linked to the terminal amino group in 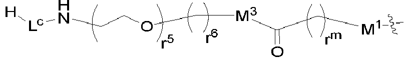 or a structural moiety correspondent thereto via formation of an amide bond;
preferably, base¹ is different, and the additional detectable label carried by the compound of formula I varies;
preferably, base² is different, and the additional detectable label carried by the compound of formula II varies;
preferably, the detectable label is a fluorescent label;
preferably, the detectable label is selected from: iF700,

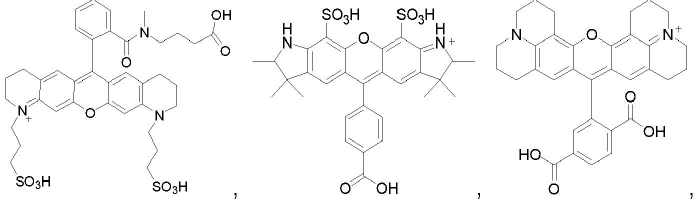

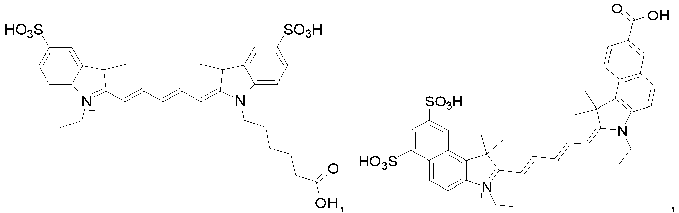

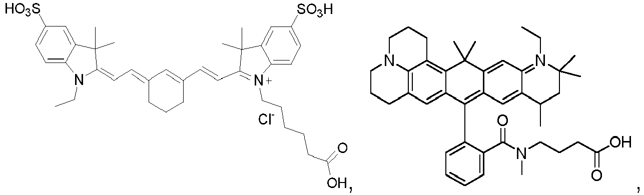

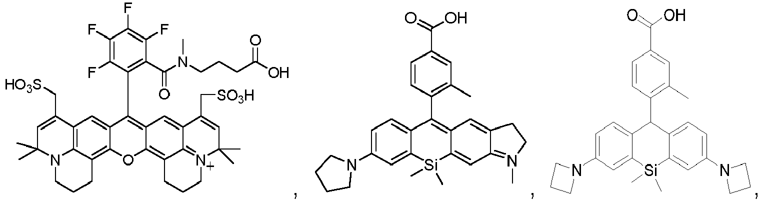

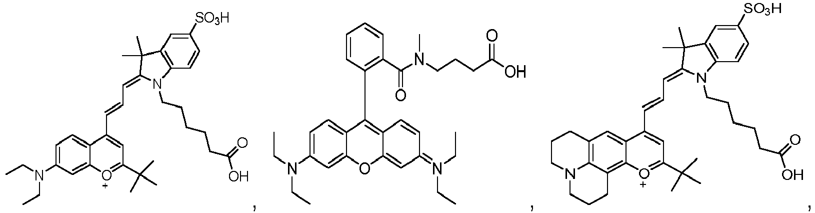

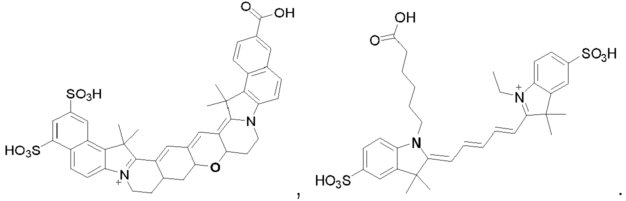

11. The compound or a salt thereof according to claim 10, wherein the compound is selected from:
| Serial Number | Structural formulae |
|---|---|
| 1A' | 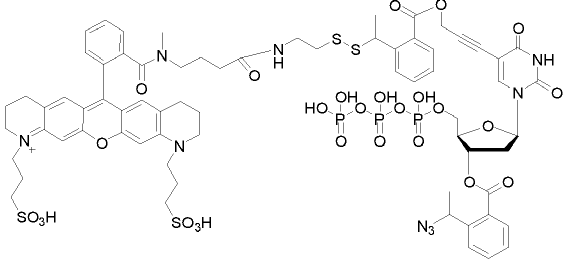 |
| 1B' | 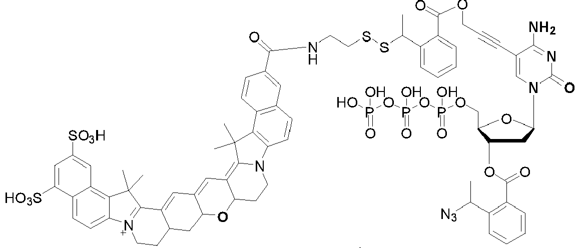 |
| 1C' | 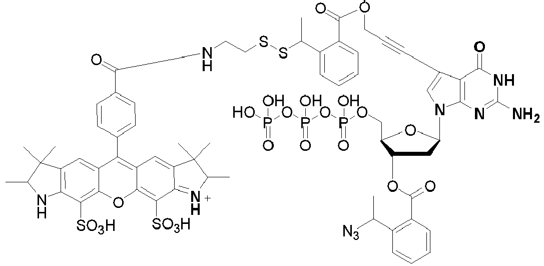 |
| 1D' | 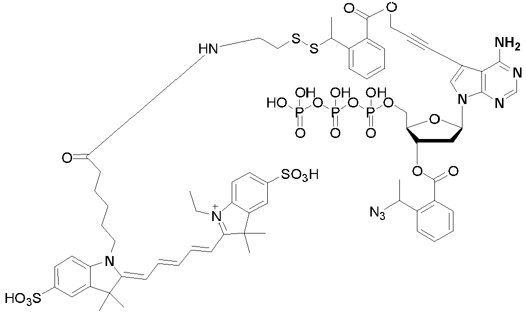 |
| 2A' | 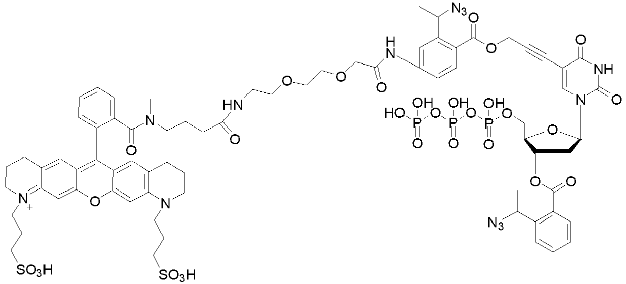 |
| 2B' | 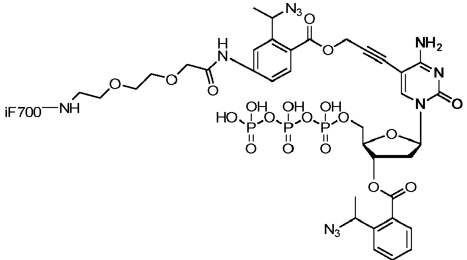 |
| 2C' | 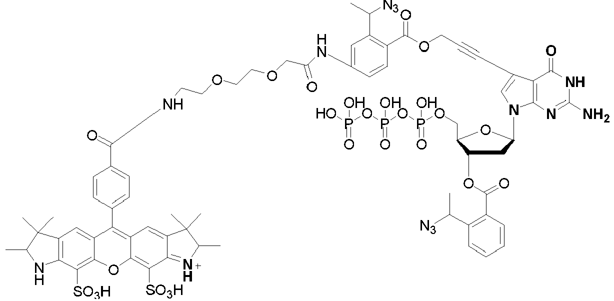 |
| 2D' | 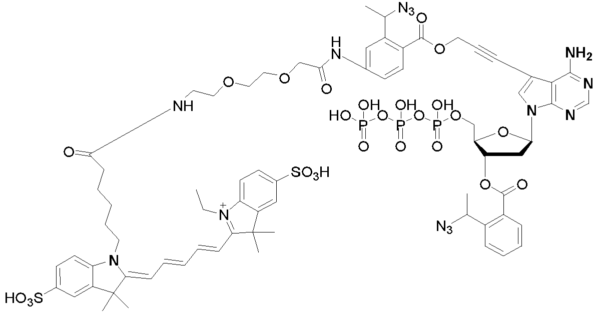 |
| 3A' | 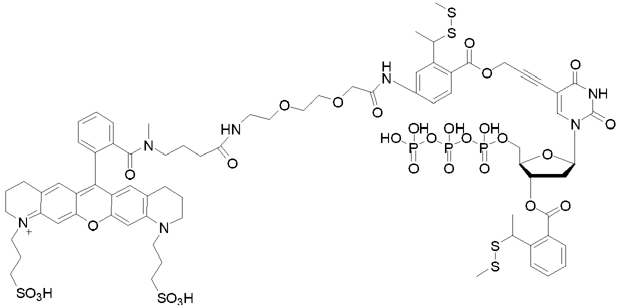 |
| 3 B' | 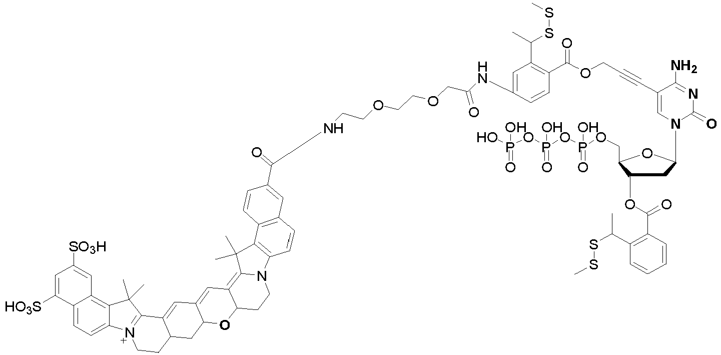 |
| 3C' | 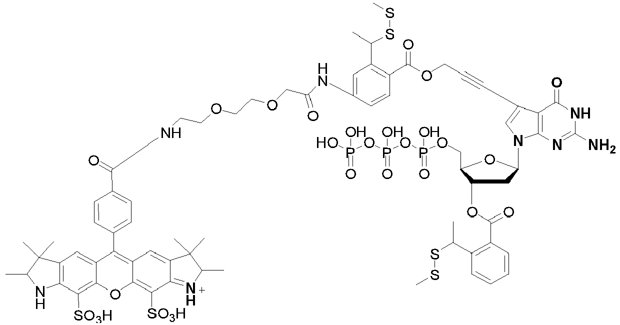 |
| 3D' | 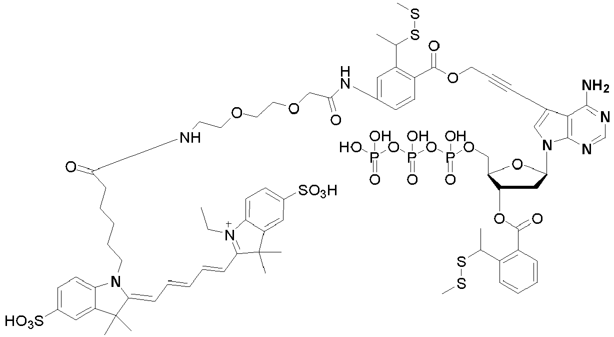 |
| 4A' | 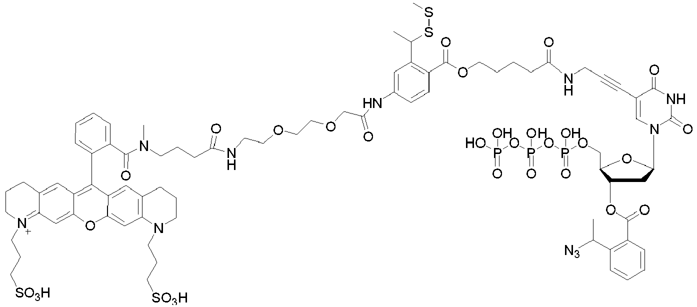 |
| 4B' | 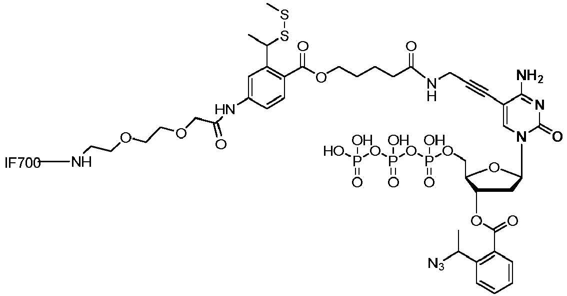 |
| 4C' | 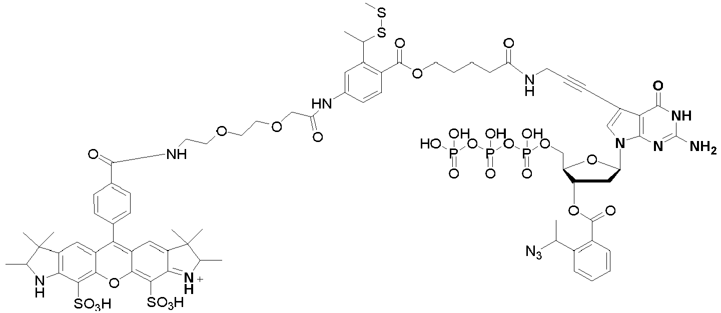 |
| 4D' | 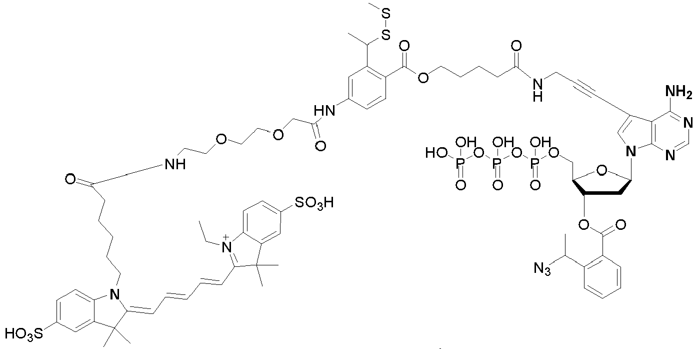 |
| 5A' | 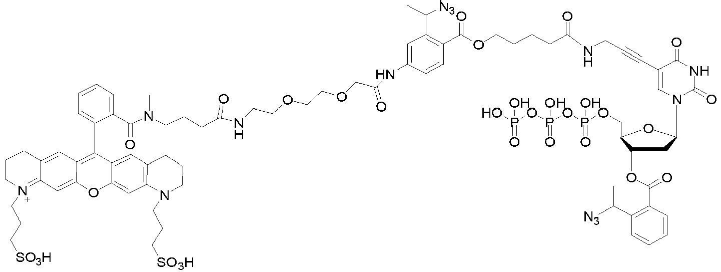 |
| 5B' | 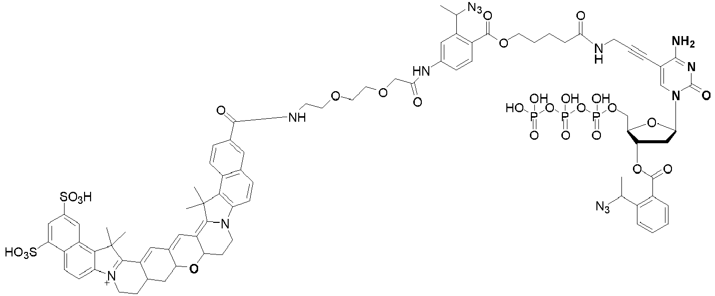 |
| 5C' | 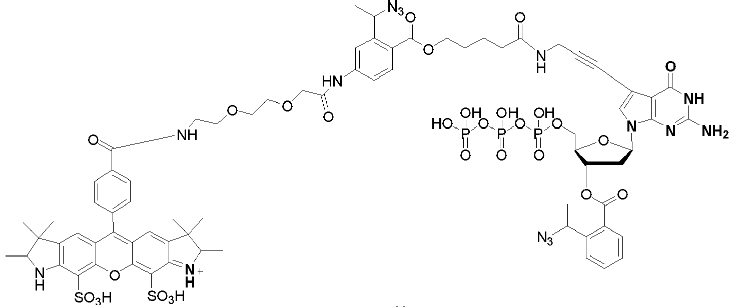 |
| 5D' | 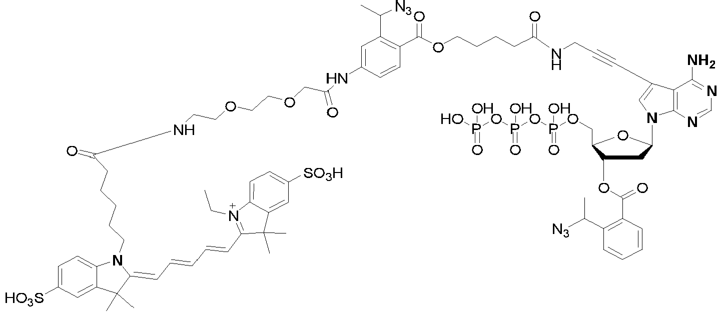 |
| 6A' | 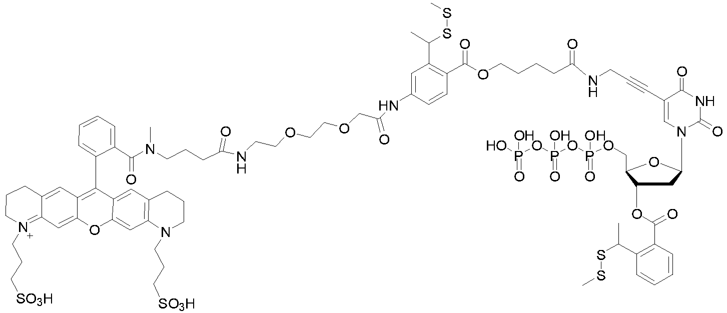 |
| 6B' | 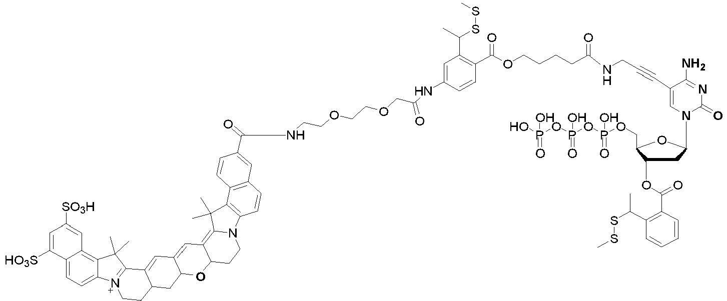 |
| 6C' | 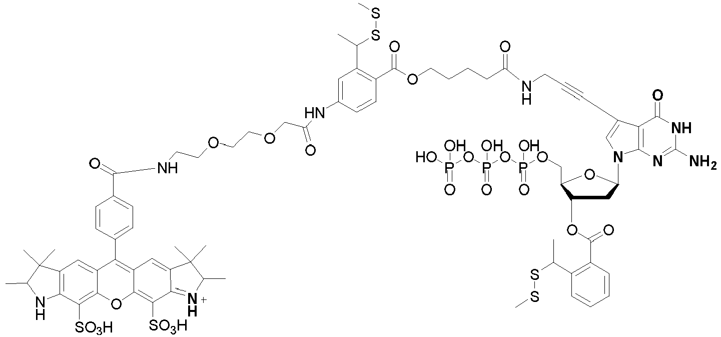 |
| 6D' | 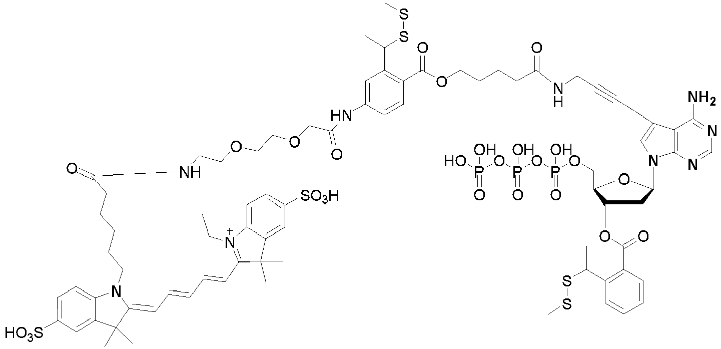 |
| 7A' | 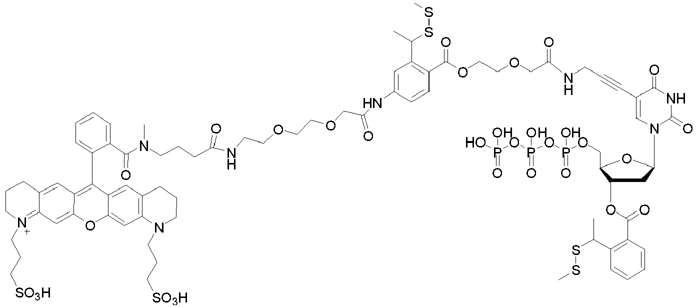 |
| 7B' | 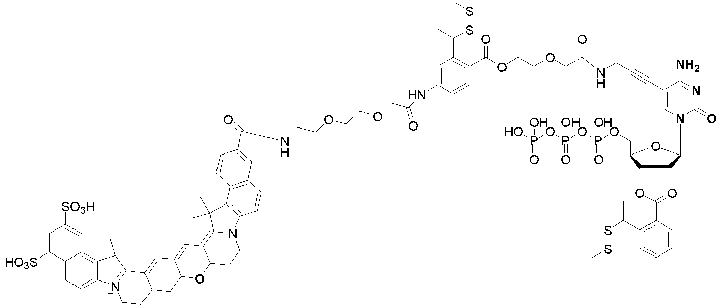 |
| 7B" | 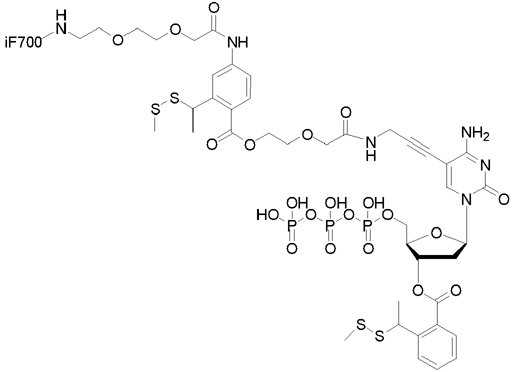 |
| 7C' | 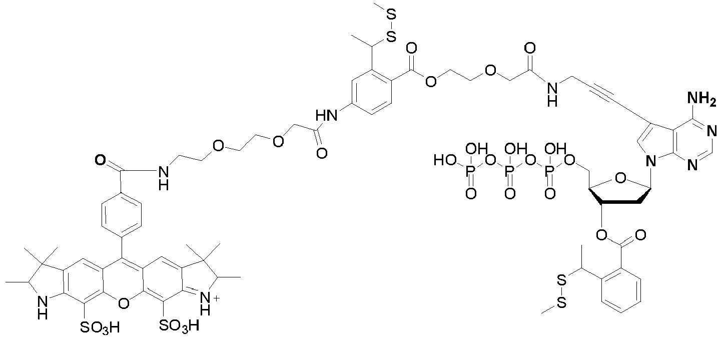 |
| 7C" | 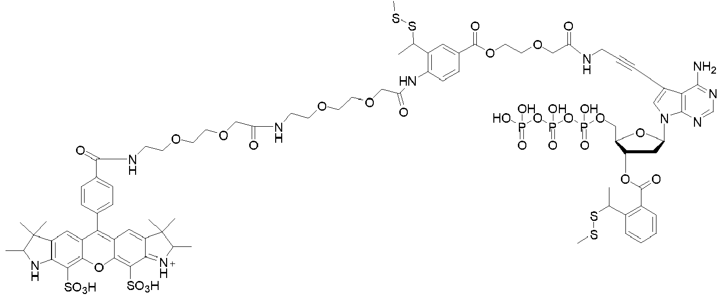 |
| 7C‴ | 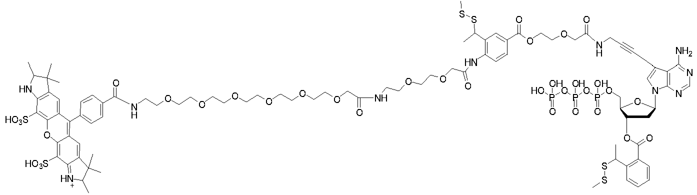 |
| 7C"" | 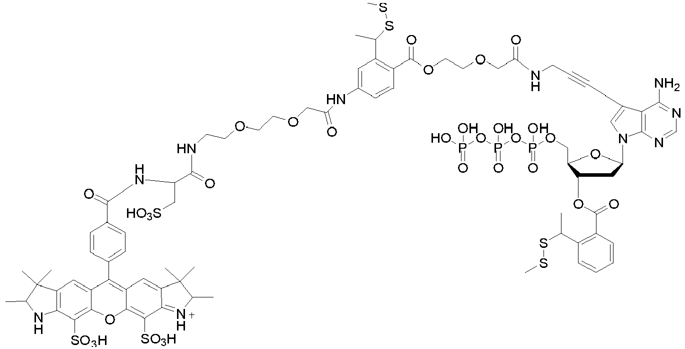 |
| 7Cʺ‴ | 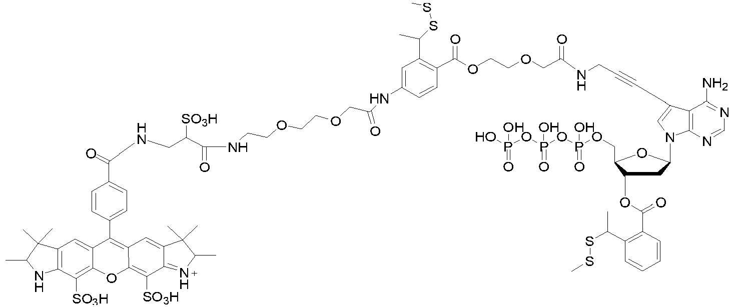 |
| 7C‴‴ | 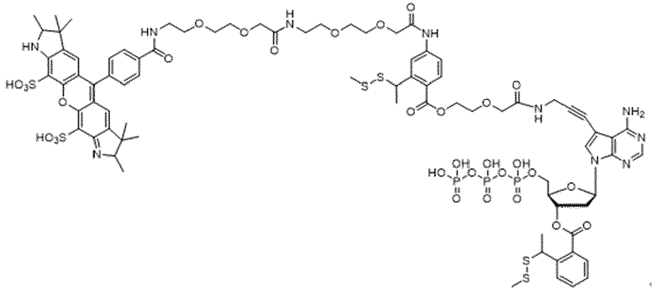 |
| 7D' | 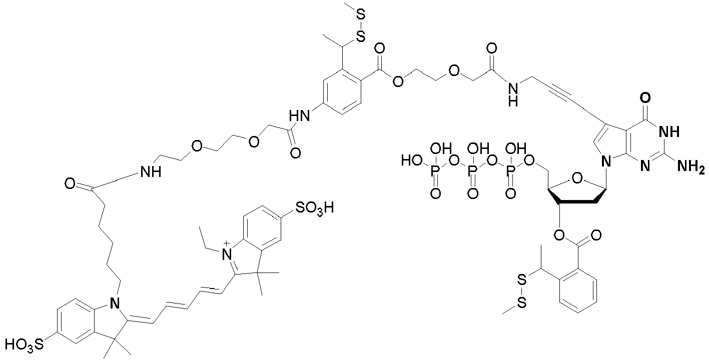 |
| 7D" | 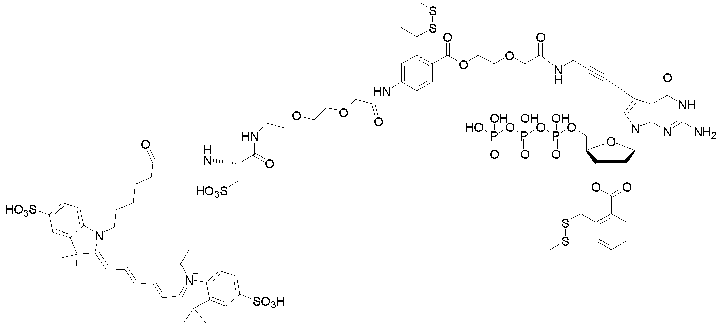 |
| 8-1A' | 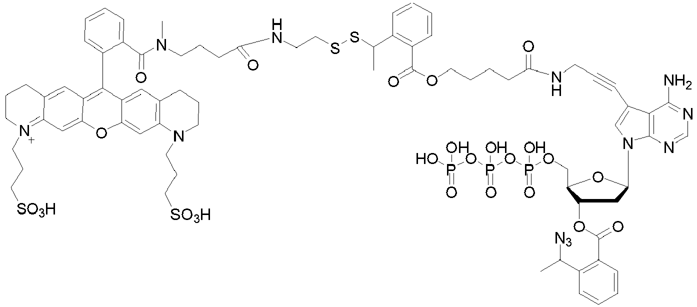 |
| 8-1B' | 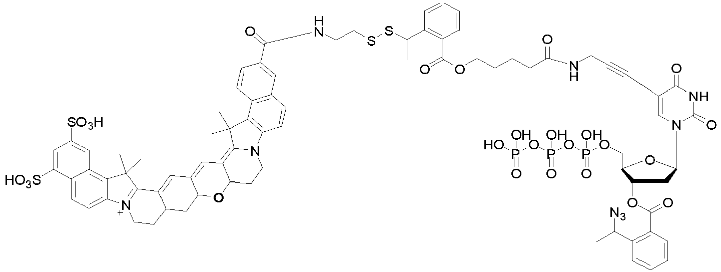 |
| 8-1C' | 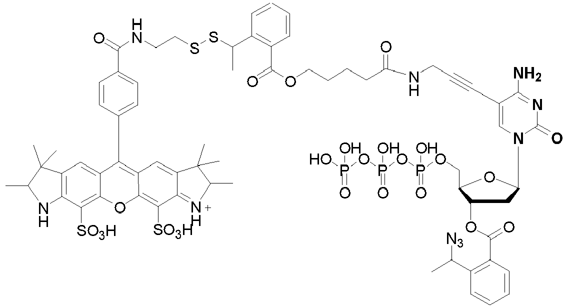 |
| 8-1D' | 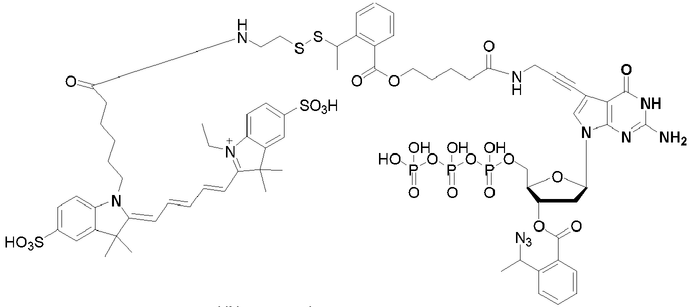 |
| 8-2A' | 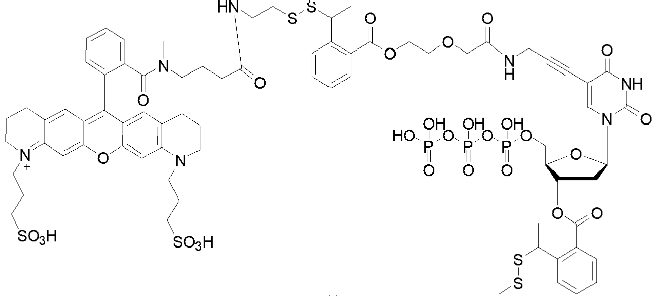 |
| 8-2B' | 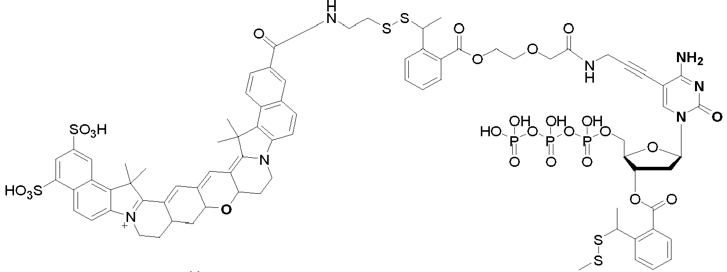 |
| 8-2C' | 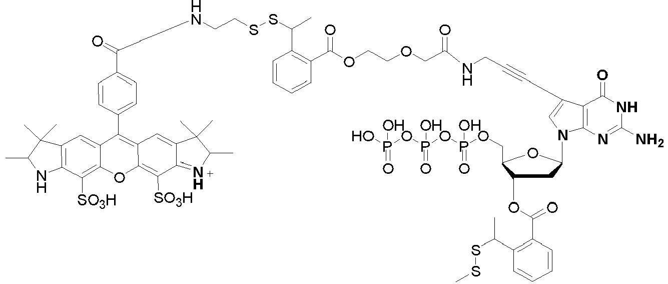 |
| 8-2D' | 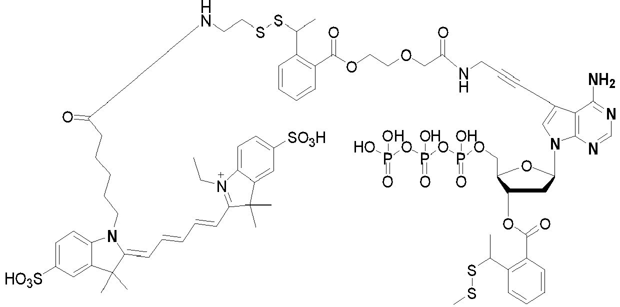 |
| 9A' | 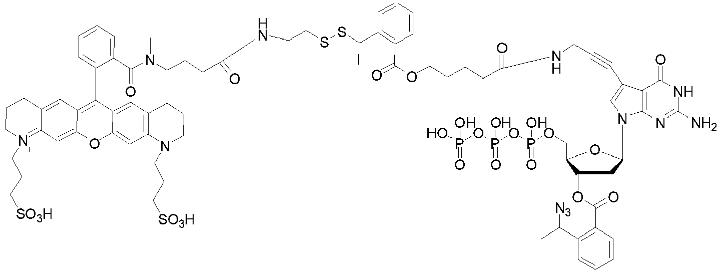 |
| 9B' | 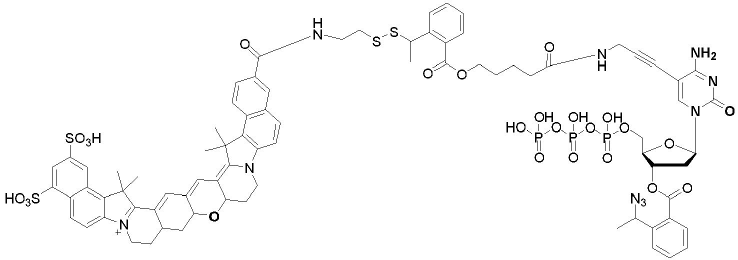 |
| 9C' | 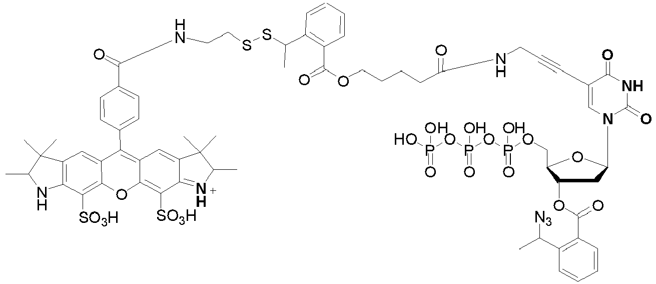 |
| 9D' | 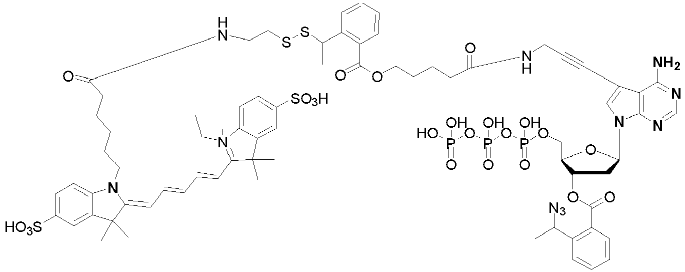 |
| 10A' | 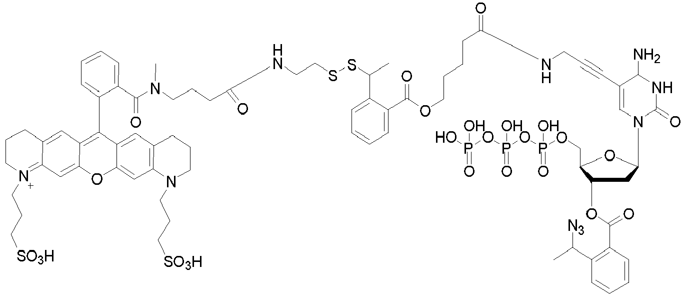 |
| 10B' | 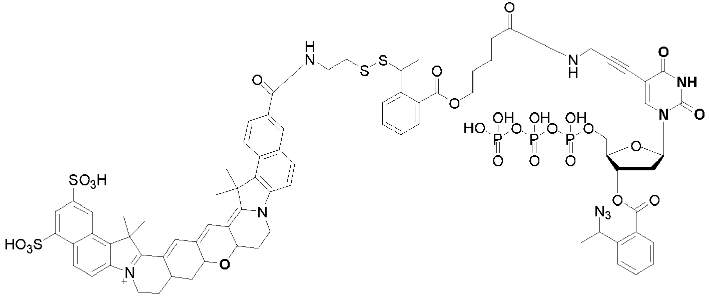 |
| 10C' | 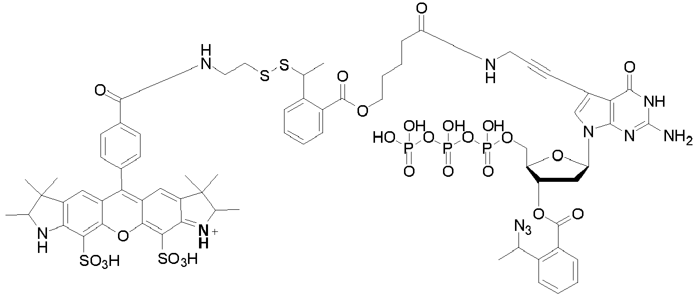 |
| 10D' | 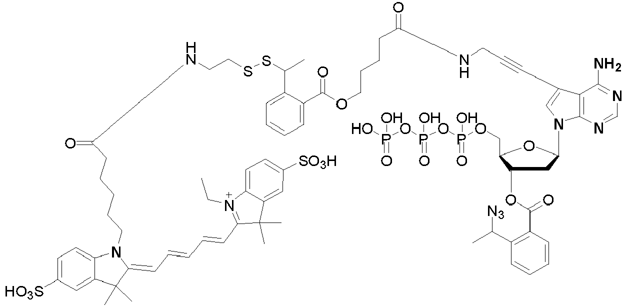 |
| 11A' | 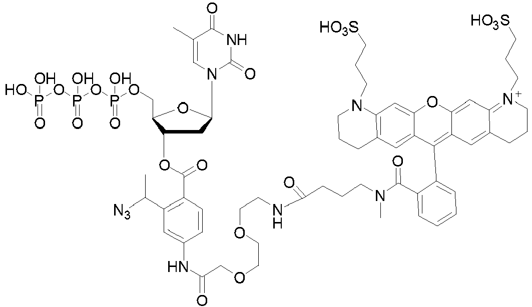 |
| 11B' | 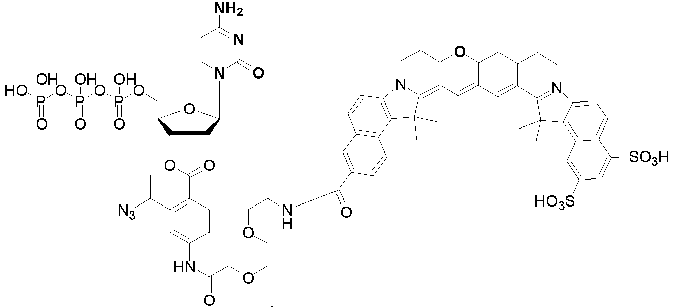 |
| 11C' | 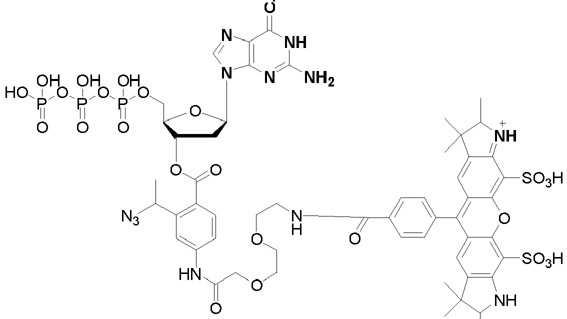 |
| 11D' | 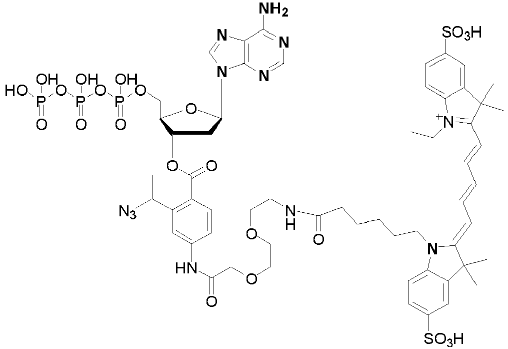 |
| 12A' | 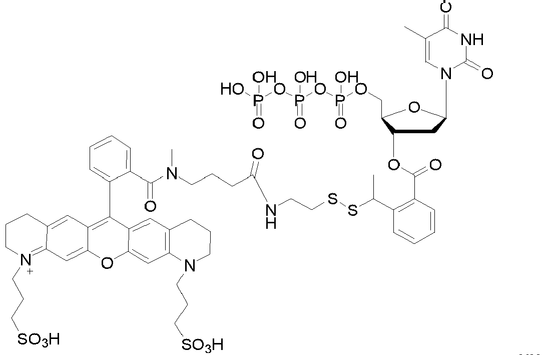 |
| 12B' | 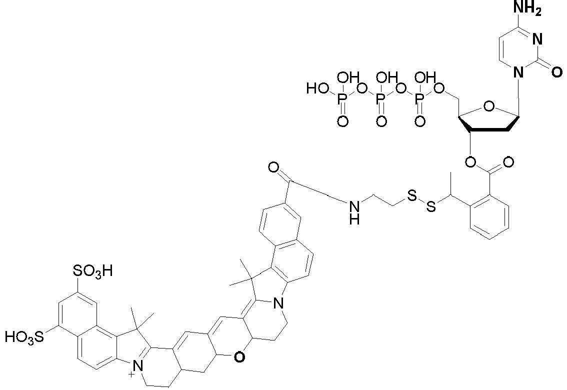 |
| 12C' | 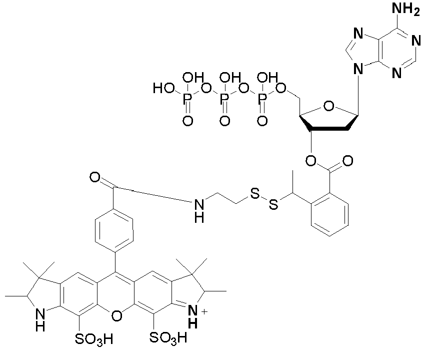 |
| 12D' | 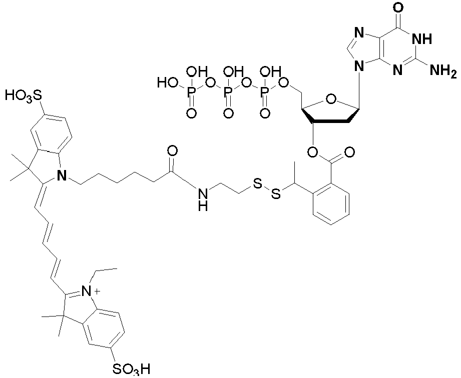 |
| 13A' | 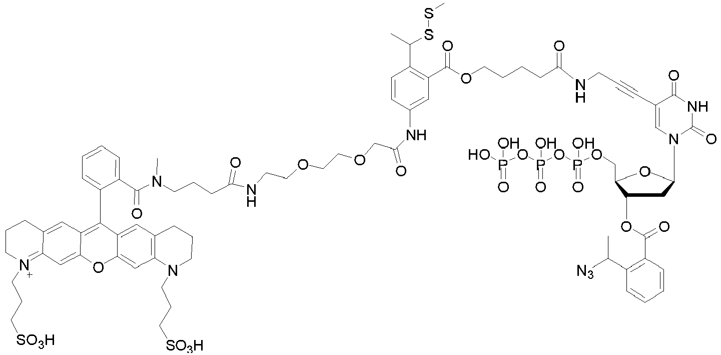 |
| 13B' | 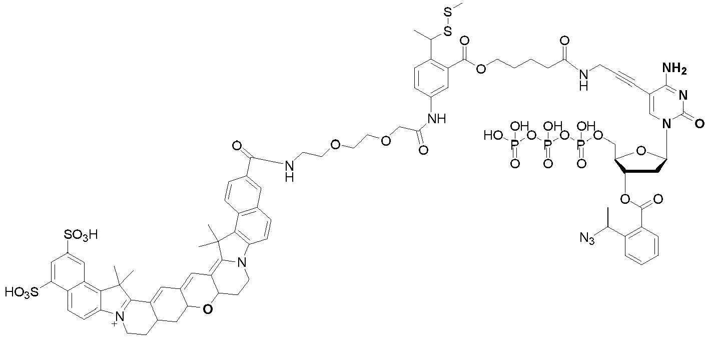 |
| 13C' | 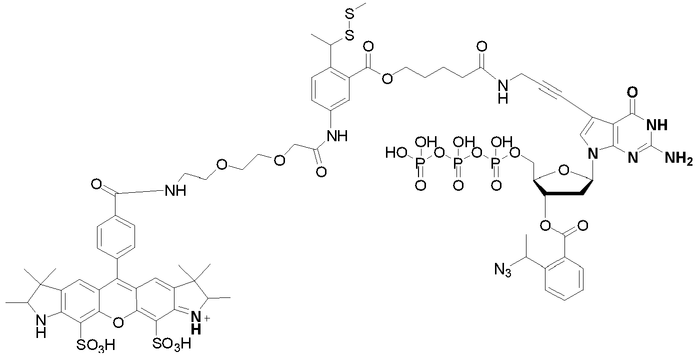 |
| 13D' | 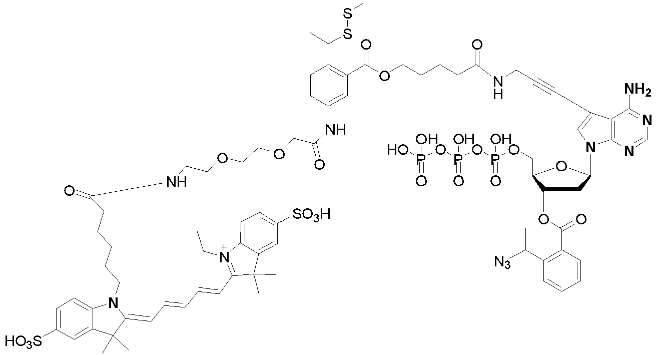 |
| 14A' | 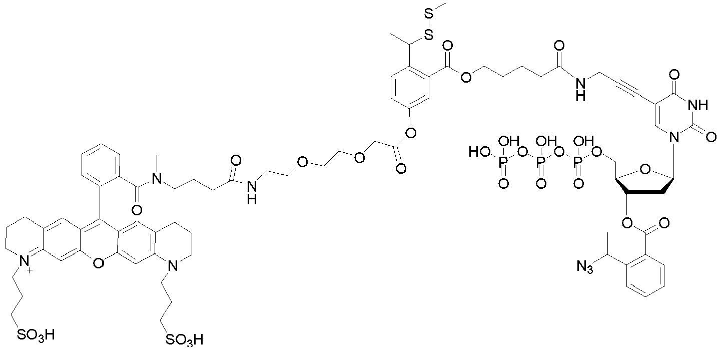 |
| 14B' | 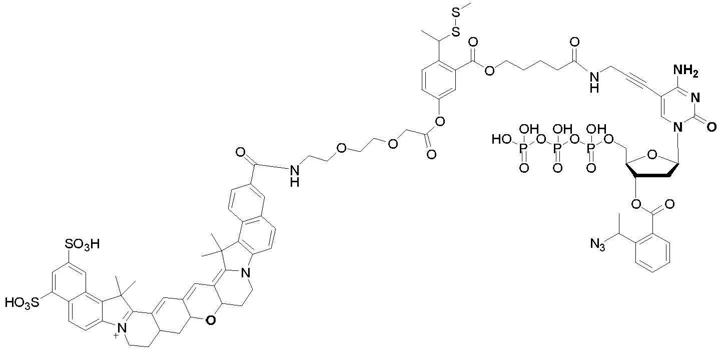 |
| 14C' | 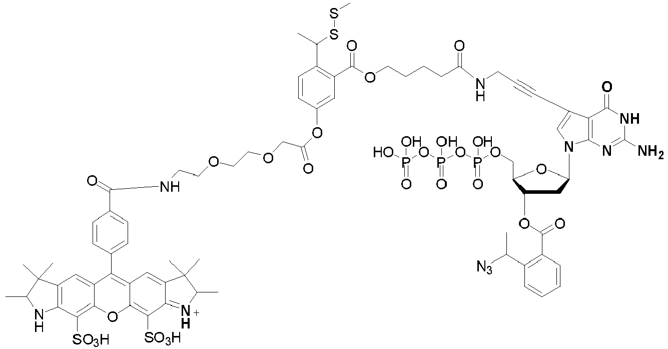 |
| 14D' | 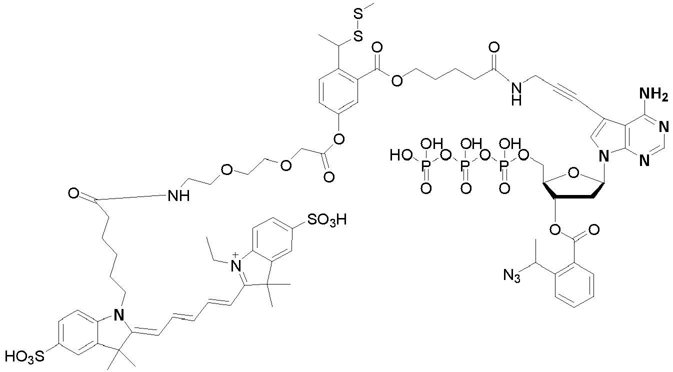 |
| 15A' | 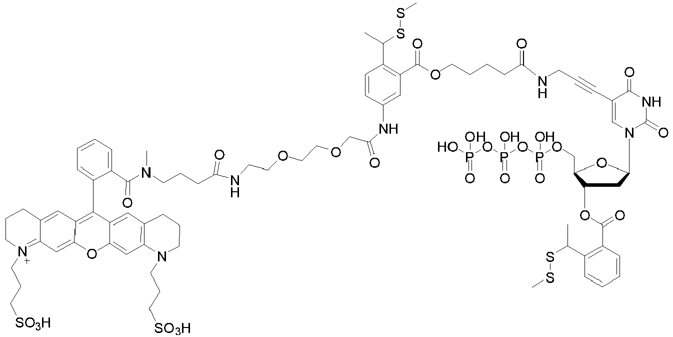 |
| 15B' | 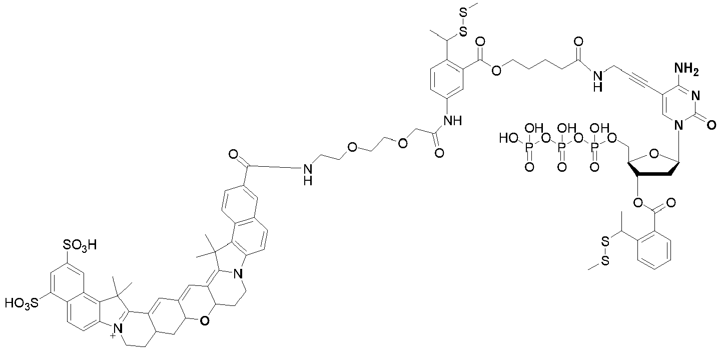 |
| 15C' | 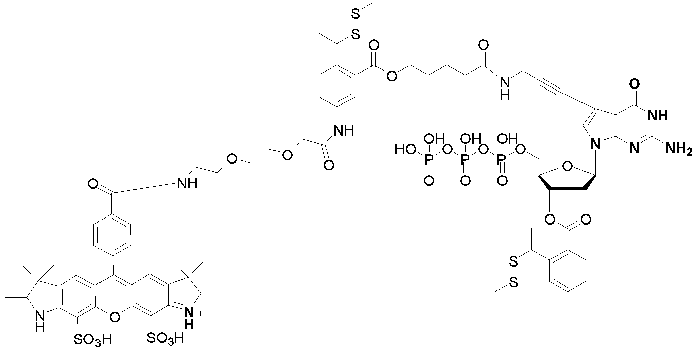 |
| 15D' | 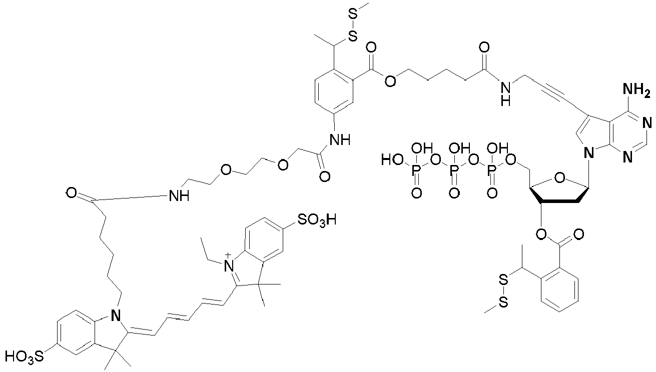 |
| 16A' | 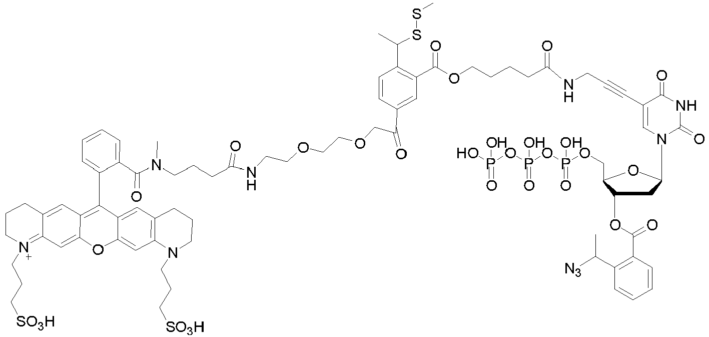 |
| 16B' | 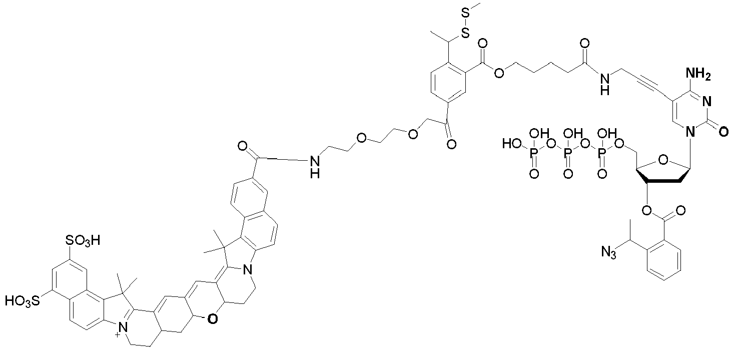 |
| 16C' | 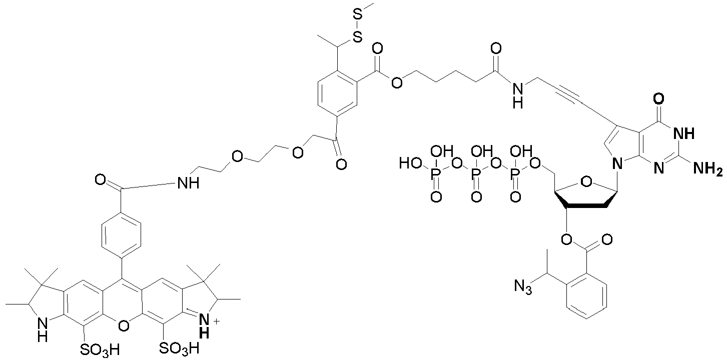 |
| 16D' | 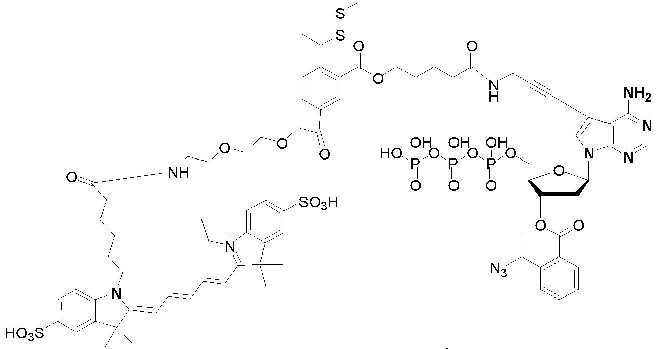 |
| 17A' | 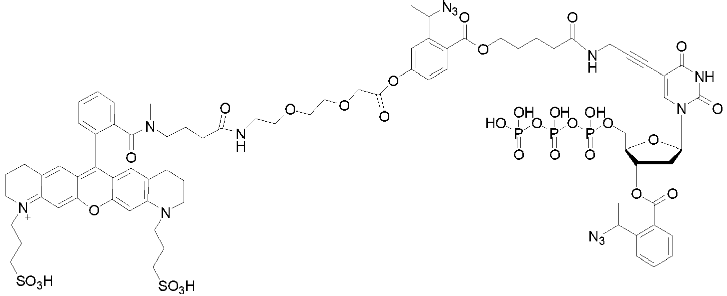 |
| 17B' | 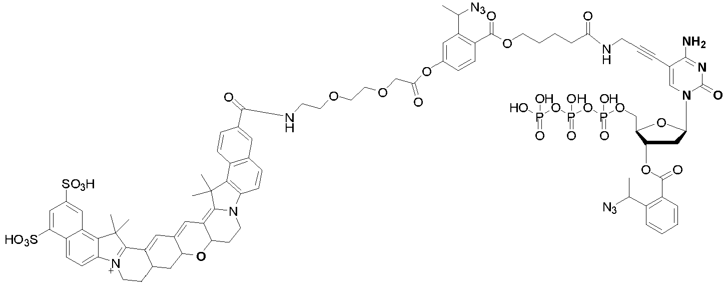 |
| 17C' | 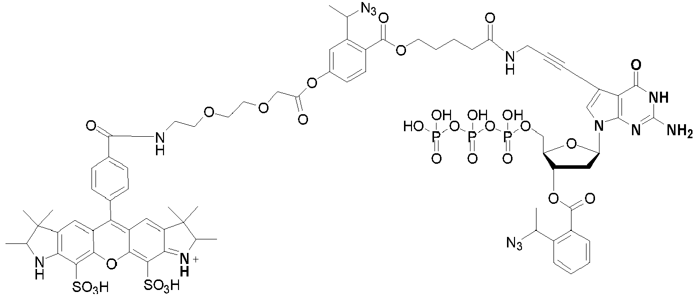 |
| 17D' | 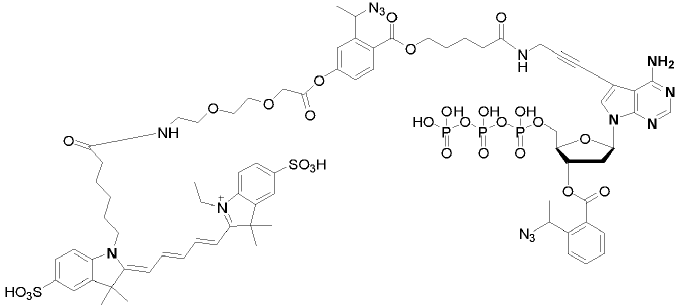 |
| 18A' | 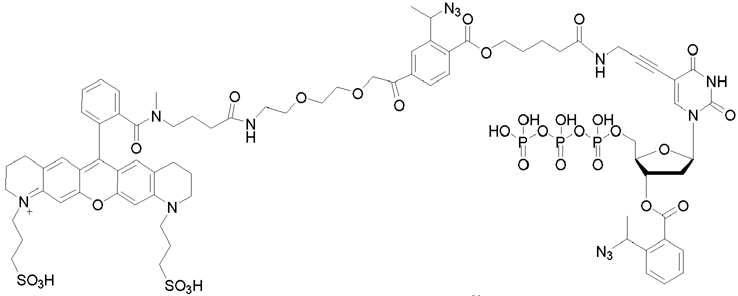 |
| 18B' | 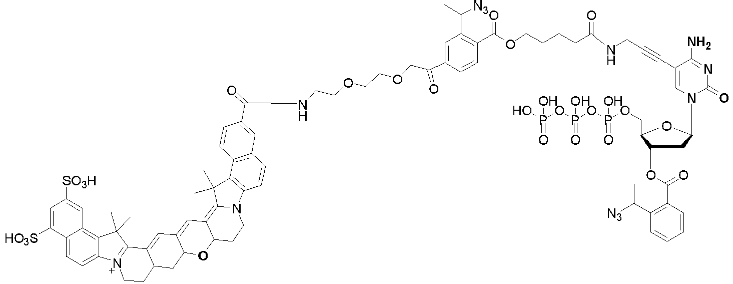 |
| 18C' | 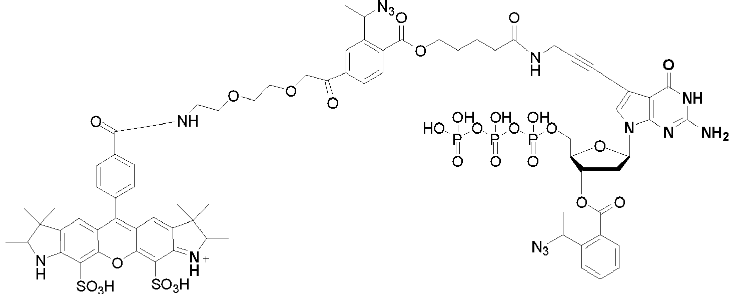 |
| 18D' | 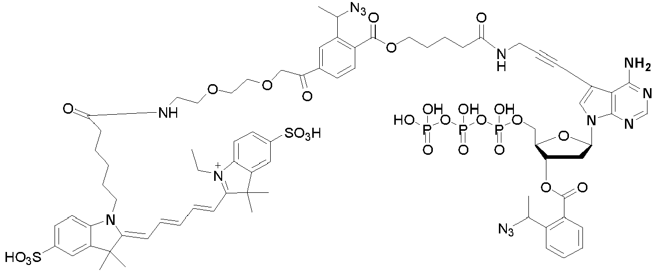 |
| 19A' | 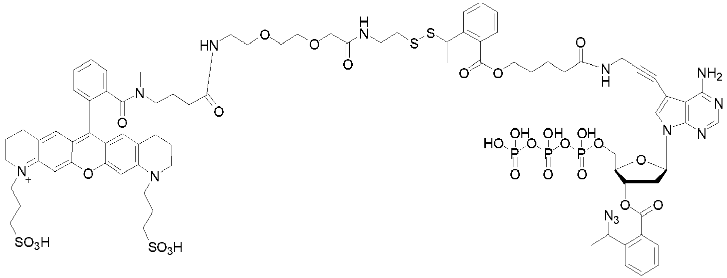 |
| 19B' | 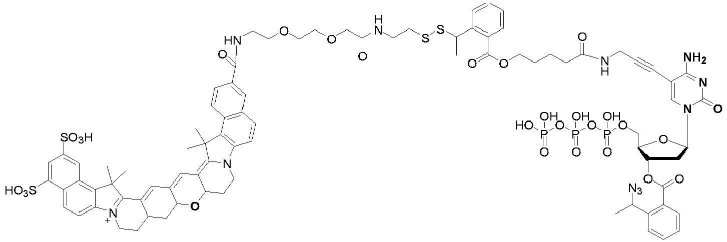 |
| 19C' | 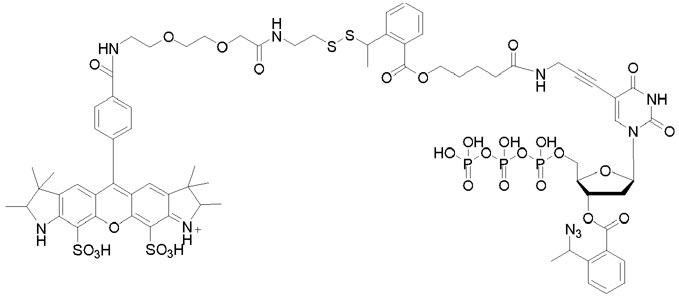 |
| 19D' | 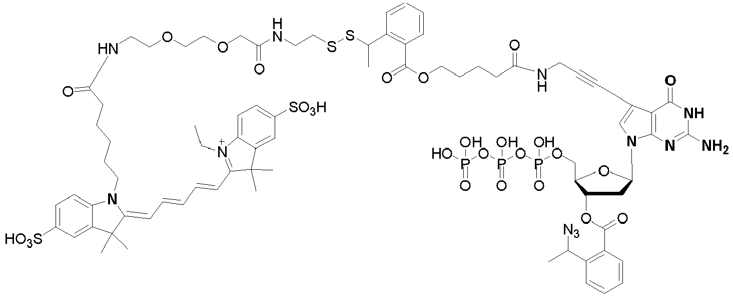 |
| 20A' | 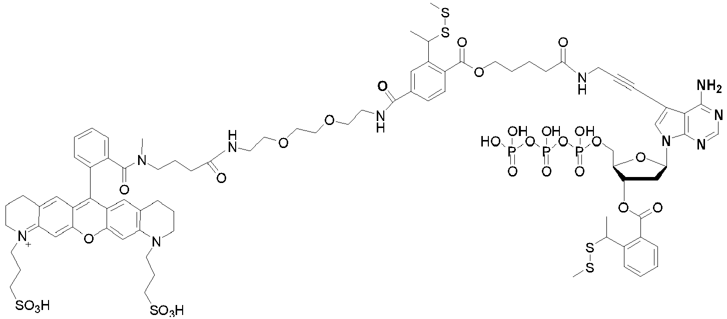 |
| 20B' | 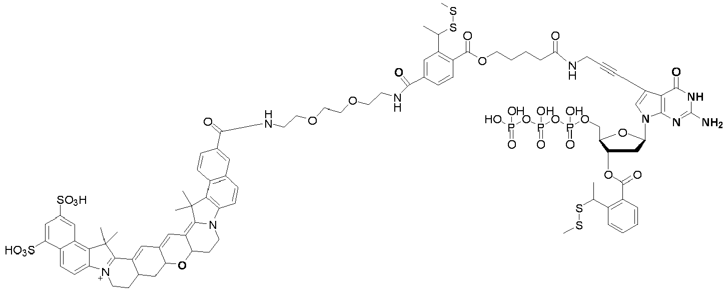 |
| 20C' | 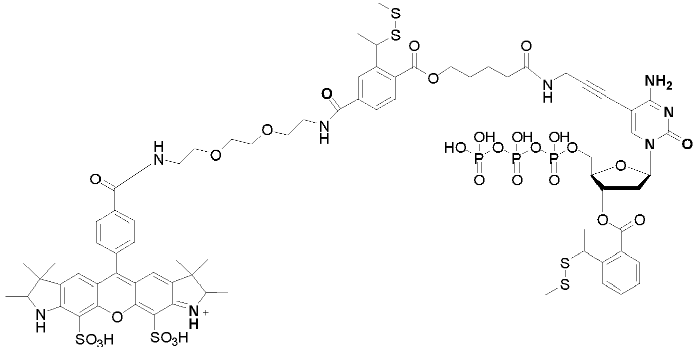 |
| 20D' | 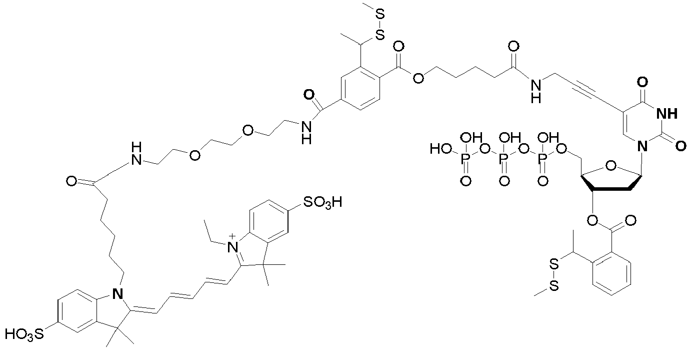 |
| 21A' | 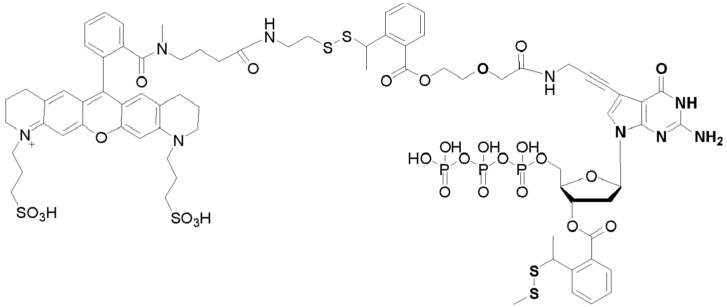 |
| 21B' | 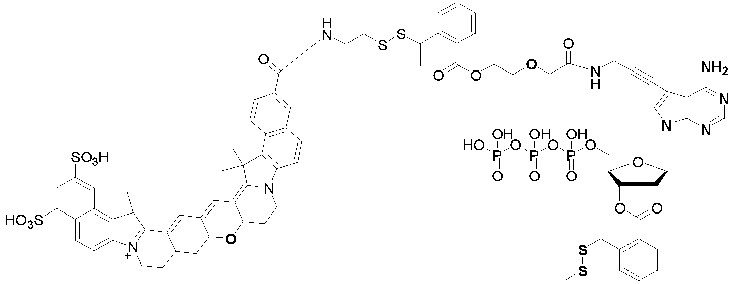 |
| 21C' | 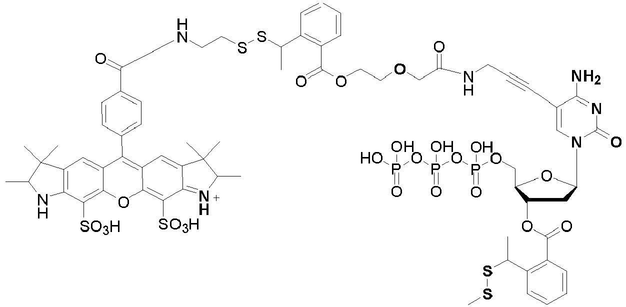 |
| 21D' | 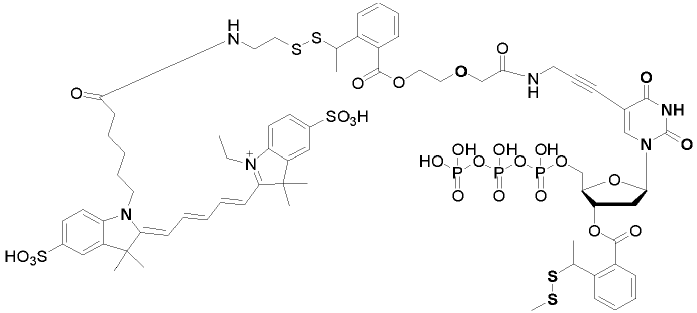 |
| 22A' | 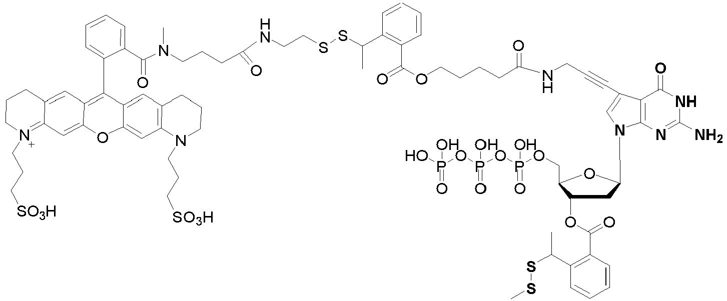 |
| 22B' | 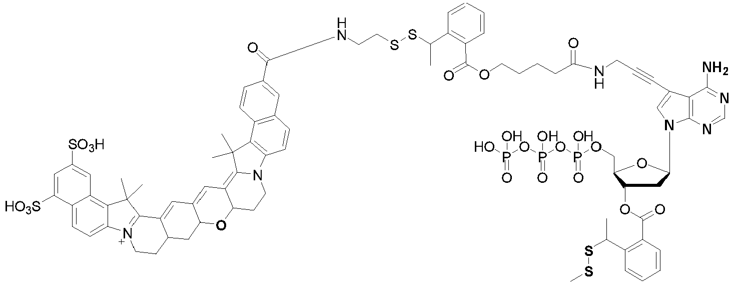 |
| 22C' | 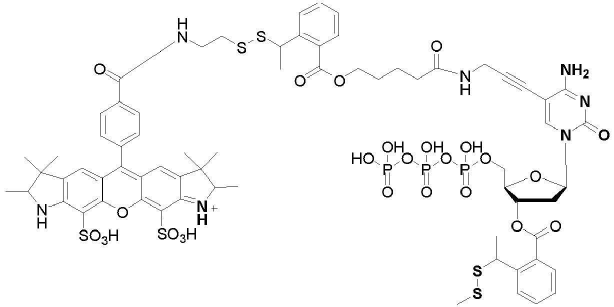 |
| 22D' | 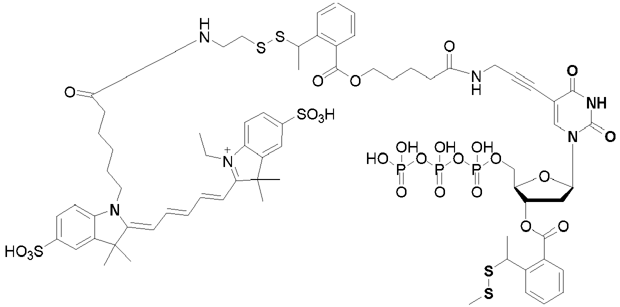 |
| 23A' | 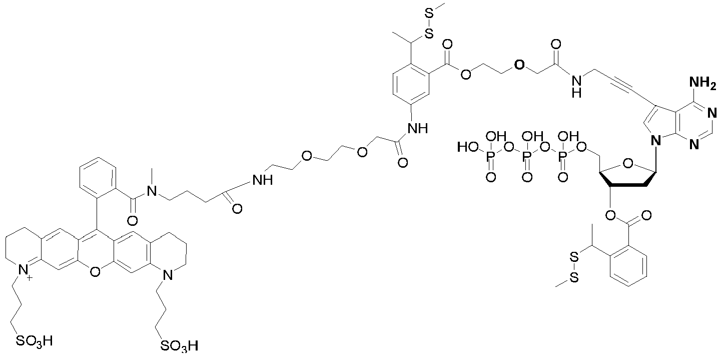 |
| 23B' | 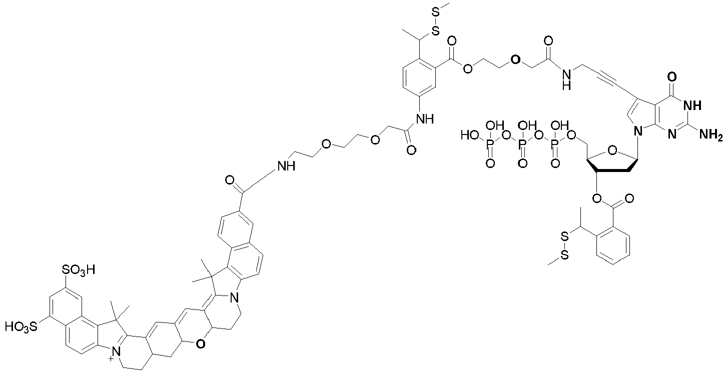 |
| 23B" | 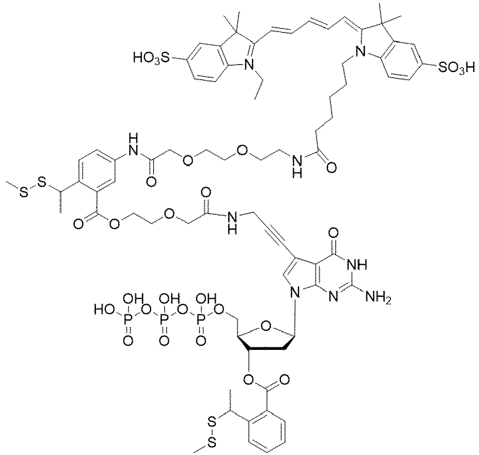 |
| 23C' | 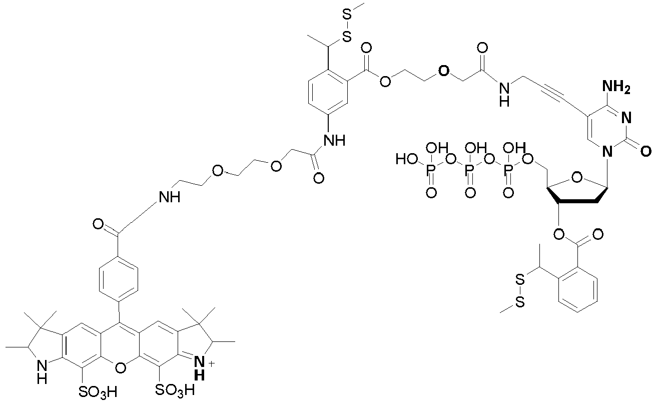 |
| 23D' | 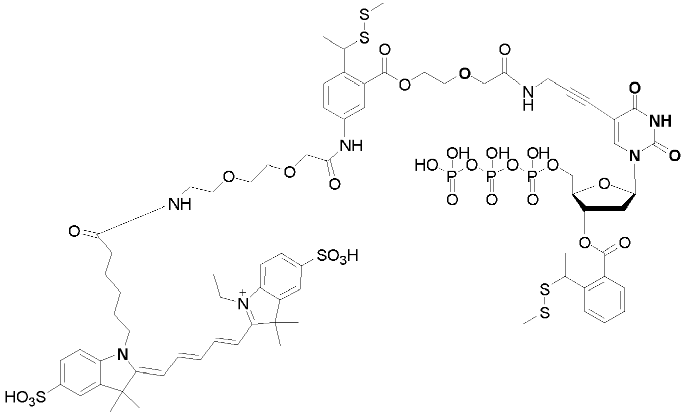 |
| 24A' | 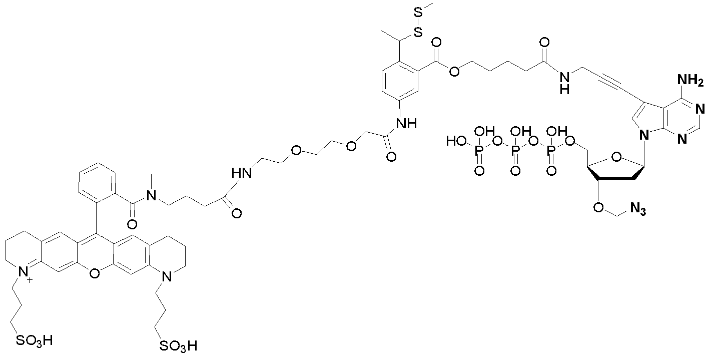 |
| 24B' | 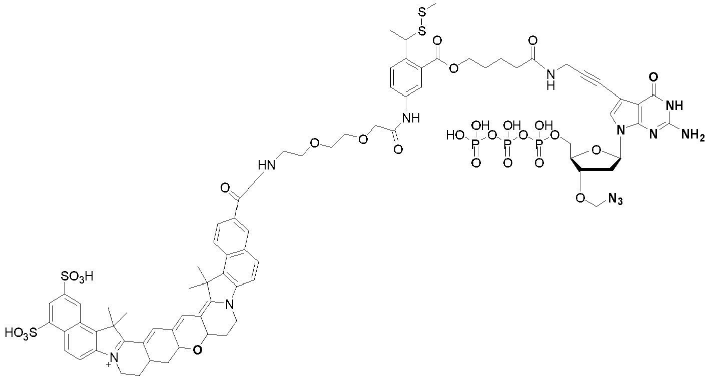 |
| 24C' | 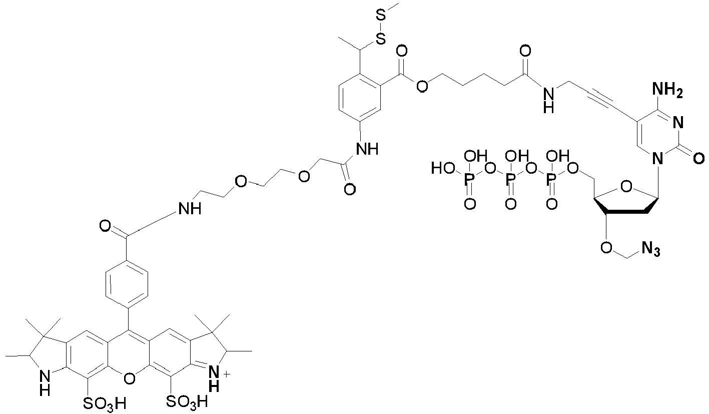 |
| 24D' | 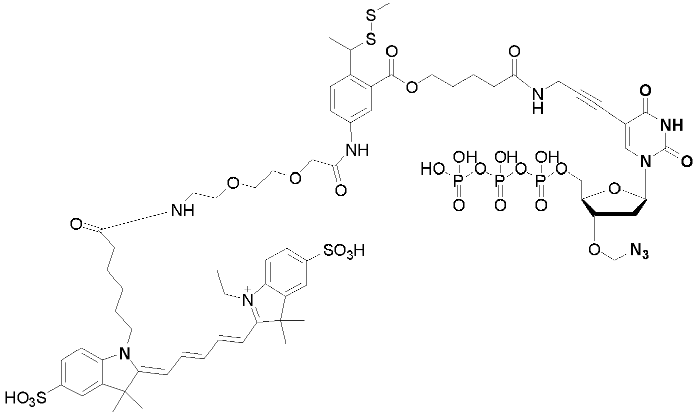 |
| 25A' | 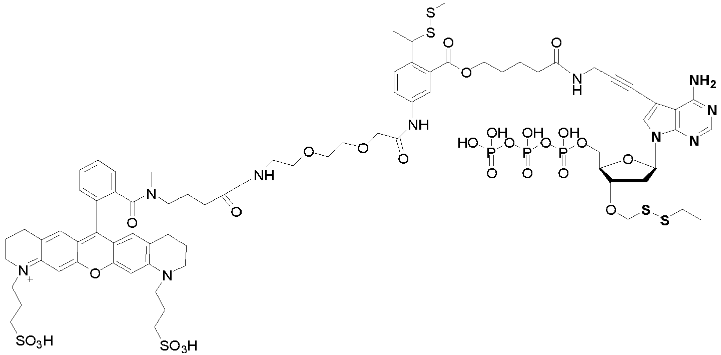 |
| 25B' | 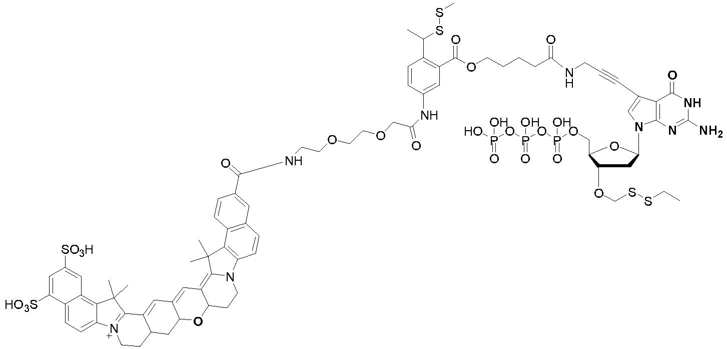 |
| 25C' | 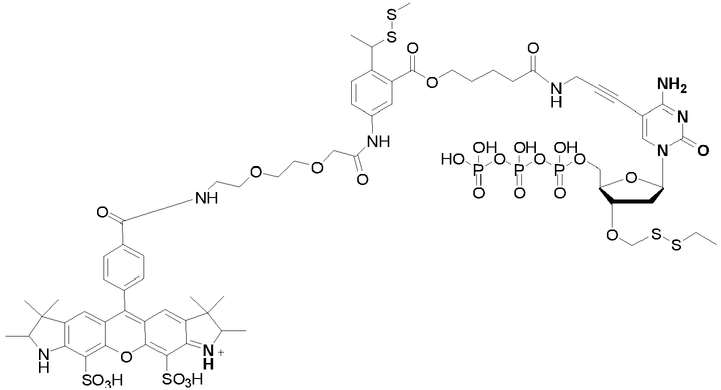 |
| 25D' | 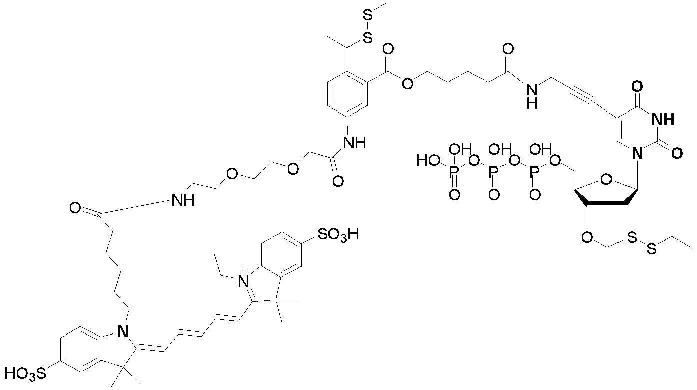 |
| 26A' | 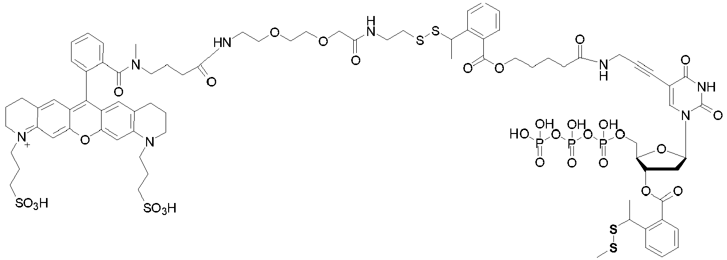 |
| 26B' | 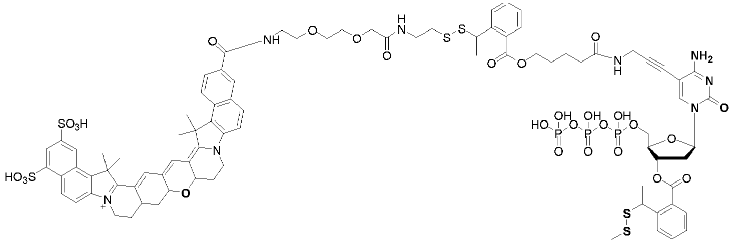 |
| 26C' | 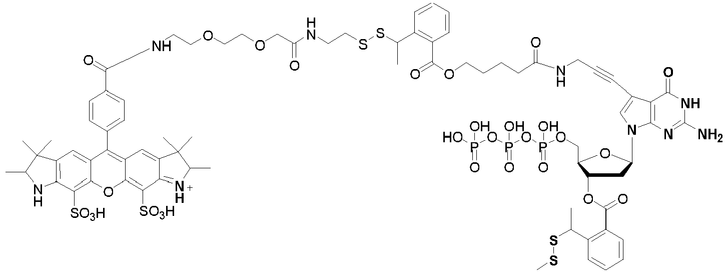 |
| 26D' | 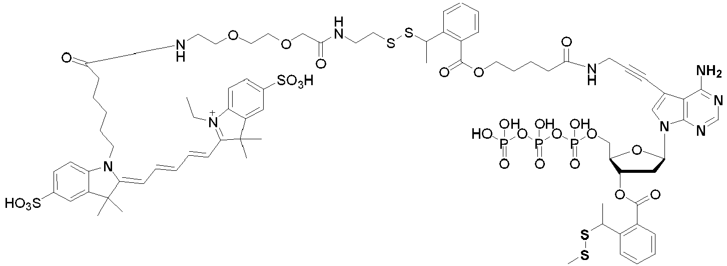 |
| 27A' | 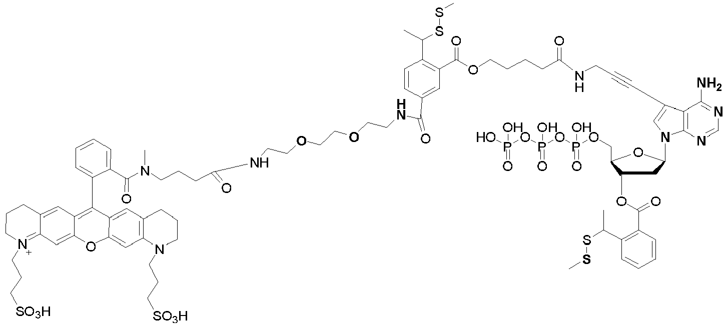 |
| 27B' | 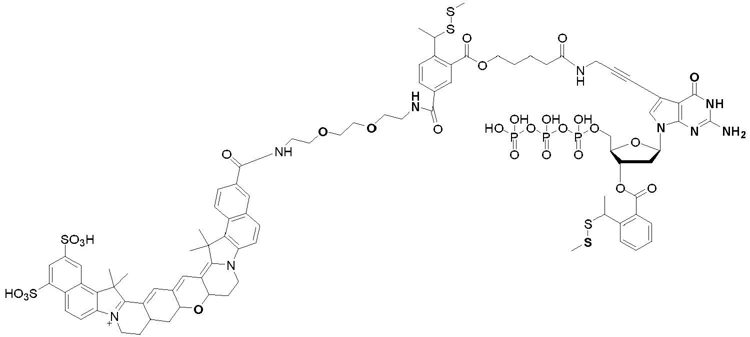 |
| 27C' | 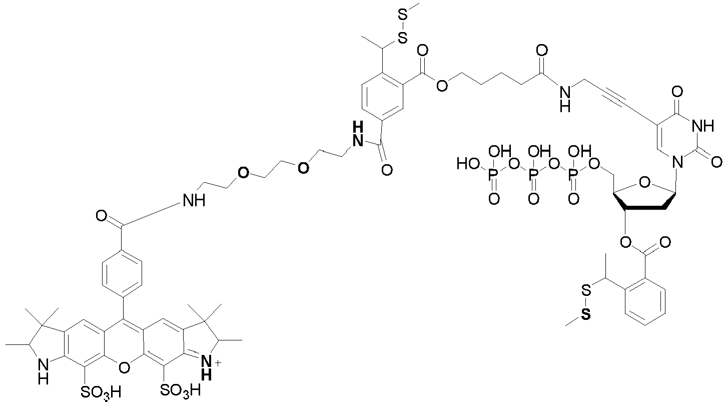 |
| 27D' | 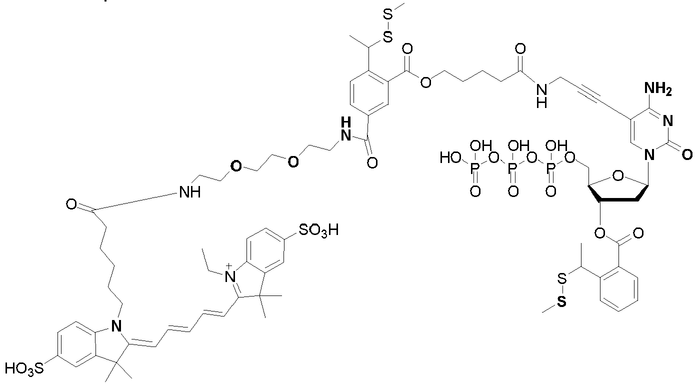 |
| 28A' | 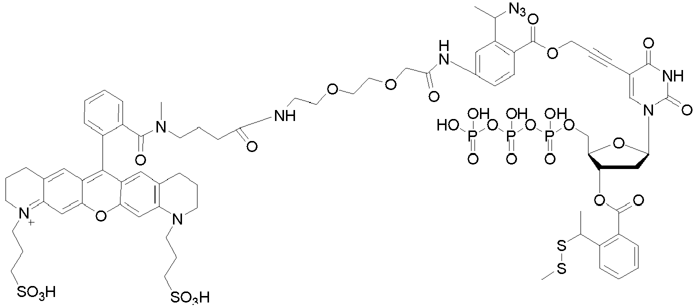 |
| 28B' | 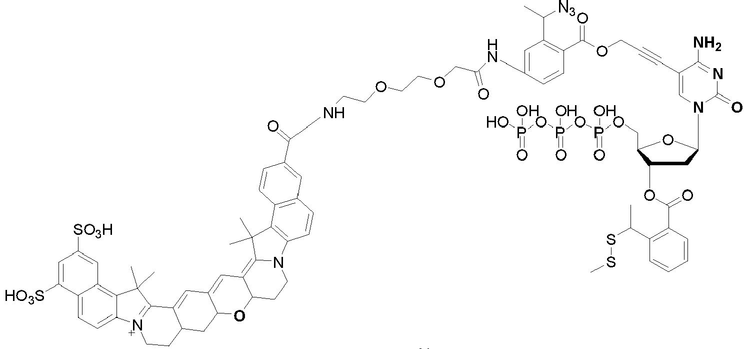 |
| 28C' | 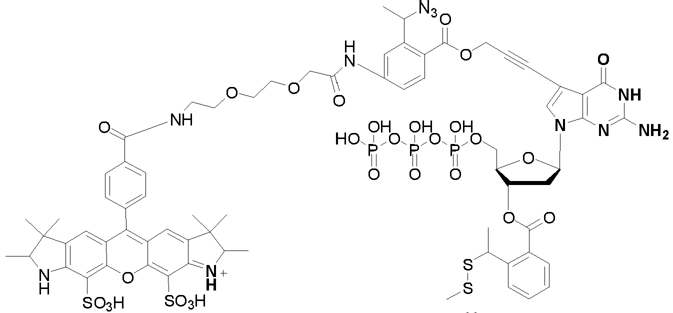 |
| 28D' | 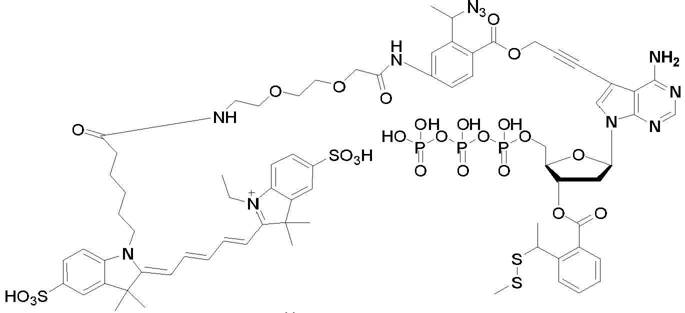 |
| 29A' | 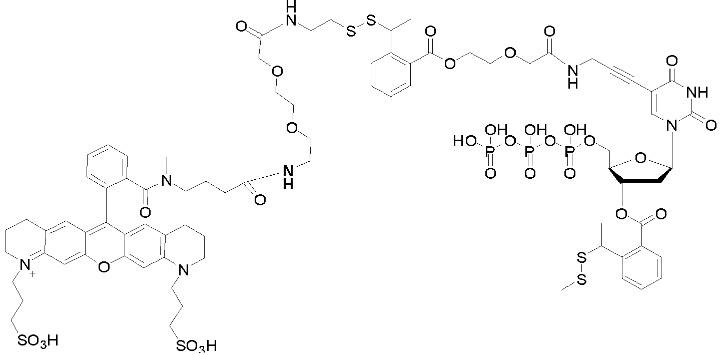 |
| 29B' | 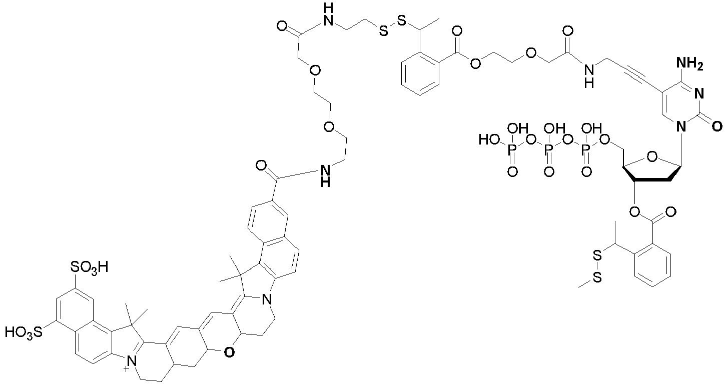 |
| 29C' | 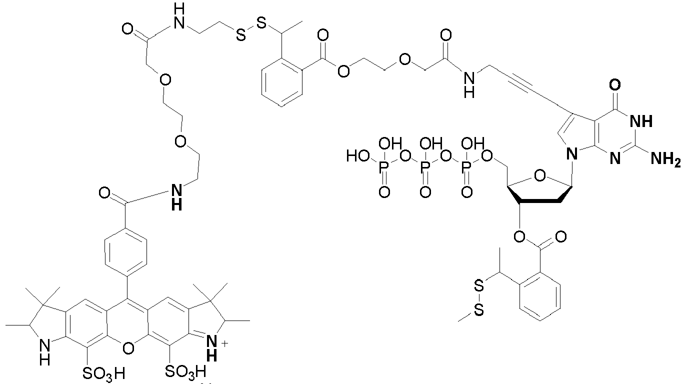 |
| 29D' | 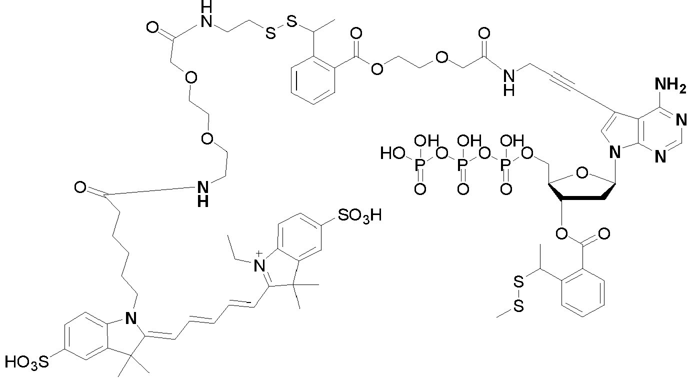 |
| 30A' | 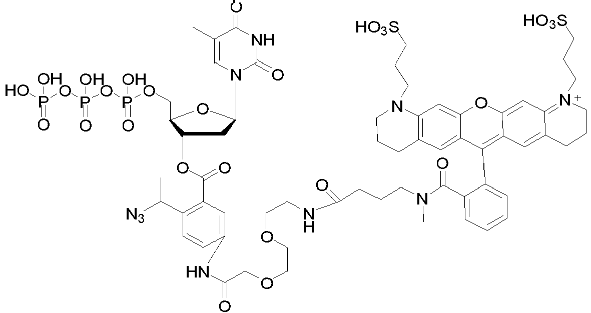 |
| 30B' | 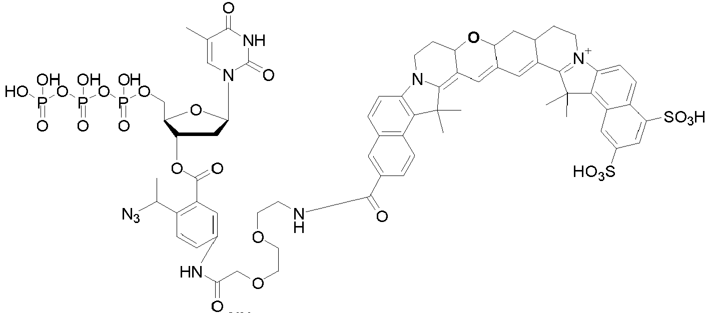 |
| 30C' | 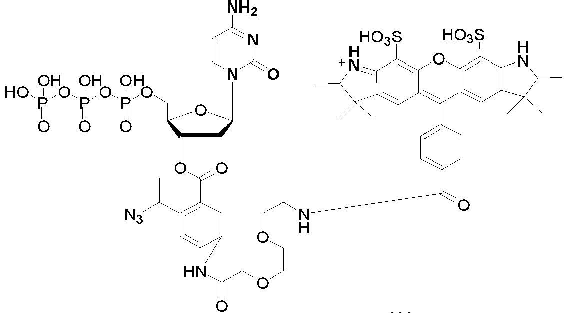 |
| 30D' | 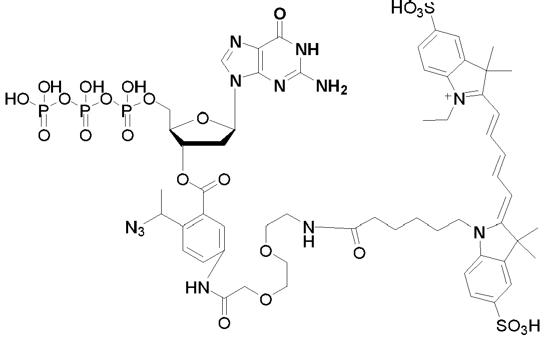 |
| 31A' | 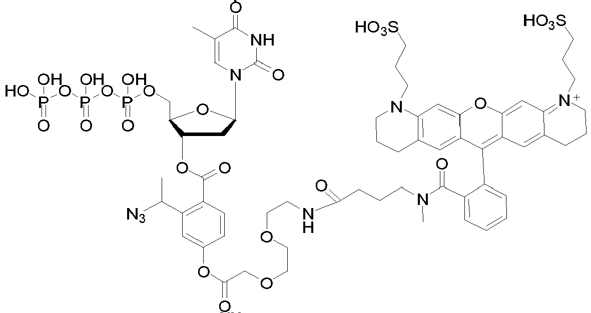 |
| 31B' | 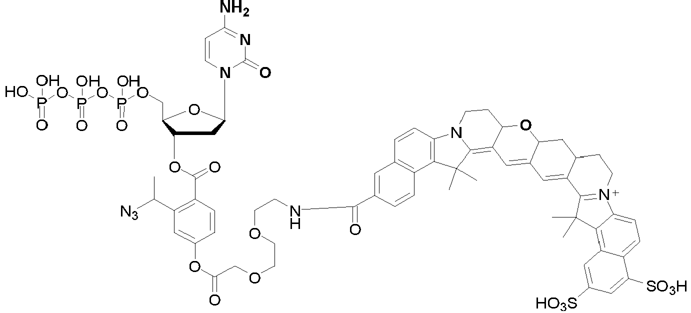 |
| 31C' | 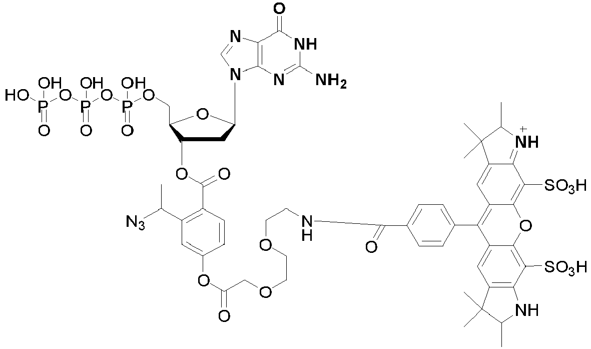 |
| 31D' | 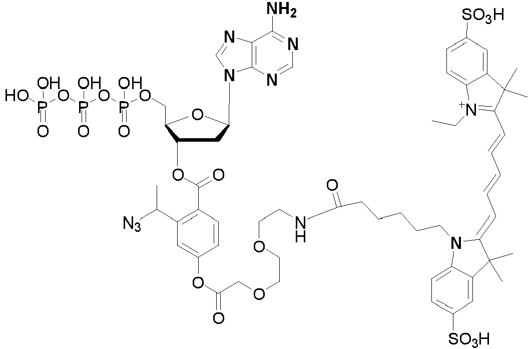 |
| 32A' | 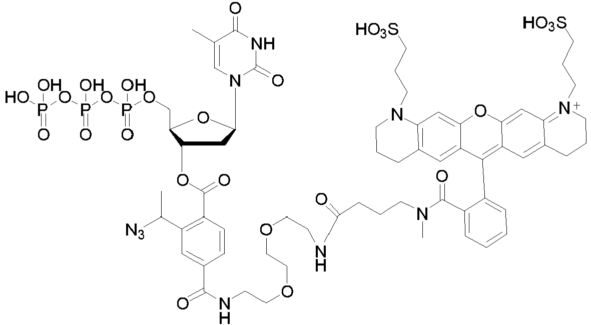 |
| 32B' | 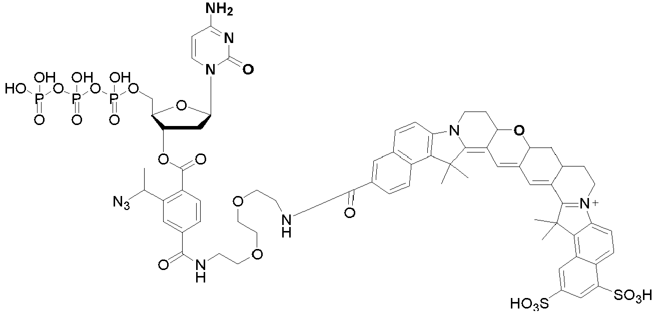 |
| 32C' | 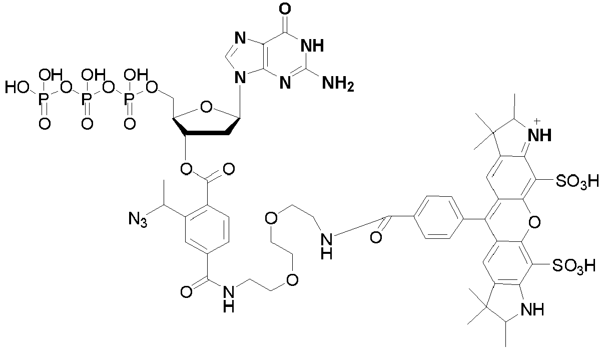 |
| 32D' | 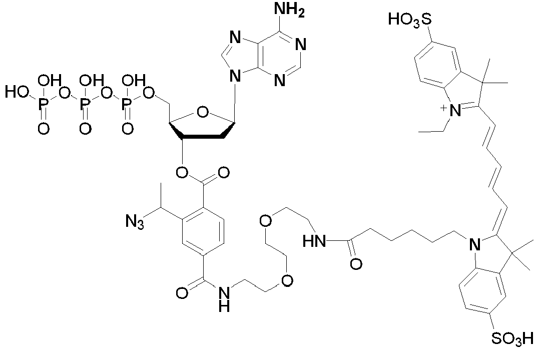 |
| 33A' | 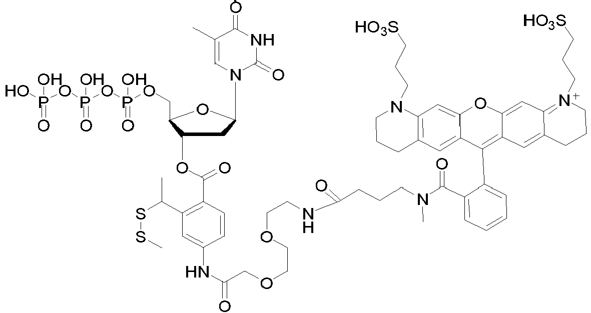 |
| 33B' | 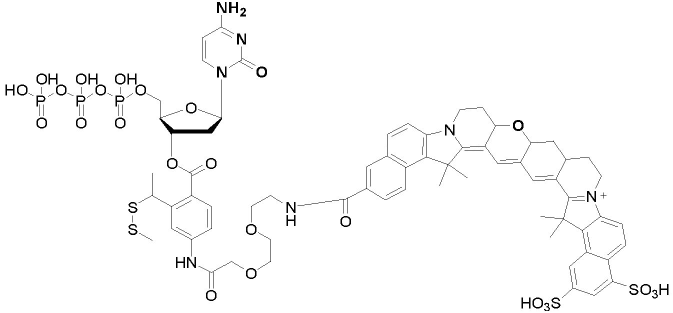 |
| 33C' | 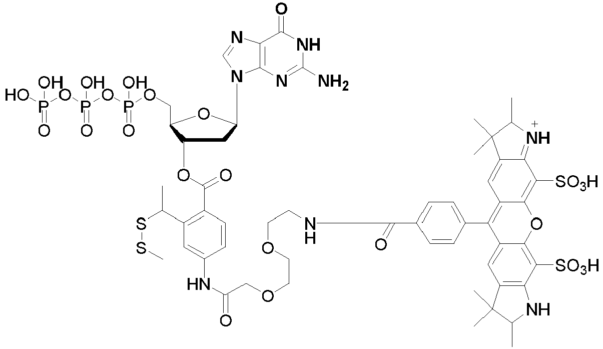 |
| 33D' | 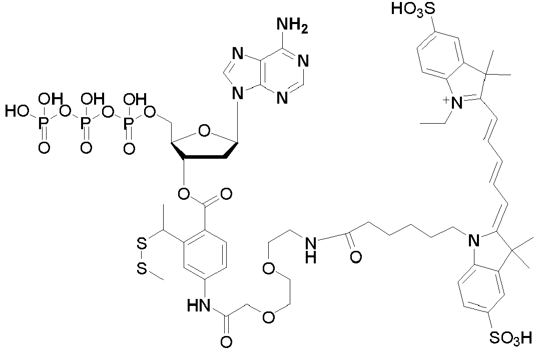 |
| 34A' | 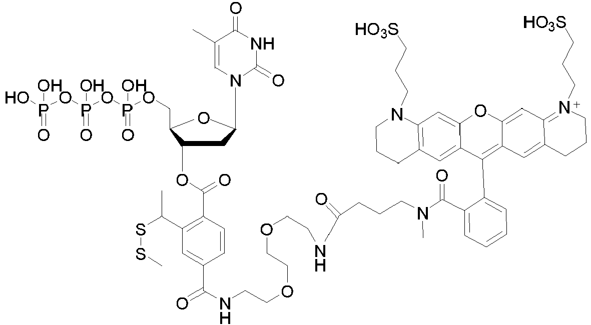 |
| 34B' | 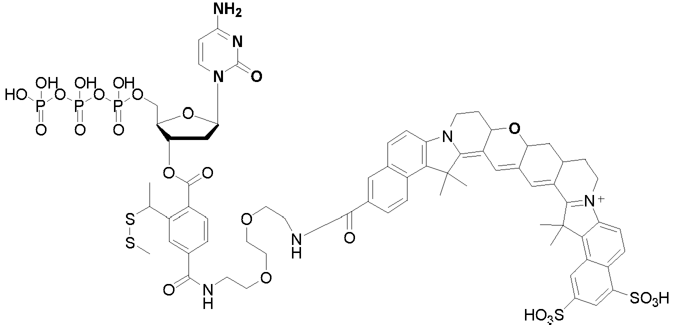 |
| 34C' | 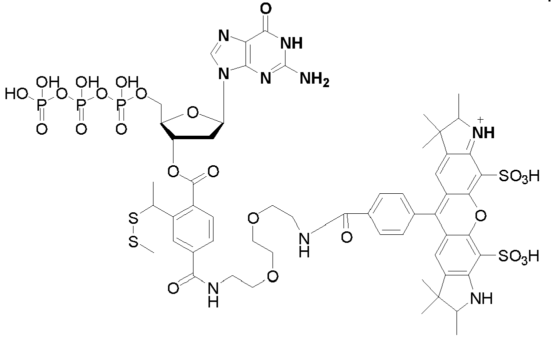 |
| 34D' | 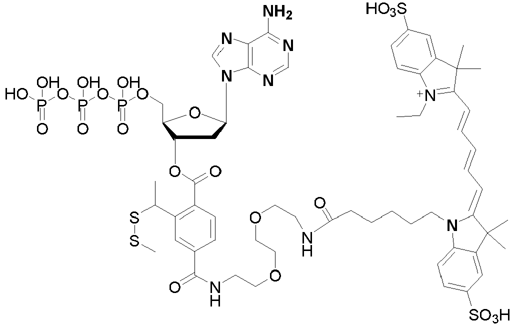 |
| 35A' | 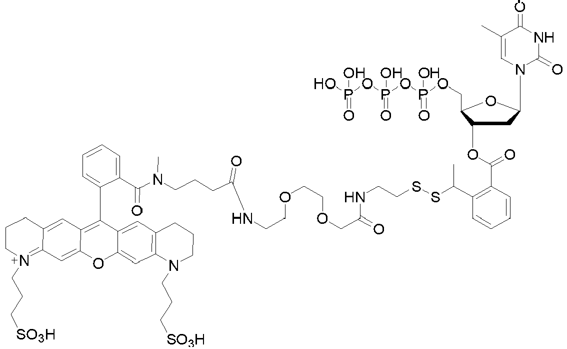 |
| 35B' | 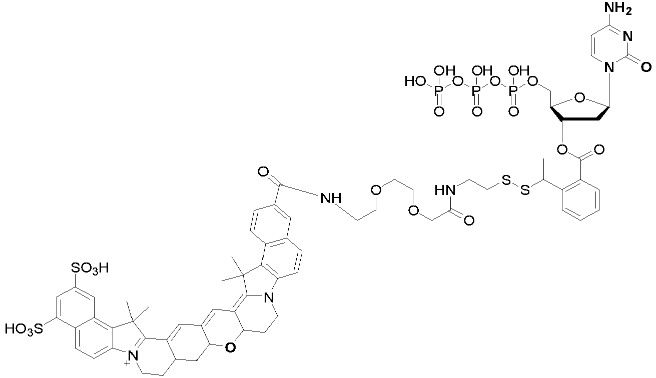 |
| 35C' | 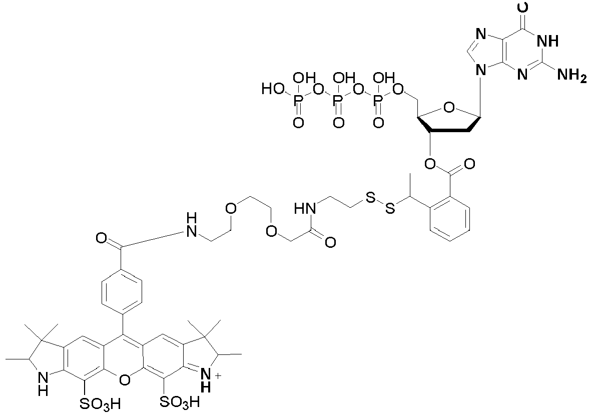 |
| 35D' | 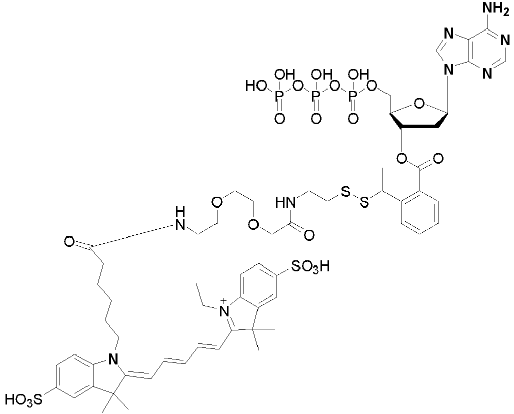 |
| | |
| 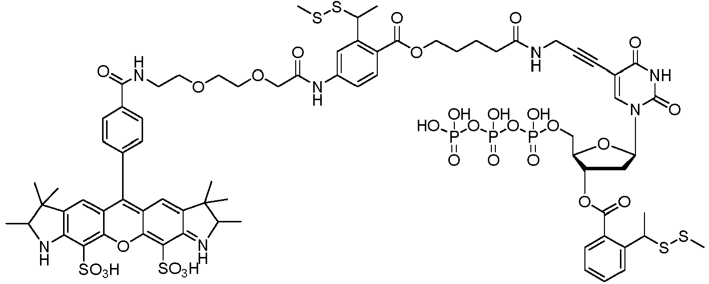 | |
| 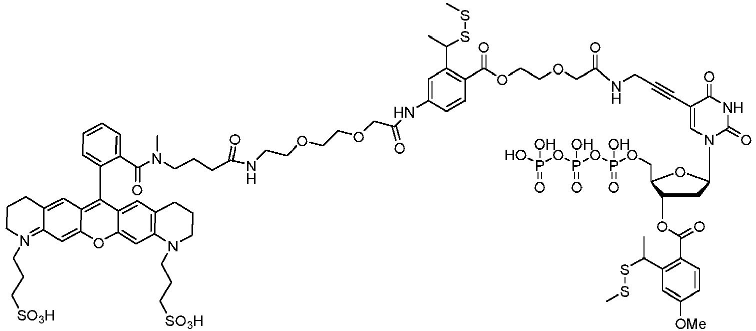 | |
| 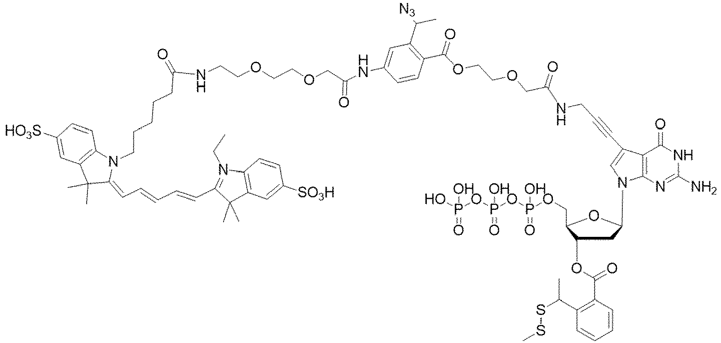 | |
| 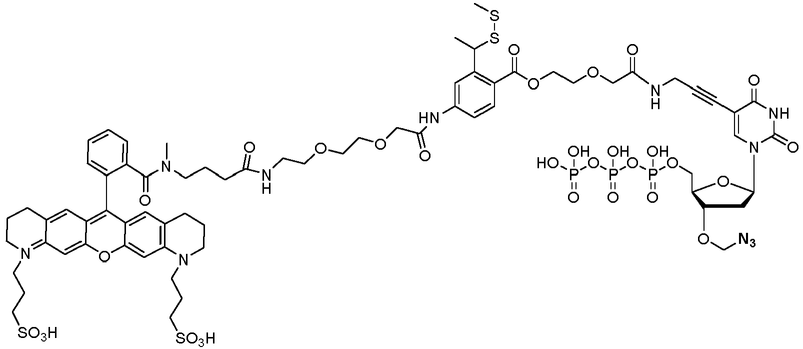 | |
| 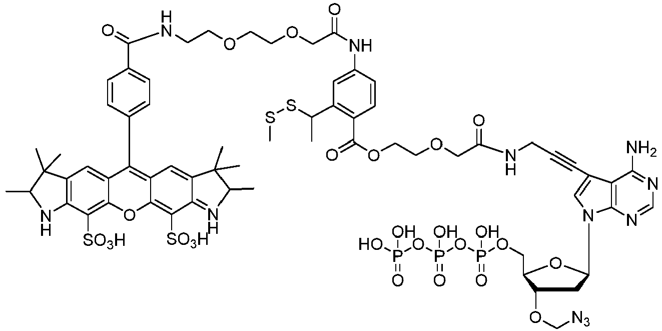 | |
| 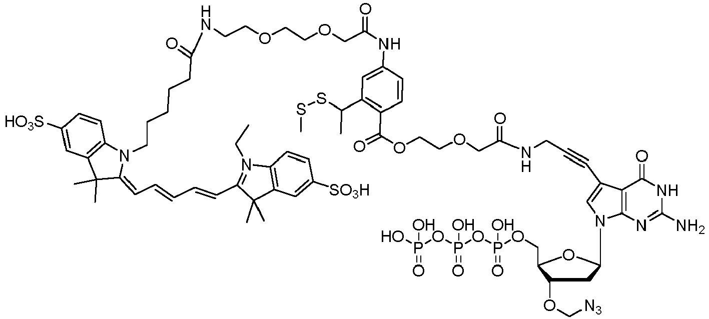 | |
| 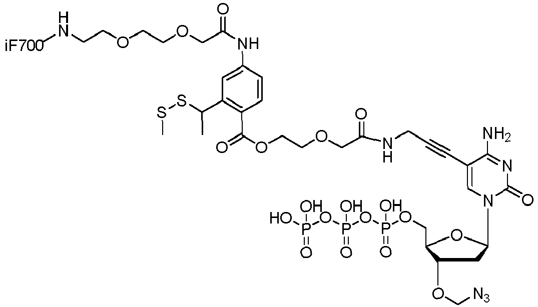 | |
| 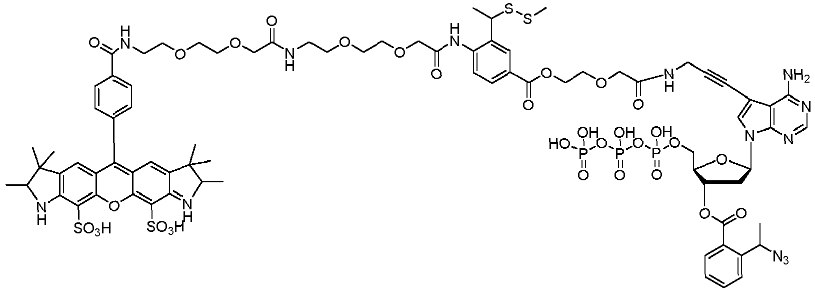 | |
| 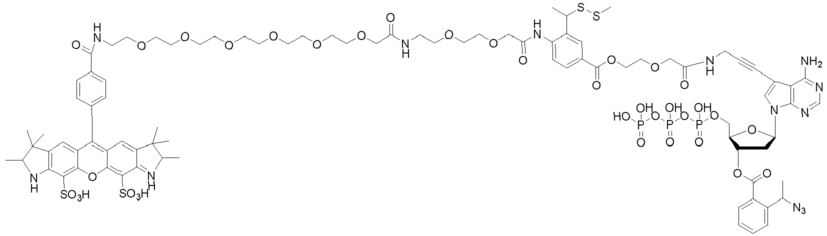 | |
| 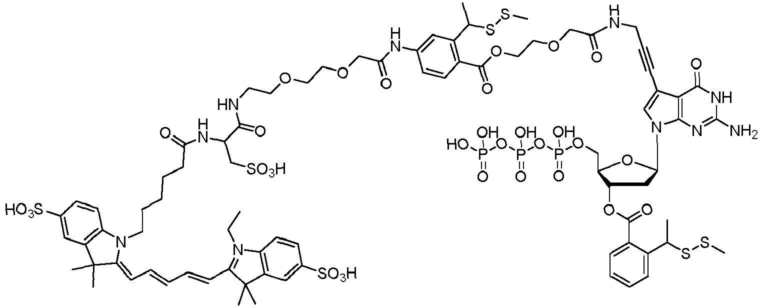 | |
| 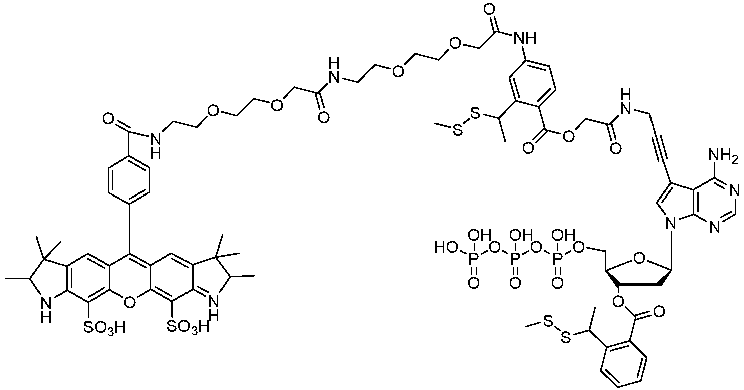 | |
| 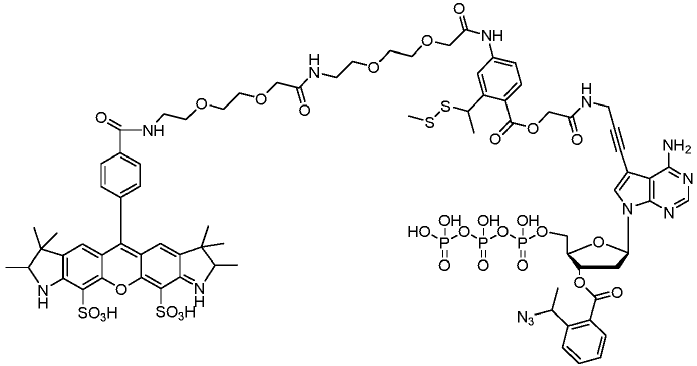 | |
| 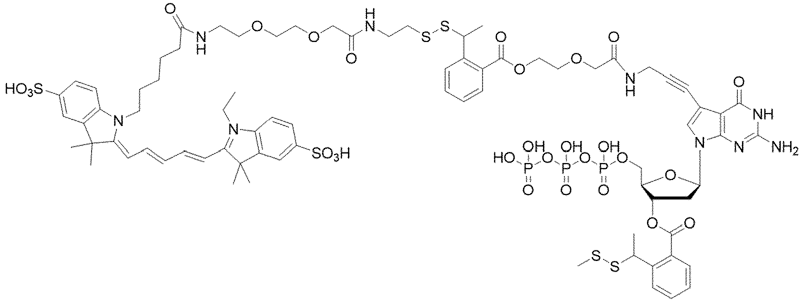 | |
| 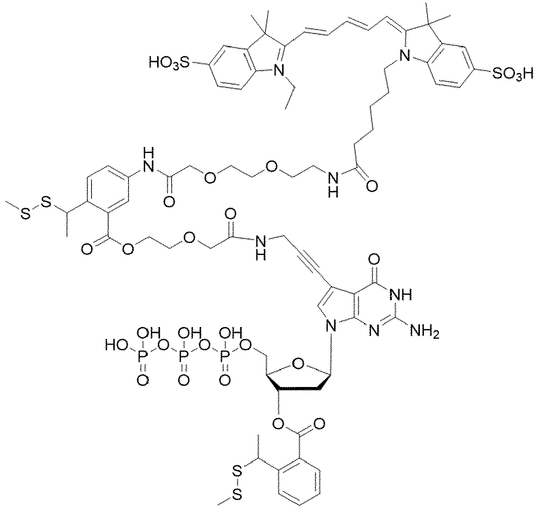 | |
| 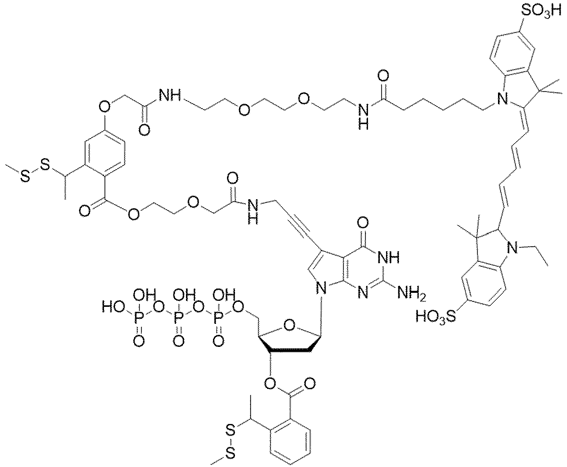 | |
| 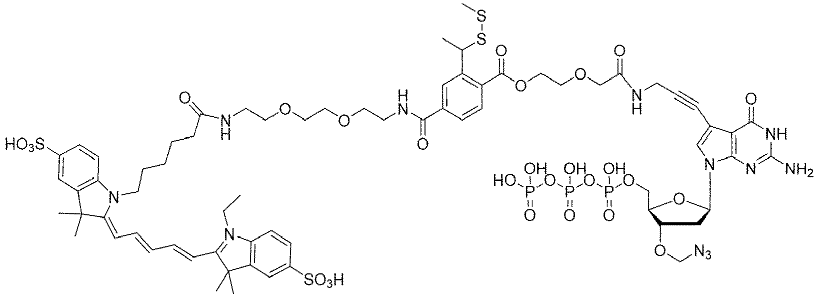 | |

12. A method for terminating a nucleic acid synthesis, which comprises incorporating the compound or a salt thereof according to any one of claims 1 to 11 into a nucleic acid molecule to be terminated;
preferably, the incorporation of the compound or a salt thereof is achieved by a terminal transferase, a terminal polymerase or a reverse transcriptase;
preferably, the method comprises incorporating the compound or a salt thereof into the nucleic acid molecule to be terminated by using a polymerase;
preferably, the method comprises performing a nucleotide polymerization reaction using a polymerase under conditions where the polymerase is allowed to carry out the nucleotide polymerization reaction, thereby incorporating the compound or a salt thereof into the 3' end of the nucleic acid molecule to be terminated

13. A method for preparing a growing polynucleotide complementary to a target single-stranded polynucleotide in a sequencing reaction, which comprises incorporating the compound or a salt thereof according to any one of claims 1 to 11 into the growing complementary polynucleotide, wherein the incorporation of the compound or a salt thereof prevents any subsequent nucleotides from being introduced into the growing complementary polynucleotide;
preferably, the incorporation of the compound or a salt thereof is achieved by a terminal transferase, a terminal polymerase or a reverse transcriptase;
preferably, the method comprises incorporating the compound or a salt thereof into the growing complementary polynucleotide by using a polymerase;
preferably, the method comprises performing a nucleotide polymerization reaction using a polymerase under conditions where the polymerase is allowed to carry out the nucleotide polymerization reaction, thereby incorporating the compound or a salt thereof into the 3' end of the growing complementary polynucleotide.

14. A nucleic acid intermediate, which is formed in determining the sequence of a target single-stranded polynucleotide, wherein,
the nucleic acid intermediate is formed according to the step:
incorporating a nucleotide complementary to the target single-stranded polynucleotide into a growing nucleic acid strand, to form the nucleic acid intermediate, wherein the incorporated complementary nucleotide is the compound or a salt thereof according to any one of claims 1 to 11;
or, the nucleic acid intermediate is formed according to the step:
incorporating a nucleotide complementary to the target single-stranded polynucleotide into a growing nucleic acid strand, to form the nucleic acid intermediate, wherein the incorporated complementary nucleotide is the compound or a salt thereof according to any one of claims 1 to 11, and the growing nucleic acid strand is pre-incorporated with at least one nucleotide complementary to the target single-stranded polynucleotide, the at least one pre-incorporated nucleotide complementary to the target single-stranded polynucleotide being the compound or a salt thereof according to any one of claims 1 to 11 in which the reversible blocking group and optional detectable label have been removed.

15. A method for determining the sequence of a target single-stranded polynucleotide, which comprises:
1) monitoring the incorporation of nucleotides complementary to the target single-stranded polynucleotide in a growing nucleic acid strand, wherein at least one complementary nucleotide incorporated is the compound or a salt thereof according to any one of claims 1 to 11, and,
2) determining the types of the incorporated nucleotides;
preferably, the reversible blocking group and optional detectable label are removed before a next complementary nucleotide is introduced;
preferably, the reversible blocking group and the detectable label are removed simultaneously;
preferably, the reversible blocking group and the detectable label are removed sequentially; for example, after the detectable label is removed, the reversible blocking group is removed, or, after the reversible blocking group is removed, the detectable label is removed.

16. The method according to claim 15, which comprises the following steps of:
(a) providing a plurality of different nucleotides, wherein at least one nucleotide is the compound or a salt thereof according to any one of claims 10 to 11, optionally, the rest of nucleotides are the compound or a salt thereof according to any one of claims 1 to 11;
(b) incorporating the plurality of different nucleotides into a sequence complementary to a target single-stranded polynucleotide, wherein the plurality of different nucleotides can be distinguished from each other during the detection;
(c) detecting the nucleotides in step (b) to determine the types of nucleotides incorporated;
(d) removing the reversible blocking groups and optional detectable labels carried by the nucleotides in step (b); and
(e) optionally repeating steps (a)-(d) one or more times;
whereby the sequence of the target single-stranded polynucleotide is determined.

17. The method according to claim 15, which comprises the following steps of:
(1) providing a first nucleotide, a second nucleotide, a third nucleotide and a fourth nucleotide, wherein at least one of the four nucleotides is the compound or a salt thereof according to any one of claims 10 to 11, optionally, the rest of nucleotides are the compound or a salt thereof according to any one of claims 1 to 11;
(2) contacting the four nucleotides with a target single-stranded polynucleotide; removing the nucleotides not incorporated into the growing nucleic acid strand; detecting the nucleotides incorporated into the growing nucleic acid strand; removing the reversible blocking groups and optional detectable labels carried in the nucleotides that are incorporated into the growing nucleic acid strand;
optionally, it also includes (3): repeating the steps (1)-(2) one or more times.

18. The method according to claim 15, which comprises the following steps of:
(a) providing a mixture comprising a duplex, nucleotides comprising at least one compound or a salt thereof according to any one of claims 10 to 11, a polymerase, and an excision reagent; wherein the duplex comprises a growing nucleic acid strand and a nucleic acid strand to be sequenced;
(b) carrying out a reaction comprising the following steps (i), (ii) and (iii), optionally, repeating the steps for one or more times:
step (i): incorporating the compound or a salt thereof into the growing nucleic acid strand by using a polymerase, to form a nucleic acid intermediate containing the reversible blocking group and optional detectable label;
step (ii): detecting the nucleic acid intermediate;
step (iii): removing the reversible blocking group and optional detectable label contained in the nucleic acid intermediate by using the excision reagent;
preferably, removal of the reversible blocking group and removal of the detectable label are performed simultaneously, or, removal of the reversible blocking group and removal of the detectable label are performed sequentially (for example, the reversible blocking group is removed first, or the detectable label is removed first);
preferably, the excision reagent used for removal of the reversible blocking group is the same as that used for removal of the detectable label;
preferably, the excision reagent used for removal of the reversible blocking group is different from that used for removal of the detectable label.

19. The method according to claim 18, wherein the duplex is linked to a support;
preferably, the growing nucleic acid strand is a primer;
preferably, the primer is annealed to the nucleic acid strand to be sequenced to form the duplex;
preferably, the duplex, the compound or a salt thereof, and the polymerase together form a reaction system containing a solution phase and a solid phase;
preferably, the compound or a salt thereof is incorporated into the growing nucleic acid strand by using a polymerase under conditions where the polymerase is allowed to carry out a nucleotide polymerization reaction, thereby forming a nucleic acid intermediate containing the reversible blocking group and optional detectable label;
preferably, the polymerase is selected from KOD polymerase or a mutant thereof (e.g., KOD POL151, KOD POL157, KOD POL171, KOD POL174, KOD POL376, KOD POL391);
preferably, before any step of detecting the nucleic acid intermediate, the solution phase of the reaction system in the previous step is removed, and the duplex linked to the support is retained;
preferably, the excision reagent is in contact with the duplex or the growing nucleic acid strand in the reaction system containing a solution phase and a solid phase;
preferably, the excision reagent can remove the reversible blocking group and optional detectable label carried by the compound that is incorporated into the growing nucleic acid strand, without affecting the phosphodiester bond on the backbone of the duplex;
preferably, after any step of removing the reversible blocking group and optional detectable label contained in the nucleic acid intermediate, the solution phase of the reaction system in this step is removed;
preferably, a washing operation is performed after any step comprising a removal operation;
preferably, after step (ii), the method further comprises: determining the type of compound incorporated into the growing nucleic acid strand in step (i) according to the signal detected in step (ii), and determining the type of nucleotide at a corresponding position in the nucleic acid strand to be sequenced based on the principle of base complementary pairing.

20. A kit, which comprises at least one compound or a salt thereof according to any one of claims 1 to 11;
preferably, the kit comprises a first compound, a second compound, a third compound and a fourth compound, wherein the first, second, third and fourth compounds each are, independently, the compounds or salts thereof according to any one of claims 1 to 11;
preferably, in the first compound, base¹ is selected from adenine, 7-deazaadenine or a tautomer thereof (e.g., 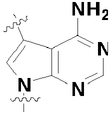 ); in the second compound, base¹ is selected from thymine, uracil or a tautomer thereof (e.g., 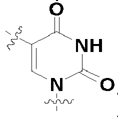 ); in the third compound, base¹ is selected from cytosine or a tautomer thereof (e.g., 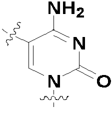 ); in the fourth compound, base¹ is selected from guanine, 7-deazaguanine or a tautomer thereof (e.g., 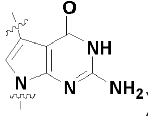 );
preferably, in the first compound, base² is selected from adenine, 7-deazaadenine or a tautomer thereof (e.g., 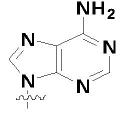 ); in the second compound, base² is selected from thymine, uracil or a tautomer thereof (e.g., 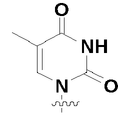 ); in the third compound, base² is selected from cytosine or a tautomer thereof (e.g., 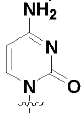 ); in the fourth compound, base² is selected from guanine, 7-deazaguanine or a tautomer thereof (e.g., 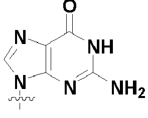 );
preferably, wherein the base¹ or base² contained in the first, second, third and fourth compounds are different from each other;
preferably, wherein the additional detectable labels carried by the first, second, third and fourth compounds are different from each other.

21. The kit according to claim 20, wherein the kit further comprises a reagent for pretreating the nucleic acid molecule; a support for linking nucleic acid molecule to be sequenced; a reagent for linking (for example, covalently or non-covalently linking) the nucleic acid molecule to be sequenced to the support; primers for initiating a nucleotide polymerization reaction; a polymerase for carrying out the nucleotide polymerization reaction; one or more buffer solutions; one or more washing solutions; or any combination thereof.

22. Use of the compound or a salt thereof according to any one of claims 1 to 11 or the kit according to any one of claims 20 to 21 for determining the sequence of a target single-stranded polynucleotide.

23. A compound of formula I-1 or a salt thereof, 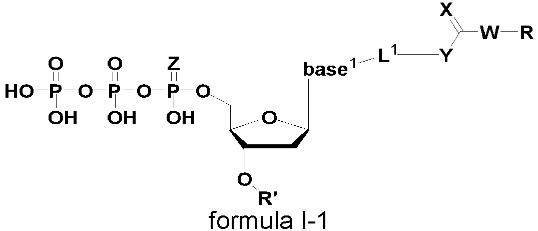 wherein:
L¹ is selected from 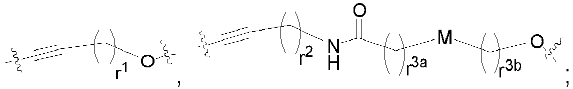
preferably, L¹ is selected from 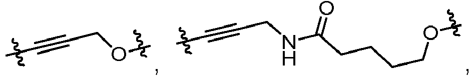 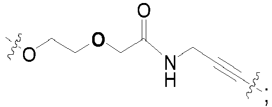
r¹, r², r^{3a}, r^{3b}, independently of each other, are selected from any integer between 1 and 6;
preferably, r¹ is selected from 1, 2, 3;
more preferably, r¹ is 1;
preferably, r² is selected from 1, 2, 3;
more preferably, r² is 1;
preferably, r^{3a}, r^{3b}, independently of each other, are selected from 1, 2, 3, 4, 5;
more preferably, r^{3a}, r^{3b}, independently of each other, are selected from 1, 2, 3;
most preferably, r^{3a} is 1;
most preferably, r^{3b} is 2;
M is selected from a direct bond, CH₂, NH, O, S;
preferably, M is selected from CH₂, O;
X is selected from O, S, NH;
preferably, X is selected from O, S;
more preferably, X is O;
Y is selected from a direct bond, O, S, NH;
preferably, Y is a direct bond;
W is selected from a direct bond, O, S, NH;
preferably, W is a direct bond;
R is 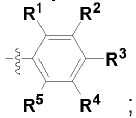
in R¹, R², R³, R⁴, R⁵, any one (e.g., R², R³ or R⁴) is 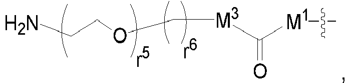 another (e.g., R¹ or R⁵) is 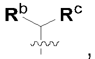 and the remaining three are H;
preferably, in R¹, R², R³, R⁴, R⁵, R³ or R⁴ is 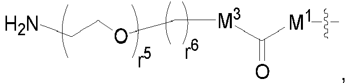 R¹ is 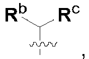 and the remaining three are H;
r⁵, r⁶, independently of each other, are selected from any integer between 1 and 6;
preferably, r⁵ is selected from 1, 2, 3;
more preferably, r⁵ is 2;
preferably, r⁶ is selected from 1, 2, 3;
more preferably, r⁶ is 1 or 2;
M¹, M³, independently of each other, are selected from a direct bond, NH, O, S;
preferably, M¹ is selected from a direct bond, NH, O;
preferably, M³ is selected from a direct bond, NH;
more preferably, when M¹ is NH, O or S, M³ is a direct bond, and when M³ is NH, O or S, M¹ is a direct bond;
in R^{b}, R^{c}, any one is selected from -N₃, -SS-C₁-C₆ alkyl (e.g., -SS-Me, -SS-Et, -SS-iPr, -SS-t-Bu), and the other is selected from C₁-C₆ alkyl;
preferably, in R^{b}, R^{c}, any one is selected from -N₃, -SS-Me, and the other is methyl;
Z is selected from O, S, BH;
preferably, Z is O;
base¹ is selected from a base, a deaza base or a tautomer thereof, for example, adenine, 7-deazaadenine, thymine, uracil, cytosine, guanine, 7-deazaguanine or a tautomer thereof;
preferably, base¹ is selected from 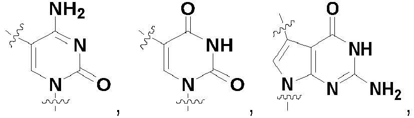 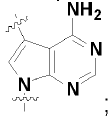
R' represents a reversible blocking group.

24. The compound or a salt thereof according to claim 23, wherein the reversible blocking group R' is selected from N₃-CH₂-, CH₃-CH₂-S-S-CH₂-, 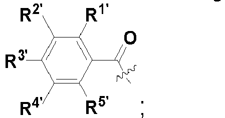
in R^{1'}, R^{2'}, R^{3'}, R^{4'}, R^{5'}, any one (e.g., R^{1'} or R^{5'}) is 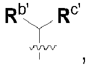 and the others are H
preferably, in R^{1'}, R^{2'}, R^{3'}, R^{4'}, R^{5'}, R^{1'} is 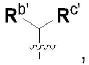 and the others are H,
in R^{b'}, R^{c'}, any one is selected from -N₃, -SS-C₁-C₆ alkyl (e.g., -SS-Me, -SS-Et, -SS-iPr, -SS-t-Bu), and the other is selected from C₁-C₆ alkyl,
preferably, in R^{b'}, R^{c'}, any one is selected from -N₃, -SS-Me, and the other is methyl.

25. The compound or a salt thereof according to any one of claims 23 to 24, wherein the compound is selected from:
| Serial Number | Structural formulae |
|---|---|
| 7A | 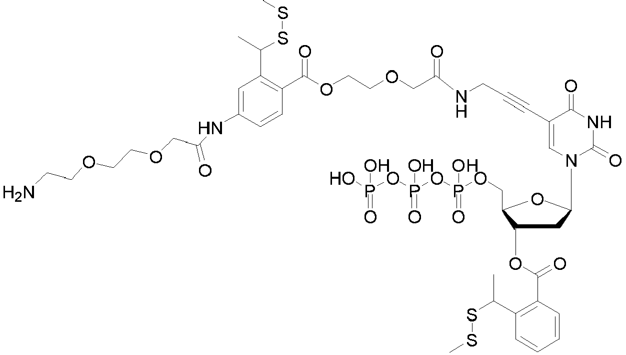 |
| 7B | 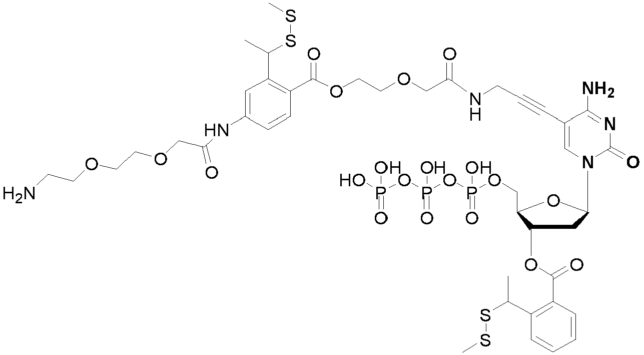 |
| 7C | 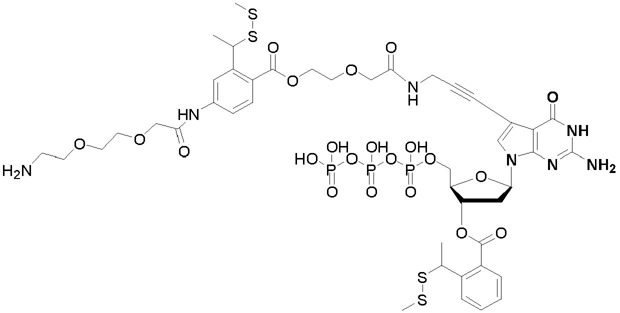 |
| 7D | 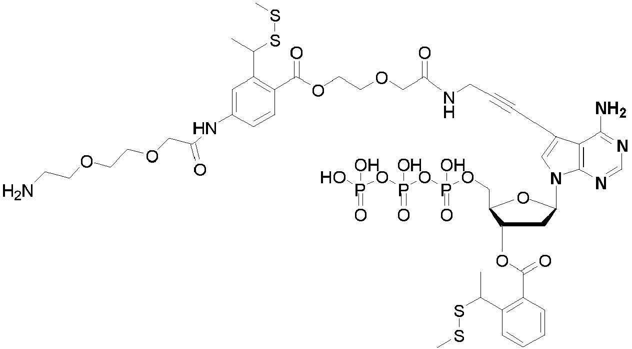 |
| 7D1 | 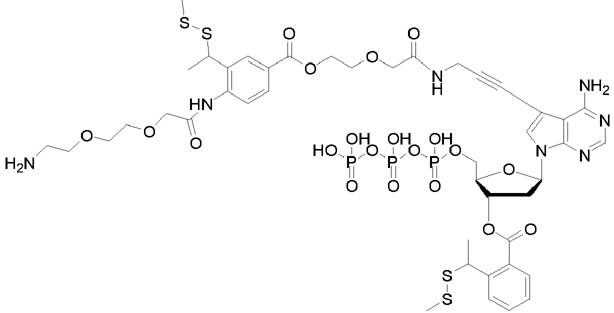 |

26. The compound or a salt thereof according to any one of claims 23 to 25, wherein the compound or a salt thereof carries an additional detectable label;
preferably, the additional detectable label carried by the compound or a salt thereof is introduced by an affinity reagent (e.g., antibody, aptamer, Affimer, Knottin), wherein the affinity reagent carries the detectable label, and the affinity reagent can specifically recognize and bind to an epitope of the compound or a salt thereof;
or preferably, the detectable label is linked to the R in the compound of formula I-1 according to any one of claims 23 to 24 or to a structural moiety corresponding to the R in the compound according to claim 25;
preferably, the detectable label is linked to the terminal amino group in 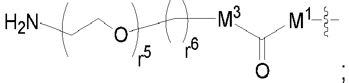
preferably, the carboxyl in the detectable label is linked to the terminal amino group in 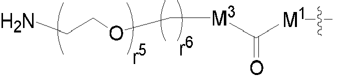 via formation of an amide bond;
preferably, base¹ is different, and the additional detectable label carried by the compound of formula I-1 varies;
preferably, the detectable label is a fluorescent label;
preferably, the detectable label is selected from: iF700,

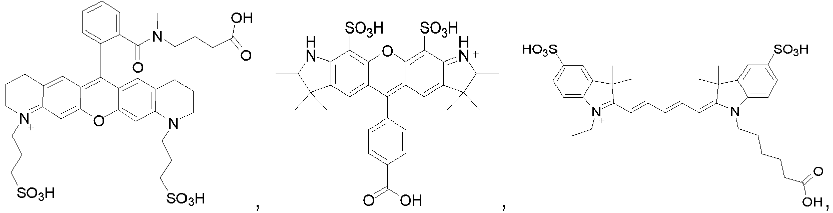

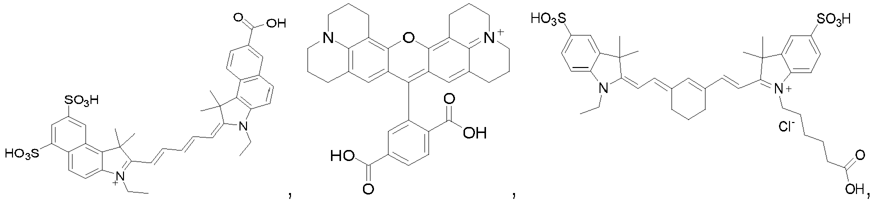

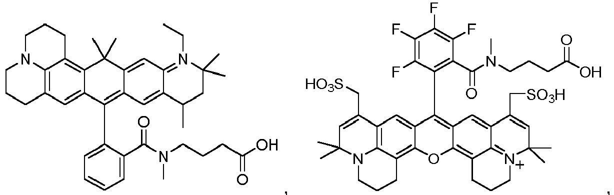

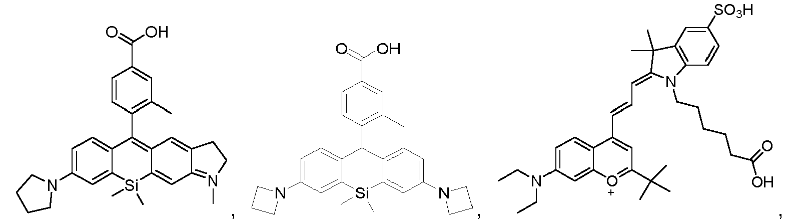

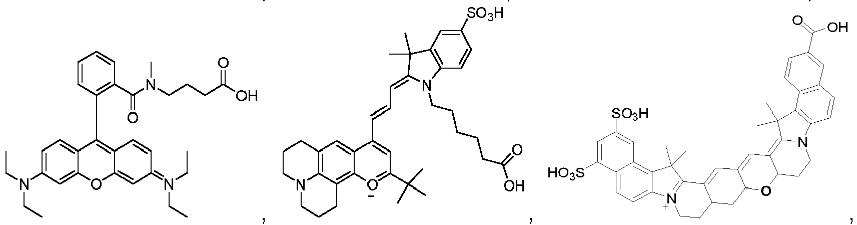

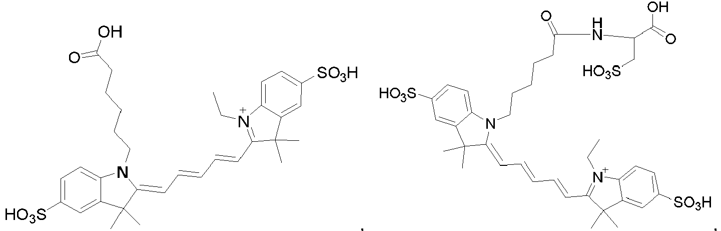

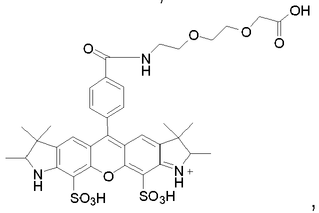

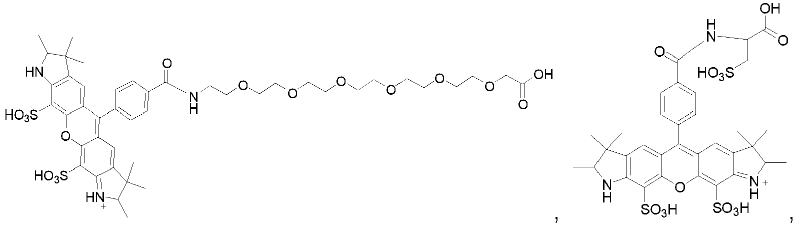

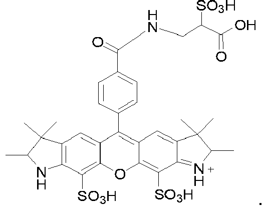

27. The compound or a salt thereof according to claim 26, wherein the compound is selected from:
| Serial Number | Structural formulae |
|---|---|
| 7A' | 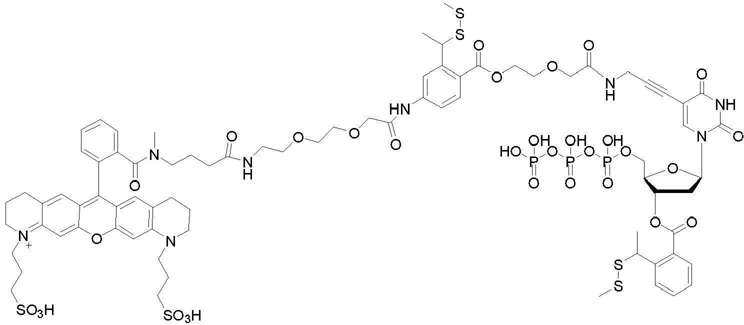 |
| 7 B' | 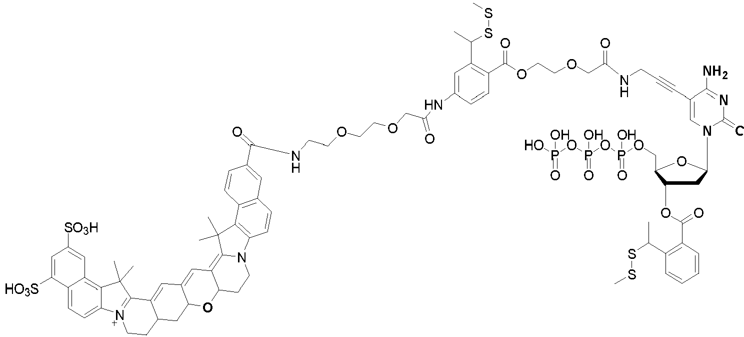 |
| 7B" | 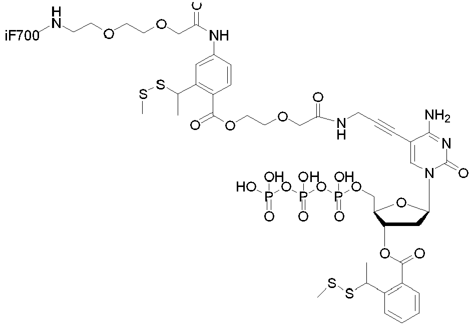 |
| 7C' | 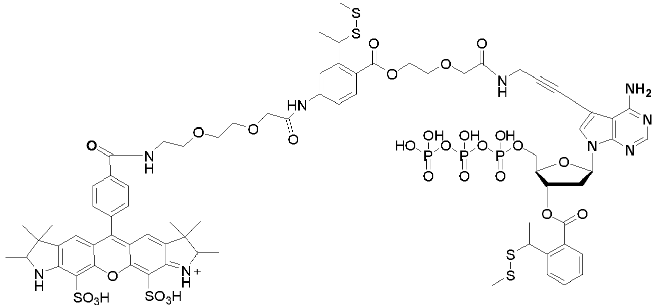 |
| 7C" | 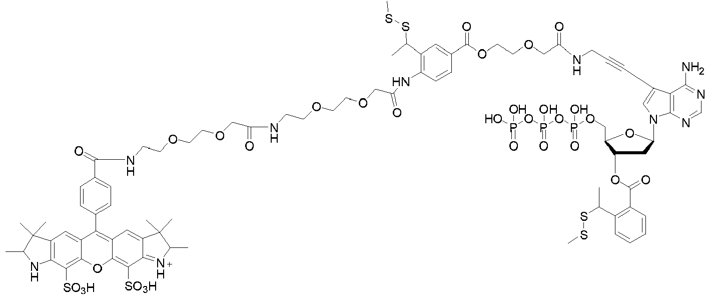 |
| 7C‴ | 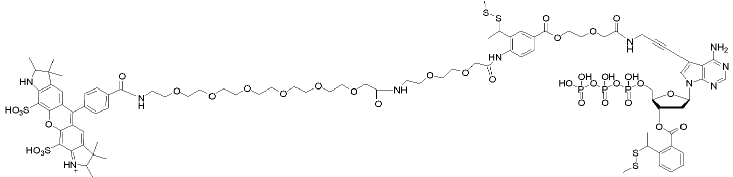 |
| 7Cʺʺ | 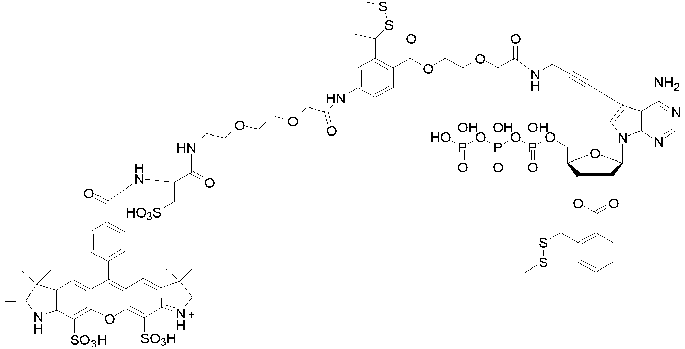 |
| 7Cʺ‴ | 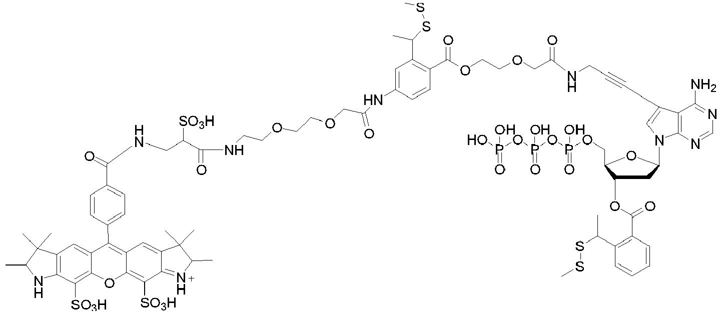 |
| 7D' | 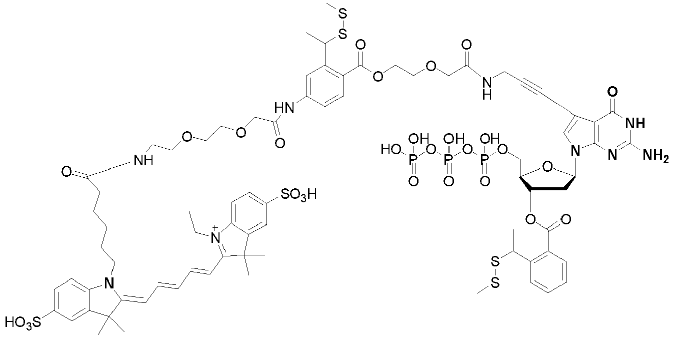 |
| 7D" | 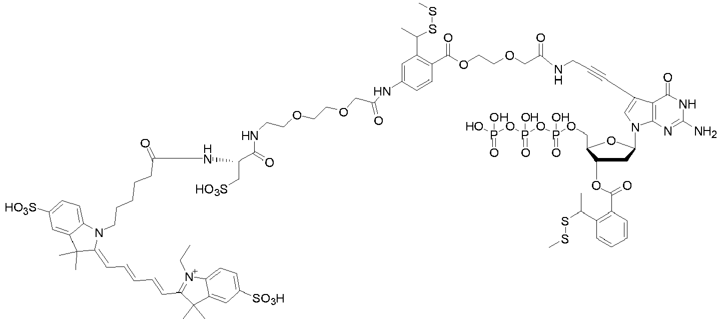 |

28. A method for terminating a nucleic acid synthesis, which comprises incorporating the compound or a salt thereof according to any one of claims 23 to 27 into a nucleic acid molecule to be terminated;
preferably, the incorporation of the compound or a salt thereof is achieved by a terminal transferase, a terminal polymerase or a reverse transcriptase;
preferably, the method comprises incorporating the compound or a salt thereof into the nucleic acid molecule to be terminated by using a polymerase;
preferably, the method comprises performing a nucleotide polymerization reaction using a polymerase under conditions where the polymerase is allowed to carry out the nucleotide polymerization reaction, thereby incorporating the compound or a salt thereof into the 3' end of the nucleic acid molecule to be terminated.

29. A method for preparing a growing polynucleotide complementary to a target single-stranded polynucleotide in a sequencing reaction, which comprises incorporating the compound or a salt thereof according to any one of 23 to 27 into the growing complementary polynucleotide, wherein the incorporation of the compound or a salt thereof prevents any subsequent nucleotides from being introduced into the growing complementary polynucleotide;
preferably, the incorporation of the compound or a salt thereof is achieved by a terminal transferase, a terminal polymerase or a reverse transcriptase;
preferably, the method comprises incorporating the compound or a salt thereof into the growing complementary polynucleotide by using a polymerase;
preferably, the method comprises performing a nucleotide polymerization reaction using a polymerase under conditions where the polymerase is allowed to carry out the nucleotide polymerization reaction, thereby incorporating the compound or a salt thereof into the 3' end of the growing complementary polynucleotide.

30. A nucleic acid intermediate, which is formed in determining the sequence of a target single-stranded polynucleotide, wherein,
the nucleic acid intermediate is formed according to the step:
incorporating a nucleotide complementary to the target single-stranded polynucleotide into a growing nucleic acid strand, to form the nucleic acid intermediate, wherein, the incorporated complementary nucleotide is the compound or a salt thereof according to any one of claims 23 to 27;
or, the nucleic acid intermediate is formed according to the step:
incorporating a nucleotide complementary to the target single-stranded polynucleotide into a growing nucleic acid strand, to form the nucleic acid intermediate, wherein, the incorporated complementary nucleotide is the compound or a salt thereof according to any one of claims 23 to 27, and the growing nucleic acid strand is pre-incorporated with at least one nucleotide complementary to the target single-stranded polynucleotide, the at least one pre-incorporated nucleotide complementary to the target single-stranded polynucleotide being the compound or a salt thereof according to any one of claims 23 to 27 in which the reversible blocking group and optional detectable label have been removed.

31. A method for determining the sequence of a target single-stranded polynucleotide, which comprises:
1) monitoring the incorporation of nucleotides complementary to the target single-stranded polynucleotide in a growing nucleic acid strand, wherein at least one complementary nucleotide incorporated is the compound or a salt thereof according to any one of claims 23 to 27, and,
2) determining the types of the incorporated nucleotides;
preferably, the reversible blocking group and optional detectable label are removed before a next complementary nucleotide is introduced;
preferably, the reversible blocking group and the detectable label are removed simultaneously;
preferably, the reversible blocking group and the detectable label are removed sequentially; for example, after the detectable label is removed, the reversible blocking group is removed, or, after the reversible blocking group is removed, the detectable label is removed.

32. The method according to claim 31, which comprises the following steps of:
(a) providing a plurality of different nucleotides, wherein at least one nucleotide is the compound or a salt thereof according to any one of claims 26 to 27, optionally, the rest of nucleotides are the compound or a salt thereof according to any one of claims 23 to 27;
(b) incorporating the plurality of different nucleotides into a sequence complementary to a target single-stranded polynucleotide, wherein the plurality of different nucleotides can be distinguished from each other during the detection;
(c) detecting the nucleotides in step (b) to determine the types of nucleotides incorporated;
(d) removing the reversible blocking groups and optional detectable labels carried by the nucleotides in step (b); and
(e) optionally repeating steps (a)-(d) one or more times;
whereby the sequence of the target single-stranded polynucleotide is determined.

33. The method according to claim 31, which comprises the following steps of:
(1) providing a first nucleotide, a second nucleotide, a third nucleotide and a fourth nucleotide, wherein at least one of the four nucleotides is the compound or a salt thereof according to any one of claims 26 to 27, optionally, the rest of nucleotides are the compound or a salt thereof according to any one of claims 23 to 27;
(2) contacting the four nucleotides with a target single-stranded polynucleotide; removing the nucleotides not incorporated into the growing nucleic acid strand; detecting the nucleotides incorporated into the growing nucleic acid strand; removing the reversible blocking groups and optional detectable labels carried in the nucleotides that are incorporated into the growing nucleic acid strand;
optionally, it also includes (3): repeating the steps (1)-(2) one or more times.

34. The method according to claim 31, which comprises the following steps of:
(a) providing a mixture comprising a duplex, nucleotides comprising at least one compound or a salt thereof according to any one of claims 26 to 27, a polymerase, and an excision reagent; wherein the duplex comprises a growing nucleic acid strand and a nucleic acid strand to be sequenced;
(b) carrying out a reaction comprising the following steps (i), (ii) and (iii), optionally, repeating the steps for one or more times:
step (i): incorporating the compound or a salt thereof into the growing nucleic acid strand by using a polymerase, to form a nucleic acid intermediate containing the reversible blocking group and optional detectable label;
step (ii): detecting the nucleic acid intermediate;
step (iii): removing the reversible blocking group and optional detectable label contained in the nucleic acid intermediate by using the excision reagent;
preferably, removal of the reversible blocking group and removal of the detectable label are performed simultaneously, or, removal of the reversible blocking group and removal of the detectable label are performed sequentially (for example, the reversible blocking group is removed first, or the detectable label is removed first);
preferably, the excision reagent used for removal of the reversible blocking group is the same as that used for removal of the detectable label;
preferably, the excision reagent used for removal of the reversible blocking group is different from that used for removal of the detectable label.

35. The method according to claim34, wherein the duplex is linked to a support;
preferably, the growing nucleic acid strand is a primer;
preferably, the primer is annealed to the nucleic acid strand to be sequenced to form the duplex;
preferably, the duplex, the compound or a salt thereof, and the polymerase together form a reaction system containing a solution phase and a solid phase;
preferably, the compound or a salt thereof is incorporated into the growing nucleic acid strand by using a polymerase under conditions where the polymerase is allowed to carry out a nucleotide polymerization reaction, thereby forming a nucleic acid intermediate containing the reversible blocking group and optional detectable label;
preferably, the polymerase is selected from KOD polymerase or a mutant thereof (e.g., KOD POL151, KOD POL157, KOD POL171, KOD POL174, KOD POL376, KOD POL391);
preferably, before any step of detecting the nucleic acid intermediate, the solution phase of the reaction system in the previous step is removed, and the duplex linked to the support is retained;
preferably, the excision reagent is in contact with the duplex or the growing nucleic acid strand in the reaction system containing a solution phase and a solid phase;
preferably, the excision reagent can remove the reversible blocking group and optional detectable label carried by the compound that is incorporated into the growing nucleic acid strand, without affecting the phosphodiester bond on the backbone of the duplex;
preferably, after any step of removing the reversible blocking group and optional detectable label contained in the nucleic acid intermediate, the solution phase of the reaction system in this step is removed;
preferably, a washing operation is performed after any step comprising a removal operation;
preferably, after step (ii), the method further comprises: determining the type of compound incorporated into the growing nucleic acid strand in step (i) according to the signal detected in step (ii), and determining the type of nucleotide at a corresponding position in the nucleic acid strand to be sequenced based on the principle of base complementary pairing.

36. A kit, which comprises at least one compound or a salt thereof according to any one of claims 23 to 27;
preferably, the kit comprises a first compound, a second compound, a third compound and a fourth compound, wherein the first, second, third and fourth compounds each are, independently, the compound or a salt thereof according to any one of claims 23 to 27;
preferably, in the first compound, base¹ is selected from adenine, 7-deazaadenine or a tautomer thereof (e.g., 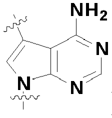 ); in the second compound, base¹ is selected from thymine, uracil or a tautomer thereof (e.g., 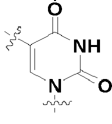 ); in the third compound, base¹ is selected from cytosine or a tautomer thereof (e.g., 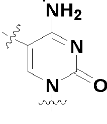 ); in the fourth compound, base¹ is selected from guanine, 7-deazaguanine or a tautomer thereof (e.g., 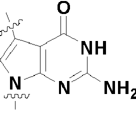 );
preferably, wherein the base¹ contained in the first, second, third and fourth compounds are different from each other;
preferably, wherein the additional detectable labels carried by the first, second, third and fourth compounds are different from each other.

37. The kit according to claim 36, wherein the kit further comprises a reagent for pretreating the nucleic acid molecule; a support for linking nucleic acid molecule to be sequenced; a reagent for linking (for example, covalently or non-covalently linking) the nucleic acid molecule to be sequenced to the support; primers for initiating a nucleotide polymerization reaction; a polymerase for carrying out the nucleotide polymerization reaction; one or more buffer solutions; one or more washing solutions; or any combination thereof.

38. Use of the compound or a salt thereof according to any one of claims 23 to 27 or the kit according to any one of claims 36 to 37 for determining the sequence of a target single-stranded polynucleotide.

39. A nucleotide analogue, which is formed by a ribose or deoxyribose, a reversible blocking group, base or deaza base or a tautomer thereof, a linker for linking to a detectable label, and an optional phosphate group, wherein the linker comprises the structure shown in the following formula A or formula A': 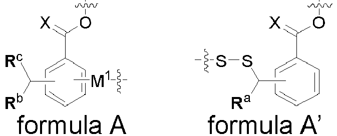 wherein:
in R^{b}, R^{c}, any one is selected from -N₃, -SS-C₁-C₆ alkyl (e.g., -SS-Me, -SS-Et, -SS-iPr, -SS-t-Bu), -ONH₂, -OCORₓ, -OCONHRₓ, -S-SO₂Rₓ, and the other is selected from H, aliphatic alkyl (e.g., C₁-C₆ alkyl, such as Me, Et, iPr, tBu), aromatic alkyl (e.g., phenyl -C₁-C₆ alkyl), cycloalkyl (e.g., C₃-C₆ cycloalkyl), wherein each Rₓ, independently, is selected from aliphatic alkyl (e.g., C₁-C₆ alkyl), cycloalkyl (e.g., C₃-C₆ cycloalkyl) or aromatic alkyl (e.g., phenyl C₁-C₆ alkyl);
preferably, in R^{b}, R^{c}, any one is selected from -N₃, -SS-C₁-C₆ alkyl (e.g., - SS-Me, -SS-Et, -SS-iPr, -SS-t-Bu), and the other is selected from C₁-C₆ alkyl;
more preferably, in R^{b}, R^{c}, any one is selected from -N₃, -SS-Me, -SS-Et, and the other is methyl;
most preferably, in R^{b}, R^{c}, any one is selected from -N₃, -SS-Me, and the other is methyl;
R^{a} is selected from H, aliphatic alkyl (e.g., C₁-C₆ alkyl, such as Me, Et, iPr, tBu), aromatic alkyl (e.g., phenyl -C₁-C₆ alkyl), cycloalkyl (e.g., C₃-C₆ cycloalkyl);
preferably, R^{a} is selected from H, C₁-C₆ alkyl;
more preferably, R^{a} is methyl;
X is selected from O, S, NH;
preferably, X is selected from O, S;
more preferably, X is O;
M¹ is selected from a direct bond, NH, O, S, CH₂;
preferably, M¹ is selected from a direct bond, NH, O;
preferably, the reversible blocking group is linked to the 3'-OH of the ribose or deoxyribose, the base or deaza base or a tautomer thereof is linked to the 1'-C of the ribose or deoxyribose, the optional phosphate group is linked to the 5'-OH of the ribose or deoxyribose, and the linker is linked to the base or deaza base or a tautomer thereof.

40. The nucleotide analogue according to claim 39, wherein, the structure of formula A is selected from formula A-1, formula A-2, formula A-3, formula A-4 or formula A-5, preferably the structure of formula A is formula A-1, formula A-2 or formula A-5; or, the structure of formula A' is selected from formula A'-1, formula A'-2 or formula A'-3, preferably the structure of formula A' is formula A'-1; 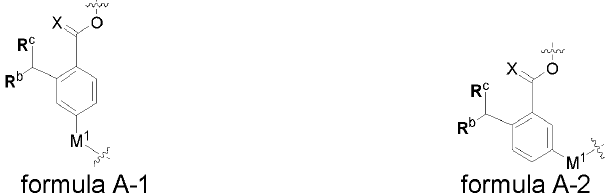 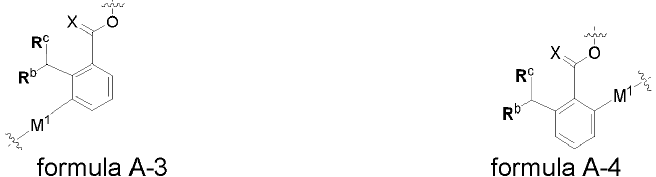 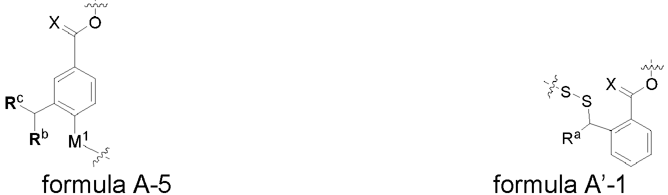 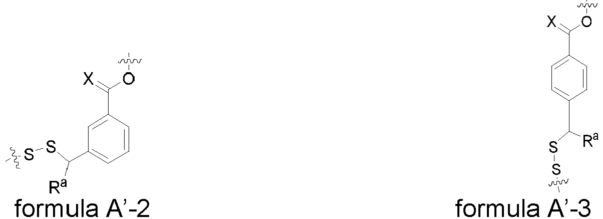 wherein:
R^{a}, R^{b}, R^{c}, X, M¹ have the same definitions as above.

41. The nucleotide analogue according to any one of claims 39 to 40, wherein, the structure of formula A is selected from: 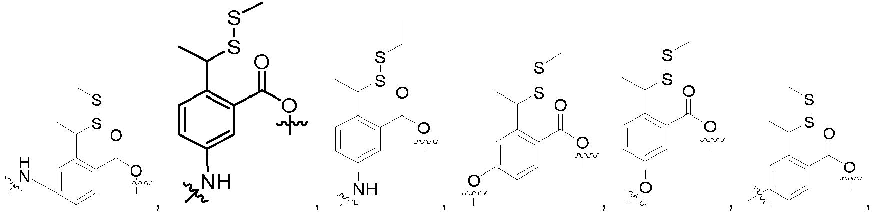 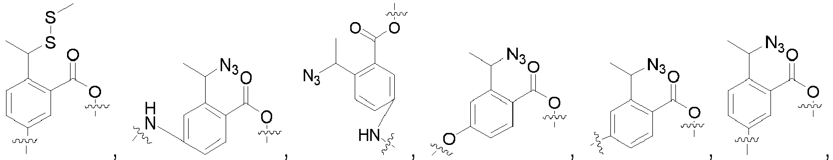 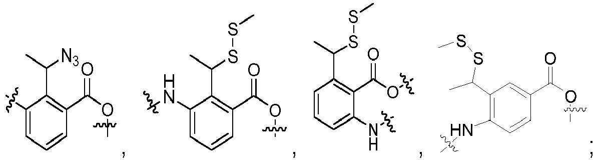 or, the structural of formula A' is

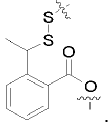

42. The nucleotide analogue according to any one of claims 39 to 41, wherein the structure of the nucleotide analogue is shown in the following formula B: 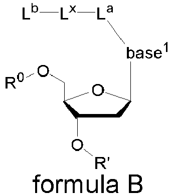
L^{x} has the structure of formula A, formula A', formula A-1, formula A-2, formula A-3, formula A-4, formula A-5, formula A'-1, formula A'-2 or formula A'-3 according to any one of claims 39 to 41, and the M¹ end or S end of L^{x} is linked to L^{b}, the O end of L^{x} is linked to L^{a}; 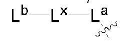
represents a linker for linking to a detectable label;
base¹ represents a base or deaza base or a tautomer thereof;
R' represents a reversible blocking group;
R⁰ represents H or phosphate group;
L^{a} represents the moiety in the linker for linking to the base or deaza base or a tautomer thereof;
L^{b} represents the moiety in the linker for linking to the detectable label.

43. The nucleotide analogue according to any one of claims 39 to 42, wherein the structure of the nucleotide analogue is shown in the following formula B: 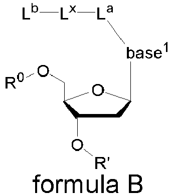 wherein:
L^{x} has the structure of formula A, formula A', formula A-1, formula A-2, formula A-3, formula A-4, formula A-5, formula A'-1, formula A'-2 or formula A'-3 as shown in any one of claims 39 to 41, and the M¹ end or S end of L^{x} is linked to L^{b}, the O end of L^{x} is linked to L^{a};
L^{a} is selected from 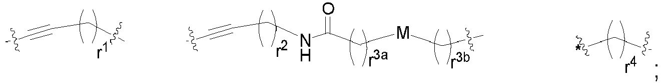
preferably, L^{a} is selected from 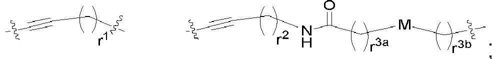
or preferably, L^{a} is selected from: 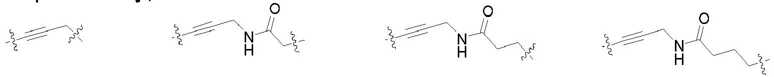 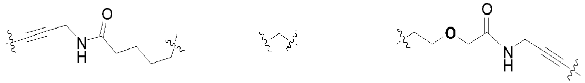
or more preferably, L^{a} is selected from: 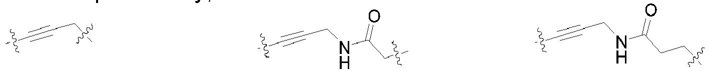 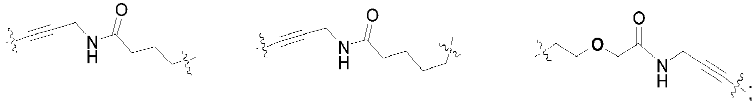
or more preferably, L^{a} is selected from: 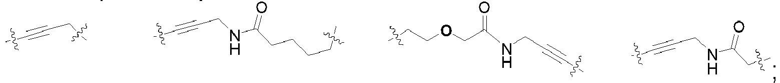
or most preferably, L^{a} is selected from: 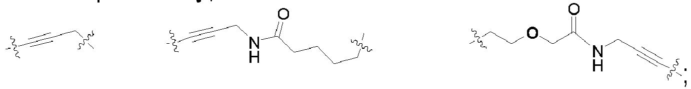
r¹, r², r^{3a}, r^{3b}, r⁴, independently of each other, are selected from any integer between 1 and 6;
preferably, r¹ is selected from 1, 2, 3;
more preferably, r¹ is 1;
preferably, r² is selected from 1, 2, 3;
more preferably, r² is 1;
preferably, r^{3a}, r^{3b}, independently of each other, are selected from 0, 1, 2, 3, 4, 5, and r^{3a}, r^{3b} being not 0 at the same time;
more preferably, r^{3a}, r^{3b}, independently of each other, are selected from 0, 1, 2, 3, and r^{3a}, r^{3b} being not 0 at the same time;
further preferably, r^{3a} is selected from 0, 1, r^{3b} is selected from 0, 2, and r^{3a}, r^{3b} being not 0 at the same time;
most preferably, r^{3a} is 1;
most preferably, r^{3b} is 2;
preferably, r⁴ is selected from 1, 2, 3;
more preferably, r⁴ is 1;
M is selected from a direct bond, CH₂, NH, O, S;
preferably, M is selected from a direct bond, CH₂, O;
more preferably, M is selected from CH₂, O;
L^{b} is selected from 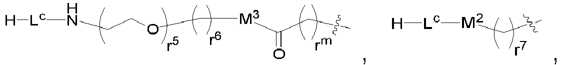 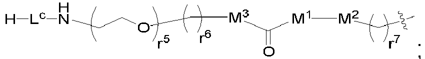
preferably, L^{b} is selected from 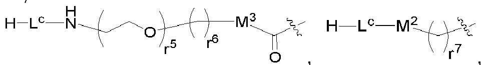 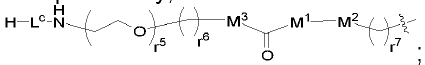
more preferably, L^{b} is selected from 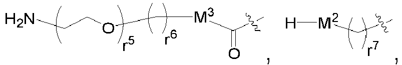 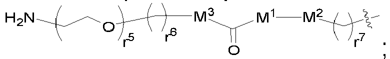
most preferably, L^{b} is selected from: 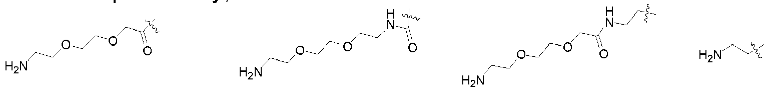 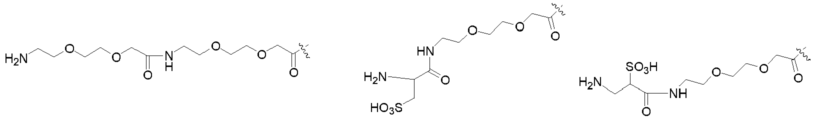 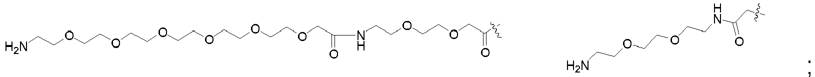
L^{c} is selected from a direct bond 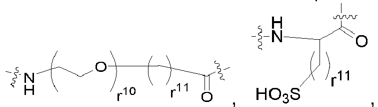 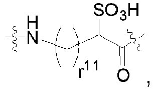 preferably, the NH end in L^{c} is linked to H in L^{b}, the =O end in L^{c} is linked to NH in L^{b};
preferably, L^{c} is selected from a direct bond 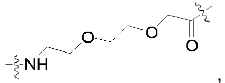 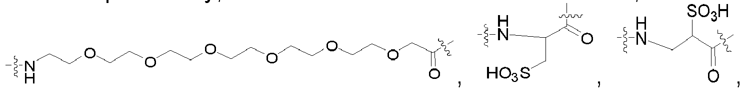 preferably, the NH end in L^{c} is linked to H in L^{b}, the =O end in L^{c} is linked to NH in L^{b};
r^{m} is selected from any integer between 0 and 6;
preferably, r^{m} is selected from 0, 1, 2, 3;
more preferably, r^{m} is selected from 0, 1;
r⁵, r⁶, r⁷, independently of each other, are selected from any integer between 1 and 6;
preferably, r⁵ is selected from 1, 2, 3;
more preferably, r⁵ is 2;
preferably, r⁶ is selected from 1, 2, 3;
more preferably, r⁶ is 1 or 2;
preferably, r⁷ is selected from 1, 2, 3;
more preferably, r⁷ is 2;
r¹⁰ is selected from any integer between 1 and 10;
preferably, r¹⁰ is selected from any integer between 2 and 6;
more preferably, r¹⁰ is 2 or 6;
r¹¹ is selected from any integer between 1 and 6;
preferably, r¹¹ is selected from 1, 2, 3;
more preferably, r¹¹ is 1;
M¹, M², M³, independently of each other, are selected from a direct bond, NH, O, S, CH₂;
preferably, M¹ is selected from a direct bond, NH, O;
preferably, M² is NH;
preferably, M³ is selected from a direct bond, NH;
base¹ represents base or deaza base;
R' represents a reversible blocking group; 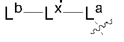
represents linker for linking to a detectable label;
R⁰ represents H or phosphate group.

44. The nucleotide analogue according to any one of claims 42 to 43, wherein the base¹ is selected from adenine, 7-deazaadenine, thymine, uracil, cytosine, guanine, 7-deazauanine or a tautomer thereof;
preferably, base¹ is selected from

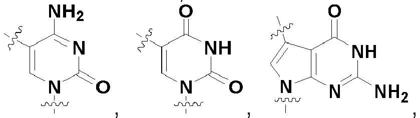

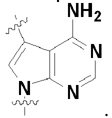

45. The nucleotide analogue according to any one of claims 42 to 44, wherein, R⁰ is selected from H monophosphate group 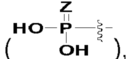 diphosphate group 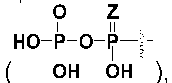 triphosphate group 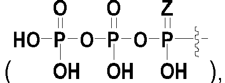 tetraphosphate group 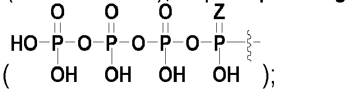 preferably, R° is a triphosphate group 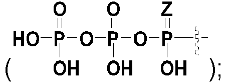
each Z, independently, is selected from O, S, BH;
preferably, Z is O.

46. The nucleotide analogue according to any one of claims 42 to 45, wherein the reversible blocking group R' is selected from N₃-C₁-C₆ alkyl, C₁-C₆ alkyl-SS-C₁-C₆ alkyl, 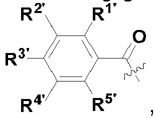 NH₂, -ONH₂, -OCOR_{z}, -OCONHR_{z}, wherein each R_{z}, independently, is selected from aliphatic alkyl (e.g., C₁-C₆ alkyl), cycloalkyl (e.g., C₃-C₆ cycloalkyl) or aromatic alkyl (e.g., phenyl C₁-C₆ alkyl);
preferably, the reversible blocking group R' is selected from N₃-C₁-C₆ alkyl, C₁-C₆ alkyl-SS-C₁-C₆ alkyl, 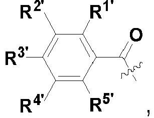 NH₂;
more preferably, the reversible blocking group R' is selected from N₃-C₁-C₆ alkyl, C₁-C₆ alkyl-SS-C₁-C₆ alkyl, 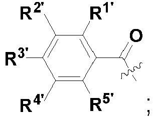
most preferably, the reversible blocking group R' is selected from N₃-CH₂-CH₃-CH₂-S-S-CH₂-, 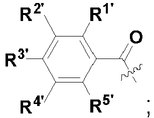
in R^{1'}, R^{2'}, R^{3'}, R^{4'}, R^{5'}, any one (e.g., R^{1'} or R^{5'}) is 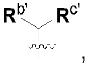 and the others, independently of each other, are selected from H, azido, nitro, amino, sulfo, carboxyl, aliphatic alkyl (e.g., C₁-C₆ alkyl), cycloalkyl (e.g., C₃-C₆ cycloalkyl), aromatic alkyl (e.g., phenyl -C₁-C₆ alkyl), F, I, Br, Cl, alkoxyl (e.g., C₁-C₆ alkoxyl),
preferably, in R^{1'}, R^{2'}, R^{3'}, R^{4'}, R^{5'}, any one (e.g., R^{1'} or R^{5'}) is 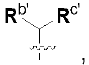 another (e.g., R^{3'}) is selected from H, C₁-C₆ alkoxyl (e.g., methoxy), and the others are H,
more preferably, in R^{1'}, R^{2'}, R^{3'}, R^{4'}, R^{5'}, any one (e.g., R^{1'} or R^{5'}) is 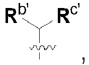 and the others are H,
most preferably, in R^{1'}, R^{2'}, R^{3'}, R^{4'}, R^{5'}, R^{1'} is 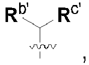 and the others are H,
in R^{b'}, R^{c'}, any one is selected from -N₃, -SS-C₁-C₆ alkyl (e.g., -SS-Me, -SS-Et, -SS-iPr, -SS-t-Bu), -ONH₂, -OCORₓ, -OCONHRₓ, and the other is selected from H, aliphatic alkyl (e.g., C₁-C₆ alkyl, such as Me, Et, iPr, tBu), aromatic alkyl (e.g., phenyl -C₁-C₆ alkyl), cycloalkyl (e.g., C₃-C₆ cycloalkyl), wherein each Rₓ, independently, is selected from aliphatic alkyl (e.g., C₁-C₆ alkyl), cycloalkyl (e.g., C₃-C₆ cycloalkyl) or aromatic alkyl (e.g., phenyl C₁-C₆ alkyl),
preferably, in R^{b'}, R^{c'}, any one is selected from -N₃, -SS-C₁-C₆ alkyl (e.g., -SS-Me, -SS-Et, -SS-iPr, -SS-t-Bu), and the other is selected from C₁-C₆ alkyl,
more preferably, in R^{b'}, R^{c'}, any one is selected from -N₃, -SS-Me, and the other is methyl.

47. The nucleotide analogue according to any one of claims 39 to 46, wherein it is selected from the compound according to claim 9.

48. The nucleotide analogue according to any one of claims 39 to 47, wherein the nucleotide analogue carries an additional detectable label, and the detectable label is linked to a linker (preferably L^{b});
preferably, the additional detectable label carried by the nucleotide analogue is introduced by an affinity reagent (e.g., antibody, aptamer, Affimer, Knottin), wherein the affinity reagent carries the detectable label, and the affinity reagent can specifically recognize and bind to an epitope of the nucleotide analogue; preferably, the detectable label is linked to the terminal amino group in L^{b};
preferably, the carboxyl in the detectable label is linked to the terminal amino group in L^{b} via formation of an amide bond;
preferably, base¹ is different, and the additional detectable label carried by the nucleotide analogue varies;
preferably, the detectable label is a fluorescent label;
preferably, the detectable label is selected from: iF700,

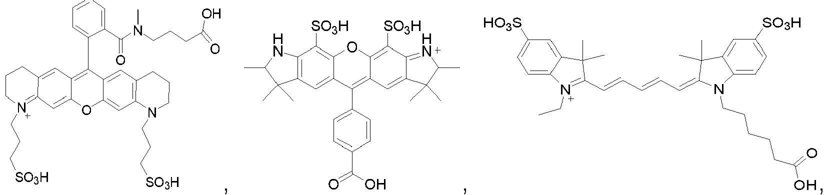

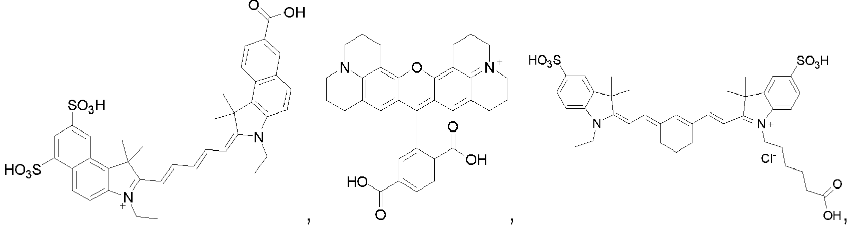

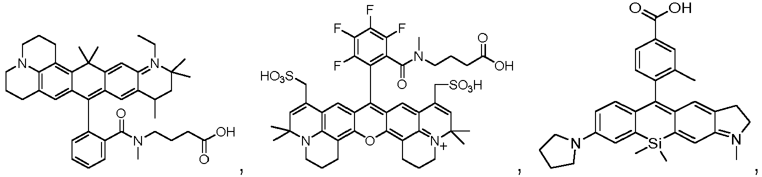

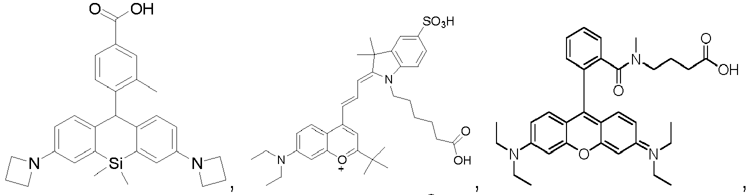

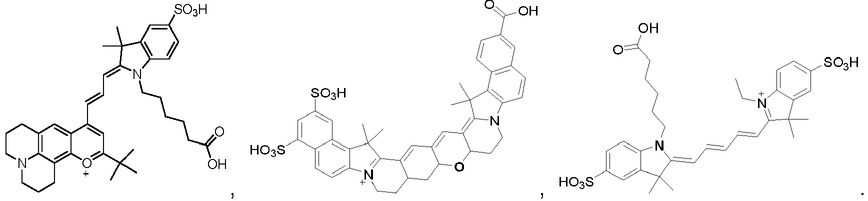

49. The nucleotide analogue according to claim 48, wherein the nucleotide analogue is selected from the compound according to claim 11.

50. A method for terminating a nucleic acid synthesis, comprising: incorporating the nucleotide analogue according to any one of claims 39 to 49 into a nucleic acid molecule to be terminated;
preferably, the incorporation of the nucleotide analogue is achieved by a terminal transferase, a terminal polymerase or a reverse transcriptase;
preferably, the method comprises incorporating the nucleotide analogue into the nucleic acid molecule to be terminated by using a polymerase;
preferably, the method comprises performing a nucleotide polymerization reaction using a polymerase under conditions where the polymerase is allowed to carry out the nucleotide polymerization reaction, thereby incorporating the nucleotide analogue into the 3' end of the nucleic acid molecule to be terminated.

51. A method for preparing a growing polynucleotide complementary to a target single-stranded polynucleotide in a sequencing reaction, comprising incorporating the nucleotide analogue according to any one of claims 39 to 49 into the growing complementary polynucleotide, wherein the incorporation of the nucleotide analogue prevents any subsequent nucleotides from being introduced into the growing complementary polynucleotide;
preferably, the incorporation of the nucleotide analogue is achieved by a terminal transferase, a terminal polymerase or a reverse transcriptase;
preferably, the method comprises incorporating the nucleotide analogue into the growing complementary polynucleotide by using a polymerase;
preferably, the method comprises performing a nucleotide polymerization reaction using a polymerase under conditions where the polymerase is allowed to carry out the nucleotide polymerization reaction, thereby incorporating the nucleotide analogue into the 3' end of the growing complementary polynucleotide.

52. A nucleic acid intermediate, which is formed in determining the sequence of a target single-stranded polynucleotide, wherein,
the nucleic acid intermediate is formed according to the step:
incorporating a nucleotide complementary to the target single-stranded polynucleotide into a growing nucleic acid strand, to form the nucleic acid intermediate, wherein the incorporated complementary nucleotide is the nucleotide analogue according to any one of claims 39 to 49;
or, the nucleic acid intermediate is formed according to the step:
incorporating a nucleotide complementary to the target single-stranded polynucleotide into a growing nucleic acid strand, to form the nucleic acid intermediate, wherein, the incorporated complementary nucleotide is the nucleotide analogue according to any one of claims 39 to 49, and the growing nucleic acid strand is pre-incorporated with at least one nucleotide complementary to the target single-stranded polynucleotide, the at least one pre-incorporated nucleotide complementary to the target single-stranded polynucleotide being the nucleotide analogue according to any one of claims 39 to 49 in which the reversible blocking group and optional detectable label have been removed.

53. A method for determining the sequence of a target single-stranded polynucleotide, which comprises:
1) monitoring the incorporation of nucleotides complementary to the target single-stranded polynucleotide in a growing nucleic acid strand, wherein at least one complementary nucleotide incorporated is the nucleotide analogue according to any one of claims 39 to 49, and,
2) determining the types of the incorporated nucleotides;
preferably, the reversible blocking group and optional detectable label are removed before a next complementary nucleotide is introduced;
preferably, the reversible blocking group and the detectable label are removed simultaneously;
preferably, the reversible blocking group and the detectable label are removed sequentially; for example, after the detectable label is removed, the reversible blocking group is removed, or, after the reversible blocking group is removed, the detectable label is removed.

54. The method according to claim 53, which comprises the following steps of:
(a) providing a plurality of different nucleotides, wherein at least one nucleotide is the nucleotide analogue according to any one of claims 48 to 49, optionally, the rest of nucleotides are the nucleotide analogue according to any one of claims 39 to 49;
(b) incorporating the plurality of different nucleotides into a sequence complementary to a target single-stranded polynucleotide, wherein the plurality of different nucleotides can be distinguished from each other during the detection;
(c) detecting the nucleotides in step (b) to determine the types of nucleotides incorporated;
(d) removing the reversible blocking groups and optional detectable labels carried by the nucleotides in step (b); and
(e) optionally repeating steps (a)-(d) one or more times;
whereby the sequence of the target single-stranded polynucleotide is determined.

55. The method according to claim 53, which comprises the following steps of:
(1) providing a first nucleotide, a second nucleotide, a third nucleotide and a fourth nucleotide, wherein at least one of the four nucleotides is the nucleotide analogue according to any one of claims 48 to 49, optionally, the rest of nucleotides are the nucleotide analogue according to any one of claims 39 to 49;
(2) contacting the four nucleotides with a target single-stranded polynucleotide; removing the nucleotides not incorporated into the growing nucleic acid strand; detecting the nucleotides incorporated into the growing nucleic acid strand; removing the reversible blocking groups and optional detectable labels carried in the nucleotides that are incorporated into the growing nucleic acid strand;
optionally, it also includes (3): repeating the steps (1)-(2) one or more times.

56. The method according to claim 53, which comprises the following steps of:
(a) providing a mixture comprising a duplex, nucleotides comprising at least one nucleotide analogue according to any one of claims 48 to 49, a polymerase, and an excision reagent; wherein the duplex comprises a growing nucleic acid strand and a nucleic acid strand to be sequenced;
(b) carrying out a reaction comprising the following steps (i), (ii) and (iii), optionally, repeating the steps for one or more times:
step (i): incorporating the nucleotide analogue into the growing nucleic acid strand by using a polymerase to form a nucleic acid intermediate containing the reversible blocking group and optional detectable label;
step (ii): detecting the nucleic acid intermediate;
step (iii): removing the reversible blocking group and optional detectable label contained in the nucleic acid intermediate by using the excision reagent;
preferably, removal of the reversible blocking group and removal of the detectable label are performed simultaneously, or, removal of the reversible blocking group and removal of the detectable label are performed sequentially (for example, the reversible blocking group is removed first, or the detectable label is removed first);
preferably, the excision reagent used for removal of the reversible blocking group is the same as that used for removal of the detectable label;
preferably, the excision reagent used for removal of the reversible blocking group is different from that used for removal of the detectable label.

57. The method according to claim 56, wherein the duplex is linked to a support;
preferably, the growing nucleic acid strand is a primer;
preferably, the primer is annealed to the nucleic acid strand to be sequenced to form the duplex;
preferably, the duplex, the nucleotide analogue, and the polymerase together form a reaction system containing a solution phase and a solid phase;
preferably, the nucleotide analogue is incorporated into the growing nucleic acid strand by using a polymerase under conditions where the polymerase is allowed to carry out a nucleotide polymerization reaction, thereby forming a nucleic acid intermediate containing the reversible blocking group and optional detectable label;
preferably, the polymerase is selected from KOD polymerase or a mutant thereof (e.g., KOD POL151, KOD POL157, KOD POL171, KOD POL174, KOD POL376, KOD POL391);
preferably, before any step of detecting the nucleic acid intermediate, the solution phase of the reaction system in the previous step is removed, and the duplex linked to the support is retained;
preferably, the excision reagent is in contact with the duplex or the growing nucleic acid strand in the reaction system containing a solution phase and a solid phase;
preferably, the excision reagent can remove the reversible blocking group and optional detectable label carried by the nucleotide analogue that is incorporated into the growing nucleic acid strand, without affecting the phosphodiester bond on the backbone of the duplex;
preferably, after any step of removing the reversible blocking group and optional detectable label contained in the nucleic acid intermediate, the solution phase of the reaction system in this step is removed;
preferably, a washing operation is performed after any step comprising a removal operation;
preferably, after step (ii), the method further comprises: determining the type of the nucleotide analogue incorporated into the growing nucleic acid strand in step (i) according to the signal detected in step (ii), and determining the type of nucleotide at a corresponding position in the nucleic acid strand to be sequenced based on the principle of base complementary pairing.

58. A kit, which comprises at least one nucleotide analogue according to any one of claims 39 to 49;
preferably, the kit comprises a first compound, a second compound, a third compound and a fourth compound, wherein the first, second, third and fourth compounds each are, independently, the nucleotide analogue according to any one of claims 39 to 49;
preferably, in the first compound, base¹ is selected from adenine, 7-deazaadenine or a tautomer thereof (e.g., 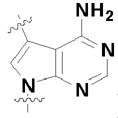 ); in the second compound, base¹ is selected from thymine, uracil or a tautomer thereof (e.g., 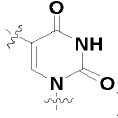 ); in the third compound, base¹ is selected from cytosine or a tautomer thereof (e.g., 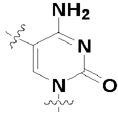 ); in the fourth compound, base¹ is selected from guanine, 7-deazaguanine or a tautomer thereof (e.g., 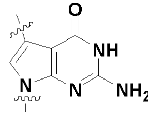 );
preferably, wherein the base¹ contained in the first, second, third and fourth compounds are different from each other;
preferably, wherein the additional detectable labels carried by the first, second, third and fourth compounds are different from each other.

59. The kit according to claim 58, wherein the kit further comprises a reagent for pretreating the nucleic acid molecule; a support for linking nucleic acid molecule to be sequenced; a reagent for linking (for example, covalently or non-covalently linking) the nucleic acid molecule to be sequenced to the support; primers for initiating a nucleotide polymerization reaction; a polymerase for carrying out the nucleotide polymerization reaction; one or more buffer solutions; one or more washing solutions; or any combination thereof.

60. Use of the nucleotide analogue according to any one of claims 39 to 49 or the kit according to any one of claims 58 to 59 for determining the sequence of a target single-stranded polynucleotide.
